(19) Europäisches Patentamt
European Patent Office
Office européen des brevets

(11) EP 3 915 991 A1

(12) **EUROPEAN PATENT APPLICATION**
published in accordance with Art. 153(4) EPC

(43) Date of publication:
**01.12.2021 Bulletin 2021/48**

(21) Application number: **20742007.6**

(22) Date of filing: **20.01.2020**

(51) Int Cl.:
*C07D 487/04* (2006.01)
*C07F 9/6584* (2006.01)
*C07F 9/6561* (2006.01)
*A61K 31/519* (2006.01)
*A61K 31/675* (2006.01)
*A61P 25/28* (2006.01)
*A61P 29/00* (2006.01)
*A61P 35/00* (2006.01)

(86) International application number:
**PCT/KR2020/000960**

(87) International publication number:
**WO 2020/149723 (23.07.2020 Gazette 2020/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.01.2019 KR 20190006913**

(71) Applicants:
- **Voronoi Co., Ltd.**
  **Yeonsu-gu**
  **Incheon 21984 (KR)**
- **Voronoibio Co., Ltd.**
  **Yeonsu-gu**
  **Incheon 21984 (KR)**

(72) Inventors:
- **JUNG, Myungho**
  **Hwaseong-si, Gyeonggi-do 18238 (KR)**
- **YUN, Jungyeon**
  **Goyang-si, Gyeonggi-do 10449 (KR)**
- **MA, Dahoon**
  **Incheon 21560 (KR)**
- **CHUNG, Sohyun**
  **Incheon 21518 (KR)**
- **JEON, Hyeonho**
  **Incheon 21982 (KR)**
- **RYU, Heesun**
  **Incheon 22231 (KR)**
- **KIM, Hyunkyung**
  **Incheon 21982 (KR)**
- **KIM, Hwan**
  **Seoul 03958 (KR)**
- **SON, Jungbeom**
  **Incheon 21986 (KR)**
- **KIM, Namdoo**
  **Incheon 22008 (KR)**
- **CHOI, Jieun**
  **Seoul 07651 (KR)**
- **KIM, Daekwon**
  **Daegu 42012 (KR)**

(74) Representative: **Plasseraud IP**
**66, rue de la Chaussée d'Antin**
**75440 Paris Cedex 09 (FR)**

# (54) PYRROLOPYRIMIDINE DERIVATIVE, AND PHARMACEUTICAL COMPOSITION FOR PREVENTING OR TREATING PROTEIN KINASE-RELATED DISEASE COMPRISING SAME AS ACTIVE INGREDIENT

(57) The present invention relates to a pyrrolopyrimidine derivative, and a pharmaceutical composition for preventing or treating protein kinase-related disease comprising the same as an active ingredient. The pyrrolopyrimidine derivative has excellent inhibitory activity against various protein kinases comprising LRRK2, and has an excellent effect of inhibiting the proliferation of triple-negative breast cancer cells. Therefore, the pharmaceutical composition comprising the pyrrolopyrimidine derivative as an active ingredient may be useful for the treatment or prevention of protein kinase-related diseases, in particular, cancer, degenerative brain diseases, and inflammatory diseases, and specifically, may be useful for the treatment of triple-negative breast cancer.

EP 3 915 991 A1

[Figure 1]

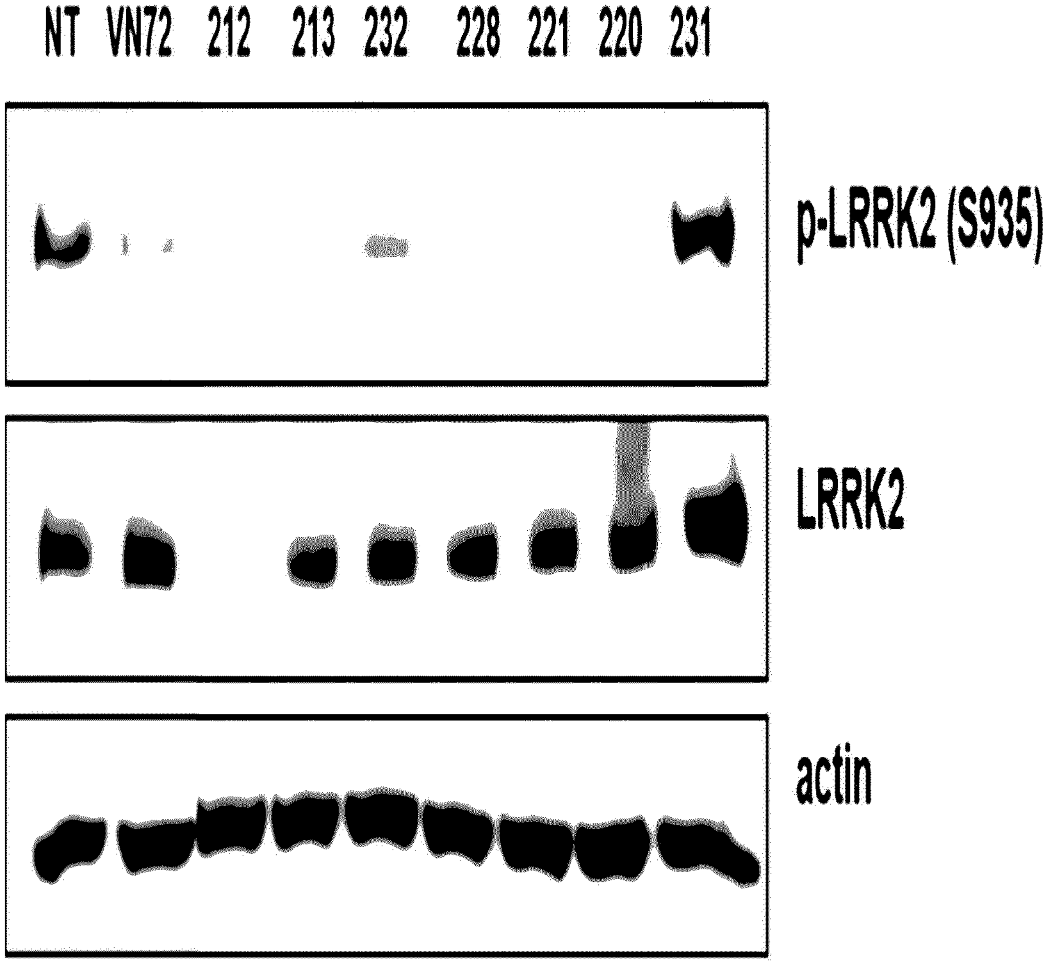

## Description

[Technical Field]

The present invention relates to a pyrrolopyrimidine derivative and a pharmaceutical composition for preventing or treating a protein kinase-related disease comprising the same as an active ingredient.

[Background Art]

A protein kinase is an enzyme that catalyzes the reaction to transfer a terminal phosphate group of adenosine triphosphate (ATP) to a specific residue (tyrosine, serine, threonine) of a protein, and is involved in signals that regulate cell activation, growth, and differentiation according to extracellular mediators and environmental changes.

Inappropriately high protein kinase activity is directly or indirectly involved in various diseases resulting from abnormal cellular functions. For example, mutations, over-expression, or failure of appropriate regulatory mechanisms of kinases involved in the inappropriate enzyme activity, or over-production or deficiency of factors involved in upstream or downstream signal transduction of cytokines or kinases may cause diseases. Therefore, the selective inhibition of kinase activity may be a beneficial target for the development of new drugs for treatment of diseases.

Brain cancer is a general term for primary brain cancer that develops in the brain tissue and the cerebral meninges surrounding the brain and secondary brain cancer that has metastasized from cancer in the skull or other parts of the body. Such brain cancer is distinguished from other cancers developed in other organs in many aspects. First, the cancers developed in lung, stomach, breast, and the like are limited to one or two types of cancer for each organ, and generally have identical or similar properties. However, many different types of cancers can develop in the brain. For example, polymorphic glioblastoma, malignant gliomas, lymphoma, blastomas, metastatic tumors, and the like can develop in the brain.

Parkinson's disease is the result of chronic progressive degeneration of neurons, but the cause of Parkinson's disease has not been fully identified yet. Although the major causes are unknown, Parkinson's disease is characterized by the degeneration of dopaminergic neurons in the substantia nigra (SN). The substantia nigra is a part of the lower brain or the brainstem that helps the regulation of unconscious movement. Dopamine deficiency in the brain caused by the loss of these neurons is known to cause observable symptoms. In a clinical aspect, the main symptoms of Parkinson's disease are expressed in the form of resting tremors, rigidity, bradykinesia, and postural instability. Not only the MAO-B inhibitor selegiline and the COMT inhibitor entacapone but also levodopa, dopamine agonists (for example, rotigotine, pramipexole, bromocryptine, ropinirole, cabergoline, pergolide, apomorphine and lisuride), anticholinergic drugs, NMDA antagonists, and β-blockers are used as medications for relieving symptoms relating to motion. Most of these drugs are involved in dopamine and/or choline signal transduction, by which the drugs affect typical motion dysfunction symptoms of Parkinson's disease.

LRRK2 (leucine-rich repeat kinase-2) is a protein belonging to the leucine-rich repeat kinase family, which has a sequence of 2,527 amino acids with high interspecific similarity. Characteristically, it contains both GTPase activity and serine-threonine kinase activity in one protein. The expressed LRRK2 is observed in various organs and tissues including the brain, and is known to be present in the cytoplasm or cell membrane and the mitochondrial outer membrane at a cellular level. In recent years, research on exact *in vivo* functions of LRRK2 has been actively conducted. LRRK2 has 5 functionally important domains which are expected to be involved in self-active regulation by autophosphorylation and cell function regulation through the protein interaction and enzymatic action. In particular, it is known that chaperone machinery, cytoskeleton arrangement, protein translational machinery, synaptic vesicle endocytosis, a mitogen-activated protein kinase signaling cascade, and an ubiquitin/autophage protein degradation pathway are regulated by LRRK2.

Parkinson's disease occurs sporadically in most cases, but 5-10% of the patients have a family history of it. From the studies using the samples of these patients, the loci of PARK 1-16 genes have been identified, among which a few loci have been confirmed to have mutations to cause Parkinson's disease. The causative genes of Parkinson's disease that cause Parkinson's disease by mutations are known to be parkin, PINK1, DJ-1, α-synuclein, leucine-rich repeat kinase 2 (LRRK2), and the like. Among them, the LRRK2 gene was first reported in 2004 as a dominant gene of a homologous chromosome like the α-synuclein. Unlike the causative genes of Parkinson's disease, patients with Parkinson's disease caused by the LRRK2 mutations have symptoms very similar to patients with sporadic Parkinson's disease. LRRK2 mutations are found not only in those Parkinson's disease patients who have a family history of it but also in 1-2% of sporadic Parkinson's disease patients. Therefore, identifying the pathogenesis of Parkinson's disease by mutations of this gene would be very helpful in understanding the pathogenesis of Parkinson's disease and developing therapeutic agents.

Also, LRRK2 is known to be involved in the transfer of mild cognitive impairment associated with Alzheimer's disease, L-Dopa induced dyskinesia, CNS disorders associated with neuronal progenitor differentiation, cancer such as brain cancer, kidney cancer, breast cancer, prostate cancer, blood cancer, lung cancer and acute myelogenous leukemia,

papillary kidney and thyroid carcinoma, multiple myeloma, amyotrophic lateral sclerosis, rheumatoid arthritis, and ankylosing spondylitis. Therefore, compounds or compositions which are effective in regulating the LRRK2 activity may provide therapeutic effects on neurodegenerative diseases, CNS disorders, cancers, acute myelogenous leukemia and multiple myeloma, inflammatory diseases, and the like.

[Related-Art Document]

[Non-Patent Document]

ACS Med. Chem. Lett., 2013, 4 (1), pp 85-90

[Disclosure]

[Technical Problem]

The object of the present invention is directed to providing a pyrrolopyrimidine derivative.

Another object of the present invention is directed to providing a pharmaceutical composition for use in preventing or treating a protein kinase-related disease.

Still another object of the present invention is directed to providing a method of preventing or treating a protein kinase-related disease.

Yet another object of the present invention is directed to providing a use of the pyrrolopyrimidine derivative or the pharmaceutically acceptable salt thereof for use in preparing medicaments for the prevention or treatment of the protein kinase-related disease.

[Technical Solution]

To solve the above problems,
according to one aspect of the present invention, there is provided a compound represented by the following Formula 1, or an isomer thereof, a solvate thereof, a hydrate thereof, or a pharmaceutically acceptable salt thereof:

[Formula 1]

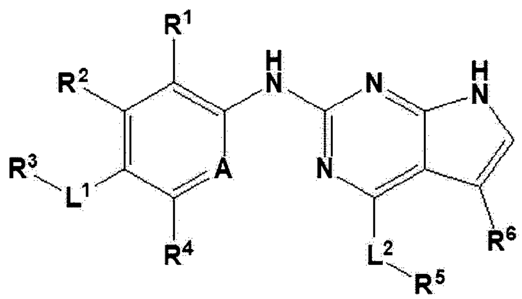

(wherein,

A represents a carbon atom or a nitrogen atom,
$R^1$ is hydrogen or a linear or branched $C_{1-6}$ alkoxy, and $R^2$ is hydrogen, or
$R^1$ and $R^2$, together with a benzene ring containing carbon atoms to which they are bonded, form an 8- to 10-membered bicyclic ring comprising one or more heteroatoms selected from the group consisting of N, O, and S,
$L^1$ is sulfonyl or carbonyl, or is absent,

when $L^1$ is sulfonyl or carbonyl, $R^3$ is a linear or branched $C_{1-6}$ alkyl, morpholinyl, piperidinyl, piperazinyl, pyrrolidinyl, amino, 2-oxa-5-azabicyclo[2.2.1]heptanyl, or azetidinyl, wherein $R^3$ is unsubstituted or substituted with one or more non-hydrogen substituents selected from the group consisting of morpholinyl, oxetanyl, 2-oxa-5-azabicyclo[2.2.1]heptanyl, piperazinyl, piperidinyl, a $C_{3-9}$ cycloalkyl, and a linear or branched $C_{1-6}$ alkyl, wherein the non-hydrogen substituents of $R^3$ are unsubstituted or substituted with a substituent selected from the group consisting of hydroxy, a $C_{3-9}$ cycloalkyl, and a linear or branched $C_{1-6}$ alkyl,
when $L^1$ is absent, $R^3$ is selected from azaphosphinane oxide and phosphoryl, wherein $R^3$ is unsubstituted or

substituted with one or more non-hydrogen substituents selected from the group consisting of oxetanyl, a $C_{3-9}$ cycloalkyl, a linear or branched $C_{1-6}$ alkyl, vinyl, tetrahydropyranyl, and benzyl, wherein non-hydrogen substituents of $R^3$ are unsubstituted or substituted with a $C_{1-6}$ alkoxy,

$R^4$ is hydrogen or a halogen,
$L^2$ is -NH- or -O-, or is absent,

when $L^2$ is -NH- or -O-, $R^5$ is selected from the group consisting of a linear or branched $C_{1-6}$ alkyl, a $C_{3-9}$ cycloalkyl, a 3- to 9-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-9}$ cycloalkyl, a $C_{1-6}$ alkylsulfonyl, a $C_{1-6}$ alkoxy, and a $C_{1-6}$ alkyl,
when $L^2$ is absent, $R^5$ is a linear or branched $C_{1-6}$ alkyl or a $C_{3-9}$ cycloalkyl, and $R^6$ is hydrogen, cyano, or a $C_{1-6}$ haloalkyl).

According to another aspect of the present invention, there is provided a pharmaceutical composition for use in preventing or treating a protein kinase-related disease, which comprises the compound of Formula 1, or the isomer thereof, the solvate thereof, the hydrate thereof, or the pharmaceutically acceptable salt thereof as an active ingredient.
According to still another aspect of the present invention, there is provided a method of preventing or treating a protein kinase-related disease, which comprises administering the pharmaceutical composition, which comprises the compound of Formula 1, or the isomer thereof, the solvate thereof, the hydrate thereof, or the pharmaceutically acceptable salt thereof as an active ingredient, to a subject in need thereof.
According to yet another aspect of the present invention, there is provided the compound represented by Formula 1, or the stereoisomer thereof, the solvate thereof, the hydrate thereof, or the pharmaceutically acceptable salt for use in preventing or treating a protein kinase-related disease.
According to yet another aspect of the present invention, there is provided a use of the compound represented by Formula 1, or the stereoisomer thereof, the solvate thereof, the hydrate thereof, or the pharmaceutically acceptable salt thereof for use in preparing medicaments for the prevention or treatment of the protein kinase-related disease.

[Advantageous Effects]

A pyrrolopyrimidine derivative according to the present invention has excellent inhibitory activity against various protein kinases including LRRK2, and has an excellent effect of inhibiting proliferation of triple-negative breast cancer cells. Therefore, the pharmaceutical composition comprising the pyrrolopyrimidine derivative as an active ingredient can be useful for the treatment or prevention of protein kinase-related diseases, in particular, cancers, degenerative brain diseases, and inflammatory diseases, and specifically can be useful for the treatment of triple-negative breast cancer.

[Description of Drawings]

FIGS. 1 to 3 are images showing the results of an experiment for inhibiting LRRK2 phosphorylation of compounds according to the present invention.

[Best Mode]

Hereinafter, the present invention will be described in detail.
In this specification, the term "halogen" may be F, Cl, Br, or I.
In this specification, the term "haloalkyl" may refer to a linear or branched alkyl (a hydrocarbon) having carbon atoms substituted with one or more halogen atoms as defined herein. Examples of the haloalkyl comprise methyl, ethyl, propyl, isopropyl, isobutyl, and *N*-butyl, which are independently substituted with one or more halogen atoms, for example F, Cl, Br, and I, but the present invention is not limited thereto.
In this specification, the term "alkyl" may refer to a linear or branched acyclic saturated hydrocarbon consisting of carbon atoms. A representative -($C_{1-8}$ alkyl) comprises -methyl, -ethyl, -*N*-propyl, -*N*-butyl, -*N*-pentyl, -*N*-hexyl, -*N*-heptyl, and -*N*-octyl; a branched chain saturated alkyl may comprise -isopropyl, -secondary (sec)-butyl, -isobutyl, -tertiary (tert)-butyl, -isopentyl, 2-methylpentyl, 3-methylpentyl, 4-methylpentyl, 2,3-dimethylbutyl, and the like. The -($C_{1-8}$ alkyl) may also be substituted or unsubstituted. For example, a $C_{1-8}$ alkyl group may be substituted with phenyl to form a benzyl group.
In this specification, the term "cycloalkyl" may refer to a non-aromatic, saturated or unsaturated carbocycle. A representative cycloalkyl comprises cyclopropyl, cyclobutyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl, and cyclooctadienyl, but the present invention is not limited thereto. The cycloalkyl group may be substituted or unsubstituted. According to one embodiment, this cycloalkyl group may be a $C_{3-8}$ cycloalkyl group.

In this specification, the term "heterocycloalkyl" may refer to a saturated or partially unsubstituted cyclic substituent having a total number of 3 to 10 ring atoms and containing 1 to 5 heteroatoms selected from N, O, and S. Unless otherwise stated, a heterocycloalkyl group can be in the form of a monocyclic, bicyclic, spirocyclic, or polycyclic ring. Also, the heterocycloalkyl may comprise a ring bridged with one or more elements. The heterocycloalkyl may be attached to the remainder of the molecule through one or more ring carbons or heteroatoms. Examples of the heterocycloalkyl comprise pyrrolidine, piperidine, *N*-methylpiperidine, imidazolidine, pyrazolidine, butyrolactam, valerolactam, imidazolidinone, hydantoin, dioxolane, phthalimide, piperidine, pyrimidine-2,4(1H,3H)-dione, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, quinuclidine, tropan, 2-azaspiro[3.3]heptane, (1R,5S)-3-azabicyclo[3.2.1]octane, (1s,4s)-2-azabicyclo[2.2.2]octane, (1R,4R)-2-oxa-5-azabicyclo[2.2.2]octane, and the like, but the present invention is not limited thereto.

In this specification, the term "aryl" may refer to any functional group or substituent derived by removing one hydrogen atom from an aromatic hydrocarbon ring. The aryl group may be a monocyclic aryl group or a polycyclic aryl group. The aryl group may have 5 or more and 30 or less, 5 or more and 20 or less, or 5 or more and 15 or less ring-forming carbon atoms. Examples of the aryl group may comprise a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylene group, a pyrenyl group, a benzofluoranthenyl group, a chrycenyl group, and the like, but the present invention is not limited thereto.

In this specification, the term "heteroaryl" may refer to an aryl cyclic group comprising one or more selected from O, N, P, Si, and S as a heteroatom. The heteroaryl group may have 2 or more and 30 or less, or 2 or more and 20 or less ring-forming carbon atoms. The heteroaryl may be a monocyclic heteroaryl or a polycyclic heteroaryl. The polycyclic heteroaryl may, for example, have a bicyclic or tricyclic structure. Examples of the heteroaryl may comprise thienyl, thiophene, furyl, pyrrolyl, pyrazolyl, imidazolyl, thiazolyl, oxazolyl, isothiazolyl, oxadiazolyl, triazolyl, pyridinyl, bipyridyl, pyrimidyl, triazinyl, triazolyl, an acrydyl group, a pyridazinyl group, pyrazinyl, quinolinyl, quinazoline, quinoxalinyl, phenoxazyl, phthalazinyl, pyrimidinyl, pyrido-pyrimidinyl, pyrido-pyrazinyl, pyrazino-pyrazinyl, isoquinoline, indole, carbazole, imidazopyridazinyl, imidazopyridinyl, imidazopyrimidinyl, pyrazolopyrimidinyl, imidazopyrazinyl or pyrazolopyridinyl, N-arylcarbazole, *N*-heteroarylcarbazole, an *N*-alkylcarbazole group, benzoxazole, benzoimidazole, benzothiazole, benzocarbazole, benzothiophene, dibenzothiophenyl, thienothiophene, benzofuranyl, phenanthroline, isooxazolyl, oxadiazolyl, thiadiazolyl, benzothiazolyl, tetrazolyl, phenothiazinyl, dibenzosilole, dibenzofuranyl, and the like, but the present invention is not limited thereto. According to one embodiment of the present invention, the heteroaryl may also comprise a bicyclic heterocycloaryl comprising an aryl ring fused to a heterocycloalkyl ring or a heteroaryl fused to a cycloalkyl ring.

The present invention provides a compound represented by the following Formula 1, or an isomer thereof, a solvate thereof, a hydrate thereof, or a pharmaceutically acceptable salt thereof.

[Formula 1]

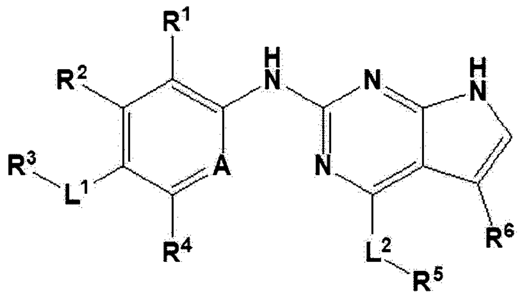

(wherein,

A represents a carbon atom or a nitrogen atom,
$R^1$ is hydrogen or a linear or branched $C_{1-6}$ alkoxy, and $R^2$ is hydrogen, or
$R^1$ and $R^2$, together with a benzene ring containing carbon atoms to which they are bonded, form a 8- to 10-membered bicyclic ring comprising one or more heteroatoms selected from the group consisting of N, O, and S,

$L^1$ is sulfonyl or carbonyl, or is absent,

when $L^1$ is sulfonyl or carbonyl, $R^3$ is a linear or branched $C_{1-6}$ alkyl, morpholinyl, piperidinyl, piperazinyl, pyrrolidinyl, amino, 2-oxa-5-azabicyclo[2.2.1]heptanyl, or azetidinyl, wherein $R^3$ is unsubstituted or substituted with one or more non-hydrogen substituents selected from the group consisting of morpholinyl, oxetanyl, 2-oxa-5-azabicyclo[2.2.1]heptanyl, piperazinyl, piperidinyl, a $C_{3-9}$ cycloalkyl, and a linear or branched $C_{1-6}$ alkyl, wherein the non-hydrogen substituents of $R^3$ are unsubstituted or substituted with a substituent selected from the group consisting of hydroxy, a $C_{3-9}$ cycloalkyl, and a linear or branched $C_{1-6}$ alkyl,
when $L^1$ is absent, $R^3$ is azaphosphinane oxide or phosphoryl, wherein $R^3$ is unsubstituted or substituted with one or more non-hydrogen substituents selected from the group consisting of oxetanyl, a $C_{3-9}$ cycloalkyl, a linear or branched $C_{1-6}$ alkyl, vinyl, tetrahydropyranyl, and benzyl, wherein the non-hydrogen substituents of $R^3$ are unsubstituted or substituted with a $C_{1-6}$ alkoxy,

$R^4$ is hydrogen or a halogen,
$L^2$ is -NH- or -O-, or is absent,

when $L^2$ is -NH- or -O-, $R^5$ is selected from the group consisting of a linear or branched $C_{1-6}$ alkyl, a $C_{3-9}$ cycloalkyl, a 3- to 9-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-9}$ cycloalkyl, a $C_{1-6}$ alkylsulfonyl, a $C_{1-6}$ alkoxy, and a $C_{1-6}$ alkyl,
when $L^2$ is absent, $R^5$ is a linear or branched $C_{1-6}$ alkyl or a $C_{3-9}$ cycloalkyl, and

$R^6$ is hydrogen, cyano, or a $C_{1-6}$ haloalkyl).

According to another embodiment of the present invention, in the compound represented by Formula 1,

A represents a carbon atom or a nitrogen atom,
$R^1$ is hydrogen or a linear or branched $C_{1-3}$ alkoxy, and $R^2$ is hydrogen,
$L^1$ is sulfonyl or carbonyl, or is absent,

when $L^1$ is sulfonyl or carbonyl, $R^3$ is a linear or branched $C_{1-3}$ alkyl, morpholinyl, piperidinyl, piperazinyl, pyrrolidinyl, amino, 2-oxa-5-azabicyclo[2.2.1]heptanyl, or azetidinyl, wherein $R^3$ is unsubstituted or substituted with one or more non-hydrogen substituents selected from the group consisting of morpholinyl, oxetanyl, 2-oxa-5-azabicyclo[2.2.1]heptanyl, piperazinyl, piperidinyl, a $C_{3-6}$ cycloalkyl, and a linear or branched $C_{1-3}$ alkyl, wherein the non-hydrogen substituents of $R^3$ are unsubstituted or substituted with a substituent selected from the group consisting of hydroxy, a $C_{3-6}$ cycloalkyl, and a linear or branched $C_{1-3}$ alkyl,
when $L^1$ is absent, $R^3$ is azaphosphinane oxide or phosphoryl, wherein $R^3$ is unsubstituted or substituted with one or more non-hydrogen substituents selected from the group consisting of oxetanyl, a $C_{3-6}$ cycloalkyl, a linear or branched $C_{1-3}$ alkyl, vinyl, tetrahydropyranyl, and benzyl, wherein the non-hydrogen substituents of $R^3$ are unsubstituted or substituted with a $C_{1-3}$ alkoxy,

$R^4$ is hydrogen, F, Cl, or Br,
$L^2$ is -NH- or -O-, or is absent,

when $L^2$ is -NH- or -O-, $R^5$ is selected from the group consisting of a linear or branched $C_{1-5}$ alkyl, a $C_{3-8}$ cycloalkyl, a 3- to 6-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-6}$ cycloalkyl, a $C_{1-3}$ alkylsulfonyl, a $C_{1-3}$ alkoxy, and a $C_{1-3}$ alkyl,
when $L^2$ is absent, $R^5$ is a linear or branched $C_{1-3}$ alkyl or a $C_{3-6}$ cycloalkyl, and

$R^6$ may be hydrogen, cyano, or trifluoromethyl.

According to still another embodiment of the present invention, in the compound represented by Formula 1,

A represents a carbon atom,
$R^1$ and $R^2$, together with a benzene ring containing carbon atoms to which they are bonded, form a 9- to 10-membered bicyclic ring comprising one or more O (oxygen) atoms as heteroatoms,
$L^1$ is sulfonyl or carbonyl,

$R^3$ is a linear or branched $C_{1-3}$ alkyl, or is morpholinyl, piperidinyl, piperazinyl, pyrrolidinyl, amino, 2-oxa-5-azabicyclo[2.2.1]heptanyl, or azetidinyl, wherein $R^3$ is unsubstituted or substituted with one or more non-hydrogen substituents selected from the group consisting of morpholinyl, oxetanyl, 2-oxa-5-azabicyclo[2.2.1]heptanyl, piperazinyl, piperidinyl, a $C_{3-6}$ cycloalkyl, and a linear or branched $C_{1-3}$ alkyl, wherein the non-hydrogen substituents of $R^3$ are unsubstituted or substituted with a substituent selected from the group consisting of hydroxy, a $C_{3-6}$ cycloalkyl, and a linear or branched $C_{1-3}$ alkyl,
$R^4$ is hydrogen, F, Cl, or Br,
$L^2$ is -NH- or -O-, or is absent,

when $L^2$ is -NH- or -O-, $R^5$ is selected from the group consisting of a linear or branched $C_{1-5}$ alkyl, a $C_{3-8}$ cycloalkyl, a 3- to 6-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-6}$ cycloalkyl, a $C_{1-3}$ alkylsulfonyl, a $C_{1-3}$ alkoxy, and a $C_{1-3}$ alkyl,
when $L^2$ is absent, $R^5$ is a linear or branched $C_{1-3}$ alkyl or a $C_{3-6}$ cycloalkyl, and $R^6$ may be cyano or trifluoromethyl.

According to yet another embodiment of the present invention,
$R^1$ and $R^2$, together with a benzene ring containing carbon atoms to which they are bonded, may form a 9- to 10-membered bicyclic ring comprising one or more O (oxygen) atoms as heteroatoms. Specifically, the 9- to 10-membered bicyclic ring may be dihydrobenzodioxin, dihydrobenzofuran, or benzodioxole, and more specifically may be 2,3-dihydrobenzo[b][1,4]dioxin, benzo[d][1,3]dioxole, or 2,3-dihydrobenzofuran.
According to yet another embodiment of the present invention, in the compound represented by Formula 1,

A represents a carbon atom,
$R^1$ and $R^2$, together with a benzene ring containing carbon atoms to which they are bonded, form a 9- to 10-membered bicyclic ring comprising one or more O (oxygen) atoms as heteroatoms, wherein the 9- to 10-membered bicyclic ring is dihydrobenzodioxin or dihydrobenzofuran,
$L^1$ is sulfonyl,
$R^3$ is piperidinyl, wherein the piperidinyl is unsubstituted or substituted with morpholinyl,
$R^4$ is hydrogen,
$L^2$ is -NH-, or is absent,
$R^5$ is a linear or branched $C_{1-5}$ alkyl or a $C_{3-8}$ cycloalkyl, and
$R^6$ may be trifluoromethyl.

According to yet another embodiment of the present invention, in the compound represented by Formula 1,

A represents a carbon atom,
$R^1$ is a linear or branched $C_{1-3}$ alkoxy, and $R^2$ is hydrogen,
$L^1$ is sulfonyl,
$R^3$ is a linear or branched $C_{1-3}$ alkyl, morpholinyl, or piperidinyl, wherein $R^3$ is unsubstituted or substituted with morpholinyl or 2-oxa-5-azabicyclo[2.2.1]heptanyl,
$R^4$ is hydrogen,
$L^2$ is -NH- or -O-, or is absent,

when $L^2$ is -NH-, $R^5$ is selected from the group consisting of a linear or branched $C_{1-5}$ alkyl, a $C_{3-8}$ cycloalkyl, a 3- to 6-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-6}$ cycloalkyl, a $C_{1-3}$ alkylsulfonyl, a $C_{1-3}$ alkoxy, and a $C_{1-3}$ alkyl,
when $L^2$ is -O-, $R^5$ is substituted with a linear or branched $C_{1-5}$ alkyl,
when $L^2$ is absent, $R^5$ is a linear or branched $C_{1-3}$ alkyl or a $C_{3-6}$ cycloalkyl, and

$R^6$ may be hydrogen, cyano, or trifluoromethyl.

According to yet another embodiment of the present invention, in the compound represented by Formula 1,

A represents a carbon atom,
$R^1$ is hydrogen or a linear or branched $C_{1-3}$ alkoxy, and $R^2$ is hydrogen,
$L^1$ is absent,
$R^3$ is selected from azaphosphinane oxide and phosphoryl, wherein $R^3$ is unsubstituted or substituted with one or

more non-hydrogen substituents selected from the group consisting of oxetanyl, a $C_{3-6}$ cycloalkyl, a linear or branched $C_{1-3}$ alkyl, vinyl, tetrahydropyranyl, and benzyl, wherein the non-hydrogen substituents of $R^3$ are unsubstituted or substituted with a $C_{1-3}$ alkoxy,
$R^4$ is hydrogen,
$L^2$ is -NH-, or is absent,

when $L^2$ is -NH-, $R^5$ is selected from the group consisting of a linear or branched $C_{1-5}$ alkyl, a $C_{3-8}$ cycloalkyl, a 3- to 6-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-6}$ cycloalkyl, a $C_{1-3}$ alkylsulfonyl, a $C_{1-3}$ alkoxy, and a $C_{1-3}$ alkyl,
when $L^2$ is absent, $R^5$ is a linear or branched $C_{1-3}$ alkyl or a $C_{3-6}$ cycloalkyl, and

$R^6$ may be hydrogen, cyano, or trifluoromethyl.

According to yet another embodiment of the present invention,
$L^1$-$R^3$ may be

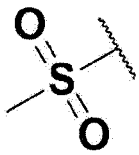 , 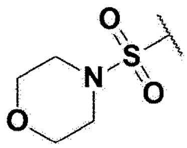 , 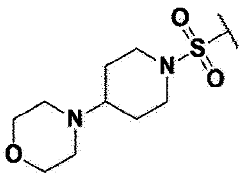 , 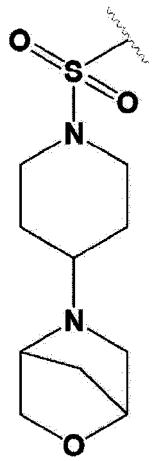 ,

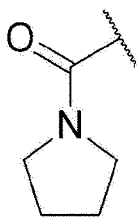 , 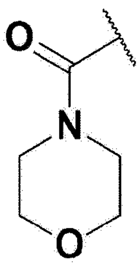 , 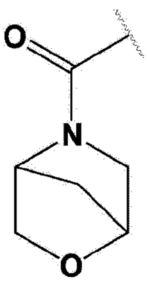 , 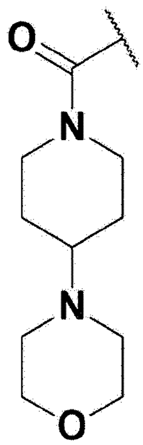 , 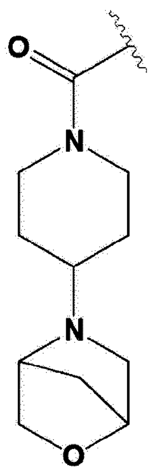 , 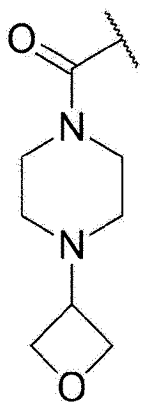 , 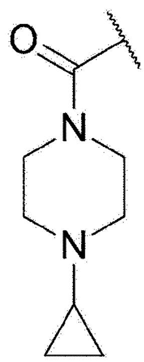 ,

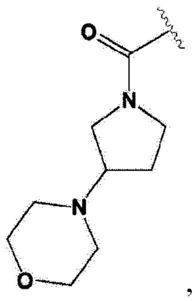, 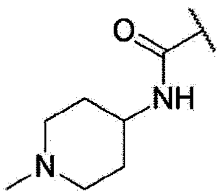, 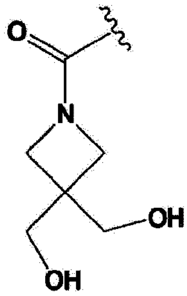, 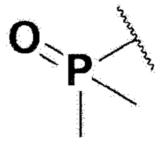, 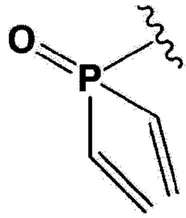, 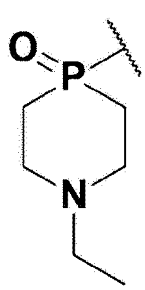,

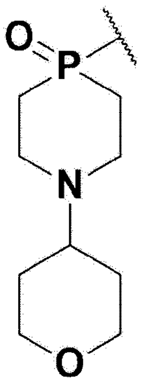, 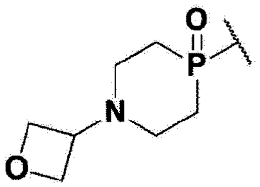, 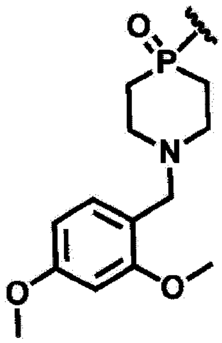, or 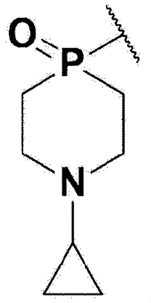.

According to yet another embodiment of the present invention, in the compound represented by Formula 1, $R^5$ may be methyl, ethyl, propyl, isopropyl, butyl, isobutyl, sec-butyl, cyclopropyl, cyclobutyl, cyclopentyl, cyclohexyl, cycloheptyl, tetrahydrofuranyl, tetrahydropyranyl, methoxyethyl, ethoxyethyl, $CH_3OCH_2CH(CH_3)$-, $CH_3OCH_2C(CH_3)_2$-, cyclopropylmethyl, dimethylaminoethyl, methylsulfonylethyl, cyclobutylmethyl, or cyclopentylmethyl. Specifically, $R^5$ may be methyl, ethyl, propyl, isopropyl, butyl, isobutyl, sec-butyl, cyclopropyl, cyclobutyl, cyclopentyl, cyclohexyl, cycloheptyl, tetrahydrofuranyl, tetrahydropyranyl, methoxyethyl, cyclopropylmethyl, methylsulfonylethyl, cyclobutylmethyl, or cyclopentylmethyl.

The compound represented by Formula 1 of the present invention may be used in the form of a pharmaceutically acceptable salt, and an acid addition salt formed by a pharmaceutically acceptable free acid is usefully used as the salt. The acid addition salt is obtained from inorganic acids such as hydrochloric acid, nitric acid, phosphoric acid, sulfuric acid, hydrobromic acid, hydroiodic acid, nitrous acid, phosphorous acid, and the like; non-toxic organic acids such as aliphatic mono- and di-carboxylate, phenyl-substituted alkanoate, hydroxy alkanoate and alkanedioate, aromatic acids, aliphatic and aromatic sulfonic acids, and the like; organic acids such as trifluoroacetic acid, acetate, benzoic acid, citric acid, lactic acid, maleic acid, gluconic acid, methanesulfonic acid, 4-toluenesulfonic acid, tartaric acid, fumaric acid, and the like. Types of such a pharmaceutically non-toxic salt comprise sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutylate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, β-hydroxybutylate, glycolate, malate, tartrate, methanesulfonate, propanesulfonate, naphthalene-1-sulfonate, naphthalene-2-sulfonate, mandelate, and the like.

The acid addition salt according to the present invention may be prepared using conventional methods. For example, the acid addition salt of the present invention may be prepared by dissolving the derivative of Formula 1 in an organic solvent such as methanol, ethanol, acetone, methylene chloride, acetonitrile, or the like, adding an organic acid or an inorganic acid thereto to form a precipitate, and filtering and drying the precipitate, or by distilling a solvent and an excessive amount of an acid under reduced pressure, drying the distillate, and then crystallizing the distillate in an organic solvent.

Also, a pharmaceutically acceptable metal salt may be prepared using a base. An alkali metal or an alkaline earth metal salt is, for example, obtained by dissolving a compound in an excessive amount of an alkali metal hydroxide or alkaline earth metal hydroxide solution, filtering the non-dissolved compound salt, and evaporating and drying the filtrate. In this case, the metal salt is pharmaceutically suitable for preparing a sodium, potassium or calcium salt. Also, a salt corresponding to the metal salt is obtained by reacting an alkali metal or alkaline earth metal salt with a suitable

silver salt (e.g., silver nitrate).

Examples of the compound represented by Formula 1 according to the present invention or the pharmaceutically acceptable salt thereof may comprise compounds of Examples 1 to 477 or pharmaceutically acceptable salts thereof as listed in [Table 1] among the following Examples.

Also, the present invention comprises all types of the compound represented by Formula 1 or the pharmaceutically acceptable salts thereof, as well as solvates, optical isomers, hydrates, and the like, which may be prepared therefrom.

The term "hydrate" refers to a compound of the present invention or a salt thereof that comprises a stoichiometric or non-stoichiometric amount of water bound via a non-covalent intermolecular force. The hydrate of the compound represented by Formula 1 of the present invention may comprise a stoichiometric or non-stoichiometric amount of water bound via the non-covalent intermolecular force. The hydrate may contain 1 or more equivalents, preferably 1 equivalent to 5 equivalents of water. Such a hydrate may be prepared by crystallizing the compound represented by Formula 1 of the present invention, or an isomer thereof or a pharmaceutically acceptable salt thereof from water or a water-containing solvent.

The term "solvate" refers to a compound of the present invention or a salt thereof that comprises a stoichiometric or non-stoichiometric amount of a solvent bound via a non-covalent intermolecular force. Preferred solvents for the solvate may comprise volatile solvents, non-toxic solvents, and/or suitable solvents to be administered to humans.

The term "isomer" refers to a compound of the present invention or a salt thereof that has the same chemical or molecular formula but differs in a structural or three-dimensional aspect. Such an isomer comprises all types of constitutional isomers such as tautomers, and the like; R or S isomers having an asymmetric carbon center; stereoisomers such as geometric isomers (trans- or cis-), and the like; optical isomers (enantiomers), and the like. Also, all the types of these isomers and mixtures thereof fall within the scope of the present invention.

As shown in the following Scheme A, still another aspect of the present invention provides a method of preparing the compound represented by Formula 1, which comprises:

reacting a compound represented by Formula 4 with a compound represented by Formula 3 to prepare a compound represented by Formula 2 (Step 1); and
reacting the compound represented by Formula 2 to prepare the compound represented by Formula 1 (Step 2).

[Scheme A]

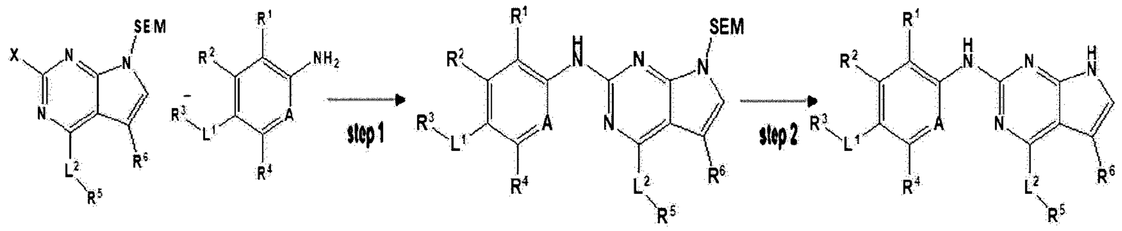


(wherein,

$R^1$, $R^2$, $R^3$, $R^4$, $R^5$, $R^6$, $L^1$, and $L^2$ are as defined in Formula 1;
SEM is a protective group; and
X is a halogen).

In the method of preparing the compound of Formula 1 as shown in Scheme A,

Step 1 is a step of reacting a primary amine of the compound represented by Formula 4 with a halogen of the compound represented by Formula 3 to prepare the compound represented by Formula 2 in which an amine bond is formed. In this case, conditions for reacting an amine with a halogen to form an amine bond are not limited, and methods widely known in the art may be used. In the present invention, a reaction may be performed as in the same manner as in Example 1, but this is merely one example and the present invention is not limited thereto.

Step 2 is a step of deprotecting an amine protective group of the compound represented by Formula 2 to prepare the compound represented by Formula 1. In this case, conditions for removing the amine protective group are not limited, and methods widely known in the art may be used. In the present invention, a reaction may be performed as in the same manner as in Example 1, but this is merely one example and the present invention is not limited

thereto. Examples of the protective group may comprise a 2-(trimethylsilyl)ethoxymethyl group, a trimethylsilyl (TMS) group, a benzyl group, an acetyl group, or the like.

Yet another aspect of the present invention provides a pharmaceutical composition for use in preventing or treating a protein kinase-related disease, which comprises the compound represented by Formula 1, or the isomer thereof, the solvate thereof, the hydrate thereof, or the pharmaceutically acceptable salt thereof as an active ingredient.

The present invention also provides a method of preventing or treating a protein kinase-related disease, which comprises: administering a pharmaceutical composition comprising the compound of Formula 1 or the pharmaceutically acceptable salt thereof as an active ingredient to a subject in need thereof.

The protein kinase may comprise one or more selected from the group consisting of CLK1, CLK2, CLK3, CLK4, CSNK1D, CSNK1E, DYRK1A, DYRK1B, DYRK2, FAK, GAK, LRRK2, LRRK2 (G2019S), PHKG1, PHKG2, PLK4, PYK2, and TTK. Specifically, the protein kinase may comprise one or more selected from the group consisting of LRRK2, LRRK2 (G2019S), DYRK1, CLK1, and TTK.

The protein kinase-related disease may comprise one or more selected from the group consisting of a cancer, a degenerative brain disease, and an inflammatory disease.

The degenerative brain disease may comprise one or more selected from the group consisting of Alzheimer's disease, Parkinson's disease, Lou Gehrig's disease, dementia, Huntington's disease, multiple sclerosis, proximal lateral sclerosis, apoplexy, stroke, and mild cognitive impairment.

The inflammatory disease may comprise one or more selected from the group consisting of dermatitis, allergies, gastric ulcers, duodenal ulcers, hepatitis, esophagitis, gastritis, enteritis, pancreatitis, colitis, nephritis, systemic edema, local edema, arthritis, keratitis, bronchitis, pleurisy, peritonitis, spondylitis, inflammatory pain, urethritis, cystitis, periodontitis, and gingivitis.

The cancer may comprise one or more selected from the group consisting of triple-negative breast cancer, brain cancer, brain tumors, benign astrocytoma, malignant astrocytoma, pituitary adenoma, meningioma, brain lymphoma, oligodendroglioma, intracranial carcinoma, ependymoma, brainstem tumors, head and neck tumors, larynx cancer, oropharyngeal cancer, nasal cavity/paranasal sinus cancer, nasopharyngeal cancer, salivary gland cancer, hypopharyngeal cancer, thyroid cancer, oral cancer, thoracic tumors, small cell lung cancer, non-small cell lung cancer, thymus cancer, mediastinal tumors, esophageal cancer, breast cancer, male breast cancer, abdominal tumors, stomach cancer, liver cancer, gallbladder cancer, biliary cancer, pancreatic cancer, small bowel cancer, colon cancer, rectal cancer, anal cancer, bladder cancer, kidney cancer, male genital tumors, penile cancer, prostate cancer, female genital tumors, cervical cancer, endometrial cancer, ovarian cancer, uterine sarcoma, vaginal cancer, female external genital cell cancer, female urethral cancer, and skin cancer.

The compound represented by Formula 1 of the present invention has an effect of inhibiting one or more protein kinases selected from the group consisting of CLK1, CLK2, CLK3, CLK4, CSNK1D, CSNK1E, DYRK1A, DYRK1B, DYRK2, FAK, GAK, LRRK2, LRRK2 (G2019S), PHKG1, PHKG2, PLK4, PYK2, and TTK, specifically one or more protein kinases selected from the group consisting of LRRK2, LRRK2 (G2019S), DYRK1, CLK1, and TTK. Therefore, the compound represented by Formula 1 of the present invention may be usefully used in the pharmaceutical composition for preventing or treating a protein kinase-related disease.

Also, the compound represented by Formula 1 of the present invention inhibits the proliferation of triple-negative breast cancer cells. Therefore, the compound represented by Formula 1 of the present invention may be usefully used to treat triple-negative breast cancer.

In addition, because the compound represented by Formula 1 of the present invention effectively inhibits LRRK2 phosphorylation in cancer-inducing cells, the compound represented by Formula 1 of the present invention may be usefully used in a pharmaceutical composition for preventing or treating an LRRK2-related disease.

The pharmaceutical composition may comprise a therapeutically effective amount of the compound of the present invention. Also, the pharmaceutical composition may further comprise a pharmaceutically acceptable carrier.

The compound represented by Formula 1 or the pharmaceutically acceptable salt thereof may be administered in various oral and parenteral formulations upon clinical administration. When a composition is formulated, the composition may be prepared using a commonly used diluent or excipient such as a filler, an extending agent, a binding agent, a wetting agent, a disintegrating agent, a surfactant, and the like. A solid preparation for oral administration comprises a tablet, a pill, a powder, granules, a capsule, and the like. Such a solid preparation is prepared by mixing at least one or more excipients, for example, starch, calcium carbonate, sucrose or lactose, gelatin, and the like with one or more compounds. Also, in addition to the simple excipient, lubricants such as magnesium stearate, talc, and the like are used. A liquid preparation for oral administration comprises a suspension, a liquid for internal use, an emulsion, a syrup, and the like. In this case, the liquid preparation for oral administration can comprise various excipients such as a wetting agent, a sweetening agent, an aromatic, a preservative, and the like in addition to generally used simple diluents such as water and liquid paraffin. A preparation for parenteral administration comprises a sterile aqueous solution, a non-aqueous solvent, a suspension, and an emulsion. Propylene glycol, polyethylene glycol, a vegetable oil (such as olive

oil), an injectable ester (such as ethyl oleate), and the like may be used as the non-aqueous solvent and the suspension.

A pharmaceutical composition comprising the compound represented by Formula 1 or the pharmaceutically acceptable salt thereof as an active ingredient may be parenterally administered, and the parenteral administration is performed by subcutaneous injection, intravenous injection, intramuscular injection, or intrathoracic injection.

In this case, for preparation into a formulation for parenteral administration, the compound represented by formula 1 or the pharmaceutically acceptable salt thereof is mixed with a stabilizing agent or a buffering agent in water to prepare a solution or suspension, which is then prepared into unit dosage forms of ampoules or vials. The composition may be sterilized and/or additionally contains adjuvants such as a preservative, a stabilizing agent, a wetting or emulsifying agent, a salt and/or buffer for the regulation of osmotic pressure, and other therapeutically useful materials. In this case, the composition may be formulated using conventional methods such as mixing, granulating, or coating.

For example, the formulations for oral administration comprise a tablet, a pill, a hard/soft capsule, a liquid, a suspending agent, an emulsifying agent, a syrup, a granule, an elixir, a troche, and the like. In this case, these formulations contain diluents (e.g., lactose, dextrose, sucrose, mannitol, sorbitol, cellulose, and/or glycine), and lubricants (e.g., silica, talc, stearic acid and a magnesium or calcium salt thereof, and/or polyethylene glycol) in addition to the active ingredient. The tablet may contain a binding agent such as magnesium aluminum silicate, starch paste, gelatin, methyl cellulose, sodium carboxymethyl cellulose, and/or polyvinyl pyrrolidone, and may contain a disintegrating agent (starch, agar, alginic acid or a sodium salt thereof, or the like) or a boiling mixture and/or an absorbing agent, a coloring agent, a flavoring agent, and a sweetening agent, when necessary.

According to yet another aspect of the present invention, there is provided the compound represented by Formula 1, or the stereoisomer thereof or the pharmaceutically acceptable salt thereof for use in preventing or treating a protein kinase-related disease.

According to yet another aspect of the present invention, there is provided a use of the compound represented by Formula 1, or the stereoisomer thereof or the pharmaceutically acceptable salt thereof for use in preparing medicaments for the prevention or treatment of the protein kinase-related disease.

The protein kinases and diseases associated with the protein kinases are as described above, and thus a specific description thereof will be omitted to avoid redundancy.

Hereinafter, the present invention will be described in detail with reference to Examples and Experimental Examples as will be described below.

However, it should be understood that the Examples and Experimental Examples described below are given for purpose of illustrating the present invention and not intended to limit the scope of the present invention.

**<Analysis and Purification Conditions >**

Compounds synthesized in Examples of the present invention were purified and structurally analyzed under the following HPLC conditions.

**1. Preparative HPLC Conditions**

Analysis Method 1: LC-MS L: 5-95AB_R_220&254

SHIMADZU LCMS-2020 was used as the equipment, and LabSolution Version 5.82 SP1 was used as the program. Chromolith@Flash RP-18E 25-2 MM was used as a column, and mobile phases A: 0.0375% TFA in water (v/v) and B: 0.01875% TFA in acetonitrile (v/v) were used.

Gradient: 0.0 min 5% B → 0.80 min 95% B →1.2 min 95% B →1.21 min 5% B → 1.5 min 5% B; Flow: 1.5 mL/min; Column Temp: 50°C; Detector: PDA (220 nm & 254 nm)

Analysis Method 2: LC-MS I: 5-95AB_R_220&254

SHIMADZU LCMS-2020 was used as the equipment, and LabSolution Version 5.72 was used as the program. Kinetex @ 5 um EVO C18 30*2.1 mm was used as a column, and mobile phases A: 0.0375% TFA in water (v/v) and B: 0.01875% TFA in acetonitrile (v/v) were used

Gradient: 0.0 min 5% B → 0.80 min 95% B →1.2 min 95% B →1.21 min 5% B → 1.5 min 5% B; Flow: 1.5 mL/min; Column Temp: 50°C; Detector: PDA (220 nm & 254 nm)

Analysis Method 3: LC-MS P: 5-95AB_R_220&254

SHIMADZU LCMS-2020 was used as the equipment, and LabSolution Version 5.89 was used as the program. Kinetex EVO C18 2.1X30 mm, 5 um was used as a column, and mobile phases A: 0.0375% TFA in water (v/v) and B:

0.01875% TFA in acetonitrile (v/v) were used.

Gradient: 0.0 min 5% B → 0.80 min 95% B →1.2 min 95% B → 1.21 min 5% B → 1.5 min 5% B; Flow: 1.5 mL/min; Column Temp: 50°C; Detector: PDA (220 nm & 254 nm)

Analysis Method 4: LC-MS B: 5-95AB_R_220&254.M

Agilent 1200/G6110A was used as the equipment, and Agilent Chemstation Rev. B. 04.03[54] was used as the program. Kinetex @ 5 um EVO C18 30*2.1 mm was used as a column, and mobile phases A: 0.0375% TFA in water (v/v) and B: 0.01875% TFA in acetonitrile (v/v) were used.

Gradient: 0.01 min 5% B → 0.80 min 95% B →1.2 min 95% B → 1.21 min 5% B → 1.5 min 5% B; Flow: 1.5 mL/min; Column Temp: 50°C; Detector: DAD (220 & 254 nm)

Analysis Method 5: LC-MS E: 5-95AB_R_220&254.M

Agilent 1100/G1956A was used as the equipment, and Agilent ChemStation Rev. B. 04.03[52] was used as the program. Kinetex @ 5 um EVO C18 30*2.1 mm was used as a column, and mobile phases A: 0.0375% TFA in water (v/v) and B: 0.01875% TFA in acetonitrile (v/v) were used.

Gradient: 0.0 min 5% B → 0.80 min 95% B → 1.2 min 95% B → 1.21 min 5% B → 1.5 min 5% B; Flow: 1.5 mL/min; Column Temp: 50°C; Detector: DAD (220 & 254 nm)

Analysis Method 6: LC-MS D: 5-95AB_R_220&254.M

Agilent 1200/G1956A was used as the equipment, and Agilent ChemStation Rev. B. 04.03[54] was used as the program. Kinetex EVO C18 30*2.1 mm, 5 um was used as a column, and mobile phases A: 0.0375% TFA in water (v/v) and B: 0.01875% TFA in acetonitrile (v/v) were used.

Gradient: 0.0 min 5% B → 0.80 min 95% B → 1.2 min 95% B → 1.21 min 5% B → 1.5 min 5% B; Flow: 1.5 mL/min; Column Temp: 50°C; Detector: DAD (220 & 254 nm)

Analysis Method 7: Preparative HPLC Conditions (ACQUITY UPLC H-Class Core System)

The equipment in which the UPLC system (ACQUITY UPLC PDA Detector) commercially available from Waters was equipped with a Mass QDA detector commercially available from Waters was used. The column used was ACQUITY UPLC® BEH C18 (1.7 μm, 2.1 X 50 mm) commercially available from Waters, and was run at a column temperature of 30°C.

The mobile phases A: water including 0.1% formic acid, and B: acetonitrile including 0.1% formic acid were used.

Gradient conditions (10% to 100% B for 3 minutes, Moving speed = 0.6 mL/min)

### 2. NMR Reading

NMR reading was performed using AVANCEIII 400 or AVANCEIII 400 HD commercially available from Bruker, and the data was represented in ppm (parts per million (δ)).

Commercially available reagents used herein were used without further purification. In the present invention, the room temperature refers to a temperature of approximately 20 to 25°C. The concentration under reduced pressure or solvent removal by distillation was performed using a rotary evaporator.

### <Preparation Example 1-1> Preparation of 2-chloro-*N*-methyl-7-((2-(trimethylsilyl)ethoxy)methyl)-7*H*-pyrrolo[2,3-*d*]pyrimidine-4-amine

The title compound was prepared in a manner as shown in the following Scheme 1.

[Scheme 1]

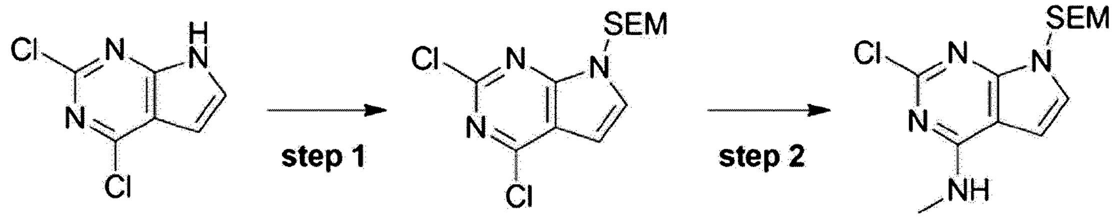


Step 1: 2,4-dichloro-7*H*-pyrrolo[2,3-*d*]pyrimidine (1.0 equivalent) was dissolved in DMF (0.62 M) under nitrogen, and NaH (1.2 equivalents) was then slowly added thereto at 0°C. The reaction mixture was reacted at 15°C for an hour, and (2-(chloromethoxy)ethyl)trimethylsilane (1.3 equivalents) was further added thereto at 0°C, and stirred at the same temperature for 1.5 hours. Distilled water was added to the resulting reaction product, and the organic matter was then extracted with MTBE (x 2). After the collected organic layer was washed with brine, the remaining water was removed using $Na_2SO_4$, and concentrated under reduced pressure. The concentrated mixture was purified by column chromatography ($SiO_2$, PE:EA), and the target compound was obtained as a yellow liquid (yield: 84%).

Step 2: 2,4-dichloro-7-((2-(trimethylsilyl)ethoxy)methyl)-7*H*-pyrrolo[2,3-*d*]pyrimidine (1.0 equivalent), DIPEA (2.9 equivalents), and methyl amine (1.5 equivalents) were dissolved in EtOH (0.32 M), and the reaction mixture was were stirred at 78°C for 16 hours. After the reaction was terminated, the organic solvent was removed by concentration under reduced pressure. An aqueous 1 N HCl solution (12.5 equivalents) was added to the resulting reaction product, and the organic matter was then extracted with EtOAc (x 3). The collected organic layer was washed with an aqueous saturated $NaHCO_3$ solution and brine, and the remaining water was then removed using $Na_2SO_4$. Then, the organic layer was concentrated under reduced pressure to obtain the target compound as a white solid (yield: 96%).

**<Preparation Example 1-2> Preparation of 2-chloro-*N*-ethyl-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 88%).

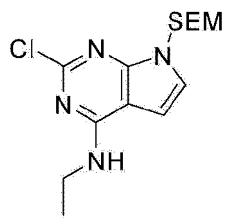

**<Preparation Example 1-3> Preparation of 2-chloro-*N*-propyl-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 86%).

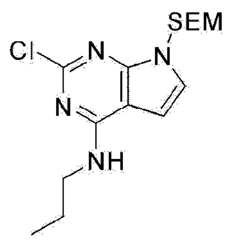

**<Preparation Example 1-4> Preparation of 2-chloro-*N*-isobutyl-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 99%).

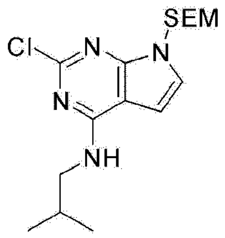

**<Preparation Example 1-5> Preparation of 2-chloro-*N*-isopropyl-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 98%).

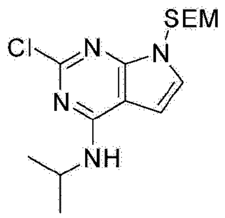

**<Preparation Example 1-6> Preparation of (R)-*N*-(sec-butyl)-2-chloro-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 90%).

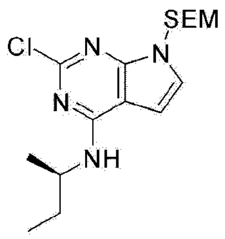

**<Preparation Example 1-7> Preparation of (*S*)-*N*-(*sec*-butyl)-2-chloro-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 67%).

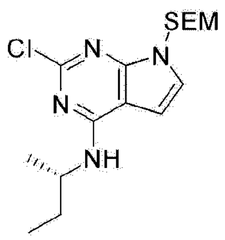

**<Preparation Example 1-8> Preparation of 2-chloro-*N*-(2-methoxyethyl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 88%).

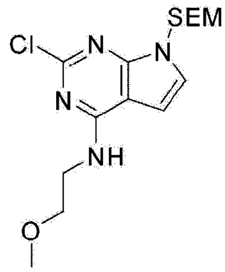

**<Preparation Example 1-9> Preparation of 2-chloro-*N*-cyclopropyl-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 97%).

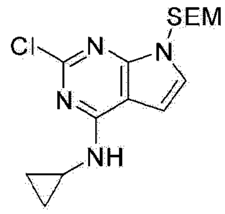

**<Preparation Example 1-10> Preparation of 2-chloro-*N*-cyclobutyl-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 96%).

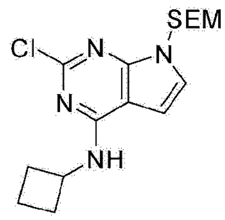

**<Preparation Example 1-11> Preparation of 2-chloro-*N*-cyclopentyl-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 91%).

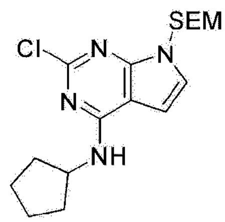

**<Preparation Example 1-12> Preparation of 2-chloro-*N*-cyclohexyl-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 95%).

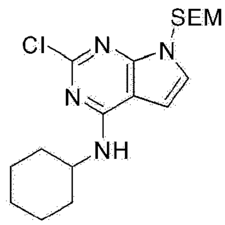

**<Preparation Example 1-13> Preparation of (*R*)-2-chloro-*N*-(tetrahydrofuran-3-yl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 78%).

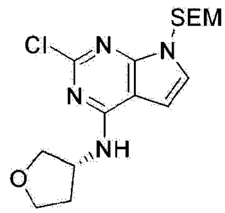

**<Preparation Example 1-14> Preparation of (*S*)-2-chloro-*N*-(tetrahydrofuran-3-yl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 98%).

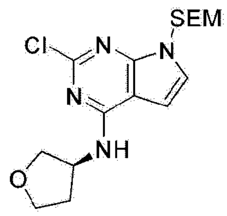

**<Preparation Example 1-15> Preparation of 2-chloro-*N*-(tetrahydro-2*H*-pyran-4-yl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 88%).

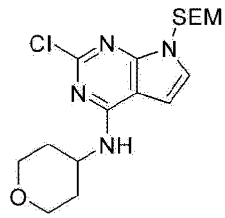

**<Preparation Example 1-16> Preparation of 2-chloro-*N*-(cyclopropylmethyl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 96%).

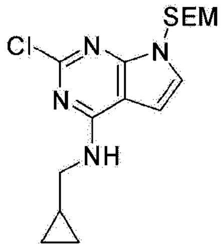

**<Preparation Example 1-17> Preparation of 2-chloro-*N*-(1-methoxy-2-methylpropan-2-yi)-7-((2-(trimethylsilyl)ethoxy)methyl)-7*H*-pyrrolo[2,3-*d*]pyrimidine-4-amine**

2,4-dichloro-7-((2-(trimethylsilyl)ethoxy)methyl)-7*H*-pyrrolo[2,3-*d*]pyrimidine (1.0 equivalent), DIPEA (2.5 equivalents), and amine (1.2 equivalents) were dissolved in NMP (0.25 M), and the reaction mixture was then stirred at 80°C for 12 hours. After the reaction was terminated, an aqueous 1 N HCl solution (12.5 equivalents) was added to the resulting reaction product, and the organic matter was then extracted with EtOAc (x 3). The collected organic layer was washed with an aqueous saturated $NaHCO_3$ solution and brine, the remaining water was then removed using $Na_2SO_4$. Then, the organic layer was concentrated under reduced pressure to obtain the target compound (yield: 48%).

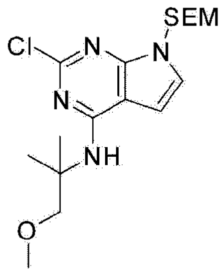

**<Preparation Example 1-18> Preparation of 2-chloro-*N*-(cyclobutylmethyl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 96%).

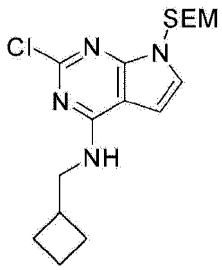

**<Preparation Example 1-19> Preparation of 2-chloro-*N*-(cyclopentylmethyl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 95%).

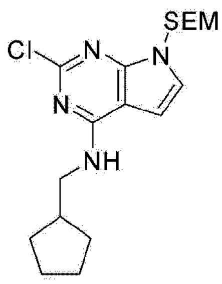

**<Preparation Example 1-20> Preparation of 2-chloro-*N*-(2-(methylsulfonyl)ethyl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 83%).

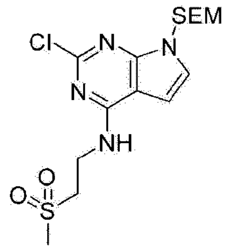

**<Preparation Example 1-21> Preparation of *N*-butyl-2-chloro-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 94%).

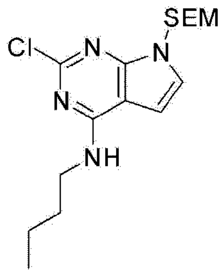

**<Preparation Example 1-22> Preparation of 2-chloro-*N*-(oxetan-3-yl)-7-((2-(trimethylsilyl)ethoxy)methyl)-7*H*-pyrrolo[2,3-*d*]pyrimidine-4-amine**

The title compound was prepared in a similar manner as in Preparation Example 1-1 (yield: 79%).

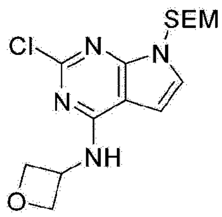

**<Preparation Example 2-1> Preparation of 2-chloro-4-(methylamino)-7-((2-(trimethylsilyl)ethoxy)methyl)-7*H*-pyrrolo[2,3-*d*]pyrimidine-5-carbonitrile**

The title compound was prepared in a manner as shown in the following Scheme 2.

[Scheme 2]

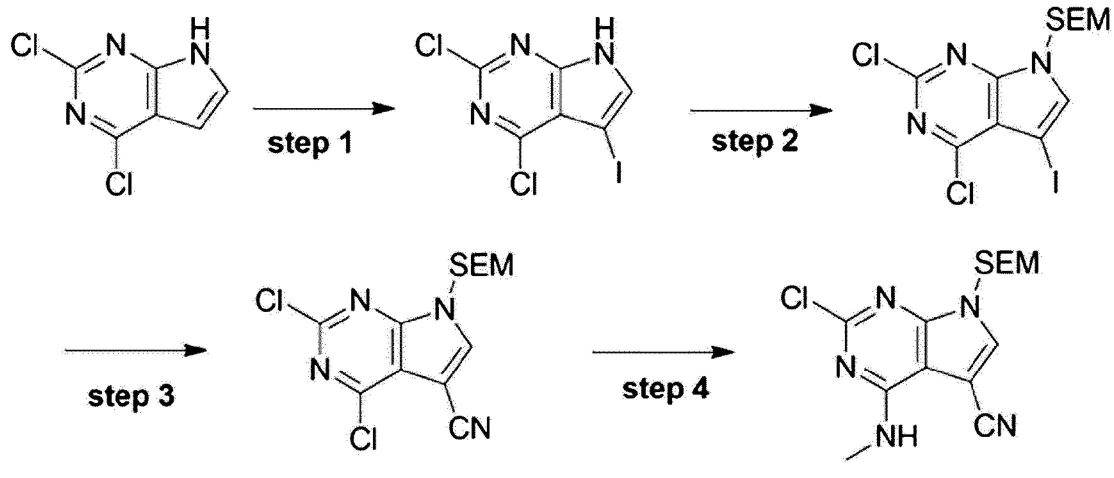


Step 1: 2,4-dichloro-7*H*-pyrrolo[2,3-*d*]pyrimidine (1.0 equivalent) was dissolved in DCM (0.5 M) under nitrogen, and NIS (1.6 equivalents) was then slowly added thereto at 0°C. The reaction mixture was stirred at room temperature for 12 hours. After the reaction was terminated, the formed solid target compound was filtered. The filtered target compound was washed with distilled water to obtain the target compound as a yellow solid.

Step 2: 2,4-dichloro-5-iodo-7*H*-pyrrolo[2,3-*d*]pyrimidine (1.0 equivalent) was dissolved in DMF (0.5 M) under nitrogen, and NaH (1.3 equivalents) was then slowly added thereto at 0°C. The reaction mixture was reacted at 0°C for 30 minutes, and (2-(chloromethoxy)ethyl)trimethylsilane (1.1 equivalents) was further added, and then stirred at 20 °C for an hour. Distilled water was added to the resulting reaction product, and the organic matter was then extracted with EA (x 3). After the collected organic layer was washed with brine, the remaining water was removed using $Na_2SO_4$, and the organic layer was concentrated under reduced pressure. The concentrated mixture was purified by column chromatography ($SiO_2$, PE:EA), and the target compound was obtained as a white solid (yield: 94%).

Step 3: 2,4-dichloro-5-iodo-7-((2-(trimethylsilyl)ethoxy)methyl)-7*H*-pyrrolo[2,3-*d*]pyrimidine (1.0 equivalent) was dissolved in NMP (0.2 M), and CuCN (2.0 equivalents) was then slowly added thereto at 0°C. The reaction mixture was stirred at 120°C for 6 hours. Cold distilled water and EA were added to the resulting reaction product, and the resulting mixture was then filtered through a Celite filter. The resulting filtrate was separated into an organic layer and an aqueous layer, and the aqueous layer was then extracted with EtOAc (x 2). After the collected organic layer was washed with brine, the remaining water was removed using $Na_2SO_4$, and the organic layer was concentrated under reduced pressure. The concentrated mixture was purified by column chromatography ($SiO_2$, PE:EA), and the target compound was obtained as a yellow solid (yield: 94%).

Step 4: 2,4-dichloro-7-((2-(trimethylsilyl)ethoxy)methyl)-7*H*-pyrrolo[2,3-*d*]pyrimidine-5-carbonitrile (1.0 equivalent), DIPEA (2.9 equivalents), and methyl amine (1.5 equivalents) were dissolved in EtOH (0.25 M), and the reaction mixture was then stirred at 80°C for 16 hours. After the reaction was terminated, the organic solvent was removed by concentration under reduced pressure. The resulting reaction product was dissolved in EtOAc, and then washed with an aqueous 1 N HCl solution and brine. Then, the remaining water was removed using $Na_2SO_4$, and the reaction product was concentrated under reduced pressure to obtain the target compound as a yellow solid (yield: 90%).

**<Preparation Example 2-2> Preparation of 2-chloro-4-(ethylamino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 73%).

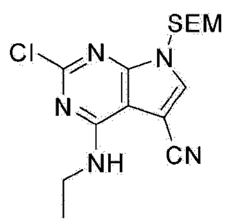

**<Preparation Example 2-3> Preparation of 2-chloro-4-(propylamino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 98%).

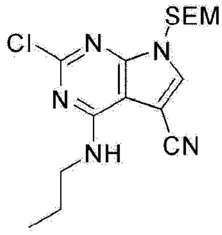

**<Preparation Example 2-4> Preparation of 2-chloro-4-(isobutylamino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 98%).

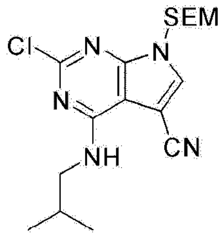

**<Preparation Example 2-5> Preparation of 2-chloro-4-(isopropylamino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 98%).

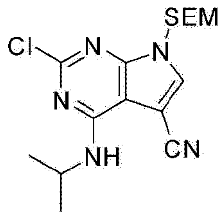

**<Preparation Example 2-6> Preparation of (*R*)-4-(*sec*-butyl)-2-chloro-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 84%).

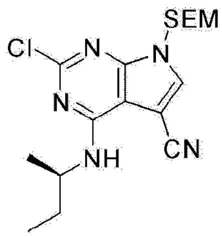

**<Preparation Example 2-7> Preparation of (*S*)-4-(*sec*-butyl)-2-chloro-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 85%).

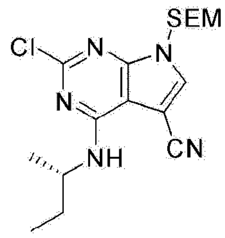

**<Preparation Example 2-8> Preparation of 2-chloro-4-((2-methoxyethyl)amino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 96%).

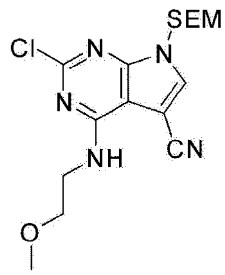

**<Preparation Example 2-9> Preparation of 2-chloro-4-(cyclopropylamino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 96%).

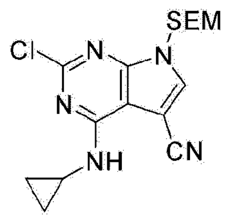

**<Preparation Example 2-10> Preparation of 2-chloro-4-(cyclobutylamino)-7-((2-(trimethylsilyl)ethoxy)methyl)-7*H* -pyrrolo[2,3-*d*]pyrimidine-5-carbonitrile**

The title compound was prepared in a similar manner as in Preparation Example 2-1 (yield: 87%).

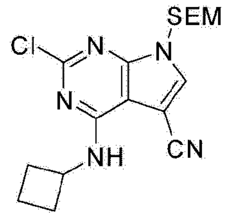

**<Preparation Example 2-11> Preparation of 2-chloro-4-(cyclopentylamino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 87%).

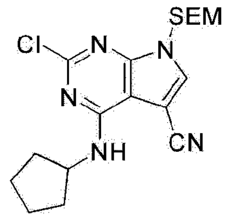

**<Preparation Example 2-12> Preparation of 2-chloro-4-(cyclohexylamino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 90%).

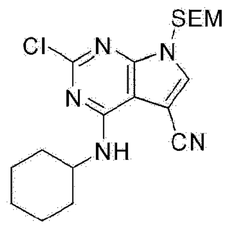

**<Preparation Example 2-13> Preparation of (*R*)-2-chloro-4-((tetrahydrofuran-3-yl)amino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 55%).

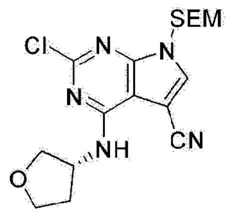

**<Preparation Example 2-14> Preparation of (*S*)-2-chloro-4-((tetrahydrofuran-3-yl)amino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 61%).

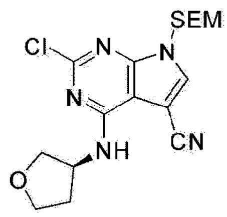

**<Preparation Example 2-15> Preparation of 2-chloro-4-((tetrahydro-2*H*-pyran-4-yl)amino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 86%).

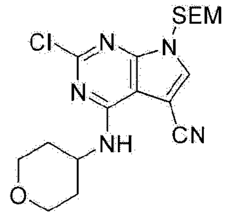

**<Preparation Example 2-16> Preparation of 2-chloro-4-((cyclopropylmethyl)amino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 73%).

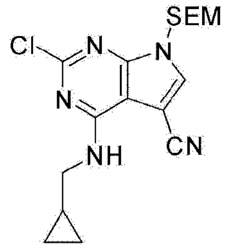

**<Preparation Example 2-17> Preparation of 2-chloro-4-((1-methoxy-2-methylpropan-2-yl)amino)-7-((2-(trimethylsilyl)ethoxy)methyl)-7*H*-pyrrolo[2,3-*d*]pyrimidine-5-carbonitrile**

2,4-dichloro-7-((2-(trimethylsilyl)ethoxy)methyl)-7*H*-pyrrolo[2,3-*d*]pyrimidine-5-carbonitrile (1.0 equivalent), DIPEA (2.5 equivalents), and amine (1.2 equivalents) were dissolved in NMP (0.25 M), and the reaction mixture was then stirred at 80°C for 12 hours. After the reaction was terminated, the organic solvent was removed by concentration under reduced pressure. The resulting reaction product was dissolved in EtOAc, and then washed with an aqueous 1 N HCl solution and brine. Then, the remaining water was removed using $Na_2SO_4$, and the reaction product was concentrated under reduced pressure to obtain the target compound as a yellow solid (yield: 97%).

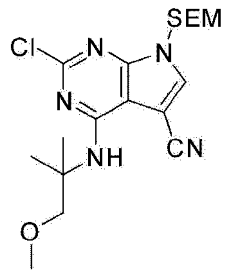

**<Preparation Example 2-18> Preparation of 2-chloro-4-((cyclobutylmethyl)amino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 86%).

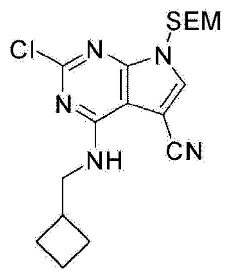

**<Preparation Example 2-19> Preparation of 2-chloro-4-((cyclopentylmethyl)amino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 78%).

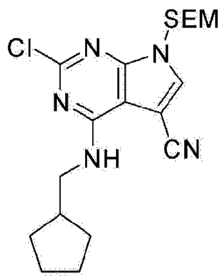

**<Preparation Example 2-20> Preparation of 2-chloro-4-((2-(methylsulfonyl)ethyl)amino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 70%).

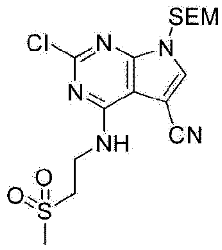

**<Preparation Example 2-21> Preparation of 4-(butylamino)-2-chloro-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 51%).

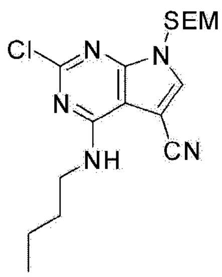

**<Preparation Example 2-22> Preparation of 2-chloro-4-(oxetan-3-ylamino)-7-((2-(trimethylsilyl)ethoxy)methyl)-7*H*-pyrrolo[2,3-*d*]pyrimidine-5-carbonitrile**

The title compound was prepared in a similar manner as in Preparation Example 2-1 (yield: 53%).

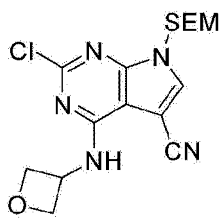

**<Preparation Example 3-1> Preparation of 2-chloro-*N*-methyl-5-(trifluoromethyl)-7-((2-(trimethylsilyl)ethoxy)methyl)-7*H*-pyrrolo[2,3-*d*]pyrimidine-4-amine**

The title compound was prepared in a manner as shown in the following Scheme 3.

[Scheme 3]

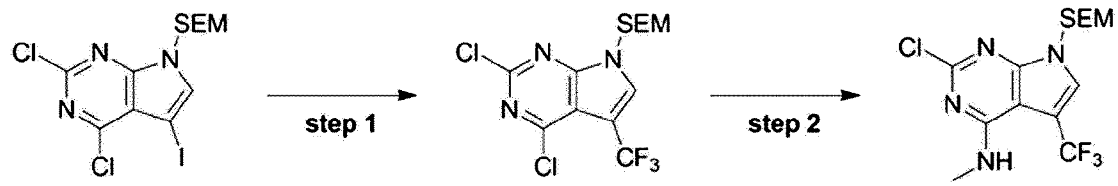


Step 1: Moisture was removed while maintaining CuI (5.0 equivalents) and KF (5.0 equivalents) at a temperature of 150°C for 2 hours under reduced pressure close to a vacuum. The resulting reaction product was cooled to room temperature, and TMS-CF3 (5.0 equivalents) was then dissolved in NMP (1.12 M) under nitrogen, and then slowly added to the reaction product through a syringe. The reaction mixture was reacted at room temperature for an hour, and 4-dichloro-3-iodo-1-((2-(trimethylsilyl)ethoxy)methyl)-1*H*-pyrrolo[2,3-*b*]pyrimidine (1.0 equivalent) was further dissolved in NMP (0.45 M) under nitrogen, and then slowly added through a syringe. The resulting reaction mixture was stirred at 50°C for 12 hours. After the reaction was terminated, the reaction product was cooled to room temperature. Then, distilled water was added to the reaction product, and the organic matter was extracted with EtOAc (x 3). After the collected organic layer was washed with brine, the remaining water was removed using $Na_2SO_4$, and the organic layer was concentrated under reduced pressure. The concentrated mixture was purified by column chromatography ($SiO_2$, PE:EA), and the target compound was obtained as a yellow liquid (yield: 62%).

Step 2: 2,4-dichloro-5-(trifluoromethyl)-7-((2-(trimethylsilyl)ethoxy)methyl)-7*H*-pyrrolo[2,3-*d*]pyrimidine (1.0 equivalent), DIPEA (2.9 equivalents), and methyl amine (1.5 equivalents) were dissolved in EtOH (0.25 M), and the reaction mixture was then stirred at 80°C for 16 hours. After the reaction was terminated, the organic solvent was removed by concentration under reduced pressure. The resulting reaction product was dissolved in EtOAc, and then washed with an aqueous 1 N HCl solution and brine. Then, the remaining water was removed using $Na_2SO_4$, and the reaction product was concentrated under reduced pressure to obtain the target compound as a brown solid (yield: 98%).

**<Preparation Example 3-2> Preparation of 2-chloro-*N*-ethyl-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 99%).

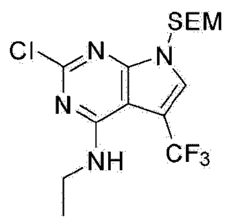

**<Preparation Example 3-3> Preparation of 2-chloro-*N*-propyl-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 90%).

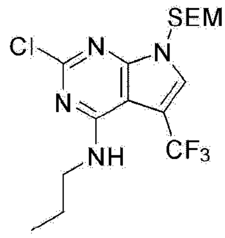

**<Preparation Example 3-4> Preparation of 2-chloro-*N*-isobutyl-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 80%).

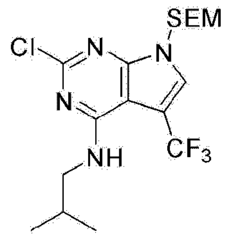

**<Preparation Example 3-5> Preparation of 2-chloro-*N*-isopropyl-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 82%).

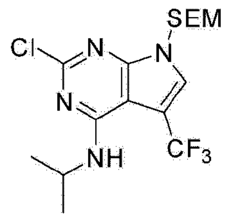

**<Preparation Example 3-6> Preparation of (*R*)-*N*-(*sec*-butyl)-2-chloro-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 62%).

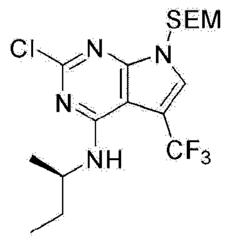

**<Preparation Example 3-7> Preparation of (*S*)-*N*-(*sec*-butyl)-2-chloro-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 53%).

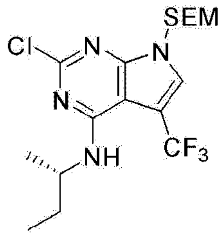

**<Preparation Example 3-8> Preparation of 2-chloro-*N*-(2-methoxyethyl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 75%).

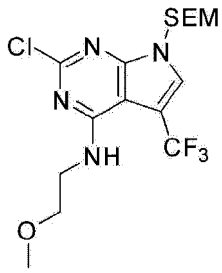

**<Preparation Example 3-9> Preparation of 2-chloro-*N*-cyclopropyl-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 68%).

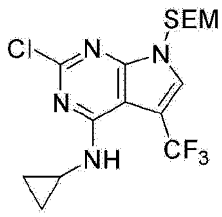

**<Preparation Example 3-10> Preparation of 2-chloro-*N*-cyclobutyl-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 74%).

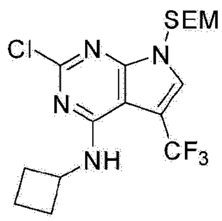

**<Preparation Example 3-11> Preparation of 2-chloro-*N*-cyclopentyl-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 74%).

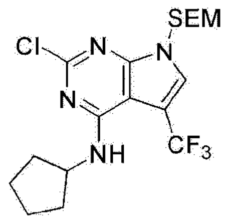

**<Preparation Example 3-12> Preparation of 2-chloro-*N*-cyclohexyl-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 71%).

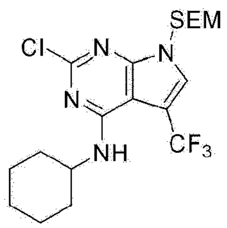

**<Preparation Example 3-13> Preparation of (*R*)-2-chloro-*N*-(tetrahydrofuran-3-yl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 66%).

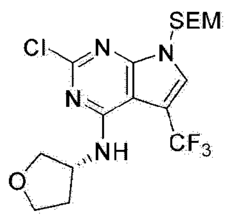

**<Preparation Example 3-14> Preparation of (*S*)-2-chloro-*N*-(tetrahydrofuran-3-yl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 66%).

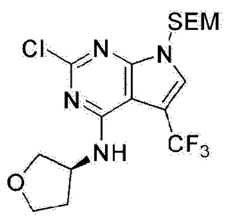

**<Preparation Example 3-15> Preparation of 2-chloro-*N*-(tetrahydro-2*H*-pyran-4-yl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 78%).

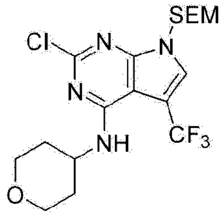

**<Preparation Example 3-16> Preparation of 2-chloro-*N*-(cyclopropylmethyl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 80%).

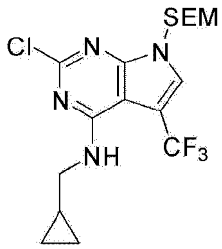

**<Preparation Example 3-17> Preparation of 2-chloro-*N*-(1-methoxy-2-methylpropan-2-yl)-5-(trifluoromethyl)-7-((2-(trimethylsilyl)ethoxy)methyl)-7*H*-pyrrolo[2,3-*d*]pyrimidine-4-amine**

2,4-dichloro-5-(trifluoromethyl)-7-((2-(trimethylsilyl)ethoxy)methyl)-7*H*-pyrrolo[2,3-*d*]pyrimidine (1.0 equivalent), DIPEA (2.5 equivalents), and amine (1.2 equivalents) were dissolved in NMP (0.25 M), and the reaction mixture was then stirred at 80°C for 12 hours. After the reaction was terminated, the organic solvent was removed by concentration under reduced pressure. The resulting reaction product was dissolved in EtOAc, and then washed with an aqueous 1 N HCl solution and brine. Then, the remaining water was removed using $Na_2SO_4$, and the reaction product was concentrated under reduced pressure to obtain the target compound (yield: 80%).

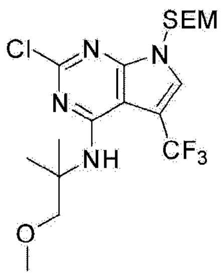

**<Preparation Example 3-18> Preparation of 2-chloro-*N*-(cyclobutylmethyl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 77%).

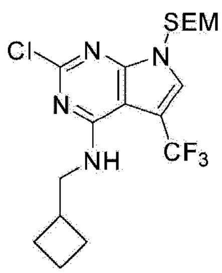

**<Preparation Example 3-19> Preparation of 2-chloro-*N*-(cyclopentylmethyl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 75%).

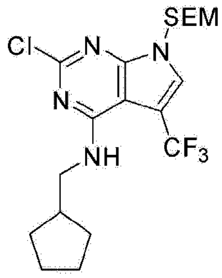

**<Preparation Example 3-20> Preparation of 2-chloro-*N*-(2-(methylsulfonyl)ethyl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 53%).

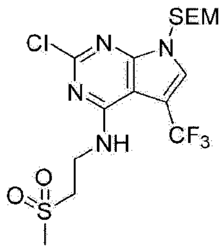

**<Preparation Example 3-21> Preparation of *N*-butyl-2-chloro-5-(trifluoromethyl)-7-((2-(trimethylsilyl)ethoxy)methyl)-7H-pyrrolo[2,3-d]pyrimidine-4-amine**

The title compound was prepared in a similar manner as in Preparation Example 3-1 (yield: 67%).

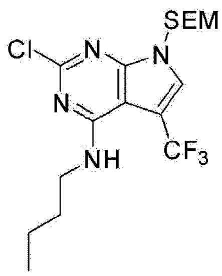

**<Preparation Example 3-22> Preparation of 2-chloro-*N*-(oxetan-3-yl)-5-(trifluoromethyl)-7-((2-(trimethylsilyl)ethoxy)methyl)-7*H*-pyrrolo[2,3-*d*]pyrimidine-4-amine**

The title compound was prepared in a similar manner as in Preparation Example 3-1 (yield: 35%).

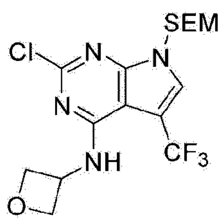

**<Preparation Example 4-1> Preparation of 2-chloro-4-cyclopropyl-7-((2-(trimethylsilyl)ethoxy)methyl)-7*H*-pyrrolo[2,3-*b*]pyrimidine-5-carbonitrile**

The title compound was prepared in a manner as shown in the following Scheme 4.

[Scheme 4]

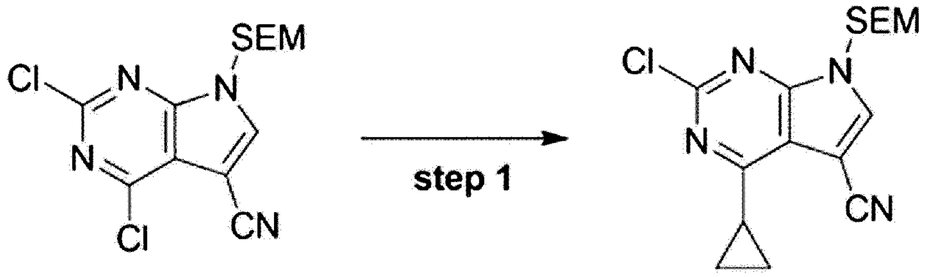


Step 1: 2,4-dichloro-7-((2-(trimethylsilyl)ethoxy)methyl)-7*H*-pyrrolo[2,3-*b*]pyrimidine-5-carbonitrile (1.0 equivalent), cyclopropylboronic acid (1.5 equivalents), and K3PO4 (3.0 equivalents) were dissolved in 1,4-dioxane (0.20 M), and then sonicated for a minute to remove gases. Pd(dppf)$Cl_2$ (0.1 equivalents) and $Ag_2O$ (0.5 equivalents) were added thereto under nitrogen, and the resulting mixture was reacted at 90°C for 16 hours. The reaction mixture was filtered through Celite, and washed several times with DCM. The resulting filtrate was concentrated, and purified by MPLC (EtOAc:Hex), and the target compound was obtained (yield: 65%).

**<Preparation Example 5-1> Preparation of 2-chloro-4-cyclopropyl-5-(trifluoromethyl)-7-((2-(trimethylsilyl)ethoxy)methyl)-7*H*-pyrrolo[2,3-*b*]pyrimidine**

The title compound was prepared in a manner as shown in the following Scheme 5.

[Scheme 5]

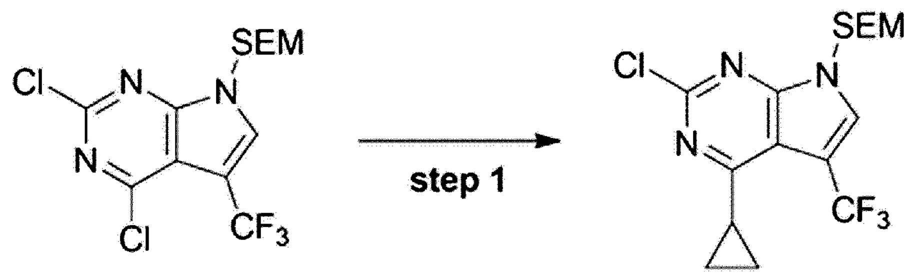


Step 1: 2,4-dichloro-5-(trifluoromethyl)-7-((2-(trimethylsilyl)ethoxy)methyl)-7*H*-pyrrolo[2,3-*b*]pyrimidine (1.0 equivalent), cyclopropylboronic acid (1.5 equivalents), and K3PO4 (3.0 equivalents) were dissolved in 1,4-dioxane (0.15 M), and then sonicated for a minute to remove gases. Pd(dppf)$Cl_2$ (0.1 equivalents) and $Ag_2O$ (0.5 equivalents) were added thereto under nitrogen, and the resulting mixture was reacted at 90°C for 16 hours. The reaction mixture was filtered through Celite, and washed several times with DCM. The resulting filtrate was concentrated, and then purified by MPLC (EtOAc:Hex), and the target compound was obtained as a white liquid (yield: 52%).

**<Preparation Example 6-1> Preparation of (5-amino-2,3-dihydro-1,4-benzodioxin-8-yl)-morpholino-methanone**

The title compound was prepared in a manner as shown in the following Scheme 6.

**[Scheme 6]**

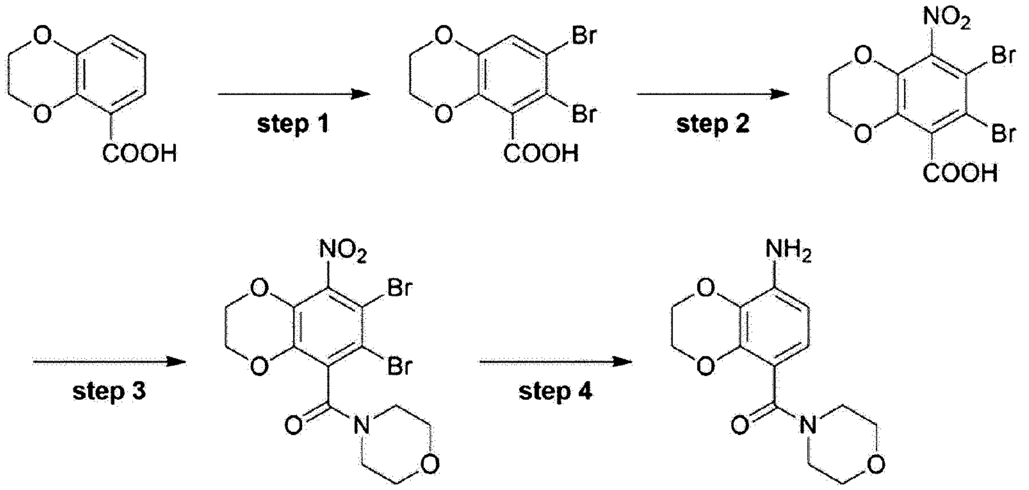


Step 1: 2,3-dihydro-1,4-benzodioxin-5-carboxylic acid (1 equivalent) was dissolved in HOAc (200 mL), and $Br_2$ (2.32 equivalents) was then added dropwise thereto. NaOAc (3.6 equivalents) was added dropwise, and the resulting mixture was then stirred at 45°C for 4 hours. After the reaction was terminated, water was added to the mixture, and the pH of the mixture was adjusted to pH 1 using HCl (30 mL). Thereafter, 50% sodium bisulfite (30 mL) was added to the mixture. The reaction solid was filtered, and then washed several times with water to obtain a solid compound (yield: 77%).

$^1$H NMR ($CDCl_3$, 400 MHZ): δ = 7.26 (s, 1H), 4.35 -.4.30 (m, 4H)

Step 2: 6,7-dibromo-2,3-dihydro-1,4-benzodioxin-5-carboxylic acid (1 equivalent) was dissolved in acetic acid (500 mL), and concentrated sulfuric acid (6.4 equivalents) was then added dropwise thereto at 0°C. Thereafter, nitric acid (1.75 equivalents) was slowly added dropwise to the mixture. In this case, the temperature was maintained at 15°C or lower. The reaction mixture was stirred at 40°C or 12 hours. After the reaction was terminated, ice water was added to the reaction mixture, and the reaction mixture was stirred for an hour. The resulting solid filtrate was filtered, and then washed several times with water to obtain the final compound (yield: 88%).

$^1$H NMR ($CDCl_3$, 400 MHZ): δ = 8.00 (s, 1H), 4.44 - 4.40 (m, 4H)

Step 3: 6,7-dibromo-5-nitro-2,3-dihydro-1,4-benzodioxin-8-carboxylic acid (1 equivalent) and morpholine (1.3 equivalents) were dissolved in DMF, and HATU (1.5 equivalents) and DIPEA (2.2 equivalents) were then added thereto. Thereafter, the mixture was stirred at 20°C for 12 hours. After the reaction was terminated, the reaction mixture was neutralized with water, and then extracted with EA (x 2). Then, the remaining water was removed using $Na_2SO_4$, and the filtrate was then concentrated to obtain a yellow solid compound (yield: 84%).

$^1$H NMR ($CDCl_3$, 400 MHZ): δ = 4.42 - 4.39 (m, 4H), 4.00 - 3.62 (m, 6H), 3.30 - 3.23 (m, 2H);

**LCMS (Method 1):** LCMS_3, Rt = 0.85 min, 453 $(M+1)^+$

Step 4: 6,7-dibromo-5-nitro-2,3-dihydro-1,4-benzodioxin-8-yl)-morpholino-methanone (30 g, 1 equivalent) was dissolved in MeOH, and TEA (3.2 equivalents) and Pd/C (6.8 g, 10% Pd absorbed on active carbon) were then added thereto. Thereafter, the mixture was stirred at 75°C for 12 hours. In this case, the hydrogen pressure was maintained at 50 psi. After the reaction was terminated, the reaction mixture was filtered through Celite, and then concentrated. The resulting filtrate was purified by silica gel chromatography (DCM/MeOH = 200/1, 20/1), and the final compound was then obtained (yield: 130%).

$^1$H NMR (DMSO-$d_6$, 400 MHZ): δ = 6.52 (d, $J$ = 8.4 Hz, 1H), 6.25 (d, $J$ = 8.4 Hz, 1H), 4.99 (s, 2H), 4.24 (s, 4H), 3.55 (s, 6H), 3.27 - 3.07 (m, 2H);

LCMS (Method 2): LCMS_4, Rt = 0.57 min, 265 $(M+1)^+$

**<Preparation Example 7-1> Preparation of (5-amino-2,3-dihydro-1,4-benzooxin-8-yl)-(4-morpholinopiperidin-1-yl)methanone**

The title compound was prepared in a manner as shown in the following Scheme 7.

[Scheme 7]

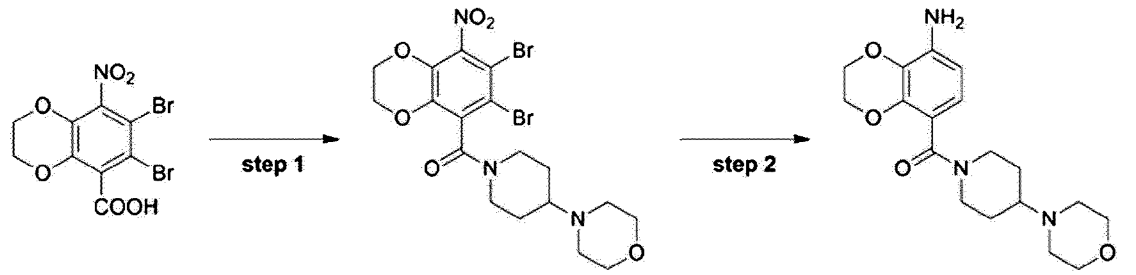


Step 1: (6,7-dibromo-5-nitro-2,3-dihydro-1,4-benzodioxin-8-yl)-(4-morpholinopiperidin-1-yl)methanone was prepared in a similar manner as in Step 3 of Preparation Example 6-1 (yield: crude 268%).

$^1$H NMR ($CDCl_3$, 400 MHZ): δ = 4.87 - 4.65 (m, 1H), 4.51 - 4.27 (m, 4H), 3.78 (q, *J* = 4.2 Hz, 4H), 3.56 - 3.51 (m, 1H), 3.17 - 3.04 (m, 1H), 2.98 - 2.85 (m, 1H), 2.68 - 2.45 (m, 5H), 2.09 - 2.00 (m, 1H), 1.97 - 1.85 (m, 1H), 1.70 - 1.57 (m, 1H), 1.54 - 1.42 (m, 1H);

LCMS (Method 1): LCMS_1, Rt = 0.76 min, 536 $(M+1)^+$;

Step 2: (5-amino-2,3-dihydro-1,4-benzooxin-8-yl)-(4-morpholinopiperidin-1-yl)methanone was prepared in a similar manner as in Step 4 of Preparation Example 6-1 (yield: 72%).

$^1$H NMR (DMSO- $d_6$, 400 MHZ): δ = 6.47 (s, 1H), 6.24 (d, *J* = 8.0 Hz, 1H), 4.94 (s, 2H), 4.43 (d, *J* = 1.6 Hz, 1H), 4.23 (s, 4H), 3.33 (s, 6H), 3.03 - 2.61 (m, 3H), 2.49 - 2.30 (m, 3H), 1.93 - 1.64 (m, 2H), 1.43 - 1.23 (m, 2H);

LCMS (Method 2): LCMS_2, Rt = 0.82 min, 348 $(M+1)^+$

**<Preparation Example 8-1> Preparation of 2-methoxy-4-morpholinosulfonyl-aniline**

The title compound was prepared in a manner as shown in the following Scheme 8.

[Scheme 8]

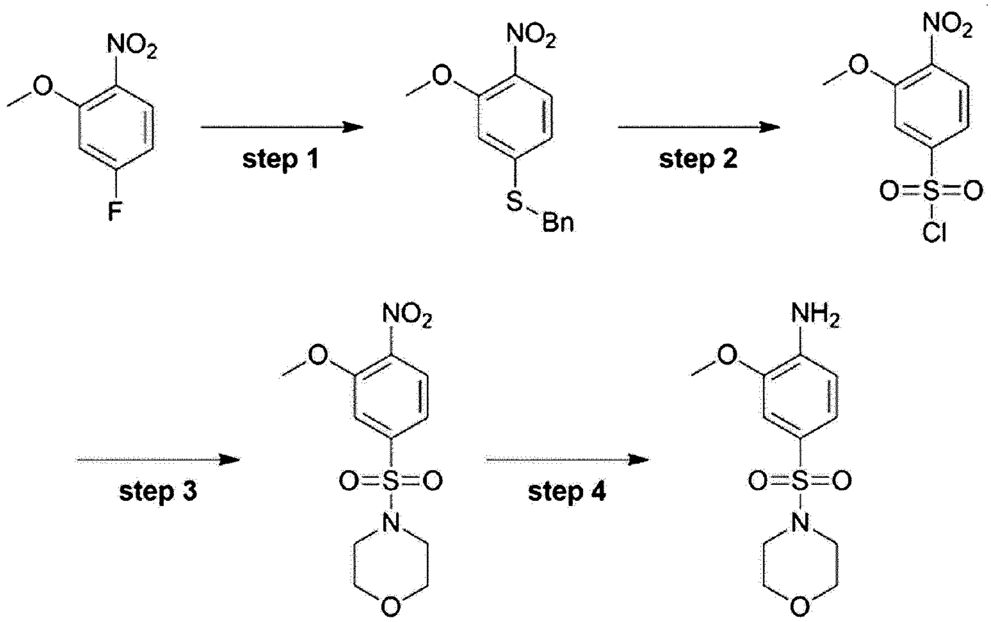


Step 1: 4-fluoro-2-methoxy-1-nitro-benzene (1 equivalent) was dissolved in ACN, and phenylmethanethiol (1.1 equivalents) and DIPEA (1 equivalent) were then slowly added dropwise thereto. The reaction mixture was stirred at 80°C for 48 hours. After the reaction was terminated, the reaction mixture was concentrated, and MTBE was added thereto. Then, a yellow solid compound was able to be obtained (yield: 14%).

$^1$H NMR ($CDCl_3$, 400 MHZ): HNMR_1, δ = 7.83 (d, *J* = 8.4 Hz, 1H), 7.43 - 7.27 (m, 5H), 6.92 - 6.84 (m, 2H), 4.23 (s, 2H), 3.86 (s, 3H)

Step 2: 4-benzylsulfanyl-2-methoxy-1-nitro-benzene (1 equivalent) was dissolved in ACN, acetic acid, and

water at 0°C, and NCS (4.24 equivalents) was added dropwise thereto. The mixture was stirred at the same temperature for 2 hours. After the reaction was terminated, the solvent was removed, and the reaction mixture was then extracted with EA (x 2). Then, the remaining water was removed using $Na_2SO_4$, and then concentrated. The concentrate was crystallized using petroether, and then filtered to obtain a yellow solid compound (yield: 97%).

$^1H$ NMR ($CDCl_3$): $\delta$ = 8.08 - 7.86 (m, 1H), 7.74 (d, *J* = 2.0 Hz, 2H), 4.09 (s, 3H);

Step 3: 3-methoxy-4-nitro-benzenesulfonyl chloride (1 equivalent) was dissolved in DCM, and DMAP (0.05 equivalents), TEA (2 equivalents), and morpholine (1.5 equivalents) were slowly added thereto at 0°C. The reaction mixture was stirred at 20°C for 4 hours. After the reaction was terminated, the reaction mixture was neutralized with water, dissolved in DCM, and then washed with an aqueous 1 N HCl solution, an aqueous NaHCO3 solution, and brine. Then, the remaining water was removed using $Na_2SO_4$, and the reaction mixture was concentrated to obtain a yellow solid compound (yield: 93%).

$^1H$ NMR ($CDCl_3$, 400 MHZ): $\delta$ = 7.94 (d, *J* = 8.4 Hz, 1H), 7.46 - 7.38 (m, 2H), 4.04 (s, 3H), 3.86 - 3.56 (m, 4H), 3.15 - 2.85 (m, 4H)

Step 4: 4-(3-methoxy-4-nitro-phenyl)sulfonylmorpholine (40 g) was dissolved in THF (10 mL), and Pd/C (5 g, 10% Pd absorbed on active carbon) was added thereto. The reaction mixture was purged three times with hydrogen, and then stirred at 20°C for 16 hours. After the reaction was terminated, the reaction mixture was filtered through Celite, and the resulting filtrate was concentrated. Then, the filtrate was crystallized by adding MTBE, and then filtered to obtain a white solid compound (yield: 74%).

$^1H$ NMR (DMSO-$d_6$, 400 MHZ): $\delta$ = 7.08 (dd, *J* = 2.0, 8.4 Hz, 1H), 6.97 (d, *J* = 2.0 Hz, 1H), 6.74 (d, *J* = 8.4 Hz, 1H), 5.76 (s, 2H), 3.83 (s, 3H), 3.69 - 3.52 (m, 4H), 2.89 - 2.71 (m, 4H);

LCMS (Method 1): LCMS_1, Rt = 0.922 min, 273.1 $(M+1)^+$;

**<Preparation Example 9-1> Preparation of 2-methoxy-4-((morpholinopiperidin-1-yl)sulfonyl)aniline**

The title compound was prepared in a manner as shown in the following Scheme 9.

[Scheme 9]

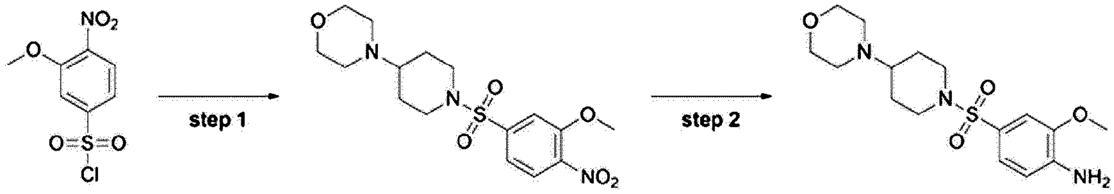


Step 1: 4-(1-(3-methoxy-4-nitro-phenyl)sulfonyl)piperidin-4-yl)morpholine was prepared in a similar manner as in Step 3 of Preparation Example 7-1 (yield: 73%).

$^1H$ NMR (DMSO-$d_6$, 400 MHZ): $\delta$ = 8.10 (d, *J* = 8.4 Hz, 1H), 7.52 - 7.43 (m, 2H), 4.02 (s, 3H), 3.69 (d, *J* = 12.0 Hz, 2H), 3.56 - 3.47 (m, 4H), 2.44 - 2.33 (m, 6H), 2.24 - 2.12 (m, 1H), 1.81 (d, *J* = 11.2 Hz, 2H), 1.41 (m, 2H).

Step 2: 2-methoxy-4-((4-morpholinopiperidin-1-yl)sulfonyl)aniline was prepared in a similar manner as in Step 4 of Preparation Example 7-1 (yield: 38%).

$^1H$ NMR (DMSO-$d_6$, 400 MHZ): $\delta$ = 7.07 (dd, *J* = 2.0, 8.4 Hz, 1H), 6.97 (d, *J* = 2.0 Hz, 1H), 6.72 (d, *J* = 8.4 Hz, 1H), 5.66 (s, 2H), 3.81 (s, 3H), 3.63 - 3.54 (m, 2H), 3.54 - 3.45 (m, 4H), 2.42 - 2.31 (m, 4H), 2.24 - 2.13 (m, 2H), 2.06 (m, 1H), 1.83 - 1.73 (m, 2H), 1.46 - 1.32 (m, 2H);

LCMS (Method 1): LCMS_1, Rt = 0.97 min, 356.1 $(M+1)^+$

**<Preparation Example 10-1> Preparation of 4-(4-amino-3-methoxyphenyl)-1-cyclopropyl-1,4-azaphosphinane 4-oxide**

The title compound was prepared in a manner as shown in the following Scheme 10.

**[Scheme 10]**

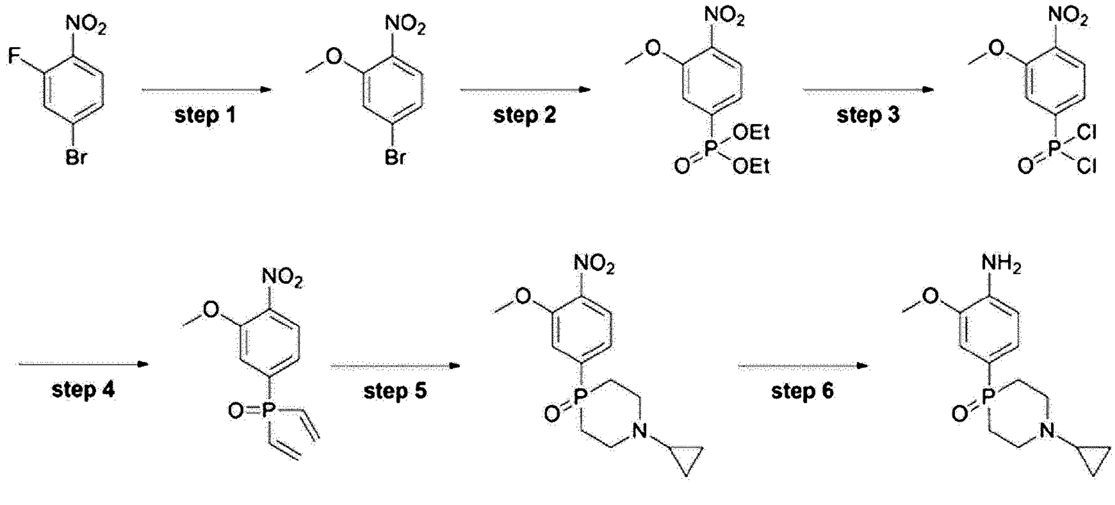


Step 1: 4-bromo-2-fluoro-1-nitro-benzene (1 equivalent) was dissolved in MeOH, and $K_2CO_3$ (1 equivalent) was added thereto. Thereafter, the reaction mixture was reacted at 65°C for 2 hours. After the reaction was terminated, the solvent was concentrated, neutralized with water, dissolved in EA (×2), and then washed with brine. Then, the remaining water was removed using $Na_2SO_4$, and concentrated to obtain a yellow solid compound (yield: 99%).

$^1$H NMR (DMSO-$d_6$, 400 MHZ): δ = 7.85 (d, *J* = 8.6 Hz, 1H), 7.61 (d, *J* = 1.9 Hz, 1H), 7.33 (dd, *J* = 2.0, 8.6 Hz, 1H), 3.96 (s, 3H).

Step 2: 1-ethoxyphosphonyloxyethane (0.3 equivalents) and 4-bromo-2-methoxy-1-nitro-benzene (1 equivalent) were dissolved in toluene, and TEA (1.7 equivalents), KOAc (0.1 equivalents), DPPF (0.04 equivalents), and Pd(OAc)2 (0.02 equivalents) were added thereto. The reaction mixture was stirred at 100°C for 12 hours under nitrogen. After the reaction was terminated, the filtrate was concentrated, and then purified by silica gel chromatography (petroleum ether/ethyl acetate = 10:1 to 1:1). A solid compound was recrystallized with petroleum ether, and then filtered to obtain a yellow compound (yield: 53%).

$^1$H NMR (DMSO-$d_6$, 400 MHZ): δ = 8.01 (dd, *J* = 4.5, 8.1 Hz, 1H), 7.53 (dd, *J* = 1.0, 14.7 Hz, 1H), 7.44 (ddd, *J* = 1.2, 8.1, 12.5 Hz, 1H), 4.13 - 4.04 (m, 4H), 4.00 (s, 3H), 1.26 (t, *J* = 7.0 Hz, 6H);

LCMS (Method 1): LCMS_2, Rt = 0.87 min, 290 $(M+1)^+$

Step 3: 4-diethoxyphosphoryl-2-methoxy-1-nitro-benzene (50 g, 167.69 mmol) was dissolved in DMF (13.72 mL), and $SOCl_2$ (60 mL) was then slowly added thereto. The reaction mixture was stirred at 80°C for 2 hours. After the reaction was terminated, the solvent was concentrated, recrystallized with (DCM/heptane), and then filtered to obtain a yellow solid compound (yield: crude 85%).

$^1$H NMR (DMSO-$d_6$, 400 MHZ): δ = 7.93 (dd, *J* = 4.0, 8.1 Hz, 1H), 7.59 - 7.49 (m, 1H), 7.39 (ddd, *J* = 1.2, 8.1, 12.2 Hz, 1H), 4.00 - 3.92 (m, 3H).

Step 4: 4-dichlorophosphoryl-2-methoxy-1-nitro-benzene (40 g, crude) was dissolved in THF (500 mL) at -78°C, and bromo(vinyl)magnesium (1 M in THF, 325.92 mL) was then slowly added dropwise thereto. The reaction mixture was stirred at -78°C for an hour. When the reaction was terminated, the reaction mixture was neutralized with aqueous $NH_4Cl$, dissolved in EA (x 2), and then washed with aqueous $NaHCO_3$ and brine. Then, the remaining water was removed using $Na_2SO_4$, and concentrated to obtain a brown oil (yield: 67%).

$^1$H NMR (DMSO-$d_6$, 400 MHZ): δ = 8.07 - 7.96 (m, 1H), 7.58 (dd, *J* = 1.1, 12.5 Hz, 1H), 7.45 (ddd, *J* = 1.3, 8.1, 10.9 Hz, 1H), 6.87 - 6.67 (m, 2H), 6.35 (dd, *J* = 1.9, 12.6 Hz, 1H), 6.27 - 6.14 (m, 3H), 3.99 (s, 3H);

LCMS (Method 1): LCMS_4, Rt = 0.76 min, 254 $(M+1)^+$

Step 5: cyclopropylamine (1.3 equivalents) and 4-divinylphosphoryl-2-methoxy-1-nitro-benzene (1 equivalent) were dissolved in THF and $H_2O$, and then reacted at 80°C for 12 hours. After the reaction was terminated, the solvent was concentrated, dissolved in DCM (x 2), and then washed with aqueous NaHCO3 and brine. Then, the remaining water was removed using $Na_2SO_4$, and concentrated. The filtrate was purified by silica chromatography (ethyl acetate/MeOH = 100:1 to 20/1), and a compound was then able to be obtained (yield: 72%)

$^1$H NMR (DMSO-$d_6$, 400 MHZ): δ = 7.99 (dd, *J* = 2.7, 8.1 Hz, 1H), 7.65 (d, *J* = 11.9 Hz, 1H), 7.58 - 7.50 (m, 1H), 7.24 (d, *J* = 8.3 Hz, 1H), 6.59 - 6.47 (m, 2H), 4.01 (s, 3H), 3.77 (d, *J* = 4.0 Hz, 6H), 3.56 (s, 2H), 2.94 - 2.70 (m, 4H), 2.38 - 2.30 (m, 2H), 1.99 - 1.86 (m, 2H);

LCMS (Method 2): LCMS_5, Rt = 0.62 min, 421 $(M+1)^+$

Step 6: 1-cyclopropyl-4-(3-methoxy-4-nitrophenyl)-1,4-azaphosphinane 4-oxide (1 equivalent) and NH4Cl were

dissolved in MeOH, and Zn was then added thereto. The reaction mixture was stirred under reflux for 12 hours. After the reaction was terminated, the reaction mixture was filtered through Celite, and a solid compound was then able to be obtained (yield: 93%).

$^1$H NMR (4 DMSO-$d_6$, 400 MHz): δ = 7.16 - 6.99 (m, 2H), 6.77 - 6.64 (m, 1H), 5.44 - 5.21 (m, 2H), 3.90 - 3.73 (m, 3H), 3.42 - 3.24 (m, 2H), 3.03 - 2.85 (m, 4H), 2.19 - 2.00 (m, 2H), 1.89 - 1.67 (m, 3H), 0.51 - 0.43 (m, 2H), 0.38 - 0.31 (m, 2H)

LCMS (Method 1): LCMS_2, Rt = 1.241 min, 281.1 $(M+H)^+$

**<Example 1> Preparation 1 of compounds according to the present invention**

The pyrrolopyrimidine derivative compounds according to the present invention were prepared in a manner as shown in the following Scheme 11.

[Scheme 11]

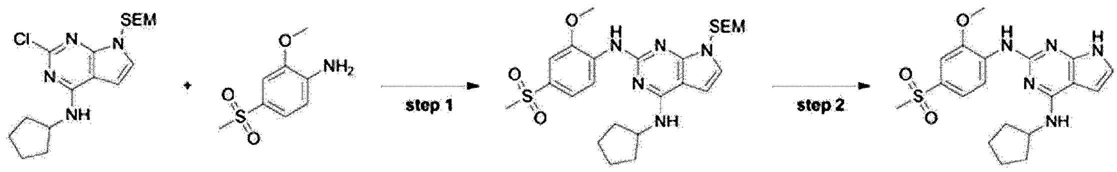


Step 1: The compound (1.0 equivalent) of Preparation Example 1-11, 2-methoxy-4-(methylsulfonyl)aniline (1.2 equivalents), and $K_2CO_3$ (5.0 equivalents) were added and dissolved in sec-BuOH (0.1 M), and then degassed by sonication for one minute. Pd2(dba)3 (0.1 equivalents) and Xphos (0.1 equivalents) were added to the reaction mixture under nitrogen, and then stirred at 100°C for 2 hours. The reaction mixture was filtered through Celite, and washed with ethyl acetate. The resulting filtrate was concentrated, and the resulting liquid mixture was used in the next step without any further purification.

Step 2: *N*[4]-cyclopentyl-*N*[2]-(2-methoxy-4-(methylsulfonyl)phenyl)-7-((2-(trimethylsilyl)ethoxy)methyl)-7*H*-pyrrolo[2,3-*d*]pyrimidine-2,4-diamine (1.0 equivalent) was dissolved in TFA (0.2 M), and then reacted at room temperature for an hour. After the reaction was terminated, the solvent was removed. The concentrated mixture was again dissolved in EtOH (0.2 M), and $NH_4OH$ (0.1 M) was then added thereto. Then, the mixture was reacted at 60°C for an hour. After the reaction, the solvent was removed by concentration under reduced pressure. The concentrated mixture was purified by Pre-HPLC, and the target compound was obtained as a solid (yield: 13%).

Compounds of Examples 2 to 477 were prepared in a similar manner as in Example 1. The chemical structures, the compound names, and NMR, mass and HPLC analysis results of the compounds of Examples 1 to 477 are summarized and listed in Table 1 below.

[Table 1]

| Example | Structure | Compound name | $^1H$ NMR, MS | Yield (%) | HPLC r.t. (min), Purity |
|---|---|---|---|---|---|
| 1 | 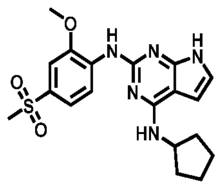 | *N*4-cyclopentyl-*N*2-(2-methoxy-4-(methylsulfonyl)phenyl)-7*H*-pyrrolo[2,3-*d*]pyrimidine-2,4-diamine | $^1H$ NMR (400 MHz, DMSO-$d_6$): δ = 11.16 (s, 1H), 8.93 (d, *J* = 8.6 Hz, 1H), 7.51 (s, 1H), 7.47 (m, 1H), 7.41 (d, *J* = 1.9 Hz, 1H), 7.30 (d, *J* = 7.0 Hz, 1H), 6.88 - 6.83 (m, 1H), 6.52 (m, 1H), 4.54 - 4.41 (m, 1H), 4.00 (s, 3H), 3.17 (s, 3H), 2.10 - 1.94 (m, 2H), 1.78 - 1.69 (m, 2H), 1.67 - 1.53 (m, 4H); LCMS (Method 2): m/z = 402.2 [M + H]+ | 37.4 | 2.83 |
| 2 | 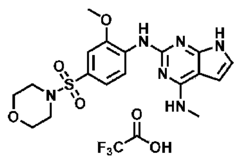 | *N*2-(2-methoxy-4-(morpholinosulfon yl)phenyl)-*N*4-methyl-7*H*-pyrrolo[2,3-*d*]pyrimidine-2,4-diamine 2,2,2-trifluoroacetate | $^1H$ NMR (400 MHz, TFA salt, DMSO-$d_6$): δ = 12.08 - 11.62 (m, 1H), 9.13 - 8.68 (m, 2H), 7.38 (dd, *J* = 1.8, 8.6 Hz, 1H), 7.27 (s, 1H), 7.04 (br s, 1H), 6.61 (br s, 1H), 4.04 (s, 3H), 3.67 - 3.63 (m, 4H), 3.08 (br s, 3H), 2.93 - 2.88 (m, 4H); LCMS (Method 2): m/z = 419.1 [M + H]+ | 21 | 2.39 |

(continued)

| Example | Structure | Compound name | $^1$H NMR, MS | Yield (%) | HPLC r.t. (min), Purity |
|---|---|---|---|---|---|
| 3 | 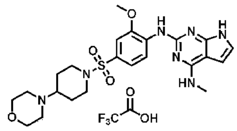 | $N^2$-(2-methoxy-4-((4-morpholinopiperid in-1-yl) sulfonyl)phenyl )-$N^4$-methyl-7*H*-pyrrolo[2,3-*d*]pyrimidine-2,4-diamine 2,2,2-trifluoroacetate | $^1$H NMR (400 MHz, TFA salt, DMSO-$d_6$): δ = 11.85 - 11.49 (m, 1H), 10.09 - 9.73 (m, 1H), 8.85 (d, *J* = 8.6 Hz, 1H), 8.74 - 7.97 (m, 1H), 7.37 (dd, *J* = 1.9, 8.6 Hz, 1H), 7.26 (d, *J* = 1.6 Hz, 1H), 6.98 (br s, 1H), 6.55 (br s, 1H), 4.02 (s, 3H), 3.98 (br d, *J* = 13.0 Hz, 2H), 3.84 - 3.73 (m, 3H), 3.64 (br d, *J* = 11.1 Hz, 2H), 3.43 - 3.30 (m, 2H), 3.25 - 3.15 (m, 1H), 3.03 (br d, *J* = 3.4 Hz, 3H), 2.25 (br t, *J* = 11.4 Hz, 2H), 2.17 - 2.02 (m, 2H), 1.73 - 1.59 (m, 1H), 1.77 - 1.57 (m, 1H); LCMS (Method 2): m/z = 502.2 [M + H]$^+$ | 16 | 2.14 |
| 4 | 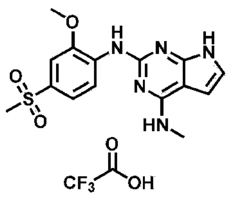 | $N^2$-(2-methoxy-4-(methylsulfonyl)p henyl)-$N^4$-methyl-7*H*-pyrrolo [2,3-*d*]pyrimidine-2,4-diamine 2,2,2-trifluoroacetate | $^1$H NMR (400 MHz, TFA salt, DMSO-$d_6$): δ = 12.13 - 11.56 (m, 1H), 9.28 - 8.20 (m, 2H), 7.66 - 7.44 (m, 2H), 7.12 - 6.87 (m, 1H), 6.59 (br s, 1H), 4.04 (s, 3H), 3.21 (s, 3H), 3.06 (br d, *J* = 3.1 Hz, 3H), 2.54 (s, 1H); LCMS (Method 2): m/z = 348.1 [M + H]$^+$ | 5.4 | 2.25 |

(continued)

| Example | Structure | Compound name | $^1$H NMR, MS | Yield (%) | HPLC r.t. (min), Purity |
|---|---|---|---|---|---|
| 5 | 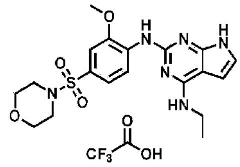 | *N*4-ethyl-*N*2-(2-methoxy-4-(morpholinosulfon yl) phenyl)-7*H*-pyrrolo[2,3-*d*] pyrimidine-2,4-diamine 2,2,2-trifluoroacetate | $^1$H NMR (400 MHz, TFA salt, DMSO-$d_6$): δ = 12.19 - 11.62 (m, 1H), 8.84 - 8.74 (m, 1H), 8.72 - 8.41 (m, 1H), 7.38 (dd, *J* = 1.9, 8.6 Hz, 1H), 7.26 (d, *J* = 1.8 Hz, 1H), 7.07 - 6.99 (m, 1H), 6.65 (br s, 1H), 4.03 (s, 3H), 3.68 - 3.62 (m, 4H), 3.59 - 3.52 (m, 2H), 2.95 - 2.85 (m, 4H), 1.29 (t, *J* = 7.2 Hz, 3H); LCMS (Method 2): m/z = 433.2 [M + H]$^+$ | 44 | 2.16 |
| 6 | 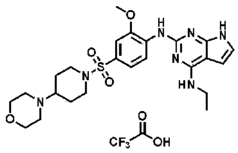 | *N*4-ethyl-*N*2-(2-methoxy-4-((4-morpholinopiperid in-1-yl)sulfonyl)phenyl )-7*H*-pyrrolo[2,3-*d*]pyrimidine-2,4-diamine 2,2,2-trifluoroacetate | $^1$H NMR (400 MHz, DMSO-$d_6$): δ = 11.79 (d, *J* = 1.8 Hz, 1H), 10.36 - 9.95 (m, 1H), 8.81 (d, *J* = 8.7 Hz, 1H), 8.67 - 8.44 (m, 1H), 7.39 (m, 1H), 7.28 (d, *J* = 2.0 Hz, 1H), 7.01 (br s, 1H), 6.63 (br s, 1H), 4.02 (s, 3H), 3.97 (br s, 1H), 3.83 (br s, 2H), 3.55 (m, 4H), 3.38 (d, *J* = 10.5 Hz, 2H), 3.21 (br t, *J* = 11.7 Hz, 2H), 3.13 - 2.92 (m, 2H), 2.33 - 2.21 (m, 2H), 2.13 (d, *J* = 11.0 Hz, 2H), 1.77 - 1.59 (m, 2H), 1.28 (t, *J* = 7.2 Hz, 3H); LCMS (Method 2): m/z = 516.2 [M + H]$^+$ | 75 | 1.87 |

(continued)

| Example | Structure | Compound name | $^1H$ NMR, MS | Yield (%) | HPLC r.t. (min), Purity |
|---|---|---|---|---|---|
| 7 | 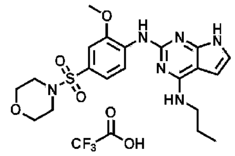 | *N*2-(2-methoxy-4-(morpholinosulfon yl) phenyl)-*N*4-propyl-7*H*-pyrrolo [2,3-*d*]pyrimidine-2,4-diamine 2,2,2-trifluoroacetate | $^1H$ NMR (400 MHz, TFA salt, DMSO-$d_6$): δ = 12.35 - 11.70 (m, 1H), 8.92 - 8.39 (m, 3H), 7.36 (dd, *J* = 1.4, 8.6 Hz, 1H), 7.30 - 7.20 (m, 1H), 7.09 - 6.97 (m, 1H), 6.65 (br s, 1H), 4.03 (s, 3H), 3.72 - 3.62 (m, 6H), 2.94 - 2.88 (m, 4H), 1.74 - 1.61 (m, 2H), 0.98 (t, *J* = 7.4 Hz, 3H); LCMS (Method 2): m/z = 447.2 [M + H]+ | 22 | 2.26 |
| 8 | 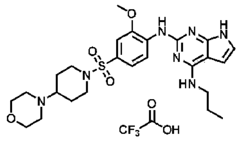 | *N*2-(2-methoxy-4-((4-morpholinopiperid in-1-yl) sulfonyl)phenyl )-*N*4-propyl-7*H*-pyrrolo[2,3-*d*]pyrimidine-2,4-diamine 2,2,2-trifluoroacetate | $^1H$ NMR (400 MHz, TFA salt, DMSO-$d_6$): δ = 11.94 - 11.62 (m, 1H), 10.51 - 9.94 (m, 1H), 8.81 (d, *J* = 8.6 Hz, 1H), 8.65 - 8.39 (m, 1H), 7.38 (m, 1H), 7.28 (d, *J* = 2.0 Hz, 1H), 7.01 (s, 1H), 6.64 (s, 1H), 4.02 (s, 3H), 3.98 (s, 2H), 3.82 (d, *J* = 12.0 Hz, 2H), 3.66 (s, 2H), 3.51 - 3.44 (m, 2H), 3.37 (s, 2H), 3.22 (t, *J* = 11.2 Hz, 1H), 3.04 (s, 2H), 2.27 (t, *J* = 11.6 Hz, 2H), 2.13 (d, *J* = 11.2 Hz, 2H), 1.75 - 1.66 (m, 4H), 0.98 (t, *J* = 7.2 Hz, 3H); LCMS (Method 2): m/z = 530.3 [M + H]+ | 30 | 1.96 |
| 9 | 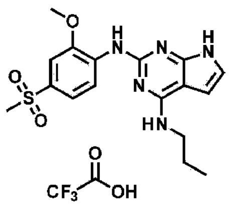 | *N*2-(2-methoxy-4-(methylsulfonyl)p henyl)-*N*4-propyl-7*H*-pyrrolo [2,3-*d*]pyrimidine-2,4-diamine 2,2,2-trifluoroacetate | $^1H$ NMR (400 MHz, TFA salt, DMSO-$d_6$): δ = 12.07 - 11.63 (m, 1H), 8.75 (d, *J* = 8.4 Hz, 2H), 7.69 - 7.35 (m, 2H), 7.03 (br s, 1H), 6.66 (br s, 1H), 4.03 (s, 3H), 3.46 (br d, *J* = 6.2 Hz, 2H), 3.21 (s, 3H), 1.69 (sxt, *J* = 7.3 Hz, 2H), 0.98 (t, *J* = 7.4 Hz, 3H); LCMS (Method 2): m/z = 376.1 [M + H]+ | 8 | 2.11 |

(continued)

| Example | Structure | Compound name | $^1H$ NMR, MS | Yield (%) | HPLC r.t. (min), Purity |
|---|---|---|---|---|---|
| 10 | 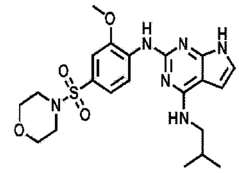 | *N*4-isobutyl-*N*2-(2-methoxy-4-(morpholinosulfon yl) phenyl)-7*H*-pyrrolo[2,3-*d*] pyrimidine-2,4-diamine | $^1H$ NMR (400 MHz, DMSO-$d_6$): δ = 11.16 (brs, 1H), 8.93 (d, *J* = 8.6 Hz, 1H), 7.52 (s, 2H), 7.30 (dd, *J* = 1.8, 8.6 Hz, 1H), 7.18 (d, *J* = 1.8 Hz, 1H), 6.90 - 6.83 (m, 1H), 6.52 (br s, 1H), 4.01 (s, 3H), 3.67 - 3.60 (m, 4H), 3.30 (br s, 2H), 2.93 - 2.86 (m, 4H), 2.06 - 1.94 (m, 1H), 0.97 (d, *J* = 6.6 Hz, 6H); LCMS (Method 2): m/z = 461.2 [M + H]$^+$ | 26 | 2.24 |
| 11 | 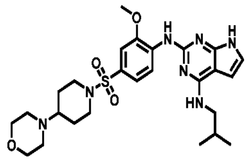 | *N*4-isobutyl-*N*2-(2-methoxy-4-((4-morpholinopiperidin-1-yl)sulfonyl)phenyl )-7*H*-pyrrolo[2,3-*d*]pyrimidine-2,4-diamine | $^1H$ NMR (400 MHz, DMSO-$d_6$): δ = 11.14 (s, 1H), 8.89 (d, *J* = 8.4 Hz, 1H), 7.53 - 7.45 (m, 2H), 7.27 (dd, *J* = 2.0, 8.4 Hz, 1H), 7.17 (d, *J* = 2.0 Hz, 1H), 6.88 - 6.82 (m, 1H), 6.50 (m, 1H), 3.98 (s, 3H), 3.63 (d, *J* = 11.6 Hz, 2H), 3.53 - 3.47 (m, 4H), 3.29 - 3.25 (m, 2H), 2.40 - 2.34 (m, 4H), 2.25 ( t, *J* = 10.8 Hz, 2H), 2.17 - 2.05 (m, 1H), 1.97 (td, *J* = 6.8, 13.4 Hz, 1H), 1.78 (d, *J* = 10.8 Hz, 2H), 1.47 - 1.32 (m, 2H), 0.95 (d, *J* = 6.8 Hz, 6H); LCMS (Method 2): m/z = 544.3 [M + H]$^+$ | 24 | 2.87 |

(continued)

| Example | Structure | Compound name | $^1H$ NMR, MS | Yield (%) | HPLC r.t. (min), Purity |
|---|---|---|---|---|---|
| 12 | 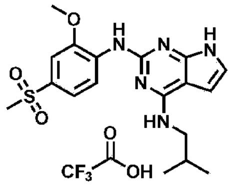 | *N*4-isobutyl-*N*2-(2-methoxy-4-(methylsulfonyl)p henyl)-7*H*-pyrrolo[2,3-*d*]pyrimidine-2,4-diamine 2,2,2-trifluoroacetate | $^1H$ NMR (400 MHz, TFA salt, DMSO-$d_6$): δ = 12.14 - 11.65 (m, 1H), 8.77 (d, *J* = 8.3 Hz, 1H), 8.73 - 8.50 (m, 1H), 7.56 - 7.46 (m, 2H), 7.05 - 6.96 (m, 1H), 6.67 (br s, 1H), 4.04 (s, 3H), 3.33 (t, *J* = 6.4 Hz, 2H), 3.22 (s, 3H), 2.09 - 1.91 (m, 1H), 0.99 (d, *J* = 6.6 Hz, 6H); LCMS (Method 2): m/z = 390.2 [M + H]+ | 5 | 2.55 |
| 13 | 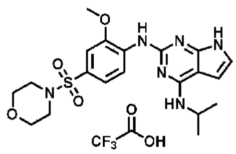 | *N*4-isopropyl-*N*2-(2-methoxy-4-(morpholinosulfon yl) phenyl)-7*H*-pyrrolo[2,3-*d*] pyrimidine-2,4-diamine 2,2,2-trifluoroacetate | $^1H$ NMR (400 MHz, TFA salt, DMSO-$d_6$): δ = 12.16 - 11.59 (m, 1H), 8.78 (d, *J* = 8.4 Hz, 1H), 8.71 - 8.12 (m, 1H), 7.38 (dd, *J* = 2.0, 8.4 Hz, 1H), 7.27 (d, *J* = 2.0 Hz, 1H), 7.03 (br s, 1H), 6.73 - 6.64 (m, 1H), 4.37 - 4.26 (m, 1H), 4.04 (s, 3H), 3.65 (br d, *J* = 4.4 Hz, 4H), 2.95 - 2.89 (m, 4H), 2.85 - 2.76 (m, 1H), 1.31 (d, *J* = 6.4 Hz, 6H); LCMS (Method 2): m/z = 467.2 [M + H]+ | 9 | 2.25 |
| 14 | 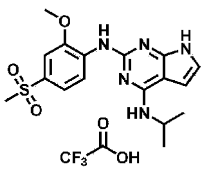 | *N*4-isopropyl-*N*2-(2-methoxy-4-(methylsulfonyl)p henyl)-7*H*-pyrrolo[2,3-*d*]pyrimidine-2,4-diamine 2,2,2-trifluoroacetate | $^1H$ NMR (400 MHz, TFA salt, DMSO-$d_6$): δ = 12.11 - 11.74 (m, 1H), 9.11 - 8.77 (m, 1H), 8.74 (d, *J* = 8.6 Hz, 1H), 8.69 - 8.26 (m, 1H), 7.58 - 7.48 (m, 2H), 7.03 (s, 1H), 6.69 (s, 1H), 4.38 - 4.22 (m, 1H), 4.04 (s, 3H), 3.22 (s, 3H), 1.31 (d, *J* = 6.4 Hz, 6H); LCMS (Method 2): m/z = 376.1 [M + H]+ | 7 | 2.43 |

(continued)

| Example | Structure | Compound name | $^1$H NMR, MS | Yield (%) | HPLC r.t. (min), Purity |
|---|---|---|---|---|---|
| 15 | 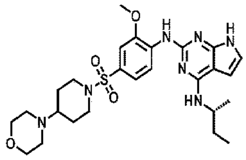 | (*R*)-*N*$^4$-(*sec*-butyl)-*N*$^2$-(2-methoxy-4-((4-morpholinopiperid in-1-yl) sulfonyl)phenyl )-7*H*-pyrrolo [2,3-*d*]pyrimidine-2,4-diamine | $^1$H NMR (400 MHz, DMSO-$d_6$): δ = 11.14 (s, 1H), 8.89 (d, *J* = 8.6 Hz, 1H), 7.49 (s, 1H), 7.30 (m, 1H), 7.21 - 7.14 (m, 2H), 6.92 - 6.83 (m, 1H), 6.59 - 6.39 (m, 1H), 4.26 - 4.15 (m, 1H), 3.99 (s, 3H), 3.65 (d, *J* = 11.6 Hz, 2H), 3.57 - 3.46 (m, 4H), 2.41 - 2.35 (m, 4H), 2.31 - 2.22 (m, 2H), 2.15 - 2.06 (m, 1H), 1.80 (d, *J* = 11.2 Hz, 2H), 1.70 - 1.51 (m, 2H), 1.49 - 1.32 (m, 2H), 1.22 (d, *J* = 6.8 Hz, 3H), 0.93 (t, *J* = 7.2 Hz, 3H); LCMS (Method 2): m/z = 544.3 [M + H]$^+$ | 21 | 2.93 |
| 16 | 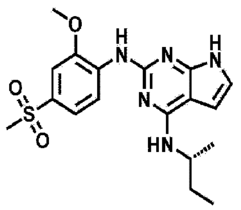 | (*R*)-*N*$^4$-(*sec*-butyl)-*N*$^2$-(2-methoxy-4-(4-methylsulfonyl) ph enyl)-7*H*-pyrrolo[2,3-*d*] pyrimidine-2,4-diamine | $^1$H NMR (400 MHz, DMSO-$d_6$): δ = 11.17 (s, 1H), 8.91 (d, *J* = 8.4 Hz, 1H), 7.51 (s, 1H), 7.48 (m, 1H), 7.42 (d, *J* = 1.6 Hz, 1H), 7.17 (d, *J* = 8.4 Hz, 1H), 6.86 (m, 1H), 6.56 - 6.51 (m, 1H), 4.27 - 4.15 (m, 1H), 4.01 (s, 3H), 3.18 (s, 3H), 1.73 - 1.48 (m, 2H), 1.22 (d, *J* = 6.4 Hz, 3H), 0.94 (t, *J* = 7.2 Hz, 3H); LCMS (Method 2): m/z = 390.2 [M + H]$^+$ | 32 | 2.83 |

(continued)

| Example | Structure | Compound name | $^1$H NMR, MS | Yield (%) | HPLC r.t. (min), Purity |
|---|---|---|---|---|---|
| 17 | 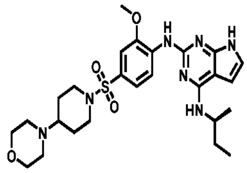 | (*S*)-$N^4$-(*sec*-butyl)-$N^2$-(2-methoxy-4-((4-morpholinopiperid in-1-yl) sulfonyl)phenyl )-7*H*-pyrrolo [2,3-*d*]pyrimidine-2,4-diamine | $^1$H NMR (400 MHz, DMSO-$d_6$): δ = 11.14 (s, 1H), 9.17 - 8.57 (m, 1H), 7.52 - 7.44 (m, 1H), 7.33 - 7.26 (m, 1H), 7.22 - 7.11 (m, 2H), 6.90 - 6.82 (m, 1H), 6.56 - 6.49 (m, 1H), 4.27 - 4.12 (m, 1H), 4.03 - 3.94 (m, 3H), 3.72 - 3.59 (m, 2H), 3.55 - 3.48 (m, 4H), 2.41 - 2.35 (m, 4H), 2.31 - 2.21 (m, 2H), 2.16 - 2.06 (m, 1H), 1.84 - 1.75 (m, 2H), 1.70 - 1.50 (m, 2H), 1.47 - 1.34 (m, 2H), 1.26 - 1.18 (m, 3H), 0.97 - 0.88 (m, 3H); LCMS (Method 2): m/z = 544.3 [M + H]$^+$ | 9 | 2.93 |
| 18 | 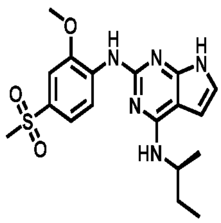 | (*S*)-$N^4$-(sec-butyl)-$N^2$-(2-methoxy-4-(4-methylsulfonyl) ph enyl)-7H-pyrrolo[2,3-*d*] pyrimidine-2,4-diamine | $^1$H NMR (400 MHz, DMSO-$d_6$): δ = 11.16 (s, 1H), 8.90 (d, *J* = 8.6 Hz, 1H), 7.50 (s, 1H), 7.47 (m, 1H), 7.41 (d, *J* = 2.0 Hz, 1H), 7.17 (d, *J* = 8.4 Hz, 1H), 6.86 (m, 1H), 6.53 (m, 1H), 4.26 - 4.14 (m, 1H), 4.01 (s, 3H), 3.18 (s, 3H), 1.70 - 1.51 (m, 2H), 1.22 (d, *J* = 6.6 Hz, 3H), 0.93 (t, *J* = 7.4 Hz, 3H); LCMS (Method 2): m/z = 390.2 [M + H]$^+$ | 45 | 2.82 |

(continued)

| Example | Structure | Compound name | $^1$H NMR, MS | Yield (%) | HPLC r.t. (min), Purity |
|---|---|---|---|---|---|
| 19 | 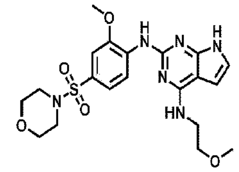 | *N*2-(2-methoxy-4-(morpholinosulfon yl) phenyl)-*N*4-(2-methoxyethyl)-7*H*-pyrrolo[2,3-*d*]pyrimidine-2,4-diamine | $^1$H NMR (400 MHz, DMSO-$d_6$): δ = 11.21 (s, 1H), 8.91 (d, *J* = 8.5 Hz, 1H), 7.56 (s, 2H), 7.31 (dd, *J* = 1.9, 8.6 Hz, 1H), 7.17 (d, *J* = 2.0 Hz, 1H), 6.88 (dd, *J* = 2.3, 3.3 Hz, 1H), 6.50 (dd, *J* = 2.0, 3.4 Hz, 1H), 4.00 (s, 3H), 3.67 - 3.59 (m, 6H), 3.58 - 3.54 (m, 2H), 3.29 (s, 3H), 2.91 - 2.83 (m, 4H); LCMS (Method 2): m/z = 463.2 [M + H]$^+$ | 19 | 2.70 |
| 20 | 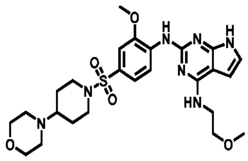 | *N*2-(2-methoxy-4-((4-morpholinopiperid in-1-yl) sulfonyl)phenyl )-*N*4-(2-methoxyethyl)-7*H*-pyrrolo [2,3-*d*]pyrimidine-2,4-diamine 2,2,2-trifluoroacetate | $^1$H NMR (400 MHz, TFA salt, DMSO-$d_6$): δ = 11.74 - 11.45 (m, 1H), 10.05 - 9.78 (m, 1H), 8.82 (d, *J* = 8.7 Hz, 1H), 8.33 - 8.04 (m, 1H), 7.43 - 7.34 (m, 1H), 7.30 - 7.22 (m, 1H), 6.98 (br s, 1H), 6.64 - 6.53 (m, 1H), 4.02 (s, 3H), 3.97 (br s, 1H), 3.81 (br d, *J* = 11.2 Hz, 2H), 3.62 - 3.57 (m, 7H), 3.42 - 3.35 (m, 2H), 3.33 - 3.29 (m, 3H), 3.26 - 3.17 (m, 1H), 3.12 - 2.97 (m, 2H), 2.27 (br t, *J* = 12.0 Hz, 2H), 2.13 (br d, *J* = 10.6 Hz, 2H), 1.77 - 1.61 (m, 2H); LCMS (Method 2): m/z = 546.2 [M + H]$^+$ | 46 | 2.09 |

(continued)

| Example | Structure | Compound name | $^1H$ NMR, MS | Yield (%) | HPLC r.t. (min), Purity |
|---|---|---|---|---|---|
| 21 | 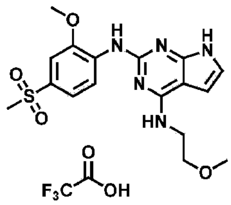 | *N*2-(2-methoxy-4-(methylsulfonyl)phenyl)-*N*4-(2-methoxyethyl)-7*H*-pyrrolo[2,3-*d*]pyrimidine-2,4-diamine 2,2,2-trifluoroacetate | $^1H$ NMR (400 MHz, TFA salt, DMSO-$d_6$): δ = 11.97 - 11.72 (m, 1H), 8.75 (d, *J* = 8.4 Hz, 1H), 8.68 - 8.34 (m, 1H), 7.53 (dd, *J* = 1.9, 8.5 Hz, 1H), 7.50 (d, *J* = 1.6 Hz, 1H), 7.02 (br s, 1H), 6.66 (br s, 1H), 4.04 (s, 3H), 3.70 - 3.66 (m, 2H), 3.62 - 3.59 (m, 2H), 3.31 (s, 3H), 3.21 (s, 3H); LCMS (Method 2): m/z = 392.1 $[M + H]^+$ | 44 | 2.21 |
| 22 | 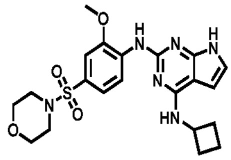 | *N*4-cyclobutyl-*N*2-(2-methoxy-4-(morpholinosulfon yl) phenyl)-7*H*-pyrrolo[2,3-*d*] pyrimidine-2,4-diamine | $^1H$ NMR (400 MHz, DMSO-$d_6$): δ = 11.18 (s, 1H), 8.93 (d, *J* = 8.6 Hz, 1H), 7.62 (d, *J* = 7.4 Hz, 1H), 7.52 (s, 1H), 7.33 (m, 1H), 7.17 (d, *J* = 2.0 Hz, 1H), 6.87 (m, 1H), 6.50 (m, 1H), 4.70 - 4.57 (m, 1H), 4.00 (s, 3H), 3.66 - 3.61 (m, 4H), 2.91 - 2.84 (m, 4H), 2.39 - 2.29 (m, 2H), 2.05 (m, 2H), 1.79 - 1.69 (m, 2H); LCMS (Method 2): m/z = 459.2 $[M + H]^+$ | 31 | 2.88 |

(continued)

| Example | Structure | Compound name | $^1$H NMR, MS | Yield (%) | HPLC r.t. (min), Purity |
|---|---|---|---|---|---|
| 23 | 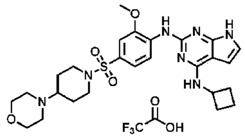 | *N*4-cyclobutyl-*N*2-(2-methoxy-4-((4-morpholinopiperid in-1-yl)sulfonyl)phenyl )-7*H*-pyrrolo[2,3-*d*]pyrimidine-2,4-diamine 2,2,2-trifluoroacetate | $^1$H NMR (400 MHz, TFA salt, DMSO-$d_6$): δ = 11.17 (s, 1H), 8.89 (d, *J* = 8.6 Hz, 1H), 7.61 (d, *J* = 7.2 Hz, 1H), 7.49 (s, 1H), 7.31 (m, 1H), 7.17 (d, *J* = 2.0 Hz, 1H), 6.86 (d, *J* = 3.2 Hz, 1H), 6.49 (d, *J* = 3.2 Hz, 1H), 4.69 - 4.56 (m, 1H), 3.98 (s, 3H), 3.64 (d, *J* = 11.8 Hz, 2H), 3.53 - 3.48 (m, 4H), 2.40 - 2.36 (m, 4H), 2.35 - 2.30 (m, 2H), 2.29 - 2.21 (m, 2H), 2.15 - 2.08 (m, 1H), 2.07 - 1.99 (m, 2H), 1.82 - 1.68 (m, 4H), 1.47 - 1.33 (m, 2H); LCMS (Method 2): m/z = 542.3 [M + H]$^+$ | 12.2 | 2.83 |
| 24 | 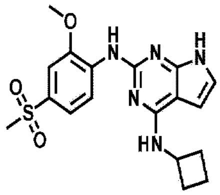 | *N*4-cyclobutyl-*N*2-(2-methoxy-4-(methylsulfonyl)p henyl)-7*H*-pyrrolo[2,3-*d*]pyrimidine-2,4-diamine | $^1$H NMR (400 MHz, DMSO-$d_6$): δ = 11.21 (s, 1H), 8.92 (d, *J* = 8.6 Hz, 1H), 7.63 (m, 1H), 7.54 - 7.48 (m, 2H), 7.42 (d, *J* = 2.0 Hz, 1H), 6.90 - 6.86 (m, 1H), 6.51 (m, 1H), 4.71 - 4.60 (m, 1H), 4.02 (s, 3H), 3.21 - 3.16 (m, 3H), 2.40 - 2.31 (m, 2H), 2.06 (m, 2H), 1.81 - 1.70 (m, 2H); LCMS (Method 2): m/z = 388.1 [M + H]$^+$ | 13 | 2.80 |

(continued)

| Example | Structure | Compound name | $^1$H NMR, MS | Yield (%) | HPLC r.t. (min), Purity |
|---|---|---|---|---|---|
| 25 | 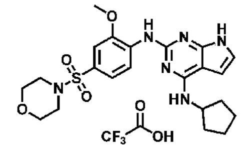 | *N*4-cyclopentyl-*N*2-(2-methoxy-4-(morpholinosulfonyl)phenyl)-7*H*-pyrrolo[2,3-*d*] pyrimidine-2,4-diamine 2,2,2-trifluoroacetate | $^1$H NMR (400 MHz, TFA salt, DMSO-$d_6$): δ = 12.29 - 11.70 (m, 1H), 8.87 - 8.21 (m, 3H), 7.38 (dd, *J* = 1.9, 8.6 Hz, 1H), 7.26 (d, *J* = 1.8 Hz, 1H), 7.03 (s, 1H), 6.71 (s, 1H), 4.39 (d, *J* = 5.8 Hz, 1H), 4.03 (s, 3H), 3.66 - 3.63 (m, 4H), 2.93 - 2.87 (m, 4H), 2.15 - 1.98 (m, 2H), 1.84 - 1.72 (m, 2H), 1.70 - 1.59 (m, 4H); LCMS (Method 2): m/z = 473.2 [M + H]+ | 22 | 2.71 |
| 26 | 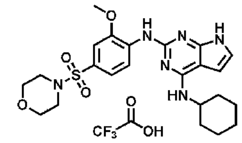 | *N*4-cyclohexyl-*N*2-(2-methoxy-4-(morpholinosulfonyl)phenyl)-7*H*-pyrrolo[2,3-*d*] pyrimidine-2,4-diamine 2,2,2-trifluoroacetate | $^1$H NMR (400 MHz, TFA salt, DMSO-$d_6$): δ = 12.00 - 11.74 (m, 1H), 8.80 - 8.71 (m, 1H), 8.46 - 8.09 (m, 1H), 7.35 (dd, *J* = 1.7, 8.7 Hz, 1H), 7.27 (d, *J* = 1.7 Hz, 1H), 7.03 (br s, 1H), 6.75 - 6.66 (m, 1H), 4.03 (s, 3H), 3.95 (br s, 1H), 3.66 - 3.63 (m, 4H), 2.95 - 2.86 (m, 4H), 2.07 - 1.95 (m, 2H), 1.89 - 1.75 (m, 2H), 1.69 (br d, *J* = 12.7 Hz, 1H), 1.46 - 1.30 (m, 4H), 1.27 - 1.08 (m, 1H); LCMS (Method 2): m/z = 487.2 [M + H]+ | 11 | 2.41 |

(continued)

| Example | Structure | Compound name | $^1$H NMR, MS | Yield (%) | HPLC r.t. (min), Purity |
|---|---|---|---|---|---|
| 27 | 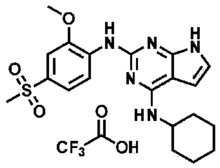 | *N*4-cyclohexyl-*N*2-(2-methoxy-4-(methylsulfonyl)phenyl)-7*H*-pyrrolo[2,3-*d*]pyrimidine-2,4-diamine 2,2,2-trifluoroacetate | $^1$H NMR (400 MHz, TFA salt, DMSO-$d_6$): δ = 11.94 - 11.69 (m, 1H), 8.73 (d, *J* = 9.2 Hz, 1H), 8.68 - 8.45 (m, 1H), 8.45 - 8.17 (m, 1H), 7.54 - 7.48 (m, 2H), 7.03 - 6.95 (m, 1H), 6.67 (br s, 1H), 4.02 (s, 3H), 3.98 - 3.92 (m, 1H), 3.21 (s, 3H), 2.01 (br d, *J* = 8.2 Hz, 2H), 1.89 - 1.63 (m, 3H), 1.47 - 1.29 (m, 4H), 1.26 - 1.13 (m, 1H); LCMS (Method 2): m/z = 416.2 [M + H]$^+$ | 49 | 2.23 |
| 28 | 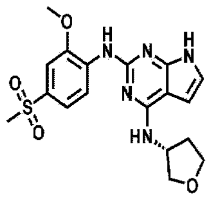 | (*R*)-*N*2-(2-methoxy-4-(methylsulfonyl)phenyl)-*N*4-(tetrahydrofuran-3-yl)-7*H*-pyrrolo[2,3-*d*]pyrimidine-2,4-diamine | $^1$H NMR (400 MHz, DMSO-$d_6$): δ = 11.53 - 10.99 (m, 1H), 9.09 - 8.70 (m, 1H), 7.59 - 7.54 (m, 2H), 7.53 - 7.47 (m, 1H), 7.43 - 7.39 (m, 1H), 6.91 - 6.85 (m, 1H), 6.55 (dd, *J* = 2.0, 3.3 Hz, 1H), 4.78 - 4.62 (m, 1H), 4.01 (s, 3H), 3.99 - 3.95 (m, 1H), 3.94 - 3.87 (m, 1H), 3.81 - 3.73 (m, 1H), 3.68 - 3.63 (m, 1H), 3.18 (s, 3H), 2.31 - 2.20 (m, 1H), 2.04 - 1.90 (m, 1H); LCMS (Method 2): m/z = 404.1 [M + H]$^+$ | 40 | 2.51 |

(continued)

| Example | Structure | Compound name | $^1H$ NMR, MS | Yield (%) | HPLC r.t. (min), Purity |
|---|---|---|---|---|---|
| 29 | 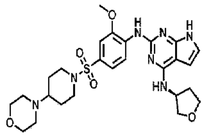 | (*S*)-*N*$^{2}$-(2-methoxy-4-((4-morpholinopiperid in-1-yl) sulfonyl) phenyl )-*N*$^{4}$-(tetrahydrofuran-3-yl)-7*H*-pyrrolo[2,3-*d*] pyrimidine-2,4-diamine | $^1H$ NMR (400 MHz, DMSO-$d_6$): δ = 11.46 - 10.88 (m, 1H), 9.11 - 8.70 (m, 1H), 7.61 - 7.48 (m, 2H), 7.32 (m, 1H), 7.18 (d, *J* = 2.0 Hz, 1H), 6.88 (m, 1H), 6.54 (m, 1H), 4.69 (m, 1H), 4.02 - 3.94 (m, 4H), 3.94 - 3.87 (m, 1H), 3.77 (m, 1H), 3.65 (m, 3H), 3.55 - 3.47 (m, 4H), 2.42 - 2.35 (m, 4H), 2.31 - 2.22 (m, 3H), 2.16 - 2.06 (m, 1H), 2.01 - 1.92 (m, 1H), 1.80 (d, *J* = 11.6 Hz, 2H), 1.48 - 1.35 (m, 2H); LCMS (Method 2): m/z = 558.2 [M + H]$^+$ | 53 | 2.64 |
| 30 | 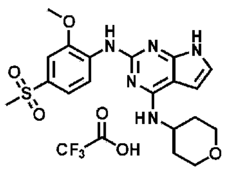 | *N*$^{2}$-(2-methoxy-4-(methylsulfonyl)p henyl)-*N*$^{4}$-(tetrahydro-2H-pyran-4-yl)-7*H*-pyrrolo[2,3-*d*] pyrimidine-2,4-diamine 2,2,2-trifluoroacetate | $^1H$ NMR (400 MHz, TFA salt, DMSO-$d_6$): δ = 11.93 - 11.50 (m, 1H), 8.73 (br t, *J* = 7.5 Hz, 1H), 8.64 - 8.40 (m, 1H), 8.39 - 8.06 (m, 1H), 7.59 - 7.52 (m, 1H), 7.50 (s, 1H), 7.03 - 6.94 (m, 1H), 6.69 - 6.59 (m, 1H), 4.28 - 4.19 (m, 1H), 4.03 (s, 3H), 3.96 (br dd, *J* = 2.4, 11.6 Hz, 2H), 3.44 - 3.40 (m, 2H), 3.22 (s, 3H), 1.95 (m, 2H), 1.63 (m, 2H); LCMS (Method 2): m/z = 418.2 [M + H]$^+$ | 13 | 2.27 |

(continued)

| Example | Structure | Compound name | $^1H$ NMR, MS | Yield (%) | HPLC r.t. (min), Purity |
|---|---|---|---|---|---|
| 31 | 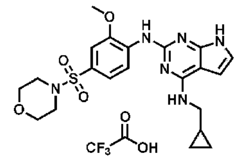 | N$^4$-(cyclopropylmethy l)-*N*$^2$-(2-methoxy-4-(morpholinosulfon yl) phenyl)-7*H*-pyrrolo[2,3-*d*] pyrimidine-2,4-diamine 2,2,2-trifluoroacetate | $^1H$ NMR (400 MHz, TFA salt, DMSO-$d_6$): δ = 12.01 - 11.66 (m, 1H), 9.02 - 8.14 (m, 3H), 7.37 (dd, *J* = 1.8, 8.6 Hz, 1H), 7.26 (d, *J* = 1.6 Hz, 1H), 7.07 - 6.93 (m, 1H), 6.69 (br s, 1H), 4.03 (s, 3H), 3.66 - 3.61 (m, 4H), 3.39 (br t, *J* = 5.9 Hz, 2H), 2.94 - 2.83 (m, 4H), 1.26 - 1.09 (m, 1H), 0.62 - 0.45 (m, 2H), 0.33 (q, *J* = 4.7 Hz, 2H); LCMS (Method 2): m/z = 459.2 [M + H]$^+$ | 23 | 2.62 |
| 32 | 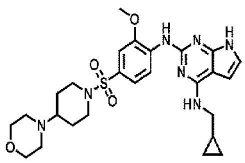 | *N*$^4$-(cyclopropylmethy l)-*N*$^2$-(2-methoxy-4-((4-morpholinopiperid in-1-yl) sulfonyl)phenyl )-7*H*-pyrrolo [2,3-*d*]pyrimidine-2,4-diamine | $^1H$ NMR (400 MHz, DMSO-$d_6$): δ = 11.18 (s, 1H), 8.90 (d, *J* = 8.4 Hz, 1H), 7.56 (t, *J* = 5.4 Hz, 1H), 7.51 (s, 1H), 7.31 (m, 1H), 7.18 (d, *J* = 1.8 Hz, 1H), 6.90 - 6.86 (m, 1H), 6.52 (m, 1H), 3.99 (s, 3H), 3.70 - 3.61 (m, 2H), 3.54 - 3.49 (m, 4H), 3.39 - 3.35 (m, 2H), 2.42 - 2.36 (m, 4H), 2.31 - 2.22 (m, 2H), 2.17 - 2.07 (m, 1H), 1.85 - 1.76 (m, 2H), 1.49 - 1.35 (m, 2H), 1.20 - 1.09 (m, 1H), 0.52 - 0.45 (m, 2H), 0.32 - 0.27 (m, 2H); LCMS (Method 2): m/z = 542.3 [M + H]+ | 11 | 2.79 |

(continued)

| Example | Structure | Compound name | $^1$H NMR, MS | Yield (%) | HPLC r.t. (min), Purity |
|---|---|---|---|---|---|
| 33 | 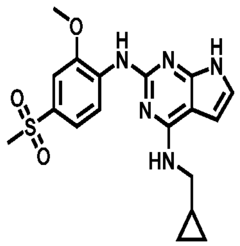 | *N*$^4$-(cyclopropylmethy l)-*N*$^2$-(2-methoxy-4-(methylsulfonyl)p henyl)-7*H*-pyrrolo[2,3-*d*]pyrimidine-2,4-diamine | $^1$H NMR (400 MHz, DMSO-$d_6$): δ = 11.19 (s, 1H), 8.90 (d, *J* = 8.5 Hz, 1H), 7.55 (t, *J* = 5.3 Hz, 1H), 7.51 (s, 1H), 7.47 (m, 1H), 7.40 (d, *J* = 1.9 Hz, 1H), 6.88 - 6.84 (m, 1H), 6.50 (m, 1H), 4.00 (s, 3H), 3.36 - 3.33 (m, 2H), 3.17 (s, 3H), 1.22 - 1.06 (m, 1H), 0.51 - 0.43 (m, 2H), 0.32 - 0.22 (m, 2H); LCMS (Method 2): m/z = 388.1 [M + H]$^+$ | 14 | 2.76 |
| 34 | 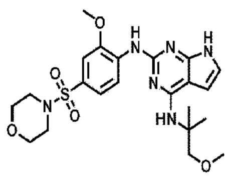 | *N*$^4$-(1-methoxy-2-methylpropan-2-yl)-*N*$^2$-(2-methoxy-4-(morpholinosulfon yl)phenyl)-7*H*-pyrrolo[2,3-*d*] pyrimidine-2,4-diamine | $^1$H NMR (400 MHz, DMSO-$d_6$): δ = 11.18 (br s, 1H), 8.83 (d, *J* = 8.6 Hz, 1H), 7.52 (s, 1H), 7.30 (dd, *J* = 1.7, 8.6 Hz, 1H), 7.19 (d, *J* = 1.7 Hz, 1H), 6.90 - 6.84 (m, 1H), 6.62 (dd, *J* = 1.9, 3.2 Hz, 1H), 6.58 (s, 1H), 4.00 (s, 3H), 3.70 (s, 2H), 3.63 (br s, 4H), 3.27 (s, 3H), 2.88 (br s, 4H), 1.46 (s, 6H); LCMS (Method 2): m/z = 491.2 [M + H]$^+$ | 30 | 2.92 |

(continued)

| Example | Structure | Compound name | $^1H$ NMR, MS | Yield (%) | HPLC r.t. (min), Purity |
|---|---|---|---|---|---|
| 35 | 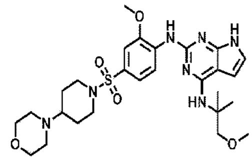 | *N*4-(1-methoxy-2-methylpropan-2-yl)-*N*2-(2-methoxy-4-((morpholinopiperidin-1-yl)sulfonyl)phenyl )-7*H*-pyrrolo[2,3-*d*]pyrimidine-2,4-diamine | $^1H$ NMR (400 MHz, DMSO-$d_6$): δ = 11.18 (s, 1H), 8.81 (d, *J* = 8.6 Hz, 1H), 7.49 (s, 1H), 7.29 (dd, *J* = 2.0, 8.6 Hz, 1H), 7.18 (d, *J* = 2.0 Hz, 1H), 6.86 (dd, *J* = 2.3, 3.4 Hz, 1H), 6.62 (dd, *J* = 2.0, 3.4 Hz, 1H), 6.59 (s, 1H), 3.99 (s, 3H), 3.70 (s, 2H), 3.64 (br d, *J* = 11.7 Hz, 2H), 3.54 - 3.47 (m, 4H), 3.26 (s, 3H), 2.41 - 2.35 (m, 4H), 2.29 - 2.20 (m, 2H), 2.14 - 2.04 (m, 1H), 1.79 (d, *J* = 10.8 Hz, 2H), 1.46 (s, 6H), 1.41 (m, 2H); LCMS (Method 2): m/z = 574.3 [M + H]+ | 41 | 2.87 |
| 36 | 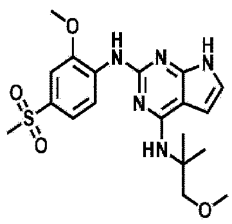 | *N*4-(1-methoxy-2-methylpropan-2-yl)-*N*2-(2-methoxy-4-(methylsulfonyl)phenyl)-7*H*-pyrrolo[2,3-*d*] pyrimidine-2,4-diamine | $^1H$ NMR (400 MHz, DMSO-$d_6$): δ = 11.19 (br s, 1H), 8.83 (d, *J* = 8.6 Hz, 1H), 7.51 (s, 1H), 7.47 (dd, *J* = 1.9, 8.5 Hz, 1H), 7.42 (d, *J* = 2.0 Hz, 1H), 6.86 (dd, *J* = 2.3, 3.4 Hz, 1H), 6.62 (dd, *J* = 2.0, 3.3 Hz, 1H), 6.58 (s, 1H), 4.01 (s, 3H), 3.70 (s, 2H), 3.27 (s, 3H), 3.18 (s, 3H), 1.47 (s, 6H); LCMS (Method 2): m/z = 420.2 [M + H]+ | 33 | 2.86 |

(continued)

| Example | Structure | Compound name | $^1$H NMR, MS | Yield (%) | HPLC r.t. (min), Purity |
|---|---|---|---|---|---|
| 37 | 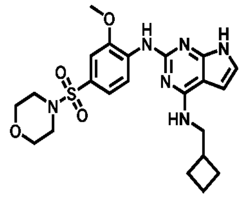 | *N*4-(cyclobutylmethyl) -*N*2-(2-methoxy-4-(morpholinosulfonyl)phenyl)-7*H*-pyrrolo[2,3-*d*] pyrimidine-2,4-diamine | $^1$H NMR (400 MHz, TFA salt, DMSO-$d_6$): δ = 12.12 - 11.56 (m, 1H), 8.78 (d, *J* = 8.7 Hz, 1H), 8.74 - 8.50 (m, 1H), 7.36 (m, 1H), 7.27 (d, *J* = 2.0 Hz, 1H), 7.03 (br s, 1H), 6.69 - 6.62 (m, 1H), 4.04 (s, 3H), 3.65 (br d, *J* = 4.4 Hz, 4H), 3.54 (br s, 2H), 2.93 - 2.88 (m, 4H), 2.75 - 2.63 (m, 1H), 2.08 (m, 2H), 1.94 - 1.84 (m, 2H), 1.84 - 1.74 (m, 2H); LCMS (Method 2): m/z = 473.2 [M + H]$^+$ | 16 | 2.33 |
| 38 | 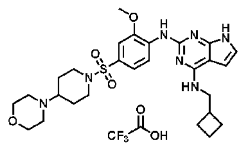 | *N*4-(cyclobutylmethyl) -*N*2-(2-methoxy-4-((4-morpholinopiperid in-1-yl) sulfonyl)phenyl )-7*H*-pyrrolo [2,3-*d*]pyrimidine-2,4-diamine 2,2,2-trifluoroacetate | $^1$H NMR (400 MHz, TFA salt, DMSO-$d_6$): δ = 11.83 - 11.49 (m, 1H), 10.26 - 9.93 (m, 1H), 8.80 (d, *J* = 8.4 Hz, 1H), 8.62 - 8.14 (m, 1H), 7.40 - 7.33 (m, 1H), 7.26 (d, *J* = 1.6 Hz, 1H), 6.98 (br s, 1H), 6.66 - 6.56 (m, 1H), 4.01 (s, 3H), 3.97 (br d, *J* = 8.4 Hz, 2H), 3.82 (br s, 2H), 3.66 (br dd, *J* = 4.4, 6.8 Hz, 2H), 3.55 - 3.51 (m, 2H), 3.42 - 3.33 (m, 2H), 3.25 - 3.15 (m, 1H), 3.09 - 2.96 (m, 2H), 2.66 (td, *J* = 7.4, 14.8 Hz, 1H), 2.24 (t, *J* = 11.5 Hz, 2H), 2.16 - 2.00 (m, 4H), 1.94 - 1.83 (m, 2H), 1.82 - 1.58 (m, 4H); LCMS (Method 2): m/z = 556.3 [M + H]$^+$ | 6 | 1.99 |

(continued)

| Example | Structure | Compound name | $^1$H NMR, MS | Yield (%) | HPLC r.t. (min), Purity |
|---|---|---|---|---|---|
| 39 | 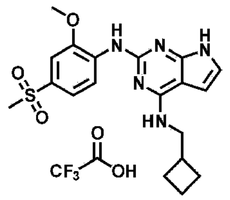 | *N*4-(cyclobutylmethyl) -*N*2-(2-methoxy-4-(methylsulfonyl)phenyl)-7*H*-pyrrolo[2,3-*d*] pyrimidine-2,4-diamine 2,2,2-trifluoroacetate | $^1$H NMR (400 MHz, TFA salt, DMSO-$d_6$): δ = 12.02 - 11.68 (m, 1H), 8.75 (d, *J* = 8.3 Hz, 1H), 8.72 - 8.54 (m, 1H), 7.55 - 7.49 (m, 2H), 7.02 (br s, 1H), 6.66 (br s, 1H), 4.04 (s, 3H), 3.54 (br d, *J* = 6.4 Hz, 2H), 3.21 (s, 3H), 2.69 (td, *J* = 7.5, 14.9 Hz, 1H), 2.13 - 2.02 (m, 2H), 1.94 - 1.84 (m, 2H), 1.83 - 1.73 (m, 2H); LCMS (Method 2): m/z = 402.2 [M + H]$^+$ | 17 | 2.17 |
| 40 | 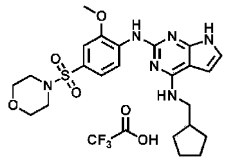 | *N*4-(cyclopentylmethyl)-*N*2-(2-methoxy-4-(morpholinosulfon yl) phenyl)-7*H*-pyrrolo[2,3-*d*] pyrimidine-2,4-diamine 2,2,2-trifluoroacetate | $^1$H NMR (400 MHz, TFA salt, DMSO-$d_6$): δ = 11.93 - 11.59 (m, 1H), 8.77 (d, *J* = 8.6 Hz, 1H), 8.71 - 8.32 (m, 1H), 7.34 (dd, *J* = 1.8, 8.6 Hz, 1H), 7.25 (d, *J* = 1.7 Hz, 1H), 7.01 (br s, 1H), 6.70 - 6.58 (m, 1H), 4.09 - 3.96 (m, 3H), 3.65 - 3.62 (m, 4H), 3.42 (br d, *J* = 6.1 Hz, 2H), 2.95 - 2.86 (m, 4H), 2.34 - 2.22 (m, 1H), 1.82 - 1.70 (m, 2H), 1.66 - 1.43 (m, 4H), 1.37 - 1.23 (m, 2H); LCMS (Method 2): m/z = 487.2 [M + H]$^+$ | 13 | 2.39 |

(continued)

| Example | Structure | Compound name | $^1$H NMR, MS | Yield (%) | HPLC r.t. (min), Purity |
|---|---|---|---|---|---|
| 41 | 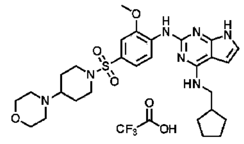 | *N*4-(cyclopentylmethyl)-N2-(2-methoxy-4-((4-morpholinopiperid in-1-yl) sulfonyl)phenyl )-7*H*-pyrrolo [2,3-*d*]pyrimidine-2,4-diamine 2,2,2-trifluoroacetate | $^1$H NMR (400 MHz, TFA salt, DMSO-$d_6$): δ = 11.65 (br dd, *J* = 2.6, 7.9 Hz, 1H), 10.07 - 9.68 (m, 1H), 8.82 (d, *J* = 8.6 Hz, 1H), 8.49 - 8.19 (m, 1H), 7.37 - 7.33 (m, 1H), 7.28 - 7.24 (m, 1H), 6.99 (br s, 1H), 6.67 - 6.56 (m, 1H), 4.02 (s, 3H), 3.98 (br d, *J* = 6.6 Hz, 1H), 3.82 (br d, *J* = 12.6 Hz, 2H), 3.68 - 3.64 (m, 2H), 3.43 (br d, *J* = 6.7 Hz, 4H), 3.27 - 3.13 (m, 2H), 3.11 - 2.97 (m, 2H), 2.35 - 2.21 (m, 3H), 2.12 (br d, *J* = 11.2 Hz, 2H), 1.82 - 1.48 (m, 8H), 1.39 - 1.26 (m, 2H); LCMS (Method 2): m/z = 570.3 [M + H]$^+$ | 4.0 | 2.06 |
| 42 | 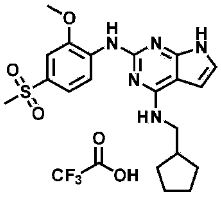 | *N*4-(cyclopentylmethyl)-*N*2-(2-methoxy-4-(methylsulfonyl)p henyl)-7*H*-pyrrolo[2,3-*d*]pyrimidine-2,4-diamine 2,2,2-trifluoroacetate | $^1$H NMR (400 MHz, TFA salt, DMSO-$d_6$): δ = 12.08 - 11.74 (m, 1H), 8.76 (d, *J* = 9.0 Hz, 1H), 8.72 - 8.59 (m, 1H), 7.56 - 7.48 (m, 2H), 7.03 (br s, 1H), 6.68 (br s, 1H), 4.04 (s, 3H), 3.48 - 3.39 (m, 2H), 3.22 (s, 3H), 2.37 - 2.20 (m, 1H), 1.85 - 1.73 (m, 2H), 1.68 - 1.47 (m, 4H), 1.38 - 1.25 (m, 2H); LCMS (Method 2): m/z = 416.2 [M + H]$^+$ | 41.9 | 2.25 |

(continued)

| Example | Structure | Compound name | $^1H$ NMR, MS | Yield (%) | HPLC r.t. (min), Purity |
|---|---|---|---|---|---|
| 43 | 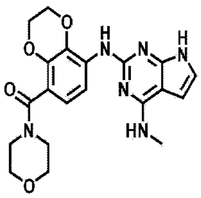 | (8-((4-(methylamino)-7*H*-pyrrolo[2,3-*d*]pyrimidin-2-yl)amino)-2,3-dihydrobenzo [*b*][1 ,4]dioxin-5-yl) (morpholino)me thanone | $^1H$ NMR (400 MHz, DMSO-$d_6$): δ = 11.12 (br s, 1H), 8.29 (d, *J* = 8.5 Hz, 1H), 7.34 (d, *J* = 3.9 Hz, 1H), 7.14 (s, 1H), 6.81 (m, 1H), 6.75 (d, *J* = 8.4 Hz, 1H), 6.40 (m, 1H), 4.43 - 4.35 (m, 2H), 4.34 - 4.28 (m, 2H), 3.60 (s, 4H), 3.54 (s, 2H), 3.29 - 3.20 (m, 2H), 2.95 (d, *J* = 4.8 Hz, 3H); LCMS (Method 2): m/z = 411.2 $[M + H]^+$ | 29.4 | 2.28 |
| 44 | 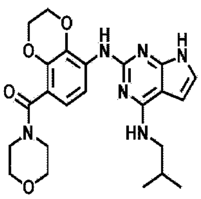 | (8-((4-(isobutylamino)-7*H*-pyrrolo[2,3-*d*]pyrimidin-2-yl)amino)-2,3-dihydrobenzo [b][1 ,4]dioxin-5-yl) (morpholino)me thanone | $^1H$ NMR (400 MHz, DMSO-$d_6$): δ = 11.08 (s, 1H), 8.28 (d, *J* = 8.4 Hz, 1H), 7.41 (t, *J* = 5.6 Hz, 1H), 7.10 (s, 1H), 6.83 - 6.79 (m, 1H), 6.73 (d, *J* = 8.5 Hz, 1H), 6.48 (m, 1H), 4.43 - 4.27 (m, 4H), 3.60 (s, 4H), 3.52 (d, *J* = 7.3 Hz, 2H), 3.26 (t, *J* = 6.3 Hz, 4H), 1.97 (td, *J* = 6.8, 13.2 Hz, 1H), 0.95 (d, *J* = 6.6 Hz, 6H); LCMS (Method 2): m/z = 453.2 $[M + H]^+$ | 40 | 2.63 |
| 45 | 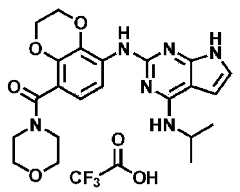 | (8-((4-(isopropylamino)-7*H*-pyrrolo[2,3-*d*]pyrimidin-2-yl)amino)-2,3-dihydrobenzo [*b*][1 ,4]dioxin-5-yl) (morpholino)me thanone 2,2,2-trifluoroacetate | $^1H$ NMR (400 MHz, TFA salt, DMSO-$d_6$): δ = 12.24 - 11.59 (m, 1H), 8.33 - 8.29 (m, 1H), 9.38 - 8.21 (m, 1H), 8.18 - 7.86 (m, 1H), 7.12 - 6.94 (m, 1H), 6.82 (d, *J* = 8.4 Hz, 1H), 6.69 (s, 1H), 4.41 (d, *J* = 2.5 Hz, 2H), 4.36 (d, *J* = 3.3 Hz, 2H), 4.27 - 4.17 (m, 1H), 3.62 (s, 4H), 3.30 - 3.17 (m, 4H), 1.30 (d, *J* = 6.4 Hz, 6H); LCMS (Method 2): m/z = 439.2 $[M + H]^+$ | 19 | 2.36 |

(continued)

| Example | Structure | Compound name | $^1$H NMR, MS | Yield (%) | HPLC r.t. (min), Purity |
|---|---|---|---|---|---|
| 46 | 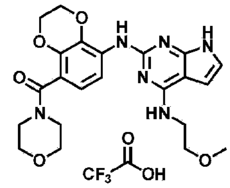 | (8-((4-((2-methoxyethyl)amino)-7*H*-pyrrolo[2,3-*d*] pyrimidin-2-yl)amino)-2,3-dihydrobenzo [b][1,4]dioxin-5-yl)(morpholino)me thanone 2,2,2-trifluoroacetate | $^1$H NMR (400 MHz, TFA salt, DMSO-$d_6$): $\delta$ = 11.73 - 11.44 (m, 1H), 10.04 - 9.65 (m, 1H), 8.82 (d, *J* = 8.7 Hz, 1H), 8.35 - 8.09 (m, 1H), 7.42 - 7.34 (m, 1H), 7.28 - 7.22 (m, 1H), 7.11 - 6.95 (m, 1H), 6.65 - 6.57 (m, 1H), 4.02 (s, 3H), 3.97 (br s, 1H), 3.81 (br d, *J* = 11.2 Hz, 2H), 3.61 - 3.57 (m, 7H), 3.42 - 3.34 (m, 2H), 3.32 - 3.29 (m, 3H), 3.26 - 3.18 (m, 1H), 3.11 - 2.93 (m, 2H), 2.26 (br t, *J* = 12.0 Hz, 2H), 2.12 (br d, *J* = 10.6 Hz, 2H), 1.75 - 1.61 (m, 2H); LCMS (Method 2): m/z = 455.2 [M + H]$^+$ | 32 | 2.15 |
| 47 | 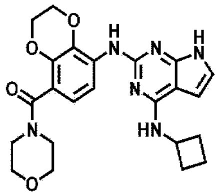 | (8-((4-(cyclobutylamino) -7*H*-pyrrolo[2,3-*d*]pyrimidin-2-yl)amino)-2,3-dihydrobenzo [*b*][1 ,4]dioxin-5-yl) (morpholino)me thanone | $^1$H NMR (400 MHz, DMSO-$d_6$): $\delta$ = 11.10 (br s, 1H), 8.26 (d, *J* = 8.5 Hz, 1H), 7.50 (d, *J* = 7.4 Hz, 1H), 7.10 (s, 1H), 6.81 (m, 1H), 6.74 (d, *J* = 8.5 Hz, 1H), 6.47 - 6.43 (m, 1H), 4.66 - 4.54 (m, 1H), 4.38 (br d, *J* = 3.0 Hz, 2H), 4.31 (br d, *J* = 3.6 Hz, 2H), 3.59 (br s, 4H), 3.52 (br s, 2H), 3.25 - 3.18 (m, 2H), 2.35 - 2.28 (m, 2H), 2.08 - 1.98 (m, 2H), 1.75 - 1.66 (m, 2H); LCMS (Method 2): m/z = 451.2 [M + H]$^+$ | 5.5 | 2.57 |

(continued)

| Example | Structure | Compound name | $^1$H NMR, MS | Yield (%) | HPLC r.t. (min), Purity |
|---|---|---|---|---|---|
| 48 | 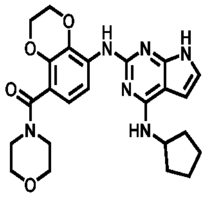 | (8-((4-(cyclopentylamino )-7*H*-pyrrolo[2,3-*d*]pyrimidin-2-yl)amino)-2,3-dihydrobenzo [*b*][1 ,4]dioxin-5-yl)(morpholino)me thanone | $^1$H NMR (400 MHz, DMSO-$d_6$): δ = 11.07 (br s, 1H), 8.28 (d, *J* = 8.4 Hz, 1H), 7.18 (br d, *J* = 7.2 Hz, 1H), 7.09 (s, 1H), 6.82 - 6.77 (m, 1H), 6.73 (d, *J* = 8.4 Hz, 1H), 6.48 (dd, *J* = 2.0, 3.2 Hz, 1H), 4.47 - 4.40 (m, 1H), 4.38 (br d, *J* = 2.0 Hz, 2H), 4.30 (br s, 2H), 3.59 (br s, 4H), 3.55 - 3.49 (m, 2H), 3.25 - 3.13 (m, 2H), 2.05 - 1.93 (m, 2H), 1.76 - 1.66 (m, 2H), 1.63 - 1.49 (m, 4H); LCMS (Method 2): m/z = 465.2 [M + H]$^+$ | 28 | 2.69 |
| 49 | 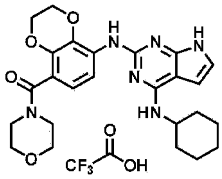 | (8-((4-(cyclohexylamino) -7*H*-pyrrolo[2,3-*d*]pyrimidin-2-yl)amino)-2,3-dihydrobenzo [*b*][1 ,4]dioxin-5-yl) (morpholino)methanone 2,2,2-trifluoroacetate | $^1$H NMR (400 MHz, TFA salt, DMSO-$d_6$): δ = 12.11 - 11.66 (m, 1H), 8.91 - 8.61 (m, 1H), 8.59 - 8.26 (m, 1H), 8.10 - 7.88 (m, 1H), 7.08 - 6.94 (m, 1H), 6.81 (d, *J* = 8.6 Hz, 1H), 6.73 - 6.64 (m, 1H), 4.47 - 4.33 (m, 4H), 3.99 - 3.86 (m, 1H), 3.62 (br s, 4H), 3.41 (br s, 2H), 3.30 - 3.22 (m, 2H), 2.06 - 1.95 (m, 2H), 1.87 -1.75 (m, 2H), 1.73 - 1.63 (m, 1H), 1.47 - 1.32 (m, 4H), 1.27 - 1.12 (m, 1H); LCMS (Method 2): m/z = 479.2 [M + H]$^+$ | 27 | 2.16 |

(continued)

| Example | Structure | Compound name | $^1$H NMR, MS | Yield (%) | HPLC r.t. (min), Purity |
|---|---|---|---|---|---|
| 50 | 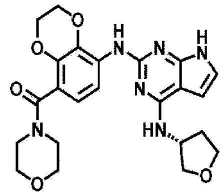 | (*R*)-morpholino (8-((4-((tetrahydrofuran-3-yl) amino)-7*H*-pyrrolo[2,3-*d*] pyrimidin-2-yl)amino)-2,3-dihydrobenzo[*b*][1 ,4]dioxin-5-yl)methanone | $^1$H NMR (400 MHz, DMSO-$d_6$): δ = 11.56 - 10.73 (m, 1H), 8.52 - 7.97 (m, 1H), 7.63 - 7.29 (m, 1H), 7.19 - 7.11 (m, 1H), 6.86 - 6.80 (m, 1H), 6.78 - 6.72 (m, 1H), 6.54 - 6.46 (m, 1H), 4.73 - 4.59 (m, 1H), 4.43 - 4.26 (m, 4H), 3.99 - 3.84 (m, 2H), 3.80 - 3.69 (m, 1H), 3.68 - 3.57 (m, 5H), 3.53 (br s, 2H), 3.28 - 3.19 (m, 2H), 2.29 - 2.19 (m, 1H), 2.03 - 1.88 (m, 1H); LCMS (Method 2): m/z = 467.2 [M + H]$^+$ | 40 | 2.39 |
| 51 | 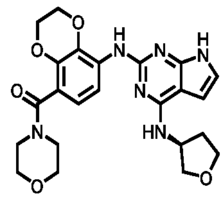 | (S)-morpholino (8-((4-((tetrahydrofuran-3-yl) amino)-7*H*-pyrrolo[2,3-*d*] pyrimidin-2-yl)amino)-2,3-dihydrobenzo[*b*][1 ,4]dioxin-5-yl)methanone | $^1$H NMR (400 MHz, DMSO-$d_6$): δ = 11.50 - 10.85 (m, 1H), 8.39 - 8.07 (m, 1H), 7.56 - 7.31 (m, 1H), 7.23 - 7.08 (m, 1H), 6.86 - 6.80 (m, 1H), 6.78 - 6.72 (m, 1H), 6.55 - 6.45 (m, 1H), 4.72 - 4.58 (m, 1H), 4.43 - 4.27 (m, 4H), 4.01 - 3.85 (m, 2H), 3.82 - 3.70 (m, 1H), 3.66 - 3.57 (m, 5H), 3.56 - 3.49 (m, 2H), 3.31 - 3.15 (m, 2H), 2.30 - 2.18 (m, 1H), 2.00 - 1.89 (m, 1H); LCMS (Method 2): m/z = 467.2 [M + H]$^+$ | 38.2 | 2.39 |

(continued)

| Example | Structure | Compound name | $^1$H NMR, MS | Yield (%) | HPLC r.t. (min), Purity |
|---|---|---|---|---|---|
| 52 | 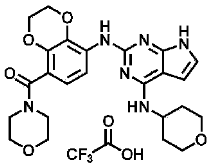 | morpholino (8-((4-((tetrahydro-2*H*-pyran-4-yl)amino)-7*H*-pyrrolo[2,3-*d*] pyrimidin-2-yl)amino)-2,3-dihydrobenzo[*b*][1 ,4]dioxin-5-yl)methanone 2,2,2-trifluoroacetate | $^1$H NMR (400 MHz, TFA salt, DMSO-$d_6$): δ = 11.83 (d, *J* = 8.8 Hz, 1H), 8.81 - 8.29 (m, 2H), 7.93 (d, *J* = 7.6 Hz, 1H), 6.99 (s, 1H), 6.83 (d, *J* = 8.4 Hz, 1H), 6.67 (s, 1H), 4.37 (m, 4H), 4.18 (d, *J* = 6.4 Hz, 1H), 4.00 - 3.93 (m, 2H), 3.62 (s, 4H), 3.54 (s, 2H), 3.47 - 3.41 (m, 2H), 3.25 (d, *J* = 1.6 Hz, 2H), 1.93 (m, 2H), 1.63 (m, 2H); LCMS (Method 2): m/z = 481.2 [M + H]$^+$ | 32 | 2.20 |
| 53 | 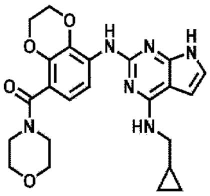 | (8-((4-((cyclopropylmeth yl) amino)-7*H*-pyrrolo[2,3-*d*] pyrimidin-2-yl)amino)-2,3-dihydrobenzo[*b*][1 ,4]dioxin-5-yl)(morpholino)me thanone | $^1$H NMR (400 MHz, DMSO-$d_6$): δ = 11.11 (s, 1H), 8.28 (d, *J* = 8.4 Hz, 1H), 7.46 (t, *J* = 5.4 Hz, 1H), 7.12 (s, 1H), 6.82 (m, 1H), 6.75 (d, *J* = 8.4 Hz, 1H), 6.48 (m, 1H), 4.43 - 4.30 (m, 4H), 3.61 (s, 4H), 3.56 - 3.50 (m, 2H), 3.39 - 3.34 (m, 2H), 3.28 - 3.18 (m, 2H), 1.20 - 1.07 (m, 1H), 0.51 - 0.44 (m, 2H), 0.32 - 0.25 (m, 2H); LCMS (Method 2): m/z = 451.2 [M + H]$^+$ | 10 | 2.54 |

(continued)

| Example | Structure | Compound name | $^1$H NMR, MS | Yield (%) | HPLC r.t. (min), Purity |
|---|---|---|---|---|---|
| 54 | 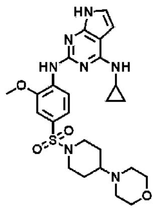 | $N^4$-cyclopropyl-$N^2$-(2-methoxy-4-((4-morpholinopiperid in-1-yl) sulfonyl)phenyl )-7*H*-pyrrolo [2,3-*d*]pyrimidine-2,4-diamine | $^1$H NMR (400 MHz, DMSO-$d_6$): δ = 11.20 (s, 1H), 9.05 (d, *J* = 8.4Hz, 1H), 7.60(s, 1H),7.53(s, 1H), 7.32 (d, *J* = 8.4Hz, 1H), 7.18 (s, 1H), 6.89(s, 1H), 6.50(s, 1H), 4.42(m, 1H), 3.99(s, 3H), 3.66 (m, 2H), 3.50(m, 4H), 2.95(m, 1H), 2.39(m, 4H), 2.26(t, *J* = 10.8 Hz, 2H), 2.15(m, 1H), 1.80(d, *J* = 11.2 Hz, 2H), 1.43 (m, 2H), 0.83 (m, 2H), 0.60 (m, 2H) ); LCMS (Method 1): m/z = 528.2 $(M + H)^+$ | 26.5 | 1.25 |
| 55 | 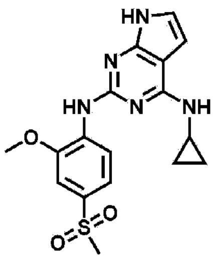 | $N^4$-cyclopropyl-$N^2$-(2-methoxy-4-(methylsulfonyl)p henyl)-7*H*-pyrrolo[2,3-*d*] pyrimidine-2,4-diamine | $^1$H NMR (400 MHz, DMSO-$d_6$): δ = 11.22 (s, 1H), 9.07(d, *J* = 8.4Hz, 1H), 7.61(s, 1H), 7.54(s, 1H), 7.50 (d, *J* = 8.4Hz, 1H), 7.42 (s, 1H), 6.89(s, 1H), 6.50(s, 1H), 4.01(s, 3H), 3.18(s, 3H), 2.96 (m, 1H), 0.83 (m, 2H), 0.60 (m, 2H); LCMS (Method 1): m/z = 374.1 $(M + H)^+$ | 30.7 | 1.18 |
| 56 | 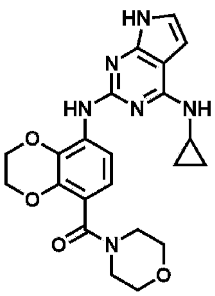 | (8-((4-(cyclopropylamino )-7*H*-pyrrolo[2,3-*d*]pyrimidin-2-yl)amino)-2,3-dihydrobenzo [*b*][1 ,4]dioxin-5-yl) (morpholino)me thanone | $^1$H NMR (400 MHz, DMSO-$d_6$): δ = 11.14 (s, 1H), 8.39(d, *J* = 8.4Hz, 1H), 7.47(s, 1H),7.13(s, 1H), 7.27 (s, 1H), 6.84(s, 1H), 6.75 (d, *J* = 8.4Hz, 1H), 6.47(s, 1H), 4.39-4.32 (m, 4H), 360-3.53(m, 6H), 3.26(m, 2H), 2.91 (m, 1H), 0.83 (m, 2H), 0.60 (m, 2H); LCMS (Method 1): m/z = 437.2 $(M + H)^+$ | 17.1 | 1.11 |

(continued)

| Example | Structure | Compound name | $^1H$ NMR, MS | Yield (%) | HPLC r.t. (min), Purity |
|---|---|---|---|---|---|
| 57 | 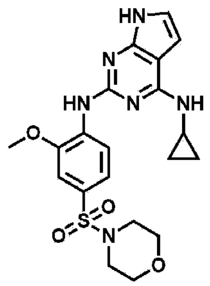 | $N^4$-cyclopropyl-$N^2$-(2-methoxy-4-(morpholinosulfonyl)phenyl)-7*H*-pyrrolo[2,3-*d*] pyrimidine-2,4-diamine | $^1H$ NMR (400 MHz, DMSO-$d_6$): δ = 11.20 (s, 1H), 9.08(d, *J* = 8.4Hz, 1H), 7.61(d, *J* = 2.8Hz, 1H), 7.55(s, 1H), 7.33(q, *J* = 2.0Hz, 1H), 6.89(s, 1H), 6.88 (s, 1H), 6.51(s, 1H), 4.01(s, 3H), 3.64(t, *J* = 3.6Hz, 4H), 3.29(s, 1H), 2.91(m, 4H), 0.84 (m, 2H), 0.60(q, *J* = 4.4Hz, 2H); LCMS (Method 1): m/z = 445.1 $(M + H)^+$ | 10.8 | 1.26 |
| 58 | 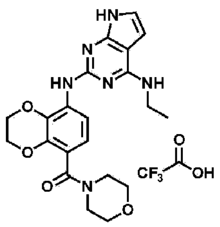 | (8-((4-(ethylamino)-7*H*-pyrrolo[2,3-*d*]pyrimidin-2-yl) amino)-2,3-dihydrobenzo[b] [1 ,4]dioxin-5-yl)(morpholino) me thanone 2,2,2-trifluoroacetate | $^1H$ NMR (400 MHz, TFA-salt, DMSO-$d_6$): δ = 12.21 - 11.53 (m, 1H), 9.12 - 8.44 (m, 1H), 8.23 - 7.68 (m, 1H), 7.01 (br s, 1H), 6.81 (d, *J* = 8.5 Hz, 1H), 6.64 (br s, 1H), 4.40 (br d, *J* = 3.0 Hz, 2H), 4.35 (br d, *J* = 3.5 Hz, 2H), 3.61 (br s, 4H), 3.56 - 3.46 (m, 4H), 3.30 - 3.14 (m, 2H), 1.27 (t, *J* = 7.2 Hz, 3H); LCMS (Method 2): m/z = 425.2$(M + H)^+$ | 6.9 | 2.27 |
| 59 | 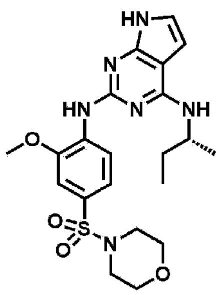 | (*R*)-$N^4$-(sec-butyl)-$N^2$-(2-methoxy-4-(morpholinosulfonyl)phenyl)-7*H*-pyrrolo[2,3-*d*] pyrimidine-2,4-diamine | $^1H$ NMR (400 MHz, DMSO-$d_6$): δ = 11.45 (s, 1H), 8.91(d, *J* = 8.8Hz, 1H), 7.51(s, 1H), 7.30(q, 2.0Hz, 1H), 7.16(t, *J* = 2.0Hz, 2H), 6.86(d, *J* = 2.4Hz, 1H), 6.53(d, *J* = 2.0Hz, 1H), 4.19(d, *J* = 1.6Hz, 1H), 4.00(d, *J* = 8.0Hz, 3H), 3.63(t, *J* = 4.0Hz, 4H), 2.87(t, *J* = 4.0Hz, 4H), 1.55(m, 2H), 1.21 (d, *J* = 6.8Hz, 3H), 0.92 (s, *J* = 7.2Hz, 3H); LCMS (Method 1): m/z = 461.1 $(M + H)^+$ | 17.9 | 0.68 |

(continued)

| Example | Structure | Compound name | $^1$H NMR, MS | Yield (%) | HPLC r.t. (min), Purity |
|---|---|---|---|---|---|
| 60 | 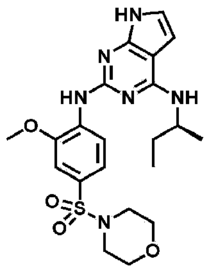 | (S)-$N^4$-(sec-butyl)-$N^2$-(2-methoxy-4-(morpholinosulfonyl)phenyl)-7*H*-pyrrolo[2,3-*d*]pyrimidine-2,4-diamine | $^1$H NMR (400 MHz, DMSO-$d_6$): δ = 11.16 (s, 1H), 8.92(d, *J* = 8.4Hz, 1H), 7.52(s, 1H), 7.31(t, *J* = 2.0Hz, 1H), 7.17(t, *J* = 1.6Hz, 2H), 6.86(t, *J* = 2.8Hz, 1H), 6.54(d, *J* = 1.6Hz, 1H), 4.20(t, *J* = 6.8Hz, 1H), 4.00(s, 3H), 3.63 (s, *J* = 4.4Hz, 4H), 2.88 (t, *J* = 4.0Hz, 4H), 1.60 (m, 2H), 1.22(d, *J* = 6.8Hz, 3H), 0.93(t, *J* = 7.6Hz, 3H); LCMS (Method 1): m/z = 461.2 $(M + H)^+$; | 15.2 | 0.82 |
| 61 | 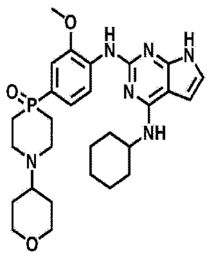 | 4-(4-((4-(cyclohexylamino)-7*H*-pyrrolo[2,3-*d*]pyrimidin-2-yl)amino)-3-methoxyphenyl)-1-(tetrahydro-2H-pyran-4-yl)-1,4-azaphosphinane 4-oxide | $^1$H NMR (400 MHz, DMSO-$d_6$): δ = 11.10 (br s, 1H), 8.81 (dd, *J* = 3.1, 8.3 Hz, 1H), 7.39 (s, 1H), 7.34 - 7.28 (m, 1H), 7.28 - 7.18 (m, 2H), 6.83 (dd, *J* = 1.9, 3.2 Hz, 1H), 6.50 (br d, *J* = 1.6 Hz, 1H), 4.04 - 3.96 (m, 4H), 3.89 (br dd, *J* = 3.7, 10.7 Hz, 2H), 3.33 - 3.23 (m, 3H), 2.98 - 2.87 (m, 4H), 2.26 - 2.12 (m, 2H), 2.00 (br d, *J* = 11.0 Hz, 2H), 1.87 - 1.75 (m, 4H), 1.64 (br d, *J* = 11.9 Hz, 3H), 1.54 - 1.36 (m, 4H), 1.35 - 1.25 (m, 2H), 1.18 (br d, *J* = 12.4 Hz, 1H); LCMS (Method 1): m/z = 539.3 $(M + H)^+$ | 33.6 | 1.88 |

(continued)

| Example | Structure | Compound name | $^1$H NMR, MS | Yield (%) | HPLC r.t. (min), Purity |
|---|---|---|---|---|---|
| 62 | 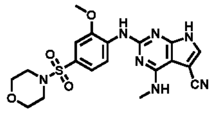 | 2-((2-methoxy-4-(morpholinosulfon yl) phenyl)amino)-4-(methylamino)-7*H*-pyrrolo [2,3-*d*]pyrimidine-5-carbonitrile | $^1$H NMR (400 MHz, DMSO-$d_6$): δ = 12.41 (br s, 1H), 8.84 (d, *J* = 8.6 Hz, 1H), 7.94 (d, *J* = 2.4 Hz, 1H), 7.84 (s, 1H), 7.35 (dd, *J* = 2.0, 8.6 Hz, 1H), 7.22 (d, *J* = 1.8 Hz, 1H), 6.70 (br s, 1H), 4.01 (s, 3H), 3.67 - 3.63 (m, 5H), 3.04 (d, *J* = 3.3 Hz, 3H), 2.92 - 2.87 (m, 4H); LCMS (Method 2): m/z = 444.1 [M + H]$^+$ | 10.6 | 2.75 |
| 63 | 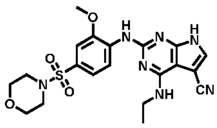 | 4-(ethylamino)-2-((2-methoxy-4-(morpholinosulfon yl)phenyl)amino)-7*H*-pyrrolo [2,3-*d*]pyrimidine-5-carbonitrile | $^1$H NMR (400 MHz, DMSO-$d_6$): 12.38 (br s, 1H), 8.81 (d, *J* = 8.6 Hz, 1H), 7.94 (d, *J* = 2.8 Hz, 1H), 7.79 (s, 1H), 7.35 (dd, *J* = 1.9, 8.6 Hz, 1H), 7.22 (d, *J* = 1.9 Hz, 1H), 6.57 (br s, 1H), 4.01 (s, 3H), 3.66 - 3.63 (m, 4H), 3.60 - 3.55 (m, 2H), 2.93 - 2.86 (m, 4H), 1.25 (t, *J* = 7.1 Hz, 3H); LCMS (Method 2): m/z = 458.2 [M + H]$^+$ | 35.2 | 2.89 |
| 64 | 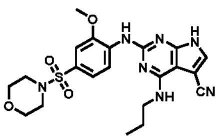 | 2-((2-methoxy-4-(morpholinosulfon yl) phenyl)amino)-4-(propylamino)-7*H*-pyrrolo [2,3-*d*]pyrimidine-5-carbonitrile | $^1$H NMR (400 MHz, DMSO-$d_6$): δ = 8.80 (d, *J* = 8.6 Hz, 1H), 7.93 (s, 1H), 7.75 (s, 1H), 7.33 (dd, *J* = 2.0, 8.6 Hz, 1H), 7.21 (d, *J* = 2.0 Hz, 1H), 6.48 (s, 1H), 4.01 (s, 3H), 3.68 - 3.61 (m, 4H), 3.57 - 3.46 (m, 2H), 2.92 - 2.86 (m, 4H), 1.74 - 1.56 (m, 2H), 0.97 (t, *J* = 7.4 Hz, 3H); LCMS (Method 2): m/z = 472.2 [M + H]$^+$ | 6.2 | 2.96 |

(continued)

| Example | Structure | Compound name | $^1$H NMR, MS | Yield (%) | HPLC r.t. (min), Purity |
|---|---|---|---|---|---|
| 65 | 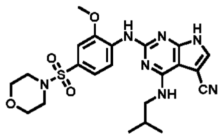 | 4-(isobutylamino)-2-((2-methoxy-4-(morpholinosulfonyl)phenyl)amino)-7*H*-pyrrolo[2,3-*d*]pyrimidine-5-carbonitrile | $^1$H NMR (400 MHz, DMSO-$d_6$): δ = 12.38 (br s, 1H), 8.78 (d, *J* = 8.6 Hz, 1H), 7.95 (d, *J* = 2.6 Hz, 1H), 7.80 (s, 1H), 7.33 (dd, *J* = 1.9, 8.6 Hz, 1H), 7.22 (d, *J* = 1.8 Hz, 1H), 6.45 (br t, *J* = 5.4 Hz, 1H), 4.01 (s, 3H), 3.66 - 3.62 (m, 4H), 3.40 (t, *J* = 6.3 Hz, 2H), 2.92 - 2.86 (m, 4H), 2.01 (td, *J* = 6.8, 13.4 Hz, 1H), 0.97 (d, *J* = 6.7 Hz, 6H); LCMS (Method 2): m/z = 486.1 [M + H]$^+$ | 18.7 | 2.88 |
| 66 | 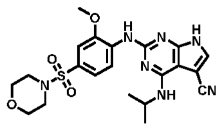 | 4-(isopropylamino)-2-((2-methoxy-4-(morpholinosulfonyl)phenyl)amino)-7*H*-pyrrolo[2,3-*d*]pyrimidine-5-carbonitrile | $^1$H NMR (400 MHz, DMSO-$d_6$): δ = 8.80 (d, *J* = 8.5 Hz, 1H), 7.94 (s, 1H), 7.77 (s, 1H), 7.35 (dd, *J* = 1.9, 8.6 Hz, 1H), 7.21 (d, *J* = 2.0 Hz, 1H), 5.93 (d, *J* = 7.8 Hz, 1H), 4.39 (dd, *J* = 6.6, 14.1 Hz, 1H), 4.00 (s, 3H), 3.70 - 3.59 (m, 4H), 2.96 - 2.83 (m, 4H), 1.30 (d, *J* = 6.5 Hz, 6H); LCMS (Method 2): m/z = 472.2 [M + H]$^+$ | 7.8 | 3.01 |
| 67 | 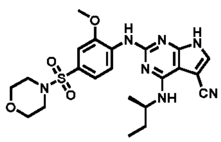 | (*R*)-4-(*sec*-butylamino)-2-((2-methoxy-4-(morpholinosulfonyl)phenyl)amino)-7*H*-pyrrolo[2,3-*d*]pyrimidine-5-carbonitrile | $^1$H NMR (400 MHz, DMSO-$d_6$): δ = 8.80 (d, *J* = 8.6 Hz, 1H), 7.93 (s, 1H), 7.75 (s, 1H), 7.33 (dd, *J* = 1.9, 8.6 Hz, 1H), 7.21 (d, *J* = 1.9 Hz, 1H), 5.80 (br d, *J* = 7.9 Hz, 1H), 4.28 - 4.17 (m, 1H), 4.00 (s, 3H), 3.67 - 3.61 (m, 4H), 2.94 - 2.84 (m, 4H), 1.76 - 1.55 (m, 2H), 1.27 (d, *J* = 6.5 Hz, 3H), 0.95 (t, *J* = 7.4 Hz, 3H); LCMS (Method 1): m/z = 486.2 [M + H]$^+$ | 25.7 | 2.79 |

(continued)

| Example | Structure | Compound name | $^1$H NMR, MS | Yield (%) | HPLC r.t. (min), Purity |
|---|---|---|---|---|---|
| 68 | 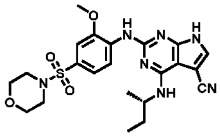 | (*S*)-4-(*sec*-butylamino)-2-((2-methoxy-4-(morpholinosulfonyl)phenyl)amino)-7*H*-pyrrolo[2,3-*d*]pyrimidine-5-carbonitrile | $^1$H NMR (400 MHz, DMSO-$d_6$): δ = 8.80 (d, *J* = 8.6 Hz, 1H), 7.94 (s, 1H), 7.76 (s, 1H), 7.34 (dd, *J* = 1.9, 8.6 Hz, 1H), 7.21 (d, *J* = 2.0 Hz, 1H), 5.83 (br d, *J* = 7.8 Hz, 1H), 4.27 - 4.18 (m, 1H), 4.00 (s, 3H), 3.69 - 3.59 (m, 4H), 2.93 - 2.84 (m, 4H), 1.75 - 1.57 (m, 2H), 1.27 (d, *J* = 6.5 Hz, 3H), 0.95 (t, *J* = 7.4 Hz, 3H); LCMS (Method 1): m/z = 486.2 $[M + H]^+$ | 18.0 | 2.79 |
| 69 | 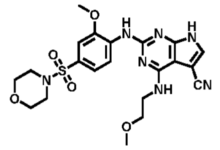 | 2-((2-methoxy-4-(morpholinosulfon yl) phenyl)amino)-4-((2-methoxyethyl)ami no)-7*H*-pyrrolo[2,3-*d*]pyrimidine-5-carbonitrile | $^1$H NMR (400 MHz, DMSO-$d_6$): δ = 12.45 (br s, 1H), 8.83 (d, *J* = 8.6 Hz, 1H), 8.00 (s, 1H), 7.83 (s, 1H), 7.38 (dd, *J* = 1.9, 8.6 Hz, 1H), 7.26 (d, *J* = 1.9 Hz, 1H), 6.45 (t, *J* = 5.5 Hz, 1H), 4.05 (s, 3H), 3.77 (q, *J* = 5.6 Hz, 2H), 3.71 - 3.66 (m, 4H), 3.66 - 3.62 (m, 2H), 3.37 (s, 3H), 2.97 - 2.87 (m, 4H); LCMS (Method 2): m/z = 488.2 $[M + H]^+$ | 12.9 | 2.80 |
| 70 | 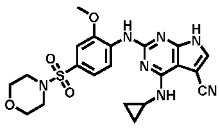 | 4-(cyclopropylamino )-2-((2-methoxy-4-(morpholinosulfonyl)phenyl)amino)-7*H*-pyrrolo[2,3-*d*]pyrimidine-5-carbonitrile | $^1$H NMR (400 MHz, DMSO-$d_6$): δ = 12.96 - 11.58 (m, 1H), 9.14 - 8.85 (m, 1H), 8.00 - 7.90 (m, 1H), 7.83 - 7.69 (m, 1H), 7.44 - 7.31 (m, 1H), 7.27 - 7.14 (m, 1H), 6.85 - 6.60 (m, 1H), 4.09 - 3.93 (m, 3H), 3.73 - 3.59 (m, 4H), 3.01 - 2.93 (m, 1H), 2.93 - 2.84 (m, 4H), 0.95 - 0.80 (m, 2H), 0.73 - 0.56 (m, 2H); LCMS (Method 2): m/z = 470.2 $[M + H]^+$ | 17.7 | 2.94 |

(continued)

| Example | Structure | Compound name | $^1H$ NMR, MS | Yield (%) | HPLC r.t. (min), Purity |
|---|---|---|---|---|---|
| 71 | 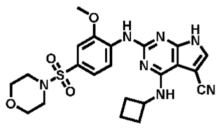 | 4-(cyclobutylamino) -2-((2-methoxy-4-(morpholinosulfonyl)phenyl)amino)-7*H*-pyrrolo[2,3-*d*]pyrimidine-5-carbonitrile | $^1H$ NMR (400 MHz, DMSO-$d_6$): δ = 12.37 (s, 1H), 8.80 (d, *J* = 8.7 Hz, 1H), 7.94 (s, 1H), 7.76 (s, 1H), 7.36 (dd, *J* = 1.9, 8.6 Hz, 1H), 7.22 (d, *J* = 1.8 Hz, 1H), 6.51 (br d, *J* = 7.3 Hz, 1H), 4.76 - 4.51 (m, 1H), 4.01 (s, 3H), 3.69 - 3.56 (m, 4H), 2.96 - 2.85 (m, 4H), 2.42 - 2.31 (m, 2H), 2.11 (m, 2H), 1.83 - 1.70 (m, 2H); LCMS (Method 2): m/z = 484.2 $[M + H]^+$ | 7.3 | 3.04 |
| 72 | 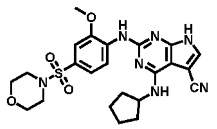 | 4-(cyclopentylamino )-2-((2-methoxy-4-(morpholinosulfonyl)phenyl)amino)-7*H*-pyrrolo[2,3-*d*]pyrimidine-5-carbonitrile | $^1H$ NMR (400 MHz, DMSO-$d_6$): δ = 8.83 (d, *J* = 8.6 Hz, 1H), 7.91 (s, 1H), 7.75 (s, 1H), 7.34 (dd, *J* = 1.8, 8.6 Hz, 1H), 7.21 (d, *J* = 1.8 Hz, 1H), 6.00 (br d, *J* = 7.2 Hz, 1H), 4.56 - 4.33 (m, 1H), 4.01 (s, 3H), 3.70 - 3.58 (m, 4H), 2.95 - 2.81 (m, 4H), 2.15 - 2.02 (m, 2H), 1.79 - 1.68 (m, 2H), 1.68 - 1.52 (m, 4H); LCMS (Method 2): m/z = 498.2 $[M + H]^+$ | 11.5 | 3.16 |

(continued)

| Example | Structure | Compound name | $^1H$ NMR, MS | Yield (%) | HPLC r.t. (min), Purity |
|---|---|---|---|---|---|
| 73 | 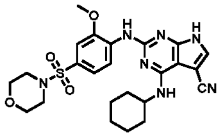 | 4-(cyclohexylamino) -2-((2-methoxy-4-(morpholinosulfonyl)phenyl)amino)-7*H*-pyrrolo [2,3-*d*]pyrimidine-5-carbonitrile | $^1H$ NMR (400 MHz, DMSO-$d_6$): δ = 8.78 (d, *J* = 8.7 Hz, 1H), 7.92 (s, 1H), 7.76 (s, 1H), 7.32 (dd, *J* = 1.9, 8.6 Hz, 1H), 7.21 (d, *J* = 1.8 Hz, 1H), 5.88 (br d, *J* = 7.6 Hz, 1H), 4.09 (br d, *J* = 4.0 Hz, 1H), 4.00 (s, 3H), 3.69 - 3.59 (m, 4H), 2.94 - 2.85 (m, 4H), 2.05 - 1.96 (m, 2H), 1.80 - 1.71 (m, 2H), 1.63 (br d, *J* = 11.7 Hz, 1H), 1.48 - 1.36 (m, 4H), 1.26 (br d, *J* = 8.6 Hz, 1H); LCMS (Method 2): m/z = 512.2 [M + H]+ | 12.7 | 3.26 |
| 74 | 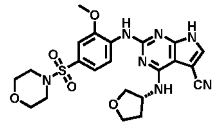 | (R)-2-((2-methoxy-4-(morpholinosulfon yl) phenyl)amino)-4-((tetrahydrofuran-3-yl) amino)-7*H*-pyrrolo[2,3-*d*] pyrimidine-5-carbonitrile | $^1H$ NMR (400 MHz, DMSO-$d_6$): δ = 8.79 (d, *J* = 8.5 Hz, 1H), 7.95 (s, 1H), 7.82 (s, 1H), 7.36 (dd, *J* = 1.9, 8.6 Hz, 1H), 7.21 (d, *J* = 2.0 Hz, 1H), 6.34 (d, *J* = 6.5 Hz, 1H), 4.75 - 4.67 (m, 1H), 4.02 - 3.97 (m, 4H), 3.94 - 3.87 (m, 1H), 3.78 (dt, *J* = 5.9, 8.3 Hz, 1H), 3.71 - 3.61 (m, 5H), 2.92 - 2.86 (m, 4H), 2.36 - 2.28 (m, 1H), 2.01 - 1.92 (m, 1H); LCMS (Method 1): m/z = 500.1 [M + H]+ | 15.0 | 2.88 |

(continued)

| Example | Structure | Compound name | $^1$H NMR, MS | Yield (%) | HPLC r.t. (min), Purity |
|---|---|---|---|---|---|
| 75 | 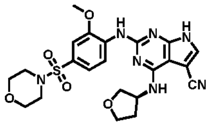 | (*S*)-2-((2-methoxy-4-(morpholinosulfon yl) phenyl)amino)-4-((tetrahydrofuran-3-yl) amino)-7*H*-pyrrolo[2,3-*d*] pyrimidine-5-carbonitrile | $^1$H NMR (400 MHz, DMSO-$d_6$): δ = 8.78 (d, *J* = 8.6 Hz, 1H), 7.95 (s, 1H), 7.81 (s, 1H), 7.35 (dd, *J* = 1.9, 8.6 Hz, 1H), 7.20 (d, *J* = 1.9 Hz, 1H), 6.34 (br d, *J* = 6.4 Hz, 1H), 4.78 - 4.61 (m, 1H), 4.01 - 3.96 (m, 4H), 3.93 - 3.86 (m, 1H), 3.78 (dt, *J* = 5.8, 8.2 Hz, 1H), 3.69 - 3.66 (m, 1H), 3.66 - 3.62 (m, 4H), 2.91 - 2.85 (m, 4H), 2.35 - 2.29 (m, 1H), 2.03 - 1.89 (m, 1H); LCMS (Method 1): m/z = 500.1 [M + H]$^+$ | 23.1 | 2.89 |
| 76 | 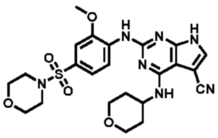 | 2-((2-methoxy-4-(morpholinosulfon yl) phenyl)amino)-4-((tetrahydro-2*H*-pyran-4-yl)amino)-7*H*-pyrrolo[2,3-*d*]pyrimidine-5-carbonitrile | $^1$H NMR (400 MHz, DMSO-$d_6$): δ = 12.36 (br s, 1H), 8.76 (d, *J* = 8.7 Hz, 1H), 7.95 (s, 1H), 7.79 (s, 1H), 7.34 (dd, *J* = 1.9, 8.6 Hz, 1H), 7.22 (d, *J* = 1.8 Hz, 1H), 6.15 (br d, *J* = 7.5 Hz, 1H), 4.33 - 4.23 (m, 1H), 4.00 (s, 3H), 3.94 - 3.87 (m, 2H), 3.67 - 3.61 (m, 4H), 3.55 - 3.46 (m, 2H), 2.93 - 2.87 (m, 4H), 2.04 - 1.95 (m, 2H), 1.72 - 1.61 (m, 2H); LCMS (Method 1): m/z = 514.1 [M + H]$^+$ | 12.0 | 2.74 |

(continued)

| Example | Structure | Compound name | $^1$H NMR, MS | Yield (%) | HPLC r.t. (min), Purity |
|---|---|---|---|---|---|
| 77 | 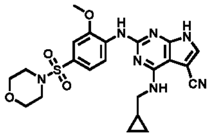 | 4-((cyclopropylmeth yl) amino)-2-((2-methoxy-4-(morpholinosulfonyl) phenyl)amino)-7*H*-pyrrolo [2,3-*d*]pyrimidine-5-carbonitrile | $^1$H NMR (400 MHz, DMSO-$d_6$): δ = 12.62 - 11.72 (m, 1H), 8.80 (d, *J* = 8.6 Hz, 1H), 7.95 (s, 1H), 7.76 (s, 1H), 7.34 (dd, *J* = 2.0, 8.6 Hz, 1H), 7.21 (d, *J* = 1.8 Hz, 1H), 6.46 (t, *J* = 5.5 Hz, 1H), 4.01 (s, 3H), 3.70 - 3.58 (m, 4H), 3.47 - 3.40 (m, 2H), 2.95 - 2.84 (m, 4H), 1.31 - 1.09 (m, 1H), 0.54 - 0.42 (m, 2H), 0.38 - 0.18 (m, 2H); LCMS (Method 2): m/z = 484.2 $[M + H]^+$ | 22.6 | 2.99 |
| 78 | 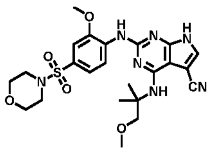 | 4-((1-methoxy-2-methylpropan-2-yl)amino)-2-((2-methoxy-4-(morpholinosulfon yl) phenyl)amino)-7*H*-pyrrolo [2,3-*d*]pyrimidine-5-carbonitrile | $^1$H NMR (400 MHz, DMSO-$d_6$): δ = 12.56 - 12.18 (m, 1H), 8.66 (d, *J* = 8.4 Hz, 1H), 7.92 (s, 1H), 7.84 (s, 1H), 7.34 (d, *J* = 1.8 Hz, 1H), 7.22 (d, *J* = 2.0 Hz, 1H), 5.88 (s, 1H), 4.00 (s, 3H), 3.68 - 3.61 (m, 4H), 3.50 (s, 2H), 3.35 (s, 3H), 2.97 - 2.84 (m, 4H), 1.51 (s, 6H); LCMS (Method 1): m/z = 516.2 $[M + H]^+$ | 6.9 | 3.13 |
| 79 | 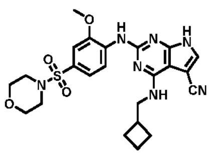 | 4-((cyclobutylmethyl )amino)-2-((2-methoxy-4-(morpholinosulfon yl) phenyl)amino)-7*H*-pyrrolo [2,3-*d*]pyrimidine-5-carbonitrile | $^1$H NMR (400 MHz, DMSO-$d_6$): δ = 8.80 (d, *J* = 8.6 Hz, 1H), 7.94 (s, 1H), 7.75 (s, 1H), 7.32 (dd, *J* = 1.8, 8.6 Hz, 1H), 7.21 (d, *J* = 1.8 Hz, 1H), 6.38 (br t, *J* = 5.4 Hz, 1H), 4.00 (s, 3H), 3.68 - 3.55 (m, 6H), 2.93 - 2.84 (m, 4H), 2.74 - 2.66 (m, 1H), 2.10 - 1.97 (m, 2H), 1.92 - 1.76 (m, 4H); LCMS (Method 1): m/z = 498.2 $[M + H]^+$ | 15.8 | 3.16 |

(continued)

| Example | Structure | Compound name | $^1H$ NMR, MS | Yield (%) | HPLC r.t. (min), Purity |
|---|---|---|---|---|---|
| 80 | 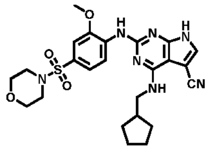 | 4-((cyclopentylmeth yl) amino)-2-((2-methoxy-4-(morpholinosulfon yl) phenyl)amino)-7*H*-pyrrolo [2,3-*d*]pyrimidine-5-carbonitrile | $^1H$ NMR (400 MHz, DMSO-$d_6$): δ = 12.71 - 12.00 (m, 1H), 8.80 (d, *J* = 8.6 Hz, 1H), 7.94 (s, 1H), 7.75 (s, 1H), 7.32 (dd, *J* = 2.0, 8.6 Hz, 1H), 7.21 (d, *J* = 1.8 Hz, 1H), 6.40 (br t, *J* = 5.6 Hz, 1H), 4.00 (s, 3H), 3.67 - 3.62 (m, 4H), 3.49 (dd, *J* = 6.0, 7.0 Hz, 2H), 2.91 - 2.85 (m, 4H), 2.35 - 2.23 (m, 1H), 1.79 - 1.68 (m, 2H), 1.67 - 1.49 (m, 4H), 1.38 - 1.26 (m, 2H); LCMS (Method 1): m/z = 512.3 [M + H]$^+$ | 15.8 | 3.27 |
| 81 | 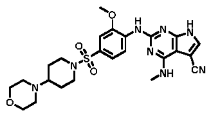 | 2-((2-methoxy-4-((4-morpholinopiperid in-1-yl) sulfonyl)phenyl )amino)-4-(methylamino)-7*H*-pyrrolo [2,3-*d*]pyrimidine-5-carbonitrile | $^1H$ NMR (400 MHz, DMSO-$d_6$): δ = 12.36 (br s, 1H), 9.73 (br s, 1H), 8.85 (d, *J* = 8.5 Hz, 1H), 7.95 (d, *J* = 2.8 Hz, 1H), 7.76 (s, 1H), 7.37 (dd, *J* = 1.8, 8.6 Hz, 1H), 7.25 (d, *J* = 1.9 Hz, 1H), 6.65 (br d, *J* = 4.4 Hz, 1H), 4.01 (s, 3H), 3.98 (br s, 2H), 3.81 (br d, *J* = 12.0 Hz, 2H), 3.69 - 3.61 (m, 8H), 3.38 (br d, *J* = 12.5 Hz, 2H), 3.22 (br s, 2H), 3.04 (d, *J* = 4.5 Hz, 3H), 2.26 (br t, *J* = 11.8 Hz, 2H), 2.12 (br d, *J* = 10.8 Hz, 2H), 1.73 - 1.63 (m, 2H); LCMS (Method 2): m/z = 527.2 [M + H]$^+$ | 6.0 | 2.43 |

(continued)

| Example | Structure | Compound name | $^1$H NMR, MS | Yield (%) | HPLC r.t. (min), Purity |
|---|---|---|---|---|---|
| 82 | 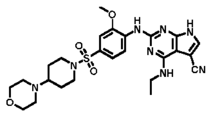 | 4-(ethylamino)-2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 12.35 (br d, *J* = 2.3 Hz, 1H), 9.78 (br s, 1H), 8.82 (d, *J* = 8.5 Hz, 1H), 7.94 (d, *J* = 2.9 Hz, 1H), 7.75 (s, 1H), 7.37 (dd, *J* = 1.9, 8.6 Hz, 1H), 7.24 (d, *J* = 1.9 Hz, 1H), 6.54 (t, *J* = 5.6 Hz, 1H), 4.01 (s, 5H), 3.81 (br d, *J* = 12.1 Hz, 2H), 3.62 (br d, *J* = 12.5 Hz, 2H), 3.59 - 3.53 (m, 2H), 3.49 (br s, 2H), 3.23 (br d, *J* = 12.4 Hz, 2H), 3.04 (br s, 2H), 2.26 (br t, *J* = 11.7 Hz, 2H), 2.12 (br d, *J* = 10.8 Hz, 2H), 1.75 - 1.61 (m, 2H), 1.25 (t, *J* = 7.1 Hz, 3H); LCMS (Method 1): m/z = 541.2 $[M + H]^+$ | 18.8 | 2.53 |
| 83 | 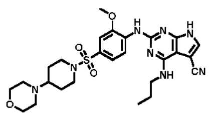 | 2-((2-methoxy-4-((4-morpholinopiperid in-1-yl) sulfonyl)phenyl )amino)-4-(propylamino)-7*H*-pyrrolo [2,3-*d*]pyrimidine-5-carbonitrile | $^1$H NMR (400 MHz, DMSO-$d_6$): δ = 12.35 (d, *J* = 2.4 Hz, 1H), 9.87 (br s, 1H), 8.81 (d, *J* = 8.6 Hz, 1H), 7.94 (d, *J* = 2.7 Hz, 1H), 7.75 (s, 1H), 7.35 (dd, *J* = 2.0, 8.6 Hz, 1H), 7.25 (d, *J* = 1.8 Hz, 1H), 4.01 (s, 5H), 3.81 (br d, *J* = 11.9 Hz, 2H), 3.64 (br s, 1H), 3.53 (br s, 2H), 3.38 (br d, *J* = 13.2 Hz, 3H), 3.27 - 3.17 (m, 1H), 3.05 (br s, 2H), 2.26 (br t, *J* = 11.4 Hz, 2H), 2.12 (br d, *J* = 11.4 Hz, 2H), 1.68 (qd, *J* = 7.3, 14.5 Hz, 4H), 0.97 (t, *J* = 7.4 Hz, 3H); LCMS (Method 2): m/z = 555.2 $[M + H]^+$ | 28.7 | 2.24 |

(continued)

| Example | Structure | Compound name | $^1$H NMR, MS | Yield (%) | HPLC r.t. (min), Purity |
|---|---|---|---|---|---|
| 84 | 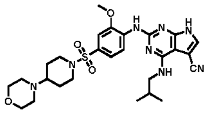 | 4-(isobutylamino)-2-((2-methoxy-4-((4-morpholinopiperid in-1-yl) sulfonyl)phenyl )amino)-7H-pyrrolo[2,3-*d*]pyrimidine-5-carbonitrile | $^1$H NMR (400 MHz, DMSO-$d_6$): δ = 12.36 (br s, 1H), 9.76 (br s, 1H), 8.80 (d, *J* = 8.6 Hz, 1H), 7.96 (d, *J* = 2.7 Hz, 1H), 7.76 (s, 1H), 7.35 (dd, *J* = 1.8, 8.5 Hz, 1H), 7.24 (d, *J* = 1.8 Hz, 1H), 4.01 (s, 3H), 3.98 (br s, 2H), 3.81 (br d, *J* = 11.4 Hz, 2H), 3.63 (br t, *J* = 12.4 Hz, 1H), 3.41 - 3.37 (m, 4H), 3.22 (br s, 2H), 3.04 (br d, *J* = 7.7 Hz, 2H), 2.25 (br t, *J* = 11.5 Hz, 2H), 2.12 (br d, *J* = 11.5 Hz, 2H), 2.01 (td, *J* = 6.8, 13.5 Hz, 1H), 1.74 - 1.61 (m, 2H), 0.97 (d, *J* = 6.7 Hz, 6H); LCMS (Method 2): m/z = 569.2 $[M + H]^+$ | 4.5 | 2.36 |
| 85 | 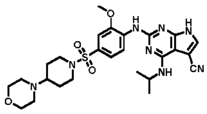 | 4-(isopropylamino)-2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 12.38 (br s, 1H), 9.81 (br s, 1H), 8.81 (d, *J* = 8.6 Hz, 1H), 7.95 (d, *J* = 2.7 Hz, 1H), 7.77 (s, 1H), 7.37 (dd, *J* = 1.8, 8.5 Hz, 1H), 7.24 (d, *J* = 1.8 Hz, 1H), 5.96 (br d, *J* = 7.3 Hz, 1H), 4.44 - 4.32 (m, 1H), 4.01 (s, 5H), 3.81 (br d, *J* = 11.5 Hz, 2H), 3.61 - 3.50 (m, 5H), 3.22 (br s, 3H), 3.04 (br s, 2H), 2.25 (br t, *J* = 11.1 Hz, 2H), 2.12 (br d, *J* = 10.4 Hz, 2H), 1.76 - 1.61 (m, 2H), 1.30 (d, *J* = 6.5 Hz, 6H); LCMS (Method 2): m/z = 555.2 $[M + H]^+$ | 4.6 | 2.27 |

(continued)

| Example | Structure | Compound name | $^1H$ NMR, MS | Yield (%) | HPLC r.t. (min), Purity |
|---|---|---|---|---|---|
| 86 | 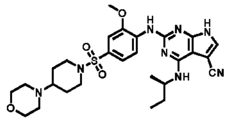 | (*R*)-4-(*sec*-butylamino)-2-((2-methoxy-4-((4-morpholinopiperid in-1-yl) sulfonyl)phenyl )amino)-7*H*-pyrrolo[2,3-*d*]pyrimidine-5-carbonitrile | $^1H$ NMR (400 MHz, DMSO-$d_6$): δ = 8.78 (d, *J* = 8.5 Hz, 1H), 7.92 (s, 1H), 7.71 (s, 1H), 7.32 (dd, *J* = 1.9, 8.6 Hz, 1H), 7.20 (d, *J* = 2.0 Hz, 1H), 5.79 (br d, *J* = 8.3 Hz, 1H), 4.27 - 4.15 (m, 1H), 3.99 (s, 3H), 3.66 (br d, *J* = 12.0 Hz, 2H), 3.54 - 3.49 (m, 4H), 2.42 - 2.36 (m, 4H), 2.30 - 2.22 (m, 2H), 2.16 - 2.08 (m, 1H), 1.80 (br d, *J* = 11.0 Hz, 2H), 1.72 - 1.56 (m, 2H), 1.49 - 1.36 (m, 2H), 1.26 (d, *J* = 6.5 Hz, 3H), 0.95 (t, *J* = 7.4 Hz, 3H); LCMS (Method 1): m/z = 569.3 [M + H]$^+$ | 4.8 | 2.39 |
| 87 | 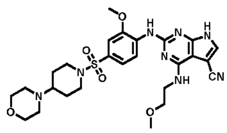 | 2-((2-methoxy-4-((4-morpholinopiperid in-1-yl) sulfonyl)phenyl )amino)-4-((2-methoxyethyl)ami no)-7*H*-pyrrolo[2,3-*d*]pyrimidine-5-carbonitrile | $^1H$ NMR (400 MHz, DMSO-$d_6$): δ = 12.46 - 12.28 (m, 1H), 8.76 (d, *J* = 8.6 Hz, 1H), 7.95 (s, 1H), 7.76 (s, 1H), 7.32 (dd, *J* = 1.9, 8.4 Hz, 1H), 7.21 (d, *J* = 1.9 Hz, 1H), 6.39 (s, 1H), 4.35 (t, *J* = 5.1 Hz, 2H), 3.99 (s, 3H), 3.73 (q, *J* = 5.5 Hz, 2H), 3.65 (br dd, *J* = 3.3, 11.0 Hz, 2H), 3.61 - 3.57 (m, 2H), 3.54 - 3.50 (m, 4H), 2.40 - 2.37 (m, 4H), 2.29 - 2.24 (m, 2H), 2.17 - 2.05 (m, 2H), 1.84 - 1.77 (m, 2H), 1.47 - 1.37 (m, 2H); LCMS (Method 1): m/z = 571.3 [M + H]$^+$ | 3.0 | 2.71 |

(continued)

| Example | Structure | Compound name | $^1$H NMR, MS | Yield (%) | HPLC r.t. (min), Purity |
|---|---|---|---|---|---|
| 88 | 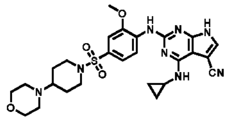 | 4-(cyclopropylamino )-2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 8.97 (d, *J* = 8.5 Hz, 1H), 7.94 (s, 1H), 7.76 (s, 1H), 7.34 (dd, *J* = 1.9, 8.6 Hz, 1H), 7.20 (d, *J* = 1.8 Hz, 1H), 6.74 (br s, 1H), 3.99 (s, 3H), 3.65 (br d, *J* = 11.6 Hz, 2H), 3.51 (br d, *J* = 4.6 Hz, 4H), 2.95 (br dd, *J* = 3.6, 6.8 Hz, 1H), 2.41 - 2.36 (m, 4H), 2.26 (br t, *J* = 11.1 Hz, 2H), 2.09 (br d, *J* = 14.6 Hz, 1H), 1.81 (br d, *J* = 11.1 Hz, 2H), 1.41 (br dd, *J* = 2.8, 11.7 Hz, 2H), 0.93 - 0.82 (m, 2H), 0.70 - 0.62 (m, 2H); LCMS (Method 2): m/z = 553.2 [M + H]$^+$ | 2.5 | 2.94 |
| 89 | 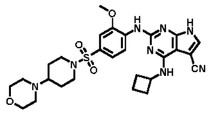 | 4-(cyclobutylamino) -2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 12.34 (br s, 1H), 8.78 (d, *J* = 8.6 Hz, 1H), 7.94 (s, 1H), 7.73 (s, 1H), 7.40 - 7.30 (m, 1H), 7.22 (d, *J* = 1.8 Hz, 1H), 6.49 (br d, *J* = 7.3 Hz, 1H), 4.64 (sxt, *J* = 8.0 Hz, 1H), 3.99 (s, 3H), 3.66 (br d, *J* = 11.6 Hz, 2H), 3.55 - 3.46 (m, 4H), 2.42 - 2.34 (m, 6H), 2.28 (br t, *J* = 11.1 Hz, 2H), 2.16 - 2.05 (m, 3H), 1.84 - 1.72 (m, 4H), 1.48 - 1.36 (m, 2H); LCMS (Method 1): m/z = 567.2 [M + H]$^+$ | 15.0 | 2.95 |

(continued)

| Example | Structure | Compound name | $^1$H NMR, MS | Yield (%) | HPLC r.t. (min), Purity |
|---|---|---|---|---|---|
| 90 | 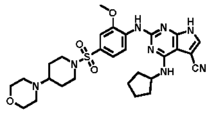 | 4-(cyclopentylamino )-2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 12.56 - 11.97 (m, 1H), 8.78 (d, *J* = 8.6 Hz, 1H), 7.93 (s, 1H), 7.75 (s, 1H), 7.33 (dd, *J* = 1.8, 8.6 Hz, 1H), 7.21 (d, *J* = 2.0 Hz, 1H), 6.04 (br d, *J* = 7.0 Hz, 1H), 4.54 - 4.41 (m, 1H), 3.99 (s, 3H), 3.66 (br d, *J* = 11.6 Hz, 2H), 3.57 - 3.47 (m, 4H), 2.43 - 2.36 (m, 4H), 2.32 - 2.22 (m, 2H), 2.16 - 2.02 (m, 3H), 1.85 - 1.52 (m, 8H), 1.42 (dq, *J* = 2.9, 11.7 Hz, 2H); LCMS (Method 2): m/z = 581.3 $[M + H]^+$ | 4.8 | 3.10 |
| 91 | 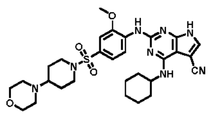 | 4-(cyclohexylamino) -2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 12.52 - 12.13 (m, 1H), 8.74 (d, *J* = 8.6 Hz, 1H), 7.94 (s, 1H), 7.75 (s, 1H), 7.31 (dd, *J* = 1.9, 8.6 Hz, 1H), 7.21 (d, *J* = 2.0 Hz, 1H), 5.92 (br d, *J* = 7.7 Hz, 1H), 4.16 - 4.02 (m, 1H), 3.99 (s, 3H), 3.66 (br d, *J* = 12.0 Hz, 2H), 3.54 - 3.49 (m, 4H), 2.42 - 2.36 (m, 4H), 2.27 (br t, *J* = 11.4 Hz, 2H), 2.15 - 2.09 (m, 1H), 2.02 (br d, *J* = 3.7 Hz, 2H), 1.85 - 1.72 (m, 4H), 1.71 - 1.54 (m, 2H), 1.45 - 1.40 (m, 4H), 1.34 - 1.21 (m, 2H); LCMS (Method 1): m/z = 595.3 $[M + H]^+$ | 5.0 | 3.15 |

(continued)

| Example | Structure | Compound name | $^1$H NMR, MS | Yield (%) | HPLC r.t. (min), Purity |
|---|---|---|---|---|---|
| 92 | 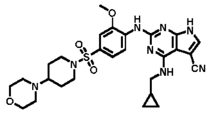 | 4-((cyclopropylmeth yl) amino)-2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 12.18 (br s, 1H), 8.57 (d, *J* = 8.6 Hz, 1H), 7.75 (s, 1H), 7.54 (s, 1H), 7.13 (dd, *J* = 1.8, 8.6 Hz, 1H), 7.01 (d, *J* = 1.9 Hz, 1H), 6.27 (t, *J* = 5.5 Hz, 1H), 3.79 (s, 3H), 3.46 (br d, *J* = 11.6 Hz, 2H), 3.36 - 3.29 (m, 4H), 3.23 (t, *J* = 6.3 Hz, 2H), 2.23 - 2.16 (m, 4H), 2.07 (br t, *J* = 11.1 Hz, 2H), 1.96 - 1.85 (m, 1H), 1.61 (br d, *J* = 11.1 Hz, 2H), 1.30 - 1.13 (m, 2H), 1.08 - 0.94 (m, 1H), 0.33 - 0.23 (m, 2H), 0.17 - 0.11 (m, 2H); LCMS (Method 2): m/z = 567.2 [M + H]$^+$ | 8.1 | 2.94 |
| 93 | 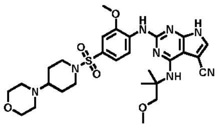 | 4-((1-methoxy-2-methylpropan-2-yl)amino)-2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 12.54 - 12.21 (m, 1H), 8.64 (d, *J* = 8.6 Hz, 1H), 7.92 (s, 1H), 7.81 (s, 1H), 7.35 - 7.31 (m, 1H), 7.22 (d, *J* = 1.8 Hz, 1H), 5.88 (s, 1H), 3.99 (s, 3H), 3.66 (br d, *J* = 11.7 Hz, 2H), 3.54 - 3.49 (m, 6H), 3.35 (s, 3H), 2.42 - 2.36 (m, 4H), 2.27 (br t, *J* = 10.9 Hz, 2H), 2.15 - 2.06 (m, 1H), 1.80 (br d, *J* = 11.1 Hz, 2H), 1.51 (s, 6H), 1.46 - 1.37 (m, 2H); LCMS (Method 1): m/z = 599.3 [M + H]$^+$ | 19.2 | 3.08 |

(continued)

| Example | Structure | Compound name | $^1H$ NMR, MS | Yield (%) | HPLC r.t. (min), Purity |
|---|---|---|---|---|---|
| 94 | 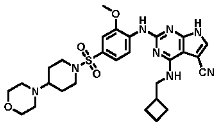 | 4-((cyclobutylmethyl )amino)-2-((2-methoxy-4-((4-morpholinopiperid in-1-yl) sulfonyl)phenyl )amino)-7*H*-pyrrolo[2,3-*d*]pyrimidine-5-carbonitrile | $^1H$ NMR (400 MHz, DMSO-$d_6$): δ = 12.46 - 12.25 (m, 1H), 8.77 (d, *J* = 8.6 Hz, 1H), 7.94 (s, 1H), 7.73 (s, 1H), 7.32 (dd, *J* = 1.8, 8.6 Hz, 1H), 7.21 (d, *J* = 1.8 Hz, 1H), 6.39 (t, *J* = 5.6 Hz, 1H), 3.99 (s, 3H), 3.69 - 3.50 (m, 8H), 2.74 - 2.64 (m, 1H), 2.41 - 2.36 (m, 4H), 2.26 (br t, *J* = 11.1 Hz, 2H), 2.16 - 2.08 (m, 1H), 2.06 - 2.00 (m, 2H), 1.93 - 1.73 (m, 6H), 1.47 - 1.35 (m, 2H); LCMS (Method 1): m/z = 581.3 [M + H]$^+$ | 19.0 | 3.10 |
| 95 | 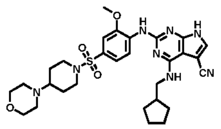 | 4-((cyclopentylmeth yl) amino)-2-((2-methoxy-4-((4-morpholinopiperid in-1-yl) sulfonyl)phenyl )amino)-7*H*-pyrrolo[2,3-*d*]pyrimidine-5-carbonitrile | $^1H$ NMR (400 MHz, DMSO-$d_6$): δ = 12.62 - 12.06 (m, 1H), 8.77 (d, *J* = 8.6 Hz, 1H), 7.94 (s, 1H), 7.73 (s, 1H), 7.31 (dd, *J* = 1.8, 8.6 Hz, 1H), 7.21 (d, *J* = 2.0 Hz, 1H), 6.40 (br t, *J* = 5.4 Hz, 1H), 3.99 (s, 3H), 3.65 (br d, *J* = 11.4 Hz, 2H), 3.56 - 3.46 (m, 6H), 2.42 - 2.35 (m, 4H), 2.28 - 2.22 (m, 2H), 2.14 - 2.07 (m, 1H), 1.83 - 1.70 (m, 4H), 1.65 - 1.50 (m, 4H), 1.46 - 1.28 (m, 4H); LCMS (Method 1): m/z = 595.3 [M + H]$^+$ | 11.2 | 3.20 |

(continued)

| Example | Structure | Compound name | $^1$H NMR, MS | Yield (%) | HPLC r.t. (min), Purity |
|---|---|---|---|---|---|
| 96 | 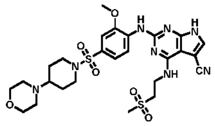 | 2-((2-methoxy-4-((4-morpholinopiperid in-1-yl) sulfonyl)phenyl )amino)-4-((2-(methylsulfonyl)et hyl) amino)-7*H*-pyrrolo[2,3-*d*] pyrimidine-5-carbonitrile | $^1$H NMR (400 MHz, DMSO-$d_6$): δ = 12.57 - 12.12 (m, 1H), 8.74 (d, *J* = 8.6 Hz, 1H), 7.98 (s, 1H), 7.85 (s, 1H), 7.35 (dd, *J* = 1.9, 8.6 Hz, 1H), 7.21 (d, *J* = 2.0 Hz, 1H), 6.87 (t, *J* = 5.8 Hz, 1H), 4.07 - 3.93 (m, 5H), 3.65 (br d, *J* = 11.5 Hz, 2H), 3.57 - 3.46 (m, 6H), 3.08 (s, 3H), 2.43 - 2.36 (m, 4H), 2.31 - 2.21 (m, 2H), 2.17 - 2.07 (m, 1H), 1.80 (br d, *J* = 10.9 Hz, 2H), 1.50 - 1.36 (m, 2H); LCMS (Method 2): m/z = 619.2 [M + H]$^+$ | 6.7 | 2.54 |
| 97 | 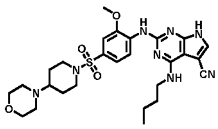 | 4-(butylamino)-2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 12.34 (br s, 1H), 8.77 (d, *J* = 8.6 Hz, 1H), 7.94 (s, 1H), 7.72 (s, 1H), 7.31 (dd, *J* = 1.9, 8.6 Hz, 1H), 7.21 (d, *J* = 2.0 Hz, 1H), 6.48 (t, *J* = 5.7 Hz, 1H), 3.99 (s, 3H), 3.65 (br d, *J* = 11.9 Hz, 2H), 3.59 - 3.48 (m, 6H), 2.42 - 2.36 (m, 4H), 2.31 - 2.21 (m, 2H), 2.17 - 2.05 (m, 1H), 1.80 (br d, *J* = 11.1 Hz, 2H), 1.64 (m, 2H), 1.47 - 1.34 (m, 4H), 0.95 (t, *J* = 7.4 Hz, 3H); LCMS (Method 2): m/z = 569.3 [M + H]$^+$ | 7.5 | 3.06 |

(continued)

| Example | Structure | Compound name | $^1$H NMR, MS | Yield (%) | HPLC r.t. (min), Purity |
|---|---|---|---|---|---|
| 98 | 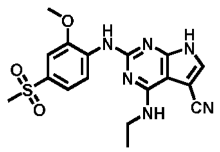 | 4-(ethylamino)-2-((2-methoxy-4-(methylsulfonyl)phenyl)amino)-7*H*-pyrrolo[2,3-*d*]pyrimidine-5-carbonitrile | $^1$H NMR (400 MHz, DMSO-$d_6$): δ = 12.41 (br s, 1H), 8.79 (d, *J* = 8.6 Hz, 1H), 7.94 (d, *J* = 2.3 Hz, 1H), 7.81 (s, 1H), 7.51 (dd, *J* = 1.9, 8.6 Hz, 1H), 7.46 (d, *J* = 1.9 Hz, 1H), 6.60 (br s, 1H), 4.02 (s, 3H), 3.66 - 3.51 (m, 2H), 3.20 (s, 3H), 1.25 (t, *J* = 7.1 Hz, 3H); LCMS (Method 2): m/z = 387.1 $[M + H]^+$ | 28.4 | 2.73 |
| 99 | 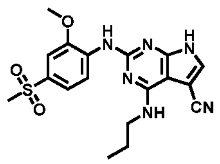 | 2-((2-methoxy-4-(methylsulfonyl)p henyl) amino)-4-(propylamino)-7*H*-pyrrolo[2,3-*d*]pyrimidine-5-carbonitrile | $^1$H NMR (400 MHz, DMSO-$d_6$): δ = 12.37 (br s, 1H), 8.79 (d, *J* = 8.5 Hz, 1H), 7.94 (s, 1H), 7.74 (s, 1H), 7.49 (dd, *J* = 2.0, 8.5 Hz, 1H), 7.46 (d, *J* = 2.0 Hz, 1H), 6.51 (t, *J* = 5.6 Hz, 1H), 4.01 (s, 3H), 3.56 - 3.45 (m, 2H), 3.20 (s, 3H), 1.68 (m, 2H), 0.97 (t, *J* = 7.4 Hz, 3H); LCMS (Method 2): m/z = 401.1 $[M + H]^+$ | 6.9 | 2.89 |
| 100 | 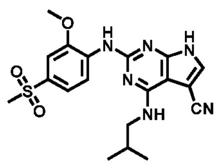 | 4-(isobutylamino)-2-((2-methoxy-4-(methylsulfonyl)phenyl)amino)-7*H*-pyrrolo[2,3-*d*]pyrimidine-5-carbonitrile | $^1$H NMR (400 MHz, DMSO-$d_6$): δ = 12.40 (br s, 1H), 8.77 (d, *J* = 8.6 Hz, 1H), 7.95 (d, *J* = 2.6 Hz, 1H), 7.79 (s, 1H), 7.54 - 7.40 (m, 2H), 6.47 (br t, *J* = 5.4 Hz, 1H), 4.01 (s, 3H), 3.39 (t, *J* = 6.3 Hz, 2H), 2.07 - 1.96 (m, 1H), 0.97 (d, *J* = 6.6 Hz, 6H); LCMS (Method 2): m/z = 415.1 $[M + H]^+$ | 14.2 | 3.16 |

(continued)

| Example | Structure | Compound name | $^1H$ NMR, MS | Yield (%) | HPLC r.t. (min), Purity |
|---|---|---|---|---|---|
| 101 | 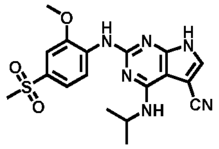 | 4-(isopropylamino)-2-((2-methoxy-4-(methylsulfonyl)phenyl)amino)-7H-pyrrolo[2,3-*d*]pyrimidine-5-carbonitrile | $^1H$ NMR (400 MHz, DMSO-$d_6$): δ = 12.60 - 11.62 (m, 1H), 8.78 (d, *J* = 8.6 Hz, 1H), 7.93 (s, 1H), 7.75 (s, 1H), 7.51 (dd, *J* = 2.0, 8.6 Hz, 1H), 7.46 (d, *J* = 2.0 Hz, 1H), 5.91 (d, *J* = 7.8 Hz, 1H), 4.39 (br d, *J* = 7.5 Hz, 1H), 4.02 (s, 3H), 3.20 (s, 3H), 1.30 (d, *J* = 6.5 Hz, 6H); LCMS (Method 2): m/z = 401.1 $[M + H]^+$ | 11.9 | 2.90 |
| 102 | 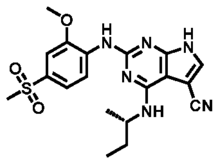 | (*S*)-4-(*sec*-butylamino)-2-((2-methoxy-4-(methylsulfonyl)phenyl)amino)-7*H*-pyrrolo[2,3-*d*]pyrimidine-5-carbonitrile | $^1H$ NMR (400 MHz, DMSO-$d_6$): δ = 8.78 (d, *J* = 8.5 Hz, 1H), 7.93 (s, 1H), 7.74 (s, 1H), 7.50 (dd, *J* = 2.0, 8.6 Hz, 1H), 7.45 (d, *J* = 2.0 Hz, 1H), 5.82 (br d, *J* = 7.8 Hz, 1H), 4.28 - 4.19 (m, 1H), 4.01 (s, 3H), 3.20 (s, 3H), 1.74 - 1.57 (m, 2H), 1.27 (d, *J* = 6.5 Hz, 3H), 0.96 (t, *J* = 7.4 Hz, 3H); LCMS (Method 1): m/z = 415.1 $[M + H]^+$ | 21.9 | 2.98 |
| 103 | 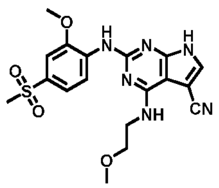 | 2-((2-methoxy-4-(methylsulfonyl)p henyl) amino)-4-((2-methoxyethyl) ami no)-7H-pyrrolo[2,3-*d*] pyrimidine-5-carbonitrile | $^1H$ NMR (400 MHz, DMSO-$d_6$): δ = 8.94 - 8.53 (m, 1H), 7.94 - 7.82 (m, 1H), 7.72 - 7.66 (m, 1H), 7.45 - 7.40 (m, 1H), 7.39 - 7.37 (m, 1H), 6.40 - 6.25 (m, 1H), 3.99 - 3.90 (m, 3H), 3.71 - 3.61 (m, 2H), 3.57 - 3.47 (m, 2H), 3.26 - 3.25 (m, 3H), 3.16 - 3.09 (m, 3H); LCMS (Method 2): m/z = 417.1 $[M + H]^+$ | 12.5 | 2.63 |

(continued)

| Example | Structure | Compound name | $^1$H NMR, MS | Yield (%) | HPLC r.t. (min), Purity |
|---|---|---|---|---|---|
| 104 | 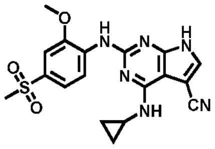 | 4-(cyclopropylamino )-2-((2-methoxy-4-(methylsulfonyl)phenyl)amino)-7*H*-pyrrolo[2,3-*d*]pyrimidine-5-carbonitrile | $^1$H NMR (400 MHz, DMSO-$d_6$): δ = 12.35 (br s, 1H), 8.97 (d, *J* = 8.6 Hz, 1H), 7.95 (s, 1H), 7.75 (s, 1H), 7.52 (dd, *J* = 1.9, 8.6 Hz, 1H), 7.46 (d, *J* = 2.0 Hz, 1H), 6.73 (br s, 1H), 4.02 (s, 3H), 3.24 - 3.14 (m, 3H), 3.03 - 2.85 (m, 1H), 0.93 - 0.79 (m, 2H), 0.75 - 0.58 (m, 2H); LCMS (Method 2): m/z = 399.1 [M + H]$^+$ | 23.8 | 2.67 |
| 105 | 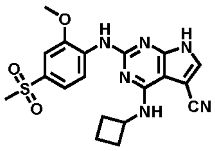 | 4-(cyclobutylamino) -2-((2-methoxy-4-(methylsulfonyl)phenyl)amino)-7*H*-pyrrolo[2,3-*d*]pyrimidine-5-carbonitrile | $^1$H NMR (400 MHz, DMSO-$d_6$): δ = 12.38 (br s, 1H), 8.80 (d, *J* = 8.7 Hz, 1H), 7.95 (s, 1H), 7.75 (s, 1H), 7.53 (dd, *J* = 2.0, 8.6 Hz, 1H), 7.46 (d, *J* = 2.0 Hz, 1H), 6.53 (br d, *J* = 7.5 Hz, 1H), 4.69 - 4.58 (m, 1H), 4.02 (s, 3H), 3.20 (s, 3H), 2.42 - 2.31 (m, 2H), 2.17 - 2.06 (m, 2H), 1.81 - 1.71 (m, 2H); LCMS (Method 1): m/z = 413.1 [M + H]$^+$ | 16.3 | 2.92 |
| 106 | 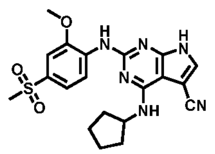 | 4-(cyclopentylamino )-2-((2-methoxy-4-(methylsulfonyl)phenyl)amino)-7*H*-pyrrolo[2,3-*d*]pyrimidine-5-carbonitrile | $^1$H NMR (400 MHz, DMSO-$d_6$): δ = 12.90 - 11.54 (m, 1H), 8.81 (d, *J* = 8.6 Hz, 1H), 7.92 (s, 1H), 7.75 (s, 1H), 7.51 (dd, *J* = 2.0, 8.6 Hz, 1H), 7.46 (d, *J* = 2.0 Hz, 1H), 6.03 (br d, *J* = 7.1 Hz, 1H), 4.61 - 4.32 (m, 1H), 4.02 (s, 3H), 3.23 - 3.14 (m, 3H), 2.17 - 2.00 (m, 2H), 1.81 - 1.48 (m, 6H); LCMS (Method 2): m/z = 427.1 [M + H]$^+$ | 7.0 | 3.08 |

(continued)

| Example | Structure | Compound name | $^1H$ NMR, MS | Yield (%) | HPLC r.t. (min), Purity |
|---|---|---|---|---|---|
| 107 | 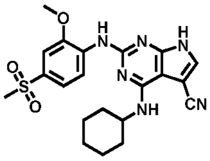 | 4-(cyclohexylamino) -2-((2-methoxy-4-(methylsulfonyl)p henyl)amino)-7*H*-pyrrolo[2,3-*d*]pyrimidine-5-carbonitrile | $^1H$ NMR (400 MHz, DMSO-$d_6$): δ = 12.66 - 11.82 (m, 1H), 8.78 (d, *J* = 8.6 Hz, 1H), 7.94 (s, 1H), 7.76 (s, 1H), 7.49 (dd, *J* = 1.9, 8.5 Hz, 1H), 7.46 (d, *J* = 2.0 Hz, 1H), 5.92 (br d, *J* = 7.5 Hz, 1H), 4.15 - 4.04 (m, 1H), 4.02 (s, 3H), 3.20 (s, 3H), 2.06 - 1.96 (m, 2H), 1.82 - 1.70 (m, 2H), 1.68 - 1.55 (m, 1H), 1.51 - 1.37 (m, 4H), 1.31 - 1.21 (m, 1H); LCMS (Method 2): m/z = 441.2 $[M + H]^+$ | 2.6 | 3.11 |
| 108 | 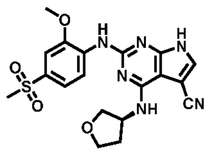 | (S)-2-((2-methoxy-4-(methylsulfonyl)p henyl) amino)-4-((tetrahydrofuran-3-yl)amino)-7*H*-pyrrolo[2,3-*d*] pyrimidine-5-carbonitrile | $^1H$ NMR (400 MHz, DMSO-$d_6$): δ = 8.77 (d, *J* = 8.6 Hz, 1H), 7.94 (s, 1H), 7.79 (s, 1H), 7.52 (dd, *J* = 2.0, 8.5 Hz, 1H), 7.45 (d, *J* = 2.0 Hz, 1H), 6.33 (d, *J* = 6.4 Hz, 1H), 4.77 - 4.66 (m, 1H), 4.01 (s, 3H), 4.00 - 3.96 (m, 1H), 3.94 - 3.87 (m, 1H), 3.78 (dt, *J* = 5.8, 8.2 Hz, 1H), 3.68 (dd, *J* = 4.1, 8.9 Hz, 1H), 3.20 (s, 3H), 2.38 - 2.28 (m, 1H), 2.01 - 1.91 (m, 1H); LCMS: m/z = 429.2 $[M + H]^+$ | 12.3 | 2.73 |
| 109 | 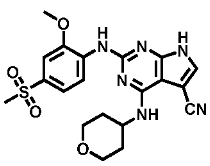 | 2-((2-methoxy-4-(methylsulfonyl)p henyl) amino)-4-((tetrahydro-2*H*-pyran-4-yl)amino)-7*H*-pyrrolo [2,3-*d*]pyrimidine-5-carbonitrile | $^1H$ NMR (400 MHz, DMSO-$d_6$): δ = 8.75 (d, *J* = 8.6 Hz, 1H), 7.95 (s, 1H), 7.78 (s, 1H), 7.54 - 7.44 (m, 2H), 6.16 (br d, *J* = 7.4 Hz, 1H), 4.32 - 4.23 (m, 1H), 4.01 (s, 3H), 3.95 - 3.89 (m, 2H), 3.56 - 3.47 (m, 2H), 3.20 (s, 3H), 2.02 - 1.96 (m, 2H), 1.72 - 1.63 (m, 2H); LCMS (Method 1): m/z = 443.1 $[M + H]^+$ | 3.9 | 2.58 |

(continued)

| Example | Structure | Compound name | $^1$H NMR, MS | Yield (%) | HPLC r.t. (min), Purity |
|---|---|---|---|---|---|
| 110 | 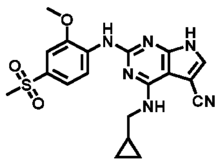 | 4-((cyclopropylmeth yl) amino)-2-((2-methoxy-4-(methylsulfonyl)p henyl) amino)-7*H*-pyrrolo[2,3-*d*] pyrimidine-5-carbonitrile | $^1$H NMR (400 MHz, DMSO-$d_6$): $\delta$ = 12.40 (br s, 1H), 8.79 (d, *J* = 8.6 Hz, 1H), 7.95 (s, 1H), 7.75 (s, 1H), 7.53 - 7.44 (m, 2H), 6.48 (t, *J* = 5.6 Hz, 1H), 4.01 (s, 3H), 3.46 - 3.41 (m, 2H), 3.20 (s, 3H), 1.30 - 1.14 (m, 1H), 0.52 - 0.44 (m, 2H), 0.37 - 0.30 (m, 2H); LCMS (Method 1): m/z = 413.1 [M + H]$^+$ | 17.5 | 2.88 |
| 111 | 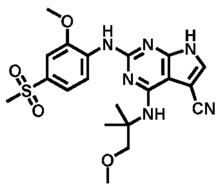 | 4-((1-methoxy-2-methylpropan-2-yl)amino)-2-((2-methoxy-4-(methylsulfonyl)p henyl) amino)-7*H*-pyrrolo[2,3-*d*] pyrimidine-5-carbonitrile | $^1$H NMR (400 MHz, DMSO-$d_6$): $\delta$ = 12.60 - 12.13 (m, 1H), 8.69 (d, *J* = 8.6 Hz, 1H), 7.92 (s, 1H), 7.81 (s, 1H), 7.50 (dd, *J* = 2.0, 8.6 Hz, 1H), 7.47 (d, *J* = 2.0 Hz, 1H), 5.89 (s, 1H), 4.01 (s, 3H), 3.51 (s, 2H), 3.36 (s, 3H), 3.20 (s, 3H), 1.52 (s, 6H); LCMS (Method 1): m/z = 445.1 [M + H]$^+$ | 25.1 | 2.99 |
| 112 | 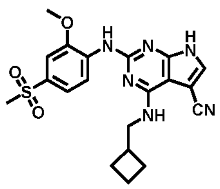 | 4-((cyclobutylmethyl )amino)-2-((2-methoxy-4-(methylsulfonyl)p henyl) amino)-7*H*-pyrrolo[2,3-*d*] pyrimidine-5-carbonitrile | $^1$H NMR (400 MHz, DMSO-$d_6$) $\delta$ = 12.52 - 12.27 (m, 1H), 8.78 (d, *J* = 8.6 Hz, 1H), 7.94 (s, 1H), 7.74 (s, 1H), 7.55 - 7.41 (m, 2H), 6.39 (t, *J* = 5.6 Hz, 1H), 4.01 (s, 3H), 3.65 - 3.54 (m, 2H), 3.20 (s, 3H), 2.70 (quin, *J* = 7.4 Hz, 1H), 2.08 - 1.97 (m, 2H), 1.92 - 1.74 (m, 4H); LCMS (Method 1): m/z = 427.1 [M + H]$^+$ | 22.2 | 3.01 |

(continued)

| Example | Structure | Compound name | $^1$H NMR, MS | Yield (%) | HPLC r.t. (min), Purity |
|---|---|---|---|---|---|
| 113 | 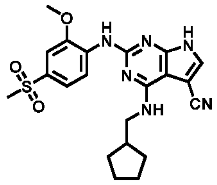 | 4-((cyclopentylmeth yl) amino)-2-((2-methoxy-4-(methylsulfonyl)p henyl) amino)-7*H*-pyrrolo[2,3-*d*] pyrimidine-5-carbonitrile | $^1$H NMR (400 MHz, DMSO-$d_6$): δ = 12.97 - 11.68 (m, 1H), 8.79 (d, *J* = 8.4 Hz, 1H), 7.93 (s, 1H), 7.74 (s, 1H), 7.51 - 7.43 (m, 2H), 6.39 (br t, *J* = 5.5 Hz, 1H), 4.01 (s, 3H), 3.64 - 3.44 (m, 2H), 3.20 (s, 3H), 2.34 - 2.25 (m, 1H), 1.80 - 1.70 (m, 2H), 1.67 - 1.48 (m, 4H), 1.38 - 1.30 (m, 2H); LCMS (Method 1): m/z = 441.1 [M + H]$^+$ | 23.9 | 3.12 |
| 114 | 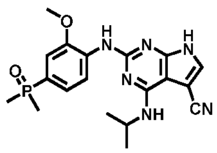 | 2-((4-(dimethylphosphor yl)-2-methoxyphenyl)a mino)-4-(isopropylamino)-7*H*-pyrrolo[2,3-*d*]pyrimidine-5-carbonitrile | $^1$H NMR (400 MHz, DMSO-$d_6$): δ = 12.42 - 12.21 (m, 1H), 8.66 (dd, *J* = 3.1, 8.6 Hz, 1H), 7.91 (s, 1H), 7.61 (s, 1H), 7.37 - 7.27 (m, 2H), 5.86 (d, *J* = 7.8 Hz, 1H), 4.43 - 4.34 (m, 1H), 3.96 (s, 3H), 1.65 (d, *J* = 13.3 Hz, 6H), 1.30 (d, *J* = 6.5 Hz, 6H); LCMS (Method 1): m/z = 399.2 [M + H]$^+$ | 32.0 | 2.63 |
| 115 | 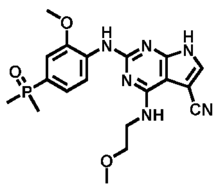 | 2-((4-(dimethylphosphor yl)-2-methoxyphenyl)a mino)-4-((2-methoxyethyl)ami no)-7*H*-pyrrolo[2,3-*d*]pyrimidine-5-carbonitrile | $^1$H NMR (400 MHz, DMSO-$d_6$): δ = 12.34 (br s, 1H), 8.62 (dd, *J* = 3.1, 8.7 Hz, 1H), 7.92 (s, 1H), 7.62 (s, 1H), 7.37 - 7.25 (m, 2H), 6.32 (t, *J* = 5.5 Hz, 1H), 3.95 (s, 3H), 3.71 (q, *J* = 5.6 Hz, 2H), 3.61 - 3.54 (m, 2H), 3.32 (s, 3H), 1.64 (d, *J* = 13.3 Hz, 6H); LCMS (Method 1): m/z = 415.1 [M + H]$^+$ | 30.7 | 2.47 |

(continued)

| Example | Structure | Compound name | $^1$H NMR, MS | Yield (%) | HPLC r.t. (min), Purity |
|---|---|---|---|---|---|
| 116 | 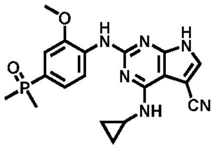 | 4-(cyclopropylamino )-2-((4-(dimethylphosphor yl)-2-methoxyphenyl)a mino)-7*H*-pyrrolo[2,3-*d*]pyrimidine-5-carbonitrile | $^1$H NMR (400 MHz, DMSO-$d_6$): $\delta$ = 12.33 (br s, 1H), 8.84 (dd, *J* = 3.1, 8.4 Hz, 1H), 7.93 (s, 1H), 7.61 (s, 1H), 7.37 - 7.29 (m, 2H), 6.67 (br s, 1H), 3.97 (s, 3H), 2.95 (qt, *J* = 3.5, 6.9 Hz, 1H), 1.64 (d, *J* = 13.3 Hz, 6H), 0.90 - 0.83 (m, 2H), 0.69 - 0.61 (m, 2H); LCMS (Method 1): m/z = 397.1 [M + H]$^+$ | 33.1 | 2.42 |
| 117 | 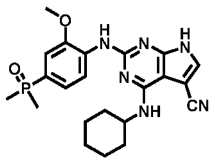 | 4-(cyclohexylamino) -2-((4-(dimethylphosphor yl)-2-methoxyphenyl)a mino)-7H-pyrrolo[2,3-*d*]pyrimidine-5-carbonitrile | $^1$H NMR (400 MHz, DMSO-$d_6$): $\delta$ = 12.38 - 12.22 (m, 1H), 8.66 - 8.59 (m, 1H), 7.91 (s, 1H), 7.61 (s, 1H), 7.36 - 7.28 (m, 2H), 5.86 (br d, *J* = 7.6 Hz, 1H), 4.13 - 4.02 (m, 1H), 3.96 (s, 3H), 2.05 - 1.96 (m, 2H), 1.81 - 1.71 (m, 2H), 1.65 (d, *J* = 13.4 Hz, 6H), 1.49 - 1.38 (m, 5H), 1.32 - 1.21 (m, 1H); LCMS (Method 1): m/z = 439.1 [M + H]$^+$ | 17.8 | 2.87 |
| 118 | 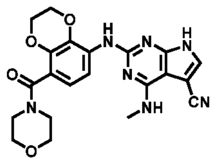 | 4-(methylamino)-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): $\delta$ = 12.41 (br s, 1H), 8.16 (d, *J* = 8.6 Hz, 1H), 7.95 (d, *J* = 2.7 Hz, 1H), 7.60 (br s, 1H), 6.83 (d, *J* = 8.6 Hz, 1H), 6.70 (br s, 1H), 4.45 (br s, 2H), 4.39 (br d, *J* = 3.4 Hz, 2H), 3.67 (br s, 4H), 3.58 (br s, 5H), 3.07 (d, *J* = 4.0 Hz, 3H); LCMS (Method 1): m/z = 436.2 [M + H]$^+$ | 6.1 | 2.37 |

(continued)

| Example | Structure | Compound name | $^1$H NMR, MS | Yield (%) | HPLC r.t. (min), Purity |
|---|---|---|---|---|---|
| 119 | 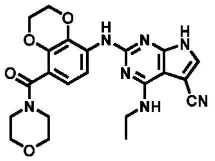 | 4-(ethylamino)-2-((8-(morpholine-4-carbonyl)-2,3-dihydrobenzo[*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.37 (br s, 1H), 8.06 (d, *J* = 8.4 Hz, 1H), 7.90 (s, 1H), 7.63 (br s, 1H), 6.78 (d, *J* = 8.5 Hz, 1H), 6.63 (br s, 1H), 4.39 (br d, *J* = 2.5 Hz, 2H), 4.33 (br d, *J* = 3.6 Hz, 2H), 3.61 (br s, 9H), 3.25 (br s, 2H), 1.23 (t, *J* = 7.1 Hz, 3H); LCMS (Method 1): m/z = 450.2 $[M + H]^+$ | 23.5 | 2.50 |
| 120 | 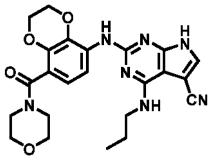 | 2-((8-(morpholine-4-carbonyl)-2,3-dihydrobenzo[*b*][1 ,4]dioxin-5-yl)amino)-4-(propylamino)-7*H*-pyrrolo[2,3-*d*]pyrimidine-5-carbonitrile | $^1$H NMR (400 MHz, DMSO-$d_6$): δ = 12.34 (br s, 1H), 8.07 (d, *J* = 8.6 Hz, 1H), 7.89 (d, *J* = 2.3 Hz, 1H), 7.55 (br s, 1H), 6.77 (d, *J* = 8.6 Hz, 1H), 6.53 (br s, 1H), 4.48 - 4.28 (m, 4H), 3.63 - 3.62 (m, 2H), 3.58 - 3.45 (m, 6H), 3.25 (br s, 2H), 1.70 - 1.58 (m, 2H), 0.95 (t, *J* = 7.4 Hz, 3H); LCMS (Method 2): m/z = 464.1 $[M + H]^+$ | 6.8 | 2.62 |
| 121 | 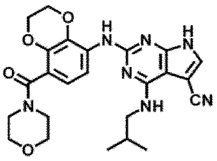 | 4-(isobutylamino)-2-((8-(morpholine-4-carbonyl)-2,3-dihydrobenzo[*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.34 (br s, 1H), 8.05 (d, *J* = 8.4 Hz, 1H), 7.89 (d, *J* = 1.7 Hz, 1H), 7.56 (br s, 1H), 6.76 (d, *J* = 8.6 Hz, 1H), 6.44 (br s, 1H), 4.45 - 4.29 (m, 4H), 3.61 (br s, 4H), 3.53 (br s, 2H), 3.37 (t, *J* = 6.3 Hz, 2H), 3.25 (br s, 2H), 1.99 (td, *J* = 6.8, 13.5 Hz, 1H), 0.96 (d, *J* = 6.7 Hz, 6H); LCMS (Method 2): m/z = 478.1 $[M + H]^+$ | 11.5 | 2.36 |

(continued)

| Example | Structure | Compound name | $^1$H NMR, MS | Yield (%) | HPLC r.t. (min), Purity |
|---|---|---|---|---|---|
| 122 | 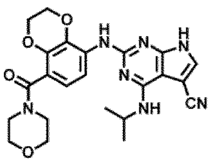 | 4-(isopropylamino)-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.35 (br s, 1H), 8.06 (d, *J* = 8.4 Hz, 1H), 7.89 (d, *J* = 2.4 Hz, 1H), 7.55 (br s, 1H), 6.78 (d, *J* = 8.6 Hz, 1H), 5.92 (br s, 1H), 4.43 - 4.28 (m, 5H), 3.61 (br s, 4H), 3.54 (br s, 2H), 3.25 (br s, 2H), 1.28 (d, *J* = 6.5 Hz, 6H); LCMS (Method 2): m/z = 464.1 [M + H]$^+$ | 21.6 | 2.67 |
| 123 | 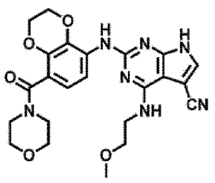 | 4-((2-methoxyethyl)ami no)-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.31 (br s, 1H), 8.06 (d, *J* = 8.4 Hz, 1H), 7.89 (s, 1H), 7.44 (s, 1H), 6.76 (d, *J* = 8.6 Hz, 1H), 6.28 (br t, *J* = 5.4 Hz, 1H), 4.38 (br s, 2H), 4.32 (br s, 2H), 3.69 (q, *J* = 5.4 Hz, 2H), 3.62 - 3.51 (m, 8H), 3.32 - 3.31 (m, 5H); LCMS (Method 1): m/z = 480.2 [M + H]$^+$ | 5.8 | 2.46 |
| 124 | 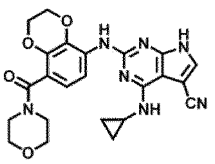 | 4-(cyclopropylamino )-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.23 (br d, *J* = 0.8 Hz, 1H), 8.18 (d, *J* = 8.5 Hz, 1H), 7.82 (s, 1H), 7.33 (s, 1H), 6.70 (d, *J* = 8.4 Hz, 1H), 6.52 (br s, 1H), 4.32 (m, 2H), 4.26 (m, 2H), 3.54 (br s, 4H), 3.47 (br s, 2H), 3.19 (br s, 2H), 2.85 (dt, *J* = 3.3, 6.9 Hz, 1H), 0.81 - 0.71 (m, 2H), 0.61 - 0.51 (m, 2H); LCMS (Method 2): m/z = 462.2 [M + H]$^+$ | 4.6 | 2.65 |

(continued)

| Example | Structure | Compound name | $^1$H NMR, MS | Yield (%) | HPLC r.t. (min), Purity |
|---|---|---|---|---|---|
| 125 | 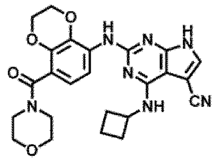 | 4-(cyclobutylamino) -2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.27 (br s, 1H), 8.09 (d, *J* = 8.4 Hz, 1H), 7.88 (s, 1H), 7.40 (s, 1H), 6.78 (d, *J* = 8.4 Hz, 1H), 6.36 (d, *J* = 7.3 Hz, 1H), 4.65 - 4.56 (m, 1H), 4.39 (br s, 2H), 4.33 (br d, *J* = 3.5 Hz, 2H), 3.66 - 3.51 (m, 6H), 3.26 (br s, 2H), 2.39 - 2.29 (m, 2H), 2.14 - 2.03 (m, 2H), 1.80 - 1.67 (m, 2H); LCMS (Method 1): m/z = 476.2 [M + H]$^+$ | 19.5 | 2.78 |
| 126 | 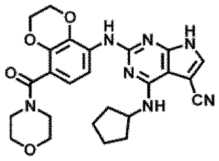 | 4-(cyclopentylamino )-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.28 (br s, 1H), 8.08 (d, *J* = 8.4 Hz, 1H), 7.86 (s, 1H), 7.40 (s, 1H), 6.75 (d, *J* = 8.5 Hz, 1H), 5.92 (d, *J* = 7.0 Hz, 1H), 4.48 - 4.39 (m, 1H), 4.37 (d, *J* = 2.5 Hz, 2H), 4.31 ( d, *J* = 3.6 Hz, 2H), 3.60 ( s, 4H), 3.52 ( s, 2H), 3.24 ( d, *J* = 1.3 Hz, 2H), 2.12 - 1.98 (m, 2H), 1.77 - 1.68 (m, 2H), 1.66 - 1.51 (m, 4H); LCMS (Method 2): m/z = 490.2 [M + H]$^+$ | 11.1 | 2.86 |

(continued)

| Example | Structure | Compound name | $^1$H NMR, MS | Yield (%) | HPLC r.t. (min), Purity |
|---|---|---|---|---|---|
| 127 | 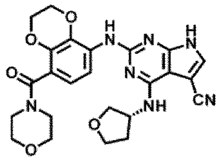 | (*R*)-2-((8-(morpholine-4-carbonyl)-2,3-dihydrobenzo [*b*][1 ,4]dioxin-5-yl)amino)-4-((tetrahydrofuran-3-yl) amino)-7*H*-pyrrolo[2,3-*d*] pyrimidine-5-carbonitrile | $^1$H NMR (400 MHz, DMSO-$d_6$): δ = 12.39 - 12.22 (m, 1H), 8.01 (d, *J* = 8.4 Hz, 1H), 7.87 (s, 1H), 7.46 (s, 1H), 6.75 (d, *J* = 8.5 Hz, 1H), 6.22 (d, *J* = 6.4 Hz, 1H), 4.71 - 4.54 (m, 1H), 4.35 (br d, *J* = 2.9 Hz, 2H), 4.30 (br d, *J* = 3.6 Hz, 2H), 3.94 (dd, *J* = 5.9, 8.9 Hz, 1H), 3.90 - 3.82 (m, 1H), 3.74 (dt, *J* = 6.0, 8.2 Hz, 1H), 3.66 - 3.55 (m, 5H), 3.51 (br d, *J* = 2.1 Hz, 2H), 3.27 - 3.13 (m, 2H), 2.30 - 2.22 (m, 1H), 2.04 - 1.85 (m, 1H); LCMS (Method 1): m/z = 492.2 [M + H]$^+$ | 26.1 | 2.54 |
| 128 | 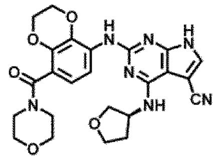 | (S)-2-((8-(morpholine-4-carbonyl)-2,3-dihydrobenzo [*b*][1 ,4]dioxin-5-yl)amino)-4-((tetrahydrofuran-3-yl) amino)-7*H*-pyrrolo[2,3-*d*] pyrimidine-5-carbonitrile | $^1$H NMR (400 MHz, DMSO-$d_6$): δ = 12.42 - 12.19 (m, 1H), 8.03 (d, *J* = 8.5 Hz, 1H), 7.89 (s, 1H), 7.48 (s, 1H), 6.77 (d, *J* = 8.5 Hz, 1H), 6.24 (d, *J* = 6.5 Hz, 1H), 4.72 - 4.58 (m, 1H), 4.40 - 4.29 (m, 4H), 3.96 (dd, *J* = 6.0, 8.9 Hz, 1H), 3.91 - 3.85 (m, 1H), 3.76 (dt, *J* = 5.9, 8.2 Hz, 1H), 3.66 - 3.63 (m, 1H), 3.62 - 3.57 (m, 4H), 3.53 (br s, 2H), 3.31 (br s, 2H), 3.28 - 3.18 (m, 2H), 2.31 - 2.25 (m, 1H), 2.00 - 1.87 (m, 1H); LCMS (Method 1): m/z = 492.2 [M + H]$^+$ | 22.6 | 2.54 |

(continued)

| Example | Structure | Compound name | $^1$H NMR, MS | Yield (%) | HPLC r.t. (min), Purity |
|---|---|---|---|---|---|
| 129 | 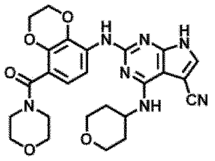 | 2-((8-(morpholine-4-carbonyl)-2,3-dihydrobenzo [*b*][1 ,4]dioxin-5-yl)amino)-4-((tetrahydro-2*H*-pyran-4-yl) amino)-7*H*-pyrrolo[2,3-*d*] pyrimidine-5-carbonitrile | $^1$H NMR (400 MHz, DMSO-$d_6$): δ = 12.29 (br s, 1H), 8.06 (d, *J* = 8.4 Hz, 1H), 7.89 (s, 1H), 7.44 (s, 1H), 6.77 (d, *J* = 8.4 Hz, 1H), 6.02 (d, *J* = 7.6 Hz, 1H), 4.38 (br d, *J* = 2.4 Hz, 2H), 4.32 (br d, *J* = 3.5 Hz, 2H), 4.29 - 4.18 (m, 1H), 3.96 - 3.88 (m, 2H), 3.61 (br s, 4H), 3.57 - 3.44 (m, 4H), 3.25 (br s, 2H), 1.98 (br dd, *J* = 1.9, 12.3 Hz, 2H), 1.69 - 1.57 (m, 2H); LCMS (Method 1): m/z = 506.2 [M + H]$^+$ | 16.8 | 2.47 |
| 130 | 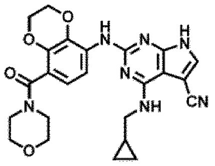 | 4-((cyclopropylmeth yl) amino)-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.14 - 11.90 (m, 1H), 7.83 (d, *J* = 8.6 Hz, 1H), 7.64 (s, 1H), 7.16 (s, 1H), 6.51 (s, 1H), 6.10 (t, *J* = 5.6 Hz, 1H), 4.20 - 4.04 (m, 4H), 3.37 (br s, 4H), 3.29 (br s, 2H), 3.15 (t, *J* = 6.2 Hz, 2H), 3.02 (br d, *J* = 11.4 Hz, 2H), 1.06 - 0.87 (m, 1H), 0.28 - 0.18 (m, 2H), 0.13 - 0.01 (m, 2H); LCMS (Method 1): m/z = 476.2 [M + H]$^+$ | 22.9 | 2.73 |
| 131 | 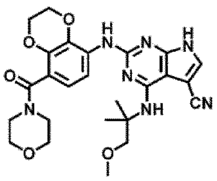 | 4-((1-methoxy-2-methylpropan-2-yl)amino)-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.38 - 12.17 (m, 1H), 7.90 (d, *J* = 8.4 Hz, 1H), 7.85 (s, 1H), 7.53 (s, 1H), 6.76 (d, *J* = 8.4 Hz, 1H), 5.77 (s, 1H), 4.40 - 4.29 (m, 4H), 3.61 (br s, 4H), 3.54 (br s, 2H), 3.49 (s, 2H), 3.34 (br s, 3H), 3.25 (br dd, *J* = 2.4, 6.9 Hz, 2H), 1.47 (s, 6H); LCMS (Method 1): m/z = 508.2 [M + H]$^+$ | 13.1 | 2.82 |

(continued)

| Example | Structure | Compound name | $^1$H NMR, MS | Yield (%) | HPLC r.t. (min), Purity |
|---|---|---|---|---|---|
| 132 | 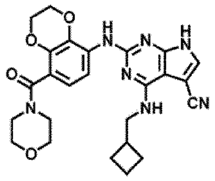 | 4-((cyclobutylmethyl )amino)-2-((8-(morpholine-4-carbonyl)-2,3-dihydrobenzo [*b*][1 ,4]dioxin-5-yl)amino)-7H-pyrrolo[2,3-*d*]pyrimidine-5-carbonitrile | $^1$H NMR (400 MHz, DMSO-$d_6$): δ = 12.29 (br s, 1H), 8.08 (d, *J* = 8.4 Hz, 1H), 7.88 (s, 1H), 7.40 (s, 1H), 6.76 (d, *J* = 8.4 Hz, 1H), 6.28 (t, *J* = 5.6 Hz, 1H), 4.50 - 4.26 (m, 4H), 3.61 (br s, 4H), 3.58 - 3.50 (m, 4H), 3.31 - 3.18 (m, 2H), 2.70 - 2.62 (m, 1H), 2.08 - 1.95 (m, 2H), 1.92 - 1.71 (m, 4H); LCMS (Method 1): m/z = 490.2 $[M + H]^+$ | 40.5 | 2.85 |
| 133 | 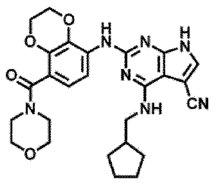 | 4-((cyclopentylmeth yl) amino)-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.29 (br s, 1H), 8.09 (d, *J* = 8.4 Hz, 1H), 7.88 (s, 1H), 7.40 (s, 1H), 6.75 (d, *J* = 8.6 Hz, 1H), 6.29 (t, *J* = 5.4 Hz, 1H), 4.38 (br d, *J* = 2.8 Hz, 2H), 4.32 (br d, *J* = 3.5 Hz, 2H), 3.61 (br s, 4H), 3.54 (br s, 2H), 3.46 (br t, *J* = 6.4 Hz, 2H), 3.30 - 3.15 (m, 2H), 2.31 - 2.23 (m, 1H), 1.77 - 1.68 (m, 2H), 1.66 - 1.58 (m, 2H), 1.58 - 1.46 (m, 2H), 1.41 - 1.19 (m, 2H); LCMS (Method 2): m/z = 504.2 [M + H]+ | 26.8 | 2.96 |

(continued)

| Example | Structure | Compound name | $^1$H NMR, MS | Yield (%) | HPLC r.t. (min), Purity |
|---|---|---|---|---|---|
| 134 | 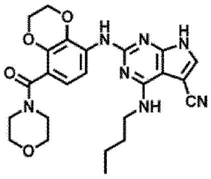 | 4-(butylamino)-2-((8-(morpholine-4-carbonyl)-2,3-dihydrobenzo[*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.67 - 11.74 (m, 1H), 8.10 (d, *J* = 8.4 Hz, 1H), 7.87 (s, 1H), 7.37 (s, 1H), 6.75 (d, *J* = 8.5 Hz, 1H), 6.34 (t, *J* = 5.7 Hz, 1H), 4.38 (br d, *J* = 2.4 Hz, 2H), 4.33 (br d, *J* = 3.6 Hz, 2H), 3.61 (br s, 4H), 3.58 - 3.45 (m, 4H), 3.26 (br dd, *J* = 2.7, 5.3 Hz, 2H), 1.62 (quin, *J* = 7.3 Hz, 2H), 1.39 (qd, *J* = 7.4, 14.9 Hz, 2H), 0.94 (t, *J* = 7.3 Hz, 3H); LCMS (Method 2): m/z = 478.2 [M + H]$^+$ | 33.4 | 2.82 |
| 135 | 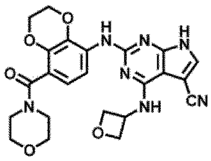 | 2-((8-(morpholine-4-carbonyl)-2,3-dihydrobenzo[*b*][1 ,4]dioxin-5-yl)amino)-4-(oxetan-3-ylamino)-7*H*-pyrrolo[2,3-*d*]pyrimidine-5-carbonitrile | $^1$H NMR (400 MHz, DMSO-$d_6$): δ = 12.26 - 11.82 (m, 1H), 8.00 (br s, 1H), 7.52 (s, 1H), 7.21 (br d, *J* = 7.8 Hz, 1H), 6.77 (d, *J* = 8.3 Hz, 1H), 4.86 (br s, 1H), 4.42 - 4.20 (m, 5H), 4.11 - 3.96 (m, 1H), 3.96 - 3.83 (m, 1H), 3.66 - 3.54 (m, 6H), 3.44 (td, *J* = 5.5, 10.7 Hz, 1H), 3.24 (br s, 2H); LCMS (Method 2): m/z = 478.2 [M + H]$^+$ | 38.6 | 2.01 |

(continued)

| Example | Structure | Compound name | $^1$H NMR, MS | Yield (%) | HPLC r.t. (min), Purity |
|---|---|---|---|---|---|
| 136 | 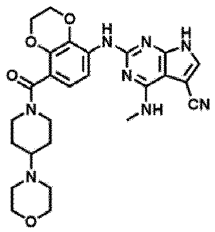 | 4-(methylamino)-2-((8-(4-morpholinopiperid ine-1-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.07 (br d, *J* = 2.2 Hz, 1H), 8.61 (d, *J* = 8.8 Hz, 1H), 7.84 - 7.77 (m, 1H), 7.63 (br s, 1H), 7.60 (s, 1H), 7.50 - 7.45 (m, 1H), 7.24 (dd, *J* = 2.6, 4.3 Hz, 2H), 5.21 (d, *J* = 6.0 Hz, 1H), 4.55 - 4.27 (m, 1H), 4.22 - 4.10 (m, 2H), 4.05 (s, 1H), 3.95 (s, 3H), 3.91 - 3.80 (m, 1H), 3.60 (br s, 4H), 3.15 (d, *J* = 4.8 Hz, 1H), 2.33 (d, *J* = 1.7 Hz, 4H), 2.01 (d, *J* = 9.3 Hz, 2H), 1.71 (br dd, *J* = 4.2, 9.0 Hz, 2H), 1.61 (br dd, *J* = 4.2, 8.6 Hz, 1H), 1.48 - 1.30 (m, 4H); LCMS (Method 2): m/z = 519.2 [M+H]$^+$ | 32.3 | 2.30 |
| 137 | 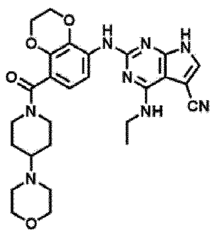 | 4-(ethylamino)-2-((8-(4-morpholinopiperid ine-1-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$) δ = 12.27 (br s, 1H), 8.06 (d, *J* = 8.5 Hz, 1H), 7.86 (s, 1H), 7.37 (s, 1H), 6.77 - 6.65 (m, 1H), 6.39 (t, *J* = 5.7 Hz, 1H), 4.50 - 4.41 (m, 1H), 4.41 - 4.25 (m, 4H), 3.55 (br d, *J* = 6.6 Hz, 4H), 3.53 - 3.47 (m, 3H), 3.04 - 2.85 (m, 1H), 2.73 (br t, *J* = 12.1 Hz, 1H), 2.45 (br s, 4H), 2.37 (br d, *J* = 10.5 Hz, 1H), 1.84 (br d, *J* = 12.5 Hz, 1H), 1.76 - 1.65 (m, 1H), 1.45 - 1.24 (m, 2H), 1.21 (t, *J* = 7.1 Hz, 3H); LCMS (Method 1): m/z = 533.3 [M + H]$^+$ | 25.5 | 2.17 |

(continued)

| Example | Structure | Compound name | $^1H$ NMR, MS | Yield (%) | HPLC r.t. (min), Purity |
|---|---|---|---|---|---|
| 138 | 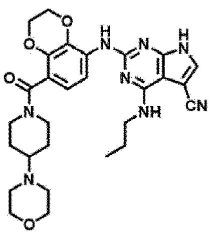 | 2-((8-(4-morpholinopiperid ine-1-carbonyl)-2,3-dihydrobenzo[*b*][1 ,4]dioxin-5-yl)amino)-4-(propylamino)-7*H*-pyrrolo[2,3-*d*]pyrimidine-5-carbonitrile | $^1H$ NMR (400 MHz, DMSO-$d_6$) δ = 12.34 - 12.15 (m, 1H), 8.05 (d, *J* = 8.5 Hz, 1H), 7.86 (s, 1H), 7.37 (s, 1H), 6.78 - 6.65 (m, 1H), 6.36 (t, *J* = 5.6 Hz, 1H), 4.50 - 4.41 (m, 1H), 4.40 - 4.26 (m, 4H), 3.56 (br s, 4H), 3.51 - 3.42 (m, 3H), 3.07 - 2.84 (m, 1H), 2.73 (br t, *J* = 11.9 Hz, 1H), 2.45 (br s, 4H), 2.40 - 2.34 (m, 1H), 1.84 (br d, *J* = 11.4 Hz, 1H), 1.77-1.69 (m, 1H), 1.68 - 1.61 (m, 2H), 1.39 - 1.16 (m, 2H), 0.94 (t, *J* = 7.4 Hz, 3H); LCMS (Method 1): m/z = 547.3 [M + H]$^+$ | 12.8 | 2.27 |
| 139 | 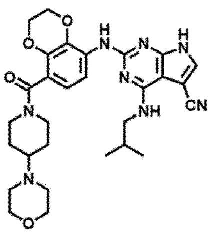 | 4-(isobutylamino)-2-((8-(4-morpholinopiperid ine-1-carbonyl)-2,3-dihydrobenzo [*b*][1 ,4]dioxin-5-yl)amino)-7*H*-pyrrolo[2,3-*d*]pyrimidine-5-carbonitrile | $^1H$ NMR (400 MHz, DMSO-$d_6$) δ = 12.27 (br s, 1H), 8.03 (d, *J* = 8.5 Hz, 1H), 7.87 (s, 1H), 7.38 (s, 1H), 6.78 - 6.61 (m, 1H), 6.28 (t, *J* = 5.6 Hz, 1H), 4.50 - 4.40 (m, 1H), 4.38 - 4.24 (m, 4H), 3.56 (br s, 4H), 3.37 - 3.35 (m, 2H), 3.06 - 2.85 (m, 1H), 2.73 (br t, *J* = 12.1 Hz, 1H), 2.45 (br s, 4H), 2.42 - 2.35 (m, 1H), 2.35 - 2.31 (m, 1H), 2.04 - 1.91 (m, 1H), 1.84 (br d, *J* = 11.3 Hz, 1H), 1.77 - 1.65 (m, 1H), 1.45 - 1.12 (m, 2H), 0.94 (d, *J* = 6.8 Hz, 6H); LCMS (Method 1): m/z = 561.3, [M + H]$^+$ | 9.7 | 2.37 |

(continued)

| Example | Structure | Compound name | $^1$H NMR, MS | Yield (%) | HPLC r.t. (min), Purity |
|---|---|---|---|---|---|
| 140 | 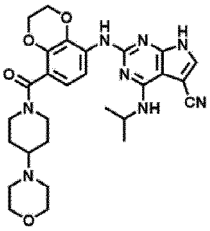 | 4-(isopropylamino)-2-((8-(4-morpholinopiperid ine-1-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 8.11 (br d, *J* = 8.4 Hz, 1H), 7.90 - 7.69 (m, 1H), 7.41 - 7.30 (m, 1H), 6.82 - 6.58 (m, 1H), 5.63 (br d, *J* = 7.1 Hz, 1H), 4.57 - 4.22 (m, 6H), 3.62 - 3.47 (m, 6H), 3.05 - 2.86 (m, 1H), 2.74 (br t, *J* = 12.1 Hz, 1H), 2.46 (br s, 4H), 2.41 - 2.35 (m, 1H), 1.84 (br d, *J* = 11.5 Hz, 1H), 1.72 (br t, *J* = 16.7 Hz, 1H), 1.27 (d, *J* = 6.5 Hz, 6H); LCMS (Method 2): m/z = 547.3 [M + H]$^+$ | 8.6 | 2.70 |
| 141 | 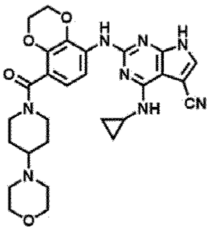 | 4-(cyclopropylamino )-2-((8-(4-morpholinopiperid ine-1-carbonyl)-2,3-dihydrobenzo[*b*][1,4]dioxin-5-yl)amino)-7*H*-pyrrolo[2,3-*d*] pyrimidine-5-carbonitrile | $^1$H NMR (400 MHz, DMSO-$d_6$): δ = 12.50 - 11.94 (m, 1H), 8.20 (d, *J* = 8.5 Hz, 1H), 7.89 (s, 1H), 7.38 (s, 1H), 6.79 - 6.67 (m, 1H), 6.58 (br s, 1H), 4.49 - 4.41 (m, 1H), 4.40 - 4.26 (m, 4H), 3.56 (br s, 4H), 3.49 (br s, 1H), 2.91 (dt, *J* = 3.2, 6.9 Hz, 1H), 2.77 - 2.69 (m, 1H), 2.52 (d, *J* = 1.9 Hz, 2H), 2.45 (br s, 4H), 1.91 - 1.78 (m, 1H), 1.77 - 1.65 (m, 1H), 1.43 - 1.14 (m, 2H), 0.87 - 0.79 (m, 2H), 0.67 - 0.59 (m, 2H); LCMS (Method 1): m/z = 545.25 [M + H]$^+$ | 17.9 | 2.16 |

(continued)

| Example | Structure | Compound name | $^1$H NMR, MS | Yield (%) | HPLC r.t. (min), Purity |
|---|---|---|---|---|---|
| 142 | 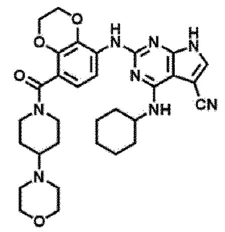 | 4-(cyclohexylamino) -2-((8-(4-morpholinopiperid ine-1-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): $\delta$ = 12.27 (br s, 1H), 8.03 (br d, *J* = 8.5 Hz, 1H), 7.87 (d, *J* = 1.6 Hz, 1H), 7.41 (s, 1H), 6.78 - 6.65 (m, 1H), 5.79 (d, *J* = 7.6 Hz, 1H), 4.52 - 4.43 (m, 1H), 4.40 - 4.27 (m, 4H), 4.05 (br dd, *J* = 2.9, 7.4 Hz, 1H), 3.61 - 3.50 (m, 5H), 3.07 - 2.86 (m, 1H), 2.80 - 2.70 (m, 1H), 2.47 - 2.38 (m, 4H), 2.00 (br s, 2H), 1.90 - 1.80 (m, 1H), 1.73 (br s, 3H), 1.64 - 1.57 (m, 1H), 1.49 - 1.13 (m, 8H); LCMS (Method 1): m/z = 587.3 [M + H]$^+$ | 32.3 | 2.16 |
| 143 | 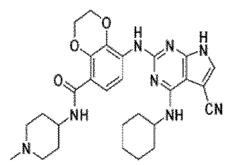 | 8-((5-cyano-4-(cyclohexylamino) -7*H*-pyrrolo[2,3-*d*]pyrimidin-2-yl)amino)-*N*-(1-methylpiperidin-4-yl)-2,3-dihydrobenzo[*b*][1 ,4]dioxin-5-carboxamide | $^1$H NMR (400 MHz, DMSO-$d_4$,) $\delta$ 8.18 (d, J = 8.00 Hz, 1H), 7.88 (s, 1H), 7.72 (d, J = 8.00 Hz, 1H), 7.43 (s, 1H), 7.35 (d, J = 12.0 Hz, 1H), 5.82 (d, J = 8.00 Hz, 1H), 4.45-4.40 (m, 4H), 4.05-4.04 (m, 1H), 3.76-3.74 (m, 1H), 2.68-2.65 (m, 2H), 2.16 (s, 3H), 2.05-2.00 (m, 5H), 1.83-1.74 (m, 4H), 1.64-1.61 (m, 1H), 1.58-1.48 (m, 2H), 1.41-1.36 (m, 4H); LCMS (Method 7): m/z = 531.3 [M + H]$^+$ | 33 | 1.34 |

(continued)

| Example | Structure | Compound name | $^1$H NMR, MS | Yield (%) | HPLC r.t. (min), Purity |
|---|---|---|---|---|---|
| 144 | 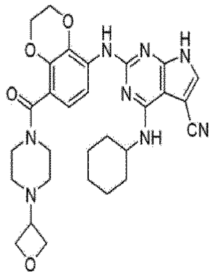 | 4-(cyclohexylamino) -2-((8-(4-(oxetan-3-yl) piperazine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_4$,) δ 8.08 (d, J = 8.0 Hz, 1H), 7.84 (s, 1H), 7.37 (s, 1H), 6.72 (d, J = 8.0 Hz, 1H), 5.73 (d, J = 8.0 Hz, 1H), 4.55-4.52 (m, 2H), 4.45-4.42 (m, 2H), 4.37-4.36 (m, 2H), 4.31-4.30 (m, 2H), 4.05-4.04 (m, 1H), 3.44-3.39 (m, 1H), 3.27-3.25 (m, 4H), 2.33-2.18 (m, 4H), 2.01-1.99 (m, 2H) 1.75-1.73 (m, 2H), 1.63-1.59 (m, 1H), 1.45-1.34 (m, 5H); LCMS (Method 7): m/z = 559.3 [M + H]$^+$ | 26 | 1.33 |
| 145 | 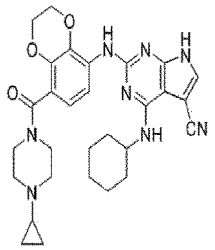 | 4-(cyclohexylamino) -2-((8-(4-cyclopropylpipera zine-1-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_4$,) δ 8.06 (d, J = 8.0 Hz, 1H), 7.86 (s, 1H), 7.39 (s, 1H), 6.72 (d, J = 8.0 Hz, 1H), 5.77 (d, J = 8.0 Hz, 1H), 4.37-4.29 (m, 4H), 4.04 (s, 1H), 3.57-3.52 (m, 3H), 3.19 - 3.17 (m, 2H), 2.56-2.44 (m, 4H), 2.00-1.97(m, 2H), 1.75-1.73 (m, 2H), 1.65-1.63 (m, 2H), 1.42-1.37 (m, 4H), 0.43-0.42 (m, 2H), 0.32-0.31 (m, 2H); LCMS (Method 7): m/z = 543.3 [M + H]$^+$ | 29 | 1.33 |

(continued)

| Example | Structure | Compound name | $^1$H NMR, MS | Yield (%) | HPLC r.t. (min), Purity |
|---|---|---|---|---|---|
| 146 | 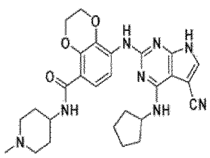 | 8-((5-cyano-4-(cyclopentylamino )-7*H*-pyrrolo[2,3-*d*]pyrimidin-2-yl)amino)-*N*-(1-methylpiperidin-4-yl)-2,3-dihydrobenzo[*b*][1 ,4]dioxin-5-carboxamide | $^1$H NMR (400 MHz, DMSO-$d_4$,) δ 8.20 (d, J = 8.0 Hz, 1H), 7.89 (s, 1H), 7.73 (d, J = 8.0 Hz, 1H), 7.44 (s, 1H), 7.35 (d, J = 8.0 Hz, 1H), 5.97 (d, J = 8.0 Hz, 1H), 4.45-4.42 (m, 5H), 3.75-3.74 (m, 1H), 2.68-2.65 (m, 2H), 2.16 (s, 3H), 2.08-2.04 (m, 4H), 1.82-1.78 (m, 2H), 1.74-1.71 (m, 2H), 1.64-1.52 (m, 2H); LCMS (Method 7): m/z = 517.3 [M + H]$^+$ | 31 | 1.25 |
| 147 | 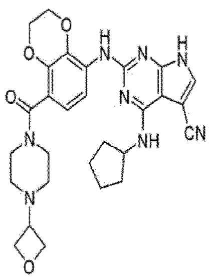 | 4-(cyclopentylamino )-2-((8-(4-(oxetan-3-yl) piperazine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_4$,) δ 8.10 (d, J = 8.00 Hz, 1H), 7.86 (s, 1H), 7.39 (s, 1H), 6.74 (d, J = 8.00 Hz, 1H), 5.91 (d, J = 8.00 Hz, 1H), 4.55-4.53 (m, 2H), 4.44-4.41 (m, 3H), 4.37-4.36 (m, 2H), 4.30-4.29 (m, 2H), 3.62-3.61 (m, 1H), 3.44-3.40 (m, 4H), 2.28-2.17 (m, 4H), 2.07-2.01 (m, 2H), 1.73-1.68 (m, 2H), 1.66-1.53 (m, 4H); LCMS (Method 7): m/z = 545.3 [M + H]$^+$ | 40 | 1.21 |

(continued)

| Example | Structure | Compound name | $^1H$ NMR, MS | Yield (%) | HPLC r.t. (min), Purity |
|---|---|---|---|---|---|
| 148 | 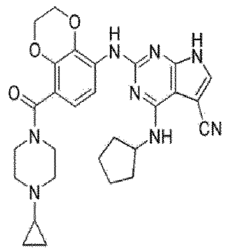 | 4-(cyclopentylamino )-2-((8-(4-cyclopropylpiperazine-1-carbonyl)-2,3-dihydrobenzo[*b*][1 ,4]dioxin-5-yl)amino)-7*H*-pyrrolo[2,3-*d*] pyrimidine-5-carbonitrile | $^1H$ NMR (400 MHz, DMSO-$d_4$,) δ 8.07 (d, J = 8.0 Hz, 1H), 7.87 (s, 1H), 7.41 (s, 1H), 6.73 (d, J = 8.0 Hz, 1H), 5.94 (d, J = 8.0 Hz, 1H), 4.46-4.41 (m, 2H), 4.37-4.29 (m, 4H), 3.56-3.52 (m, 2H), 3.18-3.17 (m, 2H), 2.54-2.49 (m, 4H), 2.06-2.03 (m, 2H), 1.72-1.71 (m, 2H), 1.65-1.53 (m, 4H), 0.43-0.42 (m, 2H), 0.32-0.31 (m, 2H); LCMS (Method 7): m/z = 529.3 $[M + H]^+$ | 28 | 1.22 |
| 149 | 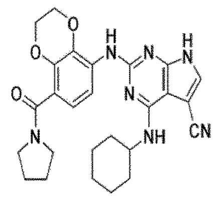 | 4-(cyclohexylamino) -2-((8-(pyrrolidine-1-carbonyl)-2,3-dihydrobenzo [*b*][1 ,4]dioxin-5-yl)amino)-7*H*-pyrrolo[2,3-*d*]pyrimidine-5-carbonitrile | $^1H$ NMR (400 MHz, Methanol-$d_4$) δ 8.28 (d, *J* = 8.6 Hz, 1H), 7.60 (s, 1H), 6.82 (d, *J* = 8.5 Hz, 1H), 4.44 (dd, *J* = 2.5, 5.4 Hz, 2H), 4.37 (dd, *J* = 2.4, 5.4 Hz, 2H), 4.22 - 4.09 (m, 1H), 3.58 (t, *J* = 6.9 Hz, 2H), 3.41 (t, *J* = 6.7 Hz, 2H), 2.21 - 2.10 (m, 2H), 1.95 - 1.80 (m, 4H), 1.78 - 1.65 (m, 1H), 1.59 - 1.33 (m, 6H), 0.94 - 0.87 (m, 1H); LCMS (Method 7): m/z = 488.2 [M + H] | 45 | 1.60 |

(continued)

| Example | Structure | Compound name | $^1H$ NMR, MS | Yield (%) | HPLC r.t. (min), Purity |
|---|---|---|---|---|---|
| 150 | 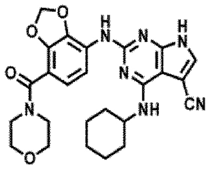 | 4-(cyclohexylamino) -2-((7-(morpholine-4-carbonyl)benzo[*d*] [1,3]dioxol-4-yl)amino)-7*H*-pyrrolo[2,3-*d*] pyrimidine-5-carbonitrile | $^1H$ NMR (400 MHz DMSO-$d_6$) δ 8.36 (s, 1H), 7.85 (d, *J* = 2.9 Hz, 1H), 7.50 (dd, *J* = 8.9, 2.5 Hz, 1H), 6.84 (dd, *J* = 8.8, 2.6 Hz, 1H), 6.06 (d, *J* = 2.5 Hz, 2H), 5.76 (s, 1H), 4.00 (s, 2H), 3.59 (s, 6H), 3.38 (s, 1H), 1.97 (d, *J* = 10.2 Hz, 2H), 1.73 (s, 2H), 1.60 (d, *J* = 12.2 Hz, 1H), 1.35 (q, *J* = 10.9 Hz, 4H), 1.25 (d, *J* = 16.4 Hz, 2H). LCMS (Method 7): m/z = 490.3 [M + H]$^+$ | 87 | 1.46 |
| 151 | 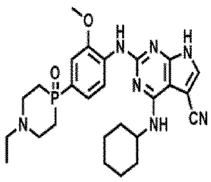 | 4-(cyclohexylamino) -2-((4-(1-ethyl-4-oxido-1,4-azaphosphinan-4-yl)-2-methoxyphenyl)a mino)-7*H*-pyrrolo[2,3-*d*]pyrimidine-5-carbonitrile | $^1H$ NMR (400 MHz, DMSO-$d_6$) δ 9.70 (d, *J* = 51.4 Hz, 1H), 8.78 - 8.69 (m, 1H), 7.94 (d, *J* = 3.0 Hz, 1H), 7.73 (s, 1H), 7.30 (d, *J* = 12.7 Hz, 2H), 5.94 (s, 1H), 5.04 (s, 1H), 4.00 (d, *J* = 7.9 Hz, 3H), 3.82 (dd, *J* = 26.5, 11.6 Hz, 2H), 3.58 (s, 2H), 2.38 - 2.20 (m, 2H), 2.02 (s, 2H), 1.75 (s, 3H), 1.63 (d, *J* = 12.3 Hz, 1H), 1.42 (q, *J* = 13.1, 11.2 Hz, 5H), 1.35 - 1.16 (m, 6H). LCMS (Method 7): m/z = 508.4 [M + H]$^+$ | 53.4 | 1.21 |
| 152 | 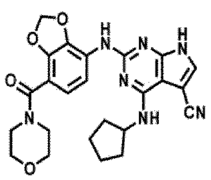 | 4-(cyclopentylamino )-2-((7-(morpholine-4-carbonyl) benzo[*d*] [1,3]dioxol-4-yl) amino)-7H-pyrrolo[2,3-*d*] pyrimidine-5-carbonitrile | $^1H$ NMR (400 MHz, DMSO-$d_6$) δ 8.35 (s, 1H), 7.84 (d, *J* = 3.5 Hz, 1H), 7.52 (dd, *J* = 8.7, 2.5 Hz, 1H), 6.84 (dd, *J* = 8.4, 2.5 Hz, 1H), 6.06 (d, *J* = 2.5 Hz, 2H), 5.94 (s, 1H), 4.40 (d, *J* = 7.8 Hz, 3H), 3.59 (s, 6H), 3.37 (s, 1H), 2.02 (s, 2H), 1.71 (s, 2H), 1.56 (s, 4H). LCMS (Method 7): m/z = 476.3 [M + H]$^+$ | 72.8 | 1.43 |

(continued)

| Example | Structure | Compound name | $^1$H NMR, MS | Yield (%) | HPLC r.t. (min), Purity |
|---|---|---|---|---|---|
| 153 | 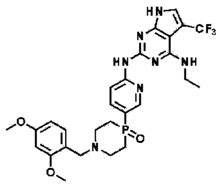 | 1-(2,4-dimethoxybenzyl)-4-(6-((4-(ethylamino)-5-(trifluoromethyl)-7*H*-pyrrolo[2,3-*d*]pyrimidin-2-yl)amino)pyridin-3-yl)-1,4-azaphosphinane 4-oxide | $^1$H NMR (400 MHz, Chloroform-*d*) δ 13.19 (s, 1H), 11.09 (s, 1H), 8.86 - 8.77 (m, 1H), 8.68 (dd, *J* = 6.4, 2.1 Hz, 1H), 7.96 (ddd, *J* = 11.0, 9.0, 2.2 Hz, 1H), 7.37 (t, *J* = 2.0 Hz, 1H), 7.24 (s, 1H), 6.54 - 6.43 (m, 2H), 5.40 (s, 1H), 3.82 (s, 3H), 3.82 (s, 3H), 3.68 (q, *J* = 5.1 Hz, 4H), 3.05 (dq, *J* = 22.8, 12.2, 11.6 Hz, 4H), 2.25 - 2.07 (m, 4H), 1.38 (t, *J* = 7.2 Hz, 3H). LCMS (Method 7): m/z = 590.3 [M + H]$^+$ | 48 | 1.20 |
| 154 | 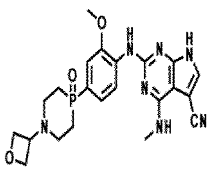 | 2-((2-methoxy-4-(1-(oxetan-3-yl)-4-oxido-1,4-azaphosphinan-4-yl)phenyl)amino)-4-(methylamino)-7*H*-pyrrolo[2,3-*d*]pyrimidine-5-carbonitrile | $^1$H NMR (400 MHz, DMSO-$d_6$): δ = 8.74 (dd, *J* = 3.1, 8.3 Hz, 1H), 7.87 (s, 1H), 7.61 (s, 1H), 7.43 - 7.23 (m, 3H), 6.47 (br d, *J* = 4.5 Hz, 1H), 4.59 - 4.52 (m, 2H), 4.44 (t, *J* = 6.1 Hz, 2H), 3.98 (s, 3H), 3.62 - 3.56 (m, 1H), 3.02 (d, *J* = 4.5 Hz, 3H), 2.73 - 2.54 (m, 4H), 2.31 - 2.22 (m, 2H), 1.93 - 1.81 (m, 2H); LCMS (Method 1): m/z = 467.2 (M + H)$^+$; | 3.4 | 2.14 |
| 155 | 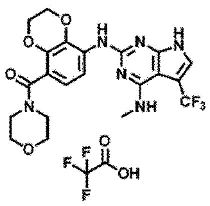 | (8-((4-(methylamino)-5-(trifluoromethyl)-7*H*-pyrrolo[2,3-*d*]pyrimidin-2-yl)amino)-2,3-dihydrobenzo[*b*][1 ,4]dioxin-5-yl)(morpholino)methanone 2,2,2-trifluoroacetate | $^1$H NMR (400 MHz, TFA-salt, DMSO-$d_6$): δ = 12.19 (br s, 1H), 8.11 (d, *J* = 8.6 Hz, 1H), 7.79 (br d, *J* = 6.7 Hz, 1H), 7.60 (s, 1H), 6.78 (d, *J* = 8.4 Hz, 1H), 6.25 (br s, 1H), 4.42 - 4.29 (m, 4H), 3.61 (br s, 4H), 3.53 (br s, 2H), 3.31 - 3.17 (m, 2H), 3.05 (d, *J* = 4.4 Hz, 3H); LCMS (Method 2): m/z = 479.1 [M + H]$^+$ | 5.8 | 2.54 |

(continued)

| Example | Structure | Compound name | $^1$H NMR, MS | Yield (%) | HPLC r.t. (min), Purity |
|---|---|---|---|---|---|
| 156 | 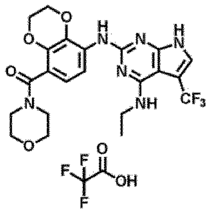 | (8-((4-(ethylamino)-5-(trifluoromethyl)-7*H*-pyrrolo [2,3-*d*]pyrimidin-2-yl)amino)-2,3-dihydrobenzo[*b*][1 ,4] dioxin-5-yl)(morpholino)me thanone 2,2,2-trifluoroacetate | $^1$H NMR (400 MHz, TFA-salt, DMSO-$d_6$): δ = 12.12 (br s, 1H), 8.08 (d, *J* = 8.4 Hz, 1H), 7.65 (br s, 1H), 7.59 (s, 1H), 6.78 (d, *J* = 8.5 Hz, 1H), 5.96 (br s, 1H), 4.44 - 4.27 (m, 4H), 3.66 - 3.61 (m, 8H), 3.25 (br s, 2H), 1.21 (t, *J* = 7.1 Hz, 3H); LCMS (Method 2): m/z = 493.2 [M + H]$^+$ | 10.1 | 2.36 |
| 157 | 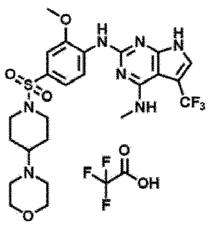 | $N^2$-(2-methoxy-4-((4-morpholinopiperid in-1-yl) sulfonyl)phenyl )-$N^4$-methyl-5-(trifluoromethyl)-7*H*-pyrrolo [2,3-*d*]pyrimidine-2,4-diamine 2,2,2-trifluoroacetate | $^1$H NMR (400 MHz, TFA-salt, DMSO-$d_6$): δ = 12.08 (d, *J* = 2.5 Hz, 1H), 10.27 - 9.80 (m, 1H), 8.88 (d, *J* = 8.6 Hz, 1H), 7.74 (s, 1H), 7.61 (s, 1H), 7.37 (dd, *J* = 1.9, 8.6 Hz, 1H), 7.24 (d, *J* = 1.9 Hz, 1H), 6.03 (br d, *J* = 4.5 Hz, 1H), 4.06 - 3.88 (m, 5H), 3.80 (br d, *J* = 11.8 Hz, 2H), 3.64 (br d, *J* = 2.0 Hz, 2H), 3.36 - 3.33 (m, 1H), 3.21 (br t, *J* = 10.4 Hz, 2H), 3.06 (d, *J* = 4.5 Hz, 5H), 2.26 (br t, *J* = 11.5 Hz, 2H), 2.12 (br d, *J* = 11.0 Hz, 2H), 1.73 - 1.61 (m, 2H); LCMS (Method 2): m/z = 570.2 [M + H]$^+$ | 33.8 | 2.27 |

(continued)

| Example | Structure | Compound name | 1H NMR, MS | Yield (%) | HPLC r.t. (min), Purity |
|---|---|---|---|---|---|
| 158 | 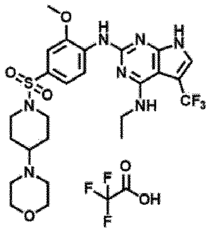 | $N^4$-ethyl-$N^2$-(2-methoxy-4-((4-morpholinopiperid in-1-yl)sulfonyl)phenyl )-5-(trifluoromethyl)-7*H*-pyrrolo [2,3-*d*]pyrimidine-2,4-diamine 2,2,2-trifluoroacetate | $^1$H NMR (400 MHz, TFA-salt, DMSO-$d_6$): δ = 12.08 (d, *J* = 2.3 Hz, 1H), 10.05 - 9.73 (m, 1H), 8.85 (d, *J* = 8.6 Hz, 1H), 7.73 (s, 1H), 7.63 (s, 1H), 7.37 (dd, *J* = 1.9, 8.6 Hz, 1H), 7.24 (d, *J* = 2.0 Hz, 1H), 5.89 - 5.80 (m, 1H), 4.03 - 3.95 (m, 5H), 3.82 (br s, 2H), 3.64 - 3.56 (m, 4H), 3.37 (br d, *J* = 11.5 Hz, 2H), 3.21 (br t, *J* = 10.5 Hz, 1H), 3.04 (br dd, *J* = 2.5, 4.5 Hz, 2H), 2.26 (br t, *J* = 11.7 Hz, 2H), 2.12 (br d, *J* = 11.5 Hz, 2H), 1.74 - 1.60 (m, 2H), 1.23 (t, *J* = 7.1 Hz, 3H); LCMS (Method 2): m/z = 584.1 [M + H]$^+$ | 4.98 | 2.38 |
| 159 | 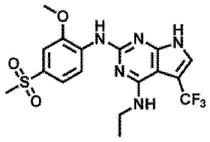 | $N^4$-ethyl-$N^2$-(2-methoxy-4-(methylsulfonyl)p henyl)-5-(trifluoromethyl)-7*H*-pyrrolo [2,3-*d*]pyrimidine-2,4-diamine | $^1$H NMR (400 MHz, DMSO-$d_6$): δ = 12.09 (br s, 1H), 8.82 (*d, J* = 8.6 Hz, 1H), 7.71 (s, 1H), 7.62 (s, 1H), 7.51 (dd, *J* = 1.9, 8.5 Hz, 1H), 7.45 (d, *J* = 1.8 Hz, 1H), 5.83 (br s, 1H), 4.01 (s, 3H), 3.60 (quin, *J* = 6.7 Hz, 2H), 3.19 (s, 3H), 1.23 (t, *J* = 7.1 Hz, 3H); LCMS (Method 2): m/z = 430.1 [M + H]$^+$ | 19.2 | 3.03 |
| 160 | 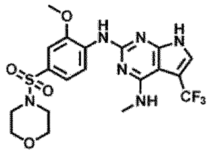 | $N^2$-(2-methoxy-4-(morpholinosulfon yl) phenyl)-$N^4$-methyl-5-(trifluoromethyl)-7*H*-pyrrolo [2,3-*d*]pyrimidine- 2,4-diamine | $^1$H NMR (400 MHz, DMSO-$d_6$): δ = 12.07 (br s, 1H), 8.88 (d, *J* = 8.6 Hz, 1H), 7.73 (s, 1H), 7.60 (s, 1H), 7.34 (dd, *J* = 1.9, 8.6 Hz, 1H), 7.20 (d, *J* = 2.0 Hz, 1H), 6.02 (br d, *J* = 4.3 Hz, 1H), 4.00 (s, 3H), 3.66 - 3.56 (m, 4H), 3.06 (d, *J* = 4.5 Hz, 3H), 2.96 - 2.83 (m, 4H); LCMS (Method 2): m/z = 487.1 [M + H]$^+$ | 4.70 | 2.99 |

(continued)

| Example | Structure | Compound name | $^1$H NMR, MS | Yield (%) | HPLC r.t. (min), Purity |
|---|---|---|---|---|---|
| 161 | 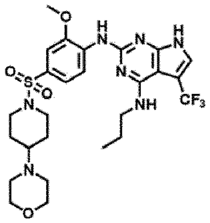 | $N^2$-(2-methoxy-4-((4-morpholinopiperid in-1-yl) sulfonyl)phenyl )-$N^4$-propyl-5-(trifluoromethyl)-7*H*-pyrrolo [2,3-*d*]pyrimidine-2,4-diamine | $^1$H NMR (400 MHz, DMSO-$d_6$): δ = 12.09 (br s, 1H), 8.80 (d, *J* = 8.6 Hz, 1H), 7.70 (s, 1H), 7.63 (s, 1H), 7.31 (dd, *J* = 2.0, 8.6 Hz, 1H), 7.20 (d, *J* = 2.0 Hz, 1H), 5.81 (br s, 1H), 3.99 (s, 3H), 3.65 (br d, *J* = 11.7 Hz, 2H), 3.56 - 3.48 (m, 6H), 2.38 (br s, 4H), 2.30 - 2.21 (m, 2H), 2.11 (br s, 1H), 1.80 (br d, *J* = 10.9 Hz, 2H), 1.65 (sxt, *J* = 7.3 Hz, 2H), 1.41 (br dd, *J* = 2.9, 11.4 Hz, 2H), 0.94 (t, *J* = 7.4 Hz, 3H); LCMS (Method 2): m/z = 598.2 [M + H]$^+$ | 4.91 | 3.29 |
| 162 | 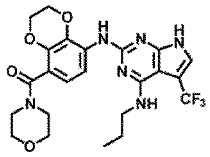 | morpholino (8-((4-(propylamino)-5-(trifluoromethyl)-7*H*-pyrrolo [2,3-*d*]pyrimidin-2-yl)amino)-2,3-dihydrobenzo[*b*][1 ,4] dioxin-5-yl)methanone | $^1$H NMR (400 MHz, DMSO-$d_6$): δ = 12.03 (br s, 1H), 8.12 (d, *J* = 8.4 Hz, 1H), 7.56 (d, *J* = 1.3 Hz, 1H), 7.35 (s, 1H), 6.75 (d, *J* = 8.6 Hz, 1H), 5.72 (br s, 1H), 4.44 - 4.23 (m, 4H), 3.70 - 3.44 (m, 8H), 3.31 - 3.17 (m, 2H), 1.63 (m, 2H), 0.97 - 0.87 (m, 3H); LCMS (Method 2): m/z = 507.2 [M + H]$^+$ | 13.5 | 3.05 |
| 163 | 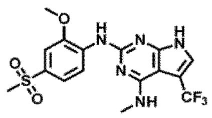 | $N^2$-(2-methoxy-4-(methylsulfonyl)p henyl)-$N^4$-methyl-5-(trifluoromethyl)-7*H*-pyrrolo [2,3-*d*]pyrimidine-2,4-diamine | $^1$H NMR (400 MHz, DMSO-$d_6$): δ = 12.08 (br d, *J* = 1.0 Hz, 1H), 8.85 (d, *J* = 8.6 Hz, 1H), 7.72 (s, 1H), 7.60 (d, *J* = 1.3 Hz, 1H), 7.51 (dd, *J* = 2.0, 8.6 Hz, 1H), 7.45 (d, *J* = 2.0 Hz, 1H), 6.01 (br d, *J* = 4.3 Hz, 1H), 4.01 (s, 3H), 3.19 (s, 3H), 3.05 (d, *J* = 4.5 Hz, 3H); LCMS (Method 2): m/z = 416.2 [M + H]$^+$ | 4.95 | 2.95 |

(continued)

| Example | Structure | Compound name | $^1$H NMR, MS | Yield (%) | HPLC r.t. (min), Purity |
|---|---|---|---|---|---|
| 164 | 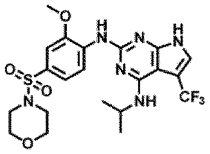 | *N*$^4$-isopropyl-*N*$^2$-(2-methoxy-4-(morpholinosulfon yl) phenyl)-5-(trifluoromethyl)-7*H*-pyrrolo[2,3-*d*]pyrimidine-2,4-diamine | $^1$H NMR (400 MHz, DMSO-$d_6$): δ = 12.12 (br s, 1H), 8.82 (d, *J* = 8.6 Hz, 1H), 7.75 (s, 1H), 7.65 (d, *J* = 1.5 Hz, 1H), 7.35 (dd, *J* = 2.0, 8.6 Hz, 1H), 7.21 (d, *J* = 2.0 Hz, 1H), 5.16 (br dd, *J* = 1.6, 7.5 Hz, 1H), 4.47 - 4.34 (m, 1H), 4.00 (s, 3H), 3.68 - 3.59 (m, 4H), 2.94 - 2.83 (m, 4H), 1.28 (d, *J* = 6.5 Hz, 6H); LCMS (Method 2): m/z = 515.2 [M + H]$^+$ | 16.3 | 3.39 |
| 165 | 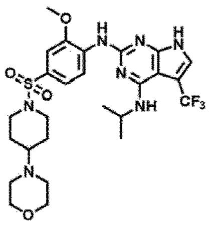 | *N*$^4$-isopropyl-*N*$^2$-(2-methoxy-4-((4-morpholinopiperid in-1-yl)sulfonyl)phenyl )-5-(trifluoromethyl)-7*H*-pyrrolo [2,3-*d*]pyrimidine-2,4-diamine | $^1$H NMR (400 MHz, DMSO-$d_6$): δ = 12.11 (br d, *J* = 2.0 Hz, 1H), 8.79 (d, *J* = 8.7 Hz, 1H), 7.72 (s, 1H), 7.65 (s, 1H), 7.34 (dd, *J* = 2.0, 8.6 Hz, 1H), 7.21 (d, *J* = 2.0 Hz, 1H), 5.20 - 5.10 (m, 1H), 4.47 - 4.35 (m, 1H), 3.99 (s, 3H), 3.69 - 3.62 (m, 2H), 3.51 (br s, 4H), 2.39 (br s, 4H), 2.31 - 2.22 (m, 2H), 2.12 (br s, 1H), 1.80 (br d, *J* = 12.2 Hz, 2H), 1.48 - 1.36 (m, 2H), 1.28 (d, *J* = 6.5 Hz, 6H); LCMS (Method 2): m/z = 598.2 [M + H]$^+$ | 16.7 | 3.33 |
| 166 | 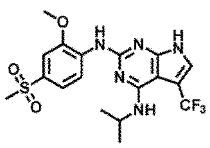 | *N*$^4$-isopropyl-*N*$^2$-(2-methoxy-4-(methylsulfonyl)p henyl)-5-(trifluoromethyl)-7*H*-pyrrolo [2,3-*d*]pyrimidine-2,4-diamine | $^1$H NMR (400 MHz, DMSO-$d_6$): δ = 12.13 (br d, *J* = 7.3 Hz, 1H), 8.80 (d, *J* = 8.6 Hz, 1H), 7.74 (s, 1H), 7.65 (d, *J* = 1.3 Hz, 1H), 7.51 (dd, *J* = 2.0, 8.6 Hz, 1H), 7.45 (d, *J* = 2.0 Hz, 1H), 5.26 - 5.05 (m, 1H), 4.49 - 4.36 (m, 1H), 4.01 (s, 3H), 3.19 (s, 3H), 1.28 (d, *J* = 6.5 Hz, 6H); LCMS (Method 2): m/z = 444.1 [M + H]$^+$ | 25.2 | 3.25 |

(continued)

| Example | Structure | Compound name | $^1H$ NMR, MS | Yield (%) | HPLC r.t. (min), Purity |
|---|---|---|---|---|---|
| 167 | 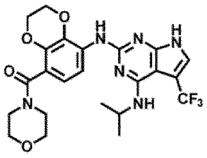 | (8-((4-(isopropylamino)-5-(trifluoromethyl)-7*H*-pyrrolo[2,3-*d*]pyrimidin-2-yl)amino)-2,3-dihydrobenzo[*b*][1 ,4]dioxin-5-yl)(morpholino)methanone | $^1H$ NMR (400 MHz, DMSO-$d_6$): δ = 12.34 - 11.74 (m, 1H), 8.11 (d, *J* = 8.6 Hz, 1H), 7.58 (d, *J* = 1.5 Hz, 1H), 7.38 (s, 1H), 6.77 (d, *J* = 8.6 Hz, 1H), 5.13 - 5.02 (m, 1H), 4.45 - 4.26 (m, 5H), 3.67 - 3.49 (m, 6H), 3.26 (br d, *J* = 5.1 Hz, 2H), 1.26 (d, *J* = 6.4 Hz, 6H); LCMS (Method 2): m/z = 507.2 $[M + H]^+$ | 25.1 | 3.09 |
| 168 | 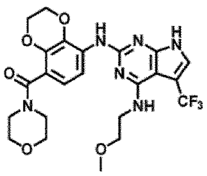 | (8-((4-((2-methoxyethyl)amino)-5-(trifluoromethyl)-7*H*-pyrrolo[2,3-*d*]pyrimidin-2-yl)amino)-2,3-dihydrobenzo[*b*][1 ,4]dioxin-5-yl)(morpholino)me thanone | $^1H$ NMR (400 MHz, DMSO-$d_6$): δ = 12.05 (br s, 1H), 8.10 (d, *J* = 8.5 Hz, 1H), 7.58 (d, *J* = 1.3 Hz, 1H), 7.40 (s, 1H), 6.76 (d, *J* = 8.5 Hz, 1H), 5.77 (br d, *J* = 1.0 Hz, 1H), 4.43 - 4.28 (m, 4H), 3.74 - 3.66 (m, 2H), 3.64 - 3.50 (m, 8H), 3.31 (s, 3H), 3.26 (br d, *J* = 2.5 Hz, 2H); LCMS (Method 2): m/z = 523.2 $[M + H]^+$ | 18.7 | 2.87 |
| 169 | 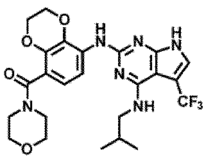 | (8-((4-(isobutylamino)-5-(trifluoromethyl)-7*H*-pyrrolo[2,3-*d*]pyrimidin-2-yl)amino)-2,3-dihydrobenzo[*b*][1 ,4]dioxin-5-yl)(morpholino)methanone | $^1H$ NMR (400 MHz, DMSO-$d_6$): δ = 12.01 (br s, 1H), 8.11 (d, *J* = 8.4 Hz, 1H), 7.57 (d, *J* = 1.1 Hz, 1H), 7.36 (s, 1H), 6.75 (d, *J* = 8.5 Hz, 1H), 5.64 (br s, 1H), 4.46 - 4.23 (m, 4H), 3.66 - 3.48 (m, 6H), 3.31 - 3.16 (m, 4H), 2.03 - 1.90 (m, 1H), 0.93 (d, *J* = 6.8 Hz, 6H); LCMS (Method 2): m/z = 521.2 $[M + H]^+$ | 15.6 | 3.20 |

(continued)

| Example | Structure | Compound name | $^1H$ NMR, MS | Yield (%) | HPLC r.t. (min), Purity |
|---|---|---|---|---|---|
| 170 | 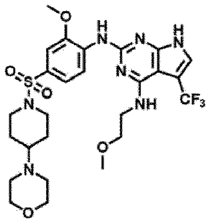 | *N*[2]-(2-methoxy-4-((4-morpholinopiperid in-1-yl) sulfonyl)phenyl )-*N*[4]-(2-methoxyethyl)-5-(trifluoromethyl)-7*H*-pyrrolo [2,3-*d*]pyrimidine-2,4-diamine | $^1H$ NMR (400 MHz, DMSO-$d_6$): δ = 12.12 (br s, 1H), 8.79 (d, *J* = 8.6 Hz, 1H), 7.74 (s, 1H), 7.65 (s, 1H), 7.32 (dd, *J* = 1.9, 8.6 Hz, 1H), 7.20 (d, *J* = 2.0 Hz, 1H), 5.86 (br d, *J* = 1.3 Hz, 1H), 3.99 (s, 3H), 3.73 (q, *J* = 5.4 Hz, 2H), 3.65 (br d, *J* = 11.4 Hz, 2H), 3.60 - 3.56 (m, 2H), 3.54 - 3.48 (m, 4H), 3.31 (s, 3H), 2.38 (br s, 4H), 2.30 - 2.22 (m, 2H), 2.16 - 2.06 (m, 1H), 1.80 (br d, *J* = 11.6 Hz, 2H), 1.47 - 1.35 (m, 2H); LCMS (Method 2): m/z = 614.2 [M + H]$^+$ | 15.5 | 3.05 |
| 171 | 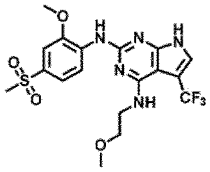 | *N*[2]-(2-methoxy-4-(methylsulfonyl)p henyl)-*N*[4]-(2-methoxyethyl)-5-(trifluoromethyl)-7*H*-pyrrolo [2,3-*d*]pyrimidine-2,4-diamine | $^1H$ NMR (400 MHz, DMSO-$d_6$): δ = 12.42 - 11.60 (m, 1H), 8.79 (d, *J* = 8.5 Hz, 1H), 7.75 (s, 1H), 7.65 (d, *J* = 1.5 Hz, 1H), 7.50 (dd, *J* = 2.0, 8.5 Hz, 1H), 7.45 (d, *J* = 2.0 Hz, 1H), 5.86 (br d, *J* = 1.3 Hz, 1H), 4.01 (s, 3H), 3.73 (q, *J* = 5.4 Hz, 2H), 3.61 - 3.54 (m, 2H), 3.31 (s, 3H), 3.19 (s, 3H); LCMS (Method 2): m/z = 460.1 [M + H]$^+$ | 19.2 | 2.93 |

(continued)

| Example | Structure | Compound name | $^1$H NMR, MS | Yield (%) | HPLC r.t. (min), Purity |
|---|---|---|---|---|---|
| 172 | 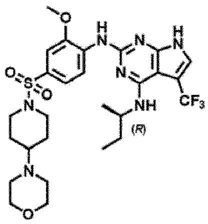 | (*R*)-*N*4-(*sec*-butyl)-*N*2-(2-methoxy-4-((4-morpholinopiperid in-1-yl) sulfonyl)phenyl )-5-(trifluoromethyl)-7*H*-pyrrolo [2,3-*d*]pyrimidine-2,4-diamine | $^1$H NMR (400 MHz, DMSO-$d_6$): δ = 12.13 (d, *J* = 2.6 Hz, 1H), 8.78 (d, *J* = 8.5 Hz, 1H), 7.76 - 7.62 (m, 2H), 7.33 (dd, *J* = 1.9, 8.5 Hz, 1H), 7.21 (d, *J* = 1.9 Hz, 1H), 5.12 (br dd, *J* = 1.6, 7.6 Hz, 1H), 4.27 (td, *J* = 6.7, 13.7 Hz, 1H), 3.99 (s, 3H), 3.65 (br d, *J* = 11.6 Hz, 2H), 3.51 (br s, 4H), 2.39 (br s, 3H), 2.31 - 2.21 (m, 2H), 2.17 - 2.06 (m, 1H), 1.80 (br d, *J* = 11.0 Hz, 2H), 1.62 (quin, *J* = 7.2 Hz, 2H), 1.49 - 1.35 (m, 2H), 1.25 (d, *J* = 6.5 Hz, 3H), 0.92 (t, *J* = 7.4 Hz, 3H); LCMS (Method 2): : m/z = 612.2 [M + H]$^+$ | 32.9 | 3.47 |
| 173 | 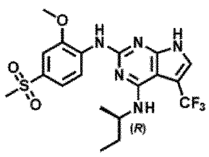 | (*R*)-*N*4-(*sec*-butyl)-*N*2-(2-methoxy-4-(methylsulfonyl)p henyl)-5-(trifluoromethyl)-7*H*-pyrrolo[2,3-*d*]pyrimidine-2,4-diamine | $^1$H NMR (400 MHz, DMSO-$d_6$): δ = 12.15 (br s, 1H), 8.79 (d, *J* = 8.5 Hz, 1H), 7.74 (s, 1H), 7.66 (s, 1H), 7.50 (dd, *J* = 2.0, 8.5 Hz, 1H), 7.45 (d, *J* = 2.0 Hz, 1H), 5.13 (br dd, *J* = 1.6, 7.7 Hz, 1H), 4.33 - 4.22 (m, 1H), 4.01 (s, 3H), 3.19 (s, 3H), 1.63 (m, 2H), 1.25 (d, *J* = 6.5 Hz, 3H), 0.93 (t, *J* = 7.4 Hz, 3H); LCMS (Method 2): m/z = 458.2 [M + H]$^+$ | 26.2 | 3.39 |

(continued)

| Example | Structure | Compound name | $^1$H NMR, MS | Yield (%) | HPLC r.t. (min), Purity |
|---|---|---|---|---|---|
| 174 | 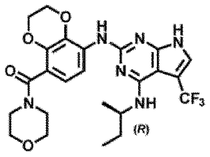 | (*R*)-(8-((4-( *sec*-butylamino)-5-(trifluoromethyl)-7*H*-pyrrolo [2,3-*d*]pyrimidin-2-yl)amino)-2,3-dihydrobenzo[*b*][1 ,4] dioxin-5-yl)(morpholino)methanone | $^1$H NMR (400 MHz, DMSO-$d_6$): δ = 12.05 (br s, 1H), 8.10 (d, *J* = 8.5 Hz, 1H), 7.59 (d, *J* = 1.3 Hz, 1H), 7.38 (s, 1H), 6.76 (d, *J* = 8.5 Hz, 1H), 5.18 - 4.92 (m, 1H), 4.43 - 4.29 (m, 4H), 4.23 (td, *J* = 6.7, 13.7 Hz, 1H), 3.70 - 3.44 (m, 6H), 3.29 - 3.19 (m, 2H), 1.60 (quin, *J* = 7.2 Hz, 2H), 1.22 (d, *J* = 6.5 Hz, 3H), 0.91 (t, *J* = 7.4 Hz, 3H); LCMS (Method 2): m/z = 521.2 [M + H]$^+$ | 7.98 | 3.23 |
| 175 | 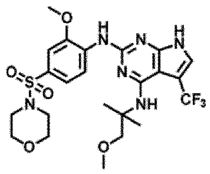 | *N*$^4$-(1-methoxy-2-methylpropan-2-yl)-*N*$^2$-(2-methoxy-4-(morpholinosulfonyl)phenyl)-5-(trifluoromethyl)-7*H*-pyrrolo[2,3-*d*]pyrimidine-2,4-diamine | $^1$H NMR (400 MHz, DMSO-$d_6$): δ = 12.12 (br s, 1H), 8.70 (d, *J* = 8.5 Hz, 1H), 7.79 (s, 1H), 7.63 (d, *J* = 1.3 Hz, 1H), 7.32 (dd, *J* = 2.0, 8.5 Hz, 1H), 7.21 (d, *J* = 2.0 Hz, 1H), 5.69 (br d, *J* = 1.9 Hz, 1H), 3.99 (s, 3H), 3.68 - 3.60 (m, 4H), 3.45 (s, 2H), 3.34 (s, 3H), 2.92 - 2.85 (m, 4H), 1.49 (s, 6H); LCMS (Method 2): m/z = 558.2 [M + H]$^+$ | 19.5 | 3.44 |

(continued)

| Example | Structure | Compound name | $^1$H NMR, MS | Yield (%) | HPLC r.t. (min), Purity |
|---|---|---|---|---|---|
| 176 | 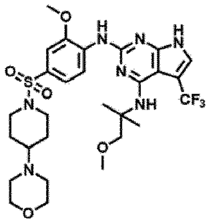 | *N*4-(1-methoxy-2-methylpropan-2-yl)-*N*2-(2-methoxy-4-((4-morpholinopiperid in-1-yl) sulfonyl)phenyl)-5-(trifluoromethyl)-7*H*-pyrrolo [2,3-*d*]pyrimidine-2,4-diamine | $^1$H NMR (400 MHz, DMSO-$d_6$): δ = 12.11 (d, *J* = 2.5 Hz, 1H), 8.68 (d, *J* = 8.5 Hz, 1H), 7.75 (s, 1H), 7.63 (s, 1H), 7.31 (dd, *J* = 1.9, 8.6 Hz, 1H), 7.21 (d, *J* = 1.9 Hz, 1H), 5.69 (br d, *J* = 2.0 Hz, 1H), 3.98 (s, 3H), 3.65 (br d, *J* = 11.6 Hz, 2H), 3.56 - 3.48 (m, 4H), 3.45 (s, 2H), 3.33 - 3.31 (m, 3H), 2.38 (br s, 4H), 2.25 (br t, *J* = 11.0 Hz, 2H), 2.16 - 2.05 (m, 1H), 1.80 (br d, *J* = 11.0 Hz, 2H), 1.49 (s, 6H), 1.46 - 1.36 (m, 2H); LCMS (Method 2): m/z = 642.2 [M + H]$^+$ | 12.7 | 3.39 |
| 177 | 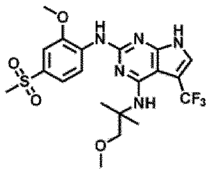 | *N*4-(1-methoxy-2-methylpropan-2-yl)-*N*2-(2-methoxy-4-(methylsulfonyl)p henyl)-5-(trifluoromethyl)-7*H*-pyrrolo[2,3-*d*]pyrimidine-2,4-diamine | $^1$H NMR (400 MHz, DMSO-$d_6$): δ = 12.14 (br s, 1H), 8.72 (d, *J* = 8.5 Hz, 1H), 7.76 (s, 1H), 7.63 (s, 1H), 7.52 - 7.44 (m, 2H), 5.70 (br d, *J* = 1.9 Hz, 1H), 4.01 (s, 3H), 3.45 (s, 2H), 3.35 (s, 3H), 3.20 (s, 3H), 1.51 (s, 6H); LCMS (Method 2): m/z = 488.2 [M + H]$^+$ | 19.5 | 3.32 |
| 178 | 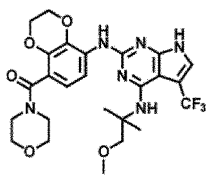 | (8-((4-((1-methoxy-2-methylpropan-2-yl)amino)-5-(trifluoromethyl)-7*H*-pyrrolo [2,3-*d*]pyrimidin-2-yl)amino)-2,3-dihydrobenzo[*b*][1 ,4] dioxin-5-yl)(morpholino)me thanone | $^1$H NMR (400 MHz, DMSO-$d_6$): δ = 12.00 (br s, 1H), 7.95 (d, *J* = 8.4 Hz, 1H), 7.55 (d, *J* = 1.3 Hz, 1H), 7.47 (s, 1H), 6.76 (d, *J* = 8.5 Hz, 1H), 5.59 (br d, *J* = 1.9 Hz, 1H), 4.42 - 4.28 (m, 4H), 3.64 - 3.50 (m, 6H), 3.43 (s, 2H), 3.33 (br s, 3H), 3.28 - 3.21 (m, 2H), 1.46 (s, 6H); LCMS (Method 2): m/z = 551.2 [M + H]$^+$ | 6.32 | 3.13 |

(continued)

| Example | Structure | Compound name | $^1H$ NMR, MS | Yield (%) | HPLC r.t. (min), Purity |
|---|---|---|---|---|---|
| 179 | 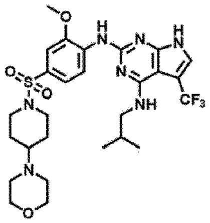 | *N*[4]-isobutyl-*N*[2]-(2-methoxy-4-((4-morpholinopiperid in-1-yl)sulfonyl)phenyl )-5-(trifluoromethyl)-7*H*-pyrrolo [2,3-*d*]pyrimidine-2,4-diamine | $^1H$ NMR (400 MHz, DMSO-$d_6$): δ = 12.08 (d, *J* = 2.3 Hz, 1H), 8.78 (d, *J* = 8.6 Hz, 1H), 7.72 (s, 1H), 7.63 (s, 1H), 7.30 (dd, *J* = 1.9, 8.5 Hz, 1H), 7.20 (d, *J* = 2.0 Hz, 1H), 5.74 (br s, 1H), 3.98 (s, 3H), 3.65 (br d, *J* = 11.8 Hz, 2H), 3.53 - 3.48 (m, 4H), 3.43 (br s, 2H), 2.38 (br s, 4H), 2.26 (br t, *J* = 11.0 Hz, 2H), 2.07 (br d, *J* = 6.0 Hz, 1H), 2.04 - 1.93 (m, 1H), 1.80 (br d, *J* = 11.1 Hz, 2H), 1.40 (br dd, *J* = 2.9, 11.8 Hz, 2H), 0.94 (d, *J* = 6.6 Hz, 6H); LCMS (Method 2): m/z = 612.2 [M + H]$^+$ | 3.27 | 3.46 |
| 180 | 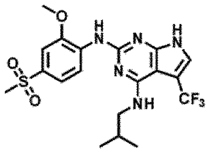 | *N*[4]-isobutyl-*N*[2]-(2-methoxy-4-(methylsulfonyl)p henyl)-5-(trifluoromethyl)-7*H*-pyrrolo [2,3-*d*]pyrimidine-2,4-diamine | $^1H$ NMR (400 MHz, DMSO-$d_6$): δ = 12.10 (br s, 1H), 8.80 (d, *J* = 8.5 Hz, 1H), 7.72 (s, 1H), 7.64 (d, *J* = 1.4 Hz, 1H), 7.51 - 7.43 (m, 2H), 5.79 - 5.70 (m, 1H), 4.01 (s, 3H), 3.45 - 3.41 (m, 2H), 3.19 (s, 3H), 2.00 (td, *J* = 6.7, 13.4 Hz, 1H), 0.95 (d, *J* = 6.8 Hz, 6H); LCMS (Method 2): m/z = 458.2 [M + H]$^+$ | 3.67 | 3.39 |
| 181 | 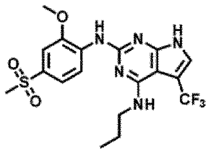 | *N*[2]-(2-methoxy-4-(methylsulfonyl)p henyl)-*N*[4]-propyl-5-(trifluoromethyl)-7*H*-pyrrolo [2,3-*d*]pyrimidine-2,4-diamine | $^1H$ NMR (400 MHz, DMSO-$d_6$): δ = 12.10 (br s, 1H), 8.81 (d, *J* = 8.6 Hz, 1H), 7.71 (s, 1H), 7.63 (d, *J* = 1.4 Hz, 1H), 7.53 - 7.43 (m, 2H), 5.85 - 5.77 (m, 1H), 4.01 (s, 3H), 3.57 - 3.50 (m, 2H), 3.19 (s, 3H), 1.65 (sxt, *J* = 7.3 Hz, 2H), 0.95 (t, *J* = 7.4 Hz, 3H); LCMS (Method 2): m/z = 444.1 [M + H]$^+$ | 8.11 | 3.26 |

(continued)

| Example | Structure | Compound name | $^1$H NMR, MS | Yield (%) | HPLC r.t. (min), Purity |
|---|---|---|---|---|---|
| 182 | 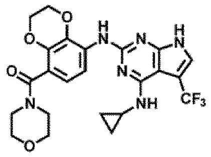 | (8-((4-(cyclopropylamino )-5-(trifluoromethyl)-7*H*-pyrrolo [2,3-*d*]pyrimidin-2-yl)amino)-2,3-dihydrobenzo[*b*][1 ,4] dioxin-5-yl)(morpholino)methanone | $^1$H NMR (400 MHz, DMSO-$d_6$): δ = 12.06 (br s, 1H), 8.30 (d, *J* = 8.5 Hz, 1H), 7.60 (d, *J* = 0.9 Hz, 1H), 7.43 - 7.33 (m, 1H), 6.78 (d, *J* = 8.5 Hz, 1H), 5.58 (br s, 1H), 4.39 (br d, *J* = 2.5 Hz, 2H), 4.33 (br d, *J* = 3.3 Hz, 2H), 3.62 - 3.51 (m, 6H), 3.25 (br dd, *J* = 1.3, 3.1 Hz, 2H), 3.00 - 2.86 (m, 1H), 0.90 - 0.81 (m, 2H), 0.63 - 0.54 (m, 2H); LCMS (Method 2): m/z = 505.2 [M + H]$^+$ | 5.95 | 2.94 |
| 183 | 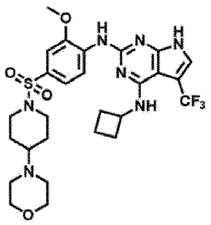 | *N*$^4$-cyclobutyl-*N*$^2$-(2-methoxy-4-((4-morpholinopiperid in-1-yl)sulfonyl)phenyl )-5-(trifluoromethyl)-7*H*-pyrrolo [2,3-*d*]pyrimidine-2,4-diamine | $^1$H NMR (400 MHz, DMSO-$d_6$): δ = 12.14 (br s, 1H), 8.79 (d, *J* = 8.5 Hz, 1H), 7.74 (s, 1H), 7.66 (s, 1H), 7.35 (dd, *J* = 1.8, 8.6 Hz, 1H), 7.21 (d, *J* = 2.0 Hz, 1H), 5.54 (br dd, *J* = 1.4, 7.1 Hz, 1H), 4.72 - 4.63 (m, 1H), 3.99 (s, 3H), 3.66 (br d, *J* = 11.6 Hz, 2H), 3.53 - 3.47 (m, 4H), 2.44 - 2.34 (m, 6H), 2.31 - 2.21 (m, 2H), 2.16 - 2.06 (m, 1H), 2.04 - 1.92 (m, 2H), 1.83 - 1.72 (m, 4H), 1.48 - 1.35 (m, 2H); LCMS (Method 2): m/z = 610.2 [M + H]$^+$ | 3.26 | 3.41 |

(continued)

| Example | Structure | Compound name | $^1$H NMR, MS | Yield (%) | HPLC r.t. (min), Purity |
|---|---|---|---|---|---|
| 184 | 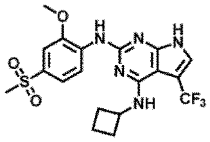 | $N^4$-cyclobutyl-$N^2$-(2-methoxy-4-(methylsulfonyl)p henyl)-5-(trifluoromethyl)-7*H*-pyrrolo [2,3-*d*]pyrimidine-2,4-diamine | $^1$H NMR (400 MHz, DMSO-$d_6$): δ = 12.16 (br s, 1H), 8.81 (d, *J* = 8.5 Hz, 1H), 7.74 (s, 1H), 7.67 (d, *J* = 1.3 Hz, 1H), 7.53 (dd, *J* = 1.9, 8.6 Hz, 1H), 7.45 (d, *J* = 2.0 Hz, 1H), 5.55 (br d, *J* = 5.8 Hz, 1H), 4.75 - 4.61 (m, 1H), 4.01 (s, 3H), 3.20 (s, 3H), 2.45 - 2.36 (m, 2H), 2.08 - 1.93 (m, 2H), 1.82 - 1.71 (m, 2H); LCMS (Method 2): m/z = 456.2 [M + H]$^+$ | 17.1 | 3.33 |
| 185 | 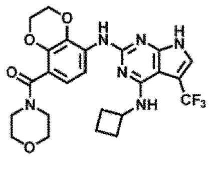 | (8-((4-(cyclobutylamino) -5-(trifluoromethyl)-7*H*-pyrrolo[2,3-*d*]pyrimidin-2-yl)amino)-2,3-dihydrobenzo [*b*][1 ,4]dioxin-5-yl) (morpholino)me thanone | $^1$H NMR (400 MHz, DMSO-$d_6$): δ = 12.31 - 11.78 (m, 1H), 8.11 (d, *J* = 8.5 Hz, 1H), 7.60 (d, *J* = 1.5 Hz, 1H), 7.43 - 7.35 (m, 1H), 6.78 (d, *J* = 8.4 Hz, 1H), 5.51 - 5.41 (m, 1H), 4.71 - 4.59 (m, 1H), 4.40 - 4.30 (m, 4H), 3.64 - 3.51 (m, 6H), 3.29 - 3.20 (m, 2H), 2.41 - 2.34 (m, 2H), 2.03 - 1.92 (m, 2H), 1.79 - 1.69 (m, 2H); LCMS (Method 2): m/z = 519.2 [M + H]$^+$ | 13.2 | 3.17 |
| 186 | 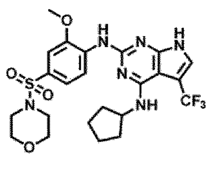 | $N^4$-cyclopentyl-$N^2$-(2-methoxy-4-(morpholinosulfon yl)phenyl)-5-(trifluoromethyl)-7*H*-pyrrolo[2,3-*d*]pyrimidine-2,4-diamine | $^1$H NMR (400 MHz, DMSO-$d_6$): δ = 12.14 (br s, 1H), 8.84 (d, *J* = 8.6 Hz, 1H), 7.77 (s, 1H), 7.66 (s, 1H), 7.34 (dd, *J* = 1.9, 8.6 Hz, 1H), 7.21 (d, *J* = 1.9 Hz, 1H), 5.28 (br dd, *J* = 1.7, 6.8 Hz, 1H), 4.64 - 4.41 (m, 1H), 4.00 (s, 3H), 3.69 - 3.57 (m, 4H), 2.95 - 2.81 (m, 4H), 2.14 - 2.03 (m, 2H), 1.73 - 1.62 (m, 4H), 1.56 - 1.49 (m, 2H); LCMS (Method 2): m/z = 541.2 [M + H]$^+$ | 1.87 | 3.58 |

(continued)

| Example | Structure | Compound name | $^1$H NMR, MS | Yield (%) | HPLC r.t. (min), Purity |
|---|---|---|---|---|---|
| 187 | 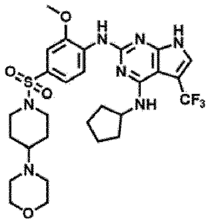 | *N*4-cyclopentyl-*N*2-(2-methoxy-4-((4-morpholinopiperid in-1-yl) sulfonyl)phenyl )-5-(trifluoromethyl)-7*H*-pyrrolo [2,3-*d*]pyrimidine-2,4-diamine | $^1$H NMR (400 MHz, DMSO-$d_6$): δ = 12.13 (br s, 1H), 8.81 (d, *J* = 8.5 Hz, 1H), 7.74 (s, 1H), 7.66 (s, 1H), 7.33 (dd, *J* = 1.9, 8.6 Hz, 1H), 7.21 (d, *J* = 2.0 Hz, 1H), 5.32 - 5.22 (m, 1H), 4.58 - 4.46 (m, 1H), 4.05 - 3.92 (m, 3H), 3.69 - 3.62 (m, 2H), 3.54 - 3.48 (m, 4H), 2.42 - 2.35 (m, 4H), 2.31 - 2.21 (m, 2H), 2.16 - 2.03 (m, 3H), 1.85 - 1.76 (m, 2H), 1.73 - 1.61 (m, 4H), 1.57 - 1.48 (m, 2H), 1.47 - 1.36 (m, 2H); LCMS (Method 2): m/z = 624.2 [M + H]+ | 5.23 | 3.52 |
| 188 | 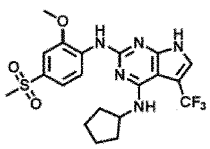 | *N*4-cyclopentyl-*N*2-(2-methoxy-4-(methylsulfonyl)p henyl)-5-(trifluoromethyl)-7*H*-pyrrolo[2,3-*d*]pyrimidine-2,4-diamine | $^1$H NMR (400 MHz, DMSO-$d_6$) δ = 12.15 (br s, 1H), 8.83 (d, *J* = 8.5 Hz, 1H), 7.75 (s, 1H), 7.66 (d, *J* = 1.4 Hz, 1H), 7.51 (dd, *J* = 1.9, 8.6 Hz, 1H), 7.45 (d, *J* = 2.0 Hz, 1H), 5.28 (br dd, *J* = 1.6, 6.8 Hz, 1H), 4.61 - 4.44 (m, 1H), 4.01 (s, 3H), 3.19 (s, 3H), 2.15 - 2.00 (m, 2H), 1.75 - 1.60 (m, 4H), 1.57 - 1.48 (m, 2H); LCMS (Method 2): m/z = 470.2 [M + H]+ | 5.22 | 3.45 |

(continued)

| Example | Structure | Compound name | $^1$H NMR, MS | Yield (%) | HPLC r.t. (min), Purity |
|---|---|---|---|---|---|
| 189 | 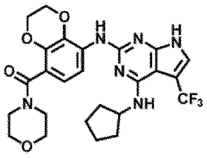 | (8-((4-(cyclopentylamino )-5-(trifluoromethyl)-7*H*-pyrrolo [2,3-*d*]pyrimidin-2-yl)amino)-2,3-dihydrobenzo[*b*][1 ,4] dioxin-5-yl)(morpholino)me thanone | $^1$H NMR (400 MHz, DMSO-$d_6$): δ = 12.05 (br s, 1H), 8.13 (d, *J* = 8.5 Hz, 1H), 7.59 (s, 1H), 7.39 (s, 1H), 6.77 (d, *J* = 8.4 Hz, 1H), 5.20 (br dd, *J* = 1.8, 6.9 Hz, 1H), 4.53 - 4.43 (m, 1H), 4.41 - 4.30 (m, 4H), 3.65 - 3.49 (m, 6H), 3.29 - 3.18 (m, 2H), 2.12 - 1.99 (m, 2H), 1.73 - 1.59 (m, 4H), 1.55 - 1.46 (m, 2H); LCMS (Method 2): m/z = 533.2 [M + H]$^+$ | 14.4 | 3.28 |
| 190 | 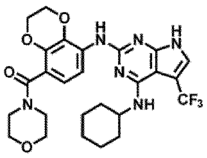 | (8-((4-(cyclohexylamino) -5-(trifluoromethyl)-7*H*-pyrrolo[2,3-*d*]pyrimidin-2-yl)amino)-2,3-dihydrobenzo [*b*][1 ,4]dioxin-5-yl) (morpholino)me thanone | $^1$H NMR (400 MHz, DMSO-$d_6$): δ = 12.01 (br s, 1H), 8.10 (d, *J* = 8.4 Hz, 1H), 7.59 (s, 1H), 7.43 - 7.32 (m, 1H), 6.75 (d, *J* = 8.4 Hz, 1H), 5.24 - 5.08 (m, 1H), 4.44 - 4.27 (m, 4H), 4.18 - 4.01 (m, 1H), 3.72 - 3.46 (m, 6H), 2.06 - 1.93 (m, 2H), 1.78 - 1.51 (m, 4H), 1.49 - 1.20 (m, 6H); LCMS (Method 2): m/z = 547.2 [M + H]$^+$ | 14.7 | 3.39 |
| 191 | 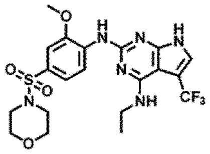 | *N*$^4$-ethyl-*N*$^2$-(2-methoxy-4-(morpholinosulfon yl) phenyl)-5-(trifluoromethyl)-7*H*-pyrrolo[2,3-*d*]pyrimidine-2,4-diamine | $^1$H NMR (400 MHz, DMSO-$d_6$): δ = 12.26 - 11.82 (m, 1H), 9.15 - 8.59 (m, 1H), 7.75 - 7.69 (m, 1H), 7.62 (s, 1H), 7.38 - 7.30 (m, 1H), 7.25 - 7.17 (m, 1H), 5.88 - 5.77 (m, 1H), 4.00 (s, 3H), 3.68 - 3.56 (m, 6H), 2.97 - 2.80 (m, 4H), 1.23 (t, *J* = 7.1 Hz, 3H); LCMS (Method 2): m/z = 501.2 [M + H]$^+$ | 11.5 | 3.23 |

(continued)

| Example | Structure | Compound name | $^1$H NMR, MS | Yield (%) | HPLC r.t. (min), Purity |
|---|---|---|---|---|---|
| 192 | 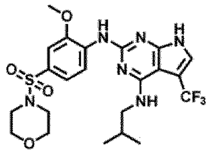 | *N*$^4$-isobutyl-*N*$^2$-(2-methoxy-4-(morpholinosulfon yl) phenyl)-5-(trifluoromethyl)-7*H*-pyrrolo[2,3-*d*]pyrimidine-2,4-diamine | $^1$H NMR (400 MHz, DMSO-$d_6$): δ = 12.09 (br s, 1H), 8.81 (d, *J* = 8.7 Hz, 1H), 7.73 (s, 1H), 7.64 (s, 1H), 7.32 (dd, *J* = 1.8, 8.6 Hz, 1H), 7.21 (d, *J* = 1.8 Hz, 1H), 5.74 (br s, 1H), 4.00 (s, 3H), 3.67 - 3.60 (m, 4H), 3.43 (t, *J* = 6.2 Hz, 2H), 2.92 - 2.86 (m, 4H), 2.05 - 1.94 (m, 1H), 0.95 (d, *J* = 6.7 Hz, 6H); LCMS (Method 2): m/z = 529.2 [M + H]$^+$ | 27.0 | 3.54 |
| 193 | 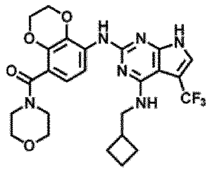 | (8-((4-((cyclobutylmethyl ) amino)-5-(trifluoromethyl)-7*H*-pyrrolo[2,3-*d*]pyrimidin-2-yl)amino)-2,3-dihydrobenzo [*b*][1 ,4]dioxin-5-yl) (morpholino)me thanone | $^1$H NMR (400 MHz, DMSO-$d_6$): δ = 12.03 (br s, 1H), 8.12 (d, *J* = 8.5 Hz, 1H), 7.61 - 7.51 (m, 1H), 7.37 (s, 1H), 6.76 (d, *J* = 8.5 Hz, 1H), 5.60 (br s, 1H), 4.39 (br s, 2H), 4.32 (br s, 2H), 3.65 - 3.51 (m, 8H), 3.30 - 3.18 (m, 2H), 2.70 - 2.63 (m, 1H), 2.06 - 1.96 (m, 2H), 1.91 - 1.81 (m, 2H), 1.80 - 1.71 (m, 2H); LCMS (Method 1): m/z = 533.2 [M + H]$^+$ | 19.8 | 2.61 |

(continued)

| Example | Structure | Compound name | $^1$H NMR, MS | Yield (%) | HPLC r.t. (min), Purity |
|---|---|---|---|---|---|
| 194 | 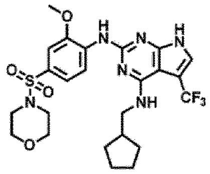 | *N*4-(cyclopentylmethy 1)-*N*2-(2-methoxy-4-(morpholinosulfon yl) phenyl)-5-(trifluoromethyl)-7*H*-pyrrolo[2,3-*d*]pyrimidine-2,4-diamine | $^1$H NMR (400 MHz, DMSO-$d_6$): δ = 12.11 (br s, 1H), 8.83 (d, *J* = 8.6 Hz, 1H), 7.74 (s, 1H), 7.65 (d, *J* = 1.5 Hz, 1H), 7.33 (dd, *J* = 1.9, 8.6 Hz, 1H), 7.21 (d, *J* = 2.0 Hz, 1H), 5.78 - 5.70 (m, 1H), 4.01 (s, 3H), 3.67 - 3.61 (m, 4H), 3.52 (dd, *J* = 5.8, 6.9 Hz, 2H), 2.92 - 2.85 (m, 4H), 2.32 - 2.22 (m, 1H), 1.77 - 1.68 (m, 2H), 1.66 - 1.58 (m, 2H), 1.56 - 1.48 (m, 2H), 1.31 (br dd, *J* = 7.0, 11.9 Hz, 2H); LCMS (Method 1): m/z = 555.2 [M + H]+ | 32.2 | 2.73 |
| 195 | 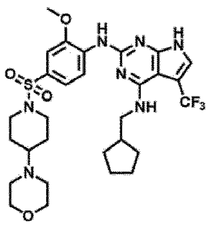 | *N*4-(cyclopentylmethy 1)-*N*2-(2-methoxy-4-((4-morpholinopiperid in-1-yl) sulfonyl)phenyl )-5-(trifluoromethyl)-7*H*-pyrrolo [2,3-*d*]pyrimidine- 2,4-diamine | $^1$H NMR (400 MHz, DMSO-$d_6$): δ = 12.10 (d, *J* = 2.3 Hz, 1H), 8.80 (d, *J* = 8.5 Hz, 1H), 7.72 (s, 1H), 7.64 (s, 1H), 7.31 (dd, *J* = 1.9, 8.6 Hz, 1H), 7.21 (d, *J* = 1.9 Hz, 1H), 5.74 (br s, 1H), 3.99 (s, 3H), 3.66 (br d, *J* = 11.8 Hz, 2H), 3.55 - 3.48 (m, 6H), 2.39 (br s, 4H), 2.31 - 2.22 (m, 3H), 2.17 - 2.06 (m, 1H), 1.80 (br d, *J* = 10.8 Hz, 2H), 1.75 - 1.67 (m, 2H), 1.66 - 1.57 (m, 2H), 1.56 - 1.48 (m, 2H), 1.47 - 1.37 (m, 2H), 1.36 - 1.25 (m, 2H); LCMS (Method 1): m/z = 638.3 [M + H]+ | 19.9 | 2.45 |

(continued)

| Example | Structure | Compound name | $^1$H NMR, MS | Yield (%) | HPLC r.t. (min), Purity |
|---|---|---|---|---|---|
| 196 | 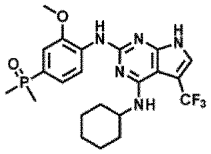 | (4-((4-(cyclohexylamino) -5-(trifluoromethyl)-7*H*-pyrrolo[2,3-*d*]pyrimidin-2-yl)amino)-3-methoxyphenyl) di methylphosphine oxide | $^1$H NMR (400 MHz, DMSO-$d_6$): $\delta$ = 12.06 (d, *J* = 2.3 Hz, 1H), 8.66 (dd, *J* = 3.1, 8.6 Hz, 1H), 7.80 - 7.43 (m, 2H), 7.38 - 7.24 (m, 2H), 5.21 (br d, *J* = 7.4 Hz, 1H), 4.19 - 4.07 (m, 1H), 3.96 (s, 3H), 2.05 - 1.97 (m, 2H), 1.71 (br dd, *J* = 4.2, 8.8 Hz, 2H), 1.64 (d, *J* = 13.3 Hz, 6H), 1.56 (br s, 1H), 1.42 - 1.29 (m, 3H), 1.51 - 1.26 (m, 2H); LCMS (Method 1): m/z = 482.2 [M + H]$^+$ | 23.3 | 2.74 |
| 197 | 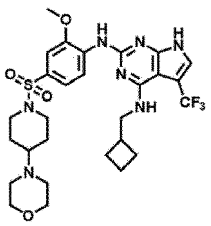 | *N*$^4$-(cyclobutylmethyl) -*N*$^2$-(2-methoxy-4-((4-morpholinopiperid in-1-yl) sulfonyl)phenyl )-5-(trifluoromethyl)-7*H*-pyrrolo [2,3-*d*]pyrimidine-2,4-diamine | $^1$H NMR (400 MHz, DMSO-$d_6$): $\delta$ = 12.09 (br s, 1H), 8.78 (d, *J* = 8.6 Hz, 1H), 7.70 (s, 1H), 7.62 (s, 1H), 7.30 (dd, *J* = 1.8, 8.6 Hz, 1H), 7.20 (d, *J* = 1.9 Hz, 1H), 5.67 (br s, 1H), 3.98 (s, 3H), 3.68 - 3.57 (m, 4H), 3.54 - 3.47 (m, 4H), 2.71 - 2.61 (m, 1H), 2.37 (br s, 4H), 2.25 (br t, *J* = 11.1 Hz, 2H), 2.15 - 2.06 (m, 1H), 2.06 - 1.94 (m, 2H), 1.89 - 1.73 (m, 6H), 1.47 - 1.33 (m, 2H); LCMS (Method 1): m/z = 624.1 [M + H]$^+$ | 14.8 | 2.57 |

(continued)

| Example | Structure | Compound name | $^1$H NMR, MS | Yield (%) | HPLC r.t. (min), Purity |
|---|---|---|---|---|---|
| 198 | 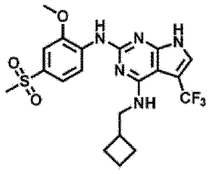 | *N*4-(cyclobutylmethyl) -*N*2-(2-methoxy-4-(methylsulfonyl)phenyl)-5-(trifluoromethyl)-7*H*-pyrrolo[2,3-*d*]pyrimidine-2,4-diamine | $^1$H NMR (400 MHz, DMSO-$d_6$): δ = 12.12 (br s, 1H), 8.80 (d, *J* = 8.5 Hz, 1H), 7.72 (s, 1H), 7.64 (s, 1H), 7.49 (dd, *J* = 2.0, 8.5 Hz, 1H), 7.45 (d, *J* = 2.0 Hz, 1H), 5.68 (br s, 1H), 4.01 (s, 3H), 3.61 (dd, *J* = 5.8, 6.9 Hz, 2H), 3.19 (s, 3H), 2.72 - 2.63 (m, 1H), 2.07 - 1.97 (m, 2H), 1.90 - 1.83 (m, 2H), 1.82 - 1.73 (m, 2H); LCMS (Method 1): m/z = 470.1 [M + H]$^+$ | 25.8 | 2.87 |
| 199 | 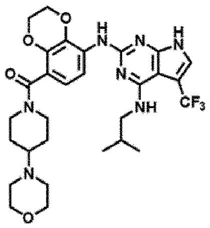 | (8-((4-(isobutylamino)-5-(trifluoromethyl)-7*H*-pyrrolo[2,3-*d*]pyrimidin-2-yl)amino)-2,3-dihydrobenzo[*b*][1 ,4]dioxin-5-yl)(4-morpholinopiperid in-1-yl) methanone | $^1$H NMR (400 MHz, DMSO-$d_6$): δ = 12.01 (d, *J* = 2.3 Hz, 1H), 8.08 (d, *J* = 8.5 Hz, 1H), 7.57 (s, 1H), 7.35 (s, 1H), 6.71 (br dd, *J* = 8.6, 19.7 Hz, 1H), 5.64 (br s, 1H), 4.52 - 4.41 (m, 1H), 4.39 - 4.27 (m, 4H), 3.57 (br s, 4H), 3.50 (br s, 1H), 3.38 (br t, *J* = 6.3 Hz, 2H), 3.31 - 3.26 (m, 1H), 3.04 - 2.87 (m, 1H), 2.79 - 2.69 (m, 1H), 2.48 - 2.41 (m, 4H), 1.97 (td, *J* = 6.8, 13.5 Hz, 1H), 1.90 - 1.80 (m, 1H), 1.78 - 1.66 (m, 1H), 1.42 - 1.17 (m, 2H), 0.93 (d, *J* = 6.6 Hz, 6H); LCMS (Method 1): m/z = 604.3 [M + H]$^+$ | 14.9 | 2.55 |

(continued)

| Example | Structure | Compound name | $^1H$ NMR, MS | Yield (%) | HPLC r.t. (min), Purity |
|---|---|---|---|---|---|
| 200 | 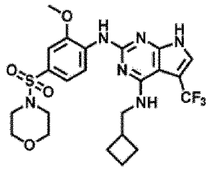 | *N*4-(cyclobutylmethyl) -*N*2-(2-methoxy-4-(morpholinosulfonyl)phenyl)-5-(trifluoromethyl)-7*H*-pyrrolo[2,3-*d*]pyrimidine-2,4-diamine | $^1H$ NMR (400 MHz, DMSO-$d_6$): $\delta$ = 12.12 (s, 1H), 8.82 (d, *J* = 8.5 Hz, 1H), 7.74 (s, 1H), 7.64 (d, *J* = 1.0 Hz, 1H), 7.33 (dd, *J* = 1.9, 8.6 Hz, 1H), 7.21 (d, *J* = 1.9 Hz, 1H), 5.69 (br s, 1H), 4.01 (s, 3H), 3.67 - 3.59 (m, 6H), 2.94 - 2.85 (m, 4H), 2.72 - 2.63 (m, 1H), 2.07 - 1.96 (m, 2H), 1.92 - 1.71 (m, 4H); LCMS (Method 1): m/z = 541.2 [M + H]+ | 23.9 | 2.98 |
| 201 | 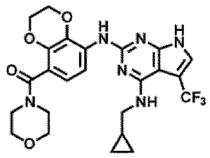 | (8-((4-((cyclopropylmeth yl) amino)-5-(trifluoromethyl)-7*H*-pyrrolo[2,3-*d*]pyrimidin-2-yl)amino)-2,3-dihydrobenzo [*b*][1 ,4]dioxin-5-yl) (morpholino)me thanone | $^1H$ NMR (400 MHz, DMSO-$d_6$): $\delta$ = 12.03 (br s, 1H), 8.12 (d, *J* = 8.5 Hz, 1H), 7.58 (s, 1H), 7.37 (s, 1H), 6.76 (d, *J* = 8.5 Hz, 1H), 5.72 (br s, 1H), 4.41 - 4.30 (m, 4H), 3.60 (br s, 4H), 3.53 (br s, 2H), 3.40 (br d, *J* = 6.4 Hz, 2H), 3.26 (br d, *J* = 2.3 Hz, 2H), 2.36 (br s, 2H), 1.26 - 1.10 (m, 1H), 0.51 - 0.40 (m, 2H), 0.35 - 0.25 (m, 2H); LCMS (Method 1): m/z = 519.2 [M + H]+ | 9.7 | 2.53 |

(continued)

| Example | Structure | Compound name | $^1$H NMR, MS | Yield (%) | HPLC r.t. (min), Purity |
|---|---|---|---|---|---|
| 202 | 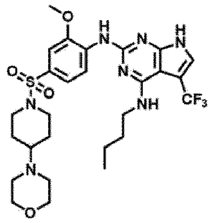 | *N*$^4$-butyl-*N*$^2$-(2-methoxy-4-((4-morpholinopiperid in-1-yl)sulfonyl)phenyl )-5-(trifluoromethyl)-7*H*-pyrrolo [2,3-*d*]pyrimidine-2,4-diamine | $^1$H NMR (400 MHz, DMSO-$d_6$): δ = 12.55 (br s, 1H), 11.91 (br s, 1H), 8.38 (d, *J* = 8.8 Hz, 1H), 7.53 (s, 1H), 7.50 (d, *J* = 2.3 Hz, 1H), 7.37 (s, 1H), 7.04 (dd, *J* = 2.3, 8.8 Hz, 1H), 5.61 (br s, 1H), 3.87 (s, 3H), 3.84 (t, *J* = 7.1 Hz, 2H), 3.57 - 3.51 (m, 2H), 3.32 - 3.26 (m, 2H), 2.49 - 2.48 (m, 2H), 2.46 (s, 1H), 2.06 (m, 2H), 1.64 - 1.50 (m, 4H), 1.43 - 1.29 (m, 4H), 0.91 (td, *J* = 7.4, 11.1 Hz, 6H); LCMS (Method 1): m/z = 612.3 [M + H]$^+$ | 27.0 | 2.54 |
| 203 | 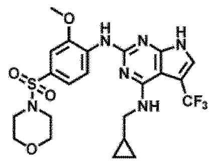 | *N*$^4$-(cyclopropylmethy 1)-*N*$^2$-(2-methoxy-4-(morpholinosulfon yl) phenyl)-5-(trifluoromethyl)-7*H*-pyrrolo[2,3-*d*]pyrimidine-2,4-diamine | $^1$H NMR (400 MHz, DMSO-$d_6$): δ = 12.13 (br s, 1H), 8.83 (d, *J* = 8.6 Hz, 1H), 7.74 (s, 1H), 7.65 (s, 1H), 7.33 (dd, *J* = 1.9, 8.6 Hz, 1H), 7.20 (d, *J* = 1.9 Hz, 1H), 5.81 (br s, 1H), 4.00 (s, 3H), 3.68 - 3.59 (m, 4H), 3.44 (dd, *J* = 5.7, 6.7 Hz, 2H), 2.93 - 2.83 (m, 4H), 1.23 - 1.14 (m, 1H), 0.50 - 0.44 (m, 2H), 0.34 - 0.28 (m, 2H); LCMS (Method 1): m/z = 527.2 [M + H]$^+$ | 12.4 | 2.91 |

(continued)

| Example | Structure | Compound name | $^1$H NMR, MS | Yield (%) | HPLC r.t. (min), Purity |
|---|---|---|---|---|---|
| 204 | 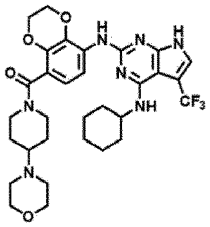 | (8-((4-(cyclohexylamino) -5-(trifluoromethyl)-7*H*-pyrrolo[2,3-*d*]pyrimidin-2-yl)amino)-2,3-dihydrobenzo [*b*][1 ,4]dioxin-5-yl)(4-morpholinopiperid in-1-yl) methanone | $^1$H NMR (400 MHz, DMSO-$d_6$): δ = 12.03 (br s, 1H), 8.06 (br d, *J* = 8.3 Hz, 1H), 7.60 (s, 1H), 7.39 (s, 1H), 6.81 - 6.58 (m, 1H), 5.17 (br d, *J* = 6.0 Hz, 1H), 4.51 - 4.42 (m, 1H), 4.38 (br s, 2H), 4.30 (br s, 2H), 4.16 - 4.00 (m, 1H), 3.62 - 3.44 (m, 6H), 3.09 - 2.84 (m, 1H), 2.74 (br s, 1H), 2.46 (br s, 4H), 2.39 (br d, *J* = 11.5 Hz, 1H), 2.04 - 1.94 (m, 2H), 1.89 - 1.81 (m, 1H), 1.77 - 1.64 (m, 3H), 1.64 - 1.56 (m, 1H), 1.49 - 1.16 (m, 8H); LCMS (Method 1): m/z = 630.3 $[M + H]^+$ | 19.9 | 2.66 |
| 205 | 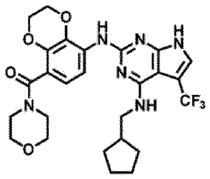 | (8-((4-((cyclopentylmeth yl) amino)-5-(trifluoromethyl)-7*H*-pyrrolo[2,3-*d*]pyrimidin-2-yl)amino)-2,3-dihydrobenzo [*b*][1 ,4]dioxin-5-yl) (morpholino)me thanone | $^1$H NMR (400 MHz, DMSO-$d_6$): δ = 12.02 (d, *J* = 2.4 Hz, 1H), 8.13 (d, *J* = 8.5 Hz, 1H), 7.58 (s, 1H), 7.37 (s, 1H), 6.76 (d, *J* = 8.5 Hz, 1H), 5.64 (br d, *J* = 0.9 Hz, 1H), 4.39 (br d, *J* = 2.4 Hz, 2H), 4.33 (br d, *J* = 3.6 Hz, 2H), 3.61 (br s, 4H), 3.54 (br s, 2H), 3.51 - 3.44 (m, 2H), 3.29 - 3.16 (m, 2H), 2.30 - 2.20 (m, 1H), 1.76 - 1.67 (m, 2H), 1.65 - 1.57 (m, 2H), 1.57 - 1.46 (m, 2H), 1.34 - 1.23 (m, 2H); LCMS (Method 1): m/z = 547.2 $[M + H]^+$ | 26.9 | 2.68 |

(continued)

| Example | Structure | Compound name | $^1H$ NMR, MS | Yield (%) | HPLC r.t. (min), Purity |
|---|---|---|---|---|---|
| 206 | 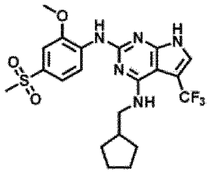 | $N^4$-(cyclopentylmethy 1)-$N^2$-(2-methoxy-4-(methylsulfonyl)p henyl)-5-(trifluoromethyl)-7*H*-pyrrolo [2,3-*d*]pyrimidine-2,4-diamine | $^1H$ NMR (400 MHz, DMSO-$d_6$): δ = 12.12 (br s, 1H), 8.82 (d, *J* = 8.6 Hz, 1H), 7.73 (s, 1H), 7.65 (d, *J* = 1.0 Hz, 1H), 7.49 (dd, *J* = 2.0, 8.5 Hz, 1H), 7.46 (d, *J* = 2.0 Hz, 1H), 5.74 (br s, 1H), 4.02 (s, 3H), 3.52 (dd, *J* = 5.7, 7.1 Hz, 2H), 3.20 (s, 3H), 2.31 - 2.24 (m, 1H), 1.78 - 1.68 (m, 2H), 1.67 - 1.59 (m, 2H), 1.58 - 1.48 (m, 2H), 1.37 - 1.26 (m, 2H); LCMS (Method 1): m/z = 484.2 [M + H]$^+$ | 21.7 | 2.94 |
| 207 | 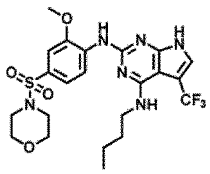 | $N^4$-butyl-$N^2$-(2-methoxy-4-(morpholinosulfon yl) phenyl)-5-(trifluoromethyl)-7*H*-pyrrolo[2,3-*d*]pyrimidine-2,4-diamine | $^1H$ NMR (400 MHz, DMSO-$d_6$): δ = 12.10 (br s, 1H), 8.83 (d, *J* = 8.6 Hz, 1H), 7.74 (s, 1H), 7.64 (s, 1H), 7.32 (dd, *J* = 1.9, 8.6 Hz, 1H), 7.21 (d, *J* = 2.0 Hz, 1H), 5.87 - 5.73 (m, 1H), 4.01 (s, 3H), 3.67 - 3.62 (m, 4H), 3.61 - 3.54 (m, 2H), 2.95 - 2.84 (m, 4H), 1.63 (m, 2H), 1.38 (m, 2H), 0.93 (t, *J* = 7.4 Hz, 3H); LCMS (Method 1): m/z = 529.2 [M + H]$^+$ | 6.7 | 2.95 |
| 208 | 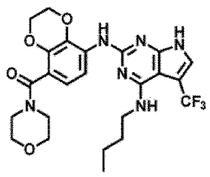 | (8-((4-(butylamino)-5-(trifluoromethyl)-7*H*-pyrrolo [2,3-*d*]pyrimidin-2-yl)amino)-2,3-dihydrobenzo[*b*][1 ,4] dioxin-5-yl)(morpholino)me thanone | $^1H$ NMR (400 MHz, DMSO-$d_6$): δ = 12.01 (br s, 1H), 8.13 (d, *J* = 8.4 Hz, 1H), 7.57 (s, 1H), 7.36 (s, 1H), 6.75 (d, *J* = 8.5 Hz, 1H), 5.69 (br s, 1H), 4.45 - 4.24 (m, 4H), 3.61 (br s, 4H), 3.58 - 3.51 (m, 4H), 3.30 - 3.15 (m, 2H), 1.61 (m, 2H), 1.42 - 1.27 (m, 2H), 0.93 (t, *J* = 7.4 Hz, 3H); LCMS (Method 1): m/z = 521.2 [M + H]$^+$ | 14.6 | 2.58 |

(continued)

| Example | Structure | Compound name | $^1H$ NMR, MS | Yield (%) | HPLC r.t. (min), Purity |
|---|---|---|---|---|---|
| 209 | 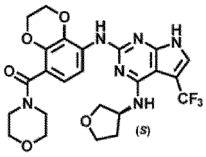 | (S)-morpholino (8-((4-((tetrahydrofuran-3-yl) amino)-5-(trifluoromethyl)-7*H*-pyrrolo[2,3-*d*]pyrimidin-2-yl)amino)-2,3-dihydrobenzo [*b*][1 ,4]dioxin-5-yl) methanone | $^1H$ NMR (400 MHz, DMSO-$d_6$): δ = 12.31 - 11.83 (m, 1H), 8.08 (d, *J* = 8.5 Hz, 1H), 7.62 (d, *J* = 1.5 Hz, 1H), 7.47 (s, 1H), 6.78 (d, *J* = 8.3 Hz, 1H), 5.42 - 5.31 (m, 1H), 4.82 - 4.64 (m, 1H), 4.39 (br d, *J* = 2.5 Hz, 2H), 4.33 (br d, *J* = 3.5 Hz, 2H), 3.97 - 3.83 (m, 2H), 3.77 (dt, *J* = 5.8, 8.4 Hz, 1H), 3.68 - 3.59 (m, 5H), 3.54 (br s, 2H), 3.30 - 3.18 (m, 2H), 2.39 - 2.24 (m, 1H), 1.96 - 1.81 (m, 1H); LCMS (Method 1): m/z = 535.2 [M + H]+ | 15.9 | 2.68 |
| 210 | 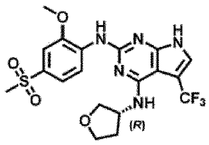 | (*R*)-*N*2-(2-methoxy-4-(methylsulfonyl)p henyl)-*N*4-(tetrahydrofuran-3-yl)-5-(trifluoromethyl)-7*H*-pyrrolo[2,3-*d*]pyrimidine-2,4-diamine | $^1H$ NMR (400 MHz, DMSO-$d_6$): δ = 12.20 (br s, 1H), 8.79 (d, *J* = 8.5 Hz, 1H), 7.81 (s, 1H), 7.69 (d, *J* = 1.3 Hz, 1H), 7.54 (dd, *J* = 2.0, 8.5 Hz, 1H), 7.46 (d, *J* = 2.0 Hz, 1H), 5.58 - 5.30 (m, 1H), 4.95 - 4.67 (m, 1H), 4.02 (s, 3H), 3.96 (m, 1H), 3.92 - 3.84 (m, 1H), 3.79 (m, 1H), 3.68 (m, 1H), 3.20 (s, 3H), 2.42 - 2.29 (m, 1H), 1.97 - 1.80 (m, 1H); LCMS (Method 1): m/z = 472.1 [M + H]+ | 21.5 | 2.90 |

(continued)

| Example | Structure | Compound name | $^1$H NMR, MS | Yield (%) | HPLC r.t. (min), Purity |
|---|---|---|---|---|---|
| 211 | 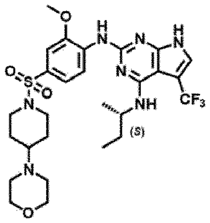 | (*S*)-*N*$^4$-(*sec*-butyl)-*N*$^2$-(2-methoxy-4-((4-morpholinopiperid in-1-yl) sulfonyl)phenyl )-5-(trifluoromethyl)-7*H*-pyrrolo [2,3-*d*]pyrimidine-2,4-diamine | $^1$H NMR (400 MHz, DMSO-$d_6$): δ = 12.13 (br s, 1H), 8.79 (d, *J* = 8.8 Hz, 1H), 7.73 (s, 1H), 7.67 (s, 1H), 7.33 (dd, *J* = 2.0, 8.5 Hz, 1H), 7.21 (d, *J* = 2.0 Hz, 1H), 5.13 (br dd, *J* = 1.4, 7.9 Hz, 1H), 4.51 - 4.15 (m, 1H), 4.00 (s, 3H), 3.66 (m, 2H), 3.55 - 3.48 (m, 4H), 2.42 - 2.35 (m, 4H), 2.32 - 2.21 (m, 2H), 2.12 (m, 1H), 1.80 (m, 2H), 1.69 - 1.58 (m, 2H), 1.42 (m, 2H), 1.25 (d, *J* = 6.4 Hz, 3H), 0.93 (t, *J* = 7.6 Hz, 3H); LCMS (Method 1): m/z = 612.3 [M + H]$^+$ | 22.9 | 2.50 |
| 212 | 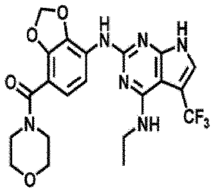 | (7-((4-(ethylamino)-5-(trifluoromethyl)-7*H*-pyrrolo [2,3-*d*]pyrimidin-2-yl)amino) benzo[*d*] [1,3]dioxol-4-yl) (morpholino)me thanone | $^1$H NMR (400 MHz, MeOD) δ 8.02 (d, *J* = 8.8 Hz, 1H), 7.44 (d, *J* = 1.1 Hz, 1H), 7.01 (d, *J* = 8.8 Hz, 1H), 6.15 (s, 2H), 3.80 (s, 6H), 3.70 (dd, *J* = 14.4, 7.2 Hz, 2H), 3.58 (s, 2H), 1.36 (t, *J* = 7.2 Hz, 3H). LCMS (Method 7): m/z = 479.3 [M + H]$^+$ | 58 | 1.42 |
| 213 | 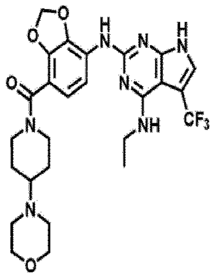 | (7-((4-(ethylamino)-5-(trifluoromethyl)-7*H*-pyrrolo [2,3-*d*]pyrimidin-2-yl)amino) benzo[*d*] [1,3]dioxol-4-yl)(4-morpholinopiperid in-1-yl) methanone | $^1$H NMR (400 MHz, MeOD) δ 7.98 (d, *J* = 8.8 Hz, 1H), 7.23 (d, *J* = 1.3 Hz, 1H), 6.81 (d, *J* = 8.8 Hz, 1H), 5.97 (s, 2H), 4.67 - 4.53 (m, 1H), 3.81 (s, 1H), 3.71 (s, 5H), 3.53 (q, *J* = 7.2 Hz, 2H), 3.23 - 3.19 (m, 4H), 3.07 (d, *J* = 14.1 Hz, 1H), 2.85 (s, 6H), 2.76 (s, 1H), 1.96 (dd, *J* = 52.6, 29.0 Hz, 3H), 1.49 (d, *J* = 10.5 Hz, 3H). LCMS (Method 7): m/z = 562.3 [M + H]$^+$ | 41 | 1.19 |

(continued)

| Example | Structure | Compound name | $^1$H NMR, MS | Yield (%) | HPLC r.t. (min), Purity |
|---|---|---|---|---|---|
| 214 | 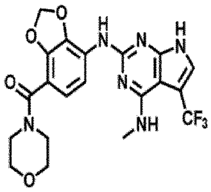 | (7-((4-(methylamino)-5-(trifluoromethyl)-7*H*-pyrrolo [2,3-*d*]pyrimidin-2-yl)amino) benzo[*d*] [1,3]dioxol-4-yl) (morpholino)me thanone | $^1$H NMR (400 MHz, MeOD) δ 8.14 (d, *J* = 8.8 Hz, 1H), 7.33 (d, *J* = 1.4 Hz, 1H), 6.94 (d, *J* = 8.8 Hz, 1H), 6.10 (s, 2H), 3.74 (s, 6H), 3.54 (s, 2H), 3.13 (s, 3H). LCMS (Method 7): m/z = 465.2 [M + H]$^+$ | 66 | 1.38 |
| 215 | 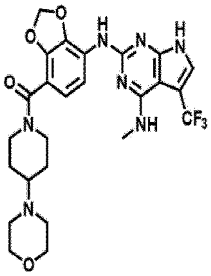 | (7-((4-(methylamino)-5-(trifluoromethyl)-7*H*-pyrrolo [2,3-*d*]pyrimidin-2-yl)amino) benzo[*d*] [1,3]dioxol-4-yl)(4-morpholinopiperid in-1-yl) methanone | $^1$H NMR (400 MHz, MeOD) δ 8.20 (d, *J* = 8.8 Hz, 1H), 7.51 (d, *J* = 1.2 Hz, 1H), 7.10 (d, *J* = 8.8 Hz, 1H), 6.25 (s, 2H), 4.13 (s, 6H), 3.66 (dd, *J* = 14.0, 7.0 Hz, 3H), 3.48 (s, 4H), 3.29 (s, 3H), 2.39 (s, 3H), 1.92 (s, 3H), 1.34 (t, *J* = 7.0 Hz, 2H). LCMS (Method 7): m/z = 548.3 [M + H]$^+$ | 93 | 1.13 |
| 216 | 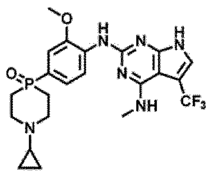 | 1-cyclopropyl-4-(3-methoxy-4-((4-(methylamino)-5-(trifluoromethyl)-7*H*-pyrrolo [2,3-*d*]pyrimidin-2-yl)amino) phenyl)-1,4-azaphosphinane 4-oxide | $^1$H NMR (400 MHz, DMSO-$d_6$) δ = 12.02 (br s, 1H), 8.74 (dd, *J* = 3.1, 8.3 Hz, 1H), 7.62 - 7.53 (m, 2H), 7.41 - 7.16 (m, 2H), 5.95 (br d, *J* = 4.1 Hz, 1H), 3.97 (s, 3H), 3.04 (d, *J* = 4.6 Hz, 3H), 3.01 - 2.88 (m, 4H), 2.19 (dt, *J* = 4.8, 9.6 Hz, 2H), 1.91 - 1.73 (m, 3H), 0.52 - 0.43 (m, 2H), 0.39 - 0.30 (m, 2H); LCMS (Method 1): m/z = 495.2 (M + H)$^+$; | 23.4 | 2.31 |

(continued)

| Example | Structure | Compound name | $^1$H NMR, MS | Yield (%) | HPLC r.t. (min), Purity |
|---|---|---|---|---|---|
| 217 | 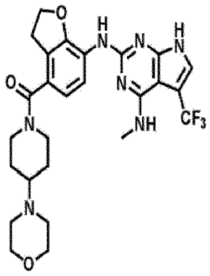 | (7-((4-(methylamino)-5-(trifluoromethyl)-7*H*-pyrrolo[2,3-*d*]pyrimidin-2-yl)amino)-2,3-dihydrobenzofuran-4-yl)(4-morpholinopiperid in-1-yl) methanone | $^1$H NMR (400 MHz, DMSO-$d_6$): δ = 11.96 (s, 1H), 8.12(d, *J* = 8.0Hz, 1H), 7.52(s, 1H), 7.40(s, 1H), 6.77 (d, *J* = 8.4Hz, 1H), 5.87 (d, *J* = 1.6Hz, 1H), 4.62 (t, *J* = 8.8Hz, 1H), 3.57 (t, *J* = 4.4Hz, 4H), 3.45 (s, 2H), 3.01(d, *J* = 4.8Hz, 3H), 2.68(s, 1H), 2.50(m, 4H), 2.49 (s, 1H), 1.81(t, *J* = 4.0Hz, 2H), 1.31(d, *J* = 8.8Hz, 2H); LCMS (Method 1): m/z = 546.3 (M + H)$^+$; | 19.2 | 1.89 |
| 218 | 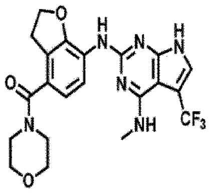 | (7-((4-(methylamino)-5-(trifluoromethyl)-7H-pyrrolo[2,3-d]pyrimidin-2-yl)amino)-2,3-dihydrobenzofuran -4-yl)(morpholino)me thanone | $^1$H NMR (400 MHz, DMSO-$d_6$) δ = 11.96 (br s, 1H), 8.17 (d, *J* = 8.3 Hz, 1H), 7.53 (d, *J* = 1.1 Hz, 1H), 7.39 (s, 1H), 6.80 (d, *J* = 8.4 Hz, 1H), 5.88 (br d, *J* = 4.0 Hz, 1H), 4.64 (t, *J* = 8.8 Hz, 2H), 3.60 (br s, 4H), 3.49 (br d, *J* = 3.9 Hz, 4H), 3.21 (t, *J* = 8.7 Hz, 2H), 3.02 (d, *J* = 4.5 Hz, 3H); LCMS (Method 1):, m/z = 463.2(M + H)$^+$; | 28.4 | 2.21 |
| 219 | 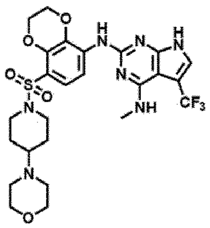 | *N*$^4$-methyl-*N*$^2$-(8-((4-morpholinopiperid in-1-yl) sulfonyl)-2,3-dihydrobenzo[*b*][1 ,4]dioxin-5-yl)-5-(trifluoromethyl)-7*H*-pyrrolo[2,3-*d*]pyrimidine-2,4-diamine | (Method 7): m/z = 598.6 [M + H]$^+$ | 59 | 1.29 |
| 220 | 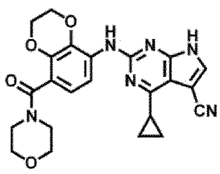 | 4-cyclopropyl-2-((8-(morpholine-4-carbonyl)-2,3-dihydrobenzo[*b*][1 ,4]dioxin-5-yl)amino)-7*H*-pyrrolo[2,3-*d*]pyrimidine-5-carbonitrile | (Method 7): m/z = 447.3 [M + H]$^+$ | 37 | 1.38 |

(continued)

| Example | Structure | Compound name | $^1H$ NMR, MS | Yield (%) | HPLC r.t. (min), Purity |
|---|---|---|---|---|---|
| 221 | 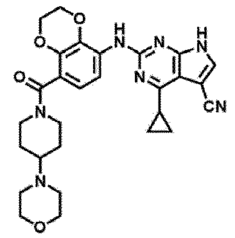 | 4-cyclopropyl-2-((8-(4-morpholinopiperid ine-1-carbonyl)-2,3-dihydrobenzo [*b*][1 ,4]dioxin-5-yl)amino)-7*H*-pyrrolo[2,3-*d*]pyrimidine-5-carbonitrile | $^1H$ NMR (400 MHz, DMSO-$d_6$) δ 12.63 (s, 1H), 8.18 (s, 1H), 7.89 - 7.82 (m, 1H), 7.78 (s, 1H), 6.75 (d, *J* = 11.2 Hz, 1H), 4.51 (s, 1H), 4.33 (d, *J* = 22.6 Hz, 4H), 3.68 - 3.48 (m, 4H), 3.11 - 2.85 (m, 2H), 2.73 (t, *J* = 12.1 Hz, 2H), 2.56 (s, 1H), 1.91 (s, 4H), 1.53 - 1.11 (m, 8H); LCMS (Method 7): m/z = 530.4 [M + H]$^+$ | 44 | 1.10 |
| 222 | 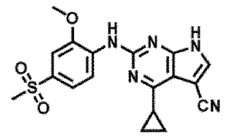 | 4-cyclopropyl-2-((2-methoxy-4-(methylsulfonyl)p henyl) amino)-7*H*-pyrrolo[2,3-*d*] pyrimidine-5-carbonitrile | $^1H$ NMR (400 MHz, DMSO-$d_6$) δ 8.76 (d, *J* = 5.2 Hz, 1H), 8.10 (s, 1H), 7.85 (s, 1H), 7.54 - 7.48 (m, 1H), 7.43 (s, 1H), 7.27 (s, 1H), 4.00 (s, 3H), 3.20 (s, 3H), 2.63 - 2.57 (m, 1H), 1.19 - 1.15 (m, 2H), 0.92 - 0.66 (m, 2H); LCMS (Method 7): m/z = 384.2 [M + H]$^+$ | 26 | 1.48 |
| 223 | 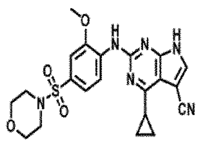 | 4-cyclopropyl-2-((2-methoxy-4-(morpholinosulfon yl) phenyl)amino)-7*H*-pyrrolo [2,3-*d*]pyrimidine-5-carbonitrile | $^1H$ NMR (400 MHz, DMSO-$d_6$) δ 8.91 (d, *J* = 7.1 Hz, 1H), 7.80 (s, 2H), 7.64 (s, 1H), 7.47 (s, 2H), 4.00 (s, 3H), 3.63 (s, 4H), 2.88 (s, 4H), 1.76 - 1.76 (m, 1H), 1.19 - 1.17 (m, 2H), 0.89 - 0.85 (m, 2H); LCMS (Method 7): m/z = 455.2 [M + H]$^+$ | 7 | 1.56 |

(continued)

| Example | Structure | Compound name | $^1H$ NMR, MS | Yield (%) | HPLC r.t. (min), Purity |
|---|---|---|---|---|---|
| 224 | 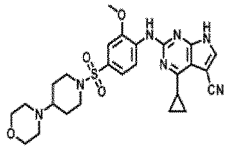 | 4-cyclopropyl-2-((2-methoxy-4-((4-morpholinopiperid in-1-yl)sulfonyl)phenyl )amino)-7*H*-pyrrolo[2,3-*d*]pyrimidine-5-carbonitrile | $^1H$ NMR (400 MHz, DMSO-$d_6$) δ 8.71 (d, *J* = 8.5 Hz, 1H), 8.15 (s, 1H), 7.90 (s, 1H), 7.33 (d, *J* = 5.5 Hz, 1H), 7.20 (s, 1H), 3.98 (s, 3H), 3.65 (d, *J* = 9.8 Hz, 2H), 3.51 (s, 5H), 3.35 (s, 1H), 2.59 (s, 1H), 2.38 (s, 4H), 2.25 (t, *J* = 10.5 Hz, 2H), 2.11 (t, *J* = 11.1 Hz, 1H), 1.88 (s, 1H), 1.80 (d, *J* = 11.0 Hz, 2H), 1.41 (dd, *J* = 23.6, 12.7 Hz, 2H), 1.20 (m, 2H); LCMS (Method 7): m/z = 538.3 [M + H]$^+$ | 19 | 1.25 |
| 225 | 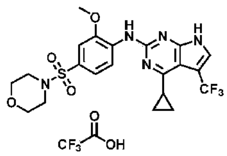 | 4-cyclopropyl-*N*-(2-methoxy-4-(morpholinosulfon yl) phenyl)-5-(trifluoromethyl)-7*H*-pyrrolo[2,3-*d*]pyrimidin-2-amine | $^1H$ NMR (400 MHz, MeOD, TFA) δ 8.82 (d, *J* = 8.6 Hz, 1H), 7.56 (s, 1H), 7.31 (dd, *J* = 8.6, 2.0 Hz, 1H), 7.20 (d, *J* = 1.9 Hz, 1H), 3.97 (s, 3H), 3.67 - 3.61 (m, 4H), 2.93 - 2.89 (m, 4H), 2.44 - 2.39 (m, 1H), 1.12 - 1.07 (m, 4H); LCMS (Method 7): m/z = 498.23 [M + H]$^+$ | 15 | 1.92 |
| 226 | 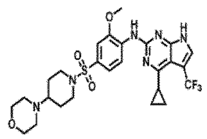 | 4-cyclopropyl-*N*-(2-methoxy-4-((morpholinopiperi din-1-yl) sulfonyl)phenyl )-5-(trifluoromethyl)-7*H*-pyrrolo [2,3-*d*]pyrimidin-2-amine | LCMS (Method 7): m/z = 581.3 [M + H]$^+$ | 7 | 1.42 |

(continued)

| Example | Structure | Compound name | $^1$H NMR, MS | Yield (%) | HPLC r.t. (min), Purity |
|---|---|---|---|---|---|
| 227 | 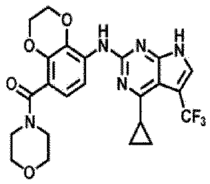 | (8-((4-cyclopropyl-5-(trifluoromethyl)-7*H*-pyrrolo[2,3-*d*]pyrimidin-2-yl)amino)-2,3-dihydrobenzo[*b*][1 ,4]dioxin-5-yl)(morpholino)methanone | $^1$H NMR (400 MHz, $CDCl_3$) δ 9.51 (s, 1H), 8.20 (d, *J* = 8.5 Hz, 1H), 7.40 (s, 1H), 7.35 (s, 1H), 6.94 (d, *J* = 8.5 Hz, 1H), 4.38 - 4.33 (m, 2H), 4.33 - 4.26 (m, 2H), 3.86 - 3.73 (m, *J* = 14.7 Hz, 4H), 3.69 - 3.58 (m, 2H), 3.45 - 3.34 (m, 2H), 2.47 - 2.39 (m, 1H), 1.35 - 1.30 (m, 2H), 1.14 - 1.09 (m, 2H); LCMS (Method 7): m/z = 490.24 $[M + H]^+$ | 29 | 1.72 |
| 228 | 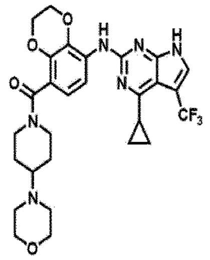 | (8-((4-cyclopropyl-5-(trifluoromethyl)-7*H*-pyrrolo[2,3-*d*]pyrimidin-2-yl)amino)-2,3-dihydrobenzo[*b*][1 ,4]dioxin-5-yl)(4-morpholinopiperid in-1-yl) methanone | LCMS (Method 7): m/z = 573.4 $[M + H]^+$ | 10 | 1.33 |
| 229 | 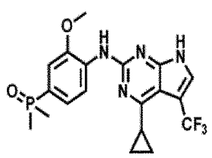 | (4-((4-cyclopropyl-5-(trifluoromethyl)-7*H*-pyrrolo[2,3-*d*]pyrimidin-2-yl)amino)-3-methoxyphenyl)dimethylphosphine oxide | $^1$H NMR (400 MHz, $CDCl_3$) δ 9.91 (s, 2H), 8.68 (dd, *J* = 8.2, 3.5 Hz, 1H), 7.41 (s, 1H), 7.35 (d, *J* = 12.4 Hz, 1H), 7.23 - 7.18 (m, 1H), 3.98 (s, 3H), 2.52 - 2.43 (m, 1H), 1.80 (s, 3H), 1.76 (s, 3H), 1.23 - 1.20 (m, 4H); LCMS (Method 7): m/z = 425.25 $[M + H]^+$ | 26 | 1.60 |

(continued)

| Example | Structure | Compound name | $^1H$ NMR, MS | Yield (%) | HPLC r.t. (min), Purity |
|---|---|---|---|---|---|
| 230 | 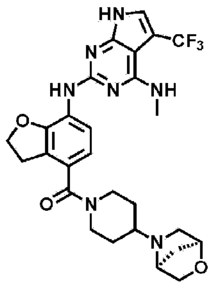 | (4-((1*S*,4*S*)-2-oxa-5-azabicyclo[2.2.1]h eptan-5-yl) piperidin-1-yl) (7-((4-(methylamino)-5-(trifluoromethyl)-7*H*-pyrrolo [2,3-*d*]pyrimidin-2-yl)amino)-2,3-dihydrobenzofuran -4-yl) methanone | $^1H$ NMR (400 MHz, Chloroform-*d*) δ 9.69 (s, 1H), 8.31 (d, *J* = 8.2 Hz, 1H), 7.05 (d, *J* = 7.1 Hz, 2H), 6.78 (dd, *J* = 8.4, 3.9 Hz, 1H), 5.29 (s, 1H), 4.67 (t, *J* = 8.7 Hz, 2H), 4.42 (s, 1H), 4.06 (d, *J* = 7.9 Hz, 1H), 3.71 (s, 1H), 3.64 (d, *J* = 7.7 Hz, 1H), 3.29 (t, *J* = 8.7 Hz, 2H), 3.15 (d, *J* = 4.6 Hz, 3H), 3.10 - 2.94 (m, 3H), 2.65 (m, 1H), 2.46 (d, *J* = 10.0 Hz, 1H), 1.83 (dd, *J* = 28.2, 9.6 Hz, 4H), 1.69 (m, 4H). LCMS (Method 7) m/z = 558.5 [M + H]+ | 50 | 1.34 |
| 231 | 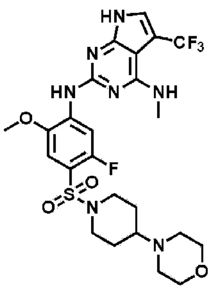 | $N^2$-(5-fluoro-2-methoxy-4-((4-morpholinopiperid in-1-yl) sulfonyl)phenyl )-$N^4$-methyl-5-(trifluoromethyl)-7*H*-pyrrolo [2,3-*d*]pyrimidine-2,4-diamine | $^1H$ NMR (400 MHz, DMSO-$d_6$) δ 12.16 (s, 1H), 8.79 (d, *J* = 13.5 Hz, 1H), 7.80 (d, *J* = 1.5 Hz, 1H), 7.65 (s, 1H), 7.15 (d, *J* = 6.3 Hz, 1H), 6.10 (d, *J* = 4.9 Hz, 1H), 3.97 (s, 3H), 3.69 (d, *J* = 11.7 Hz, 2H), 3.52 (t, *J* = 4.6 Hz, 4H), 3.05 (d, *J* = 4.5 Hz, 3H), 2.46 (s, 2H), 2.40 (t, *J* = 4.7 Hz, 4H), 2.20 (ddt, *J* = 10.8, 6.9, 3.3 Hz, 1H), 1.86 - 1.77 (m, 2H), 1.41 (qd, *J* = 11.7, 3.8 Hz, 2H). LCMS (Method 7): m/z = 588.3 [M + H]+ | 34 | 1.41 |

(continued)

| Example | Structure | Compound name | $^1H$ NMR, MS | Yield (%) | HPLC r.t. (min), Purity |
|---|---|---|---|---|---|
| 232 | 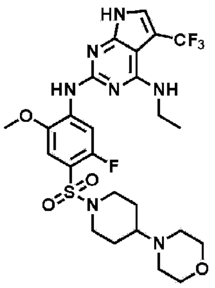 | *N*[4]-ethyl-*N*[2]-(5-fluoro- 2-methoxy-4-((4-morpholinopiperid in-1-yl) sulfonyl)phenyl )-5-(trifluoromethyl)-7*H*-pyrrolo [2,3-*d*]pyrimidine- 2,4-diamine | $^1H$ NMR (400 MHz, DMSO-$d_6$) δ 12.17 (s, 1H), 8.76 (d, *J* = 13.5 Hz, 1H), 7.80 (s, 1H), 7.66 (s, 1H), 7.15 (d, *J* = 6.2 Hz, 1H), 5.93 (t, *J* = 5.8 Hz, 1H), 3.97 (s, 3H), 3.69 (d, *J* = 11.8 Hz, 2H), 3.61 (q, *J* = 6.7 Hz, 2H), 3.52 (t, *J* = 4.5 Hz, 4H), 2.47 (s, 2H), 2.40 (t, *J* = 4.6 Hz, 4H), 2.24 - 2.14 (m, 1H), 1.82 (d, *J* = 12.5 Hz, 2H), 1.41 (qd, *J* = 11.9, 4.0 Hz, 2H), 1.23 (t, *J* = 7.1 Hz, 3H). LCMS (Method 7): m/z = 602.4 [M + H]$^+$ | 85 | 1.37 |
| 233 | 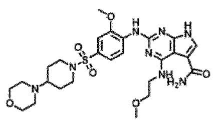 | 2-((2-methoxy-4-((4-morpholinopiperid in-1-yl) sulfonyl)phenyl )amino)-4-((2-methoxyethyl)ami no)-7H-pyrrolo[2,3-*d*]pyrimidine-5-carboxamide | $^1H$ NMR (400 MHz, DMSO-$d_6$): δ = 11.73 (d, *J* = 2.4Hz, 1H), 9.83 (t, *J* = 5.6Hz, 1H), 8.84 (d, *J* = 8.4Hz, 1H), 7.74 (d, *J* = 2.8Hz, 2H), 7.58 (s, 1H), 7.31(q, *J* = 1.6Hz, 1H), 7.19(d, *J* = 1.6Hz, 2H), 3.99(s, 3H), 3.65(t, *J* = 5.2Hz, 4H), 3.56(d, *J* = 5.2Hz, 2H), 3.52(s, 2H), 3.302 (d, *J* = 3.6Hz, 3H), 2.52 (s, 2H), 2.51(d, *J* = 1.6Hz, 4H), 2.50(s, 2H), 2.32(d, *J* = 9.2Hz, 1H), 1.80(d, *J* = 9.2Hz, 2H), 1.42(d, *J* = 10.8Hz, 2H); LCMS (Method 1): m/z = 589.2 (M + H)$^+$; | 8.5 | 0.59 |

(continued)

| Example | Structure | Compound name | $^1$H NMR, MS | Yield (%) | HPLC r.t. (min), Purity |
|---|---|---|---|---|---|
| 234 | 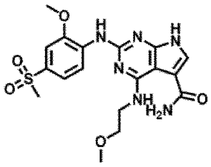 | 2-((2-methoxy-4-(methylsulfonyl)p henyl) amino)-4-((2-methoxy) amino)-7*H*-pyrrolo[2,3-*d*] pyrimidine-5-carboxamide | $^1$H NMR (400 MHz, DMSO-$d_6$): $\delta$ = 11.76 (d, *J* = 2.0Hz, 1H), 9.83 (t, *J* = 5.2Hz, 1H), 8.54 (d, *J* = 8.4Hz, 1H), 7.77 (d, *J* = 17.2Hz, 2H), 7.59(s, 1H), 7.48(d, *J* = 8.8Hz, 1H), 7.43(s, 1H), 7.21(d, *J* = 3.2Hz, 1H), 4.00(s, 3H), 3.66 (d, *J* = 5.6Hz, 2H), 3.56 (t, *J* = 5.2Hz, 2H), 3.31 (d, *J* = 2.4Hz, 2H), 3.18 (s, 3H); LCMS (Method 1): m/z = 435.1 (M + H)$^+$; | 6.9 | 0.61 |
| 235 | 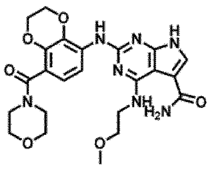 | 4-((2-methoxyethyl)ami no)-2-((8-(morpholine-4-carbonyl)-2,3-dihydrobenzo [*b*][1 ,4]dioxin-5-yl)amino)-7*H*-pyrrolo[2,3-*d*]pyrimidine-5-carboxamide | $^1$H NMR (400 MHz, DMSO-$d_6$): $\delta$ = 9.74(t, *J* = 5.6Hz, 1H), 8.18(d, *J* = 8.4Hz, 1H), 7.77(d, *J* = 5.6Hz, 1H), 7.71(t, *J* = 4.4Hz, 1H), 7.19(q, *J* = 8.0Hz, 2H), 6.75(d, *J*=8.4Hz, 1H), 6.03(m, 1H), 4.40(d, *J* = 16.0Hz, 5H), 3.54(t, *J* = 5.2Hz, 8H), 3.53(s, 7H); LCMS (Method 1): m/z = 498.2 (M + H)$^+$; | 0.8 | 0.96 |
| 236 | 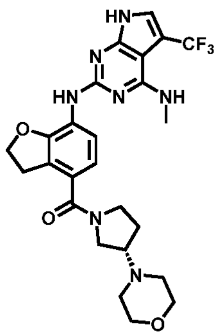 | (S)-(7-((4-(methylamino)-5-(trifluoromethyl)-7*H*-pyrrolo [2,3-*d*]pyrimidin-2-yl)amino)-2,3-dihydrobenzofuran -4-yl) (3-morpholinopyrroli din-1-yl) methanone | $^1$H NMR (400 MHz, Chloroform-*d*) $\delta$ 9.27 (s, 1H), 9.04 (s, 1H), 8.35 (t, *J* = 9.7 Hz, 1H), 7.08 (s, 2H), 6.90 (dd, *J* = 12.9, 8.3 Hz, 1H), 5.30 (s, 1H), 4.68 (h, *J* = 8.7 Hz, 2H), 3.74 - 3.70 (m, 4H), 3.67 - 3.57(m, 2H), 3.56 - 3.41 (m, 2H), 3.39 - 3.21 (m, 1H), 3.15 (d, *J* = 4.7 Hz, 3H), 2.94 - 2.72 (m, 1H), 2.60 - 2.43 (m, 3H), 2.41 - 2.32 (m, 1H), 2.27 - 2.04 (m, 1H), 1.90 - 1.75 (m, 1H). LCMS (Method 7): m/z = 532.5 [M + H]$^+$ | 43 | 1.18 |

(continued)

| Example | Structure | Compound name | $^1$H NMR, MS | Yield (%) | HPLC r.t. (min), Purity |
|---|---|---|---|---|---|
| 237 | 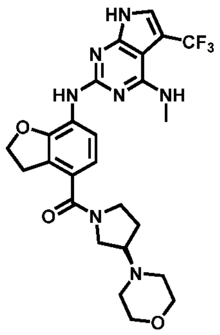 | (*R*)-(7-((4-(methylamino)-5-(trifluoromethyl)-7*H*-pyrrolo [2,3-*d*]pyrimidin-2-yl)amino)-2,3-dihydrobenzofuran -4-yl) (3-morpholinopyrroli din-1-yl) methanone | $^1$H NMR (400 MHz, Chloroform-*d*) δ 9.59 (s, 1H), 9.30 (s, 1H), 8.33 (dd, *J* = 13.4, 8.3 Hz, 1H), 7.06 (d, *J* = 12.2 Hz, 2H), 6.89 (dd, *J* = 15.2, 8.4 Hz, 1H), 5.30 (s, 1H), 4.68 (h, *J* = 8.7 Hz, 2H), 3.71 (d, *J* = 19.2 Hz, 4H), 3.61 (d, *J* = 8.1 Hz, 2H), 3.48 (p, *J* = 11.0 Hz, 2H), 3.37 - 3.23 (m, 1H), 3.15 (m, 3H), 2.92 - 2.71 (m, 1H), 2.51 (m, 3H), 2.37 (m, 1H), 2.25 - 2.04 (m, 1H), 1.81 (q, *J* = 10.4 Hz, 1H). LCMS (Method 7): m/z = 532.5 [M + H]$^+$ | 47 | 1.35 |
| 238 | 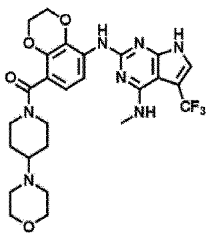 | (8-((4-(methylamino)-5-(trifluoromethyl)-7*H*-pyrrolo [2,3-*d*]pyrimidin-2-yl)amino)-2,3-dihydrobenzo[*b*][1 ,4] dioxin-5-yl)(4-morpholinopiperid in-1-yl) methanone | $^1$H NMR (400 MHz, DMSO-*d*$_6$): δ = 11.99 (d, *J* = 2.4Hz, 1H), 8.13 (d, *J* = 8.4Hz, 1H), 7.53 (s, 1H), 7.35(s, 1H), 6.72(m, 1H), 5.90(d, *J* = 4.4Hz, 1H), 4.45(t, *J* = 1.2Hz, 1H), 4.38(s, 2H), 4.29(s, 2H), 3.56 (s, 6H), 3.01(d, *J* = 4.4Hz, 3H), 2.73(t, *J* = 12.8Hz, 1H), 2.52(s, 4H), 2.32(s, 1H), 1.84 (d, *J* = 12.0Hz, 1H), 1.72(m, 1H), 1.32(m, 2H); LCMS (Method 1): m/z = 562.2 (M + H)$^+$; | 14.5 | 1.23 |

(continued)

| Example | Structure | Compound name | $^1$H NMR, MS | Yield (%) | HPLC r.t. (min), Purity |
|---|---|---|---|---|---|
| 239 | 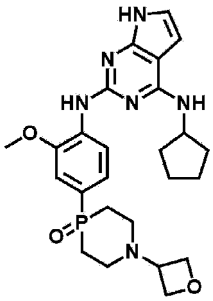 | 4-(4-((4-(cyclopentylamino )-7*H*-pyrrolo[2,3-*d*]pyrimidin-2-yl)amino)-3-methoxyphenyl)-1-(oxetan-3-yl)-1,4-azaphosphinane 4-oxide | $^1$H NMR (400 MHz, DMSO-$d_6$): δ = 11.11 (s, 1H), 8.45(t, *J* = 2.4Hz, 1H), 7.40(s, 1H), 7.28(m, 3H), 6.83 (s, 1H), 6.51(s, 1H), 4.55(t, *J* = 6.5Hz, 2H), 4.46(q, *J* = 9.6Hz, 3H), 3.97(d, *J* = 1.6Hz, 3H), 3.70(s, 2H), 2.68(m, 2H), 2.55(s, 1H), 2.01 (d, *J* = 5.2Hz, 2H), 1.85 (t, *J* = 15.2Hz, 2H), 1.82(s, 2H), 1.60(s, 4H); LCMS (Method 1): Ret.T = 0.588 min. m/z = 497.2 (M + H)$^+$; | 13 | 0.58 |
| 240 | 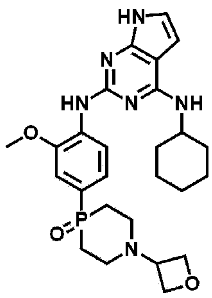 | 4-(4-((4-(cyclohexylamino) -7*H*-pyrrolo[2,3-*d*]pyrimidin-2-yl)amino)-3-methoxyphenyl)-1-(oxetan-3-yl)-1,4-azaphosphinane 4-oxide | $^1$H NMR (400 MHz, DMSO-$d_6$): δ = 11.03 (s, 1H), 8.28(d, *J* = 8.4Hz, 1H), 7.11(d, *J* = 7.6Hz, 1H), 6.96(s, 1H), 6.78(t, *J* = 3.2Hz, 1H), 6.74(t, *J* = 4.4Hz, 1H), 6.46(q, *J* = 2.0Hz, 1H), 4.60(q, *J* = 8.4Hz, 2H), 3.96(m, 1H), 3.55 (t, *J* = 4.4Hz, 4H), 3.18 (t, *J* = 8.8Hz, 2H), 2.62 (m, 2H), 2.50(s, 2H), 2.49(s, 4H), 2.38(d, *J* = 2.0Hz, 1H), 1.98(d, *J* = 11.2Hz, 2H), 1.77(d, *J* = 12.0Hz, 4H), 1.63(s, 1H), 1.35(m, 6H), 1.18 (s, 1H); LCMS (Method 1): m/z = 511.2 (M + H)$^+$; | 3.0 | 0.60 |

(continued)

| Example | Structure | Compound name | $^1$H NMR, MS | Yield (%) | HPLC r.t. (min), Purity |
|---|---|---|---|---|---|
| 241 | 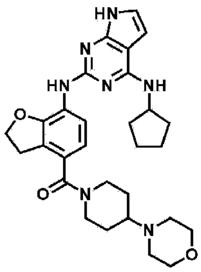 | (7-((4-(cyclopentylamino )-7*H*-pyrrolo[2,3-*d*]pyrimidin-2-yl)amino)-2,3-dihydrobenzofuran -4-yl)(4-morpholinopiperid in-1-yl) methanone | $^1$H NMR (400 MHz, DMSO-$d_6$): δ = 11.07 (s, 1H), 8.31 (d, *J* = 8.4 Hz, 1H), 7.19 (d, *J* = 7.2 Hz, 1H), 6.96 (s, 1H), 6.79 (dd, *J* = 2.4, 3.2 Hz, 1H), 6.76 (d, *J* = 8.4 Hz, 1H), 6.49 (dd, *J* = 2.0, 3.6 Hz, 1H), 4.63 (t, *J* = 8.8 Hz, 2H), 4.47 - 4.40 (m, 1H), 3.60 - 3.51 (m, 4H), 3.18 (t, *J* = 8.8 Hz, 2H), 3.03 - 2.76 (m, 2H), 2.46 ( s, 4H), 2.43 - 2.31 (m, 2H), 2.07 - 1.92 (m, 2H), 1.88 - 1.66 (m, 4H), 1.64 - 1.47 (m, 4H), 1.40 - 1.17 (m, 2H); LCMS (Method 1): m/z = 532.3 (M + H)$^+$; | 35 | 1.84 |
| 242 | 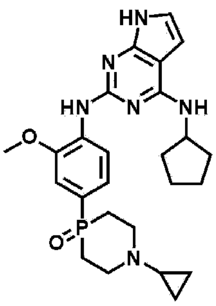 | 4-(4-((4-(cyclopentylamino )-7*H*-pyrrolo[2,3-*d*]pyrimidin-2-yl)amino)-3-methoxyphenyl)-1-cyclopropyl-1,4-azaphosphinane 4-oxide | $^1$H NMR (400 MHz, DMSO-$d_6$): δ = 11.13 (br s, 1H), 8.83 (dd, *J* = 3.3, 8.3 Hz, 1H), 7.40 (s, 1H), 7.33 (ddd, *J* = 1.3, 8.4, 11.1 Hz, 1H), 7.26 (dd, *J* = 1.5, 11.5 Hz, 2H), 6.83 (dd, *J* = 2.3, 3.3 Hz, 1H), 6.51 (dd, *J* = 1.9, 3.3 Hz, 1H), 4.54 - 4.37 (m, 1H), 3.97 (s, 3H), 3.14 - 2.85 (m, 4H), 2.18 (tdd, *J* = 5.0, 9.8, 14.6 Hz, 2H), 2.07 - 1.94 (m, 2H), 1.89 - 1.70 (m, 5H), 1.66 - 1.49 (m, 4H), 0.52 - 0.43 (m, 2H), 0.41 - 0.28 (m, 2H); LCMS (Method 1): m/z = 481.2 (M + H) +; | 7.8 | 1.85 |

(continued)

| Example | Structure | Compound name | $^1H$ NMR, MS | Yield (%) | HPLC r.t. (min), Purity |
|---|---|---|---|---|---|
| 243 | 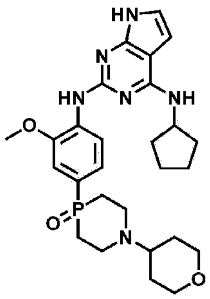 | 4-(4-((4-(cyclopentylamino )-7*H*-pyrrolo[2,3-*d*]pyrimidin-2-yl)amino)-3-methoxyphenyl)-1-(tetrahydro-2*H*-pyran-4-yl)-1,4-azaphosphinane 4-oxide | $^1H$ NMR (400 MHz, DMSO-$d_6$): δ = 11.14 (s, 1H), 8.84 (dd, *J* = 3.2, 8.4 Hz, 1H), 7.41 (s, 1H), 7.33 (m, 1H), 7.28 (s, 1H), 7.27 - 7.24 (m, 1H), 6.84 (dd, *J* = 2.0, 3.2 Hz, 1H), 6.52 (dd, *J* = 2.0, 3.2 Hz, 1H), 4.54 - 4.38 (m, 1H), 3.98 (s, 3H), 3.91 ( d, *J* = 4.0 Hz, 1H), 3.88 ( d, *J* = 3.6 Hz, 1H), 3.28 (t, *J* = 10.8 Hz, 2H), 2.99 - 2.86 (m, 4H), 2.71 - 2.64 (m, 1H), 2.26 - 2.15 (m, 2H), 2.07 - 1.99 (m, 2H), 1.91 - 1.71 (m, 4H), 1.69 - 1.44 (m, 8H); LCMS (Method 1): m/z = 525.3 (M + H)$^+$; | 13 | 2.16 |
| 244 | 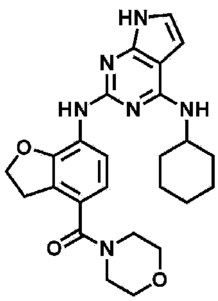 | (7-((4-(cyclohexylamino) -7*H*-pyrrolo[2,3-*d*]pyrimidin-2-yl)amino)-2,3-dihydrobenzofuran -4-yl) (morpholino)me thanone | $^1H$ NMR (400 MHz, DMSO-$d_6$): δ = 11.05 (s, 1H), 8.32 (d, *J* = 8.4 Hz, 1H), 7.12 (d, *J* = 7.8 Hz, 1H), 6.96 (s, 1H), 6.80 - 6.74 (m, 2H), 6.47 (dd, *J* = 1.9, 3.3 Hz, 1H), 4.63 (t, *J* = 8.8 Hz, 2H), 4.09 - 3.89 (m, 1H), 3.59 (br s, 4H), 3.55 - 3.40 (m, 4H), 3.21 (t, *J* = 8.8 Hz, 2H), 1.98 (br d, *J* = 11.9 Hz, 2H), 1.77 (br d, *J* = 12.4 Hz, 2H), 1.66 (br d, *J* = 12.5 Hz, 1H), 1.43 - 1.23 (m, 4H), 1.22 - 1.11 (m, 1H); LCMS (Method 1): m/z = 463.2 (M + H)$^+$; | 19.1 | 2.25 |

(continued)

| Example | Structure | Compound name | $^1H$ NMR, MS | Yield (%) | HPLC r.t. (min), Purity |
|---|---|---|---|---|---|
| 245 | 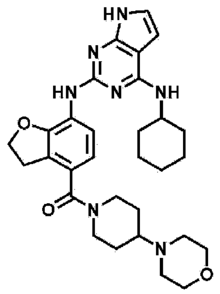 | (7-((4-(cyclohexylamino) -7*H*-pyrrolo[2,3-*d*]pyrimidin-2-yl)amino)-2,3-dihydrobenzofuran -4-yl)(4-morpholinopiperid in-1-yl) methanone | $^1H$ NMR (400 MHz, DMSO-$d_6$): δ = 11.07 (m, 1H), 8.79(q, *J* = 4.4Hz, 1H), 7.36(t, *J* = 14.8Hz, 1H),7.32(m, 2H), 7.27(s, 1H), 6.81 (q, *J* = 2.0Hz, 1H), 6.47 (d, *J* = 10.8Hz, 1H), 4.54(t, *J* = 7.6Hz, 2H), 4.42(m, 1H), 3.96(d, *J* = 15.6Hz, 4H), 3.56(m, 2H), 2.47(d, *J* = 1.2Hz, 4H), 2.01(d, *J* = 1.6Hz, 2H), 1.79(m, 4H), 1.400(s, 1H), 1.35(q, *J* = 11.6Hz, 5H), 1.24(s, 1H); LCMS (Method 1): m/z = 546.3 (M + H)$^+$; | 21.7 | 1.91 |
| 246 | 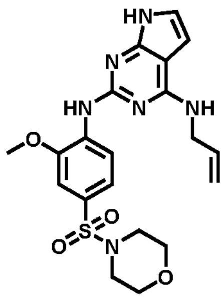 | N4-allyl-N2-(2-methoxy-4-(morpholinosulfon yl) phenyl)-7*H*-pyrrolo[2,3-*d*] pyrimidine-2,4-diamine | LCMS (Method 7): m/z = 445.51 [M + H]$^+$ | 20 | 1.40 |
| 247 | 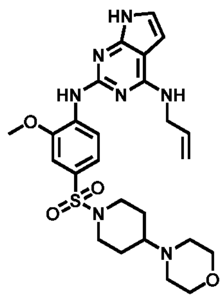 | N4-allyl-N2-(2-methoxy-4-((4-morpholinopiperid in-1-yl) sulfonyl)phenyl )-7*H*-pyrrolo [2,3-*d*] pyrimidine-2,4-diamine | LCMS (Method 7): m/z = 528.644 [M + H]$^+$ | 43 | 1.15 |

(continued)

| Example | Structure | Compound name | $^1$H NMR, MS | Yield (%) | HPLC r.t. (min), Purity |
|---|---|---|---|---|---|
| 248 | 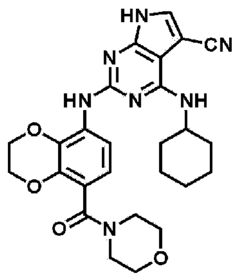 | 4-(cyclohexylamino-2-((8-(morpholine-4-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO-$d_6$): δ = 12.28 (br s, 1H), 8.08 (d, *J* = 8.4 Hz, 1H), 7.88 (s, 1H), 7.41 (s, 1H), 6.76 (d, *J* = 8.4 Hz, 1H), 5.80 (br d, *J* = 7.6 Hz, 1H), 4.38 (br d, *J* = 2.9 Hz, 2H), 4.33 (br d, *J* = 3.5 Hz, 2H), 4.05 (br dd, *J* = 2.6, 4.8 Hz, 1H), 3.61 (br s, 4H), 3.54 (br s, 2H), 3.29 - 3.19 (m, 2H), 2.06 - 1.97 (m, 2H), 1.73 (br d, *J* = 3.5 Hz, 2H), 1.67 - 1.58 (m, 1H), 1.50 - 1.33 (m, 4H), 1.32 - 1.19 (m, 1H); LCMS (Method 1): m/z = 504.2 (M + H)$^+$; | 18.7 | 2.92 |
| 249 | 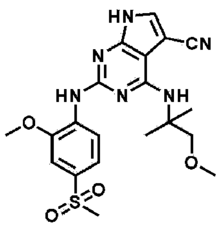 | 4-((1-methoxy-2-methylpropan-2-yl)amino)-2-((2-methoxy-4-(methylsulfonyl)p henyl) amino)-7*H*-pyrrolo[2,3-*d*] pyrimidine-5-carbonitrile | $^1$H NMR (400 MHz, DMSO-$d_6$): δ = 12.60 - 12.13 (m, 1H), 8.69 (d, *J* = 8.6 Hz, 1H), 7.92 (s, 1H), 7.81 (s, 1H), 7.50 (dd, *J* = 2.0, 8.6 Hz, 1H), 7.47 (d, *J* = 2.0 Hz, 1H), 5.89 (s, 1H), 4.01 (s, 3H), 3.51 (s, 2H), 3.36 (s, 3H), 3.20 (s, 3H), 1.52 (s, 6H); LCMS (Method 1): m/z = 445.1 (M + H)$^+$; | 25.1 | 2.99 |
| 250 | 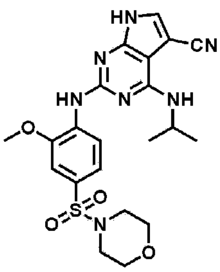 | 4-(isopropylamino)-2-((2-methoxy-4-(morpholinosulfon yl)phenyl)amino)-7*H*-pyrrolo [2,3-*d*]pyrimidine-5-carbonitrile | $^1$H NMR (400 MHz, DMSO-$d_6$): δ = 8.80 (d, *J* = 8.5 Hz, 1H), 7.94 (s, 1H), 7.77 (s, 1H), 7.35 (dd, *J* = 1.9, 8.6 Hz, 1H), 7.21 (d, *J* = 2.0 Hz, 1H), 5.93 (d, *J* = 7.8 Hz, 1H), 4.39 (dd, *J* = 6.6, 14.1 Hz, 1H), 4.00 (s, 3H), 3.70 - 3.59 (m, 4H), 2.96 - 2.83 (m, 4H), 1.30 (d, *J* = 6.5 Hz, 6H); LCMS (Method 2): m/z = 472.2 (M + H)$^+$; | 7.8 | 3.01 |

(continued)

| Example | Structure | Compound name | $^1$H NMR, MS | Yield (%) | HPLC r.t. (min), Purity |
|---|---|---|---|---|---|
| 251 | 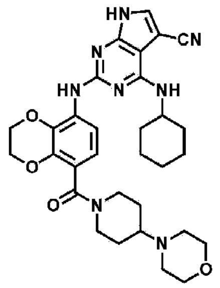 | 4-(cyclohexylamino) -2-((8-(4-morpholinopiperid ine-1-carbonyl)-2,3-dihydrobenzo [*b*][1 ,4]dioxin-5-yl)amino)-7*H*-pyrrolo[2,3-*d*]pyrimidine-5-carbonitrile | $^1$H NMR (400 MHz, DMSO) δ 12.31 (s, 1H), 9.80 (s, 1H), 8.06 (s, 1H), 7.90 (s, 1H), 7.50 (s, 1H), 6.75 (s, 1H), 4.64 (d, *J* = 12.0 Hz, 1H), 4.36 (d, *J* = 28.8 Hz, 4H), 4.02 (d, *J* = 9.7 Hz, 3H), 3.66 (s, 3H), 3.46 (s, 3H), 3.10 (s, 2H), 2.71 (s, 1H), 2.08 (d, *J* = 67.1 Hz, 4H), 1.74 (s, 2H), 1.41 (t, *J* = 71.1 Hz, 9H). LCMS (Method 7): m/z = 587.5 (M + H)$^+$ | 45.7 | 1.3 |
| 252 | 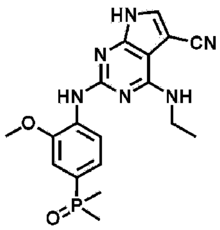 | 2-((4-(dimethylphosphor yl)-2-methoxyphenyl)a mino)-4-(ethylamino)-7*H*-pyrrolo [2,3-*d*]pyrimidine-5-carbonitrile | $^1$H NMR (400 MHz, DMSO-$d_6$): δ = 12.32 (s, 1H), 8.67(q, *J* = 3.2Hz, 1H), 7.91(s, 1H), 7.59(s, 1H), 7.32 (m, 5H), 6.46(t, *J* = 5.6Hz, 1H), 3.96(s, 3H), 3.54(m, 2H), 1.65 (d, *J* = 13.2Hz, 6H), 1.24(t, *J* = 7.2Hz, 3H); LCMS (Method 1): m/z = 385.1 (M + H)$^+$ | 50.5 | 2.47 |
| 253 | 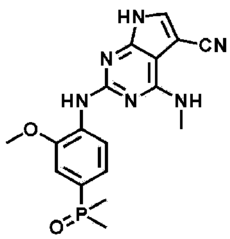 | 2-((4-(dimethylphosphor yl)-2-methoxyphenyl)a mino)-4-(methylamino)-7*H*-pyrrolo [2,3-*d*]pyrimidine-5-carbonitrile | $^1$H NMR (400 MHz, DMSO-$d_6$): δ = 12.33 (d, *J* = 1.6Hz, 1H), 8.70 (q, *J* = 3.4Hz, 1H), 7.90 (s, 1H), 7.61(s, 1H), 7.32(m, 2H), 6.55(q, *J* = 4.0Hz,1H), 3.98(d, *J* = 12.4Hz, 3H), 3.06(d, *J* = 4.4Hz, 3H), 1.66(s, 3H), 1.63(s, 3H); LCMS (Method 1): m/z = 371.1 (M + H)$^+$ | 36.9 | 2.32 |

(continued)

| Example | Structure | Compound name | $^1$H NMR, MS | Yield (%) | HPLC r.t. (min), Purity |
|---|---|---|---|---|---|
| 254 | 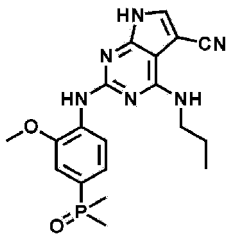 | 2-((4-(dimethylphosphor yl)-2-methoxyphenyl) amino)-4-(propylamino)-7*H*-pyrrolo [2,3-*d*]pyrimidine-5-carbonitrile | $^1$H NMR (400 MHz, DMSO-$d_6$): δ = 12.30 (s, 1H), 8.66(m, 1H), 7.91(s, 1H), 7.59(s, 1H), 7.32(m, 2H), 6.43 (t, *J* = 5.6Hz, 1H), 3.96 (s, 3H), 3.49(q, *J* = 6.2Hz, 2H), 1.66(m, 8H), 0.97(t, *J* = 7.2Hz, 3H); LCMS (Method 1): m/z = 399.1 (M + H)$^+$ | 32.5 | 2.60 |
| 255 | 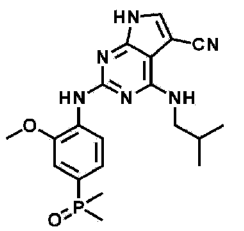 | 2-((4-(dimethylphosphor yl)-2-methoxyphenyl) amino)-4-(isobutylamino)-7*H*-pyrrolo [2,3-*d*]pyrimidine-5-carbonitrile | $^1$H NMR (400 MHz, DMSO-$d_6$): δ = 12.29 (d, *J* = 4.0Hz, 1H), 8.65 (q, *J* = 2.8Hz, 1H), 7.91 (s, 1H), 7.60(s, 1H), 7.32(m, 1H), 6.34(t, *J* = 5.6Hz, 1H), 3.96(s, 3H), 3.37(q, *J* = 6.0Hz, 2H), 2.34(s, 1H), 2.00 (s, 7H), 0.98(t, *J* = 9.6Hz, 7H); LCMS (Method 1): m/z = 413.2 (M + H)$^+$ | 16.6 | 2.73 |
| 256 | 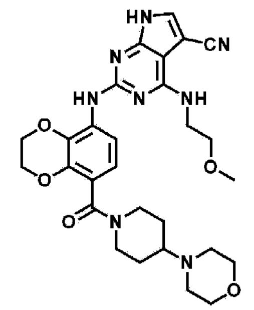 | 4-((2-methoxyethyl)ami no)-2-((8-(4-morpholinopiperid ine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_6$): δ = 12.31 (s, 1H), 8.02(d, *J* = 8.4Hz, 1H), 7.89(s, 1H), 7.43(s, 1H), 6.72 (q, *J* = 8.4Hz, 1H), 6.28 (t, *J* = 5.6Hz, 1H), 4.49 (s, 1H), 4.45(t, 2H), 4.30(s, 2H), 3.69(q, *J* = 5.6Hz, 2H), 3.57(t, *J* = 5.2Hz, 6H), 3.49(s, 1H), 3.32(t, *J* = 10.4Hz, 3H), 2.68(d, *J* = 1.6Hz, 1H), 2.51(m, 4H), 2.34 (d, *J* = 1.6Hz, 1H), 1.85 (t, *J* = 6.4Hz, 1H), 1.71 (d, *J* = 6.8Hz, 1H), 1.27 (m, 2H); LCMS (Method 1): m/z = 563.3 (M + H)$^+$ | 24.3 | 2.18 |

(continued)

| Example | Structure | Compound name | $^1H$ NMR, MS | Yield (%) | HPLC r.t. (min), Purity |
|---|---|---|---|---|---|
| 257 | 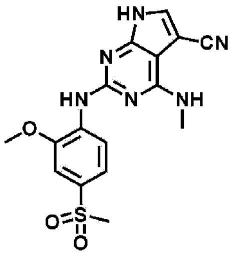 | 2-((2-methoxy-4-(methylsulfonyl)p henyl) amino)-4-(methylamino)-7*H*-pyrrolo[2,3-*d*]pyrimidine-5-carbonitrile | $^1H$ NMR (400 MHz, DMSO-$d_6$): δ = 8.84(d, *J* = 8.8Hz, 1H), 7.91(d, *J* = 5.6Hz, 1H), 7.72(s, 1H), 7.51(t, *J* = 2.4Hz, 1H), 7.45(d, *J* = 2.0Hz, 1H), 6.53(m, 1H), 4.02 (s, 3H), 3.19(s, 3H), 3.02(d, *J* = 4.4Hz, 3H); LCMS (Method 1): m/z = 373.1 $(M + H)^+$ | 7.0 | 0.72 |
| 258 | 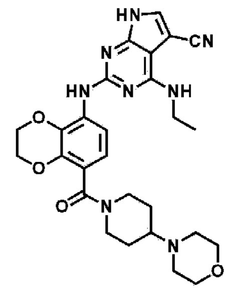 | 4-(ethylamino)-2-((8-(4-morpholinopiperid ine-1-carbonyl)-2,3-dihydrobenzo [*b*][1,4]dioxin-5-yl)amino)-7H-pyrrolo[2,3-*d*]pyrimidine-5-carbonitrile | $^1H$ NMR (400 MHz, DMSO-$d_6$): δ = 12.27 (br s, 1H), 8.06 (d, *J* = 8.5 Hz, 1H), 7.86 (s, 1H), 7.37 (s, 1H), 6.77 - 6.65 (m, 1H), 6.39 (t, *J* = 5.7 Hz, 1H), 4.50 - 4.41 (m, 1H), 4.41 - 4.25 (m, 4H), 3.55 (br d, *J* = 6.6 Hz, 4H), 3.53 - 3.47 (m, 3H), 3.04 - 2.85 (m, 1H), 2.73 (br t, *J* = 12.1 Hz, 1H), 2.45 (br s, 4H), 2.37 (br d, *J* = 10.5 Hz, 1H), 1.84 (br d, *J* = 12.5 Hz, 1H), 1.76 - 1.65 (m, 1H), 1.45 - 1.24 (m, 2H), 1.21 (t, *J* = 7.1 Hz, 3H); LCMS (Method 1): m/z = 533.3 $(M + H)^+$; | 25.5 | 2.17 |

(continued)

| Example | Structure | Compound name | $^1$H NMR, MS | Yield (%) | HPLC r.t. (min), Purity |
|---|---|---|---|---|---|
| 259 | 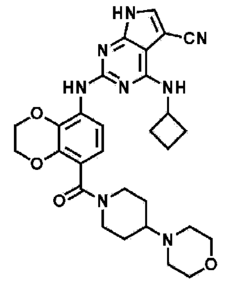 | 4-(cyclobutylamino) -2-((8-(4-morpholinopiperid ine-1-carbonyl)-2,3-dihydrobenzo [*b*][1,4]dioxin-5-yl)amino)-7*H*-pyrrolo[2,3-*d*]pyrimidine-5-carbonitrile | $^1$H NMR (400 MHz, DMSO-$d_6$): δ = 12.25 (m, 1H), 8.04(d, *J* = 8.4Hz, 1H), 7.86(s, 1H), 7.39(s, 1H), 6.74 (m, 1H), 6.34(d, *J* = 7.2Hz, 1H), 4.60(d, *J* = 7.6Hz, 1H), 4.44(m, 1H), 4.33(q, *J* = 4.33(q, *J* = 11.6Hz, 4H), 3.56 (s, 4H), 3.45(t, *J* = 40.8Hz, 1H), 2.91(s, 1H), 2.70(s, 1H), 2.50 (s, 4H), 2.34(t, *J* = 3.2Hz, 4H), 2.08(t, *J* = 10.0Hz, 2H), 1.85(d, *J* = 11.2Hz, 1H), 1.72(m, 2H), 1.29(m, 2H); LCMS (Method 1): m/z = 559.2 (M + H)$^+$ | 18.8 | 0.67 |
| 260 | 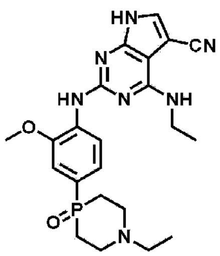 | 2-((4-(1-ethyl-4-oxido-1,4-azaphosphinan-4-yl)-2-methoxyphenyl)a mino)-4-(ethylamino)-7*H*-pyrrolo [2,3-*d*]pyrimidine-5-carbonitrile | (Method 7): m/z = 454.33 [M+H]$^+$ | 46.6 | 1.07 |
| 261 | 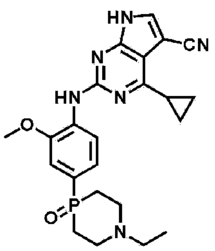 | 4-cyclopropyl-2-((4-(1-ethyl-4-oxido-1,4-azaphosphinan-4-yl)-2-methoxyphenyl)a mino)-7*H*-pyrrolo[2,3-*d*] pyrimidine-5-carbonitrile | $^1$H NMR (400 MHz, CDCl3) δ 10.85 (s, 1H), 8.65 (dd, *J* = 8.2, 3.6 Hz, 1H), 7.78 (s, 1H), 7.59 (s, 1H), 7.33 - 7.29 (m, 1H), 3.91 (s, 3H), 3.38 - 3.22 (m, 2H), 3.17 - 3.06 (m, 2H), 2.85 - 2.76 (m, 2H), 2.75 - 2.69 (m, 1H), 2.53 - 2.41 (m, 2H), 1.38 - 1.33 (m, 2H), 1.25 - 1.18 (m, 5H); (Method 7): m/z = 451.33 [M + H]$^+$ | 40 | 1.09 |

(continued)

| Example | Structure | Compound name | $^1$H NMR, MS | Yield (%) | HPLC r.t. (min), Purity |
|---|---|---|---|---|---|
| 262 | 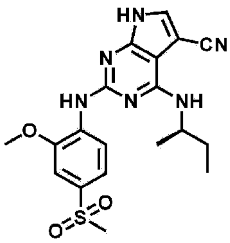 | (*R*)-4-(sec-butylamino)-2-((2-methoxy-4-(methylsulfonyl)phenyl)amino)-7*H*-pyrrolo[2,3-*d*]pyrimidine-5-carbonitrile | $^1$H NMR (400 MHz, DMSO-$d_6$): δ = 8.78(d, *J* = 8.4Hz, 1H), 7.93(s, 1H), 7.74(s, 1H), 7.50 (m, 1H), 7.45(s, 1H), 5.83(d, *J* = 8.0Hz, 1H), 4.23(q, *J* = 6.4Hz, 1H), 4.01(s, 3H), 3.20(s, 3H), 1.65(m, 2H), 1.27 (d, *J* = 6.4Hz, 3H), 0.96 (t, *J* = 7.6Hz, 3H); LCMS (Method 1): m/z = 415.2 (M + H)$^+$ | 28.3 | 415.2 |
| 263 | 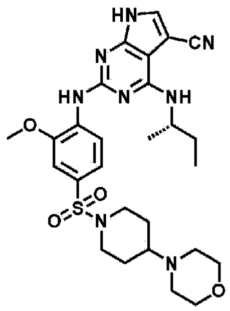 | (*S*)-4-(sec-butylamino)-2-((2-methoxy-4-((4-morpholinopiperid in-1-yl) sulfonyl)phenyl )amino)-7*H*-pyrrolo[2,3-*d*]pyrimidine-5-carbonitrile | $^1$H NMR (400 MHz, DMSO-$d_6$): δ = 8.82(d, *J* = 8.8Hz, 1H), 7.85(s, 1H), 7.66(s, 1H), 7.30 (m, 1H), 7.19(d, *J* = 1.6Hz, 1H), 5.64(q, *J* = 2.0Hz, 1H), 4.23(q, *J* = 6.4Hz, 1H), 3.99(d, *J* = 9.2Hz, 3H), 3.65(d, *J* = 11.6Hz, 2H), 3.52(t, *J* = 4.0Hz, 5H), 2.40(d, *J* = 4.4Hz, 4H), 2.39(s, 2H), 2.08(s, 1H), 1.81 (d, *J* = 10.4Hz,2H), 1.64(t, *J* = 7.2Hz, 2H), 1.40(s, 2H), 1.22(t, *J* = 6.4Hz, 3H), 0.95(t, *J* = 7.2Hz, 3H); LCMS (Method 1): m/z = 569.3 (M + H)$^+$ | 3.2 | 2.39 |
| 264 | 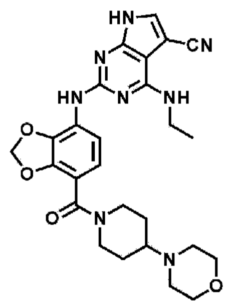 | 4-(ethylamino)-2-((7-(4-morpholino-1-carbonyl)benzo [*d*] [1,3]dioxol-4-yl)amino)-7*H*-pyrrolo[2,3-*d*]pyrimidine-5-carbonitrile | $^1$H NMR (400 MHz, DMSO-$d_6$) δ 8.16 (s, 1H), 7.81 (d, *J* = 4.1 Hz, 1H), 7.72 - 7.69 (m, 1H), 7.27 (d, *J* = 4.5 Hz, 1H), 6.81 (q, *J* = 3.5 Hz, 1H), 6.25 (d, *J* = 5.6 Hz, 1H), 6.05 (t, *J* = 3.0 Hz, 2H), 3.59 - 3.47 (m, 8H), 3.04 (d, *J* = 10.1 Hz, 2H), 2.45 (s, 4H), 1.75 (d, *J* = 3.9 Hz, 5H), 1.22 - 1.15 (m, 3H). (Method 7): m/z = 519.4 [M + H]$^+$ | 45 | 1.04 |

(continued)

| Example | Structure | Compound name | $^1$H NMR, MS | Yield (%) | HPLC r.t. (min), Purity |
|---|---|---|---|---|---|
| 265 | 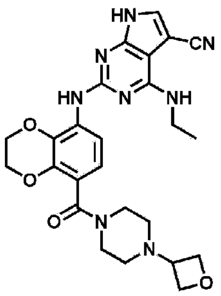 | 4-(ethylamino)-2-((8-(4-(oxetan-3-yl) piperazine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_6$) δ 8.15 - 8.08 (m, 1H), 7.83 (s, 1H), 7.33 (s, 2H), 6.76 - 6.70 (m, 1H), 6.28 (d, *J* = 2.7 Hz, 1H), 4.53 (d, *J* = 4.8 Hz, 2H), 4.47 - 4.34 (m, 4H), 4.30 (s, 2H), 3.61 (s, 2H), 3.51 (s, 1H), 3.41 (s, 4H), 2.25 (dd, *J* = 40.3, 18.8 Hz, 4H), 1.24 - 1.18 (m, 3H). (Method 7): m/z = 505.4 [M + H]$^+$ | 8 | 1.07 |
| 266 | 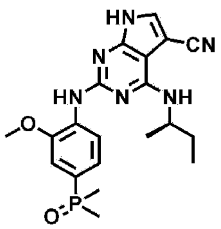 | (*R*)-4-(sec-butylamino)-2-((4-(dimethylphosphor yl)-2-methoxyphenyl)a mino)-7*H*-pyrrolo[2,3-*d*]pyrimidine-5-carbonitrile | $^1$H NMR (400 MHz, DMSO-$d_6$): δ = 12.63 - 11.92 (m, 1H), 8.64 (dd, *J* = 3.1, 8.6 Hz, 1H), 7.90 (s, 1H), 7.60 (s, 1H), 7.39 - 7.15 (m, 2H), 5.76 (br d, *J* = 8.0 Hz, 1H), 4.43 - 4.08 (m, 1H), 3.95 (s, 3H), 1.82 - 1.50 (m, 8H), 1.26 (d, *J* = 6.5 Hz, 3H), 0.95 (t, *J* = 7.4 Hz, 3H); LCMS (Method 1): m/z = 413.4(M + H)$^+$ | 41.9 | 2.77 |
| 267 | 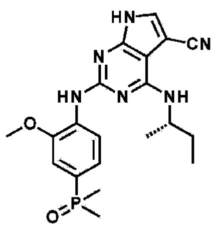 | (*S*)-4-(sec-butylamino)-2-((4-(dimethylphosphor yl)-2-methoxyphenyl)a mino)-7H-pyrrolo[2,3-*d*]pyrimidine-5-carbonitrile | $^1$H NMR (400 MHz, DMSO-$d_6$): δ = 12.75 - 11.88 (m, 1H), 8.64 (dd, *J* = 3.1, 8.6 Hz, 1H), 7.90 (s, 1H), 7.61 (s, 1H), 7.41 - 7.22 (m, 2H), 5.76 (br d, *J* = 8.0 Hz, 1H), 4.29 - 4.14 (m, 1H), 3.96 (s, 3H), 1.73 - 1.56 (m, 8H), 1.26 (d, *J* = 6.5 Hz, 3H), 0.95 (t, *J* = 7.4 Hz, 3H); LCMS (Method 1): m/z = 413.4 (M + H)$^+$; | 16.6 | 2.77 |

(continued)

| Example | Structure | Compound name | $^1H$ NMR, MS | Yield (%) | HPLC r.t. (min), Purity |
|---|---|---|---|---|---|
| 268 | 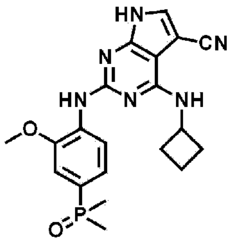 | 4-(cyclobutylamino) -2-((4-(dimethylphosphor yl)-2-methoxyphenyl)a mino)-7*H*-pyrrolo[2,3-*d*]pyrimidine-5-carbonitrile | $^1H$ NMR (400 MHz, DMSO-$d_6$): δ = 12.31 (m, 1H), 8.67(q, *J* = 3.2Hz, 1H), 7.91(s, 1H), 7.61(s, 1H),7.35 (q, *J* = 6.4Hz, 1H), 6.44 (d, *J* = 7.2Hz, 1H), 4.63 (q, *J* = 8.0Hz, 1H), 3.97 (d, *J* = 12.4Hz, 3H), 2.51(m, 2H), 2.11(d, *J* = 2.0Hz, 2H), 1.76(s, 2H), 1.64(d, *J* = 13.2Hz, 6H); LCMS (Method 1): m/z = 411.1 $(M + H)^+$ | 9.2 | 2.73 |
| 269 | 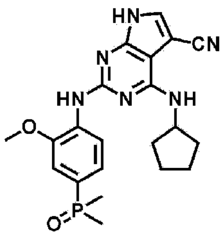 | 4-(cyclopentylamino )-2-((4-(dimethylphosphor yl)-2-methoxyphenyl)a mino)-7*H*-pyrrolo[2,3-*d*]pyrimidine-5-carbonitrile | $^1H$ NMR (400 MHz, DMSO-$d_6$): δ = 12.30 (m, 1H), 8.67(q, *J* = 3.2Hz, 1H), 7.90(d, *J* = 4.4Hz, 1H), 7.61(s, 1H), 7.32(m, 2H), 5.97 (d, *J* = 7.2Hz, 1H), 4.48 (m, 1H), 3.96(s, 3H), 2.06(q, *J* = 2.0Hz, 2H), 1.72(d, *J* = 5.6Hz, 2H), 1.60(m, 10H); LCMS (Method 1): m/z = 425.2 $(M + H)^+$ | 3.7 | 2.82 |
| 270 | 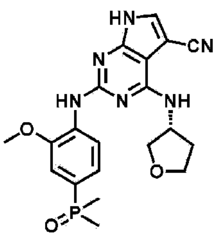 | (*R*)-2-((4-(dimethylphosphor yl)-2-methoxyphenyl)a mino)-4-((tetrahydrofuran-3-yl) amino)-7*H*-pyrrolo[2,3-*d*] pyrimidine-5-carbonitrile | $^1H$ NMR (400 MHz, DMSO-$d_6$): δ = 12.64 - 12.11 (m, 1H), 8.63 (dd, *J* = 3.1, 8.4 Hz, 1H), 7.92 (s, 1H), 7.67 (s, 1H), 7.47 - 7.22 (m, 2H), 6.28 (d, *J* = 6.3 Hz, 1H), 4.80 - 4.59 (m, 1H), 4.03 - 3.94 (m, 4H), 3.93 - 3.86 (m, 1H), 3.78 (dt, *J* = 5.9, 8.2 Hz, 1H), 3.67 (dd, *J* = 4.1, 9.0 Hz, 1H), 2.38 - 2.28 (m, 1H), 2.02 - 1.91 (m, 1H), 1.64 (d, *J* = 13.3 Hz, 6H); LCMS (Method 1): m/z = 427.4 $(M + H)^+$; | 10.5 | 2.54 |

(continued)

| Example | Structure | Compound name | $^1H$ NMR, MS | Yield (%) | HPLC r.t. (min), Purity |
|---|---|---|---|---|---|
| 271 | 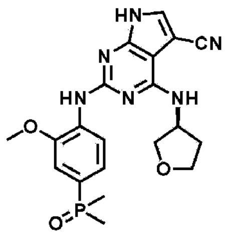 | (*S*)-2-((4-(dimethylphosphor yl)-2-methoxyphenyl)a mino)-4-((tetrahydrofuran-3-yl) amino)-7*H*-pyrrolo[2,3-*d*] pyrimidine-5-carbonitrile | $^1H$ NMR (400 MHz, DMSO-$d_6$): δ = 12.53 - 12.08 (m, 1H), 8.63 (dd, *J* = 3.1, 8.6 Hz, 1H), 7.93 (s, 1H), 7.67 (s, 1H), 7.39 - 7.29 (m, 2H), 6.29 (d, *J* = 6.5 Hz, 1H), 4.82 - 4.64 (m, 1H), 4.04 - 3.95 (m, 4H), 3.94 - 3.86 (m, 1H), 3.78 (dt, *J* = 5.9, 8.3 Hz, 1H), 3.68 (dd, *J* = 4.2, 8.9 Hz, 1H), 2.39 - 2.28 (m, 1H), 2.02 - 1.91 (m, 1H), 1.65 (d, *J* = 13.3 Hz, 6H); LCMS (Method 1): m/z = 427.4 (M + H)$^+$; | 8.7 | 2.54 |
| 272 | 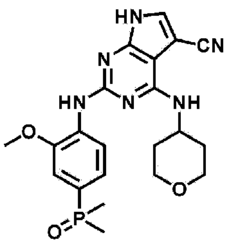 | 2-((4-(dimethylphosphor yl)-2-methoxyphenyl)a mino)-4-((tetrahydro-2*H*-pyran-4-yl) amino)-7*H*-pyrrolo[2,3-*d*] pyrimidine-5-carbonitrile | $^1H$ NMR (400 MHz, DMSO-$d_6$): δ = 12.69 - 11.91 (m, 1H), 8.62 (dd, *J* = 3.1, 8.6 Hz, 1H), 7.92 (s, 1H), 7.64 (s, 1H), 7.41 - 7.15 (m, 2H), 6.08 (d, *J* = 7.5 Hz, 1H), 4.40 - 4.17 (m, 1H), 3.96 (s, 3H), 3.94 - 3.88 (m, 2H), 3.51 (dt, *J* = 1.8, 11.4 Hz, 2H), 2.02 - 1.94 (m, 2H), 1.65 (d, *J* = 13.4 Hz, 8H); LCMS (Method 1): m/z = 441.4 (M + H)$^+$; | 7.4 | 2.58 |
| 273 | 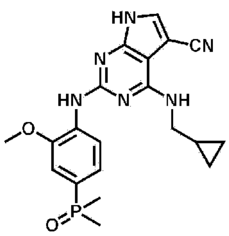 | 4-((cyclopropylmeth yl) amino)-2-((4-(dimethylphosphor yl)-2-methoxyphenyl)a mino)-7*H*-pyrrolo[2,3-*d*]pyrimidine-5-carbonitrile | $^1H$ NMR (400 MHz, DMSO-$d_6$): δ = 12.31 (d, *J* = 2.0Hz, 1H), 8.64 (q, *J* = 3.2Hz, 1H), 7.91 (s, 1H),7.60(s, 1H), 7.32(m, 2H), 6.39(t, *J* = 5.6Hz, 1H), 3.95(s, 3H), 3.42(d, *J* = 2.8Hz, 2H), 1.64(d, *J* = 13.4Hz, 6H), 1.20(m, 1H), 0.48(m, 2H), 0.32 (q, *J* = 1.2Hz, 2H); LCMS (Method 1): m/z = 411.1 (M + H)$^+$ | 11.4 | 2.27 |

(continued)

| Example | Structure | Compound name | $^1$H NMR, MS | Yield (%) | HPLC r.t. (min), Purity |
|---|---|---|---|---|---|
| 274 | 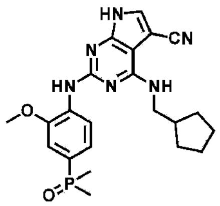 | 4-((cyclopentylmeth yl) amino)-2-((4-(dimethylphosphor yl)-2-methoxyphenyl)a mino)-7*H*-pyrrolo[2,3-*d*]pyrimidine-5-carbonitrile | $^1$H NMR (400 MHz, DMSO-$d_6$): $\delta$ = 12.30 (br d, *J* = 1.3 Hz, 1H), 8.65 (dd, *J* = 3.1, 8.7 Hz, 1H), 7.90 (s, 1H), 7.60 (s, 1H), 7.38 - 7.23 (m, 2H), 6.32 (t, *J* = 5.5 Hz, 1H), 3.96 (s, 3H), 3.49 (dd, *J* = 5.9, 7.0 Hz, 2H), 2.34 - 2.26 (m, 1H), 2.09 (s, 2H), 1.80 - 1.71 (m, 2H), 1.66 (s, 3H), 1.63 (s, 3H), 1.58 - 1.49 (m, 2H), 1.40 - 1.27 (m, 2H); LCMS (Method 1): m/z = 439.2 (M + H)$^+$; | 20.3 | 2.91 |
| 275 | 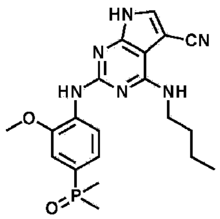 | 4-(butylamino)-2-((4-(dimethylphosphor yl)-2-methoxyphenyl)a mino)-7*H*-pyrrolo[2,3-*d*]pyrimidine-5-carbonitrile | $^1$H NMR (400 MHz, DMSO-$d_6$): $\delta$ = 12.53 - 12.06 (m, 1H), 8.65 (dd, *J* = 3.1, 7.9 Hz, 1H), 7.90 (s, 1H), 7.59 (s, 1H), 7.35 - 7.27 (m, 2H), 6.39 (t, *J* = 5.6 Hz, 1H), 3.96 (s, 3H), 3.57 - 3.50 (m, 2H), 2.09 (s, 1H), 1.65 (d, *J* = 13.4 Hz, 7H), 1.46 - 1.36 (m, 2H), 0.95 (t, *J* = 7.3 Hz, 3H); LCMS (Method 1): m/z = 413.2 (M + H)$^+$; | 22.3 | 2.76 |
| 276 | 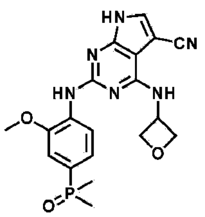 | 2-((4-(dimethylphosphor yl)-2-methoxyphenyl)a mino)-4-(oxetan-3-ylamino)-7*H*-pyrrolo[2,3-*d*]pyrimidine-5-carbonitrile | $^1$H NMR (400 MHz, DMSO-$d_6$): $\delta$ = 7.84(q, *J* = 3.2Hz, 1H), 7.55(s, 1H), 7.36(m, 2H), 4.30 (d, *J* = 5.6Hz, 1H), 4.07 (d, *J* = 10.0Hz, 1H), 3.93(t, *J* = 2.8Hz, 4H), 3.61(q, *J* = 4.0Hz, 4H), 1.68(s, 6H); LCMS (Method 1): m/z = 413.1 (M + H)$^+$ | 33.5 | 1.86 |

(continued)

| Example | Structure | Compound name | $^1$H NMR, MS | Yield (%) | HPLC r.t. (min), Purity |
|---|---|---|---|---|---|
| 277 | 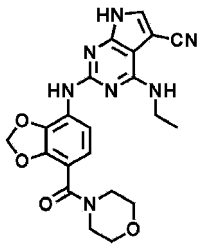 | 4-(ethylamino)-2-((7-(morpholine-4-carbonyl) benzo[*d*] [1,3]dioxol-4-yl) amino)-7*H*-pyrrolo[2,3-*d*] pyrimidine-5-carbonitrile | $^1$H NMR (400 MHz, DMSO-$d_6$) $\delta$ 7.82 (s, 1H), 7.75 - 7.64 (m, 3H), 6.85 (d, *J* = 3.4 Hz, 1H), 6.28 (d, *J* = 4.0 Hz, 1H), 6.06 (s, 2H), 3.55 (d, *J* = 32.5 Hz, 10H), 1.19 - 1.17 (m, 3H). LCMS (Method 7): m/z = 436.3 [M + H]$^+$ | 86 | 1.30 |
| 278 | 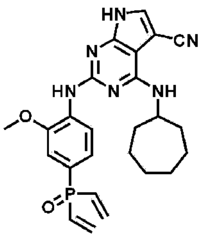 | 4-(cycloheptylamino )-2-((4-(divinylphosphoryl )-2-methoxyphenyl)a mino)-7*H*-pyrrolo[2,3-*d*]pyrimidine-5-carbonitrile | LCMS (Method 7): m/z = 477.2 [M + H]$^+$ | 47 | 1.73 |
| 279 | 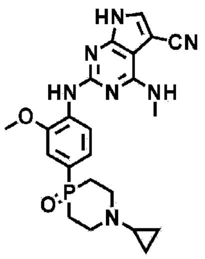 | 2-((4-(1-cyclopropyl-4-oxido-1,4-azaphosphinan-4-yl)-2-methoxyphenyl)a mino)-4-(methylamino)-7*H*-pyrrolo [2,3-*d*]pyrimidine-5-carbonitrile | $^1$H NMR (400 MHz, DMSO-$d_6$): $\delta$ = 12.42 - 12.12 (m, 1H), 8.79 - 8.65 (m, 1H), 7.95 - 7.83 (m, 1H), 7.62 (s, 1H), 7.39 - 7.26 (m, 2H), 6.63 - 6.43 (m, 1H), 4.03 - 3.89 (m, 3H), 3.02 (d, *J* = 4.5 Hz, 3H), 3.00 - 2.88 (m, 4H), 2.25 - 2.13 (m, 2H), 1.89 - 1.76 (m, 3H), 0.52 - 0.43 (m, 2H), 0.40 - 0.30 (m, 2H); LCMS (Method 1): m/z = 452.2, 226.6 (M + H)$^+$; | 8.8 | 2.15 |

(continued)

| Example | Structure | Compound name | $^1$H NMR, MS | Yield (%) | HPLC r.t. (min), Purity |
|---|---|---|---|---|---|
| 280 | 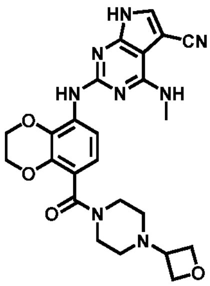 | 4-(methylamino)-2-((8-(4-(oxetan-3-yl) piperazine-1-carbonyl)-2,3-dihydrobenzo [*b*][1,4]dioxin-5-yl)amino)-7*H*-pyrrolo[2,3-*d*] pyrimidine-5-carbonitrile | $^1$H NMR (400 MHz, DMSO-$d_6$): δ = 12.48 - 11.95 (m, 1H), 8.10 (d, *J* = 8.4 Hz, 1H), 7.87 (s, 1H), 7.39 (s, 1H), 6.74 (d, *J* = 8.4 Hz, 1H), 6.55 - 6.35 (m, 1H), 4.54 (t, *J* = 6.4 Hz, 2H), 4.44 (t, *J* = 6.0 Hz, 2H), 4.38 (s, 2H), 4.31 (d, *J* = 2.8 Hz, 2H), 3.68 - 3.55 (m, 2H), 3.43 (m, 1H), 3.29 - 3.24 (m, 2H), 3.00 (d, *J* = 4.6 Hz, 3H), 2.32 - 2.13 (m, 4H); LCMS (Method 1): m/z = 491.2 (M + H)$^+$; | 5.1 | 2.09 |
| 281 | 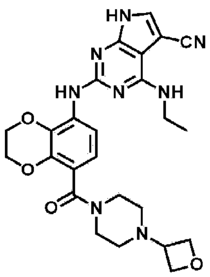 | 4-(ethylamino)-2-((8-(4-(oxetan-3-yl) piperazine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_6$) δ 8.15 - 8.08 (m, 1H), 7.83 (s, 1H), 7.33 (s, 2H), 6.76 - 6.70 (m, 1H), 6.28 (d, *J* = 2.7 Hz, 1H), 4.53 (d, *J* = 4.8 Hz, 2H), 4.47 - 4.34 (m, 4H), 4.30 (s, 2H), 3.61 (s, 2H), 3.51 (s, 1H), 3.41 (s, 4H), 2.25 (dd, *J* = 40.3, 18.8 Hz, 4H), 1.24 - 1.18 (m, 3H). LCMS (Method 7): m/z = 505.4 [M + H]$^+$ | 8 | 1.07 |

(continued)

| Example | Structure | Compound name | $^1$H NMR, MS | Yield (%) | HPLC r.t. (min), Purity |
|---|---|---|---|---|---|
| 282 | 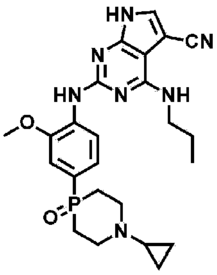 | 2-((4-(1-cyclopropyl-4-oxido-1,4-azaphosphinan-4-yl)-2-methoxyphenyl)a mino)-4-(propylamino)-7*H*-pyrrolo [2,3-*d*]pyrimidine-5-carbonitrile | $^1$H NMR (400 MHz, DMSO-$d_6$): δ = 12.43 - 12.16 (m, 1H), 8.82 - 8.49 (m, 1H), 7.99 - 7.79 (m, 1H), 7.70 - 7.50 (m, 1H), 7.43 - 7.21 (m, 2H), 6.47 - 6.33 (m, 1H), 4.00 - 3.92 (m, 3H), 3.54 - 3.44 (m, 2H), 3.07 - 2.89 (m, 4H), 2.26 - 2.11 (m, 2H), 1.91 - 1.77 (m, 3H), 1.66 (sxt, *J* = 7.3 Hz, 2H), 0.96 (t, *J* = 7.4 Hz, 3H), 0.52 - 0.43 (m, 2H), 0.39 - 0.30 (m, 2H); LCMS (Method 1): m/z = 480.2, 240.6 (M + H)$^+$; | 16.7 | 2.36 |
| 283 | 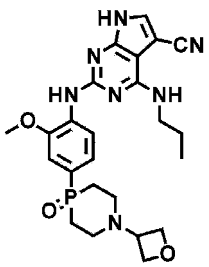 | 2-((2-methoxy-4-(1-(oxetan-3-yl)-4-oxido-1,4-azaphosphinan-4-yl)phenyl) amino)-4-(propylamino)-7*H*-pyrrolo[2,3-*d*]pyrimidine-5-carbonitrile | $^1$H NMR (400 MHz, DMSO-$d_6$): δ = 12.44 - 11.94 (m, 1H), 8.69 (dd, *J* = 3.1, 8.3 Hz, 1H), 7.90 (s, 1H), 7.62 (s, 1H), 7.41 - 7.22 (m, 2H), 6.42 (br t, *J* = 5.6 Hz, 1H), 4.59 - 4.53 (m, 2H), 4.47 - 4.41 (m, 2H), 4.04 - 3.93 (m, 3H), 3.64 - 3.56 (m, 1H), 3.54 - 3.45 (m, 2H), 2.76 - 2.64 (m, 2H), 2.63 - 2.54 (m, 2H), 2.31 - 2.22 (m, 2H), 1.93 - 1.80 (m, 2H), 1.74 - 1.61 (m, 2H), 1.00 - 0.91 (m, 3H); LCMS (Method 1): m/z = 496.2 (M + H)$^+$; | 5.4 | 2.36 |

(continued)

| Example | Structure | Compound name | $^1$H NMR, MS | Yield (%) | HPLC r.t. (min), Purity |
|---|---|---|---|---|---|
| 284 | 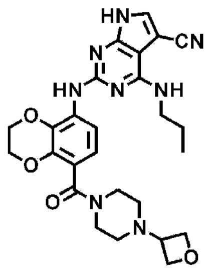 | 2-((8-(4-(oxetan-3-yl) piperazine-1-carbonyl)-2,3-dihydrobenzo[*b*][1 ,4]dioxin-5-yl)amino)-4-(propylamino)-7*H*-pyrrolo[2,3-*d*]pyrimidine-5-carbonitrile | $^1$H NMR (400 MHz, DMSO-$d_6$): δ = 12.25 ( s, 1H), 8.08 (d, *J* = 8.4 Hz, 1H), 7.87 (s, 1H), 7.38 (s, 1H), 6.73 (d, *J* = 8.4 Hz, 1H), 6.36 (t, *J* = 5.6 Hz, 1H), 4.54 (t, *J* = 6.4 Hz, 2H), 4.44 (t, *J* = 6.0 Hz, 2H), 4.38 (s, 2H), 4.31 (d, *J* = 2.8 Hz, 2H), 3.62 (d, *J* = 2.4 Hz, 2H), 3.50 - 3.42 (m, 3H), 3.30 - 3.19 (m, 2H), 2.32 - 2.11 (m, 4H), 1.65 (m, 2H), 0.95 (t, *J* = 7.2 Hz, 3H); LCMS (Method 1): m/z = 519.2 (M + H)$^+$; | 36.0 | 2.30 |
| 285 | 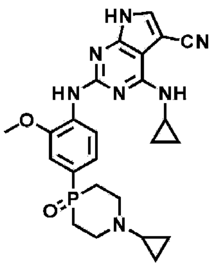 | 2-((4-(1-cyclopropyl-4-oxido-1,4-azaphosphinan-4-yl)-2-methoxyphenyl)a mino)-4-(cyclopropylamino )-7*H*-pyrrolo[2,3-*d*]pyrimidine-5-carbonitrile | $^1$H NMR (400 MHz, DMSO-$d_6$): δ = 12.66 - 12.10 (m, 1H), 8.87 (dd, *J* = 3.1, 8.3 Hz, 1H), 7.97 - 7.90 (m, 1H), 7.64 (s, 1H), 7.41 - 7.34 (m, 1H), 7.31 (dd, *J* = 1.3, 11.7 Hz, 1H), 6.65 (br s, 1H), 3.98 (s, 3H), 3.07 - 2.92 (m, 5H), 2.28 - 2.15 (m, 2H), 1.91 - 1.78 (m, 3H), 0.93 - 0.82 (m, 2H), 0.71 - 0.61 (m, 2H), 0.53 - 0.43 (m, 2H), 0.41 - 0.28 (m, 2H); LCMS (Method 1): m/z = 478.2 (M + H)$^+$ | 25.1 | 2.25 |

(continued)

| Example | Structure | Compound name | $^1$H NMR, MS | Yield (%) | HPLC r.t. (min), Purity |
|---|---|---|---|---|---|
| 286 | 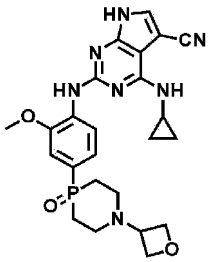 | 4-(cyclopropylamino )-2-((2-methoxy-4-(1-(oxetan-3-yl)-4-oxido-1,4-azaphosphinan-4-yl)phenyl)amino)-7*H*-pyrrolo [2,3-*d*]pyrimidine-5-carbonitrile | $^1$H NMR (400 MHz, DMSO-$d_6$): δ = 12.45 - 11.97 (m, 1H), 8.87 (dd, *J* = 3.1, 8.3 Hz, 1H), 7.91 (s, 1H), 7.63 (s, 1H), 7.42 - 7.35 (m, 1H), 7.35 - 7.27 (m, 1H), 6.65 (br s, 1H), 4.59 - 4.51 (m, 2H), 4.47 - 4.38 (m, 2H), 3.97 (s, 3H), 3.59 (quin, *J* = 6.4 Hz, 1H), 2.94 (tt, *J* = 3.5, 6.9 Hz, 1H), 2.75 - 2.65 (m, 2H), 2.61 - 2.53 (m, 2H), 2.30 - 2.19 (m, 2H), 1.93 - 1.79 (m, 2H), 0.97 - 0.80 (m, 2H), 0.72 - 0.60 (m, 2H); LCMS (Method 1): m/z = 494.2 (M + H)$^+$; | 10.6 | 2.23 |
| 287 | 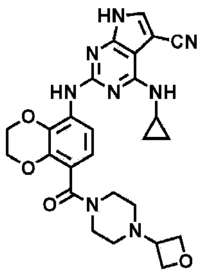 | 4-(cyclopropylamino )-2-((8-(4-(oxetan-3-yl) piperazine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_6$): δ = 12.29 ( s, 1H), 8.23 (d, *J* = 8.4 Hz, 1H), 7.89 (s, 1H), 7.38 (s, 1H), 6.75 (d, *J* = 8.4 Hz, 1H), 6.57 (br s, 1H), 4.54 (t, *J* = 6.4 Hz, 2H), 4.44 (t, *J* = 6.0 Hz, 2H), 4.39 (s, 2H), 4.31 (d, *J* = 2.8 Hz, 2H), 3.62 (s, 2H), 3.43 (m, 1H), 3.31 - 3.19 (m, 2H), 2.93 (m, 1H), 2.31 - 2.05 (m, 4H), 0.89 - 0.78 (m, 2H), 0.69 - 0.58 (m, 2H); LCMS (Method 1): m/z = 517.2 (M + H)$^+$; | 14.7 | 2.18 |

(continued)

| Example | Structure | Compound name | $^1$H NMR, MS | Yield (%) | HPLC r.t. (min), Purity |
|---|---|---|---|---|---|
| 288 | 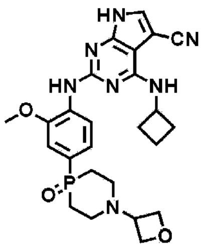 | 4-(cyclobutylamino) -2-((2-methoxy-4-(1-(oxetan-3-yl)-4-oxido-1,4-azaphosphinan-4-yl)phenyl)amino)-7*H*-pyrrolo [2,3-*d*]pyrimidine-5-carbonitrile | $^1$H NMR (400 MHz, DMSO-$d_6$): δ = 8.70 (dd, *J* = 3.1, 8.3 Hz, 1H), 7.88 (s, 1H), 7.60 (s, 1H), 7.45 - 7.29 (m, 2H), 6.37 (br d, *J* = 7.5 Hz, 1H), 4.70 - 4.59 (m, 1H), 4.57 - 4.51 (m, 2H), 4.43 (t, *J* = 6.1 Hz, 2H), 4.01 - 3.90 (m, 3H), 3.62 - 3.55 (m, 1H), 2.77 - 2.65 (m, 2H), 2.62 - 2.53 (m, 2H), 2.39 - 2.33 (m, 2H), 2.30 - 2.21 (m, 2H), 2.15 - 2.02 (m, 2H), 1.95 - 1.80 (m, 2H), 1.75 (td, *J* = 4.8, 9.7 Hz, 2H); LCMS (Method 1): m/z = 508.1 (M + H)$^+$; | 5.0 | 2.42 |
| 289 | 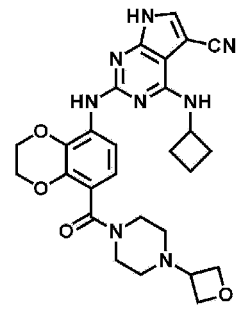 | 4-(cyclobutylamino) -2-((8-(4-(oxetan-3-yl) piperazine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_6$): δ = 12.78 - 11.93 (m, 1H), 8.12 - 8.03 (m, 1H), 7.88 (s, 1H), 7.40 (s, 1H), 6.75 (d, *J* = 8.4 Hz, 1H), 6.37 (d, *J* = 7.2 Hz, 1H), 4.64 - 4.57 (m, 1H), 4.57 - 4.51 (m, 2H), 4.44 (t, *J* = 6.0 Hz, 2H), 4.40 - 4.35 (m, 2H), 4.31 (d, *J* = 2.8 Hz, 2H), 3.70 - 3.56 (m, 2H), 3.46 - 3.40 (m, 1H), 3.30 - 3.19 (m, 2H), 2.34 - 2.19 (m, 6H), 2.12 - 2.06 (m, 2H), 1.76 - 1.70 (m, 2H); LCMS (Method 1): m/z = 531.2 (M + H)$^+$; | 16.2 | 2.38 |

(continued)

| Example | Structure | Compound name | $^1$H NMR, MS | Yield (%) | HPLC r.t. (min), Purity |
|---|---|---|---|---|---|
| 290 | 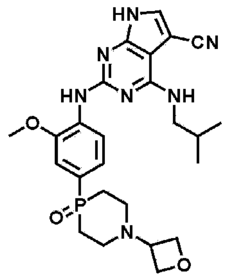 | 2-((4-(1-cyclopropyl-4-oxido-1,4-azaphosphinan-4-yl)-2-methoxyphenyl)a mino)-4-(isobutylamino)-7H-pyrrolo [2,3-d]pyrimidine-5-carbonitrile | $^1$H NMR (400 MHz, DMSO-$d_6$): δ = 12.31 (br s, 1H), 8.66 (dd, $J$ = 3.1, 8.2 Hz, 1H), 7.90 (s, 1H), 7.61 (s, 1H), 7.41 - 7.24 (m, 2H), 6.32 (t, $J$ = 5.7 Hz, 1H), 4.03 - 3.91 (m, 3H), 3.38 (t, $J$ = 6.4 Hz, 2H), 3.08 - 2.88 (m, 4H), 2.19 (tdd, $J$ = 5.0, 9.9, 14.6 Hz, 2H), 2.00 (quind, $J$ = 6.8, 13.5 Hz, 1H), 1.89 - 1.76 (m, 3H), 0.96 (d, $J$ = 6.6 Hz, 6H), 0.51 - 0.43 (m, 2H), 0.40 - 0.31 (m, 2H); LCMS (Method 1): m/z = 494.2 (M + H)$^+$; | 16.4 | 2.14 |
| 291 | 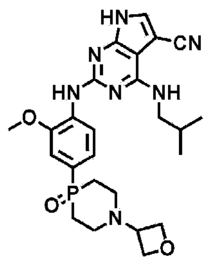 | 4-(isobutylamino)-2-((2-methoxy-4-(1-(oxetan-3-yl)-4-oxido-1,4-azaphosphinan-4-yl)phenyl)amino)-7H-pyrrolo [2,3-d]pyrimidine-5-carbonitrile | $^1$H NMR (400 MHz, DMSO-$d_6$): δ = 12.74 - 11.76 (m, 1H), 8.69 (m, 1H), 7.91 (s, 1H), 7.62 (s, 1H), 7.44 - 7.10 (m, 2H), 6.33 (m, 1H), 4.63 - 4.50 (m, 2H), 4.45 (t, $J$ = 6.0 Hz, 2H), 3.98 (s, 3H), 3.60 (t, $J$ = 6.4 Hz, 1H), 3.42 - 3.36 (m, 2H), 2.78 - 2.66 (m, 2H), 2.63 - 2.54 (m, 2H), 2.31 - 2.18 (m, 2H), 2.01 (td, $J$ = 6.8, 13.6 Hz, 1H), 1.87 (m, 2H), 0.98 (d, $J$ = 6.8 Hz, 6H); LCMS (Method 1): m/z = 510.2 (M + H)$^+$; | 5.0 | 2.45 |

(continued)

| Example | Structure | Compound name | $^1$H NMR, MS | Yield (%) | HPLC r.t. (min), Purity |
|---|---|---|---|---|---|
| 292 | 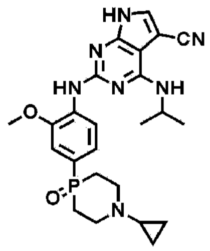 | 2-((4-(1-cyclopropyl-4-oxido-1,4-azaphosphinan-4-yl)-2-methoxyphenyl)a mino)-4-(isopropylamino)-7H-pyrrolo[2,3-d]pyrimidine-5-carbonitrile | $^1$H NMR (400 MHz, DMSO-$d_6$): δ = 12.48 - 12.19 (m, 1H), 8.68 (dd, *J* = 3.1, 8.3 Hz, 1H), 7.91 (s, 1H), 7.67 - 7.56 (m, 1H), 7.38 - 7.28 (m, 2H), 5.85 (d, *J* = 7.8 Hz, 1H), 4.46 - 4.32 (m, 1H), 3.97 (s, 3H), 2.99 - 2.92 (m, 3H), 2.26 - 2.13 (m, 2H), 1.94 - 1.75 (m, 4H), 1.30 (d, *J* = 6.4 Hz, 6H), 0.51 - 0.42 (m, 2H), 0.38 - 0.31 (m, 2H); LCMS (Method 1): m/z = 480.2 (M + H)$^+$; | 24.4 | 2.39 |
| 293 | 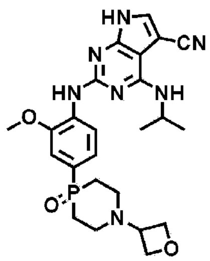 | 4-(isopropylamino)-2-((2-methoxy-4-(1-(oxetan-3-yl)-4-oxido-1,4-azaphosphinan-4-yl)phenyl)amino)-7*H*-pyrrolo [2,3-*d*]pyrimidine-5-carbonitrile | $^1$H NMR (400 MHz, DMSO-$d_6$): δ = 12.63 - 11.97 (m, 1H), 8.69 (dd, *J* = 3.1, 8.3 Hz, 1H), 7.90 (s, 1H), 7.63 (s, 1H), 7.48 - 7.25 (m, 2H), 5.85 (d, *J* = 7.8 Hz, 1H), 4.61 - 4.50 (m, 2H), 4.47 - 4.31 (m, 3H), 4.04 - 3.91 (m, 3H), 3.59 (quin, *J* = 6.5 Hz, 1H), 2.77 - 2.67 (m, 2H), 2.62 - 2.58 (m, 2H), 2.31 - 2.20 (m, 2H), 1.95 - 1.79 (m, 2H), 1.29 (d, *J* = 6.5 Hz, 6H); LCMS (Method 1): m/z = 496.2 (M + H)$^+$; | 4.1 | 2.38 |

(continued)

| Example | Structure | Compound name | $^1$H NMR, MS | Yield (%) | HPLC r.t. (min), Purity |
|---|---|---|---|---|---|
| 294 | 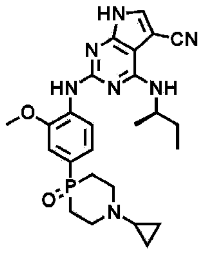 | (*R*)-4-(sec-butylamino)-2-((4-(1-cyclopropyl-4-oxido-1,4-azaphosphinan-4-yl)-2-methoxyphenyl)a mino)-7*H*-pyrrolo[2,3-*d*]pyrimidine-5-carbonitrile | $^1$H NMR (400 MHz, DMSO-$d_6$): δ = 12.38 - 12.15 (m, 1H), 8.65 (dd, *J* = 3.2, 8.2 Hz, 1H), 7.89 (s, 1H), 7.61 (s, 1H), 7.46 - 7.15 (m, 2H), 5.75 (br d, *J* = 8.1 Hz, 1H), 4.36 - 4.12 (m, 1H), 3.96 (s, 3H), 3.06 - 2.85 (m, 4H), 2.18 (dt, *J* = 4.8, 9.7 Hz, 2H), 1.89 - 1.77 (m, 3H), 1.71 - 1.52 (m, 2H), 1.30 - 1.21 (m, 3H), 0.94 (t, *J* = 7.4 Hz, 3H), 0.54 - 0.43 (m, 2H), 0.38 - 0.31 (m, 2H); LCMS (Method 1): m/z = 494.2 (M + H)$^+$; | 19.4 | 2.15 |
| 295 | 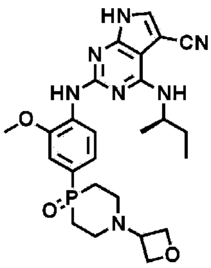 | (*R*)-4-(sec-butylamino)-2-((2-methoxy-4-(1-(oxetan-3-yl)-4-oxido-1,4-azaphosphinan-4-yl)phenyl)amino)-7*H*-pyrrolo [2,3-*d*]pyrimidine-5-carbonitrile | $^1$H NMR (400 MHz, DMSO-$d_6$): δ = 12.62 - 11.97 (m, 1H), 8.69 (dd, *J* = 3.1, 8.1 Hz, 1H), 7.89 (s, 1H), 7.62 (s, 1H), 7.40 - 7.30 (m, 2H), 5.73 (br d, *J* = 7.2 Hz, 1H), 4.55 (t, *J* = 6.5 Hz, 2H), 4.44 (t, *J* = 6.2 Hz, 2H), 4.22 (s, 1H), 3.98 (s, 3H), 3.59 (br t, *J* = 6.4 Hz, 1H), 2.76 - 2.69 (m, 2H), 2.61 (br s, 2H), 2.26 (br dd, *J* = 3.7, 4.6 Hz, 2H), 1.87 (br d, *J* = 2.6 Hz, 2H), 1.72 - 1.57 (m, 2H), 1.26 (d, *J* = 6.5 Hz, 3H), 0.95 (t, *J* = 7.5 Hz, 3H); LCMS (Method 1): m/z = 510.2 (M + H)$^+$; | 4.7 | 2.46 |

(continued)

| Example | Structure | Compound name | $^1H$ NMR, MS | Yield (%) | HPLC r.t. (min), Purity |
|---|---|---|---|---|---|
| 296 | 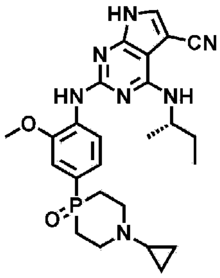 | (*S*)-4-(sec-butylamino)-2-((4-(1-cyclopropyl-4-oxido-1,4-azaphosphinan-4-yl)-2-methoxyphenyl)a mino)-7H-pyrrolo[2,3-*d*]pyrimidine-5-carbonitrile | $^1H$ NMR (400 MHz, DMSO-$d_6$): δ = 12.46 - 12.24 (m, 1H), 8.67 (dd, *J* = 3.1, 8.2 Hz, 1H), 7.91 (s, 1H), 7.63 (s, 1H), 7.42 - 7.24 (m, 2H), 5.77 (br d, *J* = 7.7 Hz, 1H), 4.35 - 4.13 (m, 1H), 3.97 (s, 3H), 3.13 - 2.79 (m, 4H), 2.26 - 2.14 (m, 2H), 1.90 - 1.77 (m, 3H), 1.74 - 1.53 (m, 2H), 1.30 - 1.22 (m, 3H), 1.00 - 0.89 (m, 3H), 0.53 - 0.44 (m, 2H), 0.40 - 0.32 (m, 2H); LCMS (Method 1): m/z = 494.2 (M + H)$^+$; | 5.7 | 2.15 |
| 297 | 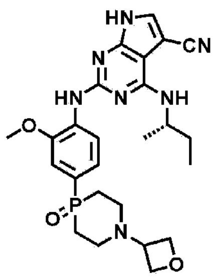 | (*S*)-4-(sec-butylamino)-2-((2-methoxy-4-(1-(oxetan-3-yl)-4-oxido-1,4-azaphosphinan-4-yl)phenyl)amino)-7*H*-pyrrolo [2,3-*d*]pyrimidine-5-carbonitrile | $^1H$ NMR (400 MHz, DMSO-$d_6$): δ = 12.56 - 11.87 (m, 1H), 8.67 (dd, *J* = 3.1, 8.3 Hz, 1H), 7.90 (s, 1H), 7.63 (s, 1H), 7.40 - 7.28 (m, 2H), 5.76 (br d, *J* = 8.3 Hz, 1H), 4.61 - 4.52 (m, 2H), 4.44 (t, *J* = 6.2 Hz, 2H), 4.29 - 4.15 (m, 1H), 4.04 - 3.90 (m, 3H), 3.59 (quin, *J* = 6.4 Hz, 1H), 2.77 - 2.68 (m, 1H), 2.61 (br s, 2H), 2.31 - 2.21 (m, 2H), 1.96 - 1.79 (m, 2H), 1.74 - 1.56 (m, 2H), 1.29 - 1.23 (m, 3H), 1.00 - 0.92 (m, 3H); LCMS (Method 1): m/z = 510.2 (M + H)$^+$; | 5.1 | 2.46 |

(continued)

| Example | Structure | Compound name | $^1H$ NMR, MS | Yield (%) | HPLC r.t. (min), Purity |
|---|---|---|---|---|---|
| 298 | 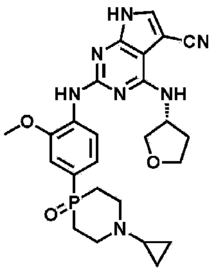 | (*R*)-2-((4-(1-cyclopropyl-4-oxido-1,4-azaphosphinan-4-yl)-2-methoxyphenyl)a mino)-4-((tetrahydrofuran-3-yl) amino)-7*H*-pyrrolo[2,3-*d*] pyrimidine-5-carbonitrile | $^1H$ NMR (400 MHz, DMSO-$d_6$): δ = 12.45 - 12.25 (m, 1H), 8.65 (dd, *J* = 3.1, 8.2 Hz, 1H), 7.93 (s, 1H), 7.69 (s, 1H), 7.43 - 7.25 (m, 2H), 6.28 (d, *J* = 6.4 Hz, 1H), 4.76 - 4.64 (m, 1H), 4.02 - 3.86 (m, 5H), 3.78 (dt, *J* = 5.9, 8.2 Hz, 1H), 3.71 - 3.64 (m, 1H), 3.08 - 2.86 (m, 4H), 2.36 - 2.32 (m, 1H), 2.25 - 2.11 (m, 2H), 1.99 - 1.74 (m, 4H), 0.54 - 0.42 (m, 2H), 0.40 - 0.29 (m, 2H); LCMS (Method 1): m/z = 508.2 (M + H)$^+$; | 25.1 | 2.29 |
| 299 | 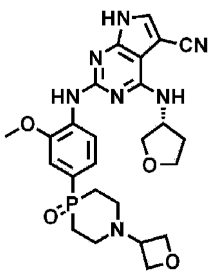 | (*R*)-2-((2-methoxy-4-(1-(oxetan-3-yl)-4-oxido-1,4-azaphosphinan-4-yl) phenyl)amino)-4-((tetrahydrofuran-3-yl) amino)-7*H*-pyrrolo[2,3-*d*] pyrimidine-5-carbonitrile | $^1H$ NMR (400 MHz, DMSO-$d_6$): δ = 12.63 - 11.95 (m, 1H), 8.67 (m, 1H), 7.93 (s, 1H), 7.70 (s, 1H), 7.54 - 7.19 (m, 2H), 6.28 (d, *J* = 6.4 Hz, 1H), 4.77 - 4.63 (m, 1H), 4.60 - 4.53 (m, 2H), 4.44 (t, *J* = 6.0 Hz, 2H), 4.02 - 3.96 (m, 4H), 3.94 - 3.87 (m, 1H), 3.79 m, 1H), 3.68 (m, 1H), 3.60 (t, *J* = 6.4 Hz, 1H), 2.77 - 2.64 (m, 2H), 2.63 - 2.54 (m, 2H), 2.39 - 2.21 (m, 4H), 2.03 - 1.76 (m, 3H); LCMS (Method 1): m/z = 524.2 (M + H)$^+$; | 9.2 | 2.27 |

(continued)

| Example | Structure | Compound name | $^1H$ NMR, MS | Yield (%) | HPLC r.t. (min), Purity |
|---|---|---|---|---|---|
| 300 | 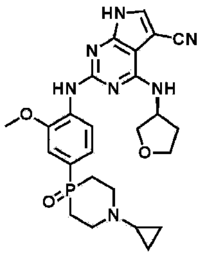 | (*S*)-2-((4-(1-cyclopropyl-4-oxido-1,4-azaphosphinan-4-yl)-2-methoxyphenyl)a mino)-4-((tetrahydrofuran-3-yl) amino)-7*H*-pyrrolo[2,3-*d*] pyrimidine-5-carbonitrile | $^1H$ NMR (400 MHz, DMSO-$d_6$): δ = 8.65 (dd, *J* = 2.8, 8.3 Hz, 1H), 7.92 (s, 1H), 7.69 (s, 1H), 7.40 - 7.34 (m, 1H), 7.33 - 7.29 (m, 1H), 7.24 - 7.17 (m, 1H), 6.32 - 6.25 (m, 1H), 4.75 - 4.68 (m, 1H), 4.01 - 3.95 (m, 3H), 3.94 - 3.87 (m, 2H), 3.81 - 3.75 (m, 1H), 3.71 - 3.65 (m, 1H), 3.07 - 2.90 (m, 4H), 2.26 - 2.10 (m, 2H), 2.02 - 1.73 (m, 5H), 0.50 - 0.43 (m, 2H), 0.38 - 0.32 (m, 2H); LCMS (Method 1): m/z = 508.2 (M + H)$^+$ | 36.3 | 2.29 |
| 301 | 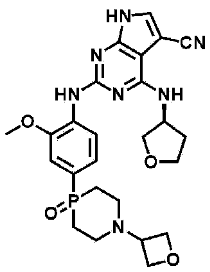 | (*S*)-2-((2-methoxy-4-(1-(oxetan-3-yl)-4-oxido-1,4-azaphosphinan-4-yl) phenyl)amino)-4-((tetrahydrofuran-3-yl) amino)-7*H*-pyrrolo[2,3-*d*] pyrimidine-5-carbonitrile | $^1H$ NMR (400 MHz, DMSO-$d_6$): δ = 8.67 (dd, *J* = 3.2, 8.3 Hz, 1H), 7.93 (s, 1H), 7.70 (s, 1H), 7.48 - 7.26 (m, 3H), 6.29 (d, *J* = 6.4 Hz, 1H), 4.77 - 4.67 (m, 1H), 4.56 (t, *J* = 6.5 Hz, 2H), 4.45 (t, J = 6.2 Hz, 2H), 4.02 - 4.00 (m, 1H), 4.00 - 3.98 (m, 3H), 3.94 - 3.87 (m, 2H), 3.84 - 3.75 (m, 2H), 3.68 (dd, J = 4.2, 9.0 Hz, 1H), 3.64 - 3.58 (m, 1H), 2.78 - 2.71 (m, 2H), 2.30 - 2.23 (m, 2H), 2.02 - 1.82 (m, 4H); LCMS (Method 1): m/z = 524.2 (M + H)$^+$; | 6.4 | 2.27 |

(continued)

| Example | Structure | Compound name | $^1$H NMR, MS | Yield (%) | HPLC r.t. (min), Purity |
|---|---|---|---|---|---|
| 302 | 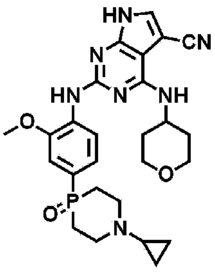 | 2-((4-(1-cyclopropyl-4-oxido-1,4-azaphosphinan-4-yl)-2-methoxyphenyl)a mino)-4-((tetrahydro-2*H*-pyran-4-yl) amino)-7*H*-pyrrolo[2,3-*d*] pyrimidine-5-carbonitrile | $^1$H NMR (400 MHz, DMSO-$d_6$): δ = 8.64 (dd, *J* = 3.0, 8.2 Hz, 1H), 7.92 (s, 1H), 7.66 (s, 1H), 7.45 - 7.13 (m, 3H), 6.09 (br d, *J* = 7.5 Hz, 1H), 4.27 (br dd, *J* = 3.6, 7.2 Hz, 1H), 3.97 (s, 3H), 3.93 - 3.89 (m, 3H), 3.55 - 3.48 (m, 2H), 3.02 - 2.88 (m, 4H), 2.24 - 2.15 (m, 2H), 1.99 (br d, *J* = 12.4 Hz, 2H), 1.85 - 1.79 (m, 2H), 1.70 - 1.61 (m, 2H), 0.53 - 0.46 (m, 2H), 0.38 - 0.34 (m, 2H); LCMS (Method 1): m/z = 522.2 (M + H)$^+$; | 14.0 | 2.32 |
| 303 | 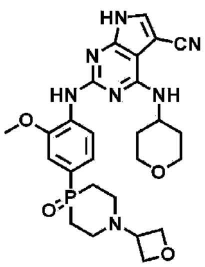 | 2-((2-methoxy-4-(1-(oxetan-3-yl)-4-oxido-1,4-azaphosphinan-4-yl)phenyl) amino)-4-((tetrahydro-2*H*-pyran-4-yl)amino)-7*H*-pyrrolo [2,3-*d*]pyrimidine-5-carbonitrile | $^1$H NMR (400 MHz, DMSO-$d_6$): δ = 12.51 - 11.98 (m, 1H), 8.65 (dd, *J* = 3.1, 8.3 Hz, 1H), 7.93 (s, 1H), 7.67 (s, 1H), 7.43 - 7.29 (m, 2H), 6.10 (br d, *J* = 7.5 Hz, 1H), 4.62 - 4.53 (m, 2H), 4.45 (t, *J* = 6.1 Hz, 2H), 4.34 - 4.20 (m, 1H), 4.00 - 3.95 (m, 3H), 3.95 - 3.85 (m, 3H), 3.64 - 3.57 (m, 1H), 3.56 - 3.47 (m, 2H), 2.72 (br dd, *J* = 5.3, 9.8 Hz, 1H), 2.58 (br d, *J* = 11.3 Hz, 2H), 2.29 - 2.20 (m, 2H), 1.99 (br d, *J* = 10.5 Hz, 2H), 1.93 - 1.78 (m, 2H), 1.73 - 1.59 (m, 2H); LCMS (Method 1): m/z = 538.2 (M + H)$^+$; | 5.9 | 2.30 |

(continued)

| Example | Structure | Compound name | $^1$H NMR, MS | Yield (%) | HPLC r.t. (min), Purity |
|---|---|---|---|---|---|
| 304 | 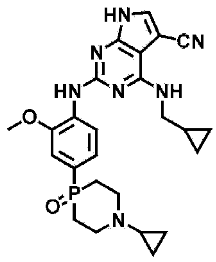 | 2-((4-(1-cyclopropyl-4-oxido-1,4-azaphosphinan-4-yl)-2-methoxyphenyl)a mino)-4-((cyclopropylmeth yl) amino)-7*H*-pyrrolo[2,3-*d*] pyrimidine-5-carbonitrile | $^1$H NMR (400 MHz, DMSO-$d_6$): δ = 12.54 - 12.08 (m, 1H), 8.68 (dd, *J* = 3.1, 8.3 Hz, 1H), 7.92 (s, 1H), 7.73 - 7.49 (m, 1H), 7.42 - 7.25 (m, 2H), 6.39 (t, *J* = 5.6 Hz, 1H), 4.02 - 3.92 (m, 3H), 3.45 - 3.41 (m, 2H), 3.07 - 2.92 (m, 4H), 2.20 (tdd, *J* = 4.8, 9.8, 14.7 Hz, 2H), 1.95 - 1.74 (m, 3H), 1.26 - 1.18 (m, 1H), 0.50 - 0.43 (m, 4H), 0.39 - 0.32 (m, 4H); LCMS (Method 1): m/z = 492.2 (M + H)$^+$ | 13.3 | 2.42 |
| 305 | 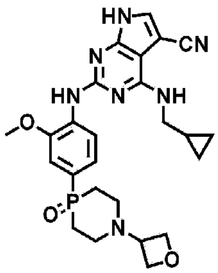 | 4-((cyclopropylmeth yl) amino)-2-((2-methoxy-4-(1-(oxetan-3-yl)-4-oxido-1,4-azaphosphinan-4-yl) phenyl)amino)-7*H*-pyrrolo [2,3-*d*]pyrimidine-5-carbonitrile | $^1$H NMR (400 MHz, DMSO-$d_6$): δ = 8.83 - 8.58 (m, 1H), 7.99 - 7.85 (m, 1H), 7.68 - 7.60 (m, 1H), 7.43 - 7.29 (m, 2H), 6.47 - 6.28 (m, 1H), 4.64 - 4.52 (m, 2H), 4.48 - 4.35 (m, 2H), 4.03 - 3.94 (m, 3H), 3.63 - 3.56 (m, 1H), 3.46 - 3.39 (m, 2H), 2.79 - 2.67 (m, 2H), 2.61 - 2.54 (m, 2H), 2.30 - 2.20 (m, 2H), 1.95 - 1.80 (m, 2H), 1.30 - 1.12 (m, 1H), 0.53 - 0.42 (m, 2H), 0.38 - 0.28 (m, 2H); LCMS (Method 1): m/z = 508.2 (M + H)$^+$; | 5.1 | 2.40 |

(continued)

| Example | Structure | Compound name | $^1$H NMR, MS | Yield (%) | HPLC r.t. (min), Purity |
|---|---|---|---|---|---|
| 306 | 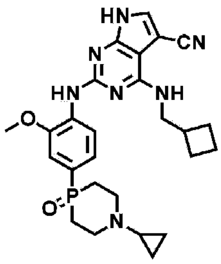 | 4-((cyclobutylmethyl )amino)-2-((4-(1-cyclopropyl-4-oxido-1,4-azaphosphinan-4-yl)-2-methoxyphenyl)a mino)-7*H*-pyrrolo[2,3-*d*]pyrimidine-5-carbonitrile | $^1$H NMR (400 MHz, DMSO-$d_6$): δ = 12.76 - 11.73 (m, 1H), 8.66 (dd, *J* = 3.1, 8.3 Hz, 1H), 7.90 (s, 1H), 7.61 (s, 1H), 7.38 - 7.25 (m, 2H), 6.29 (t, *J* = 5.6 Hz, 1H), 4.03 - 3.90 (m, 3H), 3.62 - 3.55 (m, 2H), 3.07 - 2.89 (m, 4H), 2.75 - 2.63 (m, 2H), 2.19 (tdd, *J* = 4.9, 9.9, 14.7 Hz, 2H), 2.08 - 1.98 (m, 2H), 1.88 - 1.85 (m, 2H), 1.84 - 1.75 (m, 4H), 0.54 - 0.43 (m, 2H), 0.39 - 0.32 (m, 2H); LCMS (Method 1): m/z = 506.2 (M + H)$^+$; | 20.2 | 2.19 |
| 307 | 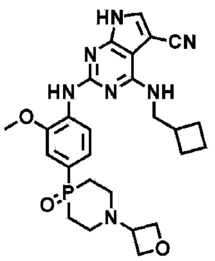 | 4-((cyclobutylmethyl )amino)-2-((2-methoxy-4-(1-(oxetan-3-yl)-4-oxido-1,4-azaphosphinan-4-yl)phenyl) amino)-7*H*-pyrrolo[2,3-*d*] pyrimidine-5-carbonitrile | $^1$H NMR (400 MHz, DMSO-$d_6$): δ = 12.58 - 12.06 (m, 1H), 8.68 (dd, *J* = 3.2, 8.3 Hz, 1H), 7.90 (s, 1H), 7.62 (s, 1H), 7.39 - 7.31 (m, 2H), 6.30 (t, *J* = 5.8 Hz, 1H), 4.60 - 4.51 (m, 2H), 4.44 (t, *J* = 6.2 Hz, 2H), 4.03 - 3.92 (m, 3H), 3.63 - 3.55 (m, 3H), 2.76 - 2.68 (m, 3H), 2.62 (br d, *J* = 6.5 Hz, 2H), 2.27 (dt, *J* = 5.6, 9.7 Hz, 2H), 2.07 - 2.00 (m, 2H), 1.92 - 1.78 (m, 6H); LAMS (Method 1): m/z = 522.2 (M + H)$^+$; | 5.1 | 2.50 |

(continued)

| Example | Structure | Compound name | $^1H$ NMR, MS | Yield (%) | HPLC r.t. (min), Purity |
|---|---|---|---|---|---|
| 308 | 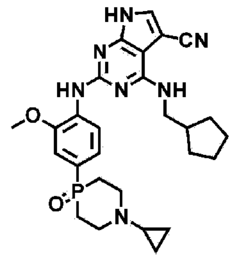 | 4-((cyclopentylmeth yl) amino)-2-((4-(1-cyclopropyl-4-oxido-1,4-azaphosphinan-4-yl)-2-methoxyphenyl)a mino)-7*H*-pyrrolo[2,3-*d*] pyrimidine-5-carbonitrile | $^1H$ NMR (400 MHz, DMSO-$d_6$): δ = 12.42 - 12.21 (m, 1H), 8.67 (dd, *J* = 3.1, 8.2 Hz, 1H), 7.90 (s, 1H), 7.62 (s, 1H), 7.39 - 7.26 (m, 2H), 6.31 (t, *J* = 5.6 Hz, 1H), 3.97 (s, 3H), 3.49 (dd, *J* = 5.9, 6.9 Hz, 2H), 3.07 - 2.90 (m, 4H), 2.33 - 2.16 (m, 3H), 1.90 - 1.70 (m, 5H), 1.67 - 1.49 (m, 4H), 1.41 - 1.26 (m, 2H), 0.53 - 0.43 (m, 2H), 0.40 - 0.31 (m, 2H); LCMS (Method 1): m/z = 520.2 (M + H)$^+$; | 27.0 | 2.26 |
| 309 | 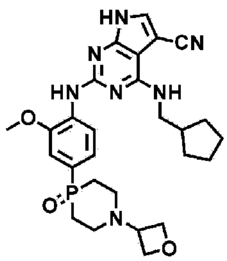 | 4-((cyclopentylmeth yl) amino)-2-((2-methoxy-4-(1-(oxetan-3-yl)-4-oxido-1,4-azaphosphinan-4-yl) phenyl)amino)-7*H*-pyrrolo [2,3-*d*]pyrimidine-5-carbonitrile | $^1H$ NMR (400 MHz, DMSO-$d_6$): δ = 12.48 - 12.16 (m, 1H), 8.69 (dd, *J* = 3.0, 8.2 Hz, 1H), 7.91 (s, 1H), 7.63 (s, 1H), 7.35 (br d, *J* = 4.9 Hz, 2H), 6.32 (br t, *J* = 5.5 Hz, 1H), 4.61 - 4.54 (m, 2H), 4.45 (t, *J* = 6.1 Hz, 2H), 3.98 (s, 4H), 3.51 - 3.46 (m, 2H), 2.31 - 2.22 (m, 4H), 1.91 - 1.81 (m, 2H), 1.79 - 1.72 (m, 2H), 1.66 - 1.61 (m, 2H), 1.57 - 1.51 (m, 2H), 1.39 - 1.30 (m, 2H), 1.27 - 1.20 (m, 3H); LCMS (Method 1): m/z = 536.3 (M + H)$^+$; | 2.5 | 2.26 |

(continued)

| Example | Structure | Compound name | $^1$H NMR, MS | Yield (%) | HPLC r.t. (min), Purity |
|---|---|---|---|---|---|
| 310 | 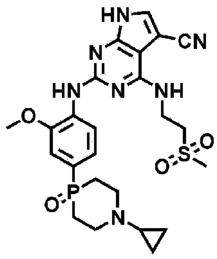 | 2-((4-(1-cyclopropyl-4-oxido-1,4-azaphosphinan-4-yl)-2-methoxyphenyl)a mino)-4-((2-(methylsulfonyl)et hyl) amino)-7H-pyrrolo[2,3-*d*] pyrimidine-5-carbonitrile | $^1$H NMR (400 MHz, DMSO-$d_6$): δ = 12.70 - 11.89 (m, 1H), 8.84 - 8.37 (m, 1H), 8.08 - 7.87 (m, 1H), 7.78 - 7.68 (m, 1H), 7.41 - 7.25 (m, 2H), 6.86 - 6.74 (m, 1H), 4.07 - 3.82 (m, 5H), 3.59 - 3.45 (m, 2H), 3.11 - 2.83 (m, 7H), 2.26 - 2.10 (m, 2H), 1.91 - 1.74 (m, 3H), 0.54 - 0.42 (m, 2H), 0.40 - 0.28 (m, 2H); LCMS (Method 1): m/z = 544.2 $(M + H)^+$; | 22.7 | 2.18 |
| 311 | 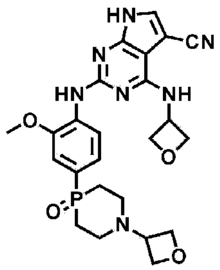 | 2-((2-methoxy-4-(1-(oxetan-3-yl)-4-oxido-1,4-azaphosphinan-4-yl)phenyl) amino)-4-(oxetan-3-ylamino)-7*H*-pyrrolo[2,3-*d*]pyrimidine-5-carbonitrile | $^1$H NMR (400 MHz, DMSO-$d_6$): δ = 12.19 - 11.95 (m, 1H), 7.94 (br s, 2H), 7.63 - 7.49 (m, 1H), 7.43 - 7.29 (m, 2H), 4.93 - 4.78 (m, 1H), 4.60 - 4.52 (m, 2H), 4.45 (t, *J* = 6.1 Hz, 2H), 4.35 - 4.25 (m, 1H), 4.13 - 4.02 (m, 1H), 3.97 - 3.85 (m, 4H), 3.67 - 3.56 (m, 2H), 3.50 - 3.39 (m, 1H), 2.75 - 2.58 (m, 4H), 2.32 - 2.22 (m, 2H), 1.99 - 1.86 (m, 2H); LCMS (Method 1): m/z = 510.2 $(M + H)^+$; | 19.7 | 2.45 |

(continued)

| Example | Structure | Compound name | $^1$H NMR, MS | Yield (%) | HPLC r.t. (min), Purity |
|---|---|---|---|---|---|
| 312 | 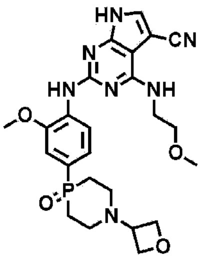 | 2-((2-methoxy-4-(1-(oxetan-3-yl)-4-oxido-1,4-azaphosphinan-4-yl)phenyl) amino)-4-((2-methoxyethyl) ami no)-7*H*-pyrrolo[2,3-*d*] pyrimidine-5-carbonitrile | $^1$H NMR (400 MHz, DMSO-$d_6$): δ = 12.57 - 11.98 (m, 1H), 8.66 (dd, *J* = 3.1, 8.2 Hz, 1H), 7.92 (s, 1H), 7.73 - 7.60 (m, 1H), 7.39 - 7.30 (m, 2H), 6.33 (t, *J* = 5.4 Hz, 1H), 4.60 - 4.51 (m, 2H), 4.44 (t, *J* = 6.2 Hz, 2H), 4.06 - 3.91 (m, 3H), 3.77 - 3.68 (m, 2H), 3.63 - 3.53 (m, 3H), 3.32 - 3.32 (m, 3H), 2.75 - 2.70 (m, 1H), 2.62 - 2.57 (m, 4H), 2.30 - 2.24 (m, 1H), 1.96 - 1.80 (m, 2H); LCMS (Method 1): m/z = 512.2 (M + H)$^+$; | 5.5 | 2.25 |
| 313 | 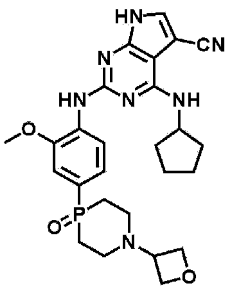 | 4-(cyclopentylamino )-2-((2-methoxy-4-(1-(oxetan-3-yl)-4-oxido-1,4-azaphosphinan-4-yl)phenyl)amino)-7*H*-pyrrolo [2,3-*d*]pyrimidine-5-carbonitrile | $^1$H NMR (400 MHz, DMSO-$d_6$): δ = 12.53 - 12.01 (m, 1H), 8.70 (dd, *J* = 3.1, 8.1 Hz, 1H), 7.90 (s, 1H), 7.64 (s, 1H), 7.40 - 7.31 (m, 2H), 5.98 (br d, *J* = 6.8 Hz, 1H), 4.55 (t, *J* = 6.5 Hz, 2H), 4.51 - 4.40 (m, 3H), 3.98 (s, 3H), 3.63 - 3.56 (m, 1H), 2.77 - 2.68 (m, 2H), 2.62 - 2.58 (m, 2H), 2.31 - 2.22 (m, 2H), 2.13 - 2.02 (m, 2H), 1.94 - 1.80 (m, 2H), 1.80 - 1.69 (m, 2H), 1.68 - 1.53 (m, 4H); LCMS (Method 1): m/z = 435.1, 437.1 (M + H)$^+$; | 5.7 | 2.74 |

(continued)

| Example | Structure | Compound name | $^1$H NMR, MS | Yield (%) | HPLC r.t. (min), Purity |
|---|---|---|---|---|---|
| 314 | 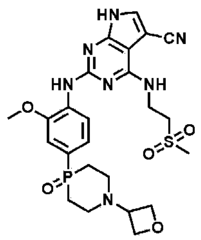 | 2-((2-methoxy-4-(1-(oxetan-3-yl)-4-oxido-1,4-azaphosphinan-4-yl)phenyl) amino)-4-((2-(methylsulfonyl) et hyl)amino)-7*H*-pyrrolo[2,3-*d*]pyrimidine-5-carbonitrile | $^1$H NMR (400 MHz, DMSO-$d_6$): δ = 12.24 (s, 1H), 8.95 - 8.35 (m, 1H), 7.94 (s, 1H), 7.74 (s, 1H), 7.40 - 7.31 (m, 2H), 6.78 (t, *J* = 5.7 Hz, 1H), 4.55 (t, *J* = 6.5 Hz, 2H), 4.44 (t, *J* = 6.1 Hz, 2H), 4.01 - 3.92 (m, 5H), 3.62 - 3.56 (m, 1H), 3.51 (br t, *J* = 6.9 Hz, 2H), 3.07 (s, 3H), 2.76 - 2.71 (m, 1H), 2.63 - 2.57 (m, 4H), 2.30 - 2.19 (m, 2H), 1.95 - 1.80 (m, 2H); LCMS (Method 1): m/z = 560.2 (M + H)$^+$; | 3.0 | 2.16 |
| 315 | 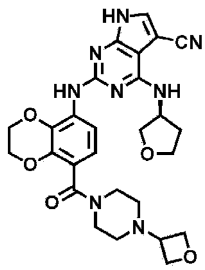 | (*S*)-2-((8-(4-(oxetan-3-yl) piperazine-1-carbonyl)-2,3-dihydrobenzo[*b*][1 ,4]dioxin-5-yl)amino)-4-((tetrahydrofuran-3-yl) amino)-7*H*-pyrrolo[2,3-*d*] pyrimidine-5-carbonitrile | $^1$H NMR (400 MHz, DMSO-$d_6$): δ = 12.34 (br s, 1H), 8.02 (d, *J* = 8.5 Hz, 1H), 7.90 (s, 1H), 7.49 (s, 1H), 6.75 (d, *J* = 8.4 Hz, 1H), 6.25 (d, *J* = 6.4 Hz, 1H), 4.71 - 4.61 (m, 1H), 4.58 - 4.50 (m, 2H), 4.43 (t, *J* = 6.0 Hz, 2H), 4.37 (br s, 2H), 4.30 (br s, 2H), 4.00 - 3.93 (m, 1H), 3.91 - 3.85 (m, 1H), 3.76 (dt, *J* = 5.9, 8.2 Hz, 1H), 3.69 - 3.57 (m, 3H), 3.42 (quin, *J* = 6.3 Hz, 1H), 3.31 - 3.20 (m, 2H), 2.33 - 2.16 (m, 5H), 1.99 - 1.89 (m, 1H); LCMS (Method 1): m/z = 547.2 (M + H)$^+$; | 10.9 | 2.23 |

(continued)

| Example | Structure | Compound name | $^1$H NMR, MS | Yield (%) | HPLC r.t. (min), Purity |
|---|---|---|---|---|---|
| 316 | 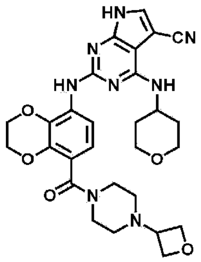 | 2-((8-(4-(oxetan-3-yl) piperazine-1-carbonyl)-2,3-dihydrobenzo[*b*][1 ,4]dioxin-5-yl)amino)-4-((tetrahydro-2*H*-pyran-4-yl)amino)-7*H*-pyrrolo[2,3-*d*]pyrimidine-5-carbonitrile | $^1$H NMR (400 MHz, DMSO-$d_6$): δ = 12.30 (br s, 1H), 8.04 (d, *J* = 8.5 Hz, 1H), 7.89 (s, 1H), 7.44 (s, 1H), 6.75 (d, *J* = 8.5 Hz, 1H), 6.04 (d, *J* = 7.6 Hz, 1H), 4.57 - 4.51 (m, 2H), 4.43 (t, *J* = 6.0 Hz, 2H), 4.37 (br s, 2H), 4.30 (br s, 2H), 4.27 - 4.18 (m, 1H), 3.94 - 3.87 (m, 2H), 3.68 - 3.57 (m, 2H), 3.52 - 3.39 (m, 3H), 3.31 - 3.20 (m, 2H), 2.31 - 2.13 (m, 4H), 2.00 - 1.93 (m, 2H), 1.71 - 1.59 (m, 2H); LCMS (Method 1): m/z = 561.3 (M + H)$^+$; | 38.5 | 2.25 |
| 317 | 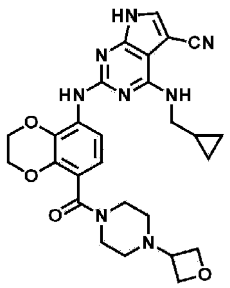 | 4-((cyclopropylmeth yl) amino)-2-((8-(4-(oxetan-3-yl) piperazine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_6$): δ = 12.44 - 12.09 (m, 1H), 8.06 (d, *J* = 8.4 Hz, 1H), 7.89 (s, 1H), 7.41 (s, 1H), 6.74 (d, *J* = 8.5 Hz, 1H), 6.37 (t, *J* = 5.6 Hz, 1H), 4.59 - 4.50 (m, 2H), 4.43 (t, *J* = 6.1 Hz, 2H), 4.37 (br s, 2H), 4.30 (br s, 2H), 3.62 (br s, 2H), 3.44 - 3.37 (m, 3H), 3.30 - 3.20 (m, 2H), 2.32 - 2.12 (m, 4H), 1.30 - 1.13 (m, 1H), 0.52 - 0.41 (m, 2H), 0.37 - 0.26 (m, 2H); LCMS (Method 1): m/z = 531.3 (M + H)$^+$; | 8.6 | 2.35 |

(continued)

| Example | Structure | Compound name | $^1$H NMR, MS | Yield (%) | HPLC r.t. (min), Purity |
|---|---|---|---|---|---|
| 318 | 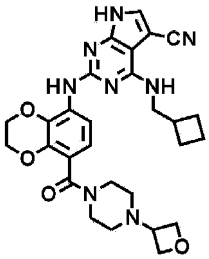 | 4-((cyclobutylmethyl )amino)-2-((8-(4-(oxetan-3-yl) piperazine-1-carbonyl)-2,3-dihydrobenzo[*b*][1 ,4]dioxin-5-yl)amino)-7*H*-pyrrolo[2,3-*d*] pyrimidine-5-carbonitrile | $^1$H NMR (400 MHz, DMSO-$d_6$): δ = 12.28 (d, *J* = 2.0Hz, 1H), 8.07 (m, 1H), 7.87(s, 1H), 7.39(s, 1H), 6.73(t, *J* = 7.6Hz, 1H), 6.27(t, *J* = 5.6Hz, 1H), 4.53(t, *J* = 6.4Hz, 2H), 4.41(m, 4H), 4.30(s, 2H), 3.55 (q, *J* = 16.4Hz, 4H), 3.42(t, *J* = 6.4Hz, 1H), 3.27(d, *J* = 2.0Hz, 2H), 2.67(d, *J* = 2.0Hz, 1H), 2.28(s, 4H), 2.01(d, *J* = 8.4Hz, 2H), 1.84(m, 4H); LCMS (Method 1): m/z = 545.2 (M + H)$^+$ | 13.5 | 2.13 |
| 319 | 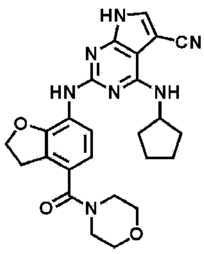 | 4-(cyclopentylamino )-2-((4-(morpholine-4-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.25 (d, *J* = 2.4 Hz, 1H), 8.05 (d, *J* = 8.3 Hz, 1H), 7.85 (d, *J* = 2.8 Hz, 1H), 7.54 (s, 1H), 6.79 (d, *J* = 8.3 Hz, 1H), 5.89 (d, *J* = 7.0 Hz, 1H), 4.62 (t, *J* = 8.8 Hz, 2H), 4.48 - 4.34 (m, 1H), 3.60 (br s, 3H), 3.55 - 3.40 (m, 3H), 3.56 (br s, 1H), 3.21 (t, *J* = 8.7 Hz, 2H), 2.11 - 1.99 (m, 2H), 1.77 - 1.68 (m, 2H), 1.65 - 1.46 (m, 4H); LCMS (Method 1): m/z = 474.2 (M + H)$^+$; | 23.4 | 2.46 |

(continued)

| Example | Structure | Compound name | $^1H$ NMR, MS | Yield (%) | HPLC r.t. (min), Purity |
|---|---|---|---|---|---|
| 320 | 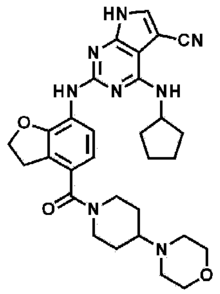 | 4-(cyclopentylamino )-2-((4-(4-morpholinopiperid ine-1-carbonyl)-2,3-dihydrobenzofuran -7-yl) amino)-7*H*-pyrrolo[2,3-*d*] pyrimidine-5-carbonitrile | $^1H$ NMR (400 MHz, DMSO-$d_6$): δ = 12.24 (d, *J* = 2.4 Hz, 1H), 8.01 (br d, *J* = 8.0 Hz, 1H), 7.85 (d, *J* = 2.8 Hz, 1H), 7.56 (s, 1H), 6.77 (d, *J* = 8.3 Hz, 1H), 5.88 (br d, *J* = 7.0 Hz, 1H), 4.62 (t, *J* = 8.7 Hz, 2H), 4.46 - 4.37 (m, 1H), 4.10 - 3.67 (m, 2H), 3.66 - 3.48 (m, 4H), 3.18 (br t, *J* = 8.6 Hz, 2H), 3.08 - 2.75 (m, 2H), 2.49 - 2.34 (m, 4H), 2.10 - 1.99 (m, 2H), 1.93 - 1.68 (m, 4H), 1.65 - 1.47 (m, 5H), 1.43 - 1.21 (m, 2H); LCMS (Method 1): m/z = 557.3 $(M + H)^+$; | 36.5 | 2.10 |
| 321 | 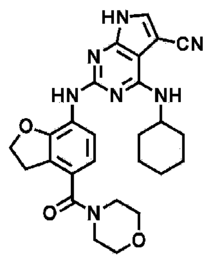 | 4-(cyclohexylamino) -2-((4-(morpholine-4-carbonyl)-2,3-dihydrobenzofuran -7-yl) amino)-7*H*-pyrrolo[2,3-*d*] pyrimidine-5-carbonitrile | $^1H$ NMR (400 MHz, DMSO-$d_6$): δ = 12.39 - 11.85 (m, 1H), 8.02 (d, *J* = 8.4 Hz, 1H), 7.84 (s, 1H), 7.56 (s, 1H), 6.77 (d, *J* = 8.3 Hz, 1H), 5.72 (d, *J* = 7.8 Hz, 1H), 4.61 (t, *J* = 8.8 Hz, 2H), 4.11 - 3.92 (m, 1H), 3.65 - 3.54 (m, 4H), 3.52 - 3.43 (m, 2H), 3.20 (t, *J* = 8.7 Hz, 2H), 2.53 - 2.51 (m, 2H), 1.98 (br s, 2H), 1.72 (br d, *J* = 3.3 Hz, 2H), 1.60 (br d, *J* = 11.6 Hz, 1H), 1.44 - 1.30 (m, 4H), 1.23 (br d, *J* = 3.3 Hz, 1H); LCMS (Method 1): m/z = 488.2 $(M + H)^+$; | 23.1 | 2.53 |

(continued)

| Example | Structure | Compound name | $^1$H NMR, MS | Yield (%) | HPLC r.t. (min), Purity |
|---|---|---|---|---|---|
| 322 | 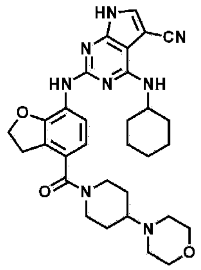 | 4-(cyclohexylamino) -2-((4-(4-morpholinopiperid ine-1-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.31 - 12.06 (m, 1H), 8.05 - 7.94 (m, 1H), 7.90 - 7.78 (m, 1H), 7.63 - 7.50 (m, 1H), 6.95 - 6.59 (m, 1H), 5.82 - 5.63 (m, 1H), 4.69 - 4.55 (m, 2H), 4.12 - 3.93 (m, 2H), 3.91 - 3.52 (m, 4H), 3.50 - 3.36 (m, 2H), 3.25 - 3.16 (m, 2H), 3.14 - 2.73 (m, 3H), 2.49 - 2.36 (m, 1H), 2.28 - 2.04 (m, 1H), 2.05 - 1.94 (m, 2H), 1.88 - 1.68 (m, 3H), 1.67 - 1.50 (m, 2H), 1.48 - 1.03 (m, 6H); LCMS (Method 1): m/z = 571.3 (M + H)$^+$; | 14.2 | 2.17 |
| 323 | 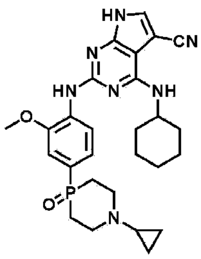 | 4-(cyclohexylamino) -2-((4-(1-cyclopropyl-4-oxido-1,4-azaphosphinan-4-yl)-2-methoxyphenyl)a mino)-7*H*-pyrrolo[2,3-*d*]pyrimidine-5-carbonitrile | $^1$H NMR (400 MHz, DMSO-$d_6$): δ = 12.33 (br s, 1H), 8.72 - 8.60 (m, 1H), 7.92 (d, *J* = 2.8 Hz, 1H), 7.64 (s, 1H), 7.41 - 7.21 (m, 2H), 5.87 (br d, *J* = 7.1 Hz, 1H), 4.13 - 4.04 (m, 1H), 3.97 (s, 3H), 3.14 - 2.83 (m, 4H), 2.27 - 2.15 (m, 2H), 2.07 - 1.97 (m, 2H), 1.85 (br d, *J* = 14.3 Hz, 2H), 1.79 - 1.73 (m, 2H), 1.64 (br d, *J* = 11.1 Hz, 1H), 1.49 - 1.38 (m, 4H), 1.32 - 1.21 (m, 1H), 0.53 - 0.44 (m, 2H), 0.42 - 0.30 (m, 2H); LCMS (Method 1): m/z = 520.3 (M + H)$^+$; | 15.7 | 2.25 |

(continued)

| Example | Structure | Compound name | $^1H$ NMR, MS | Yield (%) | HPLC r.t. (min), Purity |
|---|---|---|---|---|---|
| 324 | 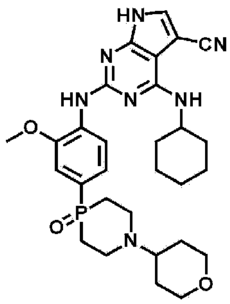 | 4-(cyclohexylamino) -2-((2-methoxy-4-(4-oxido-1-(tetrahydro-2*H*-pyran-4-yl)-1,4-azaphosphinan-4-yl) phenyl)amino)-7*H*-pyrrolo [2,3-*d*]pyrimidine-5-carbonitrile | $^1H$ NMR (400 MHz, DMSO-$d_6$): δ = 12.47 - 12.17 (m, 1H), 8.65 (dd, *J* = 3.1, 8.2 Hz, 1H), 7.90 (s, 1H), 7.62 (s, 1H), 7.35 - 7.27 (m, 2H), 5.85 (br d, *J* = 7.5 Hz, 1H), 4.11 - 4.01 (m, 1H), 3.96 (s, 3H), 3.88 (br dd, *J* = 3.6, 10.6 Hz, 2H), 3.27 (br t, *J* = 11.0 Hz, 2H), 2.99 - 2.83 (m, 4H), 2.73 - 2.63 (m, 1H), 2.27 - 2.12 (m, 2H), 2.04 - 1.94 (m, 2H), 1.91 - 1.70 (m, 4H), 1.63 (br d, *J* = 10.4 Hz, 3H), 1.54 - 1.34 (m, 6H), 1.31 - 1.18 (m, 1H); LCMS (Method 1): m/z = 564.3 (M + H)$^+$ | 4.1 | 2.54 |
| 325 | 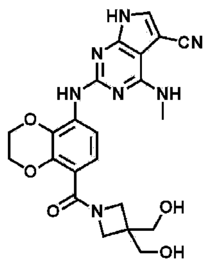 | 2-((8-(3,3-bis (hydroxymethyl )azetidine-1-carbonyl)-2,3-dihydrobenzo [*b*][1 ,4]dioxin-5-yl)amino)-4-(methylamino)-7*H*-pyrrolo [2,3-*d*]pyrimidine-5-carbonitrile | $^1H$ NMR (400 MHz, DMSO-$d_6$): δ = 12.40 - 11.99 (m, 1H), 8.12 (d, *J* = 8.6 Hz, 1H), 7.89 (s, 1H), 7.41 (s, 1H), 6.86 (d, *J* = 8.6 Hz, 1H), 6.52 (q, *J* = 4.3 Hz, 1H), 4.83 (t, *J* = 5.4 Hz, 2H), 4.41 - 4.30 (m, 4H), 3.67 (d, *J* = 1.3 Hz, 4H), 3.48 (d, *J* = 5.5 Hz, 4H), 3.00 (d, *J* = 4.5 Hz, 3H); LCMS (Method 1): m/z = 466.2 (M + H)$^+$; | 9.2 | 2.13 |
| 326 | 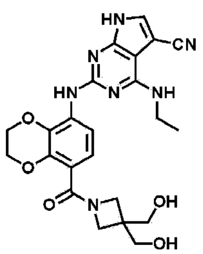 | 2-((8-(3,3-bis (hydroxymethyl )azetidine-1-carbonyl)-2,3-dihydrobenzo [*b*][1 ,4]dioxin-5-yl)amino)-4-(ethylamino)-7*H*-pyrrolo [2,3-*d*]pyrimidine-5-carbonitrile | $^1H$ NMR (400 MHz, DMSO-$d_6$): δ = 12.29 (br s, 1H), 8.10 (d, *J* = 8.6 Hz, 1H), 7.89 (s, 1H), 7.39 (s, 1H), 6.86 (d, *J* = 8.5 Hz, 1H), 6.43 (t, *J* = 5.6 Hz, 1H), 4.87 - 4.79 (m, 2H), 4.42 - 4.30 (m, 4H), 3.67 (s, 4H), 3.59 - 3.45 (m, 6H), 1.22 (t, *J* = 7.1 Hz, 3H); LCMS (Method 1): m/z = 480.2 (M + H)$^+$; | 24.4 | 2.24 |

(continued)

| Example | Structure | Compound name | $^1H$ NMR, MS | Yield (%) | HPLC r.t. (min), Purity |
|---|---|---|---|---|---|
| 327 | 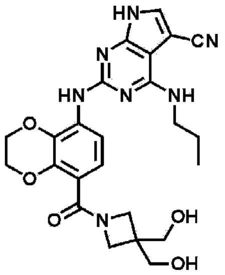 | 2-((8-(3,3-bis (hydroxymethyl )azetidine-1-carbonyl)-2,3-dihydrobenzo [*b*][1 ,4]dioxin-5-yl)amino)-4-(propylamino)-7*H*-pyrrolo [2,3-*d*]pyrimidine-5-carbonitrile | $^1H$ NMR (400 MHz, DMSO-$d_6$): δ = 12.27 (br s, 1H), 8.10 (d, *J* = 8.5 Hz, 1H), 7.89 (s, 1H), 7.39 (s, 1H), 6.85 (d, *J* = 8.5 Hz, 1H), 6.40 (t, *J* = 5.7 Hz, 1H), 4.83 (t, *J* = 5.4 Hz, 2H), 4.43 - 4.30 (m, 4H), 3.67 (s, 4H), 3.51 - 3.45 (m, 6H), 1.65 (sxt, *J* = 7.3 Hz, 2H), 0.95 (t, *J* = 7.4 Hz, 3H); LCMS (Method 1): m/z = 494.2 $(M + H)^+$; | 27.6 | 2.34 |
| 328 | 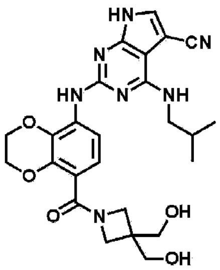 | 2-((8-(3,3-bis (hydroxymethyl )azetidine-1-carbonyl)-2,3-dihydrobenzo [*b*][1 ,4]dioxin-5-yl)amino)-4-(isobutylamino)-7*H*-pyrrolo [2,3-*d*]pyrimidine-5-carbonitrile | $^1H$ NMR (400 MHz, DMSO-$d_6$): δ = 12.29 (br s, 1H), 8.09 (d, *J* = 8.6 Hz, 1H), 7.89 (s, 1H), 7.40 (s, 1H), 6.85 (d, *J* = 8.6 Hz, 1H), 6.40 - 6.23 (m, 1H), 4.83 (t, *J* = 5.4 Hz, 2H), 4.43 - 4.31 (m, 4H), 3.67 (s, 4H), 3.48 (d, *J* = 5.5 Hz, 4H), 3.39 - 3.35 (m, 2H), 1.99 (td, *J* = 6.8, 13.5 Hz, 1H), 0.96 (d, *J* = 6.8 Hz, 6H); LCMS (Method 1): m/z = 508.2 $(M + H)^+$; | 24.0 | 2.45 |
| 329 | 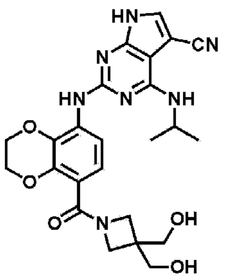 | 2-((8-(3,3-bis (hydroxymethyl )azetidine-1-carbonyl)-2,3-dihydrobenzo [*b*][1 ,4]dioxin-5-yl)amino)-4-(isopropylamino)-7H-pyrrolo[2,3-*d*]pyrimidine-5-carbonitrile | $^1H$ NMR (400 MHz, DMSO-$d_6$): δ = 12.39 - 12.16 (m, 1H), 8.08 (d, *J* = 8.6 Hz, 1H), 7.89 (s, 1H), 7.42 (s, 1H), 6.86 (d, *J* = 8.5 Hz, 1H), 5.82 (d, *J* = 7.8 Hz, 1H), 4.83 (t, *J* = 5.4 Hz, 2H), 4.42 - 4.31 (m, 5H), 3.67 (s, 4H), 3.48 (d, *J* = 5.5 Hz, 4H), 1.28 (d, *J* = 6.4 Hz, 6H); LCMS (Method 1): m/z = 494.2 $(M + H)^+$; | 5.1 | 2.37 |

(continued)

| Example | Structure | Compound name | $^1H$ NMR, MS | Yield (%) | HPLC r.t. (min), Purity |
|---|---|---|---|---|---|
| 330 | 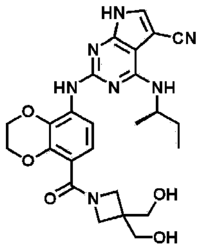 | (*R*)-2-((8-(3,3-bis (hydroxymethyl )azetidine-1-carbonyl)-2,3-dihydrobenzo [*b*][1 ,4]dioxin-5-yl)amino)-4-(sec-butylamino)-7*H*-pyrrolo[2,3-*d*]pyrimidine-5-carbonitrile | $^1H$ NMR (400 MHz, DMSO-$d_6$): δ = 12.29 (br s, 1H), 8.08 (d, *J* = 8.6 Hz, 1H), 7.89 (s, 1H), 7.41 (s, 1H), 6.86 (d, *J* = 8.5 Hz, 1H), 5.73 (brd, *J* = 8.1 Hz, 1H), 4.83 (t, *J* = 5.4 Hz, 2H), 4.42 - 4.31 (m, 4H), 4.22 - 4.12 (m, 1H), 3.67 (s, 4H), 3.48 (d, *J* = 5.4 Hz, 4H), 1.71 - 1.55 (m, 2H), 1.25 (d, *J* = 6.5 Hz, 3H), 0.94 (t, *J* = 7.4 Hz, 3H); LCMS (Method 1): m/z = 508.2 $(M + H)^+$; | 27.5 | 2.46 |
| 331 | 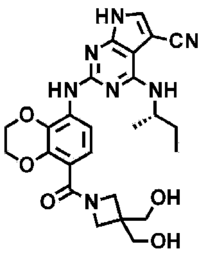 | (S)-2-((8-(3,3-bis (hydroxymethyl )azetidine-1-carbonyl)-2,3-dihydrobenzo [*b*][1 ,4]dioxin-5-yl)amino)-4-(sec-butylamino)-7*H*-pyrrolo[2,3-*d*]pyrimidine-5-carbonitrile | $^1H$ NMR (400 MHz, DMSO-$d_6$): δ = 12.30 (s, 1H), 8.08 (d, *J* = 8.4 Hz, 1H), 7.89 (s, 1H), 7.41 (s, 1H), 6.86 (d, *J* = 8.4 Hz, 1H), 5.73 ( d, *J* = 8.0 Hz, 1H), 4.83 (t, *J* = 5.6 Hz, 2H), 4.41 - 4.36 (m, 2H), 4.36 - 4.31 (m, 2H), 4.24 - 4.11 (m, 1H), 3.67 (s, 4H), 3.48 (d, *J* = 5.6 Hz, 4H), 1.78 - 1.45 (m, 2H), 1.25 (d, *J* = 6.4 Hz, 3H), 0.94 (t, *J* = 7.2 Hz, 3H); LCMS (Method 1): m/z = 508.2 $(M + H)^+$; | 25.0 | 2.46 |

(continued)

| Example | Structure | Compound name | $^1$H NMR, MS | Yield (%) | HPLC r.t. (min), Purity |
|---|---|---|---|---|---|
| 332 | 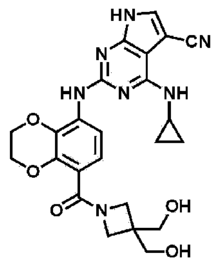 | 2-((8-(3,3-bis (hydroxymethyl )azetidine-1-carbonyl)-2,3-dihydrobenzo [*b*][1 ,4]dioxin-5-yl)amino)-4-(cyclopropylamino )-7*H*-pyrrolo[2,3-*d*]pyrimidine-5-carbonitrile | $^1$H NMR (400 MHz, DMSO-$d_6$): δ = 12.35 - 12.15 (m, 1H), 8.25 (d, *J* = 8.6 Hz, 1H), 7.90 (s, 1H), 7.40 (s, 1H), 6.87 (d, *J* = 8.5 Hz, 1H), 6.61 (br s, 1H), 4.83 (t, *J* = 5.4 Hz, 2H), 4.42 - 4.31 (m, 4H), 3.67 (d, *J* = 2.8 Hz, 4H), 3.48 (d, *J* = 5.3 Hz, 4H), 2.92 (qt, *J* = 3.5, 6.9 Hz, 1H), 0.89 - 0.78 (m, 2H), 0.70 - 0.57 (m, 2H); LCMS (Method 1): m/z = 492.2 (M + H)$^+$; | 7.4 | 2.22 |
| 333 | 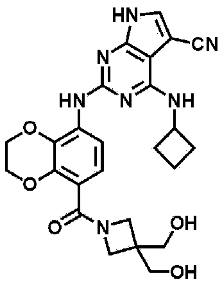 | 2-((8-(3,3-bis (hydroxymethyl )azetidine-1-carbonyl)-2,3-dihydrobenzo [*b*][1 ,4]dioxin-5-yl)amino)-4-(cyclobutylamino) -7*H*-pyrrolo[2,3-*d*]pyrimidine-5-carbonitrile | $^1$H NMR (400 MHz, DMSO-$d_6$): δ = 12.30 (br s, 1H), 8.08 (d, *J* = 8.5 Hz, 1H), 7.89 (s, 1H), 7.41 (s, 1H), 6.86 (d, *J* = 8.5 Hz, 1H), 6.40 (d, *J* = 7.1 Hz, 1H), 4.82 (t, *J* = 5.4 Hz, 2H), 4.66 - 4.53 (m, 1H), 4.42 - 4.36 (m, 2H), 4.35 - 4.29 (m, 2H), 3.66 (s, 4H), 3.47 (d, *J* = 5.5 Hz, 4H), 2.36 - 2.31 (m, 2H), 2.17 - 1.99 (m, 2H), 1.79 - 1.64 (m, 2H); LCMS (Method 1): m/z = 506.2 (M + H) +; | 9.7 | 2.42 |

(continued)

| Example | Structure | Compound name | $^1$H NMR, MS | Yield (%) | HPLC r.t. (min), Purity |
|---|---|---|---|---|---|
| 334 | 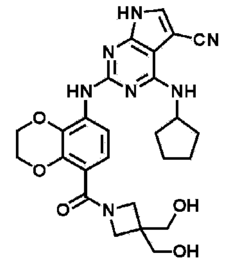 | 2-((8-(3,3-bis (hydroxymethyl )azetidine-1-carbonyl)-2,3-dihydrobenzo [*b*][1 ,4]dioxin-5-yl)amino)-4-(cyclopentylamino )-7*H*-pyrrolo[2,3-*d*]pyrimidine-5-carbonitrile | $^1$H NMR (400 MHz, DMSO-$d_6$): δ = 12.29 (d, *J* = 1.2 Hz, 1H), 8.10 (d, *J* = 8.4 Hz, 1H), 7.88 (s, 1H), 7.42 (s, 1H), 6.86 (d, *J* = 8.4 Hz, 1H), 5.95 (d, *J* = 7.2 Hz, 1H), 4.83 (t, *J* = 5.6 Hz, 2H), 4.49 - 4.41 (m, 1H), 4.39 (dd, *J* = 2.0, 5.6 Hz, 2H), 4.36 - 4.30 (m, 2H), 3.67 (s, 4H), 3.48 (d, *J* = 5.6 Hz, 4H), 2.13 - 1.91 (m, 2H), 1.79 - 1.68 (m, 2H), 1.67 - 1.47 (m, 4H); LCMS (Method 1): m/z = 520.2 (M + H)+; | 13.4 | 2.50 |
| 335 | 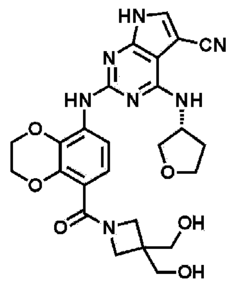 | (R)-2-((8-(3,3-bis (hydroxymethyl )azetidine-1-carbonyl)-2,3-dihydrobenzo [*b*][1 ,4]dioxin-5-yl)amino)-4-((tetrahydrofuran-3-yl) amino)-7*H*-pyrrolo[2,3-*d*] pyrimidine-5-carbonitrile | $^1$H NMR (400 MHz, DMSO-$d_6$): δ = 12.59 - 12.03 (m, 1H), 8.09 (d, *J* = 8.5 Hz, 1H), 7.89 (s, 1H), 7.42 (s, 1H), 6.87 (d, *J* = 8.5 Hz, 1H), 6.41 (br d, *J* = 7.4 Hz, 1H), 4.84 (t, *J* = 5.3 Hz, 2H), 4.71 - 4.54 (m, 1H), 4.42 - 4.37 (m, 2H), 4.36 - 4.31 (m, 2H), 3.67 (s, 4H), 3.48 (d, *J* = 5.5 Hz, 4H), 2.38 - 2.30 (m, 2H), 2.09 (dquin, *J* = 2.6, 9.3 Hz, 2H), 1.81 - 1.63 (m, 2H); LCMS (Method 1): m/z = 522.2 (M + H)$^+$; | 3.2 | 2.28 |

(continued)

| Example | Structure | Compound name | $^1$H NMR, MS | Yield (%) | HPLC r.t. (min), Purity |
|---|---|---|---|---|---|
| 336 | 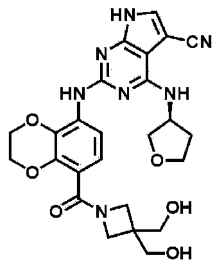 | (S)-2-((8-(3,3-bis (hydroxymethyl )azetidine-1-carbonyl)-2,3-dihydrobenzo [*b*][1 ,4]dioxin-5-yl)amino)-4-((tetrahydrofuran-3-yl) amino)-7*H*-pyrrolo[2,3-*d*] pyrimidine-5-carbonitrile | $^1$H NMR (400 MHz, DMSO-$d_6$): δ = 12.59 - 12.24 (m, 1H), 8.05 (d, *J* = 8.5 Hz, 1H), 7.91 (s, 1H), 7.49 (s, 1H), 6.87 (d, *J* = 8.5 Hz, 1H), 6.26 (d, *J* = 6.4 Hz, 1H), 4.83 (t, *J* = 5.4 Hz, 2H), 4.72 - 4.64 (m, 1H), 4.43 - 4.37 (m, 2H), 4.36 - 4.30 (m, 2H), 3.97 (dd, *J* = 6.0, 9.0 Hz, 1H), 3.93 - 3.85 (m, 1H), 3.81 - 3.72 (m, 1H), 3.67 (s, 4H), 3.65 - 3.62 (m, 1H), 3.48 (d, *J* = 5.5 Hz, 4H), 2.32 - 2.22 (m, 1H), 2.01 - 1.89 (m, 1H); LCMS (Method 1): m/z = 522.2 (M + H)$^+$; | 2.5 | 2.28 |
| 337 | 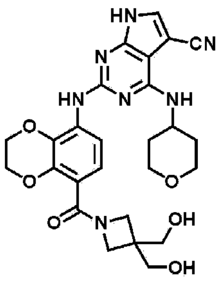 | 2-((8-(3,3-bis (hydroxymethyl )azetidine-1-carbonyl)-2,3-dihydrobenzo [b][1 ,4]dioxin-5-yl)amino)-4-((tetrahydro-2*H*-pyran-4-yl) amino)-7*H*-pyrrolo[2,3-*d*] pyrimidine-5-carbonitrile | $^1$H NMR (400 MHz, DMSO-$d_6$): δ = 12.28 (br s, 1H), 8.04 (d, *J* = 8.6 Hz, 1H), 7.88 (s, 1H), 7.42 (s, 1H), 6.84 (d, *J* = 8.6 Hz, 1H), 6.03 (d, *J* = 7.5 Hz, 1H), 4.80 (t, *J* = 5.4 Hz, 2H), 4.40 - 4.34 (m, 2H), 4.33 - 4.29 (m, 2H), 4.21 (td, *J* = 3.7, 7.5 Hz, 1H), 3.93 - 3.82 (m, 2H), 3.64 (s, 4H), 3.50 - 3.41 (m, 6H), 1.95 (br dd, *J* = 1.8, 12.2 Hz, 2H), 1.71 - 1.52 (m, 2H); LCMS (Method 1): m/z = 536.2, (M + H)$^+$; | 22.9 | 2.31 |

(continued)

| Example | Structure | Compound name | $^1$H NMR, MS | Yield (%) | HPLC r.t. (min), Purity |
|---|---|---|---|---|---|
| 338 | 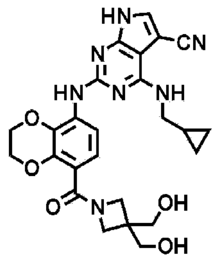 | 2-((8-(3,3-bis (hydroxymethyl )azetidine-1-carbonyl)-2,3-dihydrobenzo [*b*][1,4]dioxin-5-yl)amino)-4-((cyclopropylmeth yl) amino)-7*H*-pyrrolo[2,3-*d*] pyrimidine-5-carbonitrile | $^1$H NMR (400 MHz, DMSO-$d_6$): $\delta$ = 12.31 (br s, 1H), 8.07 (d, *J*= 8.5 Hz, 1H), 7.89 (s, 1H), 7.41 (s, 1H), 6.85 (d, *J* = 8.6 Hz, 1H), 6.38 (t, *J* = 5.5 Hz, 1H), 4.82 (t, *J* = 5.4 Hz, 2H), 4.42 - 4.36 (m, 2H), 4.35 - 4.30 (m, 2H), 3.66 (s, 4H), 3.47 (d, *J* = 5.5 Hz, 4H), 3.42 - 3.35 (m, 2H), 1.29 - 1.11 (m, 1H), 0.51 - 0.41 (m, 2H), 0.34 - 0.20 (m, 2H); LCMS (Method 1): m/z = 506.2, (M + H)$^+$; | 25.6 | 2.39 |
| 339 | 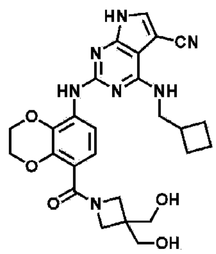 | 2-((8-(3,3-bis (hydroxymethyl )azetidine-1-carbonyl)-2,3-dihydrobenzo [*b*][1,4]dioxin-5-yl)amino)-4-((cyclobutylmethyl )amino)-7*H*-pyrrolo[2,3-*d*]pyrimidine-5-carbonitrile | $^1$H NMR (400 MHz, DMSO-$d_6$): $\delta$ = 12.28 (br s, 1H), 8.08 (d, *J*= 8.5 Hz, 1H), 7.88 (s, 1H), 7.39 (s, 1H), 6.84 (d, *J* = 8.6 Hz, 1H), 6.29 (t, *J* = 5.7 Hz, 1H), 4.82 (t, *J* = 5.4 Hz, 2H), 4.42 - 4.36 (m, 2H), 4.35 - 4.28 (m, 2H), 3.66 (s, 4H), 3.61 - 3.52 (m, 2H), 3.47 (d, *J* = 5.4 Hz, 4H), 2.70 - 2.64 (m, 1H), 2.08 - 1.96 (m, 2H), 1.91 - 1.74 (m, 4H); LCMS (Method 1): m/z = 520.2 (M + H)$^+$; | 26.1 | 2.50 |

(continued)

| Example | Structure | Compound name | $^1$H NMR, MS | Yield (%) | HPLC r.t. (min), Purity |
|---|---|---|---|---|---|
| 340 | 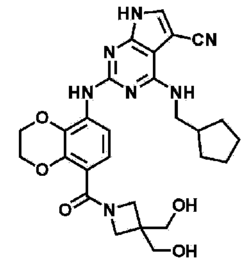 | 2-((8-(3,3-bis (hydroxymethyl )azetidine-1-carbonyl)-2,3-dihydrobenzo [*b*][1,4]dioxin-5-yl)amino)-4-((cyclopentylmeth yl) amino)-7*H*-pyrrolo[2,3-*d*] pyrimidine-5-carbonitrile | $^1$H NMR (400 MHz, DMSO-$d_6$): δ = 12.28 (br s, 1H), 8.08 (d, *J*= 8.5 Hz, 1H), 7.88 (s, 1H), 7.39 (s, 1H), 6.84 (d, *J* = 8.5 Hz, 1H), 6.29 (t, *J* = 5.5 Hz, 1H), 4.82 (t, *J* = 5.4 Hz, 2H), 4.40 - 4.36 (m, 2H), 4.34 - 4.29 (m, 2H), 3.66 (s, 4H), 3.53 - 3.41 (m, 6H), 2.31 - 2.17 (m, 1H), 1.78 - 1.68 (m, 2H), 1.67 - 1.58 (m, 2H), 1.57 - 1.46 (m, 2H), 1.36 - 1.26 (m, 2H); LCMS (Method 1): m/z = 534.2 (M + H)$^+$; | 24.3 | 2.58 |
| 341 | 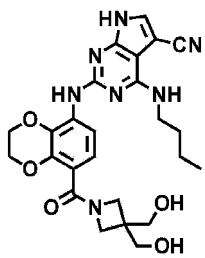 | 2-((8-(3,3-bis (hydroxymethyl )azetidine-1-carbonyl)-2,3-dihydrobenzo [*b*][1,4]dioxin-5-yl)amino)-4-(butylamino)-7*H*-pyrrolo [2,3-*d*]pyrimidine-5-carbonitrile | $^1$H NMR (400 MHz, DMSO-$d_6$): δ = 12.40 - 12.19 (m, 1H), 8.09 (d, *J*=8.6 Hz, 1H), 7.88 (s, 1H), 7.39 (s, 1H), 6.88 - 6.81 (m, 1H), 6.38 (t, *J* = 5.6 Hz, 1H), 4.83 (t, *J* = 5.4 Hz, 2H), 4.41 - 4.29 (m, 4H), 3.67 (s, 4H), 3.50 - 3.47 (m, 4H), 1.68 - 1.56 (m, 2H), 1.46 - 1.34 (m, 2H), 0.98 - 0.91 (m, 3H); LCMS (Method 1): m/z = 508.2 (M + H)$^+$; | 3.4 | 2.45 |

(continued)

| Example | Structure | Compound name | $^1H$ NMR, MS | Yield (%) | HPLC r.t. (min), Purity |
|---|---|---|---|---|---|
| 342 | 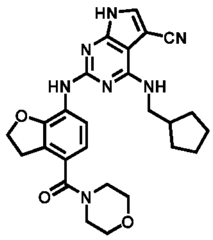 | 4-((cyclopentylmeth yl) amino)-2-((4-(morpholine-4-carbonyl)-2,3-dihydrobenzofuran -7-yl) amino)-7*H*-pyrrolo[2,3-*d*] pyrimidine-5-carbonitrile | $^1H$ NMR (400 MHz, DMSO-$d_6$): δ = 12.22 (br s, 1H), 8.04 (d, *J* = 8.4 Hz, 1H), 7.84 (s, 1H), 7.51 (s, 1H), 6.77 (d, J = 8.3 Hz, 1H), 6.24 (t, J = 5.5 Hz, 1H), 4.61 (t, *J* = 8.8 Hz, 2H), 3.59 (br s, 5H), 3.52 - 3.38 (m, 5H), 3.20 (t, *J* = 8.8 Hz, 2H), 2.26 (td, *J* = 7.4, 14.8 Hz, 1H), 1.76 - 1.65 (m, 2H), 1.63 - 1.56 (m, 2H), 1.54 - 1.45 (m, 2H), 1.37 - 1.23 (m, 2H); LCMS (Method 1): m/z = 488.2 (M + H)$^+$; | 22.4 | 2.48 |
| 343 | 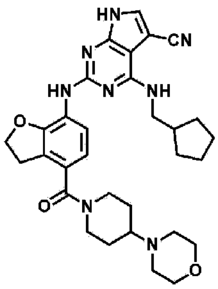 | 4-((cyclopentylmeth yl) amino)-2-((4-(4-morpholinopiperid ine-1-carbonyl)-2,3-dihydrobenzofuran -7-yl) amino)-7*H*-pyrrolo[2,3-*d*] pyrimidine-5-carbonitrile | $^1H$ NMR (400 MHz, DMSO-$d_6$): δ = 12.21 (s, 1H), 8.01 (d, *J* = 8.4 Hz, 1H), 7.84 (s, 1H), 7.50 (s, 1H), 6.75 (d, *J* = 8.4 Hz, 1H), 6.21 (t, *J* = 5.6 Hz, 1H), 4.61 (t, *J* = 8.8 Hz, 2H), 3.64 - 3.52 (m, 4H), 3.45 (m, 2H), 3.31 - 3.27 (m, 1H), 3.31 - 3.26 (m, 1H), 3.18 (t, *J* = 8.8 Hz, 2H), 3.08 - 2.74 (m, 2H), 2.47 ( s, 4H), 2.43 - 2.36 (m, 1H), 2.27 (m, 1H), 1.91 - 1.76 (m, 2H), 1.76 - 1.67 (m, 2H), 1.66 - 1.57 (m, 2H), 1.56 - 1.45 (m, 2H), 1.39 - 1.22 (m, 4H); LCMS (Method 1): m/z = 571.3.2 (M + H)$^+$; | 32.6 | 2.12 |

(continued)

| Example | Structure | Compound name | $^1$H NMR, MS | Yield (%) | HPLC r.t. (min), Purity |
|---|---|---|---|---|---|
| 344 | 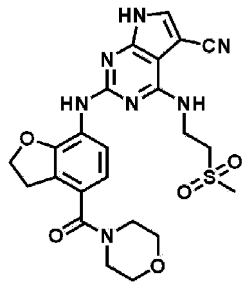 | 4-((2-(methylsulfonyl)et hyl) amino)-2-((4-(morpholine-4-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.27 (br s, 1H), 7.96 (d, *J*= 8.3 Hz, 1H), 7.88 (s, 1H), 7.79 (s, 1H), 6.78 (d, *J* = 8.3 Hz, 1H), 6.71 (t, *J* = 5.8 Hz, 1H), 4.61 (t, *J* = 8.8 Hz, 2H), 3.95 - 3.83 (m, 2H), 3.66 - 3.53 (m, 5H), 3.53 - 3.41 (m, 5H), 3.20 (t, *J*= 8.7 Hz, 2H), 3.05 - 3.01 (m, 3H); LCMS (Method 1): m/z = 512.2 $(M + H)^+$; | 32.9 | 2.00 |
| 345 | 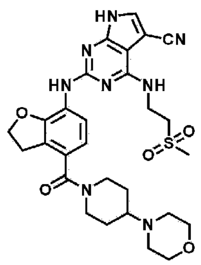 | 4-((2-(methylsulfonyl)et hyl) amino)-2-((4-(4-morpholinopiperid ine-1-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.53 - 11.87 (m, 1H), 7.95 (d, *J* = 8.4 Hz, 1H), 7.87 (s, 1H), 7.74 (s, 1H), 6.77 (d, *J* = 8.4 Hz, 1H), 6.69 (t, *J* = 5.6 Hz, 1H), 4.61 (t, *J* = 8.8 Hz, 3H), 4.09 - 3.82 (m, 3H), 3.62 - 3.54 (m, 5H), 3.49 (t, *J* = 6.8 Hz, 2H), 3.18 (t, *J* = 8.8 Hz, 2H), 2.47 (d, *J* = 4.0 Hz, 5H), 2.42 - 2.35 (m, 1H), 1.80 (d, *J* = 3.2 Hz, 2H), 1.38 - 1.25 (m, 2H); LCMS (Method 1): m/z = 595.2 $(M + H)^+$; | 21.6 | 1.74 |
| 346 | 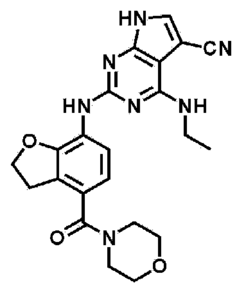 | 4-(ethylamino)-2-((4-(morpholine-4-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.23 (br s, 1H), 8.07 (d, *J*= 8.4 Hz, 1H), 7.85 (s, 1H), 7.48 (s, 1H), 6.79 (d, *J* = 8.3 Hz, 1H), 6.36 (t, *J* = 5.6 Hz, 1H), 4.62 (t, *J* = 8.8 Hz, 2H), 3.66 - 3.56 (m, 4H), 3.56 - 3.48 (m, 4H), 3.48 - 3.39 (m, 2H), 3.20 (t, *J* = 8.8 Hz, 2H), 1.20 (t, *J* = 7.1 Hz, 3H); LCMS (Method 1): m/z = 434.2 $(M + H)^+$; | 52.4 | 2.48 |

(continued)

| Example | Structure | Compound name | $^1$H NMR, MS | Yield (%) | HPLC r.t. (min), Purity |
|---|---|---|---|---|---|
| 347 | 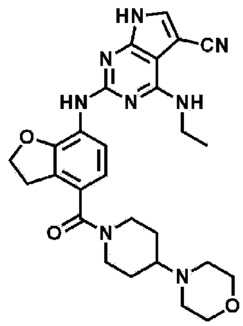 | 4-(ethylamino)-2-((4-(4-morpholinopiperid ine-1-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.24 (s, 1H), 8.03 (d, *J* = 8.4 Hz, 1H), 7.85 (s, 1H), 7.49 (s, 1H), 6.76 (d, *J* = 8.4 Hz, 1H), 6.36 (t, *J* = 5.6 Hz, 1H), 4.62 (t, *J* = 8.8 Hz, 2H), 4.53 - 4.25 (m, 1H), 3.80 - 3.59 (m, 1H), 3.65 - 3.47 (m, 7H), 3.17 (t, *J*= 8.8 Hz, 2H), 3.10 - 2.71 (m, 2H), 2.49 - 2.35 (m, 5H), 1.81 ( d, *J* = 2.4 Hz, 2H), 1.31 (d, *J* = 9.6 Hz, 2H), 1.21 (t, *J* = 7.2 Hz, 3H); LCMS (Method 1): m/z = 517.3 (M + H)$^+$; | 45.5 | 2.18 |
| 348 | 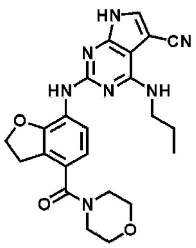 | 2-((4-(morpholine-4-carbonyl)-2,3-dihydrobenzofuran -7-yl) amino)-4-(propylamino)-7*H*-pyrrolo[2,3-*d*]pyrimidine-5-carbonitrile | $^1$H NMR (400 MHz, DMSO-$d_6$): δ = 12.22 (br s, 1H), 8.05 (d, *J*= 8.3 Hz, 1H), 7.84 (s, 1H), 7.49 (s, 1H), 6.77 (d, *J* = 8.4 Hz, 1H), 6.34 (t, *J* = 5.7 Hz, 1H), 4.61 (t, *J* = 8.8 Hz, 2H), 3.59 (br s, 4H), 3.55 - 3.36 (m, 6H), 3.20 (t, *J*= 8.8 Hz, 2H), 1.71 - 1.54 (m, 2H), 0.92 (t, *J* = 7.4 Hz, 3H); LCMS (Method 1): m/z = 448.2 (M + H)$^+$; | 50.3 | 2.60 |

(continued)

| Example | Structure | Compound name | $^1$H NMR, MS | Yield (%) | HPLC r.t. (min), Purity |
|---|---|---|---|---|---|
| 349 | 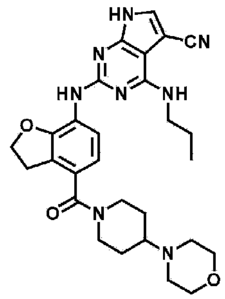 | 2-((4-(4-morpholinopiperidine-1-carbonyl)-2,3-dihydrobenzofuran -7-yl) amino)-4-(propylamino)-7*H*-pyrrolo[2,3-*d*]pyrimidine-5-carbonitrile | $^1$H NMR (400 MHz, DMSO-$d_6$): δ = 12.32 (s, 1H), 8.11 (d, *J* = 8.4 Hz, 1H), 7.94 (s, 1H), 7.60 (s, 1H), 6.85 (d, *J* = 8.4 Hz, 1H), 6.44 (t, *J* = 5.6 Hz, 1H), 4.71 (t, *J* = 8.8 Hz, 2H), 4.62 - 4.28 (m, 1H), 3.95 - 3.73 (m, 1H), 3.72 - 3.61 (m, 4H), 3.59 - 3.49 (m, 2H), 3.27 (t, *J* = 8.8 Hz, 2H), 3.16 - 2.82 (m, 2H), 2.59 - 2.45 (m, 5H), 1.90 ( d, *J* = 2.4 Hz, 2H), 1.73 (m, 2H), 1.40 (d, *J* = 10.4 Hz, 2H), 1.02 (t, *J* = 7.2 Hz, 3H); LCMS (Method 1): m/z = 531.3 (M + H)$^+$; | 42.9 | 2.28 |
| 350 | 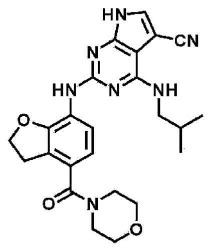 | 4-(isobutylamino)-2-((4-(morpholine-4-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.22 (br s, 1H), 8.04 (d, *J* = 8.4 Hz, 1H), 7.85 (s, 1H), 7.52 (s, 1H), 6.78 (d, *J* = 8.4 Hz, 1H), 6.26 (t, *J* = 5.8 Hz, 1H), 4.62 (t, *J* = 8.8 Hz, 2H), 3.59 (br s, 4H), 3.48 (br d, *J* = 2.4 Hz, 2H), 3.32 (br s, 4H), 3.20 (t, *J* = 8.8 Hz, 2H), 1.97 (td, *J* = 6.8, 13.5 Hz, 1H), 0.93 (d, *J* = 6.8 Hz, 6H); LCMS (Method 1): m/z = 462.2 (M + H)$^+$; | 34.3 | 2.39 |

(continued)

| Example | Structure | Compound name | $^1$H NMR, MS | Yield (%) | HPLC r.t. (min), Purity |
|---|---|---|---|---|---|
| 351 | 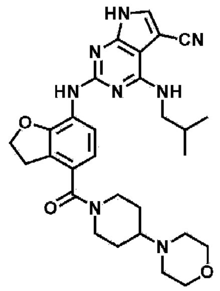 | 4-(isobutylamino)-2-((4-(4-morpholinopiperid ine-1-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.22 (br s, 1H), 7.98 (d, *J*= 8.3 Hz, 1H), 7.84 (s, 1H), 7.53 (s, 1H), 6.74 (d, *J* = 8.3 Hz, 1H), 6.25 (t, *J* = 5.8 Hz, 1H), 4.60 (t, *J* = 8.8 Hz, 2H), 3.61 - 3.49 (m, 4H), 3.31 (br s, 4H), 3.16 (t, *J* = 8.8 Hz, 2H), 3.07 - 2.72 (m, 2H), 2.46 (br d, *J* = 3.9 Hz, 4H), 2.41 - 2.34 (m, 1H), 1.96 (td, *J* = 6.8, 13.5 Hz, 1H), 1.79 (br d, *J* = 3.4 Hz, 2H), 1.37 - 1.20 (m, 2H), 0.92 (d, *J* = 6.8 Hz, 6H); LCMS (Method 1): m/z = 545.3 $(M + H)^+$; | 34.7 | 2.06 |
| 352 | 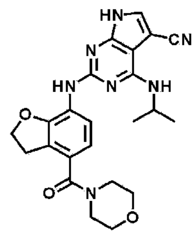 | 4-(isopropylamino)-2-((4-(morpholine-4-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.23 (br d, *J* = 8.3 Hz, 1H), 8.04 (d, *J* = 8.4 Hz, 1H), 7.85 (s, 1H), 7.53 (s, 1H), 6.79 (d, *J* = 8.3 Hz, 1H), 5.75 (d, *J* = 7.6 Hz, 1H), 4.62 (t, *J* = 8.8 Hz, 2H), 4.33 (dd, *J* = 6.6, 13.9 Hz, 1H), 3.69 - 3.41 (m, 8H), 3.20 (t, *J* = 8.8 Hz, 2H), 1.27 (d, *J* = 6.4 Hz, 6H); LCMS (Method 1): m/z = 448.2 $(M + H)^+$; | 20.1 | 2.63 |

(continued)

| Example | Structure | Compound name | $^1$H NMR, MS | Yield (%) | HPLC r.t. (min), Purity |
|---|---|---|---|---|---|
| 353 | 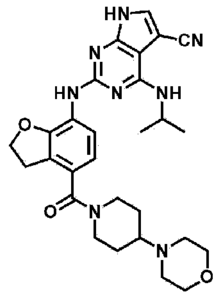 | 4-(isopropylamino)-2-((4-(4-morpholinopiperid ine-1-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.35 - 12.15 (m, 1H), 7.98 (d, *J*=8.3 Hz, 1H), 7.84 (s, 1H), 7.53 (s, 1H), 6.76 (d, *J* = 8.3 Hz, 1H), 5.73 (d, *J* = 7.6 Hz, 1H), 4.60 (t, *J* = 8.8 Hz, 2H), 4.32 (qd, *J* = 6.6, 13.7 Hz, 1H), 3.58 - 3.52 (m, 4H), 3.22 - 3.13 (m, 2H), 3.03 - 2.70 (m, 2H), 2.59 - 2.52 (m, 2H), 2.48 - 2.42 (m, 4H), 2.41 - 2.35 (m, 1H), 1.91 - 1.71 (m, 2H), 1.30 (br s, 2H), 1.26 (d, *J* = 6.4 Hz, 6H); LCMS (Method 1): m/z = 531.3(M + H)$^+$; | 7.1 | 2.31 |
| 354 | 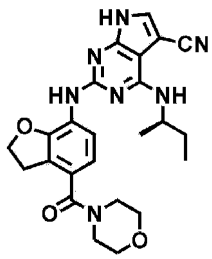 | (*R*)-4-(sec-butylamino)-2-((4-(morpholine-4-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.38 - 12.08 (m, 1H), 8.02 (d, *J* = 8.3 Hz, 1H), 7.85 (s, 1H), 7.54 (s, 1H), 6.79 (d, *J* = 8.3 Hz, 1H), 5.66 (d, *J* = 8.0 Hz, 1H), 4.62 (t, *J* = 8.8 Hz, 2H), 4.23 - 4.13 (m, 1H), 3.68 - 3.44 (m, 8H), 3.20 (t, *J* = 8.7 Hz, 2H), 1.73 - 1.53 (m, 2H), 1.23 (d, *J* = 6.5 Hz, 3H), 0.93 (t, *J* = 7.4 Hz, 3H); LCMS (Method 1): m/z = 462.2 (M + H)$^+$; | 7.6 | 2.40 |

(continued)

| Example | Structure | Compound name | $^1$H NMR, MS | Yield (%) | HPLC r.t. (min), Purity |
|---|---|---|---|---|---|
| 355 | 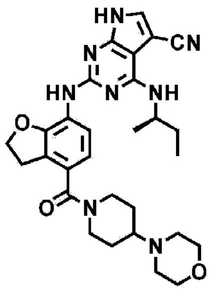 | (*R*)-4-(sec-butylamino)-2-((4-(4-morpholinopiperidine-1-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.23 (br dd, *J* = 4.1, 6.6 Hz, 1H), 7.96 (d, *J* = 8.3 Hz, 1H), 7.84 (s, 1H), 7.54 (s, 1H), 6.75 (d, *J* = 8.3 Hz, 1H), 5.65 (d, *J* = 8.0 Hz, 1H), 4.60 (t, *J* = 8.8 Hz, 2H), 4.22 - 4.11 (m, 1H), 3.58 - 3.53 (m, 4H), 3.16 (t, *J* = 8.7 Hz, 2H), 3.05 - 2.74 (m, 2H), 2.45 (br d, *J* = 4.0 Hz, 4H), 2.41 - 2.34 (m, 1H), 1.89 - 1.71 (m, 2H), 1.70 - 1.61 (m, 1H), 1.60 - 1.50 (m, 1H), 1.38 - 1.27 (m, 2H), 1.22 (d, *J* = 6.5 Hz, 3H), 0.92 (t, *J* = 7.4 Hz, 3H); LCMS (Method 1): m/z = 545.3 (M + H)$^+$; | 19.5 | 2.39 |
| 356 | 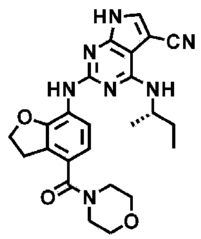 | (*S*)-4-(sec-butylamino)-2-((4-(morpholine-4-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.36 - 12.09 (m, 1H), 8.02 (d, *J* = 8.3 Hz, 1H), 7.85 (s, 1H), 7.54 (s, 1H), 6.79 (d, *J* = 8.3 Hz, 1H), 5.67 (d, *J* = 8.0 Hz, 1H), 4.62 (t, *J* = 8.7 Hz, 2H), 4.23 - 4.13 (m, 1H), 3.68 - 3.40 (m, 7H), 3.20 (t, *J* = 8.8 Hz, 2H), 1.72 - 1.52 (m, 2H), 1.23 (d, *J* = 6.5 Hz, 3H), 0.93 (t, *J* = 7.4 Hz, 3H); LCMS (Method 1): m/z = 462.2 (M + H)$^+$; | 8.9 | 2.40 |

(continued)

| Example | Structure | Compound name | $^1$H NMR, MS | Yield (%) | HPLC r.t. (min), Purity |
|---|---|---|---|---|---|
| 357 | 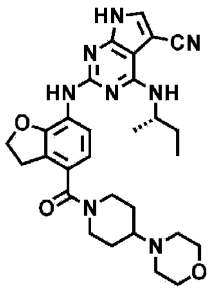 | (S)-4-(sec-butylamino)-2-((4-(4-morpholinopiperidine-1-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.24 (br s, 1H), 7.97 (d, *J*= 8.3 Hz, 1H), 7.84 (s, 1H), 7.54 (s, 1H), 6.75 (d, *J* = 8.3 Hz, 1H), 5.65 (d, *J* = 8.0 Hz, 1H), 4.60 (t, *J* = 8.8 Hz, 2H), 4.24 - 4.08 (m, 1H), 3.59 - 3.52 (m, 4H), 3.16 (t, *J* = 8.7 Hz, 2H), 3.05 - 2.71 (m, 2H), 2.45 (br s, 4H), 2.41 - 2.36 (m, 1H), 1.86 - 1.72 (m, 2H), 1.71 - 1.61 (m, 1H), 1.56 (td, *J* = 6.8, 14.0 Hz, 1H), 1.30 (br d, *J* = 10.4 Hz, 2H), 1.22 (d, *J* = 6.5 Hz, 3H), 0.92 (t, *J* = 7.4 Hz, 3H); LCMS (Method 1): m/z = 545.3 $(M + H)^+$; | 13.7 | 2.39 |
| 358 | 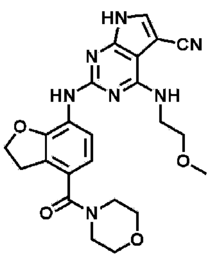 | 4-((2-methoxyethyl)ami no)-2-((4-(morpholine-4-carbonyl)-2,3-dihydrobenzofuran -7-yl) amino)-7*H*-pyrrolo[2,3-*d*] pyrimidine-5-carbonitrile | $^1$H NMR (400 MHz, DMSO-$d_6$): δ = 12.27 (br s, 1H), 8.02 (d, *J*= 8.3 Hz, 1H), 7.87 (s, 1H), 7.59 (s, 1H), 6.78 (d, *J* = 8.3 Hz, 1H), 6.26 (t, *J* = 5.6 Hz, 1H), 4.62 (t, *J* = 8.8 Hz, 2H), 3.68 (q, *J* = 5.6 Hz, 2H), 3.63 - 3.38 (m, 10H), 3.31 (s, 3H), 3.20 (t, *J* = 8.8 Hz, 2H); LCMS (Method 1): m/z = 464.2 $(M + H)^+$; | 10.7 | 2.47 |

(continued)

| Example | Structure | Compound name | $^1H$ NMR, MS | Yield (%) | HPLC r.t. (min), Purity |
|---|---|---|---|---|---|
| 359 | 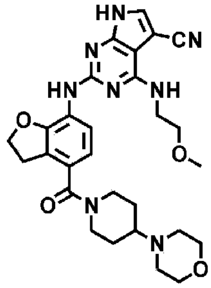 | 4-((2-methoxyethyl)ami no)-2-((4-(4-morpholinopiperid ine-1-carbonyl)-2,3-dihydrobenzofuran -7-yl) amino)-7*H*-pyrrolo[2,3-*d*] pyrimidine-5-carbonitrile | $^1H$ NMR (400 MHz, DMSO-$d_6$): δ = 12.49 - 11.91 (m, 1H), 7.97 (d, *J* = 8.3 Hz, 1H), 7.85 (s, 1H), 7.58 (s, 1H), 6.75 (d, *J* = 8.3 Hz, 1H), 6.24 (t, *J* = 5.6 Hz, 1H), 4.60 (t, *J* = 8.8 Hz, 2H), 4.53 - 4.25 (m, 1H), 3.67 (q, *J* = 5.5 Hz, 2H), 3.58 - 3.52 (m, 5H), 3.30 (s, 3H), 3.16 (t, *J* = 8.7 Hz, 2H), 3.07 - 2.71 (m, 2H), 2.52 (br d, *J* = 1.9 Hz, 2H), 2.46 (br d, *J* = 3.6 Hz, 4H), 2.42 - 2.34 (m, 1H), 1.98 - 1.58 (m, 2H), 1.45 - 1.12 (m, 2H); LCMS (Method 1): m/z = 547.3 (M + H)$^+$; | 35.7 | 2.19 |
| 360 | 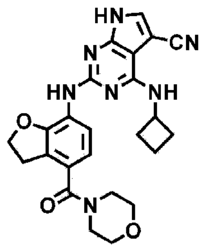 | 4-(cyclobutylamino) -2-((4-(morpholine-4-carbonyl)-2,3-dihydrobenzofuran -7-yl) amino)-7*H*-pyrrolo[2,3-*d*] pyrimidine-5-carbonitrile | $^1H$ NMR (400 MHz, DMSO-$d_6$): δ = 12.24 (s, 1H), 8.04 (d, *J* = 8.4 Hz, 1H), 7.86 (s, 1H), 7.54 (s, 1H), 6.80 (d, *J* = 8.4 Hz, 1H), 6.34 (d, *J* = 7.2 Hz, 1H), 4.72 - 4.44 (m, 3H), 3.90 - 3.37 (m, 8H), 3.21 (t, *J* = 8.8 Hz, 2H), 2.38 - 2.24 (m, 2H), 2.16 - 1.98 (m, 2H), 1.82 - 1.59 (m, 2H); LCMS (Method 1): m/z = 460.2 (M + H)$^+$; | 43.4 | 2.69 |
| 361 | 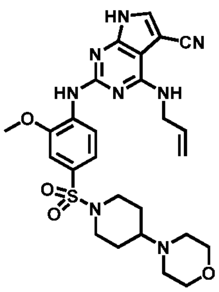 | 4-(allylamino)-2-((2-methoxy-4-((4-morpholinopiperid in-1-yl)sulfonyl)phenyl )amino)-7*H*-pyrrolo[2,3-*d*]pyrimidine-5-carbonitrile | LCMS (Method 7): m/z = 553.39 [M + H]$^+$ | 11 | 1.36 |

(continued)

| Example | Structure | Compound name | $^1$H NMR, MS | Yield (%) | HPLC r.t. (min), Purity |
|---|---|---|---|---|---|
| 362 | 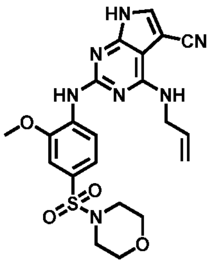 | 4-(allylamino)-2-((2-methoxy-4-(morpholinosulfon yl) phenyl)amino)-7*H*-pyrrolo [2,3-*d*] pyrimidine-5-carbonitrile | LCMS (Method 7): m/z = 470.520 $[M + H]^+$ | 15 | 1.63 |
| 363 | 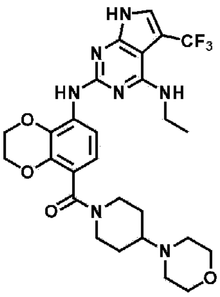 | (8-((4-(ethylamino)-5-(trifluoromethyl)-7*H*-pyrrolo [2,3-*d*]pyrimidin-2-yl)amino)-2,3-dihydrobenzo[*b*][1 ,4] dioxin-5-yl)(4-morpholinopiperid in-1-yl) methanone | $^1$H NMR (400 MHz, DMSO-$d_6$): δ = 12.00 (d, *J* = 2.0Hz, 1H), 8.09 (m, 1H), 7.55(s, 1H), 7.36(d, *J* = 12.0Hz, 1H), 6.73(q, *J* = 8.0Hz, 1H), 5.72(d, *J* = 5.2Hz, 1H), 4.35(q, *J* = 16.8Hz, 5H), 3.56(m, 7H), 2.74(s, 1H), 2.46 (s, 4H), 2.33(s, 1H), 1.83(t, *J* = 12.0Hz, 2H), 1.24(m, 2H), 1.16(s, 3H); LCMS (Method 1): m/z = 576.3 $(M + H)^+$; | 19.5 | 1.32 |
| 364 | 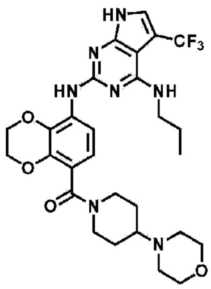 | (4-morpholinopiperid in-1-yl) (8-((4-(propylamino)-5-(trifluoromethyl)-7*H*-pyrrolo [2,3-*d*]pyrimidin-2-yl)amino)-2,3-dihydrobenzo[*b*][1 ,4] dioxin-5-yl)methanone | $^1$H NMR (400 MHz, DMSO-$d_6$): δ = 12.00 (d, *J* = 1.6Hz, 1H), 8.08 (t, *J* = 4.8Hz, 1H), 7.56 (s, 1H), 7.34(s, 1H), 6.72(q, *J* = 8.4Hz, 1H), 5.71(s, 1H), 4.43(m, 5H), 3.51(m, 4H), 3.38 (d, *J* = 5.2Hz, 2H), 2.67 (q, *J* = 2.0Hz, 1H), 2.50 (m, 6H), 2.33(d, *J* = 1.6Hz, 1H), 1.84(d, *J* = 10.0Hz, 1H), 1.65(m, 2H), 1.30(m, 1H), 0.92 (t, *J* = 7.2Hz, 3H); LCMS (Method 1): m/z = 590.2 $(M + H)^+$; | 16.3 | 1.39 |

(continued)

| Example | Structure | Compound name | $^1$H NMR, MS | Yield (%) | HPLC r.t. (min), Purity |
|---|---|---|---|---|---|
| 365 | 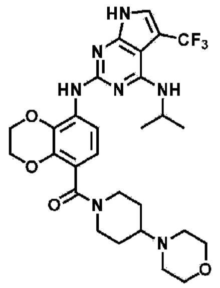 | (8-((4-(isopropylamino)-5-(trifluoromethyl)-7*H*-pyrrolo[2,3-*d*]pyrimidin-2-yl)amino)-2,3-dihydrobenzo[*b*][1,4]dioxin-5-yl)(4-morpholinopiperid in-1-yl) methanone | $^1$H NMR (400 MHz, DMSO-$d_6$): δ = 12.04 (d, *J* = 2.0Hz, 1H), 8.06 (d, *J* = 8.8Hz, 1H), 7.58 (s, 1H), 7.40(d, *J* = 7.2Hz, 1H), 6.74(q, *J* = 8.8Hz, 1H), 5.07(d, *J* = 6.0Hz, 1H), 4.37(m, 6H), 3.43(m, 5H), 2.72 (d, *J* = 13.6Hz, 1H), 2.54(s, 1H), 2.46(s, 4H), 2.39(d, *J* = 2.4Hz, 1H), 1.84(d, *J* = 10.4Hz, 1H), 1.72(s, 1H), 1.28(m, 8H); LCMS (Method 1): m/z = 590.3 (M + H)$^+$; | 11.5 | 1.05 |
| 366 | 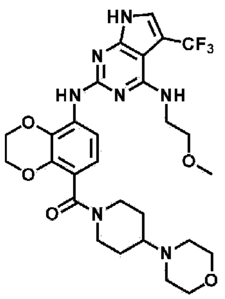 | (8-((4-((2-methoxyethyl)amino)-5-(trifluoromethyl)-7*H*-pyrrolo[2,3-*d*]pyrimidin-2-yl)amino)-2,3-dihydrobenzo[*b*][1 ,4]dioxin-5-yl)4-morpholinopiperid in-1-yl) methanone | $^1$H NMR (400 MHz, DMSO-$d_6$): δ = 12.03 (s, 1H), 8.07(d, *J* = 8.4Hz, 1H), 7.58(s, 1H), 7.42(d, *J* = 10.0Hz, 1H), 7.20(s, 1H), 6.72(m, 1H), 5.77 (s, 1H), 4.45(m, 5H), 3.70(t, *J* = 5.6Hz, 3H), 3.57(t, *J* = 5.2Hz, 8H), 2.34(s, 4H), 2.08(s, 1H), 1.32(m, 2H); LCMS (Method 1): m/z = 606.3 (M + H)$^+$; | 15.4 | 0.99 |
| 367 | 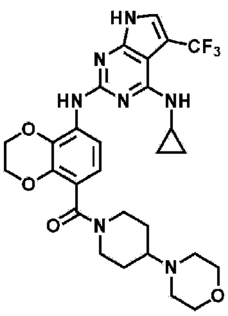 | (8-((4-(cyclopropylamino )-5-(trifluoromethyl)-7*H*-pyrrolo[2,3-*d*]pyrimidin-2-yl)amino)-2,3-dihydrobenzo[*b*][1 ,4] dioxin-5-yl)(4-morpholinopiperid in-1-yl) methanone | $^1$H NMR (400 MHz, DMSO-$d_6$): δ = 12.03 (t, *J* = 6.4Hz, 1H), 8.22 (t, *J* = 8.4Hz, 1H), 7.86 (s, 1H), 7.57(s, 1H), 7.37(s, 1H), 6.71(m, 1H), 5.55(s, 1H), 4.41 (m, 5H), 3.47(d, *J* = 2.0Hz, 5H), 2.90(s, 2H), 2.65(t, *J* = 1.6Hz, 1H), 2.48(s, 4H), 2.47 (d, *J* = 2.0Hz, 1H), 1.82 (d, *J* = 13.2Hz, 1H), 1.69(t, *J* = 3.6Hz, 1H), 1.27(t, *J* = 8.4Hz, 2H), 0.82(q, *J* = 6.4Hz, 2H), 0.56(m, 2H); LCMS (Method 1): m/z = 588.4 (M + H)+; | 14.8 | 1.03 |

(continued)

| Example | Structure | Compound name | $^1H$ NMR, MS | Yield (%) | HPLC r.t. (min), Purity |
|---|---|---|---|---|---|
| 368 | 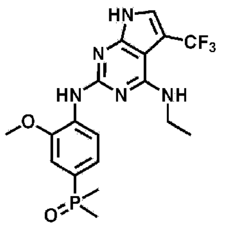 | (4-((4-(ethylamino)-5-(trifluoromethyl)-7*H*-pyrrolo [2,3-*d*]pyrimidin-2-yl)amino)-3-methoxyphenyl)di methylphosphine oxide | $^1H$ NMR (400 MHz, CDC13-d) δ 9.50 (s, 1H), 8.73 (dd, *J* = 8.2, 3.5 Hz, 1H), 7.61 (s, 1H), 7.35 (dd, *J* = 12.3, 1.5 Hz, 1H), 7.20 - 7.13 (m, 1H), 7.12 (d, *J* = 1.3 Hz, 1H), 5.31 (s, 1H), 3.99 (s, 3H), 3.71 - 3.63 (m, 2H), 1.76 (s, 3H), 1.73 (s, 3H), 1.34 (t, *J* = 7.2 Hz, 3H); LCMS (Method 7): m/z = 428.25 $[M + H]^+$ | 40 | 1.42 |
| 369 | 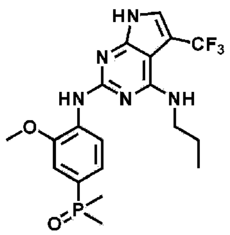 | (3-methoxy-4-((4-(propylamino)-5-(trifluoromethyl)-7*H*-pyrrolo [2,3-*d*]pyrimidin-2-yl)amino) phenyl) dimethylphosphine oxide | $^1H$ NMR (400 MHz, DMSO-*d*$_6$): δ = 12.04 (s, 1H), 8.68(q, *J* = 2.8Hz, 1H), 7.59(d, *J* = 10.8Hz, 1H), 7.33(m, 1H), 5.76(s, 1H), 3.97 (s, 3H), 3.54(t, *J* = 6.0Hz, 2H), 1.64(t, *J* = 6.4Hz, 8H), 0.96(q, *J* = 7.2Hz, 3H); LCMS (Method 1): Ret.T = 2.793min, m/z = 442.1 $(M + H)^+$; | 26.2 | 2.79 |
| 370 | 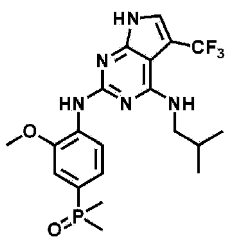 | (4-((4-(isobutylamino)-5-(trifluoromethyl)-7*H*-pyrrolo [2,3-*d*]pyrimidin-2-yl)amino)-3-methoxyphenyl)di methylphosphine oxide | $^1H$ NMR (400 MHz, DMSO-*d*$_6$): δ = 12.05 (s, 1H), 8.68(q, *J* = 3.2Hz, 1H), 7.85(s, 1H), 7.60(t, 0.8Hz, 1H), 7.32(m, 2H), 5.69 (s, 1H), 3.97(s, 3H), 3.42(t, *J* = 6.4Hz, 2H), 2.00(t, *J* = 6.8Hz, 1H), 1.64(d, *J* = 1.2Hz, 6H), 0.96(t, *J* = 6.4Hz, 6H); LCMS (Method 1): m/z = 456.1 $(M + H)^+$; | 32.4 | 2.91 |

(continued)

| Example | Structure | Compound name | $^1$H NMR, MS | Yield (%) | HPLC r.t. (min), Purity |
|---|---|---|---|---|---|
| 371 | 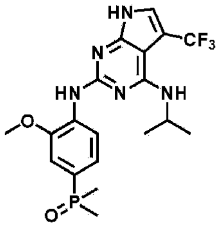 | (4-((4-(isopropylamino)-5-(trifluoromethyl)-7*H*-pyrrolo [2,3-*d*]pyrimidin-2-yl)amino)-3-methoxyphenyl)di methylphosphine oxide | $^1$H NMR (400 MHz, DMSO-$d_6$): δ = 12.06 (d, *J* = 2.0Hz, 1H), 8.67 (d, *J* = 2.8Hz, 1H), 7.59 (d, *J* = 11.6Hz, 2H), 7.32(m, 2H), 5.11(t, *J* = 0.8Hz, 1H), 4.05(t, *J* = 6.4Hz, 1H), 3.95(s, 3H), 1.60(s, 3H), 1.58 (s, 3H), 1.30(s, 3H), 1.23(s, 3H); LCMS (Method 1): m/z = 442.1 (M + H)$^+$; | 13.6 | 2.86 |
| 372 | 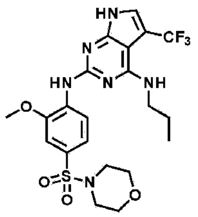 | $N^2$-(2-methoxy-4-(morpholinosulfon yl) phenyl)-$N^4$-propyl-5-(trifluoromethyl)-7*H*-pyrrolo [2,3-*d*]pyrimidine-2,4-diamine | $^1$H NMR (400 MHz, DMSO-$d_6$): δ = 12.07 (d, *J* = 1.6Hz, 1H), 8.83 (d, *J* = 8.8Hz, 1H), 7.73 (s, 1H), 7.63(d, *J* = 1.2Hz, 1H), 7.33(t, *J* = 2.0Hz, 1H), 7.21(d, *J* = 1.6Hz, 1H), 5.80(s, 1H), 4.01(s, 3H), 3.64 (t, *J* = 4.0Hz, 4H), 3.55 (m, 2H), 2.89(t, *J* = 4.4Hz, 4H), 1.64(m, 2H), 0.94(q, *J* = 7.2Hz, 3H); LCMS (Method 1): m/z = 515 (M + H)$^+$; | 20.4 | 0.88 |
| 373 | 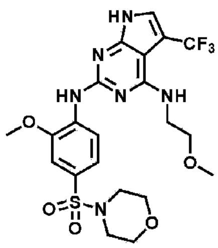 | $N^2$-(2-methoxy-4-(morpholinosulfon yl) phenyl)-$N^4$-(2-methoxyethyl)-5-(trifluoromethyl)-7*H*-pyrrolo [2,3-*d*]pyrimidine-2,4-diamine | $^1$H NMR (400 MHz, DMSO-$d_6$): δ = 8.82(d, *J* = 8.8Hz, 1H), 7.76(s, 1H), 7.65(s, 1H), 7.34 (q, *J* = 1.6Hz, 1H), 7.21 (d, *J* = 1.6Hz, 1H), 5.86 (s, 1H), 4.00(s, 4H), 3.73(t, *J* = 5.6Hz, 2H), 3.63(t, *J* = 4.4Hz, 4H), 3.58(t, *J* = 5.2Hz, 2H), 3.32(s, 3H), 2.88(t, *J* = 4.0Hz, 4H); LCMS (Method 1): m/z = 531 (M + H)$^+$; | 20.2 | 0.84 |

(continued)

| Example | Structure | Compound name | $^1H$ NMR, MS | Yield (%) | HPLC r.t. (min), Purity |
|---|---|---|---|---|---|
| 374 | 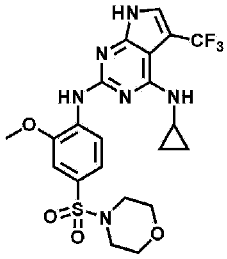 | *N*4-cyclopropyl-*N*2-(2-methoxy-4-(morpholinosulfonyl)phenyl)-5-(trifluoromethyl)-7*H*-pyrrolo[2,3-*d*]pyrimidine-2,4-diamine | $^1H$ NMR (400 MHz, DMSO-$d_6$): δ = 9.05(d, *J* = 8.8Hz, 1H), 7.78(s, 1H), 7.67(d, *J* = 1.2Hz, 1H), 7.36(q, *J* = 2.0Hz, 1H), 7.22(d, *J* = 2.0Hz, 1H), 5.70(s, 1H), 4.02 (s, 3H), 3.64(t, *J* = 4.0Hz, 4H), 2.99(m, 1H), 2.89(t, *J* = 4.4Hz, 4H), 0.90(m, 2H), 0.63 (m, 2H); LCMS (Method 1): m/z = 513 $(M + H)^+$; | 20.1 | 0.84 |
| 375 | 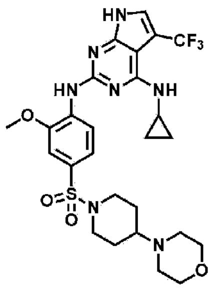 | *N*4-cyclopropyl-N2-(2-methoxy-4-((4-morpholinopiperid in-1-yl) sulfonyl)phenyl )-5-(trifluoromethyl)-7*H*-pyrrolo [2,3-*d*]pyrimidine-2,4-diamine | $^1H$ NMR (400 MHz, DMSO-$d_6$): δ = 12.11 (m, 1H), 9.01(d, *J* = 8.4Hz, 1H), 7.75(s, 1H), 7.66(d, *J* = 1.2Hz, 1H), 7.35(q, *J* = 1.6Hz, 1H), 7.21(d, *J* = 1.6Hz, 1H), 5.69(s, 1H), 4.00 (s, 3H), 3.65(d, *J* = 12.0Hz, 2H), 3.52(t, *J* = 4.0Hz, 4H), 2.98(t, *J* = 3.6Hz, 1H), 2.39 (3.6Hz, 4H), 2.33(s, 2H), 2.12(s, 1H), 1.85 (d, *J* = 11.2Hz, 2H), 1.43(q, *J* = 9.6Hz, 2H), 0.62(m, 2H); LCMS (Method 1): m/z = 596.3 $(M + H)^+$; | 20.5 | 0.75 |
| 376 | 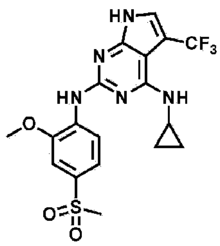 | *N*4-cyclopropyl-*N*2-(2-methoxy-4-(methylsulfonyl)phenyl)-5-(trifluoromethyl)-7*H*-pyrrolo[2,3-*d*]pyrimidine-2,4-diamine | $^1H$ NMR (400 MHz, DMSO-$d_6$): δ = 12.11 (m, 1H), 9.02(d, *J* = 8.4Hz, 1H), 7.80(d, *J* = 12.4Hz, 1H), 7.66(s, 1H), 7.53(d, *J* = 8.8Hz, 1H), 7.47(d, *J* = 12.8Hz, 1H), 5.69(s, 1H), 4.07(t, *J* = 2.0Hz, 3H), 3.25(s, 3H), 2.98 (t, *J* = 3.2Hz, 1H), 0.88 (m, 2H), 0.67(d, *J* = 5.6Hz, 2H); LCMS (Method 1): m/z = 442.2 $(M + H)^+$; | 20.3 | 0.82 |

(continued)

| Example | Structure | Compound name | $^1$H NMR, MS | Yield (%) | HPLC r.t. (min), Purity |
|---|---|---|---|---|---|
| 377 | 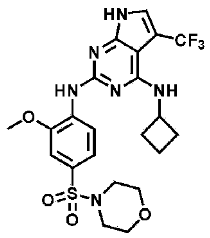 | *N*$^4$-cyclobutyl-*N*$_2$-(2-methoxy-4-(morpholinosulfon yl) phenyl)-5-(trifluoromethyl)-7*H*-pyrrolo[2,3-*d*]pyrimidine-2,4-diamine | $^1$H NMR (400 MHz, DMSO-$d_6$): δ = 12.12 (s, 1H), 8.81(d, *J* = 8.4Hz, 1H), 7.75(s, 1H), 7.66(d, *J* = 1.6Hz, 1H), 7.35(q, *J* = 4.0Hz, 1H), 7.20(d, *J* = 2.0Hz, 1H), 5.55(d, *J* = 6.8Hz, 1H), 4.67(q, *J* = 7.6Hz, 1H), 4.00(s, 3H), 3.63 (t, *J* = 4.4Hz, 4H), 2.89 (t, *J* = 4.4Hz, 4H), 2.39 (d, *J* = 7.2Hz, 2H), 1.99 (d, *J* = 2.4Hz, 2H), 1.77 (t, *J* = 8.0Hz, 2H); LCMS (Method 1): m/z = 527.2 (M + H)$^+$; | 5.4 | 0.94 |
| 378 | 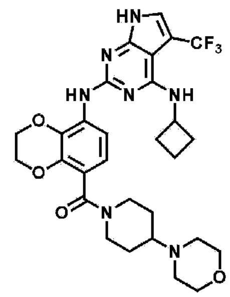 | (8-((4-(cyclobutylamino) -5-(trifluoromethyl)-7*H*-pyrrolo[2,3-*d*]pyrimidin-2-yl)amino)-2,3-dihydrobenzo [*b*][1 ,4]dioxin-5-yl)(4-morpholinopiperid in-1-yl) methanone | $^1$H NMR (400 MHz, DMSO-$d_6$): δ = 12.00 (q, *J* = 3.6Hz, 1H), 8.07 (d, *J* = 8.4Hz, 1H), 7.59 (s, 1H),7.38(d, *J* = 7.6Hz, 1H), 6.76(m, 1H), 5.46(d, *J* = 5.6Hz, 1H), 4.65(d, *J* = 8.4Hz, 1H), 4.45(m, 1H), 4.34 (q, *J* = 11.6Hz, 4H), 3.51(d, *J* = 5.2Hz, 4H), 3.41(s, 1H), 3.02(m, 1H), 2.67(d, *J* = 1.6Hz, 1H), 2.50(s, 4H), 2.39 (t, *J* = 2.0Hz, 2H), 1.97 (d, *J* = 10.4Hz, 2H), 1.76(t, *J* = 5.6Hz, 1H), 1.73(q, *J* = 4.8Hz, 3H), 1.30(m, 2H); LCMS (Method 1): m/z = 602.3 (M + H)$^+$; | 18.8 | 0.74 |

(continued)

| Example | Structure | Compound name | $^1H$ NMR, MS | Yield (%) | HPLC r.t. (min), Purity |
|---|---|---|---|---|---|
| 379 | 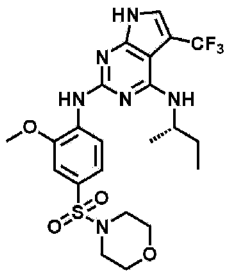 | (*S*)-*N*4-(sec-butyl)-*N*2-(2-methoxy-4-(morpholinosulfonyl)phenyl)-5-(trifluoromethyl)-7*H*-pyrrolo[2,3-*d*]pyrimidine-2,4-diamine | $^1H$ NMR (400 MHz, DMSO-$d_6$): δ = 12.13 (br s, 1H), 8.81 (d, *J* = 8.6 Hz, 1H), 7.75 (s, 1H), 7.66 (d, *J* = 1.3 Hz, 1H), 7.34 (dd, *J* = 1.8, 8.6 Hz, 1H), 7.21 (d, *J* = 1.8 Hz, 1H), 5.13 (br d, *J* = 6.5 Hz, 1H), 4.27 (td, *J* = 6.7, 13.6 Hz, 1H), 4.00 (s, 3H), 3.69 - 3.58 (m, 4H), 2.92 - 2.79 (m, 4H), 1.63 (quin, *J* = 7.1 Hz, 2H), 1.25 (d, *J* = 6.5 Hz, 3H), 0.93 (t, *J* = 7.4 Hz, 3H); LCMS (Method 1): m/z = 529.2 $(M + H)^+$; | 11.5 | 2.92 |
| 380 | 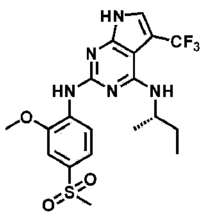 | (*S*)-*N*4-(sec-butyl)-*N*2-(2-methoxy-4-(methylsulfonyl)phenyl)-5-(trifluoromethyl)-7*H*-pyrrolo[2,3-*d*]pyrimidine-2,4-diamine | $^1H$ NMR (400 MHz, DMSO-$d_6$): δ = 12.15 (br s, 1H), 8.80 (d, *J* = 8.6 Hz, 1H), 7.74 (s, 1H), 7.67 (s, 1H), 7.51 (dd, *J* = 2.0, 8.5 Hz, 1H), 7.46 (d, *J* = 2.0 Hz, 1H), 5.20 - 5.11 (m, 1H), 4.28 (td, *J* = 6.7, 13.6 Hz, 1H), 4.02 (s, 3H), 3.20 (s, 3H), 1.64 (quin, *J* = 7.2 Hz, 2H), 1.26 (d, *J* = 6.5 Hz, 3H), 0.94 (t, *J* = 7.4 Hz, 3H); LCMS (Method 1):, m/z = 458.1 $(M + H)^+$; | 7.4 | 2.80 |

(continued)

| Example | Structure | Compound name | $^1H$ NMR, MS | Yield (%) | HPLC r.t. (min), Purity |
|---|---|---|---|---|---|
| 381 | 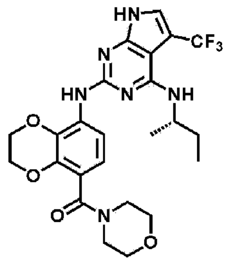 | (S)-(8-((4-(sec-butylamino)-5-(trifluoromethyl)-7*H*-pyrrolo [2,3-*d*]pyrimidin-2-yl)amino)-2,3-dihydrobenzo[*b*][1 ,4] dioxin-5-yl)(morpholino)me thanone | $^1H$ NMR (400 MHz, DMSO-$d_6$): δ = 12.05 (br s, 1H), 8.11 (d, *J* = 8.5 Hz, 1H), 7.60 (s, 1H), 7.39 (s, 1H), 6.77 (d, *J* = 8.4 Hz, 1H), 5.06 (br dd, *J* = 1.4,7.7 Hz, 1H), 4.39 (br d, *J* = 2.4 Hz, 2H), 4.33 (br d, *J* = 3.5 Hz, 2H), 4.27 - 4.20 (m, 1H), 3.61 (br s, 4H), 3.54 (br s, 2H), 3.30 - 3.20 (m, 2H), 1.65 - 1.58 (m, 2H), 1.23 (d, *J* = 6.5 Hz, 3H), 0.92 (t, *J* = 7.4 Hz, 3H); LCMS (Method 1): m/z = 521.2 $(M + H)^+$; | 29.5 | 2.55 |
| 382 | 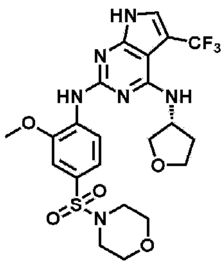 | (*R*)-$N^2$-(2-methoxy-4-(morpholinosulfon yl) phenyl)-$N^4$-(tetrahydrofuran-3-yl)-5-(trifluoromethyl)-7*H*-pyrrolo[2,3-*d*]pyrimidine-2,4-diamine | $^1H$ NMR (400 MHz, DMSO-$d_6$): δ = 12.19 (br s, 1H), 8.80 (d, *J* = 8.5 Hz, 1H), 7.82 (s, 1H), 7.69 (s, 1H), 7.36 (dd, *J* = 1.7, 8.6 Hz, 1H), 7.21 (d, *J* = 1.9 Hz, 1H), 5.48 - 5.39 (m, 1H), 4.82 - 4.72 (m, 1H), 4.00 (s, 3H), 3.95 (dd, *J* = 5.6, 9.2 Hz, 1H), 3.90 - 3.83 (m, 1H), 3.81 - 3.74 (m, 1H), 3.66 - 3.62 (m, 4H), 2.93 - 2.85 (m, 4H), 2.11 - 2.05 (m, 2H), 1.94 - 1.84 (m, 1H); LCMS (Method 1): m/z = 543.3 (M + $H)^+$; | 11.4 | 2.72 |

(continued)

| Example | Structure | Compound name | $^1H$ NMR, MS | Yield (%) | HPLC r.t. (min), Purity |
|---|---|---|---|---|---|
| 383 | 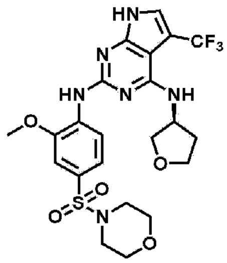 | (*S*)-$N^2$-(2-methoxy-4-(morpholinosulfon yl) phenyl)-$N^4$-(tetrahydrofuran-3-yl)-5-(trifluoromethyl)-7*H*-pyrrolo[2,3-*d*]pyrimidine-2,4-diamine | $^1H$ NMR (400 MHz, DMSO-$d_6$): $\delta$ = 12.53 - 11.81 (m, 1H), 9.01 - 8.56 (m, 1H), 7.86 - 7.77 (m, 1H), 7.73 - 7.65 (m, 1H), 7.39 - 7.33 (m, 1H), 7.24 - 7.17 (m, 1H), 5.52 - 5.35 (m, 1H), 4.87 - 4.67 (m, 1H), 4.00 (s, 3H), 3.95 (dd, *J* = 5.5, 9.1 Hz, 1H), 3.90 - 3.83 (m, 1H), 3.81 - 3.74 (m, 1H), 3.66 - 3.62 (m, 4H), 2.97 - 2.83 (m, 4H), 2.09 - 2.07 (m, 2H), 1.97 - 1.84 (m, 1H); LCMS (Method 1): m/z = 543.2 $(M + H)^+$; | 11.4 | 2.72 |
| 384 | 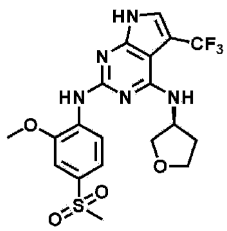 | (*S*)-$N^2$-(2-methoxy-4-(methylsulfonyl)p henyl)-$N^4$-(tetrahydrofuran-3-yl)-5-(trifluoromethyl)-7*H*-pyrrolo[2,3-*d*]pyrimidine-2,4-diamine | $^1H$ NMR (400 MHz, DMSO-$d_6$): $\delta$ = 12.20 (br d, *J* = 5.1 Hz, 1H), 8.78 (d, *J* = 8.6 Hz, 1H), 7.80 (s, 1H), 7.69 (d, *J* = 1.5 Hz, 1H), 7.53 (dd, *J* = 1.9, 8.6 Hz, 1H), 7.46 (d, *J* = 2.0 Hz, 1H), 5.43 (br dd, *J* = 1.6, 6.5 Hz, 1H), 4.83 - 4.72 (m, 1H), 4.01 (s, 3H), 3.97 - 3.92 (m, 1H), 3.91 - 3.83 (m, 1H), 3.81 - 3.74 (m, 1H), 3.67 (dd, *J* = 3.3, 9.1 Hz, 1H), 3.20 (s, 3H), 2.41 - 2.30 (m, 1H), 1.94 - 1.84 (m, 1H); LCMS (Method 1): m/z = 472.1 $(M + H)^+$; | 11.4 | 2.90 |

(continued)

| Example | Structure | Compound name | $^1$H NMR, MS | Yield (%) | HPLC r.t. (min), Purity |
|---|---|---|---|---|---|
| 385 | 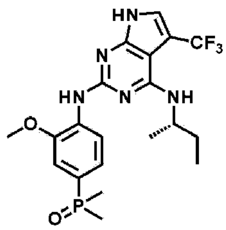 | (S)-(4-((4-(sec-butylamino)-5-(trifluoromethyl)-7*H*-pyrrolo[2,3-*d*]pyrimidin-2-yl)amino)-3-methoxyphenyl)di methylphosphine oxide | $^1$H NMR (400 MHz, DMSO-$d_6$): $\delta$ = 12.08 (br s, 1H), 8.68 (dd, *J* = 3.1, 8.5 Hz, 1H), 7.72 - 7.50 (m, 2H), 7.39 - 7.26 (m, 2H), 5.76 (s, 1H), 5.17 - 5.00 (m, 1H), 4.28 (td, *J* = 6.7, 13.7 Hz, 1H), 3.97 (s, 3H), 1.65 (d, *J* = 13.3 Hz, 7H), 1.26 (d, *J* = 6.5 Hz, 3H), 0.94 (t, *J* = 7.4 Hz, 3H); LCMS (Method 1): m/z = 456.2 (M + H)$^+$; | 27.7 | 2.98 |
| 386 | 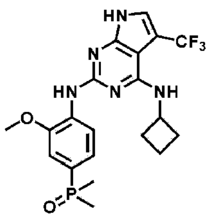 | (4-((4-(cyclobutylamino) -5-(trifluoromethyl)-7*H*-pyrrolo[2,3-*d*]pyrimidin-2-yl)amino)-3-methoxyphenyl) di methylphosphine oxide | $^1$H NMR (400 MHz, DMSO-$d_6$): $\delta$ = 12.09 (d, *J* = 2.3 Hz, 1H), 8.68 (dd, *J* = 3.1, 8.1 Hz, 1H), 7.68 - 7.55 (m, 2H), 7.41 - 7.24 (m, 2H), 5.50 (br dd, *J* = 1.4, 7.1 Hz, 1H), 4.78 - 4.61 (m, 1H), 3.96 (s, 3H), 2.45 - 2.36 (m, 2H), 2.06 - 1.94 (m, 2H), 1.83 - 1.71 (m, 2H), 1.64 (d, *J* = 13.4 Hz, 6H); LCMS (Method 1): m/z = 454.1 (M + H)$^+$; | 26.4 | 2.96 |
| 387 | 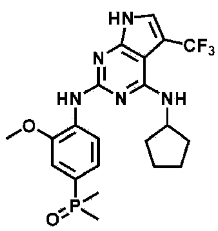 | (4-((4-(cyclopentylamino )-5-(trifluoromethyl)-7*H*-pyrrolo[2,3-*d*]pyrimidin-2-yl)amino)-3-methoxyphenyl)di methylphosphine oxide | $^1$H NMR (400 MHz, DMSO-$d_6$): $\delta$ = 12.08 (br d, *J* = 1.6 Hz, 1H), 8.71 (dd, *J* = 3.1, 8.6 Hz, 1H), 7.69 - 7.57 (m, 2H), 7.40 - 7.28 (m, 2H), 5.24 (br dd, *J* = 1.6, 6.9 Hz, 1H), 4.57 - 4.47 (m, 1H), 3.97 (s, 3H), 2.15 - 2.03 (m, 2H), 1.76 - 1.61 (m, 11H), 1.57 - 1.47 (m, 2H); LCMS (Method 1): m/z = 468.2 (M + H)$^+$; | 30.7 | 3.02 |

(continued)

| Example | Structure | Compound name | $^1$H NMR, MS | Yield (%) | HPLC r.t. (min), Purity |
|---|---|---|---|---|---|
| 388 | 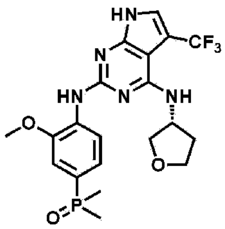 | (*R*)-(3-methoxy-4-((4-((tetrahydrofuran-3-yl) amino)-5-(trifluoromethyl)-7*H*-pyrrolo[2,3-*d*]pyrimidin-2-yl)amino)phenyl)d imethylphosphine oxide | $^1$H NMR (400 MHz, DMSO-$d_6$) δ = 12.12 (br s, 1H), 8.65 (dd, *J* = 3.0, 8.0 Hz, 1H), 7.65 (s, 2H), 7.43 - 7.18 (m, 2H), 5.46 - 5.33 (m, 1H), 4.83 - 4.67 (m, 1H), 3.96 (s, 3H), 3.95 - 3.91 (m, 1H), 3.90 - 3.83 (m, 1H), 3.77 (dt, *J* = 5.6, 8.4 Hz, 1H), 3.66 (dd, *J* = 3.3, 9.2 Hz, 1H), 2.37 - 2.31 (m, 1H), 1.95 - 1.83 (m, 1H), 1.65 (s, 3H), 1.62 (s, 3H); LCMS (Method 1): m/z = 470.1 (M + H)$^+$; | 22.7 | 2.78 |
| 389 | 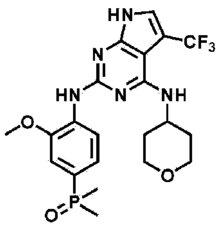 | (3-methoxy-4-((4-((tetrahydro-2*H*-pyran-4-yl)amino)-5-(trifluoromethyl)-7*H*-pyrrolo [2,3-*d*]pyrimidin-2-yl)amino) phenyl)d imethylphosphine oxide | $^1$H NMR (400 MHz, DMSO-$d_6$) δ = 12.10 (br s, 1H), 8.64 (dd, *J* = 3.1, 8.7 Hz, 1H), 7.68 - 7.61 (m, 2H), 7.37 - 7.27 (m, 2H), 5.29 - 5.20 (m, 1H), 4.39 - 4.26 (m, 1H), 3.96 (s, 3H), 3.89 (td, *J* = 3.3, 11.4 Hz, 2H), 3.53 (dt, *J* = 1.9, 11.2 Hz, 2H), 2.06 - 1.98 (m, 2H), 1.67 (s, 3H), 1.64 (s, 3H), 1.62 - 1.50 (m, 2H); LCMS (Method 1): m/z = 484.2 (M + H)$^+$; | 30.4 | 2.80 |

(continued)

| Example | Structure | Compound name | $^1H$ NMR, MS | Yield (%) | HPLC r.t. (min), Purity |
|---|---|---|---|---|---|
| 390 | 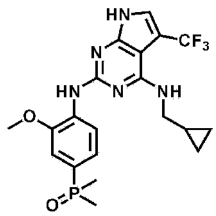 | (4-((4-((cyclopropylmeth yl) amino)-5-(trifluoromethyl)-7*H*-pyrrolo[2,3-*d*]pyrimidin-2-yl)amino)-3-methoxyphenyl) di methylphosphine oxide | $^1H$ NMR (400 MHz, DMSO-$d_6$) $\delta$ = 12.07 (br d, *J* = 1.0 Hz, 1H), 8.70 (dd, *J* = 3.1, 8.6 Hz, 1H), 7.62 (s, 1H), 7.59 (s, 1H), 7.37 - 7.29 (m, 2H), 5.77 - 5.71 (m, 1H), 3.97 (s, 3H), 3.45 (dd, *J* = 5.7, 6.7 Hz, 2H), 1.67 (s, 3H), 1.63 (s, 3H), 1.21 (ddd, *J* = 3.0, 4.7, 7.9 Hz, 1H), 0.53 - 0.45 (m, 2H), 0.35 - 0.27 (m, 2H); LCMS (Method 1): m/z = 454.2 (M + H)$^+$; | 27.4 | 2.89 |
| 391 | 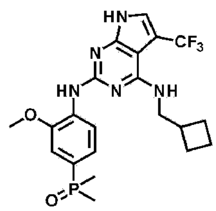 | (4-((4-((cyclobutylmethyl ) amino)-5-(trifluoromethyl)-7*H*-pyrrolo[2,3-*d*]pyrimidin-2-yl)amino)-3-methoxyphenyl) di methylphosphine oxide | $^1H$ NMR (400 MHz, DMSO-$d_6$) $\delta$ = 12.05 (br s, 1H), 8.68 (dd, *J* = 3.1, 8.6 Hz, 1H), 7.69 - 7.55 (m, 2H), 7.38 - 7.27 (m, 2H), 5.62 (br s, 1H), 3.97 (s, 3H), 3.61 (dd, *J* = 5.8, 6.8 Hz, 2H), 2.67 (quin, *J* = 7.5 Hz, 1H), 2.07 - 1.98 (m, 2H), 1.93 - 1.83 (m, 2H), 1.82 - 1.71 (m, 2H), 1.66 (s, 3H), 1.63 (s, 3H); LCMS (Method 1): m/z = 468.2 (M + H)$^+$; | 27.2 | 3.01 |
| 392 | 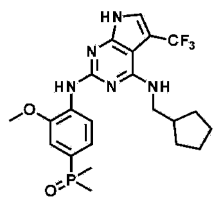 | (4-((4-((cyclopentylmeth yl) amino)-5-(trifluoromethyl)-7*H*-pyrrolo[2,3-*d*]pyrimidin-2-yl)amino)-3-methoxyphenyl) di methylphosphine oxide | $^1H$ NMR (400 MHz, DMSO-$d_6$) $\delta$ = 12.03 (br s, 1H), 8.67 (dd, *J* = 3.1, 8.6 Hz, 1H), 7.59 (s, 1H), 7.57 (s, 1H), 7.33 - 7.26 (m, 2H), 5.66 (br s, 1H), 3.95 (s, 3H), 3.50 (dd, *J* = 5.7, 7.1 Hz, 2H), 2.31 - 2.22 (m, 1H), 1.80 - 1.67 (m, 2H), 1.65 (s, 3H), 1.64 - 1.57 (m, 5H), 1.56 - 1.45 (m, 2H), 1.36 - 1.19 (m, 2H); LCMS (Method 1): m/z = 482.2 (M + H)$^+$; | 23.1 | 2.76 |

(continued)

| Example | Structure | Compound name | $^1$H NMR, MS | Yield (%) | HPLC r.t. (min), Purity |
|---|---|---|---|---|---|
| 393 | 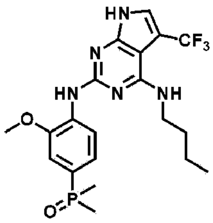 | (4-((4-(butylamino)-5-(trifluoromethyl)-7*H*-pyrrolo [2,3-*d*]pyrimidin-2-yl)amino)-3-methoxyphenyl)di methylphosphine oxide | $^1$H NMR (400 MHz, DMSO-$d_6$) δ = 12.02 (br d, *J* = 0.9 Hz, 1H), 8.67 (dd, *J* = 3.1, 8.6 Hz, 1H), 7.66 - 7.50 (m, 2H), 7.39 - 7.25 (m, 2H), 5.71 (br s, 1H), 3.96 (s, 3H), 3.61 - 3.51 (m, 2H), 1.65 (s, 3H), 1.64 - 1.57 (m, 5H), 1.44 - 1.32 (m, 2H), 0.93 (t, *J* = 7.4 Hz, 3H); LCMS (Method 1): m/z = 456.2 (M + H)$^+$; | 24.5 | 2.94 |
| 394 | 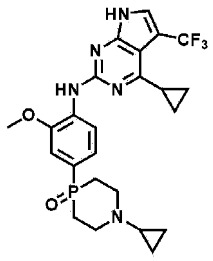 | 1 -cyclopropyl-4-(4-((4-cyclopropyl-5-(trifluoromethyl)-7*H*-pyrrolo [2,3-*d*]pyrimidin-2-yl)amino)-3-methoxy phenyl)-1,4-azaphosphinane4-oxide | $^1$H NMR (400 MHz, DMSO-$d_6$) δ 8.58 (s, 1H), 7.87 (d, *J* = 16.2 Hz, 2H), 7.43 - 7.28 (m, 2H), 3.97 (s, 3H), 2.98 (d, *J* = 15.5 Hz, 4H), 2.35 (s, 1H), 2.21 (s, 2H), 1.93 - 1.73 (m, 4H), 1.14 (s, 3H), 0.48 (s, 2H), 0.36 (s, 2H). LCMS (Method 7): m/z = 506.3 [M + H]$^+$ | 20 | 1.41 |
| 395 | 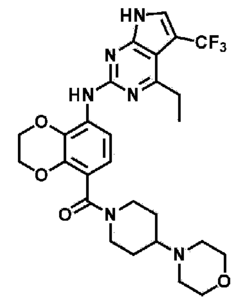 | (8-((4-ethyl-5-(trifluoromethyl)-7*H*-pyrrolo [2,3-*d*] pyrimidin-2-yl)amino)-2,3-dihydrobenzo [*b*][1,4] dioxin-5-yl)(4-morpholinopiperid in-1-yl) methanone | $^1$H NMR (400 MHz, DMSO-$d_6$) δ 8.01 - 7.94 (m, 1H), 7.85 (s, 2H), 7.54 - 7.21 (m, 1H), 6.76 (d, *J* = 14.7 Hz, 1H), 4.46 (s, 1H), 4.33 (d, *J* = 24.7 Hz, 4H), 3.53 (d, *J* = 24.8 Hz, 6H), 2.93 (d, *J* = 4.0 Hz, 3H), 2.74 (t, *J* = 12.2 Hz, 1H), 2.45 (s, 4H), 1.86 (s, 3H), 1.72 (s, 1H), 1.32 - 1.28 (m, 3H). LCMS (Method 7): m/z = 594.4 [M + H]$^+$ | 24 | 1.33 |

(continued)

| Example | Structure | Compound name | $^1$H NMR, MS | Yield (%) | HPLC r.t. (min), Purity |
|---|---|---|---|---|---|
| 396 | 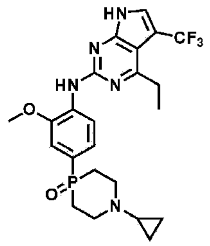 | 1 -cyclopropyl-4-(4-((4-ethyl-5-(trifluoromethyl)-7*H*-pyrrolo [2,3-*d*] pyrimidin-2-yl)amino)-3-methoxyphenyl)-1,4-azaphosphinane-4-oxide | $^1$H NMR (400 MHz, DMSO-$d_6$) δ 8.68 (d, *J* = 3.3 Hz, 1H), 8.00 (s, 1H), 7.90 (s, 1H), 7.43 - 7.30 (m, 2H), 3.98 (s, 3H), 2.98 (d, *J* = 14.9 Hz, 5H), 2.21 (s, 2H), 1.92 - 1.76 (m, 4H), 1.32 (s, 3H), 0.48 (s, 2H), 0.36 (s, 2H). LCMS (Method 7): m/z = 494.3 [M + H]$^+$ | 10 | 1.38 |
| 397 | 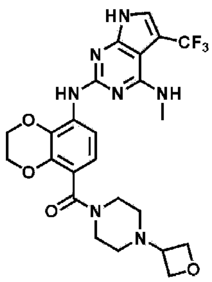 | (8-((4-(methylamino)-5-(trifluoromethyl)-7*H*-pyrrolo [2,3-*d*]pyrimidin-2-yl)amino)-2,3-dihydrobenzo[*b*][1 ,4] dioxin-5-yl)(4-(oxetan-3-yl) piperazin-1-yl)methanone | $^1$H NMR (400 MHz, DMSO-$d_6$) δ = 11.99 (br s, 1H), 8.15 (d, *J* = 8.5 Hz, 1H), 7.54 (s, 1H), 7.35 (s, 1H), 6.75 (d, *J* = 8.5 Hz, 1H), 5.91 (br d, *J* = 3.4 Hz, 1H), 4.59 - 4.50 (m, 2H), 4.44 (t, *J* = 6.1 Hz, 2H), 4.38 (br d, *J* = 2.6 Hz, 2H), 4.31 (br s, 2H), 3.63 (br s, 2H), 3.48 - 3.39 (m, 1H), 3.03 (d, *J* = 4.6 Hz, 3H), 2.46 - 2.44 (m, 2H), 2.29 (br s, 2H), 2.25 - 2.13 (m, 2H); LCMS (Method 1): m/z = 534.2 (M + H)$^+$; | 28.9 | 2.24 |
| 398 | 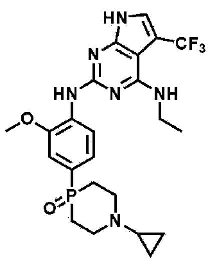 | 1 -cyclopropyl-4-(4-((4-(ethylamino)-5-(trifluoromethyl)-7*H*-pyrrolo [2,3-*d*]pyrimidin-2-yl)amino)-3-methoxyphenyl)-1,4-azaphosphinane 4-oxide | $^1$H NMR (400 MHz, DMSO-$d_6$): δ = 12.27 - 11.76 (m, 1H), 8.98 - 8.44 (m, 1H), 7.65 - 7.52 (m, 2H), 7.40 - 7.26 (m, 2H), 5.82 - 5.72 (m, 1H), 4.03 - 3.91 (m, 3H), 3.65 - 3.53 (m, 2H), 3.07 - 2.89 (m, 4H), 2.26 - 2.14 (m, 2H), 1.91 - 1.75 (m, 3H), 1.22 (t, *J* = 7.1 Hz, 3H), 0.52 - 0.44 (m, 2H), 0.40 - 0.31 (m, 2H); LCMS (Method 1): m/z = 509.2(M + H)$^+$; | 21.9 | 2.13 |

(continued)

| Example | Structure | Compound name | $^1$H NMR, MS | Yield (%) | HPLC r.t. (min), Purity |
|---|---|---|---|---|---|
| 399 | 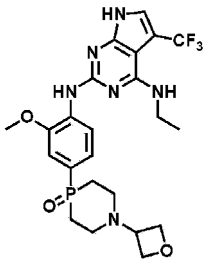 | 4-(4-((4-(ethylamino)-5-(trifluoromethyl)-7*H*-pyrrolo[2,3-*d*]pyrimidin-2-yl)amino)-3-methoxyphenyl)-1-(oxetan-3-yl)-1,4-azaphosphinane 4-oxide | $^1$H NMR (400 MHz, DMSO-$d_6$): δ = 12.06 (br d, *J* = 5.8 Hz, 1H), 8.74 (dd, *J* = 3.1, 8.3 Hz, 1H), 7.66 - 7.53 (m, 2H), 7.43 - 7.23 (m, 2H), 5.89 - 5.68 (m, 1H), 4.62 - 4.52 (m, 2H), 4.49 - 4.40 (m, 2H), 3.99 (s, 3H), 3.66 - 3.53 (m, 3H), 2.78 - 2.57 (m, 4H), 2.31 - 2.19 (m, 2H), 1.97 - 1.79 (m, 2H), 1.23 (t, *J* = 7.1 Hz, 3H); LCMS (Method 1): m/z = 525.2 (M + H)$^+$; | 9.1 | 2.44 |
| 400 | 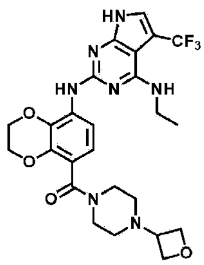 | (8-((4-(ethylamino)-5-(trifluoromethyl)-7*H*-pyrrolo[2,3-*d*]pyrimidin-2-yl)amino)-2,3-dihydrobenzo[*b*][1 ,4]dioxin-5-yl)(4-(oxetan-3-yl)piperazin-1-yl)methanone | $^1$H NMR (400 MHz, DMSO-$d_6$): δ = 12.18 - 11.84 (m, 1H), 8.13 (d, *J* = 8.4 Hz, 1H), 7.56 (d, *J* = 1.4 Hz, 1H), 7.35 (s, 1H), 6.75 (d, *J* = 8.5 Hz, 1H), 5.80 - 5.63 (m, 1H), 4.58 - 4.51 (m, 2H), 4.44 (t, *J* = 6.0 Hz, 2H), 4.38 (br d, *J* = 2.3 Hz, 2H), 4.34 - 4.27 (m, 2H), 3.68 - 3.53 (m, 4H), 3.47 - 3.39 (m, 1H), 3.27 - 3.21 (m, 2H), 2.32 - 2.14 (m, 4H), 1.26 - 1.16 (m, 3H); LCMS (Method 1): m/z = 548.2 (M + H)$^+$; | 24.7 | 2.06 |

(continued)

| Example | Structure | Compound name | $^1$H NMR, MS | Yield (%) | HPLC r.t. (min), Purity |
|---|---|---|---|---|---|
| 401 | 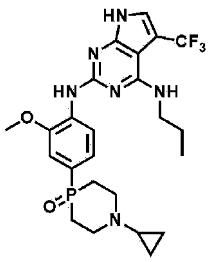 | 1-cyclopropyl-4-(3-methoxy-4-((4-(propylamino)-5-(trifluoromethyl)-7*H*-pyrrolo[2,3-*d*]pyrimidin-2-yl)amino)phenyl)-1,4-azaphosphinane 4-oxide | $^1$H NMR (400 MHz, DMSO-$d_6$): δ = 12.00 (br s, 1H), 8.12 (d, *J* = 8.5 Hz, 1H), 7.56 (d, *J* = 1.3 Hz, 1H), 7.35 (s, 1H), 6.74 (d, *J* = 8.5 Hz, 1H), 5.71 (br s, 1H), 4.57 - 4.51 (m, 2H), 4.44 (t, *J* = 6.0 Hz, 2H), 4.38 (br d, *J* = 2.5 Hz, 2H), 4.31 (br s, 2H), 3.62 (br s, 2H), 3.55 - 3.48 (m, 2H), 3.43 (td, *J* = 6.3, 12.5 Hz, 1H), 3.26 - 3.20 (m, 1H), 2.33 - 2.12 (m, 4H), 1.64 (sxt, *J* = 7.3 Hz, 2H), 0.93 (t, *J* = 7.4 Hz, 3H); LCMS (Method 1): m/z = 523.2 (M + H)$^+$; | 16.0 | 2.23 |
| 402 | 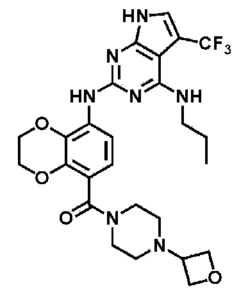 | (4-(oxetan-3-yl)piperazin-1 -yl)(8-((4-(propylamino)-5-(trifluoromethyl)-7*H*-pyrrolo[2,3-*d*]pyrimidin-2-yl)amino)-2,3-dihydrobenzo[*b*][1 ,4]dioxin-5-yl)methanone | $^1$H NMR (400 MHz, DMSO-$d_6$): δ = 12.17 - 11.92 (m, 1H), 8.71 (dd, *J* = 3.1, 8.3 Hz, 1H), 7.60 (s, 2H), 7.44 - 7.23 (m, 2H), 5.75 (br t, *J* = 4.6 Hz, 1H), 3.98 (s, 3H), 3.59 - 3.49 (m, 2H), 3.07 - 2.90 (m, 4H), 2.27 - 2.14 (m, 2H), 1.91 - 1.76 (m, 3H), 1.66 (sxt, *J* = 7.3 Hz, 2H), 0.95 (t, *J* = 7.4 Hz, 3H), 0.53 - 0.44 (m, 2H), 0.39 - 0.32 (m, 2H); LCMS (Method 1): m/z = 562.2 (M + H)$^+$; | 17.6 | 2.16 |

(continued)

| Example | Structure | Compound name | $^1H$ NMR, MS | Yield (%) | HPLC r.t. (min), Purity |
|---|---|---|---|---|---|
| 403 | 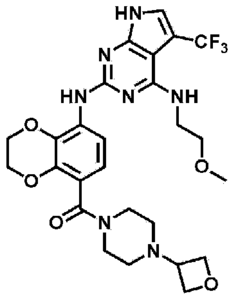 | (8-((4-((2-methoxyethyl)amino)-5-(trifluoromethyl)-7*H*-pyrrolo[2,3-*d*]pyrimidin-2-yl)amino)-2,3-dihydrobenzo[*b*][1 ,4]dioxin-5-yl)(4-(oxetan-3-yl)piperazin-1-yl) methanone | $^1H$ NMR (400 MHz, DMSO-$d_6$): δ = 12.16 - 11.85 (m, 1H), 8.14 - 8.03 (m, 1H), 7.63 - 7.52 (m, 1H), 7.45 - 7.34 (m, 1H), 6.83 - 6.63 (m, 1H), 5.84 - 5.69 (m, 1H), 4.58 - 4.48 (m, 2H), 4.46 - 4.40 (m, 2H), 4.39 - 4.28 (m, 4H), 3.73 - 3.67 (m, 2H), 3.66 - 3.59 (m, 2H), 3.58 - 3.53 (m, 2H), 3.46 - 3.39 (m, 1H), 3.31 (s, 3H), 3.29 (br s, 2H), 2.31 - 2.13 (m, 4H); LCMS (Method 1): m/z = 578.2 (M + H)$^+$; | 26.0 | 2.38 |
| 404 | 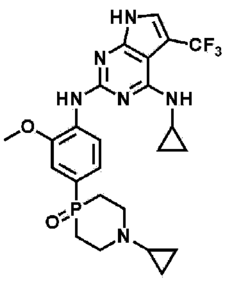 | 1 -cyclopropyl-4-(4-((4-(cyclopropylamino ) 5-(trifluoromethyl)-7*H*-pyrrolo[2,3-*d*]pyrimidin-2-yl)amino)3-methoxyphenyl)-1,4-azaphosphinane 4-oxide | $^1H$ NMR (400 MHz, DMSO-$d_6$): δ = 12.09 (br s, 1H), 8.92 (dd, *J* = 3.1, 8.3 Hz, 1H), 7.70 - 7.54 (m, 2H), 7.47 - 7.21 (m, 2H), 5.65 (br s, 1H), 3.99 (s, 3H), 3.07 - 2.89 (m, 5H), 2.26 - 2.13 (m, 2H), 1.92 - 1.73 (m, 3H), 0.97 - 0.82 (m, 2H), 0.68 - 0.58 (m, 2H), 0.53 - 0.44 (m, 2H), 0.40 - 0.31 (m, 2H); LCMS (Method 1): m/z = 521.2 (M + H)$^+$; | 16.9 | 2.12 |

(continued)

| Example | Structure | Compound name | $^1$H NMR, MS | Yield (%) | HPLC r.t. (min), Purity |
|---|---|---|---|---|---|
| 405 | 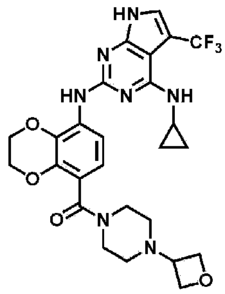 | (8-((4-(cyclopropylamino )-5-(trifluoromethyl)-7*H*-pyrrolo [2,3-*d*]pyrimidin-2-yl)amino)-2,3-dihydrobenzo[*b*][1 ,4] dioxin-5-yl)(4-(oxetan-3-yl) piperazin-1-yl)methanone | $^1$H NMR (400 MHz, DMSO-$d_6$): δ = 12.05 (br s, 1H), 8.29 (d, *J* = 8.4 Hz, 1H), 7.60 (s, 1H), 7.39 (s, 1H), 6.76 (d, *J* = 8.4 Hz, 1H), 5.58 (br s, 1H), 4.57 - 4.52 (m, 2H), 4.44 (t, *J* = 6.1 Hz, 2H), 4.39 (br d, *J* = 2.5 Hz, 2H), 4.32 (br d, *J* = 3.0 Hz, 2H), 3.63 (br s, 2H), 3.48 - 3.41 (m, 1H), 3.30 - 3.22 (m, 2H), 2.95 (qt, *J* = 3.5, 6.8 Hz, 1H), 2.32 - 2.10 (m, 4H), 0.91 - 0.76 (m, 2H), 0.63 - 0.55 (m, 2H); LCMS (Method 1): m/z = 560.2 (M + H)$^+$; | 21.9 | 2.05 |
| 406 | 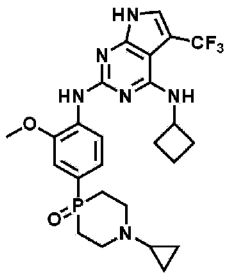 | 4-(4-((4-((cyclobutylamino )-5-(trifluoromethyl)-7*H*-pyrrolo [2,3-*d*]pyrimidin-2-yl)amino)-3-methoxyphenyl)-1-cyclopropyl-1,4-azaphosphinane 4-oxide | $^1$H NMR (400 MHz, DMSO-$d_6$): δ = 12.25 - 11.85 (m, 1H), 8.84 - 8.55 (m, 1H), 7.66 - 7.59 (m, 2H), 7.40 - 7.34 (m, 1H), 7.30 (dd, *J* = 1.3, 11.9 Hz, 1H), 5.54 - 5.47 (m, 1H), 4.74 - 4.62 (m, 1H), 4.03 - 3.92 (m, 3H), 3.29 (br s, 2H), 3.07 - 2.88 (m, 4H), 2.25 - 2.15 (m, 2H), 2.05 - 1.94 (m, 2H), 1.89 - 1.85 (m, 1H), 1.84 - 1.81 (m, 1H), 1.80 - 1.74 (m, 2H), 0.56 - 0.42 (m, 2H), 0.41 - 0.30 (m, 2H); LCMS (Method 1): m/z = 535.2, 268.1 (M + H)$^+$; | 13.4 | 2.33 |

(continued)

| Example | Structure | Compound name | $^1$H NMR, MS | Yield (%) | HPLC r.t. (min), Purity |
|---|---|---|---|---|---|
| 407 | 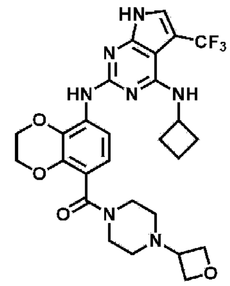 | (8-((4-(cyclobutylamino) -5-(trifluoromethyl)- 7*H*-pyrrolo[2,3-*d*]pyrimidin-2-yl)amino)-2,3-dihydrobenzo [*b*][1 ,4]dioxin-5-yl)(4-(oxetan-3-yl)piperazin-1-yl) methanone | $^1$H NMR (400 MHz, DMSO-$d_6$): δ = 12.05 (s, 1H), 8.10 (d, *J* = 8.4 Hz, 1H), 7.60 (d, J = 1.2 Hz, 1H), 7.39 (s, 1H), 6.76 (d, J = 8.4 Hz, 1H), 5.47 (m, 1H), 4.72 - 4.60 (m, 1H), 4.58 - 4.52 (m, 2H), 4.44 (t, J = 6.0 Hz, 2H), 4.39 (s, 2H), 4.31 (s, 2H), 3.63 (d, J = 2.8 Hz, 2H), 3.44 (t, J = 6.4 Hz, 1H), 3.30 (s, 4H), 2.44 - 2.36 (m, 2H), 2.32 - 2.17 (m, 4H), 2.06 - 1.89 (m, 2H), 1.76 (m, 2H); LCMS (Method 1): m/z = 574.2 $(M + H)^+$; | 21.5 | 2.26 |
| 408 | 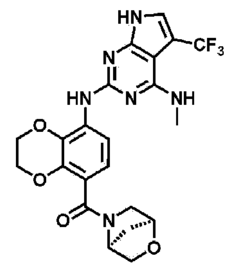 | ((1*S*,4*S*)-2-oxa-5-azabicyclo [2.2.1]h eptan-5-yl) (8-((4-(methylamino)- 5-(trifluoromethyl)-7*H*-pyrrolo [2,3-*d*]pyrimidin-2-yl)amino)- 2,3-dihydrobenzo[*b*][1 ,4] dioxin-5-yl)methanone | $^1$H NMR (400 MHz, MeOD) δ 8.37 (dd, *J* = 10.3, 8.7 Hz, 1H), 7.34 (s, 1H), 6.90 (d, *J* = 8.6 Hz, 1H), 4.73 (s, 1H), 4.44 (d, *J* = 9.4 Hz, 2H), 4.37 (dd, *J* = 9.4, 6.2 Hz, 3H), 3.95 (d, *J* = 7.5 Hz, 1H), 3.79 (d, *J* = 7.5 Hz, 1H), 3.62 - 3.55 (m, 1H), 3.48 (d, *J* = 12.1 Hz, 1H), 3.15 (d, *J* = 2.0 Hz, 3H). LCMS (Method 7): m/z = 491.2 $[M + H]^+$ | 37 | 1.37 |
| 409 | 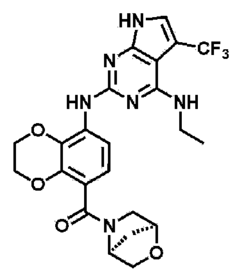 | ((1*S*,4*S*)-2-oxa-5-azabicyclo [2.2.1]h eptan-5-yl) (8-((4-(ethylamino)- 5-(trifluoromethyl)-7*H*-pyrrolo [2,3-*d*]pyrimidin-2-yl)amino)- 2,3-dihydrobenzo[*b*][1 ,4] dioxin-5-yl)methanone | $^1$H NMR (400 MHz, MeOD) δ 8.37 - 8.30 (m, 1H), 7.35 (d, *J* = 1.3 Hz, 1H), 6.90 (d, *J* = 8.6 Hz, 1H), 4.72 (s, 1H), 4.47 - 4.31 (m, 6H), 3.94 (d, *J* = 7.5 Hz, 1H), 3.78 (d, *J* = 7.4 Hz, 1H), 3.69 - 3.63 (m, 2H), 3.57 (d, *J* = 12.8 Hz, 1H), 3.50 - 3.42 (m, 1H), 1.34 - 1.29 (m, 3H). LCMS (Method 7): m/z = 505.3 $[M + H]^+$ | 55 | 1.46 |

(continued)

| Example | Structure | Compound name | $^1H$ NMR, MS | Yield (%) | HPLC r.t. (min), Purity |
|---|---|---|---|---|---|
| 410 | 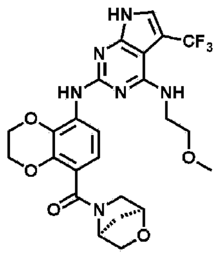 | ((1*S*,4*S*)-2-oxa-5-azabicyclo [2.2.1] heptan-5-yl)(8-((4-((2-methoxyethyl)ami no)-5-(trifluoromethyl)-7*H*-pyrrolo [2,3-*d*]pyrimidin-2-yl)amino)-2,3-dihydrobenzo[*b*][1 ,4] dioxin-5-yl)methanone | $^1H$ NMR (400 MHz, MeOD) δ 8.26 (dd, *J* = 8.7, 2.2 Hz, 1H), 7.37 (s, 1H), 6.90 (dd, *J* = 8.6, 4.7 Hz, 1H), 4.46 - 4.32 (m, 6H), 3.94 (d, *J* = 7.3 Hz,1H), 3.79 (d, *J* = 5.8 Hz, 3H), 3.67 (q, *J* = 5.3 Hz, 3H), 3.62 (s, 2H), 3.44 (d, *J* = 3.2 Hz, 4H). LCMS (Method 7): m/z = 535.3 [M + H]$^+$ | 60 | 1.45 |
| 411 | 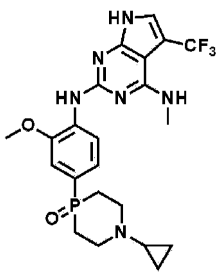 | 1-cyclopropyl-4-(3-methoxy-4-((4-(methylamino)-5-(trifluoromethyl)-7*H*-pyrrolo [2,3-*d*]pyrimidin-2-yl)amino) phenyl)-1,4-azaphosphinane 4-oxide | $^1H$ NMR (400 MHz, CDCl3) δ 9.51 (s, 1H), 8.76 (dd, *J* = 8.3, 3.6 Hz, 1H), 7.64 (s, 1H), 7.33 (d, *J* = 12.2 Hz, 1H), 7.15 (s, 1H), 3.97 (s, 3H), 3.27 - 3.20 (m, 1H), 3.18 (d, *J* = 4.7 Hz, 3H), 3.16 - 3.08 (m, 2H), 2.19 - 1.98 (m, 6H), 0.58 - 0.49 (m, 2H), 0.49 - 0.41 (m, 2H); LCMS (Method 7): m/z = 495.28 [M + H]$^+$ | 17.8 | 1.20 |
| 412 | 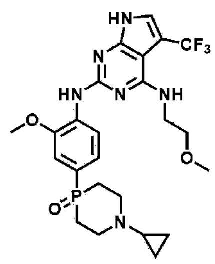 | 1-cyclopropyl-4-(3-methoxy-4-((4-((2-methoxyethyl)ami no)-5-(trifluoromethyl)-7*H*-pyrrolo[2,3-*d*]pyrimidin-2-yl)amino)phenyl)-1,4-azaphosphinane 4-oxide | $^1H$ NMR (400 MHz, DMSO-$d_6$): δ = 12.09 (br s, 1H), 8.69 (dd, *J* = 3.1, 8.3 Hz, 1H), 7.73 - 7.50 (m, 2H), 7.41 - 7.25 (m, 2H), 5.81 (br s, 1H), 3.97 (s, 3H), 3.73 (q, *J* = 5.4 Hz, 2H), 3.63 - 3.52 (m, 2H), 3.31 - 3.30 (m, 3H), 3.06 - 2.90 (m, 4H), 2.25 - 2.14 (m, 2H), 1.90 - 1.76 (m, 3H), 0.52 - 0.42 (m, 2H), 0.40 - 0.32 (m, 2H); LCMS (Method 1): m/z = 539.2 (M + H)$^+$; | 16.4 | 2.13 |

(continued)

| Example | Structure | Compound name | $^1$H NMR, MS | Yield (%) | HPLC r.t. (min), Purity |
|---|---|---|---|---|---|
| 413 | 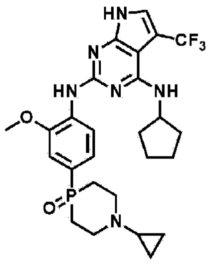 | 4-(4-((4-((cyclopentylamin o)-5-(trifluoromethyl)-7*H*-pyrrolo [2,3-*d*]pyrimidin-2-yl)amino)-3-methoxyphenyl)-1-cyclopropyl-1,4-azaphosphinane 4-oxide | $^1$H NMR (400 MHz, DMSO-$d_6$): $\delta$ = 12.09 (br s, 1H), 8.73 (dd, *J* = 3.1, 8.3 Hz, 1H), 7.63 (s, 2H), 7.40 - 7.25 (m, 2H), 5.25 (br d, *J* = 5.6 Hz, 1H), 4.59 - 4.45 (m, 1H), 4.03 - 3.93 (m, 3H), 3.07 - 2.89 (m, 4H), 2.27 - 2.16 (m, 2H), 2.12 - 2.03 (m, 2H), 1.88 - 1.80 (m, 2H), 1.73 - 1.64 (m, 4H), 1.54 (td, *J* = 5.6, 11.1 Hz, 3H), 0.53 - 0.47 (m, 2H), 0.41 - 0.32 (m, 2H); LCMS (Method 1): m/z = 549.2 (M + H)$^+$; | 32.5 | 2.38 |
| 414 | 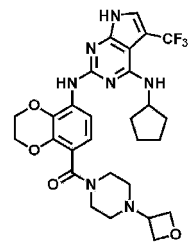 | (8-((4-(cyclopentylamino )-5-(trifluoromethyl)-7*H*-pyrrolo [2,3-*d*]pyrimidin-2-yl)amino)-2,3-dihydrobenzo[*b*][1 ,4] dioxin-5-yl)(4-(oxetan-3-yl) piperazin-1-yl)methanone | $^1$H NMR (400 MHz, DMSO-$d_6$): $\delta$ = 12.28 - 11.72 (m, 1H), 8.35 - 7.92 (m, 1H), 7.63 - 7.54 (m, 1H), 7.42 - 7.36 (m, 1H), 6.79 - 6.68 (m, 1H), 5.27 - 5.14 (m, 1H), 4.57 - 4.51 (m, 2H), 4.51 - 4.46 (m, 1H), 4.43 (t, *J* = 6.1 Hz, 2H), 4.40 - 4.28 (m, 4H), 3.70 - 3.54 (m, 2H), 3.46 - 3.40 (m, 1H), 3.29 (br s, 2H), 2.31 - 2.26 (m, 2H), 2.25 - 2.14 (m, 2H), 2.10 - 2.00 (m, 2H), 1.74 - 1.59 (m, 4H), 1.56 - 1.45 (m, 2H); LCMS (Method 1): m/z = 588.2 (M + H)$^+$; | 33.0 | 2.32 |

(continued)

| Example | Structure | Compound name | $^1$H NMR, MS | Yield (%) | HPLC r.t. (min), Purity |
|---|---|---|---|---|---|
| 415 | 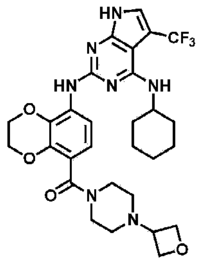 | (8-((4-(cyclohexylamino) -5-(trifluoromethyl)-7*H*-pyrrolo[2,3-*d*]pyrimidin-2-yl)amino)-2,3-dihydrobenzo [*b*][1 ,4]dioxin-5-yl)(4-(oxetan-3-yl)piperazin-1-yl) methanone | $^1$H NMR (400 MHz, DMSO-$d_6$): δ = 12.43 - 11.52 (m, 1H), 8.09 (d, *J* = 8.5 Hz, 1H), 7.59 (d, *J* = 1.5 Hz, 1H), 7.37 (s, 1H), 6.73 (d, *J* = 8.5 Hz, 1H), 5.16 (br d, *J* = 6.8 Hz, 1H), 4.53 (t, *J* = 6.5 Hz, 2H), 4.43 (t, *J* = 6.0 Hz, 2H), 4.38 (br s, 2H), 4.30 (br s, 2H), 4.14 - 4.04 (m, 1H), 3.62 (br d, *J* = 2.8 Hz, 2H), 3.43 (quin, *J* = 6.2 Hz, 1H), 3.26 (br s, 2H), 2.28 (br s, 2H), 2.25 - 2.13 (m, 2H), 2.03 - 1.95 (m, 2H), 1.69 (br dd, *J* = 4.0, 9.0 Hz, 2H), 1.59 (br dd, *J* = 4.1, 8.8 Hz, 1H), 1.46 - 1.25 (m, 5H); LCMS (Method 1): m/z = 602.3 (M + H)$^+$; | 36.0 | 1.90 |
| 416 | 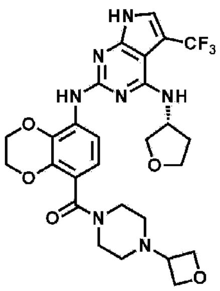 | (*R*)-(4-(oxetan-3-yl)piperazin-1-yl)(8-((4-((tetrahydrofuran-3-yl)amino)-5-(trifluoromethyl)-7*H*-pyrrolo [2,3-*d*]pyrimidin-2-yl)amino)-2,3-dihydrobenzo[*b*][1,4] dioxin-5-yl)methanone | $^1$H NMR (400 MHz, DMSO-$d_6$) δ = 12.09 (br s, 1H), 8.06 (d, *J* = 8.5 Hz, 1H), 7.62 (d, *J* = 1.4 Hz, 1H), 7.46 (s, 1H), 6.75 (d, *J* = 8.5 Hz, 1H), 5.36 (br d, *J* = 5.3 Hz, 1H), 4.82 - 4.65 (m, 1H), 4.54 (t, *J* = 6.5 Hz, 2H), 4.44 (t, *J* = 6.1 Hz, 2H), 4.38 (br s, 2H), 4.31 (br s, 2H), 3.92 (dd, *J* = 5.6, 9.2 Hz, 1H), 3.86 (q, *J* = 7.4 Hz, 1H), 3.76 (dt, *J* = 5.7, 8.3 Hz, 1H), 3.64 (br dd, *J* = 3.3, 9.1 Hz, 3H), 3.47 - 3.39 (m, 1H), 3.27 (br d, *J* = 3.9 Hz, 1H), 2.36 - 2.13 (m, 6H), 1.94 - 1.81 (m, 1H); LCMS (Method 1): m/z = 590.2(M + H)$^+$; | 28.4 | 2.42 |

(continued)

| Example | Structure | Compound name | $^1$H NMR, MS | Yield (%) | HPLC r.t. (min), Purity |
|---|---|---|---|---|---|
| 417 | 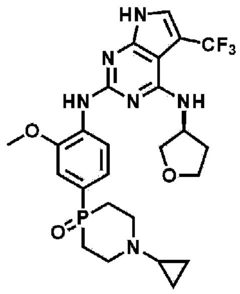 | (*S*)-1-cyclopropyl-4-(3-methoxy-4-((4-((tetrahydrofuran-3-yl) amino)-5-(trifluoromethyl)-7*H*-pyrrolo[2,3-*d*]pyrimidin-2-yl)amino)phenyl)-1,4-azaphosphinane 4-oxide | $^1$H NMR (400 MHz, DMSO-$d_6$): δ = 12.24 - 12.07 (m, 1H), 8.68 (dd, *J* = 3.0, 8.3 Hz, 1H), 7.75 - 7.61 (m, 2H), 7.45 - 7.19 (m, 2H), 5.50 - 5.30 (m, 1H), 4.82 - 4.70 (m, 1H), 4.01 - 3.93 (m, 4H), 3.91 - 3.84 (m, 1H), 3.78 (dt, *J* = 5.5, 8.4 Hz, 1H), 3.67 (dd, *J* = 3.3, 9.0 Hz, 1H), 3.05 - 2.91 (m, 4H), 2.42 - 2.33 (m, 1H), 2.27 - 2.14 (m, 2H), 1.93 - 1.78 (m, 4H), 0.56 - 0.45 (m, 2H), 0.41 - 0.32 (m, 2H); LCMS (Method 1): m/z = 551.2 (M + H)$^+$; | 24.6 | 2.16 |
| 418 | 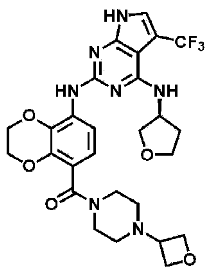 | (S)-(4-(oxetan-3-yl)piperazin-1-yl)(8-((4-((tetrahydrofuran-3-yl)amino)-5-(trifluoromethyl)-7*H*-pyrrolo [2,3-*d*]pyrimidin-2-yl)amino)-2,3-dihydrobenzo[*b*][1 ,4] dioxin-5-yl)methanone | $^1$H NMR (400 MHz, DMSO-$d_6$): δ = 12.07 (s, 1H), 8.03(d, *J* = 8.4Hz, 1H), 7.59(d, *J* = 1.2Hz, 1H), 7.44(s, 1H), 6.73(d, *J* = 8.8Hz, 1H), 5.34(q, *J* = 1.2Hz, 1H), 4.71(s, 1H), 4.52 (t, *J* = 6.4Hz, 2H), 4.42 (t, *J* = 6.0Hz, 2H), 4.36 (s, 2H), 4.28(s, 2H), 3.90(d, *J* = 3.6Hz, 1H), 3.83(s, 1H), 3.74(d, *J* = 6.0Hz, 1H), 3.60(t, *J* = 3.6Hz, 3H), 3.41(s, 1H), 3.28(d, *J* = 9.6Hz, 1H), 2.28(q, *J* = 12.8Hz, 6H), 1.84(q, *J* = 3.6Hz, 1H); LCMS (Method 1): m/z = 590.2 (M + H)$^+$; | 34.9 | 2.42 |

(continued)

| Example | Structure | Compound name | $^1$H NMR, MS | Yield (%) | HPLC r.t. (min), Purity |
|---|---|---|---|---|---|
| 419 | 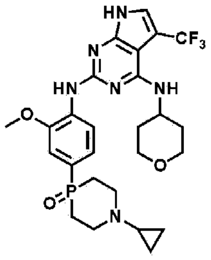 | 1-cyclopropyl-4-(3-methoxy-4-((4-((tetrahydro-2*H*-pyran-4-yl)amino)-5-(trifluoro)-7*H*-pyrrolo[2,3-*d*]pyrimidin-2-yl) amino)phenyl)-1,4-azaphosphinane 4-oxide | $^1$H NMR (400 MHz, DMSO-$d_6$): δ = 12.03 (br s, 1H), 8.59 (dd, *J* = 3.1, 8.3 Hz, 1H), 7.68 - 7.50 (m, 2H), 7.36 - 7.17 (m, 2H), 5.18 (br dd, *J* = 1.2, 6.9 Hz, 1H), 4.33 - 4.21 (m, 1H), 3.90 (s, 3H), 3.85 - 3.78 (m, 2H), 3.47 (br t, *J* = 10.3 Hz, 2H), 3.01 - 2.82 (m, 4H), 2.19 - 2.08 (m, 2H), 1.95 (br d, *J* = 10.3 Hz, 2H), 1.84 - 1.71 (m, 3H), 1.55 - 1.43 (m, 2H), 0.46 - 0.39 (m, 2H), 0.33 - 0.26 (m, 2H); LCMS (Method 1): m/z = 565.2 (M + H)$^+$; | 25.5 | 2.51 |
| 420 | 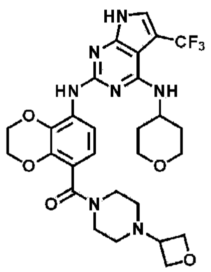 | (4-(oxetan-3-yl)piperazin-1-yl)(8-((4-((tetrahydro-2*H*-pyran-4-yl)amino)-5-(trifluoromethyl)-7*H*-pyrrolo [2,3-*d*]pyrimidin-2-yl)amino)-2,3-dihydrobenzo[*b*][1 ,4] dioxin-5-yl)methanone | $^1$H NMR (400 MHz, DMSO-$d_6$) δ = 12.05 (br s, 1H), 8.06 (d, *J* = 8.5 Hz, 1H), 7.60 (d, *J* = 1.4 Hz, 1H), 7.41 (s, 1H), 6.74 (d, *J* = 8.5 Hz, 1H), 5.19 (br dd, *J* = 1.0, 7.3 Hz, 1H), 4.53 (t, *J* = 6.5 Hz, 2H), 4.43 (t, *J* = 6.0 Hz, 2H), 4.37 (br s, 2H), 4.33 - 4.23 (m, 3H), 3.91 - 3.81 (m, 2H), 3.67 - 3.57 (m, 2H), 3.54 - 3.46 (m, 2H), 3.45 - 3.39 (m, 1H), 3.28 - 3.18 (m, 2H), 2.29 (br s, 2H), 2.25 - 2.15 (m, 2H), 2.00 (br d, *J* = 10.1 Hz, 2H), 1.62 - 1.48 (m, 2H); LCMS (Method 1): m/z = 604.2 (M + H)$^+$; | 29.3 | 2.45 |

(continued)

| Example | Structure | Compound name | $^1$H NMR, MS | Yield (%) | HPLC r.t. (min), Purity |
|---|---|---|---|---|---|
| 421 | 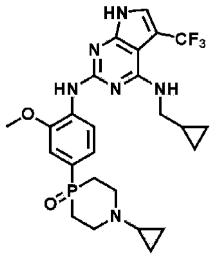 | 1 -cyclopropyl-4-(4-((4-((cyclopropylmeth yl) amino)-5-(trifluoromethyl)-7*H*-pyrrolo[2,3-*d*]pyrimidin-2-yl)amino)-3-methoxyphenyl)-1,4-azaphosphinane 4-oxide | $^1$H NMR (400 MHz, DMSO-$d_6$): δ = 12.00 (br s, 1H), 8.64 (dd, *J* = 3.1, 8.3 Hz, 1H), 7.54 (s, 2H), 7.36 - 7.15 (m, 2H), 5.67 (br s, 1H), 3.90 (s, 3H), 3.41 - 3.34 (m, 2H), 3.00 - 2.83 (m, 4H), 2.20 - 2.07 (m, 2H), 1.84 - 1.69 (m, 3H), 1.20 - 1.06 (m, 1H), 0.47 - 0.37 (m, 4H), 0.32 - 0.22 (m, 4H); LCMS (Method 1): m/z = 535.2 $(M + H)^+$; | 18.2 | 2.28 |
| 422 | 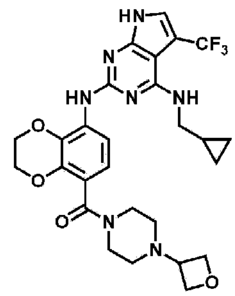 | (8-((4-((cyclopropylmeth yl) amino)-5-(trifluoromethyl)-7*H*-pyrrolo[2,3-*d*]pyrimidin-2-yl)amino)-2,3-dihydrobenzo [*b*][1 ,4]dioxin-5-yl)(4-(oxetan-3-yl)piperazin-1-yl) methanone | $^1$H NMR (400 MHz, DMSO-$d_6$) δ = 12.01 (br s, 1H), 8.10 (d, *J* = 8.5 Hz, 1H), 7.57 (d, *J* = 1.1 Hz, 1H), 7.37 (s, 1H), 6.74 (d, *J* = 8.5 Hz, 1H), 5.71 (br s, 1H), 4.54 (t, *J* = 6.5 Hz, 2H), 4.44 (t, *J* = 6.1 Hz, 2H), 4.38 (br s, 2H), 4.30 (br s, 2H), 3.62 (br s, 2H), 3.46 - 3.38 (m, 3H), 3.29 - 3.20 (m, 2H), 2.29 (br s, 2H), 2.26 - 2.17 (m, 2H), 1.25 - 1.11 (m, 1H), 0.51 - 0.40 (m, 2H), 0.34 - 0.26 (m, 2H); LCMS (Method 1): m/z = 574.2 $(M + H)^+$; | 30.0 | 2.21 |

(continued)

| Example | Structure | Compound name | $^1$H NMR, MS | Yield (%) | HPLC r.t. (min), Purity |
|---|---|---|---|---|---|
| 423 | 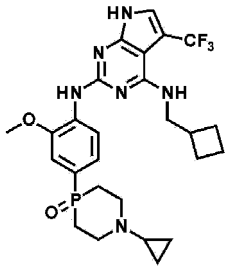 | 4-(4-((4-((cyclobutylmethyl ) amino)-5-(trifluoromethyl)-7*H*-pyrrolo[2,3-*d*]pyrimidin-2-yl)amino)-3-methoxyphenyl)-1-cyclopropyl-1,4-azaphosphinane 4-oxide | $^1$H NMR (400 MHz, DMSO-$d_6$): δ = 11.99 (br s, 1H), 8.63 (dd, *J* = 3.2, 8.0 Hz, 1H), 7.53 (s, 2H), 7.31 - 7.20 (m, 2H), 5.55 ( s, 1H), 3.91 (s, 3H), 3.63 - 3.48 (m, 2H), 3.02 - 2.82 (m, 4H), 2.60 (m, 2H), 2.18 - 2.07 (m, 2H), 2.00 - 1.91 (m, 2H), 1.87 - 1.64 (m, 8H), 0.46 - 0.38 (m, 2H), 0.32 - 0.24 (m, 2H); LCMS (Method 1): m/z = 548.2 (M + H)$^+$; | 7.9 | 2.38 |
| 424 | 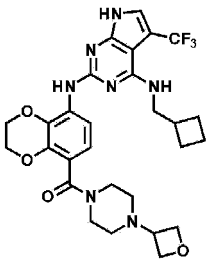 | (8-((4-((cyclobutylmethyl ) amino)-5-(trifluoromethyl)-7*H*-pyrrolo[2,3-*d*]pyrimidin-2-yl)amino)-2,3-dihydrobenzo [*b*][1 ,4]dioxin-5-yl)(4-(oxetan-3-yl)piperazin-1-yl) methanone | $^1$H NMR (400 MHz, DMSO-$d_6$) δ = 12.48 - 11.70 (m, 1H), 8.11 (d, *J* = 8.5 Hz, 1H), 7.56 (d, *J* = 1.4 Hz, 1H), 7.35 (s, 1H), 6.73 (d, *J* = 8.4 Hz, 1H), 5.57 (br s, 1H), 4.54 (t, *J* = 6.5 Hz, 2H), 4.44 (t, *J* = 6.0 Hz, 2H), 4.38 (br s, 2H), 4.31 (br d, *J* = 2.9 Hz, 2H), 3.65 - 3.56 (m, 4H), 3.46 - 3.40 (m, 1H), 3.28 - 3.22 (m, 2H), 2.68 - 2.61 (m, 2H), 2.29 (br s, 2H), 2.23 - 2.18 (m, 1H), 2.07 - 1.95 (m, 2H), 1.92 - 1.82 (m, 2H), 1.80 - 1.71 (m, 2H); LCMS (Method 1): m/z = 588.2 (M + H)$^+$; | 18.1 | 2.31 |

(continued)

| Example | Structure | Compound name | $^1$H NMR, MS | Yield (%) | HPLC r.t. (min), Purity |
|---|---|---|---|---|---|
| 425 | 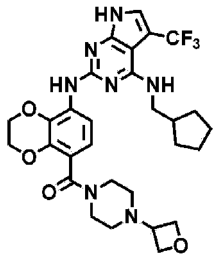 | (8-((4-((cyclopentylmeth yl) amino)-5-(trifluoromethyl)-7*H*-pyrrolo[2,3-*d*]pyrimidin-2-yl)amino)-2,3-dihydrobenzo [*b*][1 ,4]dioxin-5-yl)(4-(oxetan-3-yl)piperazin-1-yl) methanone | $^1$H NMR (400 MHz, DMSO-$d_6$) δ = 12.21 - 11.53 (m, 1H), 8.05 (d, *J* = 8.5 Hz, 1H), 7.49 (d, *J* = 1.5 Hz, 1H), 7.31 - 7.21 (m, 1H), 6.66 (d, *J* = 8.5 Hz, 1H), 5.53 (br s, 1H), 4.47 (t, *J* = 6.5 Hz, 2H), 4.36 (t, *J* = 6.0 Hz, 2H), 4.31 (br s, 2H), 4.23 (br s, 2H), 3.64 - 3.50 (m, 2H), 3.46 - 3.39 (m, 2H), 3.38 - 3.33 (m, 1H), 2.25 - 2.06 (m, 5H), 1.70 - 1.60 (m, 2H), 1.58 - 1.51 (m, 2H), 1.49 - 1.41 (m, 2H), 1.28 - 1.15 (m, 2H); LCMS (Method 1): m/z = 602.3 (M + H)$^+$; | 9.8 | 2.37 |
| 426 | 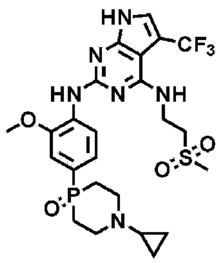 | 1-cyclopropyl-4-(3-methoxy-4-((4-((2-(methylsulfonyl)et hyl)amino)-5-(trifluoromethyl)-7*H*-pyrrolo [2,3-*d*]pyrimidin-2-yl)amino) phenyl)-1,4-azaphosphinane 4-oxide | $^1$H NMR (400 MHz, DMSO-$d_6$): δ = 12.03 (s, 1H), 8.59 (dd, *J* = 3.2, 8.0 Hz, 1H), 7.64 (s, 1H), 7.55 (s, 1H), 7.33 - 7.18 (m, 2H), 6.16 ( t, *J* = 5.2 Hz, 1H), 3.95 (q, *J* = 6.4 Hz, 2H), 3.90 (s, 3H), 3.44 (t, *J* = 6.4 Hz, 2H), 2.99 (s, 3H), 2.97 - 2.79 (m, 4H), 2.11 (m, 2H), 1.87 - 1.70 (m, 3H), 0.45 - 0.36 (m, 2H), 0.33 - 0.17 (m, 2H); LCMS (Method 1): m/z = 586.2 (M + H)$^+$; | 24.3 | 2.34 |

(continued)

| Example | Structure | Compound name | $^1$H NMR, MS | Yield (%) | HPLC r.t. (min), Purity |
|---|---|---|---|---|---|
| 427 | 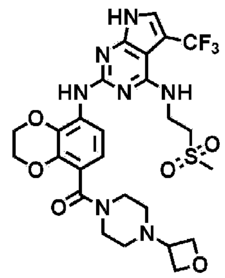 | (8-((4-((2-(methylsulfonyl)ethyl)amino)-5-(trifluoromethyl)-7*H*-pyrrolo[2,3-*d*]pyrimidin-2-yl)amino)-2,3-dihydrobenzo[*b*][1 ,4]dioxin-5-yl)(4-(oxetan-3-yl)piperazin-1-yl)methanone | $^1$H NMR (400 MHz, DMSO-$d_6$): δ = 11.98 (br s, 1H), 7.99 (d, *J* = 8.4 Hz, 1H), 7.51 (s, 1H), 7.41 (s, 1H), 6.68 (d, *J* = 8.5 Hz, 1H), 6.11 (br t, *J* = 5.0 Hz, 1H), 4.47 (t, *J* = 6.5 Hz, 2H), 4.37 (t, *J* = 6.0 Hz, 2H), 4.33 - 4.23 (m, 4H), 3.92 (q, *J* = 6.3 Hz, 2H), 3.55 (br s, 2H), 3.42 (br t, *J* = 6.5 Hz, 2H), 3.39 - 3.33 (m, 1H), 3.22 - 3.14 (m, 2H), 3.00 - 2.96 (m, 3H), 2.25 - 2.08 (m, 4H); LCMS (Method 1): m/z = 583.2 (M + H)$^+$; | 40.4 | 2.29 |
| 428 | 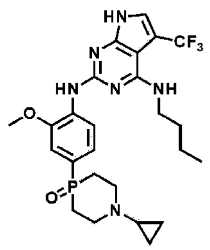 | 4-(4-((4-(butylamino)-5-(trifluoromethyl)-7*H*-pyrrolo[2,3-*d*]pyrimidin-2-yl)amino)-3-methoxyphenyl)-1-cyclopropyl-1,4-azaphosphinane 4-oxide | $^1$H NMR (400 MHz, DMSO-$d_6$): δ = 11.97 (br s, 1H), 8.63 (dd, *J* = 3.2, 8.0 Hz, 1H), 7.53 (s, 2H), 7.38 - 7.07 (m, 2H), 5.65 ( s, 1H), 3.90 (s, 3H), 3.50 (q, *J* = 6.8 Hz, 2H), 3.03 - 2.80 (m, 4H), 2.13 (m, 2H), 1.85 - 1.66 (m, 3H), 1.55 (m, 2H), 1.39 - 1.24 (m, 2H), 0.86 (t, *J* = 7.4 Hz, 3H), 0.48 - 0.37 (m, 2H), 0.34 - 0.19 (m, 2H); LCMS (Method 1): m/z = 536.2 (M + H)$^+$; | 14.0 | 2.32 |

(continued)

| Example | Structure | Compound name | $^1$H NMR, MS | Yield (%) | HPLC r.t. (min), Purity |
|---|---|---|---|---|---|
| 429 | 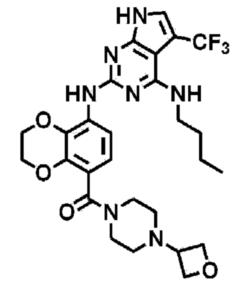 | (8-((4-(butylamino)-5-(trifluoromethyl)-7*H*-pyrrolo[2,3-*d*]pyrimidin-2-yl)amino)-2,3-dihydrobenzo[*b*][1 ,4]dioxin-5-yl)(4-(oxetan-3-yl)piperazin-1-yl)methanone | $^1$H NMR (400 MHz, DMSO-$d_6$): δ = 11.99 (br d, *J* = 1.6 Hz, 1H), 8.11 (d, *J* = 8.4 Hz, 1H), 7.56 (s, 1H), 7.35 (s, 1H), 6.73 (d, *J* = 8.5 Hz, 1H), 5.67 (br s, 1H), 4.58 - 4.51 (m, 2H), 4.44 (t, *J* = 6.1 Hz, 2H), 4.38 (br s, 2H), 4.31 (br s, 2H), 3.70 - 3.60 (m, 2H), 3.55 (q, *J* = 6.6 Hz, 2H), 3.43 (td, *J* = 6.2, 12.4 Hz, 1H), 3.30 (br s, 2H), 2.32 - 2.11 (m, 4H), 1.61 (quin, *J* = 7.3 Hz, 2H), 1.44 - 1.33 (m, 2H), 0.93 (t, *J* = 7.3 Hz, 3H); LCMS (Method 1): m/z = 576.2 (M + H)$^+$; | 19.9 | 2.26 |
| 430 | 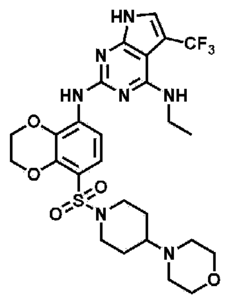 | *N*$^4$-ethyl-*N*$^2$-(8-((4-morpholinopiperid in-1-yl) sulfonyl)-2,3-dihydrobenzo[*b*][1 ,4]dioxin- 5-yl)-5-(trifluoromethyl)-7*H*-pyrrolo[2,3-*d*]pyrimidine-2,4-diamine | LCMS (Method 7): m/z = 612.3 [M + H]$^+$ | 53 | 1.40 |
| 431 | 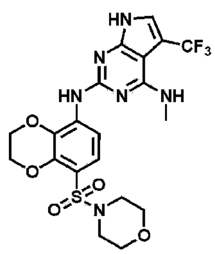 | *N*$^4$-methyl-*N*$^2$-(8-(morpholinosulfon yl)-2,3-dihydrobenzo[*b*][1 ,4]dioxin-5-yl)-5-(trifluoromethyl)-7*H*-pyrrolo[2,3-*d*]pyrimidine-2,4-diamine | LCMS (Method 7): m/z = 598.3 [M + H]$^+$ | 59 | 1.29 |

(continued)

| Example | Structure | Compound name | $^1$H NMR, MS | Yield (%) | HPLC r.t. (min), Purity |
|---|---|---|---|---|---|
| 432 | 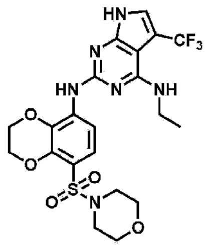 | *N*4-ethyl-*N*2-(8-(morpholinosulfon yl)-2,3-dihydrobenzo[*b*][1 ,4] dioxin-5-yl)-5-(trifluoromethyl)-7*H*-pyrrolo [2,3-*d*]pyrimidine-2,4-diamine | $^1$H NMR (400 MHz, DMSO-$d_6$) δ 12.09 (s, 1H), 8.32 (d, *J* = 9.0 Hz, 1H), 7.62 (d, *J* = 1.2 Hz, 1H), 7.54 (s, 1H), 7.26 (d, *J* = 9.0 Hz, 1H), 5.83 (s, 1H), 4.49 - 4.38 (m, 4H), 3.60 (dt, *J* = 13.7, 5.5 Hz, 6H), 3.06 - 3.01 (m, 4H), 1.22 (t, *J* = 7.1 Hz, 3H). LCMS (Method 7): m/z = 529.2 [M + H]$^+$ | 52 | 1.72 |
| 433 | 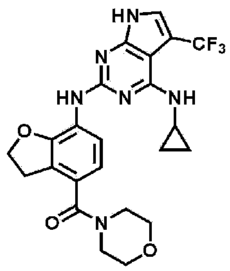 | (7-((4-(cyclopropylamino )-5-(trifluoromethyl)-7*H*-pyrrolo [2,3-*d*]pyrimidin-2-yl)amino)-2,3-dihydrobenzofuran -4-yl) (morpholino)me thanone | $^1$H NMR (400 MHz, DMSO-$d_6$): δ = 12.03 (br d, *J* = 1.4 Hz, 1H), 8.29 (d, *J* = 8.3 Hz, 1H), 7.57 (s, 1H), 7.42 (s, 1H), 6.80 (d, *J* = 8.3 Hz, 1H), 5.55 (br s, 1H), 4.64 (t, *J* = 8.8 Hz, 2H), 3.59 (br s, 4H), 3.49 (br s, 4H), 3.21 (t, *J* = 8.8 Hz, 2H), 2.93 (br dd, *J* = 3.8, 6.9 Hz, 1H), 0.89 - 0.77 (m, 2H), 0.64 - 0.53 (m, 2H); LCMS (Method 1): m/z = 489.2 (M + H)$^+$; | 30.5 | 2.36 |
| 434 | 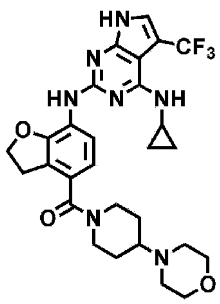 | (7-((4-(cyclopropylamino )-5-(trifluoromethyl)-7*H*-pyrrolo [2,3-*d*]pyrimidin-2-yl)amino)-2,3-dihydrobenzofuran -4-yl) (4-morpholinopiperid in-1-yl) methanone | $^1$H NMR (400 MHz, DMSO-$d_6$) δ = 12.00 (d, *J* = 2.5 Hz, 1H), 8.24 (br d, *J* = 8.3 Hz, 1H), 7.55 (s, 1H), 7.40 (s, 1H), 6.76 (d, *J* = 8.3 Hz, 1H), 5.53 (br s, 1H), 4.65 - 4.49 (m, 2H), 4.45 - 4.20 (m, 1H), 3.66 - 3.47 (m, 4H), 3.20 - 3.11 (m, 2H), 3.10 - 2.70 (m, 3H), 2.50 (br s, 2H), 2.46 - 2.41 (m, 2H), 2.38 (br dd, *J* = 1.9, 3.9 Hz, 1H), 1.93 - 1.62 (m, 2H), 1.50 - 1.21 (m, 2H), 0.84 - 0.68 (m, 2H), 0.61 - 0.50 (m, 2H); LCMS (Method 1): m/z = 572.3 (M + H)$^+$; | 29.9 | 2.03 |

(continued)

| Example | Structure | Compound name | $^1H$ NMR, MS | Yield (%) | HPLC r.t. (min), Purity |
|---|---|---|---|---|---|
| 435 | 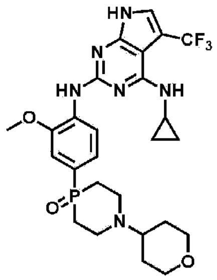 | 4-(4-((4-(cyclopropylamino )-5-(trifluoromethyl)-7*H*-pyrrolo [2,3-*d*]pyrimidin-2-yl)amino)-3-methoxyphenyl)-1-(tetrahydro-2*H*-pyran-4-yl)-1,4-azaphosphinane 4-oxide | $^1H$ NMR (400 MHz, DMSO-$d_6$): $\delta$ = 12.11 (br s, 1H), 8.93 (br d, *J* = 5.0 Hz, 1H), 7.66 (br d, *J* = 5.1 Hz, 2H), 7.51 - 7.23 (m, 2H), 5.66 (br s, 1H), 3.99 (s, 3H), 3.90 (br d, *J* = 8.1 Hz, 2H), 3.31 - 3.24 (m, 2H), 3.05 - 2.81 (m, 5H), 2.76 - 2.61 (m, 1H), 2.50 - 2.39 (m, 2H), 2.29 - 2.10 (m, 2H), 1.92 - 1.76 (m, 2H), 1.72 - 1.59 (m, 2H), 1.56 - 1.42 (m, 2H), 0.94 - 0.85 (m, 2H), 0.67 - 0.58 (m, 2H); LCMS (Method 1): m/z = 565.2 (M + H)$^+$; | 17.7 | 2.41 |
| 436 | 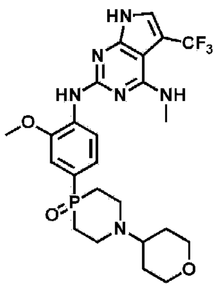 | 4-(3-methoxy-4-((4-(methylamino)-5-(trifluoromethyl)-7*H*-pyrrolo [2,3-*d*]pyrimidin-2-yl)amino) phenyl)-1-(tetrahydro-2*H*-pyran-4-yl)-1,4-azaphosphinane 4-oxide | $^1H$ NMR (400 MHz, DMSO-$d_6$): $\delta$ = 12.05 (d, *J* = 2.4 Hz, 1H), 8.75 (dd, *J* = 3.1, 8.3 Hz, 1H), 7.60 (s, 1H), 7.58 (s, 1H), 7.38 - 7.32 (m, 1H), 7.30 (dd, *J* = 1.4, 11.8 Hz, 1H), 5.97 (br d, *J* = 4.3 Hz, 1H), 3.98 (s, 3H), 3.89 (br dd, *J* = 3.7, 10.8 Hz, 2H), 3.32 - 3.23 (m, 3H), 3.04 (d, *J* = 4.5 Hz, 3H), 2.97 - 2.87 (m, 4H), 2.25 - 2.15 (m, 2H), 1.89 - 1.78 (m, 2H), 1.64 (br d, *J* = 12.3 Hz, 2H), 1.54 - 1.42 (m, 2H); LCMS (Method 1): m/z = 539.2 (M + H)$^+$; | 5.2 | 2.29 |

(continued)

| Example | Structure | Compound name | $^1$H NMR, MS | Yield (%) | HPLC r.t. (min), Purity |
|---|---|---|---|---|---|
| 437 | 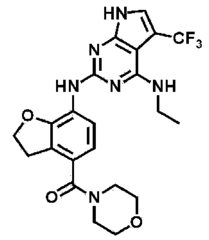 | (7-((4-(ethylamino)-5-(trifluoromethyl)-7*H*-pyrrolo [2,3-*d*]pyrimidin-2-yl)amino)-2,3-dihydrobenzofuran -4-yl) (morpholino)me thanone | $^1$H NMR (400 MHz, DMSO-$d_6$): δ = 11.96 (br s, 1H), 8.12 (d, *J* = 8.3 Hz, 1H), 7.53 (d, *J* = 1.3 Hz, 1H), 7.41 (s, 1H), 6.79 (d, *J* = 8.3 Hz, 1H), 5.76 - 5.64 (m, 1H), 4.62 (t, *J* = 8.8 Hz, 2H), 3.64 - 3.57 (m, 4H), 3.55 (br dd, *J* = 5.8, 6.9 Hz, 3H), 3.52 (br s, 3H), 3.20 (t, *J* = 8.8 Hz, 2H), 1.19 (t, *J* = 7.1 Hz, 3H); LCMS (Method 1): m/z = 477.2 $(M + H)^+$; | 31.8 | 2.35 |
| 438 | 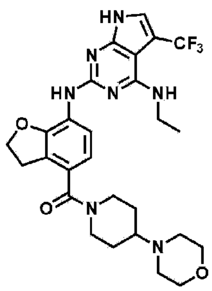 | (7-((4-(ethylamino)-5-(trifluoromethyl)-7*H*-pyrrolo [2,3-*d*]pyrimidin-2-yl)amino)-2,3-dihydrobenzofuran -4-yl) (4-morpholinopiperid in-1-yl) methanone | $^1$H NMR (400 MHz, DMSO-$d_6$): δ = 12.24 - 11.66 (m, 1H), 8.08 (d, *J* = 8.4 Hz, 1H), 7.53 (d, *J* = 1.6 Hz, 1H), 7.42 (s, 1H), 6.77 (d, *J* = 8.4 Hz, 1H), 5.70 ( t, *J* = 5.2 Hz, 1H), 4.62 (t, *J* = 8.8 Hz, 2H), 4.51 - 4.23 (m, 1H), 3.86 - 3.65 (m, 1H), 3.61 - 3.49 (m, 7H), 3.32 (s, 1H), 3.23 - 3.12 (m, 2H), 3.08 - 2.75 (m, 2H), 2.48 - 2.45 (m, 4H), 2.43 - 2.36 (m, 1H), 1.92 - 1.63 (m, 2H), 1.36 - 1.26 (m, 2H), 1.19 (t, *J* = 7.2 Hz, 3H); LCMS (Method 1): m/z = 560.3 $(M + H)^+$; | 27.5 | 2.02 |

(continued)

| Example | Structure | Compound name | $^1H$ NMR, MS | Yield (%) | HPLC r.t. (min), Purity |
|---|---|---|---|---|---|
| 439 | 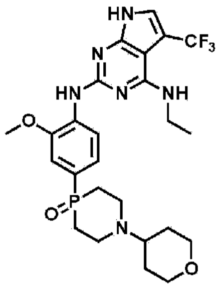 | 4-(4-((4-(ethylamino)-5-(trifluoromethyl)-7*H*-pyrrolo[2,3-*d*]pyrimidin-2-yl)amino)-3-methoxyphenyl)-1-(tetrahydro-2*H*-pyran-4-yl)-1,4-azaphosphinane 4-oxide | $^1H$ NMR (400 MHz, DMSO-$d_6$): δ = 12.05 (br s, 1H), 8.72 (dd, *J* = 3.1, 8.3 Hz, 1H), 7.59 (s, 2H), 7.38 - 7.26 (m, 2H), 5.79 (br s, 1H), 3.97 (s, 3H), 3.89 (br dd, *J* = 3.3, 11.1 Hz, 2H), 3.59 (dd, *J* = 5.8, 6.9 Hz, 2H), 3.32 - 3.23 (m, 3H), 2.97 - 2.87 (m, 4H), 2.20 (br dd, *J* = 8.0, 13.8 Hz, 2H), 1.90 - 1.77 (m, 2H), 1.68 - 1.60 (m, 2H), 1.48 (br dd, *J* = 4.3, 11.9 Hz, 2H), 1.22 (t, *J* = 7.1 Hz, 3H); LCMS (Method 1): m/z = 523.2 (M + H)$^+$; | 3.4 | 2.43 |
| 440 | 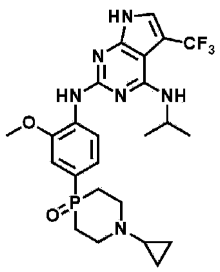 | 1 -cyclopropyl-4-(4-((4-(isopropylamino)-5-(trifluoromethyl)-7*H*-pyrrolo[2,3-*d*]pyrimidin-2-yl)amino)-3-methoxyphenyl)-1,4-azaphosphinane 4-oxide | $^1H$ NMR (400 MHz, DMSO-$d_6$): δ = 12.11 ( d, *J* = 2.0 Hz, 1H), 8.71 (dd, *J* = 3.2, 8.4 Hz, 1H), 7.70 - 7.56 (m, 2H), 7.37 (m, 1H), 7.31 (dd, *J* = 1.6, 11.6 Hz, 1H), 5.13 (br dd, *J* = 1.6, 7.2 Hz, 1H), 4.50 - 4.30 (m, 1H), 4.04 - 3.92 (m, 3H), 3.08 - 2.89 (m, 4H), 2.25 - 2.13 (m, 2H), 1.90 - 1.75 (m, 3H), 1.28 (d, *J* = 6.4 Hz, 6H), 0.54 - 0.43 (m, 2H), 0.40 - 0.30 (m, 2H); LCMS (Method 1): m/z = 523.2 (M + H)$^+$; | 5.1 | 2.28 |

(continued)

| Example | Structure | Compound name | $^1H$ NMR, MS | Yield (%) | HPLC r.t. (min), Purity |
|---|---|---|---|---|---|
| 441 | 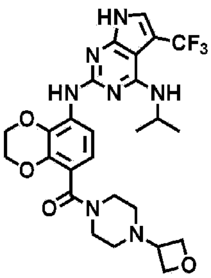 | (8-((4-(isopropylamino)-5-(trifluoromethyl)-7*H*-pyrrolo [2,3-*d*]pyrimidin-2-yl)amino)-2,3-dihydrobenzo[*b*][1 ,4] dioxin-5-yl)(4-(oxetan-3-yl) piperazin-1-yl)methanone | $^1H$ NMR (400 MHz, DMSO-$d_6$): δ = 12.06 (br s, 1H), 8.10 (d, *J* = 8.5 Hz, 1H), 7.60 (s, 1H), 7.39 (s, 1H), 6.75 (d, *J* = 8.5 Hz, 1H), 5.14 - 5.00 (m, 1H), 4.57 - 4.52 (m, 2H), 4.46 - 4.42 (m, 2H), 4.40 - 4.37 (m, 2H), 4.31 (br d, *J* = 2.8 Hz, 2H), 3.62 (br s, 2H), 3.43 (quin, *J* = 6.3 Hz, 1H), 3.31 - 3.20 (m, 2H), 2.31 - 2.14 (m, 4H), 1.26 (d, *J* = 6.5 Hz, 6H); LCMS (Method 1): m/z = 562.2 (M + H)$^+$; | 10.1 | 2.21 |
| 442 | 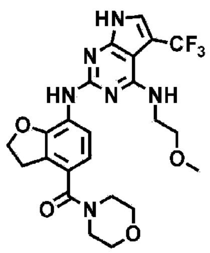 | (7-((4-((2-methoxyethyl)ami no)-5-(trifluoromethyl)-7*H*-pyrrolo[2,3-*d*]pyrimidin-2-yl)amino)-2,3-dihydrobenzofuran -4-yl) (morpholino)me thanone | $^1H$ NMR (400 MHz, DMSO-$d_6$): δ = 12.33 - 11.66 (m, 1H), 8.07 (d, *J* = 8.3 Hz, 1H), 7.55 (d, *J* = 1.5 Hz, 1H), 7.50 (s, 1H), 6.78 (d, *J* = 8.3 Hz, 1H), 5.74 (br d, *J* = 1.3 Hz, 1H), 4.62 (t, *J* = 8.8 Hz, 2H), 3.69 (q, *J* = 5.5 Hz, 2H), 3.59 (br s, 4H), 3.56 - 3.52 (m, 3H), 3.48 (br d, *J* = 5.1 Hz, 3H), 3.30 (s, 3H), 3.20 (t, *J* = 8.7 Hz, 2H); LCMS (Method 1): m/z = 507.2 (M + H)$^+$; | 17.8 | 2.34 |

(continued)

| Example | Structure | Compound name | $^1H$ NMR, MS | Yield (%) | HPLC r.t. (min), Purity |
|---|---|---|---|---|---|
| 443 | 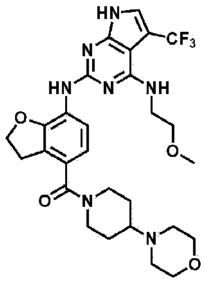 | (7-((4-((2-methoxyethyl)ami no)-5-(trifluoromethyl)-7*H*-pyrrolo [2,3-*d*]pyrimidin-2-yl)amino)-2,3-dihydrobenzofuran -4-yl)(4-morpholinopiperid in-1-yl) methanone | $^1H$ NMR (400 MHz, DMSO-$d_6$) $\delta$ = 12.00 (br s, 1H), 8.04 (d, *J* = 8.3 Hz, 1H), 7.56 (s, 1H), 7.50 (s, 1H), 6.77 (d, *J* = 8.3 Hz, 1H), 5.74 (br d, *J* = 0.8 Hz, 1H), 4.62 (t, *J* = 8.8 Hz, 2H), 3.70 (q, *J* = 5.5 Hz, 2H), 3.59 - 3.53 (m, 6H), 3.31 (s, 3H), 3.18 (t, *J* = 8.7 Hz, 2H), 2.53 (br d, *J* = 1.9 Hz, 4H), 2.47 (br s, 4H), 2.44 - 2.36 (m, 1H), 1.81 (br d, *J* = 4.1 Hz, 2H), 1.40 - 1.21 (m, 2H); LCMS (Method 1): m/z = 590.3 (M + H)$^+$; | 24.4 | 2.02 |
| 444 | 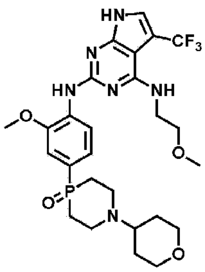 | 4-(3-methoxy-4-((4-((2-methoxyethyl)ami no)-5-(trifluoromethyl)-7*H*-pyrrolo [2,3-*d*]pyrimidin-2-yl)amino) phenyl)-1-(tetrahydro-2*H*-pyran-4-yl)-1,4-azaphosphinane 4-oxide | $^1H$ NMR (400 MHz, DMSO-$d_6$): $\delta$ = 12.24 ( s, 1H), 8.82 (dd, *J* = 3.2, 8.4 Hz, 1H), 7.76 (s, 2H), 7.50 - 7.46 (m, 1H), 7.43 (dd, *J* = 1.6, 12 Hz, 1H), 5.95 ( s, 1H), 4.10 (s, 3H), 4.02 (dd, *J* = 3.6, 10.8 Hz, 2H), 3.86 (q, *J* = 5.6 Hz, 2H), 3.75 - 3.66 (m, 2H), 3.44 (s, 3H), 3.43 - 3.37 (m, 1H), 3.38 (s, 1H), 3.12 - 2.96 (m, 4H), 2.88 - 2.80 (m, 1H), 2.42 - 2.24 (m, 2H), 2.05 - 1.88 (m, 2H), 1.77 (br d, *J* = 11.2 Hz, 2H), 1.68 - 1.47 (m, 2H); LCMS (Method 1): m/z = 583.2 (M + H)$^+$; | 24.9 | 2.42 |

(continued)

| Example | Structure | Compound name | $^1H$ NMR, MS | Yield (%) | HPLC r.t. (min), Purity |
|---|---|---|---|---|---|
| 445 | 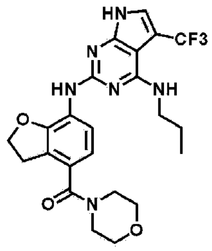 | morpholino (7-((4-(propylamino)-5-(trifluoromethyl)-7*H*-pyrrolo [2,3-*d*]pyrimidin-2-yl)amino)-2,3-dihydrobenzofuran -4-yl) methanone | $^1H$ NMR (400 MHz, DMSO-$d_6$): $\delta$ = 11.94 (br d, *J* = 2.0 Hz, 1H), 8.10 (d, *J* = 8.3 Hz, 1H), 7.54 (d, *J* = 1.5 Hz, 1H), 7.43 (s, 1H), 6.78 (d, *J* = 8.3 Hz, 1H), 5.75 - 5.62 (m, 1H), 4.62 (t, *J* = 8.8 Hz, 2H), 3.59 (br s, 4H), 3.53 - 3.44 (m, 4H), 3.33 - 3.30 (m, 2H), 3.20 (t, *J* = 8.7 Hz, 2H), 1.61 (sxt, *J* = 7.3 Hz, 2H), 0.91 (t, *J* = 7.4 Hz, 3H); LCMS (Method 1): m/z = 491.2 $(M + H)^+$; | 20.5 | 2.42 |
| 446 | 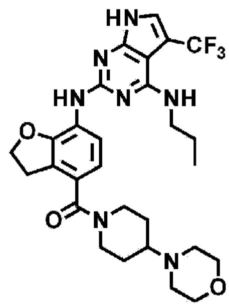 | (4-morpholinopiperid in-1-yl) (7-((4-(propylamino)-5-(trifluoromethyl)-7*H*-pyrrolo [2,3-*d*]pyrimidin-2-yl)amino)-2,3-dihydrobenzofuran -4-yl) methanone | $^1H$ NMR (400 MHz, DMSO-$d_6$) $\delta$ = 11.96 (br s, 1H), 8.06 (d, *J* = 8.4 Hz, 1H), 7.54 (s, 1H), 7.44 (s, 1H), 6.76 (d, *J* = 8.3 Hz, 1H), 5.74 - 5.63 (m, 1H), 4.62 (t, *J* = 8.8 Hz, 2H), 3.60 - 3.53 (m, 4H), 3.51 - 3.43 (m, 2H), 3.18 (t, *J* = 8.8 Hz, 2H), 3.07 - 2.73 (m, 2H), 2.54 - 2.52 (m, 2H), 2.46 (br s, 4H), 2.42 - 2.36 (m, 1H), 1.91 - 1.69 (m, 2H), 1.61 (sxt, *J* = 7.3 Hz, 2H), 1.42 - 1.20 (m, 2H), 0.91 (t, *J* = 7.4 Hz, 3H); LCMS (Method 1): m/z = 574.3 $(M + H)^+$; | 19.2 | 2.08 |

(continued)

| Example | Structure | Compound name | $^1H$ NMR, MS | Yield (%) | HPLC r.t. (min), Purity |
|---|---|---|---|---|---|
| 447 | 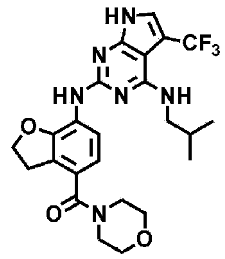 | (7-((4-(isobutylamino)-5-(trifluoromethyl)-7*H*-pyrrolo [2,3-*d*]pyrimidin-2-yl)amino)-2,3-dihydrobenzofuran -4-yl)(morpholino)me thanone | $^1H$ NMR (400 MHz, DMSO-$d_6$): δ = 11.97 (br s, 1H), 8.08 (d, *J* = 8.3 Hz, 1H), 7.55 (s, 1H), 7.46 (s, 1H), 6.78 (d, *J* = 8.3 Hz, 1H), 5.73 - 5.46 (m, 1H), 4.62 (t, *J* = 8.8 Hz, 2H), 3.59 (br s, 4H), 3.54 - 3.41 (m, 3H), 3.39 - 3.34 (m, 3H), 3.20 (t, *J* = 8.8 Hz, 2H), 1.96 (td, *J* = 6.7, 13.5 Hz, 1H), 0.91 (d, *J* = 6.8 Hz, 6H); LCMS (Method 1): m/z = 505.2 $(M + H)^+$; | 23.1 | 2.52 |
| 448 | 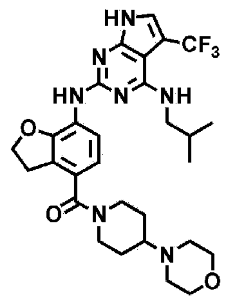 | (7-((4-(isobutylamino)-5-(trifluoromethyl)-7*H*-pyrrolo [2,3-*d*]pyrimidin-2-yl)amino)-2,3-dihydrobenzofuran -4-yl)(4-morpholinopiperid in-1-yl) methanone | $^1H$ NMR (400 MHz, DMSO-$d_6$) δ = 11.96 (br s, 1H), 8.03 (d, *J* = 8.3 Hz, 1H), 7.54 (d, *J* = 1.3 Hz, 1H), 7.49 - 7.43 (m, 1H), 6.75 (d, *J* = 8.3 Hz, 1H), 5.62 (br s, 1H), 4.61 (t, *J* = 8.8 Hz, 2H), 3.58 - 3.53 (m, 4H), 3.39 - 3.35 (m, 2H), 3.31 (s, 2H), 3.17 (t, *J* = 8.8 Hz, 2H), 3.07 - 2.73 (m, 2H), 2.47 - 2.42 (m, 4H), 2.41 - 2.35 (m, 1H), 2.03 - 1.90 (m, 1H), 1.85 - 1.70 (m, 2H), 1.40 - 1.23 (m, 2H), 0.91 (d, *J* = 6.6 Hz, 6H); LCMS (Method 1): m/z = 588.3 $(M + H)^+$; | 20.8 | 2.17 |

(continued)

| Example | Structure | Compound name | $^1$H NMR, MS | Yield (%) | HPLC r.t. (min), Purity |
|---|---|---|---|---|---|
| 449 | 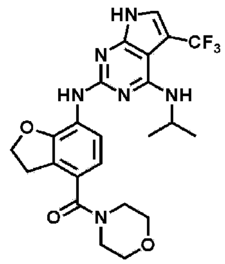 | (7-((4-(isopropylamino)-5-(trifluoromethyl)-7*H*-pyrrolo [2,3-*d*]pyrimidin-2-yl)amino)-2,3-dihydrobenzofuran -4-yl) (morpholino)me thanone | $^1$H NMR (400 MHz, DMSO-$d_6$): δ = 11.99 (br d, *J* = 1.1 Hz, 1H), 8.07 (d, *J* = 8.3 Hz, 1H), 7.56 (d, *J* = 1.5 Hz, 1H), 7.52 - 7.44 (m, 1H), 6.79 (d, *J* = 8.3 Hz, 1H), 5.04 (br dd, *J* = 1.3, 7.4 Hz, 1H), 4.62 (t, *J* = 8.8 Hz, 2H), 4.35 (qd, *J* = 6.6, 13.4 Hz, 1H), 3.59 (br s, 4H), 3.54 - 3.40 (m, 3H), 3.31 (s, 1H), 3.20 (t, *J* = 8.7 Hz, 2H), 1.24 (d, *J* = 6.5 Hz, 6H); LCMS (Method 1): m/z = 491.2 (M + H)$^+$; | 22.5 | 2.47 |
| 450 | 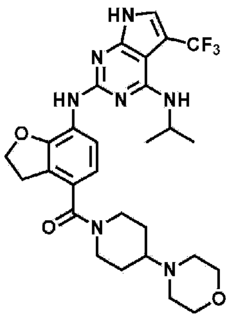 | (7-((4-(isopropylamino)-5-(trifluoromethyl)-7*H*-pyrrolo [2,3-*d*]pyrimidin-2-yl)amino)-2,3-dihydrobenzofuran -4-yl) (4-morpholinopiperid in-1-yl) methanone | $^1$H NMR (400 MHz, DMSO-$d_6$) δ = 11.98 (br s, 1H), 8.01 (d, *J* = 8.3 Hz, 1H), 7.54 (s, 1H), 7.47 (s, 1H), 6.74 (d, *J* = 8.3 Hz, 1H), 5.02 (br dd, *J* = 1.6, 7.3 Hz, 1H), 4.59 (t, *J* = 8.8 Hz, 2H), 4.33 (qd, *J* = 6.6, 13.4 Hz, 1H), 3.58 - 3.52 (m, 4H), 3.30 (br s, 2H), 3.15 (t, *J* = 8.8 Hz, 2H), 3.07 - 2.70 (m, 2H), 2.44 (br s, 4H), 2.40 - 2.33 (m, 1H), 1.86 - 1.70 (m, 2H), 1.38 - 1.26 (m, 2H), 1.22 (d, *J* = 6.4 Hz, 6H); LCMS (Method 1): m/z = 574.3 (M + H)$^+$; | 19.2 | 2.13 |

(continued)

| Example | Structure | Compound name | $^1$H NMR, MS | Yield (%) | HPLC r.t. (min), Purity |
|---|---|---|---|---|---|
| 451 | 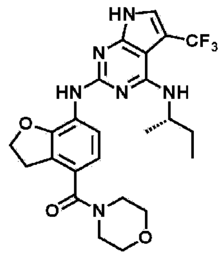 | (S)-(7-((4-(sec-butylamino)-5-(trifluoromethyl)-7*H*-pyrrolo [2,3-*d*]pyrimidin-2-yl)amino)-2,3-dihydrobenzofuran -4-yl) (morpholino)me thanone | $^1$H NMR (400 MHz, DMSO-$d_6$): $\delta$ = 12.00 (br s, 1H), 8.06 (d, *J* = 8.3 Hz, 1H), 7.57 (d, *J* = 1.4 Hz, 1H), 7.49 (s, 1H), 6.78 (d, *J* = 8.3 Hz, 1H), 5.08 - 4.95 (m, 1H), 4.62 (t, *J* = 8.8 Hz, 2H), 4.30 - 4.15 (m, 1H), 3.59 (br s, 4H), 3.48 (br d, *J* = 5.3 Hz, 2H), 3.31 (s, 1H), 3.20 (t, *J* = 8.8 Hz, 2H), 1.59 (dquin, *J* = 2.8, 7.1 Hz, 2H), 1.21 (d, *J* = 6.5 Hz, 3H), 0.90 (t, *J* = 7.4 Hz, 3H); LCMS (Method 1): m/z = 505.2 (M + H)$^+$; | 25.4 | 2.55 |
| 452 | 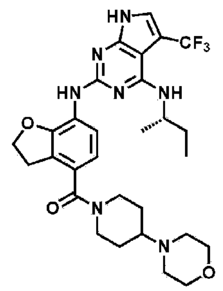 | (*S*)-(7-((4-(sec-butylamino)-5-(trifluoromethyl)-7*H*-pyrrolo [2,3-*d*]pyrimidin-2-yl)amino)-2,3-dihydrobenzofuran -4-yl) (4-morpholinopiperid in-1-yl) methanone | $^1$H NMR (400 MHz, DMSO-$d_6$) $\delta$ = 12.08 (br d, *J* = 1.1 Hz, 1H), 8.12 (d, *J* = 8.4 Hz, 1H), 7.66 (d, *J* = 1.3 Hz, 1H), 7.58 (s, 1H), 6.87 (d, *J* = 8.3 Hz, 1H), 5.12 (br dd, *J* = 1.4, 7.8 Hz, 1H), 4.72 (t, *J* = 8.8 Hz, 2H), 4.32 (td, *J* = 6.8, 13.5 Hz, 1H), 3.69 - 3.65 (m, 4H), 3.41 (br s, 2H), 3.28 (t, *J* = 8.8 Hz, 2H), 3.16 - 2.87 (m, 2H), 2.57 (br d, *J* = 3.8 Hz, 4H), 2.53 - 2.47 (m, 1H), 1.90 (br s, 2H), 1.74 - 1.62 (m, 2H), 1.48 - 1.37 (m, 2H), 1.32 (d, *J* = 6.5 Hz, 3H), 1.01 (t, *J* = 7.4 Hz, 3H); LCMS (Method 1): m/z = 588.3 (M + H)$^+$; | 13.8 | 2.20 |

(continued)

| Example | Structure | Compound name | $^1$H NMR, MS | Yield (%) | HPLC r.t. (min), Purity |
|---|---|---|---|---|---|
| 453 | 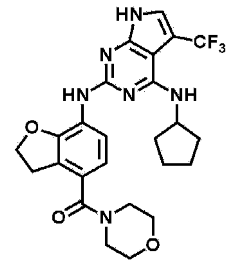 | (7-((4-(cyclopentylamino )-5-(trifluoromethyl)-7*H*-pyrrolo [2,3-*d*]pyrimidin-2-yl)amino)-2,3-dihydrobenzofuran -4-yl)(morpholino)me thanone | $^1$H NMR (400 MHz, DMSO-$d_6$) $\delta$ = 11.99 (br s, 1H), 8.08 (d, *J* = 8.3 Hz, 1H), 7.56 (d, *J* = 1.6 Hz, 1H), 7.50 (s, 1H), 6.78 (d, *J* = 8.3 Hz, 1H), 5.23 - 5.10 (m, 1H), 4.62 (t, *J* = 8.8 Hz, 2H), 4.50 - 4.37 (m, 1H), 3.59 (br s, 4H), 3.56 - 3.39 (m, 4H), 3.20 (t, *J* = 8.7 Hz, 2H), 2.10 - 1.96 (m, 2H), 1.72 - 1.57 (m, 4H), 1.53 - 1.39 (m, 2H); LCMS (Method 1): m/z = 517.2(M + H)$^+$; | 19.4 | 2.58 |
| 454 | 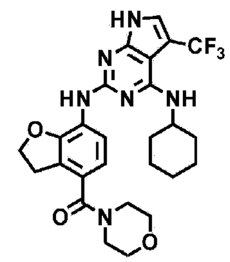 | (7-((4-(cyclohexylamino) -5-(trifluoromethyl)-7*H*-pyrrolo[2,3-*d*]pyrimidin-2-yl)amino)-2,3-dihydrobenzofuran -4-yl)(morpholino)me thanone | $^1$H NMR (400 MHz, DMSO-$d_6$): $\delta$ = 11.99 (br s, 1H), 8.05 (d, *J* = 8.3 Hz, 1H), 7.65 - 7.42 (m, 2H), 6.78 (d, *J* = 8.3 Hz, 1H), 5.12 (br d, *J* = 6.1 Hz, 1H), 4.62 (t, *J* = 8.8 Hz, 2H), 4.17 - 3.97 (m, 1H), 3.59 (br s, 4H), 3.54 - 3.40 (m, 3H), 3.20 (t, *J* = 8.7 Hz, 2H), 2.03 - 1.91 (m, 2H), 1.69 (br dd, *J* = 3.8, 8.9 Hz, 2H), 1.59 (br dd, *J* = 3.7, 9.1 Hz, 1H), 1.48 - 1.18 (m, 6H); LCMS (Method 1): m/z = 531.2 (M + H)$^+$; | 11.3 | 2.98 |

(continued)

| Example | Structure | Compound name | $^1$H NMR, MS | Yield (%) | HPLC r.t. (min), Purity |
|---|---|---|---|---|---|
| 455 | 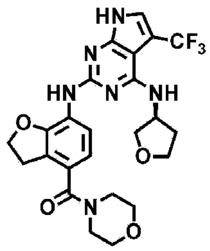 | (*S*)-morpholino (7-((4-((tetrahydrofuran-3-yl) amino)-5-(trifluoromethyl)-7*H*-pyrrolo[2,3-*d*]pyrimidin-2-yl)amino)-2,3-dihydrobenzofuran -4-yl) methanone | $^1$H NMR (400 MHz, DMSO-$d_6$) δ = 12.03 (br s, 1H), 8.01 (d, *J* = 8.3 Hz, 1H), 7.62 (s, 1H), 7.57 (d, *J* = 1.4 Hz, 1H), 6.81 - 6.75 (m, 1H), 5.30 (br dd, *J* = 1.7, 6.3 Hz, 1H), 4.73 - 4.65 (m, 1H), 4.60 (t, *J* = 8.8 Hz, 2H), 3.89 (dd, *J* = 5.6, 9.1 Hz, 1H), 3.86 - 3.79 (m, 1H), 3.73 (dt, *J* = 5.7, 8.3 Hz, 1H), 3.64 - 3.54 (m, 6H), 3.52 - 3.42 (m, 3H), 3.18 (t, *J* = 8.7 Hz, 2H), 2.32 - 2.23 (m, 1H), 1.91 - 1.78 (m, 1H); LCMS (Method 1): m/z = 519.2 (M + H)$^+$; | 21.5 | 2.66 |
| 456 | 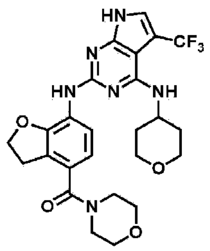 | morpholino (7-((4-((tetrahydro-2*H*-pyran-4-yl)amino)-5-(trifluoromethyl)-7*H*-pyrrolo [2,3-*d*]pyrimidin-2-yl)amino)-2,3-dihydrobenzofuran -4-yl) methanone | $^1$H NMR (400 MHz, DMSO-$d_6$): δ = 12.23 - 11.79 (m, 1H), 8.03 (d, *J* = 8.4 Hz, 1H), 7.68 - 7.53 (m, 2H), 6.88 - 6.72 (m, 1H), 5.25 - 5.07 (m, 1H), 4.75 - 4.53 (m, 2H), 4.33 - 4.15 (m, 1H), 3.96 - 3.78 (m, 2H), 3.65 - 3.53 (m, 5H), 3.53 - 3.39 (m, 5H), 3.20 (t, *J* = 8.8 Hz, 2H), 1.98 (br dd, *J* = 2.1, 12.4 Hz, 2H), 1.63 - 1.39 (m, 2H); LCMS (Method 1): m/z = 533.2 (M + H)$^+$; | 23.7 | 2.69 |

(continued)

| Example | Structure | Compound name | $^1$H NMR, MS | Yield (%) | HPLC r.t. (min), Purity |
|---|---|---|---|---|---|
| 457 | 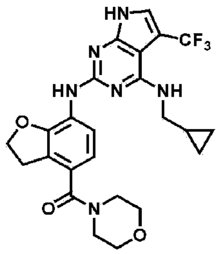 | (7-((4-((cyclopropylmeth yl) amino)-5-(trifluoromethyl)-7*H*-pyrrolo[2,3-*d*]pyrimidin-2-yl)amino)-2,3-dihydrobenzofuran -4-yl) (morpholino)me thanone | $^1$H NMR (400 MHz, DMSO-$d_6$) δ = 11.96 (br s, 1H), 8.06 (d, *J* = 8.3 Hz, 1H), 7.53 (s, 1H), 7.45 (s, 1H), 6.77 (d, *J* = 8.4 Hz, 1H), 5.68 (br s, 1H), 4.60 (t, *J* = 8.8 Hz, 2H), 3.57 (br s, 4H), 3.52 - 3.42 (m, 3H), 3.38 (dd, *J* = 5.7, 6.7 Hz, 2H), 3.30 (s, 2H), 3.18 (t, *J* = 8.8 Hz, 2H), 1.22 - 1.06 (m, 1H), 0.47 - 0.38 (m, 2H), 0.31 - 0.18 (m, 2H); LCMS (Method 1): m/z = 503.2 (M + H)$^+$; | 19.5 | 2.45 |
| 458 | 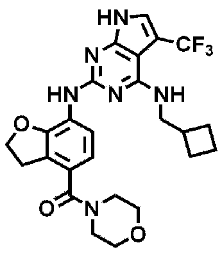 | (7-((4-((cyclobutylmethyl ) amino)-5-(trifluoromethyl)-7*H*-pyrrolo[2,3-*d*]pyrimidin-2-yl)amino)-2,3-dihydrobenzofuran -4-yl) (morpholino)me thanone | $^1$H NMR (400 MHz, DMSO-$d_6$) δ = 11.97 (br d, *J* = 0.9 Hz, 1H), 8.09 (d, *J* = 8.3 Hz, 1H), 7.54 (d, *J* = 1.5 Hz, 1H), 7.46 (s, 1H), 6.78 (d, *J* = 8.3 Hz, 1H), 5.57 (br s, 1H), 4.63 (t, *J* = 8.8 Hz, 2H), 3.70 - 3.42 (m, 10H), 3.21 (t, *J* = 8.8 Hz, 2H), 2.69 - 2.59 (m, 1H), 2.05 - 1.95 (m, 2H), 1.91 - 1.81 (m, 2H), 1.79 - 1.67 (m, 2H); LCMS (Method 1): m/z = 517.2 (M + H)$^+$; | 13.6 | 2.56 |

(continued)

| Example | Structure | Compound name | $^1$H NMR, MS | Yield (%) | HPLC r.t. (min), Purity |
|---|---|---|---|---|---|
| 459 | 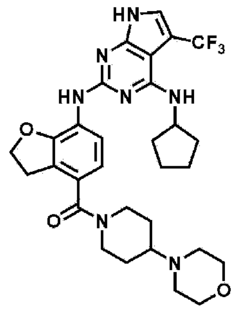 | (7-((4-(cyclopentylamino )-5-(trifluoromethyl)-7*H*-pyrrolo [2,3-*d*]pyrimidin-2-yl)amino)-2,3-dihydrobenzofuran -4-yl)(4-morpholinopiperid in-1-yl) methanone | $^1$H NMR (400 MHz, DMSO-$d_6$): $\delta$ = 12.00 (br s, 1H), 8.05 (d, *J* = 8.3 Hz, 1H), 7.62 - 7.42 (m, 2H), 6.77 (d, *J* = 8.3 Hz, 1H), 5.17 (br dd, *J* = 1.6, 6.9 Hz, 1H), 4.62 (t, *J* = 8.8 Hz, 2H), 4.52 - 4.43 (m, 1H), 3.83 - 3.62 (m, 1H), 3.60 - 3.53 (m, 4H), 3.18 (t, *J* = 8.7 Hz, 2H), 3.09 - 2.74 (m, 2H), 2.50 - 2.36 (m, 6H), 2.10 - 2.00 (m, 2H), 1.90 - 1.76 (m, 2H), 1.73 - 1.57 (m, 4H), 1.49 (qd, *J* = 6.2, 12.1 Hz, 2H), 1.38 - 1.21 (m, 2H); LCMS (Method 1): m/z = 600.3 (M + H)$^+$; | 21.9 | 2.24 |
| 460 | 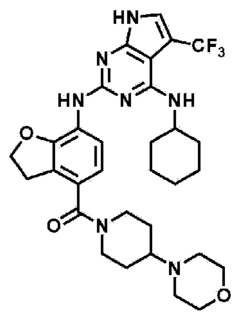 | (7-((4-(cyclohexylamino) -5-(trifluoromethyl)-7*H*-pyrrolo[2,3-*d*]pyrimidin-2-yl)amino)-2,3-dihydrobenzofuran -4-yl)(4-morpholinopiperid in-1-yl) methanone | $^1$H NMR (400 MHz, DMSO-$d_6$): $\delta$ = 11.97 (br d, *J* = 2.0 Hz, 1H), 8.01 (d, *J* = 8.3 Hz, 1H), 7.59 - 7.47 (m, 2H), 6.76 (d, *J* = 8.3 Hz, 1H), 5.17 - 5.04 (m, 1H), 4.62 (t, *J* = 8.7 Hz, 2H), 4.51 - 4.30 (m, 1H), 4.16 - 4.01 (m, 1H), 3.62 - 3.51 (m, 4H), 3.18 (t, *J* = 8.8 Hz, 2H), 3.10 - 2.76 (m, 2H), 2.49 - 2.36 (m, 6H), 2.04 - 1.94 (m, 2H), 1.89 - 1.76 (m, 2H), 1.70 (br dd, *J* = 3.9, 8.6 Hz, 2H), 1.59 (br dd, *J* = 4.6, 9.1 Hz, 1H), 1.47 - 1.20 (m, 7H); LCMS (Method 1): m/z = 614.3 (M + H)$^+$; | 14.0 | 2.30 |

(continued)

| Example | Structure | Compound name | $^1$H NMR, MS | Yield (%) | HPLC r.t. (min), Purity |
|---|---|---|---|---|---|
| 461 | 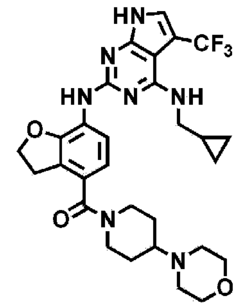 | (7-((4-((cyclopropylmeth yl) amino)-5-(trifluoromethyl)-7*H*-pyrrolo[2,3-*d*]pyrimidin-2-yl)amino)-2,3-dihydrobenzofuran -4-yl)(4-morpholinopiperid in-1-yl) methanone | $^1$H NMR (400 MHz, DMSO-$d_6$): δ = 12.07 (br s, 1H), 8.15 (d, *J* = 8.3 Hz, 1H), 7.72 - 7.52 (m, 2H), 6.87 (d, *J* = 8.3 Hz, 1H), 5.79 (br s, 1H), 4.72 (t, *J* = 8.7 Hz, 2H), 4.61 - 4.38 (m, 1H), 3.70 - 3.63 (m, 4H), 3.55 - 3.48 (m, 2H), 3.28 (t, *J* = 8.7 Hz, 2H), 3.19 - 2.87 (m, 2H), 2.59 - 2.43 (m, 7H), 2.00 - 1.81 (m, 2H), 1.50 - 1.35 (m, 2H), 1.33 - 1.21 (m, 1H), 0.59 - 0.52 (m, 2H), 0.43 - 0.34 (m, 2H); LCMS (Method 1): m/z = 586.3 (M + H)$^+$; | 19.9 | 2.12 |
| 462 | 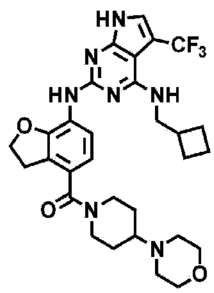 | (7-((4-((cyclobutylmethyl ) amino)-5-(trifluoromethyl)-7*H*-pyrrolo[2,3-*d*]pyrimidin-2-yl)amino)-2,3-dihydrobenzofuran -4-yl)(4-morpholinopiperid in-1-yl) methanone | $^1$H NMR (400 MHz, DMSO-$d_6$): δ = 12.07 (br s, 1H), 8.15 (d, *J* = 8.3 Hz, 1H), 7.69 - 7.48 (m, 2H), 6.87 (d, *J* = 8.3 Hz, 1H), 5.66 (br s, 1H), 4.73 (t, *J* = 8.8 Hz, 2H), 4.64 - 4.35 (m, 1H), 3.73 - 3.62 (m, 6H), 3.29 (t, *J* = 8.8 Hz, 2H), 3.19 - 2.88 (m, 2H), 2.78 - 2.69 (m, 1H), 2.58 - 2.47 (m, 6H), 2.15 - 2.06 (m, 2H), 2.03 - 1.80 (m, 6H), 1.49 - 1.35 (m, 2H); LCMS (Method 1): m/z = 600.3 (M + H)$^+$; | 16.2 | 2.23 |

(continued)

| Example | Structure | Compound name | $^1$H NMR, MS | Yield (%) | HPLC r.t. (min), Purity |
|---|---|---|---|---|---|
| 463 | 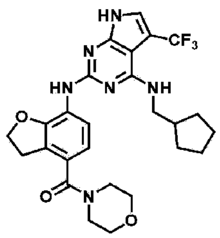 | (7-((4-((cyclopentylmeth yl) amino)-5-(trifluoromethyl)-7*H*-pyrrolo[2,3-*d*]pyrimidin-2-yl)amino)-2,3-dihydrobenzofuran -4-yl) (morpholino)me thanone | $^1$H NMR (400 MHz, DMSO-$d_6$): $\delta$ = 12.07 (br s, 1H), 8.20 (d, *J* = 8.3 Hz, 1H), 7.71 - 7.45 (m, 2H), 6.88 (d, *J* = 8.4 Hz, 1H), 5.71 (br s, 1H), 4.73 (t, *J* = 8.7 Hz, 2H), 3.75 - 3.49 (m, 10H), 3.31 (t, *J* = 8.8 Hz, 2H), 2.40 - 2.29 (m, 1H), 1.85 - 1.75 (m, 2H), 1.74 - 1.66 (m, 2H), 1.65 - 1.56 (m, 2H), 1.43 - 1.32 (m, 2H); LCMS (Method 1): m/z = 531.2 (M + H)$^+$; | 23.8 | 2.64 |
| 464 | 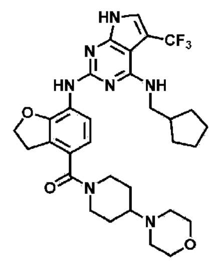 | (7-((4-((cyclopentylmeth yl) amino)-5-(trifluoromethyl)-7*H*-pyrrolo[2,3-*d*]pyrimidin-2-yl)amino)-2,3-dihydrobenzofuran -4-yl)(4-morpholinopiperid in-1-yl) methanone | $^1$H NMR (400 MHz, DMSO-$d_6$): $\delta$ = 12.05 (br s, 1H), 8.16 (d, *J* = 8.3 Hz, 1H), 7.68 - 7.44 (m, 2H), 6.86 (d, *J* = 8.3 Hz, 1H), 5.70 (br s, 1H), 4.72 (t, *J* = 8.8 Hz, 2H), 4.62 - 4.36 (m, 1H), 3.71 - 3.62 (m, 4H), 3.57 (dd, *J* = 5.8, 7.1 Hz, 2H), 3.28 (t, *J* = 8.8 Hz, 2H), 3.16 - 2.85 (m, 2H), 2.59 - 2.44 (m, 6H), 2.40 - 2.29 (m, 1H), 1.96 - 1.85 (m, 2H), 1.83 - 1.76 (m, 2H), 1.74 - 1.58 (m, 4H), 1.49 - 1.30 (m, 4H); LCMS (Method 1): m/z = 614.3 (M + H)$^+$; | 18.6 | 2.28 |

(continued)

| Example | Structure | Compound name | $^1$H NMR, MS | Yield (%) | HPLC r.t. (min), Purity |
|---|---|---|---|---|---|
| 465 | 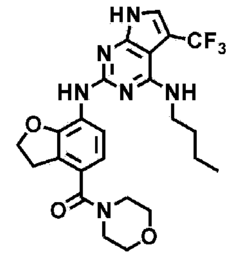 | (7-((4-(butylamino)-5-(trifluoromethyl)-7*H*-pyrrolo[2,3-*d*]pyrimidin-2-yl)amino)-2,3-dihydrobenzofuran-4-yl)(morpholino)methanone | $^1$H NMR (400 MHz, DMSO-$d_6$): δ = 11.95 (br s, 1H), 8.12 (d, *J* = 8.3 Hz, 1H), 7.54 (d, *J* = 1.3 Hz, 1H), 7.40 (s, 1H), 6.78 (d, *J* = 8.4 Hz, 1H), 5.71 - 5.57 (m, 1H), 4.63 (t, *J* = 8.8 Hz, 2H), 3.67 - 3.42 (m, 10H), 3.21 (t, *J* = 8.8 Hz, 2H), 1.60 (quin, *J* = 7.3 Hz, 2H), 1.40 - 1.31 (m, 2H), 0.92 (t, *J* = 7.3 Hz, 3H); LCMS (Method 1): m/z = 505.2 (M + H)$^+$; | 25.4 | 2.52 |
| 466 | 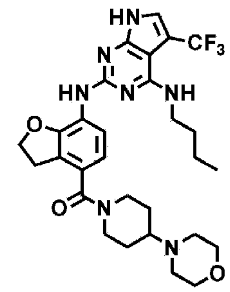 | (7-((4-(butylamino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone | $^1$H NMR (400 MHz, DMSO-$d_6$): δ = 12.04 (br s, 1H), 8.18 (d, *J* = 8.3 Hz, 1H), 7.63 (s, 1H), 7.51 (s, 1H), 6.86 (d, *J* = 8.3 Hz, 1H), 5.79 - 5.68 (m, 1H), 4.73 (t, *J* = 8.7 Hz, 2H), 4.60 - 4.31 (m, 1H), 3.71 - 3.60 (m, 6H), 3.28 (t, *J* = 8.7 Hz, 2H), 3.16 - 2.86 (m, 2H), 2.58 - 2.48 (m, 6H), 1.98 - 1.81 (m, 2H), 1.70 (quin, *J* = 7.3 Hz, 2H), 1.64 - 1.61 (m, 1H), 1.52 - 1.34 (m, 4H), 1.02 (t, *J* = 7.4 Hz, 3H); LCMS (Method 1): m/z = 588.3 (M + H)$^+$; | 22.5 | 2.18 |

(continued)

| Example | Structure | Compound name | $^1$H NMR, MS | Yield (%) | HPLC r.t. (min), Purity |
|---|---|---|---|---|---|
| 467 | 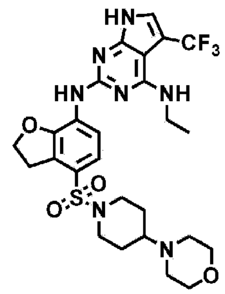 | $N^4$-ethyl-$N^2$-(4-((4-morpholinopiperid in-1-yl) sulfonyl)-2,3-dihydrobenzofuran -7-yl)-5-(trifluoromethyl)-7*H*-pyrrolo [2,3-*d*]pyrimidine-2,4-diamine | $^1$H NMR (400 MHz, DMSO-$d_6$) δ 12.04 (d, *J* = 2.8 Hz, 1H), 8.45 (d, *J* = 8.7 Hz, 1H), 7.58 (d, *J* = 15.2 Hz, 2H), 7.18 (d, *J* = 8.7 Hz, 1H), 5.81 (s, 1H), 4.70 (t, *J* = 8.8 Hz, 2H), 3.98 (s, 1H), 3.74 (s, 2H), 3.58 (p, *J* = 6.9 Hz, 4H), 3.49 (t, *J* = 8.8 Hz, 3H), 3.05 (s, 2H), 2.39 (t, *J* = 11.7 Hz, 3H), 2.14 (d, *J* = 31.3 Hz, 2H), 1.50 (d, *J* = 105.7 Hz, 2H), 1.21 (t, *J* = 7.1 Hz, 3H). LCMS (Method 7): m/z = 596.5[M+1]$^+$ | 89 | 1.51 |
| 468 | 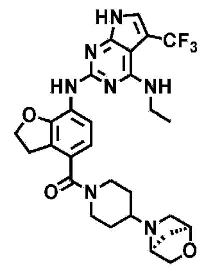 | (4-((1*S*,4*S*)-2-oxa-5-azabicyclo [2.2.1]heptan-5-yl) piperidin-1-yl) (7-((4-(ethylamino)-5-(trifluoromethyl)-7*H*-pyrrolo [2,3-*d*]pyrimidin-2-yl)amino)-2,3-dihydrobenzofuran -4-yl) methanone | $^1$H NMR (400 MHz, DMSO-$d_6$) δ 12.47 (s, 1H), 11.41 (d, *J* = 211.1 Hz, 1H), 8.67 (s, 1H), 8.00 (d, *J* = 8.3 Hz, 1H), 7.66 (s, 1H), 6.80 (dd, *J* = 8.3, 6.7 Hz, 1H), 6.54 (s, 1H), 4.73 - 4.58 (m, 4H), 4.50 (s, 1H), 4.35 (d, *J* = 9.2 Hz, 1H), 4.20 (d, *J* = 10.3 Hz, 1H), 3.39 (d, *J* = 4.0 Hz, 3H), 3.31 - 3.18 (m, 3H), 3.11 (d, *J* = 11.1 Hz, 1H), 2.31 (dd, *J* = 16.6, 5.0 Hz, 1H), 2.17 (t, *J* = 10.8 Hz, 1H), 2.12 - 1.90 (m, 3H), 1.89 - 1.68 (m, 2H), 1.24 - 1.18 (m, 4H). LCMS (Method 7): m/z = 572.4[M+1]$^+$ | 83 | 1.29 |
| 469 | 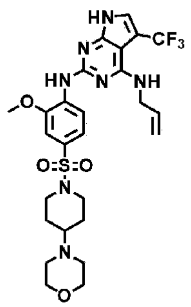 | $N^4$-allyl-$N_2$-(2-methoxy -4-((4-morpholinopiperid in-1-yl) sulfonyl)phenyl )-5-(trifluoromethyl)-7*H*-pyrrolo [2,3-*d*]pyrimidine-2,4-diamine | LCMS (Method 7): m/z = 569.37[M+1]$^+$ | 14 | 1.54 |

(continued)

| Example | Structure | Compound name | $^1$H NMR, MS | Yield (%) | HPLC r.t. (min), Purity |
|---|---|---|---|---|---|
| 470 | 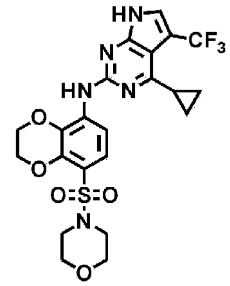 | 4-cyclopropyl-*N*-(8-(morpholinosulfon yl)-2,3-dihydrobenzo [*b*][1,4]dioxin-5-yl)-5-(trifluoromethyl)-7*H*-pyrrolo[2,3-*d*]pyrimidin-2-amine | $^1$H NMR (400 MHz, DMSO-$d_6$) δ 12.43 (s, 1H), 8.14 (d, *J* = 8.9 Hz, 1H), 7.93 (s, 1H), 7.86 (s, 1H), 7.26 (d, *J* = 8.9 Hz, 1H), 4.42 (dd, *J* = 16.9, 4.7 Hz, 4H), 3.64 - 3.60 (m, 4H), 3.04 (t, *J* = 4.8 Hz, 4H), 2.35 (dd, *J* = 8.4, 4.1 Hz, 1H), 2.09 (s, 1H), 1.16 (td, *J* = 7.8, 7.1, 4.4 Hz, 3H). LCMS (Method 7): m/z = 526.3[M+1]$^+$ | 50 | 2.02 |
| 471 | 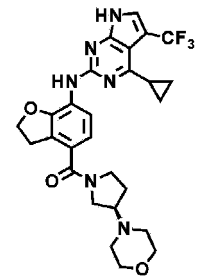 | (*R*)-(7-((4-cyclopropyl-5-(trifluoromethyl)-7*H*-pyrrolo [2,3-*d*]pyrimidin-2-yl)amino)-2,3-dihydrobenzofuran -4-yl)(3-morpholinopyrroli din-1-yl) methanone | $^1$H NMR (400 MHz, Methanol-d4) δ 8.34 (d, *J* = 8.4 Hz, 1H), 7.59 (s, 1H), 6.95 (d, *J* = 8.5 Hz, 1H), 4.70 (p, *J* = 8.4 Hz, 2H), 3.72 (d, *J* = 24.3 Hz, 4H), 3.53 - 3.36 (m, 2H), 2.59 (d, *J* = 16.8 Hz, 2H), 2.51 - 2.37 (m, 2H), 2.18 (s, 2H), 1.32 (d, *J* = 10.5 Hz, 6H), 1.15 (dd, *J* = 8.0, 3.3 Hz, 2H), 0.91 (d, *J* = 7.2 Hz,2H); LCMS (Method 7): m/z = 543.4 [M + H]$^+$ | 52 | 1.54 |
| 472 | 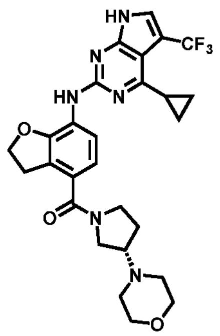 | (S)-(7-((4-cyclopropyl-5-(trifluoromethyl)-7*H*-pyrrolo [2,3-*d*]pyrimidin-2-yl)amino)-2,3-dihydrobenzofuran -4-yl)(3-morpholinopyrroli din-1-yl) methanone | $^1$H NMR (400 MHz, Methanol-d4) δ 8.28 (d, *J* = 8.5 Hz, 1H), 7.94 (s, 1H), 7.04 (t, *J* = 7.9 Hz, 1H), 4.76(H, *J* = 8.6 Hz, 2H), 4.11 (s, 4H), 3.88 (s, 4H), 3.71 - 3.56 (m, 2H), 3.47 (d, *J* = 23.0 Hz, 1H), 3.28 (s,1H), 2.70 - 2.44 (m, 2H), 2.30 (s, 1H), 1.78 - 1.60 (m, 1H), 1.49 (d, *J* = 6.9 Hz, 3H), 1.43 - 1.37 (m, 1H),1.32 (s, 2H);LCMS (Method 7): m/z = 543.4 [M + H]$^+$ | 45 | 1.53 |

(continued)

| Example | Structure | Compound name | $^1$H NMR, MS | Yield (%) | HPLC r.t. (min), Purity |
|---|---|---|---|---|---|
| 473 | 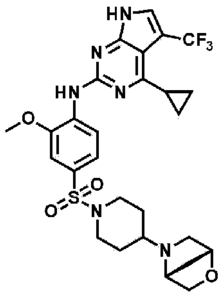 | *N*-(4-((4-((1*R*,4*R*)-2-oxa-5-azabicyclo[2.2.1]h eptan-5-yl) piperidin-1-yl)sulfonyl)-2-methoxyphenyl)-4-cyclopropyl-5-(trifluoromethyl)-7*H*-pyrrolo [2,3-*d*]pyrimidin-2-amine | $^1$H NMR (400 MHz, DMSO-$d_6$) δ 12.49 (d, *J* = 2.8 Hz, 1H), 11.43 (s, 1H), 10.87 (s, 1H), 8.70 (dd, *J* = 8.6, 4.2 Hz, 1H), 8.15 - 7.82 (m, 2H), 7.38 (dd, *J* = 8.6, 1.9 Hz, 1H), 7.26 (d, *J* = 2.0 Hz, 1H), 4.67 - 4.55 (m, 1H), 4.49 (s, 1H), 4.10 (d, *J* = 10.3 Hz, 1H), 4.00 (s, 3H), 3.76 (t, *J* = 13.1 Hz, 2H), 3.36 (q, *J* = 19.1, 14.7 Hz, 2H), 2.41 - 2.29 (m, 2H), 2.22 (dt, *J* = 12.8, 9.6 Hz, 1H), 2.18 - 1.96 (m, 4H), 1.93 - 1.73 (m, 2H), 1.28 (dd, *J* = 7.6, 4.0 Hz, 2H), 1.18 (dq, *J* = 7.2, 3.6 Hz, 2H); LCMS (Method 7): m/z = 593.4 [M + H]$^+$ | 44 | 1.67 |
| 474 | 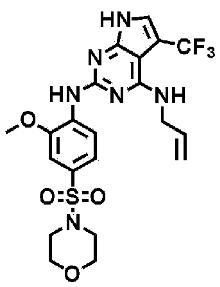 | *N*$^4$-allyl-*N*$^2$-(2-methoxy-4-(morpholinosulfon yl) phenyl)-5-(trifluoromethyl)-7*H*-pyrrolo[2,3-*d*]pyrimidine-2,4-diamine | LCMS (Method 7): m/z = 513.36 [M + H]$^+$ | 30 | 2.01 |

(continued)

| Example | Structure | Compound name | $^1$H NMR, MS | Yield (%) | HPLC r.t. (min), Purity |
|---|---|---|---|---|---|
| 475 | 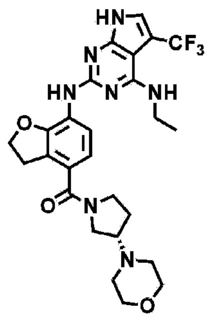 | (*S*)-(7-((4-(ethylamino)-5-(trifluoromethyl)-7*H*-pyrrolo [2,3-*d*]pyrimidin-2-yl)amino)-2,3-dihydrobenzofuran -4-yl) (3-morpholinopyrroli din-1-yl) methanone | $^1$H NMR (400 MHz, Chloroform-d) δ 9.17 (s, 1H), 8.97 (s, 1H), 8.33 (t, *J* = 9.2 Hz, 1H), 7.09 (d, *J* = 7.1Hz, 1H), 7.05 (s, 1H), 6.93 - 6.85 (m, 1H), 5.25 (s, 1H), 4.68 (h, *J* = 7.6 Hz, 2H), 4.02 - 3.88 (m, 1H), 3.82 (t, *J* = 11.1 Hz, 1H), 3.72 (d, *J* = 18.0 Hz, 4H), 3.63 (dd, *J* = 7.3, 5.3 Hz, 3H), 3.49 (m, 2H), 3.37 - 3.23 (m, 1H), 2.83 (d, *J* = 39.2 Hz, 1H), 2.51 (s, 3H), 2.38 (s, 1H), 1.88 - 1.77 (m, 1H), 1.31 (t, *J* = 7.2Hz, 3H); LCMS (Method 7): m/z = 546.4 [M + H]$^+$ | 58 | 1.28 |
| 476 | 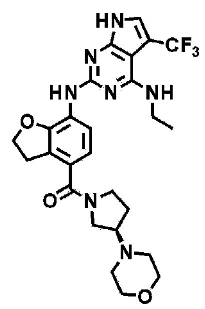 | (*R*)-(7-((4-(ethylamino)-5-(trifluoromethyl)-7*H*-pyrrolo [2,3-*d*]pyrimidin-2-yl)amino)-2,3-dihydrobenzofuran -4-yl) (3-morpholinopyrroli din-1-yl) methanone | $^1$H NMR (400 MHz, Chloroform-d) δ 9.82 (s, 1H), 9.50 (s, 1H), 8.30 (dd, *J* = 14.1, 8.3 Hz, 1H), 7.08 - 6.98 (m, 2H), 6.88 (dd, *J* = 17.6, 8.4 Hz, 1H), 5.25 (s, 1H), 4.68 (h, *J* = 8.7 Hz, 2H), 3.96 - 3.82 (m, 2H), 3.71 (d, *J* = 20.4 Hz, 4H), 3.62 (td, *J* = 7.3, 5.1 Hz, 2H), 3.56 - 3.41 (m, 2H), 3.37 - 3.22 (m, 1H), 2.91 - 2.72 (m, 1H), 2.59 - 2.44 (m, 3H), 2.40 - 2.30 (m, 1H), 2.08 (m, 1H), 1.81 (q, *J* = 10.3 Hz, 1H), 1.31 (t, *J* = 7.2 Hz, 3H); LCMS (Method 7): m/z = 546.5 [M + H]$^+$ | 65 | 1.28 |

(continued)

| Example | Structure | Compound name | $^1H$ NMR, MS | Yield (%) | HPLC r.t. (min), Purity |
|---|---|---|---|---|---|
| 477 | 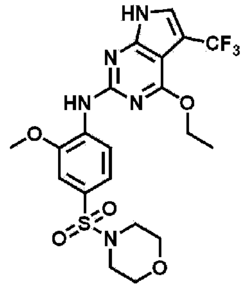 | 4-ethoxy-*N*-(2-methoxy-4-(morpholinosulfon yl) phenyl)-5-(trifluoromethyl)-7*H*-pyrrolo[2,3-*d*]pyrimidin-2-amine | $^1H$ NMR (400 MHz, DMSO-$d_6$) δ 12.38 (s, 1H), 8.77 (d, *J* = 8.6 Hz, 1H), 8.09 (s, 1H), 7.76 (d, *J* = 1.8 Hz, 1H), 7.38 (dd, *J* = 8.5, 1.9 Hz, 1H), 7.24 (d, *J* = 1.9 Hz, 1H), 4.09 (s, 3H), 4.01 (s, 3H), 3.64 (t, *J* = 4.6 Hz, 4H), 2.90 (t, *J* = 4.7 Hz, 4H); LCMS (Method 7): m/z = 488.26 $[M + H]^+$ | 23 | 1.77 |

**<Experimental Example 1> Evaluation 1 of inhibitory activity of compound according to the present invention against enzymes**

To evaluate an inhibitory activity of the compound according to the present invention against LRRK2, LRRK2 (G2019S), DYRK1, CLK1, and TTK kinases, experiments were performed as follows.

1) LRRK2

Each of the Example compounds was reacted with a purified human LRRK2 (Invitrogen #PR8604B) enzyme to evaluate an ability to inhibit an enzyme using a method as described below. A composition of 40 mM Tris-HCl (pH 7.4), 20 mM $MgCl_2$, 0.5 mg/mL BSA, and 50 μM DTT was used as a reaction buffer, and all reactions of test substances were performed in the reaction buffer. Each of the compounds was diluted 12-fold from a 10 mM DMSO stock using a serial dilution method, and enzyme activities were measured at final compound concentrations of 50, 10, 2, 0.4, 0.08, 0.016, 0.0032, 0.00064, 0.000128, 0.0000256, 0.00000512, and 0.000001024 μM. In the test, purified ATP (10 μM) and an enzyme substrate (0.2 μg) were reacted with a human LRRK2 (25 ng) enzyme at 25°C for 2 hours, and the enzyme activities were determined using an *in vitro* ADP-Glo™ kinase assay (Promega). An enzyme-activity reaction solution, an ADP-Glo reaction solution, and an enzyme-ability detection solution were reacted at a ratio of 2:2:1 to measure a degree of inhibition of the enzyme activity with luminescence. The degree of inhibition of the enzyme activity according to the treatment concentration of each of the compounds was calculated based on the fluorescence of the enzyme activity of the solvent control which was not treated with the compound. In this case, the concentration of each of the compounds inhibiting 50% of the enzyme activity was determined to be an $IC_{50}$ (nM) value, and calculated using Prism (Version 5.01; GraphPad) software. The results are listed in Table 2 below.

2) LRRK2 G2019S

Each of the Example compounds was reacted with a purified human LRRK2 G2019S (L10-12GG, SignalChem) enzyme to evaluate an ability to inhibit an enzyme using a method as described below. A composition of 40 mM Tris-HCl (pH 7.4), 20 mM $MgCl_2$, 0.5 mg/mL BSA, and 50 μM DTT was used as a reaction buffer, and all reactions of test substances were performed in the reaction buffer. Each of the compounds was diluted 12-fold from a 10 mM DMSO stock using a serial dilution method, and enzyme activities were measured at final compound concentrations of 50, 10, 2, 0.4, 0.08, 0.016, 0.0032, 0.00064, 0.000128, 0.0000256, 0.00000512, and 0.000001024 μM. In the test, purified ATP (25 μM) and an enzyme substrate (0.2 μg) were reacted with a human LRRK2 G2019S (16 ng) enzyme at 25°C for 2 hours, and the enzyme activities were then determined using an *in vitro* ADP-Glo™ kinase assay (Promega). An enzyme-activity reaction solution, an ADP-Glo reaction solution, and an enzyme-ability detection solution were reacted at a ratio of 2:2:1 to measure a degree of inhibition of the enzyme activity with luminescence. The degree of inhibition of the enzyme activity according to the treatment concentration of each of the compounds was calculated based on the fluorescence of the enzyme activity of the solvent control which was not treated with the compound. In this case, the

concentration of each of the compounds inhibiting 50% of the enzyme activity was determined to be an $IC_{50}$ (nM) value, and calculated using Prism (Version 5.01; GraphPad) software. The results are listed in Table 2 below.

3) GST-DYRKla

Each of the Example compounds was reacted with a purified human GST-DYRK1A (full length, Thermo Scientifics) enzyme to evaluate an ability to inhibit an enzyme using a method as described below. A composition of 40 mM Tris-HCl (pH 7.4), 20 mM $MgCl_2$, 0.5 mg/mL BSA, and 50 $\mu$M DTT was used as a reaction buffer, and all reactions of test substances were performed in the reaction buffer. In the test, purified ATP (10 $\mu$M) and a specific substrate solution were reacted with a human GST-DYRK1A (full length, 10 ng) enzyme at 25°C for an hour, and the enzyme activities were then determined using an *in vitro* ADP-Glo™ kinase assay (Promega). An enzyme-activity reaction solution, an ADP-Glo reaction solution, and an enzyme-ability detection solution were reacted at a ratio of 2:2:1 to measure a degree of inhibition of the enzyme activity with luminescence. The degree of inhibition of the enzyme activity according to the treatment concentration of each of the compounds was calculated based on the fluorescence of the enzyme activity of the solvent control which was not treated with the compound. In this case, the concentration of each of the compounds inhibiting 50% of the enzyme activity was determined to be an $IC_{50}$ (nM) value, and the $IC_{50}$ (nM) value of each of the compounds was determined from 3 sets of data, and calculated using Prism (Version 5.01; GraphPad) software.

4) GST-CLK1

Each of the Example compounds was reacted with a purified human GST-CLK1 (129-end, SignalChem) enzyme to evaluate an ability to inhibit an enzyme using a method as described below. A composition of 40 mM Tris-HCl (pH 7.4), 20 mM $MgCl_2$, 0.5 mg/mL BSA, and 50 $\mu$M DTT was used as a reaction buffer, and all reactions of test substances were performed in the reaction buffer. In the test, purified ATP (10 $\mu$M) and a specific substrate solution were reacted with a human GST-CLK1 (129-end, 3 ng) enzyme at 25°C for an hour, and the enzyme activities were then determined using an *in vitro* ADP-Glo™ kinase assay (Promega). An enzyme-activity reaction solution, an ADP-Glo reaction solution, and an enzyme-ability detection solution were reacted at a ratio of 2:2:1 to measure a degree of inhibition of the enzyme activity with luminescence. The degree of inhibition of the enzyme activity according to the treatment concentration of each of the compounds was calculated based on the fluorescence of the enzyme activity of the solvent control which was not treated with the compound. In this case, the concentration of each of the compounds inhibiting 50% of the enzyme activity was determined to be an $IC_{50}$ (nM) value. The $IC_{50}$ (nM) value of each of the compounds was determined from 3 sets of data, and calculated using Prism (Version 5.01; GraphPad) software. The results are listed in Table 2 below.

5) TTK

Each of the Example compounds was reacted with a purified human TTK (SignalChem #T20-10G) enzyme to evaluate an ability to inhibit an enzyme using a method as described below. A composition of 40 mM Tris-HCl (pH 7.4), 20 mM $MgCl_2$, 0.1 mg/mL BSA (a 5X kinase buffer, SignalChem #K03-09), and 50 $\mu$M DTT (SignalChem #D86-09B) was used as a reaction buffer, and all reactions of test substances were performed in the reaction buffer. Each of the compounds was diluted 12-fold from a 10 mM DMSO stock using a serial dilution method, and enzyme activities were measured at final compound concentrations of 1, 0.333333, 0.111111, 0.037037, 0.012346, 0.004115, 0.001372, 0.000457, 0.000152, 0.000051, and 0.000017 $\mu$M. In the test, purified ATP (5 $\mu$M, Promega #V6930) and an MBP enzyme substrate (0.2 $\mu$g, SignalChem M42-51N) were reacted with a human TTK (7.5 ng) enzyme at 25°C for 4 hours, and the enzyme activities were then determined using an *in vitro* ADP-Glo™ kinase assay (Promega #V6930). An enzyme-activity reaction solution, an ADP-Glo reaction solution, and an enzyme-ability detection solution were reacted at a ratio of 2:2:1 to measure a degree of inhibition of the enzyme activity with luminescence. The degree of inhibition of the enzyme activity according to the treatment concentration of each of the compounds was calculated based on the fluorescence of the enzyme activity of the solvent control which was not treated with the compound. In this case, the concentration of each of the compounds inhibiting 50% of the enzyme activity was determined to be an $IC_{50}$ (nM) value, and calculated using Prism (Version 8.2 GraphPad) software.

Meanwhile, $IC_{50}$ values of some of the compounds against the enzymes were measured using the Kinase HotSpot service (Reaction Biology Corporation), and the tests were performed at an ATP concentration of 10 uM under the same conditions. The inhibitory activities of the compounds were measured at a 3-fold concentration gradient starting from a maximum compound concentration of 10 mM, and the values measured using the Kinase HotSpot service (Reaction Biology Corporation) are indicated by an asterisk (*).

All the experimental methods were performed as provided by the Kinase HotSpot Customer Protocol (http://www.reactionbiology.com//Kinase_Assay_Protocol). The results of experiments are listed in Table 2 below.

In Table 2 below, the following designations are used to evaluate the inhibitory ability against the enzyme.

0 - 100 nM = A; 101 - 300 nM = B; and 301 - 1,000 nM = C

[Table 2]

| Example | LRRK2 | LRRK2 (G2019S) | DYRK1 | CLK1 | TTK | Example | LRRK2 | LRRK2 (G2019S) | DYRK1 | CLK1 | TTK |
|---|---|---|---|---|---|---|---|---|---|---|---|
| 1 | | A | C | C | | 240 | | | C | C | |
| 2 | | A | C | C | | 241 | | | C | C | |
| 3 | | A | C | C | | 242 | | | C | C | |
| 4 | | A | C | C | | 243 | | | C | C | |
| 5 | | A | C | C | | 244 | A | | C | C | |
| 6 | | A | C | C | | 245 | | | C | C | |
| 7 | | A | C | C | | 246 | A | A | C | C | |
| 8 | | A | C | C | | 247 | A | A | C | C | |
| 9 | | A | C | C | | 248 | | A | C | C | |
| 10 | | A | C | c | | 249 | A | | C | c | |
| 11 | | A | C | c | | 250 | A | | C | c | |
| 12 | | A | C | C | | 251 | A | A | C | C | |
| 13 | | A | C | C | | 252 | | | B | C | |
| 14 | | A | C | C | | 253 | | | C | C | |
| 15 | | | C | C | | 254 | | | B | B | |
| 16 | | | C | C | | 255 | | | C | C | |
| 17 | | | C | C | | 256 | A | | A | A | |
| 18 | | | C | C | | 257 | A | A | C | C | |
| 19 | A | A | C | C | | 258 | | | C | A | |
| 21 | | A | C | C | | 259 | A | A | B | A | |
| 22 | | A | C | C | | 260 | A | | B | B | |
| 23 | | A | C | C | | 261 | A | | *A | A | A |
| 24 | | A | C | C | | 262 | | | C | C | |
| 25 | | A | C | C | | 263 | | | C | C | |
| 26 | | A | C | C | | 264 | | | B | A | |
| 27 | | A | C | C | | 265 | | | B | B | |
| 28 | | | C | C | | 266 | | | B | A | |
| 29 | | | C | C | | 267 | | | C | C | |
| 30 | | A | C | C | | 268 | | | C | B | |
| 31 | | A | C | C | | 269 | | | C | B | |
| 32 | | A | C | C | | 270 | | | C | c | |
| 33 | | A | C | C | | 271 | | | C | B | |
| 34 | | A | C | C | | 272 | | | C | C | |

(continued)

| Example | LRRK2 | LRRK2 (G2019S) | DYRK1 | CLK1 | TTK | Example | LRRK2 | LRRK2 (G2019S) | DYRK1 | CLK1 | TTK |
|---|---|---|---|---|---|---|---|---|---|---|---|
| 35 | | A | C | B | | 273 | | | C | B | |
| 36 | | A | C | C | | 274 | | | C | C | |
| 37 | | A | C | C | | 275 | | | C | B | |
| 38 | | A | C | C | | 276 | | | C | C | |
| 39 | | A | C | C | | 277 | A | | C | C | |
| 40 | | C | c | c | | 279 | | | C | c | |
| 41 | | A | C | c | | 280 | | | B | A | |
| 42 | | B | C | C | | 281 | | | B | A | |
| 43 | A | A | C | C | | 282 | | | C | B | |
| 44 | | A | C | C | | 283 | | | C | B | |
| 45 | | A | C | C | | 284 | | | C | A | |
| 46 | | A | C | C | | 285 | | | C | B | |
| 47 | | A | C | C | | 286 | | | C | A | |
| 48 | | A | C | C | | 287 | | | C | A | |
| 49 | | A | C | C | | 288 | | A | A | A | |
| 50 | | | C | C | | 289 | | A | C | A | |
| 51 | | | C | C | | 290 | | | C | c | |
| 52 | | A | C | C | | 291 | | | C | B | |
| 53 | | A | C | C | | 292 | | | C | A | |
| 54 | | A | C | C | | 293 | | | B | A | |
| 55 | | A | C | C | | 294 | | | C | A | |
| 56 | | A | C | C | | 295 | | | C | A | |
| 57 | | A | C | C | | 296 | | | C | C | |
| 58 | A | A | C | C | | 297 | | | C | B | |
| 59 | | A | C | C | | 298 | | | C | C | |
| 60 | | B | C | C | | 299 | | | C | B | |
| 61 | | | C | C | A | 300 | | | C | A | |
| 62 | A | A | C | C | | 301 | | | C | A | |
| 63 | A | A | C | C | | 302 | | | C | c | |
| 64 | A | A | C | C | | 303 | | | C | c | |
| 65 | | A | C | C | | 304 | | | C | c | |
| 66 | A | | C | C | | 305 | | | B | A | |
| 67 | | | C | C | A | 306 | | | C | c | |
| 68 | | | C | C | | 307 | | | C | B | |
| 69 | A | A | C | C | | 308 | | | C | c | |
| 70 | A | A | C | C | | 309 | | | C | c | |
| 71 | A | A | C | C | | 310 | | | C | B | |

(continued)

| Example | LRRK2 | LRRK2 (G2019S) | DYRK1 | CLK1 | TTK | Example | LRRK2 | LRRK2 (G2019S) | DYRK1 | CLK1 | TTK |
|---|---|---|---|---|---|---|---|---|---|---|---|
| 72 | C | C | C | C | | 311 | | | c | c | |
| 73 | A | C | C | C | | 312 | | | B | A | |
| 74 | | | C | C | | 313 | | | B | B | A |
| 75 | | | C | C | | 314 | | A | C | C | |
| 76 | | A | C | C | | 315 | | | C | A | |
| 77 | A | A | C | C | | 316 | | | C | A | |
| 78 | A | | C | C | | 317 | | | C | A | |
| 79 | | | C | C | | 318 | | | C | B | |
| 80 | | A | C | c | | 319 | A | | C | A | |
| 81 | A | A | C | A | | 320 | | | A | A | |
| 82 | A | A | C | A | | 321 | B | | C | B | |
| 83 | A | A | C | B | | 322 | A | A | A | A | |
| 84 | | A | C | C | | 323 | | | B | C | |
| 85 | | A | C | B | | 324 | | | C | A | |
| 86 | | | C | C | | 325 | | | B | A | |
| 87 | A | A | B | A | | 326 | | | B | A | |
| 88 | A | A | B | B | | 327 | | | C | A | |
| 89 | A | A | C | B | | 328 | | | C | A | |
| 90 | B | A | C | C | | 329 | | | C | A | |
| 91 | A | A | C | C | | 330 | | | C | A | |
| 92 | A | | C | C | | 331 | | | C | A | |
| 93 | A | | C | B | | 332 | | B | C | A | |
| 94 | A | | C | C | | 333 | | | C | A | |
| 95 | A | | C | C | | 334 | | | C | A | |
| 96 | A | A | C | A | | 335 | | | B | A | |
| 97 | A | A | C | C | | 336 | | | C | A | |
| 98 | A | A | C | C | | 337 | | | C | A | |
| 99 | A | B | C | C | | 338 | | | B | A | |
| 100 | | A | C | c | | 339 | | | C | A | |
| 101 | A | A | C | C | | 340 | | | C | A | |
| 102 | | | C | C | | 341 | | | C | A | |
| 103 | A | A | C | C | | 342 | | | C | C | |
| 104 | A | A | C | B | | 343 | | A | C | C | |
| 105 | A | A | C | C | | 344 | | | C | A | |
| 106 | A | A | C | C | | 345 | | A | C | A | |
| 107 | C | C | C | C | | 346 | | | B | A | |
| 108 | | | C | A | | 347 | A | A | A | A | |

(continued)

| Example | LRRK2 | LRRK2 (G2019S) | DYRK1 | CLK1 | TTK | Example | LRRK2 | LRRK2 (G2019S) | DYRK1 | CLK1 | TTK |
|---|---|---|---|---|---|---|---|---|---|---|---|
| 109 | A | | C | C | | 348 | | | C | A | |
| 110 | | A | C | c | | 349 | | | A | A | |
| 111 | A | | C | C | | 350 | | | C | A | |
| 112 | A | | C | C | | 351 | | | C | A | |
| 113 | A | | C | C | | 352 | | | C | A | |
| 114 | | | C | C | | 353 | | A | A | A | |
| 115 | | | C | A | | 354 | | A | C | A | |
| 116 | | | C | A | | 355 | | A | A | A | |
| 117 | | | C | C | | 356 | | A | C | C | |
| 118 | A | A | C | A | | 357 | | A | C | A | |
| 119 | A | A | C | A | | 358 | | | B | B | |
| 120 | A | A | B | A | | 359 | | | A | A | A |
| 121 | | A | C | B | | 360 | | | B | A | |
| 122 | A | A | B | A | | 361 | A | A | C | A | A |
| 123 | A | A | B | A | | 362 | A | A | C | C | |
| 124 | A | A | B | B | | 363 | A | A | B | A | |
| 125 | A | A | C | B | | 364 | A | A | A | A | |
| 126 | A | | C | C | | 365 | | | A | A | |
| 127 | | | C | A | | 366 | A | A | A | A | |
| 128 | | | C | B | | 367 | A | A | B | A | |
| 129 | | | C | C | | 368 | | | B | A | |
| 130 | A | A | C | C | | 369 | | | B | A | |
| 131 | A | | C | A | | 370 | | | C | A | |
| 132 | A | | C | C | | 371 | | | C | B | |
| 133 | A | | C | C | A | 372 | B | A | C | C | |
| 134 | A | A | C | C | | 373 | A | A | C | C | |
| 135 | C | C | C | C | | 374 | A | A | C | C | |
| 136 | A | A | B | A | | 375 | A | A | C | B | |
| 137 | A | A | B | A | | 376 | A | A | C | B | |
| 138 | A | A | B | A | A | 377 | B | B | C | A | |
| 139 | | | C | A | | 378 | A | A | B | A | |
| 140 | A | | C | A | | 379 | | | C | C | |
| 141 | A | A | C | A | | 380 | | | C | c | |
| 142 | | | C | A | | 381 | | A | C | C | |
| 143 | | | C | A | A | 382 | | A | C | C | |
| 144 | | | C | B | A | 383 | | A | C | C | |
| 145 | | | C | C | A | 384 | | A | C | A | |

(continued)

| Example | LRRK2 | LRRK2 (G2019S) | DYRK1 | CLK1 | TTK | Example | LRRK2 | LRRK2 (G2019S) | DYRK1 | CLK1 | TTK |
|---|---|---|---|---|---|---|---|---|---|---|---|
| 146 | | | | | A | 385 | | A | C | C | |
| 147 | | | C | A | A | 386 | | | C | C | |
| 148 | | | C | C | A | 387 | | A | C | C | |
| 149 | | | C | C | A | 388 | | A | A | A | |
| 150 | | | C | C | A | 389 | | A | C | A | |
| 151 | | | C | B | A | 390 | | A | C | A | |
| 152 | | | B | A | A | 391 | | A | C | C | |
| 153 | | A | A | A | | 392 | | C | C | C | |
| 154 | | | C | c | | 393 | | A | C | C | |
| 155 | A | A | A | A | | 394 | A | | *A | C | |
| 156 | A | A | B | A | | 395 | A | | *A | B | |
| 157 | *A | A | A | A | A | 396 | B | | *A | C | |
| 158 | A | A | *A | A | A | 397 | A | A | B | A | A |
| 159 | A | C | C | C | | 398 | | A | B | A | |
| 160 | A | C | C | C | | 399 | A | | A | A | |
| 161 | A | B | *A | *A | B | 400 | | A | A | A | |
| 162 | A | A | C | C | | 401 | A | | C | B | |
| 163 | A | A | B | C | | 402 | | A | A | A | |
| 164 | A | C | C | C | | 403 | A | | A | A | A |
| 165 | A | B | C | C | | 404 | A | | A | A | |
| 166 | A | B | C | C | | 405 | A | A | A | A | |
| 167 | A | A | C | C | | 406 | | B | C | B | |
| 168 | A | A | A | A | | 407 | A | | A | A | |
| 169 | A | | C | C | | 408 | A | | A | A | |
| 170 | A | A | B | A | | 409 | A | | A | A | |
| 171 | A | A | C | C | | 410 | A | | A | A | |
| 172 | C | C | C | C | | 411 | A | | B | A | |
| 173 | A | | C | C | | 412 | A | | A | A | |
| 174 | A | A | C | C | | 413 | | C | C | C | A |
| 175 | C | C | C | C | | 414 | A | A | A | A | A |
| 176 | C | C | C | C | | 415 | | C | C | C | A |
| 177 | C | C | C | C | | 416 | | A | A | A | |
| 178 | A | A | C | C | | 417 | | A | C | A | |
| 179 | A | B | C | C | | 418 | | A | A | A | |
| 180 | | | C | C | | 419 | | A | C | A | |
| 181 | B | B | C | C | | 420 | A | | A | A | |
| 182 | A | A | C | C | | 421 | | A | C | C | |

(continued)

| Example | LRRK2 | LRRK2 (G2019S) | DYRK1 | CLK1 | TTK | Example | LRRK2 | LRRK2 (G2019S) | DYRK1 | CLK1 | TTK |
|---|---|---|---|---|---|---|---|---|---|---|---|
| 183 | A | B | C | C | | 422 | | A | C | A | |
| 184 | A | B | C | B | | 423 | | A | C | C | |
| 185 | A | B | C | B | | 424 | A | | C | C | |
| 186 | B | | C | C | | 425 | | A | C | C | |
| 187 | A | | C | C | | 426 | | A | A | A | |
| 188 | C | | C | c | | 427 | | A | A | A | |
| 189 | A | | B | B | | 428 | | A | C | C | |
| 190 | A | | C | c | | 429 | | A | A | A | |
| 191 | B | C | C | C | | 430 | A | A | C | B | A |
| 192 | | | B | C | | 431 | A | A | C | C | |
| 193 | | | C | C | | 432 | A | B | C | C | |
| 194 | | | C | C | | 433 | | A | C | A | |
| 195 | | | C | C | | 434 | A | A | A | A | A |
| 196 | | | B | C | | 435 | A | | A | A | A |
| 197 | A | B | C | C | | 436 | A | | A | A | |
| 198 | | | C | C | | 437 | | | A | A | |
| 199 | | | C | A | | 438 | A | A | A | A | A |
| 200 | | | C | C | | 439 | A | | A | A | |
| 201 | | | C | A | | 440 | | A | C | C | |
| 202 | A | B | C | C | | 441 | | | B | A | |
| 203 | | | C | B | | 442 | | | A | A | |
| 204 | | | B | C | | 443 | A | A | A | A | A |
| 205 | | | C | C | | 444 | A | | A | A | |
| 206 | | | C | C | | 445 | | | A | A | |
| 207 | | | C | C | | 446 | A | A | A | A | A |
| 208 | | | C | C | | 447 | | A | C | C | |
| 209 | | A | C | A | | 448 | A | A | B | A | |
| 210 | | A | C | C | | 449 | | A | C | A | |
| 211 | A | B | C | C | | 450 | | | A | A | |
| 212 | A | A | A | A | | 451 | | A | C | C | |
| 213 | A | A | A | A | | 452 | A | | B | A | |
| 214 | A | | A | A | | 453 | | A | C | C | |
| 215 | A | | B | B | | 454 | | A | C | C | |
| 216 | | A | B | B | | 455 | | A | A | A | |
| 217 | A | A | A | A | A | 456 | | A | C | A | |
| 218 | | | A | A | | 457 | | A | C | A | |
| 219 | A | A | A | A | A | 458 | | A | C | C | |

(continued)

| Example | LRRK2 | LRRK2 (G2019S) | DYRK1 | CLK1 | TTK | Example | LRRK2 | LRRK2 (G2019S) | DYRK1 | CLK1 | TTK |
|---|---|---|---|---|---|---|---|---|---|---|---|
| 220 | A | | *A | A | | 459 | | A | C | C | |
| 221 | A | | A | A | A | 460 | A | | C | C | |
| 222 | A | | C | C | | 461 | A | A | A | A | |
| 223 | B | | *B | C | B | 462 | | | B | C | |
| 224 | A | | *A | B | A | 463 | | C | C | c | |
| 225 | *A | *A | *A | *A | A | 464 | | A | C | C | |
| 226 | B | B | *A | C | A | 465 | | A | C | C | |
| 227 | *A | *A | *A | *A | B | 466 | | A | C | C | |
| 228 | A | A | *A | A | A | 467 | A | | C | C | |
| 229 | | | *A | B | | 468 | A | A | A | A | A |
| 230 | A | A | A | A | | 469 | *A | A | *A | *A | |
| 231 | | A | B | A | B | 470 | | | *A | C | |
| 232 | | A | C | B | C | 471 | | | *A | A | |
| 233 | | | C | C | | 472 | *A | | *A | *A | A |
| 234 | | | C | C | | 473 | *A | | *A | *A | A |
| 235 | C | C | C | C | | 474 | C | | c | c | |
| 236 | A | A | A | A | | 475 | A | A | A | A | |
| 237 | A | A | A | A | | 476 | A | A | A | A | |
| 238 | A | A | A | A | | 477 | | | | | |
| 239 | | | C | C | | | | | | | |
| Values indicated by * are data measured using Reaction Biology. | | | | | | | | | | | |

As shown in Table 2, it can be seen that the Example compounds of the present invention had an effect of inhibiting the LRRK2, LRRK2 (G2019S), DYRK1, CLK1, and TTK kinases.

This indicates that the Example compounds of the present invention have inhibitory activity against the enzymes as enumerated above. These results suggest that the Example compounds of the present invention are effective for use in the prevention or treatment of diseases associated with the enzymes as enumerated above.

Therefore, the compound represented by Formula 1 of the present invention may be effectively used in the pharmaceutical composition for preventing or treating diseases associated with the LRRK2, LRRK2 (G2019S), DYRK1, CLK1, and TTK kinases.

**<Experimental Example 2> Evaluation of inhibitory activity against proliferation of MDA-MB-231, MDA-MB-468, and SHP-77 cancer cells**

To evaluate the inhibitory activity of the compounds of the present invention against the proliferation of cancer cells, experiments were performed as follows.

To evaluate an inhibitory effect on the proliferation of cancer cells, each of an MDA-MB-231 cell line (Korean Cell Line Bank #30026) and an MDA-MB-468 cell line as triple-negative breast cancer cell lines, and SHP-77 (ATCC #CRL-2195) as a small-cell lung cancer cell line was cultured in DMEM (HyClone #SH30243) or an RPMI medium (HyClone #SH3027.01) to analyze a cell growth rate. More specifically, a cell line was plated on a 96-well flat bottom plate (Corning #3903) at a density of 2,000 cells/100 μL per well, and then treated with 11 concentrations of the Example compound, a solution of which was diluted 3-fold so that a final concentration of the compound was 10.000000, 3.333333, 1.111111, 0.370370, 0.123457, 0.041152, 0.013717, 0.004572, 0.001524, 0.000508, and 0.000169 μM. After 72 hours, each of the culture media was treated with 100 μL of Cell Titer-Glo (Promega G7573), and then cultured at room

temperature for 10 minutes. Then, a degree of luminescence was measured using a microplate reader. A $GI_{50}$ value was calculated from the measured degree of luminescence using Prism (Version 8.2 GraphPad) software.

In Table 3 below, the following designations are used to evaluate cell proliferation inhibitory activity.

$$0 - 50 \text{ nM} = A; 51 - 100 \text{ nM} = B; 101 - 300 \text{ nM} = C; 301 - 1,000 \text{ nM} = D;$$

[Table 3]

| Example | MDA-MB-468 ($GI_{50}$ (nM)) | MDA-MB-231 ($GI_{50}$ (nM)) | SHP-77 ($GI_{50}$ (nM)) | Example | MDA-MB-468 ($GI_{50}$ (nM)) | MDA-MB-231 ($GI_{50}$ (nM)) | SHP-77 ($GI_{50}$ (nM)) |
|---|---|---|---|---|---|---|---|
| 61 | | D | | 211 | | D | D |
| 62 | | B | B | 217 | | D | |
| 63 | | D | D | 219 | | D | D |
| 64 | | A | A | 220 | | C | |
| 65 | | A | A | 221 | | D | |
| 66 | | A | A | 223 | | D | D |
| 67 | | A | A | 224 | | A | |
| 68 | | C | C | 225 | | D | D |
| 69 | | D | D | 226 | | D | D |
| 70 | | D | D | 227 | | D | |
| 71 | | C | B | 228 | | D | |
| 72 | | A | A | 231 | | D | C |
| 73 | C | A | A | 232 | | D | D |
| 74 | | C | C | 241 | | D | |
| 75 | C | B | A | 242 | | D | |
| 77 | | A | A | 243 | | D | |
| 78 | | A | A | 244 | | A | |
| 79 | | B | A | 245 | | B | |
| 80 | | C | B | 250 | | A | A |
| 90 | B | B | C | 261 | | D | |
| 91 | B | B | A | 319 | | A | |
| 95 | C | C | | 320 | | D | |
| 117 | A | A | | 321 | | A | |
| 126 | B | C | | 359 | | D | D |
| 133 | B | C | | 361 | | A | |
| 143 | B | B | | 362 | | A | A |
| 144 | B | A | | 372 | | D | D |
| 145 | B | B | | 373 | | D | D |
| 146 | C | C | | 374 | | D | D |
| 147 | B | C | | 375 | | D | C |
| 148 | C | C | | 377 | | D | D |

(continued)

| Example | MDA-MB-468 ($GI_{50}$ (nM)) | MDA-MB-231 ($GI_{50}$ (nM)) | SHP-77 ($GI_{50}$ (nM)) | Example | MDA-MB-468 ($GI_{50}$ (nM)) | MDA-MB-231 ($GI_{50}$ (nM)) | SHP-77 ($GI_{50}$ (nM)) |
|---|---|---|---|---|---|---|---|
| 149 | B | A | | 382 | | D | C |
| 150 | B | B | | 383 | | D | C |
| 151 | B | C | | 394 | | D | |
| 152 | B | B | | 397 | | D | |
| 157 | | D | D | 400 | | D | |
| 158 | | C | B | 402 | | D | |
| 160 | | D | B | 403 | | D | |
| 161 | | D | D | 405 | | D | |
| 164 | | D | B | 407 | | D | |
| 165 | | A | B | 412 | | D | |
| 170 | | D | C | 414 | | C | C |
| 172 | | D | C | 415 | | C | |
| 175 | | D | C | 430 | | D | D |
| 176 | | C | | 431 | | D | D |
| 183 | | D | D | 432 | | D | D |
| 186 | | D | D | 434 | | D | |
| 187 | | D | D | 438 | | D | |
| 191 | | D | C | 443 | | D | |
| 192 | | D | D | 444 | | D | |
| 194 | | D | D | 446 | | D | |
| 195 | | D | D | 467 | | C | B |
| 197 | | D | D | 468 | | D | |
| 200 | | D | D | 469 | | C | C |
| 202 | | D | D | 472 | | D | |
| 203 | | D | D | 473 | | D | C |
| 207 | | D | D | 82 | | A | A |
| 93 | | A | A | 88 | | B | C |
| 92 | | A | A | 83 | | A | A |
| 84 | | A | A | 263 | | B | A |
| 86 | | A | A | 87 | | A | C |
| 97 | | A | A | 85 | | A | A |
| 89 | | A | B | | | | |

As shown in Table 3, it can be seen that the Example compounds according to the present invention inhibited the proliferation of triple-negative breast cancer cells.

Therefore, the compound represented by Formula 1 of the present invention may be useful in the treatment of triple-negative breast cancer.

**<Experimental Example 3> Evaluation of inhibitory activity against cytokine secretion of human-derived monocytes**

To evaluate an inhibitory effect on cytokine secretion of monocytes, THP-1 cells (ATCC, # TIB-202) as a human-derived monocyte line were cultured in an RPMI-1640 (Hyclone, SH30027.01) medium supplemented with 10% fetal bovine serum (Hyclone, SH30084.03), 1% penicillin streptomycin (Welgene, LS202-02), and 50 μM 2-mercaptoethanol (Gibco, # 21985023). In the test, the cells were seeded in a 48-well plate (SPL, #30048) at a density of 1.5 to 2 x $10^5$ cells/250 μL per well, and cultured at 37°C for 16 hours in a 5% $CO_2$ incubator. Thereafter, the compound was diluted with DMSO so that the final concentration of the compound was 0.5 μM. Then, the cells were treated with the diluted compound before an hour of treatment with lipopolysaccharides (LPS) (Sigma, #L6529). After the treatment with the compound, the cells were treated with LPS so that the final concentration was 500 ng/mL. Then, a cell culture broth was collected after 24 hours of culture, and levels of cytokine IL-6 (R&D Systems, #D6050) and TNF-α (R&D Systems, #DTA00D) comprised in the culture broth were measured using respective ELISA kits. After an experiment was performed according to the manufacturer's guidelines, cell analysis was performed by measuring the optical density at 450 nm using a microplate reader. The results are listed in Table 4 below.

[Table 4]

| Example | THP1/IL6(% inhib) | THP-1/TNF-α (% inhib) | THP-1 GI50 (uM) | Example | THP1/IL6(% inhib) | THP-1/TNF-α (% inhib) | THP-1 GI50 (uM) |
|---|---|---|---|---|---|---|---|
| 157 | 1.6 | 6.7 | | 414 | 47.3 | 40.3 | 4.16 |
| 212 | 12.6 | | | 416 | 45.6 | 8.1 | |
| 213 | 32.4 | 11.5 | | 418 | 35.5 | 8.5 | |
| 217 | 23.4 | | | 420 | 20.2 | 31.6 | |
| 219 | 10.9 | 6.6 | | 430 | 21 | 4.5 | |
| 230 | 34 | 1 | | 434 | 34.1 | 16 | |
| 236 | 22.9 | 13.6 | | 435 | 22.2 | 6.6 | |
| 237 | 41.1 | 18.9 | | 438 | 25.8 | 10.8 | |
| 347 | 14.1 | 13.3 | | 442 | 31.4 | 1.9 | |
| 349 | 32.9 | 36.7 | | 443 | 4.6 | 10.8 | |
| 359 | 32 | 33.2 | 13.61 | 444 | 20 | 14.4 | |
| 361 | 21.7 | 9.3 | | 446 | 17.3 | 22.5 | |
| 366 | 1.1 | 19.2 | | 448 | 26.2 | 17.7 | |
| 397 | 32 | 5.6 | | 450 | 11.4 | 7.5 | |
| 400 | 24.7 | 1 | | 461 | 25.7 | 2.3 | |
| 402 | 18.2 | 1.4 | | 468 | 22.4 | 16.5 | |
| 403 | 30.1 | 40.4 | 40.37 | 472 | 31.5 | | |
| 407 | 20.2 | 5.3 | | 473 | 31 | 11.3 | |
| 408 | 19.5 | 4.3 | | 475 | 42.5 | 4.7 | |
| 410 | 17.8 | | | 476 | 47.4 | 24.5 | |

**<Experimental Example 4> Evaluation of inhibition of *tau* phosphorylation**

An inhibitory effect on tau phosphorylation was determined using the ClariCELL™ Kinase Cell-Based Assay service (commercially available from CarnaBio USA, Inc.). Human embryonic kidney (HEK 293) cells temporarily expressing human DYRK1A and tau were exposed to the compound, and then lysed to release cell proteins. In this case, the released tau was captured on a plate, and a degree of phosphorylation was quantified by ELISA using a tau phosphorylation-specific antibody. The results are listed in Table 5 below.

[Table 5]

| Ex mple | % inhibition at 1 uM | % inhibition at 0.5 uM | % inhibition at 0.25 uM | Example | % inhibition at 1 uM | % inhibition at 0.5 uM | % inhibition at 0.25 uM |
|---|---|---|---|---|---|---|---|
| 157 | 83.9 | 71.0 | 41.2 | 408 | 48.5 | 40.3 | 20.6 |
| 158 | 66.8 | 59.7 | 25.4 | 411 | 70.8 | 48.9 | 28.5 |
| 161 | 50.0 | 27.9 | 0.0 | 416 | 86.8 | 75.9 | 54.2 |
| 217 | 77.9 | 63.7 | 32.0 | 418 | 80.2 | 55.4 | 46.2 |
| 218 | 99.4 | 84.3 | 57.7 | 422 | 33.2 | 19.6 | 7.4 |
| 219 | 84.2 | 58.8 | 42.1 | 430 | 65.3 | 41.6 | 10.8 |
| 224 | 39.9 | 15.6 | 3.6 | 434 | 87.3 | 65.2 | 47.4 |
| 225 | 29.9 | -2.5 | 1.0 | 435 | 73.4 | 55.5 | 22.7 |
| 227 | 32.7 | 22.8 | 7.4 | 438 | 86.8 | 69.7 | 46.3 |
| 228 | 49.0 | 24.7 | 9.3 | 439 | 55.7 | 43.6 | 18.8 |
| 236 | 67.0 | 59.5 | 34.1 | 440 | 36.3 | 27.8 | 9.6 |
| 315 | 16.6 | 8.4 | -1.8 | 441 | 75.5 | 57.4 | 19.6 |
| 316 | 15.8 | 8.3 | 2.3 | 442 | 80.8 | 51.5 | 45.0 |
| 322 | 64.3 | 34.2 | -10.5 | 444 | 65.6 | 45.7 | 21.7 |
| 325 | 1.9 | 13.5 | 6.3 | 446 | 84.9 | 61.1 | 25.7 |
| 326 | -1.8 | 13.6 | -0.6 | 450 | 84.9 | 66.8 | 37.2 |
| 343 | 52.0 | 16.7 | -44.3 | 461 | 72.2 | 46.9 | 18.2 |
| 347 | 51.5 | 42.6 | 24.7 | 464 | 44.2 | 12.4 | 11.5 |
| 349 | 53.4 | 37.0 | 21.0 | 466 | 71.3 | 39.2 | 9.5 |
| 357 | 35.4 | 33.1 | 19.7 | 468 | 92.5 | 75.4 | 50.3 |
| 359 | 55.9 | 42.8 | 23.4 | 470 | 29.1 | 19.2 | 5.8 |
| 397 | 52.8 | 41.1 | 12.9 | 471 | 42.8 | 29.6 | 3.9 |
| 400 | 69.4 | 46.5 | 22.3 | 472 | 56.5 | 36.5 | 23.6 |
| 402 | 61.7 | 26.5 | 7.9 | 473 | 73.3 | 35.8 | 16.3 |
| 405 | 70.7 | 44.0 | 17.5 | 475 | 86.8 | 65.3 | 42.6 |
| 407 | 58.6 | 39.4 | 20.7 | | | | |

**<Experimental Example 5> Evaluation of inhibition of phosphorylation of LRRK2 (leucine-rich repeat kinase-2)**

To evaluate an inhibitory effect of the compound represented by Formula 1 of the present invention on phosphorylation of LRRK2, an experiment was performed as follows. The results are shown in FIGS. 1, 2, and 3.

Specifically, an NIH3T3 cell line, which is a fibroblast, was treated with the compound, and it was confirmed using a Western blot method that LRRK2 phosphorylation was inhibited in the cells. An NIH3T3 cell line was seeded in a 60 mm dish at a density of 6 X $10^5$ cells/$\mu$L, and attached for a day. Thereafter, the compound was added so that the final concentration of the compound was 100 nM and a content of DMSO in a culture broth was 0.1%, and the cells were than cultured at 37°C for 24 hours in a $CO_2$ incubator. The culture broth was removed, and the cells were washed twice with PBS. Then, the cells were lysed with a 1X RIPA buffer supplemented with a phosphatase inhibitor and protease inhibitor, and collected. The collected cells were centrifuged at 4°C and 14,000 rpm for 15 minutes, and the supernatant was subjected to a Bradford assay to quantify a protein, which was then sampled with a 5X sample buffer. An equivalent amount of the protein was electrophoresed on SDS PAGE gel, and then transferred to a nitrocellulose membrane. The

membrane was blocked with 5% skim milk for an hour, and primary antibodies anti-LRRK2 (ab133474) and anti-LRRK2 (phospho S935, (ab133450)), and actin were added thereto, and reacted for 16 hours in a refrigerator. The membrane was washed with a 1X TBS-T buffer (0.05% Tween20), and a secondary antibody was then added, and attached to the membrane for an hour. Then, the membrane was washed, and reacted with an ECL substrate, and the protein was then detected using LAS500.

As shown in FIGS. 1 to 3, it can be seen that the Example compounds according to the present invention significantly inhibited the phosphorylation of LRRK2 in the fibroblasts (i. e., an NIH3T3 cell line). Also, it can be seen that an amount of the detected P-LRRK2 was significantly low as compared to when the cells were not treated with the compound according to the present invention. This indicates that the compound according to the present invention effectively inhibits the phosphorylation of LRRK2.

Therefore, because the compound represented by Formula 1 according to the present invention effectively inhibits LRRK2 phosphorylation in cancer-inducing cells, the compound of Formula 1 according to the present invention may be effectively used in the pharmaceutical composition for treating or preventing an LRRK2-related disease.

**<Experimental Example 6> Evaluation of inhibitory activity of compounds according to the present invention against various kinases**

To evaluate the inhibitory activity of the compound according to the present invention against more enzymes, an experiment was performed as follows.

Specifically, the enzyme (kinase) selectivity of Example compounds 238, 361, 411, 157, 161, 228, and 158 selected from the Example compounds of the present invention was determined by commissioning DiscoverX Corp., and an experiment was performed using a ScanMAX™ Kinase analysis panel. In this case, a concentration of the drug with which the enzymes were treated was 1 μM in DMSO, and the percent control (% control) was defined in the same manner as in the following Expression 1. The results are listed in Table 4 below.

[Expression 1]

(Example Compound - Positive Control)/(Negative Control - Positive Control) X 100
wherein the positive control represents a compound having a percent control of 0%, and the negative control represents DMSO having a percent control of 100%. For the enzyme selectivity of the present invention, it was considered that the compound has an inhibitory activity on each of the enzymes when the percent control for the corresponding enzyme was < 35% (i. e., less than 35%).

[Table 6]

| Kinase | Example 238 | Example 361 | Example 411 | Example 157 | Example 161 | Example 228 | Example 158 |
|---|---|---|---|---|---|---|---|
| CLK1 | 16 | 2.4 | 7.2 | 6.7 | 5.5 | 6.1 | 1 |
| CLK2 | 0.65 | 3.9 | 0 | 2.9 | 3.9 | 5.5 | 22 |
| CLK3 | 7.8 | 21 | 21 | 3.7 | 11 | 25 | 11 |
| CLK4 | 3.6 | 2.8 | 1.4 | 0.55 | 0.65 | 0.05 | 0.3 |
| CSNK1D | 16 | 3.9 | 14 | 2.9 | 15 | 45 | 5.3 |
| CSNK1E | 32 | 0.05 | 31 | 2.4 | 12 | 40 | 3.3 |
| DYRK1A | 0.25 | 2.6 | 0.2 | 0.05 | 2 | 0.5 | 0.75 |
| DYRK1B | 0 | 6.1 | 4.5 | 0 | 0 | 0 | 0.8 |
| DYRK2 | 19 | 10 | 28 | 7 | 33 | 16 | 25 |
| FAK | 7.8 | 0.75 | 10 | 0.85 | 2.3 | 24 | 1.1 |
| GAK | 3.1 | 2.7 | 2.4 | 4.2 | 4 | 3 | 21 |
| LRRK2 | 0.45 | 0 | 1.9 | 0.7 | 1 | 0.3 | 0 |
| LRRK2 (G2019S) | 0.05 | 0 | 1.3 | 1.6 | 0 | 1.1 | 3.6 |
| PHKG1 | 3.6 | 9.7 | 17 | 7.8 | 24 | 30 | 25 |

(continued)

| Kinase | Example 238 | Example 361 | Example 411 | Example 157 | Example 161 | Example 228 | Example 158 |
|---|---|---|---|---|---|---|---|
| PHKG2 | 2.2 | 3 | 2.5 | 5.8 | 21 | 5.5 | 51 |
| PLK4 | 7.8 | 0.3 | 23 | 10 | 11 | 3.2 | 26 |
| PYK2 | 5.2 | 2.2 | 13 | 5.5 | 1.3 | 22 | 3 |
| TTK | 2.9 | 2.4 | 2.5 | 4 | 4.2 | 2.9 | 2.6 |

As shown in Table 6, it can be seen that the Example compounds of the present invention have inhibitory activity on the CLK1, CLK2, CLK3, CLK4, CSNK1D, CSNK1E, DYRK1A, DYRK1B, DYRK2, FAK, GAK, LRRK2, LRRK2 (G2019S), PHKG1, PHKG2, PLK4, PYK2, and TTK kinases because the Example compounds of the present invention have a percent control of less than 35% with respect to the corresponding enzymes.

Therefore, the compound represented by Formula 1 according to the present invention may be effectively used to treat diseases associated with the protein kinases.

## Claims

1. A compound represented by the following Formula 1, or an isomer thereof, a solvate thereof, a hydrate thereof, or a pharmaceutically acceptable salt thereof:

[Formula 1]

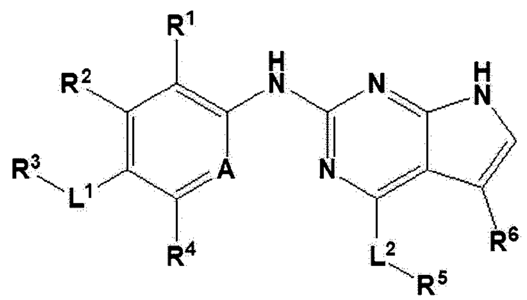

(wherein,

A represents a carbon atom or a nitrogen atom,
$R^1$ is hydrogen, or a linear or branched $C_{1-6}$ alkoxy, and $R^2$ is hydrogen, or
$R^1$ and $R^2$, together with a benzene ring containing carbon atoms to which they are bonded, form an 8- to 10-membered bicyclic ring comprising one or more heteroatoms selected from the group consisting of N, O, and S,
$L^1$ is sulfonyl or carbonyl, or is absent,

when $L^1$ is sulfonyl or carbonyl, $R^3$ is a linear or branched $C_{1-6}$ alkyl, morpholinyl, piperidinyl, piperazinyl, pyrrolidinyl, amino, 2-oxa-5-azabicyclo[2.2.1]heptanyl, or azetidinyl, wherein $R^3$ is unsubstituted or substituted with one or more non-hydrogen substituents selected from the group consisting of morpholinyl, oxetanyl, 2-oxa-5-azabicyclo[2.2.1]heptanyl, piperazinyl, piperidinyl, a $C_{3-9}$ cycloalkyl, and a linear or branched $C_{1-6}$ alkyl, wherein the non-hydrogen substituents of $R^3$ are unsubstituted or substituted with a substituent selected from the group consisting of hydroxy, a $C_{3-9}$ cycloalkyl, and a linear or branched $C_{1-6}$ alkyl,
when $L^1$ is absent, $R^3$ is azaphosphinane oxide or phosphoryl, wherein $R^3$ is unsubstituted or substituted with one or more non-hydrogen substituents selected from the group consisting of oxetanyl, a $C_{3-9}$ cycloalkyl, a linear or branched $C_{1-6}$ alkyl, vinyl, tetrahydropyranyl, and benzyl, wherein the non-hydrogen substituents of $R^3$ are unsubstituted or substituted with a $C_{1-6}$ alkoxy,

$R^4$ is hydrogen or a halogen,
$L^2$ is -NH- or -O-, or is absent,

when $L^2$ is -NH- or -O-, $R^5$ is selected from the group consisting of a linear or branched $C_{1-6}$ alkyl, a $C_{3-9}$ cycloalkyl, a 3- to 9-membered heterocycloalkyl containing one or more O (oxygen) atoms as heteroatoms, and allyl, wherein $R^5$ is unsubstituted or substituted with one or more non-hydrogen substituents selected from the group consisting of a $C_{3-9}$ cycloalkyl, a $C_{1-6}$ alkylsulfonyl, a $C_{1-6}$ alkoxy, and a $C_{1-6}$ alkyl,
when $L^2$ is absent, $R^5$ is a linear or branched $C_{1-6}$ alkyl or a $C_{3-9}$ cycloalkyl, and

$R^6$ is hydrogen, cyano, or a $C_{1-6}$ haloalkyl.

**2.** The compound of Formula 1, or the isomer thereof, the solvate thereof, the hydrate thereof, or the pharmaceutically acceptable salt thereof of claim 1, wherein A represents a carbon atom or a nitrogen atom,

$R^1$ is hydrogen or a linear or branched $C_{1-3}$ alkoxy, and $R^2$ is hydrogen,
$L^1$ is sulfonyl or carbonyl, or is absent,

when $L^1$ is sulfonyl or carbonyl, $R^3$ is a linear or branched $C_{1-3}$ alkyl, morpholinyl, piperidinyl, piperazinyl, pyrrolidinyl, amino, 2-oxa-5-azabicyclo[2.2.1]heptanyl, or azetidinyl, wherein $R^3$ is unsubstituted or substituted with one or more non-hydrogen substituents selected from the group consisting of morpholinyl, oxetanyl, 2-oxa-5-azabicyclo[2.2.1]heptanyl, piperazinyl, piperidinyl, a $C_{3-6}$ cycloalkyl, and a linear or branched $C_{1-3}$ alkyl, wherein the non-hydrogen substituents of $R^3$ are unsubstituted or substituted with a substituent selected from the group consisting of hydroxy, a $C_{3-6}$ cycloalkyl, and a linear or branched $C_{1-3}$ alkyl,
when $L^1$ is absent, $R^3$ is azaphosphinane oxide or phosphoryl, wherein $R^3$ is unsubstituted or substituted with one or more non-hydrogen substituents selected from the group consisting of oxetanyl, a $C_{3-6}$ cycloalkyl, a linear or branched $C_{1-3}$ alkyl, vinyl, tetrahydropyranyl, and benzyl, wherein the non-hydrogen substituents of $R^3$ are unsubstituted or substituted with a $C_{1-3}$ alkoxy,

$R^4$ is hydrogen, F, Cl, or Br,
$L^2$ is -NH- or -O-, or is absent,

when $L^2$ is -NH- or -O-, $R^5$ is selected from the group consisting of a linear or branched $C_{1-5}$ alkyl, a $C_{3-8}$ cycloalkyl, a 3- to 6-membered heterocycloalkyl containing one or more O (oxygen) atoms as heteroatoms, and allyl, wherein $R^5$ is unsubstituted or substituted with one or more non-hydrogen substituents selected from the group consisting of a $C_{3-6}$ cycloalkyl, a $C_{1-3}$ alkylsulfonyl, a $C_{1-3}$ alkoxy, and a $C_{1-3}$ alkyl,
when $L^2$ is absent, $R^5$ is a linear or branched $C_{1-3}$ alkyl or a $C_{3-6}$ cycloalkyl, and

$R^6$ is hydrogen, cyano, or trifluoromethyl.

**3.** The compound of Formula 1, or the isomer thereof, the solvate thereof, the hydrate thereof, or the pharmaceutically acceptable salt thereof of claim 1, wherein A represents a carbon atom,

$R^1$ and $R^2$, together with a benzene ring containing carbon atoms to which they are bonded, form a 9- to 10-membered bicyclic ring comprising one or more O (oxygen) atoms as heteroatoms,
$L^1$ is sulfonyl or carbonyl,
$R^3$ is a linear or branched $C_{1-3}$ alkyl, or is morpholinyl, piperidinyl, piperazinyl, pyrrolidinyl, amino, 2-oxa-5-azabicyclo[2.2.1]heptanyl, or azetidinyl, wherein $R^3$ is unsubstituted or substituted with one or more non-hydrogen substituents selected from the group consisting of morpholinyl, oxetanyl, 2-oxa-5-azabicyclo[2.2.1]heptanyl, piperazinyl, piperidinyl, a $C_{3-6}$ cycloalkyl, and a linear or branched $C_{1-3}$ alkyl, wherein the non-hydrogen substituents of $R^3$ are unsubstituted or substituted with a substituent selected from the group consisting of hydroxy, a $C_{3-6}$ cycloalkyl, and a linear or branched $C_{1-3}$ alkyl,
$R^4$ is hydrogen, F, Cl, or Br,
$L^2$ is -NH- or -O-, or is absent,

when $L^2$ is -NH- or -O-, $R^5$ is selected from the group consisting of a linear or branched $C_{1-5}$ alkyl, a $C_{3-8}$ cycloalkyl, a 3- to 6-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-6}$ cycloalkyl, a $C_{1-3}$ alkylsulfonyl, a $C_{1-3}$ alkoxy, and a $C_{1-3}$ alkyl,
when $L^2$ is absent, $R^5$ is a linear or branched $C_{1-3}$ alkyl or a $C_{3-6}$ cycloalkyl, and

$R^6$ is cyano or trifluoromethyl.

**4.** The compound of Formula 1, or the isomer thereof, the solvate thereof, the hydrate thereof, or the pharmaceutically acceptable salt thereof of claim 3, wherein $R^1$ and $R^2$, together with a benzene ring containing carbon atoms to which they are bonded, form a 9- to 10-membered bicyclic ring comprising one or more O (oxygen) atoms as heteroatoms, and the 9- to 10-membered bicyclic ring is dihydrobenzodioxin, dihydrobenzofuran, or benzodioxole.

**5.** The compound of Formula 1, or the isomer thereof, the solvate thereof, the hydrate thereof, or the pharmaceutically acceptable salt thereof of claim 1, wherein A represents a carbon atom,

$R^1$ and $R^2$, together with a benzene ring containing carbon atoms to which they are bonded, form a 9- to 10-membered bicyclic ring comprising one or more O (oxygen) atoms as heteroatoms, and the 9- to 10-membered bicyclic ring is dihydrobenzodioxin or dihydrobenzofuran,
$L^1$ is sulfonyl,
$R^3$ is piperidinyl, wherein the piperidinyl is unsubstituted or substituted with morpholinyl,
$R^4$ is hydrogen,
$L^2$ is -NH-, or is absent,
$R^5$ is a linear or branched $C_{1-5}$ alkyl or a $C_{3-8}$ cycloalkyl, and
$R^6$ is trifluoromethyl.

**6.** The compound of Formula 1, or the isomer thereof, the solvate thereof, the hydrate thereof, or the pharmaceutically acceptable salt thereof of claim 1, wherein A represents a carbon atom,

$R^1$ is a linear or branched $C_{1-3}$ alkoxy, and $R^2$ is hydrogen,
$L^1$ is sulfonyl,
$R^3$ is a linear or branched $C_{1-3}$alkyl, morpholinyl, or piperidinyl, wherein $R^3$ is unsubstituted or substituted with morpholinyl or 2-oxa-5-azabicyclo[2.2.1]heptanyl,
$R^4$ is hydrogen,
$L^2$ is -NH- or -O-, or is absent,

when $L^2$ is -NH-, $R^5$ is selected from the group consisting of a linear or branched $C_{1-5}$ alkyl, a $C_{3-8}$ cycloalkyl, a 3- to 6-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-6}$ cycloalkyl, a $C_{1-3}$ alkylsulfonyl, a $C_{1-3}$ alkoxy, and a $C_{1-3}$ alkyl,
when $L^2$ is -O-, $R^5$ is substituted with a linear or branched $C_{1-5}$ alkyl,
when $L^2$ is absent, $R^5$ is a linear or branched $C_{1-3}$ alkyl or a $C_{3-6}$ cycloalkyl, and

$R^6$ is hydrogen, cyano, or trifluoromethyl.

**7.** The compound of Formula 1, or the isomer thereof, the solvate thereof, the hydrate thereof, or the pharmaceutically acceptable salt thereof of claim 1, wherein A represents a carbon atom,

$R^1$ is hydrogen or a linear or branched $C_{1-3}$ alkoxy, and $R^2$ is hydrogen,
$L^1$ is absent,
$R^3$ is selected from azaphosphinane oxide and phosphoryl, wherein $R^3$ is unsubstituted or substituted with one or more non-hydrogen substituents selected from the group consisting of oxetanyl, a $C_{3-6}$ cycloalkyl, a linear or branched $C_{1-3}$ alkyl, vinyl, tetrahydropyranyl, and benzyl, wherein the non-hydrogen substituents of $R^3$ are unsubstituted or substituted with a $C_{1-3}$ alkoxy,
$R^4$ is hydrogen,
$L^2$ is -NH-, or is absent,

when $L^2$ is -NH-, $R^5$ is selected from the group consisting of a linear or branched $C_{1-5}$ alkyl, a $C_{3-8}$ cycloalkyl, a 3- to 6-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-6}$ cycloalkyl, a $C_{1-3}$ alkylsulfonyl, a $C_{1-3}$ alkoxy, and a $C_{1-3}$ alkyl,
when $L^2$ is absent, $R^5$ is a linear or branched $C_{1-3}$ alkyl or a $C_{3-6}$ cycloalkyl, and

$R^6$ is hydrogen, cyano, or trifluoromethyl.

**8.** The compound of Formula 1, or the isomer thereof, the solvate thereof, the hydrate thereof, or the pharmaceutically

acceptable salt thereof of claim 1, wherein $L^1$-$R^3$ is

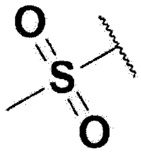, 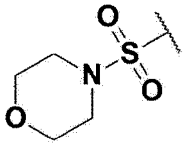, 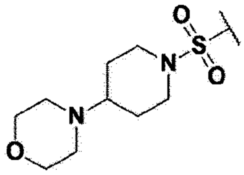, 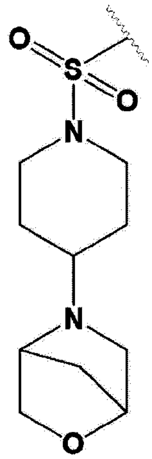, 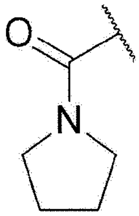, 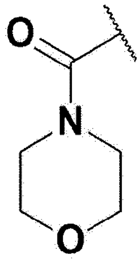,

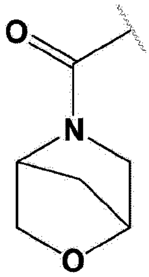, 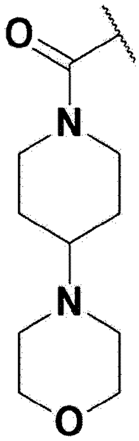, 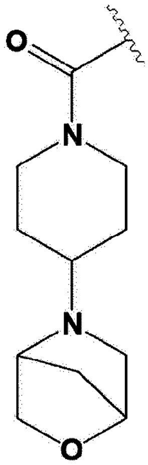, 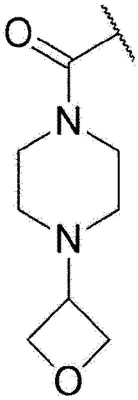, 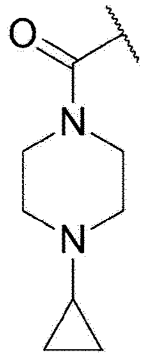, 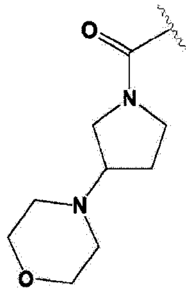,

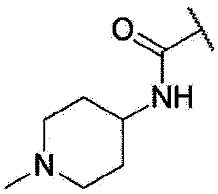, 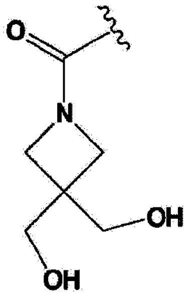, 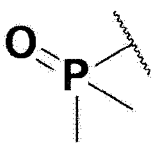, 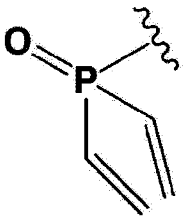, 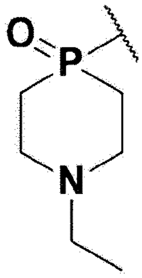, 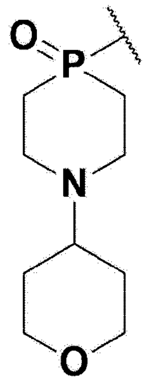,

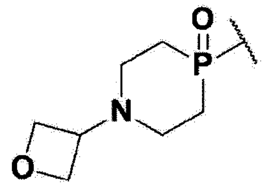, 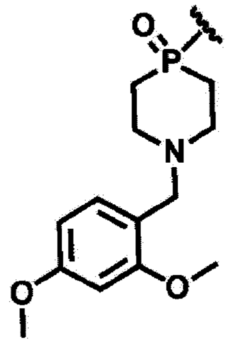, or 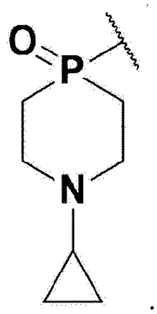.

**9.** The compound of Formula 1, or the isomer thereof, the solvate thereof, the hydrate thereof, or the pharmaceutically acceptable salt thereof of claim 1, wherein the compound represented by Formula 1 comprises any one selected from the group consisting of the following compounds:

(1) $N^4$-cyclopentyl-$N^2$-(2-methoxy-4-(methylsulfonyl)phenyl)-7H-pyrrolo[2,3-d]pyrimidine-2,4-diamine;
(2) $N^2$-(2-methoxy-4-(morpholinosulfonyl)phenyl)-$N^4$-methyl-7H-pyrrolo[2,3-d]pyrimidine-2,4-diamine;
(3) $N^2$-(2-methoxy-4-((4-morpholinopiperidin-1-yl)sulfonyl)phenyl)-$N^4$-methyl-7H-pyrrolo [2,3-d] pyrimidine-2,4-diamine;
(4) $N^2$-(2-methoxy-4-(methylsulfonyl)phenyl)-$N^4$-methyl-7H-pyrrolo[2,3-d]pyrimidine-2,4-diamine;
(5) $N^4$-ethyl-$N^2$-(2-methoxy-4-(morpholinosulfonyl)phenyl)-7H-pyrrolo[2,3-d]pyrimidine-2,4-diamine;
(6) $N^4$-ethyl-$N^2$-(2-methoxy-4-((4-morpholinopiperidin-1-yl)sulfonyl)phenyl)-7H-pyrrolo [2,3-d] pyrimidine-2,4-diamine;
(7) $N^2$-(2-methoxy-4-(morpholinosulfonyl)phenyl)-$N^4$-propyl-7H-pyrrolo[2,3-d]pyrimidine-2,4-diamine;
(8) $N^2$-(2-methoxy-4-((4-morpholinopiperidin-1-yl)sulfonyl)phenyl)-$N^4$-propyl-7H-pyrrolo [2,3 -d] pyrimidine-2,4-diamine;
(9) $N^2$-(2-methoxy-4-(methylsulfonyl)phenyl)-$N^4$-propyl-7H-pyrrolo[2,3-d]pyrimidine-2,4-diamine;
(10) $N^4$-isobutyl-$N^2$-(2-methoxy-4-(morpholinosulfonyl)phenyl)-7H-pyrrolo[2,3-d]pyrimidine-2,4-diamine;
(11) $N^4$-isobutyl-$N^2$-(2-methoxy-4-((4-morpholinopiperidin-1-yl)sulfonyl)phenyl)-7H-pyrrolo[2,3-d]pyrimidine-2,4-diamine;
(12) $N^4$-isobutyl-$N^2$-(2-methoxy-4-(methylsulfonyl)phenyl)-7H-pyrrolo[2,3-d]pyrimidine-2,4-diamine;
(13) $N^4$-isopropyl-$N^2$-(2-methoxy-4-(morpholinosulfonyl)phenyl)-7H-pyrrolo[2,3-d]pyrimidine-2,4-diamine;
(14) $N^4$-isopropyl-$N^2$-(2-methoxy-4-(methylsulfonyl)phenyl)-7H-pyrrolo[2,3-d]pyrimidine-2,4-diamine;
(15) (R)-$N^4$-(sec-butyl)-$N^2$-(2-methoxy-4-((4-morpholinopiperidin-1-yl)sulfonyl)phenyl)-7H-pyrrolo[2,3-d]pyrimidine-2,4-diamine;
(16) (R)-$N^4$-(sec-butyl)-$N^2$-(2-methoxy-4-(4-methylsulfonyl)phenyl)-7H-pyrrolo[2,3-d]pyrimidine-2,4-diamine;
(17) (S)-$N^4$-(sec-butyl)-$N^2$-(2-methoxy-4-((4-morpholinopiperidin-1-yl)sulfonyl)phenyl)-7H-pyrrolo[2,3-d]pyrimidine-2,4-diamine;
(18) (S)-$N^4$-(sec-butyl)-$N^2$-(2-methoxy-4-(4-methylsulfonyl)phenyl)-7H-pyrrolo[2,3-d]pyrimidine-2,4-diamine;
(19) $N^2$-(2-methoxy-4-(morpholinosulfonyl)phenyl)-$N^4$-(2-methoxyethyl)-7H-pyrrolo [2,3 -d] pyrimidine-2,4-diamine;
(20) $N^2$-(2-methoxy-4-((4-morpholinopiperidin-1-yl)sulfonyl)phenyl)-$N^4$-(2-methoxyethyl)-7H-pyrrolo[2,3-d]pyrimidine-2,4-diamine;
(21) $N^2$-(2-methoxy-4-(methylsulfonyl)phenyl)-$N^4$-(2-methoxyethyl)-7H-pyrrolo [2,3 -d] pyrimidine-2,4-diamine;
(22) $N^4$-cyclobutyl-$N^2$-(2-methoxy-4-(morpholinosulfonyl)phenyl)-7H-pyrrolo[2,3-d]pyrimidine-2,4-diamine;
(23) $N^4$-cyclobutyl-$N^2$-(2-methoxy-4-((4-morpholinopiperidin-1-yl)sulfonyl)phenyl)-7H-pyrrolo[2,3-d]pyrimidine-2,4-diamine;
(24) $N^4$-cyclobutyl-$N^2$-(2-methoxy-4-(methylsulfonyl)phenyl)-7H-pyrrolo[2,3-d]pyrimidine-2,4-diamine;
(25) $N^4$-cyclopentyl-$N^2$-(2-methoxy-4-(morpholinosulfonyl)phenyl)-7H-pyrrolo[2,3-d]pyrimidine-2,4-diamine;
(26) $N^4$-cyclohexyl-$N^2$-(2-methoxy-4-(morpholinosulfonyl)phenyl)-7H-pyrrolo[2,3-d]pyrimidine-2,4-diamine;
(27) $N^4$-cyclohexyl-$N^2$-(2-methoxy-4-(methylsulfonyl)phenyl)-7H-pyrrolo[2,3-d]pyrimidine-2,4-diamine;
(28) (R)-$N^2$-(2-methoxy-4-(methylsulfonyl)phenyl)-$N^4$-(tetrahydrofuran-3-yl)-7H-pyrrolo[2,3-d]pyrimidine-2,4-diamine;
(29) (S)-$N^2$-(2-methoxy-4-((4-morpholinopiperidin-1-yl)sulfonyl)phenyl)-$N^4$-(tetrahydrofuran-3-yl)-7H-pyrrolo[2,3-d]pyrimidine-2,4-diamine;
(30) $N^2$-(2-methoxy-4-(methylsulfonyl)phenyl)-$N^4$-(tetrahydro-2H-pyran-4-yl)-7H-pyrrolo [2,3 -d] pyrimidine-2,4-

diamine;
(31) $N^4$-(cyclopropylmethyl)-$N^2$-(2-methoxy-4-(morpholinosulfonyl)phenyl)-7H-pyrrolo [2,3-d]pyrimidine-2,4-diamine;
(32) $N^4$-(cyclopropylmethyl)-$N^2$-(2-methoxy-4-((4-morpholinopiperidin-1-yl)sulfonyl)phenyl)-7H-pyrrolo[2,3-d]pyrimidine-2,4-diamine;
(33) $N^4$-(cyclopropylmethyl)-$N^2$-(2-methoxy-4-(methylsulfonyl)phenyl)-7H-pyrrolo [2,3-d]pyrimidine-2,4-diamine;
(34) $N^4$-(1-methoxy-2-methylpropan-2-yl)-$N^2$-(2-methoxy-4-(morpholinosulfonyl)phenyl)-7H-pyrrolo[2,3-d]pyrimidine-2,4-diamine;
(35) $N^4$-(1-methoxy-2-methylpropan-2-yl)-$N^2$-(2-methoxy-4-((morpholinopiperidin-1-yl)sulfonyl)phenyl)-7H-pyrrolo[2,3-d]pyrimidine-2,4-diamine;
(36) $N^4$-(1-methoxy-2-methylpropan-2-yl)-$N^2$-(2-methoxy-4-(methylsulfonyl)phenyl)-7H-pyrrolo[2,3-d]pyrimidine-2,4-diamine;
(37) $N^4$-(cyclobutylmethyl)-$N^2$-(2-methoxy-4-(morpholinosulfonyl)phenyl)-7H-pyrrolo [2,3-d]pyrimidine-2,4-diamine;
(38) $N^4$-(cyclobutylmethyl)-$N^2$-(2-methoxy-4-((4-morpholinopiperidin-1-yl)sulfonyl)phenyl)-7H-pyrrolo[2,3-d]pyrimidine-2,4-diamine;
(39) $N^4$-(cyclobutylmethyl)-$N^2$-(2-methoxy-4-(methylsulfonyl)phenyl)-7H-pyrrolo [2,3-d]pyrimidine-2,4-diamine;
(40) $N^4$-(cyclopentylmethyl)-$N^2$-(2-methoxy-4-(morpholinosulfonyl)phenyl)-7H-pyrrolo [2,3-d]pyrimidine-2,4-diamine;
(41) $N^4$-(cyclopentylmethyl)-$N^2$-(2-methoxy-4-((4-morpholinopiperidin-1-yl)sulfonyl)phenyl)-7H-pyrrolo[2,3-d]pyrimidine-2,4-diamine;
(42) $N^4$-(cyclopentylmethyl)-$N^2$-(2-methoxy-4-(methylsulfonyl)phenyl)-7H-pyrrolo [2,3-d]pyrimidine-2,4-diamine;
(43) (8-((4-(methylamino)-7H-pyrrolo[2,3-d]pyrimidin-2-yl)amino)-2,3-dihydrobenzo[b][1,4]dioxin-5-yl)(morpholino)methanone;
(44) (8-((4-(isobutylamino)-7H-pyrrolo[2,3-d]pyrimidin-2-yl)amino)-2,3-dihydrobenzo[b][1,4]dioxin-5-yl)(morpholino)methanone;
(45) (8-((4-(isopropylamino)-7H-pyrrolo[2,3-d]pyrimidin-2-yl)amino)-2,3-dihydrobenzo[b][1,4]dioxin-5-yl)(morpholino)methanone;
(46) (8-((4-((2-methoxyethyl)amino)-7H-pyrrolo[2,3-d]pyrimidin-2-yl)amino)-2,3-dihydrobenzo[b][1,4]dioxin-5-yl)(morpholino)methanone;
(47) (8-((4-(cyclobutylamino)-7H-pyrrolo[2,3-d]pyrimidin-2-yl)amino)-2,3-dihydrobenzo[b][1,4]dioxin-5-yl)(morpholino)methanone;
(48) (8-((4-(cyclopentylamino)-7H-pyrrolo[2,3-d]pyrimidin-2-yl)amino)-2,3-dihydrobenzo[b][1,4]dioxin-5-yl)(morpholino)methanone;
(49) (8-((4-(cyclohexylamino)-7H-pyrrolo[2,3-d]pyrimidin-2-yl)amino)-2,3-dihydrobenzo[b][1,4]dioxin-5-yl)(morpholino)methanone;
(50) (R)-morpholino(8-((4-((tetrahydrofuran-3-yl)amino)-7H-pyrrolo[2,3-d]pyrimidin-2-yl)amino)-2,3-dihydrobenzo[b][1,4]dioxin-5-yl)methanone;
(51) (S)-morpholino(8-((4-((tetrahydrofuran-3-yl)amino)-7H-pyrrolo[2,3-d]pyrimidin-2-yl)amino)-2,3-dihydrobenzo[b][1,4]dioxin-5-yl)methanone;
(52) morpholino(8-((4-((tetrahydro-2H-pyran-4-yl)amino)-7H-pyrrolo[2,3-d]pyrimidin-2-yl)amino)-2,3-dihydrobenzo[b][1,4]dioxin-5-yl)methanone;
(53) (8-((4-((cyclopropylmethyl)amino)-7H-pyrrolo[2,3-d]pyrimidin-2-yl)amino)-2,3-dihydrobenzo[b][l,4]dioxin-5-yl)(morpholino)methanone;
(54) $N^4$-cyclopropyl-$N^2$-(2-methoxy-4-((4-morpholinopiperidin-1-yl)sulfonyl)phenyl)-7H-pyrrolo[2,3-d]pyrimidine-2,4-diamine;
(55) $N^4$-cyclopropyl-$N^2$-(2-methoxy-4-(methylsulfonyl)phenyl)-7H-pyrrolo[2,3-d]pyrimidine-2,4-diamine;
(56) (8-((4-(cyclopropylamino)-7H-pyrrolo[2,3-d]pyrimidin-2-yl)amino)-2,3-dihydrobenzo[b][1,4]dioxin-5-yl)(morpholino)methanone;
(57) $N^4$-cyclopropyl-$N^2$-(2-methoxy-4-(morpholinosulfonyl)phenyl)-7H-pyrrolo[2,3-d]pyrimidine-2,4-diamine;
(58) (8-((4-(ethylamino)-7H-pyrrolo[2,3-d]pyrimidin-2-yl)amino)-2,3-dihydrobenzo[b][1,4]dioxin-5-yl)(morpholino)methanone;
(59) (R)-$N^4$-(sec-butyl)-$N^2$-(2-methoxy-4-(morpholinosulfonyl)phenyl)-7H-pyrrolo [2,3 -d] pyrimidine-2,4-diamine;
(60) (S)-$N^4$-(sec-butyl)-$N^2$-(2-methoxy-4-(morpholinosulfonyl)phenyl)-7H-pyrrolo [2,3 -d] pyrimidine-2,4-diamine;

(61) 4-(4-((4-(cyclohexylamino)-7H-pyrrolo[2,3-d]pyrimidin-2-yl)amino)-3-methoxyphenyl)-1-(tetrahydro-2H-pyran-4-yl)-1,4-azaphosphinane 4-oxide;
(62) 2-((2-methoxy-4-(morpholinosulfonyl)phenyl)amino)-4-(methylamino)-7H-pyrrolo[2,3-d]pyrimidine-5-carbonitrile;
(63) 4-(ethylamino)-2-((2-methoxy-4-(morpholinosulfonyl)phenyl)amino)-7H-pyrrolo[2,3-d]pyrimidine-5-carbonitrile;
(64) 2-((2-methoxy-4-(morpholinosulfonyl)phenyl)amino)-4-(propylamino)-7H-pyrrolo[2,3-dlpyrimidine-5-carbonitrile;
(65) 4-(isobutylamino)-2-((2-methoxy-4-(morpholinosulfonyl)phenyl)amino)-7H-pyrrolo[2,3-d]pyrimidine-5-carbonitrile;
(66) 4-(isopropylamino)-2-((2-methoxy-4-(morpholinosulfonyl)phenyl)amino)-7H-pyrrolo[2,3-d]pyrimidine-5-carbonitrile;
(67) (R)-4-(sec-butylamino)-2-((2-methoxy-4-(morpholinosulfonyl)phenyl)amino)-7H-pyrrolo[2,3-d]pyrimidine-5-carbonitrile;
(68) (S)-4-(sec-butylamino)-2-((2-methoxy-4-(morpholinosulfonyl)phenyl)amino)-7H-pyrrolo[2,3-d]pyrimidine-5-carbonitrile;
(69) 2-((2-methoxy-4-(morpholinosulfonyl)phenyl)amino)-4-((2-methoxyethyl) amino)-7H-pyrrolo[2,3-d]pyrimidine-5-carbonitrile;
(70) 4-(cyclopropylamino)-2-((2-methoxy-4-(morpholinosulfonyl)phenyl)amino)-7H-pyrrolo[2,3-d]pyrimidine-5-carbonitrile;
(71) 4-(cyclobutylamino)-2-((2-methoxy-4-(morpholinosulfonyl)phenyl)amino)-7H-pyrrolo[2,3-dlpyrimidine-5-carbonitrile;
(72) 4-(cyclopentylamino)-2-((2-methoxy-4-(morpholinosulfonyl)phenyl)amino)-7H-pyrrolo[2,3-d]pyrimidine-5-carbonitrile;
(73) 4-(cyclohexylamino)-2-((2-methoxy-4-(morpholinosulfonyl)phenyl)amino)-7H-pyrrolo[2,3-d]pyrimidine-5-carbonitrile;
(74) (R)-2-((2-methoxy-4-(morpholinosulfonyl)phenyl)amino)-4-((tetrahydrofuran-3-yl)amino)-7H-pyrrolo[2,3-d]pyrimidine-5-carbonitrile;
(75) (S)-2-((2-methoxy-4-(morpholinosulfonyl)phenyl)amino)-4-((tetrahydrofuran-3-yl)amino)-7H-pyrrolo[2,3-d] pyrimidine-5-carbonitrile;
(76) 2-((2-methoxy-4-(morpholinosulfonyl)phenyl)amino)-4-((tetrahydro-2H-pyran-4-yl)amino)-7H-pyrrolo[2,3-d]pyrimidine-5-carbonitrile;
(77) 4-((cyclopropylmethyl)amino)-2-((2-methoxy-4-(morpholinosulfonyl)phenyl)amino)-7H-pyrrolo[2,3-d]pyrimidine-5-carbonitrile;
(78) 4-((1-methoxy-2-methylpropan-2-yl)amino)-2-((2-methoxy-4-(morpholinosulfonyl)phenyl)amino)-7H-pyrrolo[2,3-d]pyrimidine-5-carbonitrile;
(79) 4-((cyclobutylmethyl)amino)-2-((2-methoxy-4-(morpholinosulfonyl)phenyl)amino)-7H-pyrrolo[2,3-d]pyrimidine-5-carbonitrile;
(80) 4-((cyclopentylmethyl)amino)-2-((2-methoxy-4-(morpholinosulfonyl)phenyl)amino)-7H-pyrrolo[2,3-d]pyrimidine-5-carbonitrile;
(81) 2-((2-methoxy-4-((4-morpholinopiperidin-1-yl)sulfonyl)phenyl)amino)-4-(methylamino)-7H-pyrrolo[2,3-d] pyrimidine-5-carbonitrile;
(82) 4-(ethylamino)-2-((2-methoxy-4-((4-morpholinopiperidin-1-yl)sulfonyl)phenyl)amino)-7H-pyrrolo[2,3-d]pyrimidine-5-carbonitrile;
(83) 2-((2-methoxy-4-((4-morpholinopiperidin-1-yl)sulfonyl)phenyl)amino)-4-(propylamino)-7H-pyrrolo[2,3-d] pyrimidine-5-carbonitrile;
(84) 4-(isobutylamino)-2-((2-methoxy-4-((4-morpholinopiperidin-1-yl)sulfonyl)phenyl)amino)-7H-pyrrolo[2,3-d] pyrimidine-5-carbonitrile;
(85) 4-(isopropylamino)-2-((2-methoxy-4-((4-morpholinopiperidin-1-yl)sulfonyl)phenyl)amino)-7H-pyrrolo[2,3-d]pyrimidine-5-carbonitrile;
(86) (R)-4-(sec-butylamino)-2-((2-methoxy-4-((4-morpholinopiperidin-1-yl)sulfonyl)phenyl)amino)-7H-pyrrolo[2,3-d]pyrimidine-5-carbonitrile;
(87) 2-((2-methoxy-4-((4-morpholinopiperidin-1-yl)sulfonyl)phenyl)amino)-4-((2-methoxyethyl)amino)-7H-pyrrolo[2,3-d]pyrimidine-5-carbonitrile;
(88) 4-(cyclopropylamino)-2-((2-methoxy-4-((4-morpholinopiperidin-1-yl)sulfonyl)phenyl)amino)-7H-pyrrolo[2,3-d]pyrimidine-5-carbonitrile;
(89) 4-(cyclobutylamino)-2-((2-methoxy-4-((4-morpholinopiperidin-1-yl)sulfonyl)phenyl)amino)-7H-pyrrolo[2,3-d]pyrimidine-5-carbonitrile;

(90) 4-(cyclopentylamino)-2-((2-methoxy-4-((4-morpholinopiperidin-1-yl)sulfonyl)phenyl)amino)-7H-pyrrolo[2,3-d]pyrimidine-5-carbonitrile;
(91) 4-(cyclohexylamino)-2-((2-methoxy-4-((4-morpholinopiperidin-1-yl)sulfonyl)phenyl)amino)-7H-pyrrolo[2,3-d]pyrimidine-5-carbonitrile;
(92) 4-((cyclopropylmethyl)amino)-2-((2-methoxy-4-((4-morpholinopiperidin-1-yl)sulfonyl)phenyl)amino)-7H-pyrrolo[2,3-d]pyrimidine-5-carbonitrile;
(93) 4-((l-methoxy-2-methylpropan-2-yl)amino)-2-((2-methoxy-4-((4-morpholinopiperidin-1-yl)sulfonyl)phenyl)amino)-7H-pyrrolo[2,3-d]pyrimidine-5-carbonitrile;
(94) 4-((cyclobutylmethyl)amino)-2-((2-methoxy-4-((4-morpholinopiperidin-1-yl)sulfonyl)phenyl)amino)-7H-pyrrolo[2,3-d]pyrimidine-5-carbonitrile;
(95) 4-((cyclopentylmethyl)amino)-2-((2-methoxy-4-((4-morpholinopiperidin-1-yl)sulfonyl)phenyl)amino)-7H-pyrrolo[2,3-d]pyrimidine-5-carbonitrile;
(96) 2-((2-methoxy-4-((4-morpholinopiperidin-1-yl)sulfonyl)phenyl)amino)-4-((2-(methylsulfonyl)ethyl)amino)-7H-pyrrolo[2,3-d]pyrimidine-5-carbonitrile;
(97) 4-(butylamino)-2-((2-methoxy-4-((4-morpholinopiperidin-1-yl)sulfonyl)phenyl)amino)-7H-pyrrolo[2,3-d]pyrimidine-5-carbonitrile;
(98) 4-(ethylamino)-2-((2-methoxy-4-(methylsulfonyl)phenyl)amino)-7H-pyrrolo[2,3-d]pyrimidine-5-carbonitrile;
(99) 2-((2-methoxy-4-(methylsulfonyl)phenyl)amino)-4-(propylamino)-7H-pyrrolo[2,3-d]pyrimidine-5-carbonitrile;
(100) 4-(isobutylamino)-2-((2-methoxy-4-(methylsulfonyl)phenyl)amino)-7H-pyrrolo[2,3-dlpyrimidine-5-carbonitrile;
(101) 4-(isopropylamino)-2-((2-methoxy-4-(methylsulfonyl)phenyl)amino)-7H-pyrrolo[2,3-d]pyrimidine-5-carbonitrile;
(102) (S)-4-(sec-butylamino)-2-((2-methoxy-4-(methylsulfonyl)phenyl)amino)-7H-pyrrolo[2,3-d]pyrimidine-5-carbonitrile;
(103) 2-((2-methoxy-4-(methylsulfonyl)phenyl)amino)-4-((2-methoxyethyl)amino)-7H-pyrrolo[2,3-d]pyrimidine-5-carbonitrile;
(104) 4-(cyclopropylamino)-2-((2-methoxy-4-(methylsulfonyl)phenyl)amino)-7H-pyrrolo[2,3-d]pyrimidine-5-carbonitrile;
(105) 4-(cyclobutylamino)-2-((2-methoxy-4-(methylsulfonyl)phenyl)amino)-7H-pyrrolo[2,3-d]pyrimidine-5-carbonitrile;
(106) 4-(cyclopentylamino)-2-((2-methoxy-4-(methylsulfonyl)phenyl)amino)-7H-pyrrolo[2,3-d]pyrimidine-5-carbonitrile;
(107) 4-(cyclohexylamino)-2-((2-methoxy-4-(methylsulfonyl)phenyl)amino)-7H-pyrrolo[2,3-d]pyrimidine-5-carbonitrile;
(108) (S)-2-((2-methoxy-4-(methylsulfonyl)phenyl)amino)-4-((tetrahydrofuran-3-yl)amino)-7H-pyrrolo[2,3-d]pyrimidine-5-carbonitrile;
(109) 2-((2-methoxy-4-(methylsulfonyl)phenyl)amino)-4-((tetrahydro-2H-pyran-4-yl)amino)-7H-pyrrolo[2,3-d]pyrimidine-5-carbonitrile;
(110) 4-((cyclopropylmethyl)amino)-2-((2-methoxy-4-(methylsulfonyl)phenyl)amino)-7H-pyrrolo[2,3-d]pyrimidine-5-carbonitrile;
(111) 4-((l-methoxy-2-methylpropan-2-yl)amino)-2-((2-methoxy-4-(methylsulfonyl)phenyl)amino)-7H-pyrrolo[2,3-d]pyrimidine-5-carbonitrile;
(112) 4-((cyclobutylmethyl)amino)-2-((2-methoxy-4-(methylsulfonyl)phenyl)amino)-7H-pyrrolo[2,3-d]pyrimidine-5-carbonitrile;
(113) 4-((cyclopentylmethyl)amino)-2-((2-methoxy-4-(methylsulfonyl)phenyl)amino)-7H-pyrrolo[2,3-d]pyrimidine-5-carbonitrile;
(114) 2-((4-(dimethylphosphoryl)-2-methoxyphenyl)amino)-4-(isopropylamino)-7H-pyrrolo[2,3-d]pyrimidine-5-carbonitrile;
(115) 2-((4-(dimethylphosphoryl)-2-methoxyphenyl)amino)-4-((2-methoxyethyl)amino)-7H-pyrrolo[2,3-d]pyrimidine-5-carbonitrile;
(116) 4-(cyclopropylamino)-2-((4-(dimethylphosphoryl)-2-methoxyphenyl)amino)-7H-pyrrolo[2,3-d]pyrimidine-5-carbonitrile;
(117) 4-(cyclohexylamino)-2-((4-(dimethylphosphoryl)-2-methoxyphenyl)amino)-7H-pyrrolo[2,3-d]pyrimidine-5-carbonitrile;
(118) 4-(methylamino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(119) 4-(ethylamino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(120) 2-((8-(morpholine-4-carbonyl)-2,3-dihydrobenzo[b] [1,4]dioxin-5-yl)amino)-4-(propylamino)-7H-pyrrolo[2,3-d]pyrimidine-5-carbonitrile;
(121) 4-(isobutylamino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(122) 4-(isopropylamino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(123) 4-((2-methoxyethyl)amino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(124) 4-(cyclopropylamino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(125) 4-(cyclobutylamino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(126) 4-(cyclopentylamino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(127) (R)-2-((8-(morpholine-4-carbonyl)-2,3-dihydrobenzo[b][l,4]dioxin-5-yl)amino)-4-((tetrahydrofuran-3-yl)amino)-7H-pyrrolo[2,3-d]pyrimidine-5-carbonitrile;
(128) (S)-2-((8-(morpholine-4-carbonyl)-2,3-dihydrobenzo[b][1,4]dioxin-5-yl)amino)-4-((tetrahydrofuran-3-yl)amino)-7H-pyrrolo[2,3-d]pyrimidine-5-carbonitrile;
(129) 2-((8-(morpholine-4-carbonyl)-2,3-dihydrobenzo[b] [1,4]dioxin-5-yl)amino)-4-((tetrahydro-2H-pyran-4-yl)amino)-7H-pyrrolo[2,3-d]pyrimidine-5-carbonitrile;
(130) 4-((cyclopropylmethyl)amino)-2-((8-(morpholine-4-carbonyl)-2,3-dihydrobenzo[b][1,4]dioxin-5-yl)amino)-7H-pyrrolo[2,3-d]pyrimidine-5-carbonitrile;
(131) 4-((1-methoxy-2-methylpropan-2-yl)amino)-2-((8-(morpholine-4-carbonyl)-2,3-dihydrobenzo[b][l,4]dioxin-5-yl)amino)-7H-pyrrolo[2,3-d]pyrimidine-5-carbonitrile;
(132) 4-((cyclobutylmethyl)amino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(133) 4-((cyclopentylmethyl)amino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(134) 4-(butylamino)-2-((8-(morpholine-4-carbonyl)-2,3-dihydrobenzo[b] [1,4]dioxin-5-yl)amino)-7H-pyrrolo[2,3-d]pyrimidine-5-carbonitrile;
(135) 2-((8-(morpholine-4-carbonyl)-2,3-dihydrobenzo[b][1,4]dioxin-5-yl)amino)-4-(oxetan-3-ylamino)-7H-pyrrolo[2,3-d]pyrimidine- 5-carbonitrile;
(136) 4-(methylamino)-2-((8-(4-morpholinopiperidine-l-carbonyl)-2,3-dihydrobenzo[b][1,4]dioxin-5-yl)amino)-7H-pyrrolo[2,3-d]pyrimidine-5-carbonitrile;
(137) 4-(ethylamino)-2-((8-(4-morpholinopiperidine-l-carbonyl)-2,3-dihydrobenzo[b][1,4]dioxin-5-yl)amino)-7H-pyrrolo[2,3-d]pyrimidine-5-carbonitrile;
(138) 2-((8-(4-morpholinopiperidine-1-carbonyl)-2,3-dihydrobenzo[b][1,4]dioxin-5-yl)amino)-4-(propylamino)-7H-pyrrolo[2,3-d]pyrimidine-5-carbonitrile;
(139) 4-(isobutylamino)-2-((8-(4-morpholinopiperidine-1-carbonyl)-2,3-dihydrobenzo[b][1,4]dioxin-5-yl)amino)-7H-pyrrolo[2,3-d]pyrimidine-5-carbonitrile;
(140) 4-(isopropylamino)-2-((8-(4-morpholinopiperidine-1-carbonyl)-2,3-dihydrobenzo[b][1,4]dioxin-5-yl)amino)-7H-pyrrolo[2,3-d]pyrimidine-5-carbonitrile;
(141) 4-(cyclopropylamino)-2-((8-(4-morpholinopiperidine-1-carbonyl)-2,3-dihydrobenzo[b][1,4]dioxin-5-yl)amino)-7H-pyrrolo[2,3-d]pyrimidine-5-carbonitrile;
(142) 4-(cyclohexylamino)-2-((8-(4-morpholinopiperidine-1-carbonyl)-2,3-dihydrobenzo[b][1,4]dioxin-5-yl)amino)-7H-pyrrolo[2,3-d]pyrimidine-5-carbonitrile;
(143) 8-((5-cyano-4-(cyclohexylamino)-7H-pyrrolo[2,3-d]pyrimidin-2-yl)amino)-N-(1-methylpiperidin-4-yl)-2,3-dihydrobenzo[b][1,4]dioxin-5-carboxyamide;
(144) 4-(cyclohexylamino)-2-((8-(4-(oxetan-3-yl)piperazine-1-carbonyl)-2,3-dihydrobenzo[b][1,4]dioxin-5-yl)amino)-7H-pyrrolo[2,3-d]pyrimidine-5-carbonitrile;
(145) 4-(cyclohexylamino)-2-((8-(4-cyclopropylpiperazine-1-carbonyl)-2,3-dihydrobenzo[b] [1,4]dioxin-5-yl)amino)-7H-pyrrolo[2,3-d]pyrimidine-5-carbonitrile;
(146) 8-((5-cyano-4-(cyclopentylamino)-7H-pyrrolo[2,3-d]pyrimidin-2-yl)amino)-N-(1-methylpiperidin-4-yl)-2,3-dihydrobenzo[b][1,4]dioxin-5-carboxyamide;
(147) 4-(cyclopentylamino)-2-((8-(4-(oxetan-3-yl)piperazine-1-carbonyl)-2,3-dihydrobenzo[b][1,4]dioxin-5-yl)amino)-7H-pyrrolo[2,3-d]pyrimidine-5-carbonitrile;
(148) 4-(cyclopentylamino)-2-((8-(4-cyclopropylpiperazine-1-carbonyl)-2,3-dihydrobenzo[b][1,4]dioxin-5-yl)amino)-7H-pyrrolo[2,3-d]pyrimidine-5-carbonitrile;
(149) 4-(cyclohexylamino)-2-((8-(pyrrolidine-1-carbonyl)-2,3-dihydrobenzo[b][1,4]dioxin-5-yl)amino)-7H-pyrrolo[2,3-d]pyrimidine-5-carbonitrile;
(150) 4-(cyclohexylamino)-2-((7-(morpholine-4-carbonyl)benzo[d][1,3]dioxol-4-yl)amino)-7H-pyrrolo[2,3-d]pyrimidine-5-carbonitrile;
(151) 4-(cyclohexylamino)-2-((4-(1-ethyl-4-oxido-1,4-azaphosphinan-4-yl)-2-methoxyphenyl)amino)-7H-pyrrolo[2,3-d]pyrimidine-5-carbonitrile;
(152) 4-(cyclopentylamino)-2-((7-(morpholine-4-carbonyl)benzo[d][1,3]dioxol-4-yl)amino)-7H-pyrrolo[2,3-d]pyrimidine-5-carbonitrile;
(153) 1-(2,4-dimethoxybenzyl)-4-(6-((4-(ethylamino)-5-(trifluoromethyl)-7H-pyrrolo[2,3-d]pyrimidin-2-yl)amino)pyridin-3-yl)-1,4-azaphosphinane 4-oxide;
(154) 2-((2-methoxy-4-(1-(oxetan-3-yl)-4-oxido-1,4-azaphosphinan-4-yl)phenyl)amino)-4-(methylamino)-7H-pyrrolo[2,3-d]pyrimidine-5-carbonitrile;
(155) (8-((4-(methylamino)-5-(trifluoromethyl)-7H-pyrrolo[2,3-d]pyrimidin-2-yl)amino)-2,3-dihydrobenzo[b][1,4]dioxin-5-yl)(morpholino)methanone;
(156) (8-((4-(ethylamino)-5-(trifluoromethyl)-7H-pyrrolo[2,3-d]pyrimidin-2-yl)amino)-2,3-dihydrobenzo[b][1,4]dioxin-5-yl)(morpholino)methanone;
(157) $N^2$-(2-methoxy-4-((4-morpholinopiperidin-1-yl)sulfonyl)phenyl)-$N^4$-methyl-5-(trifluoromethyl)-7H-pyrrolo[2,3-d]pyrimidine-2,4-diamine;
(158) $N^4$-ethyl-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;
(159) $N^4$-ethyl-$N^2$-(2-methoxy-4-(methylsulfonyl)phenyl)-5-(trifluoromethyl)-7H-pyrrolo[2,3-d]pyrimidine-2,4-diamine;
(160) $N^2$-(2-methoxy-4-(morpholinosulfonyl)phenyl)-$N^4$-methyl-5-(trifluoromethyl)-7H-pyrrolo[2,3-d]pyrimidine-2,4-diamine;
(161) $N^2$-(2-methoxy-4-((4-morpholinopiperidin-1-yl)sulfonyl)phenyl)-$N^4$-propyl-5-(trifluoromethyl)-7H-pyrrolo[2,3-d]pyrimidine-2,4-diamine;
(162) morpholino(8-((4-(propylamino)-5-(trifluoromethyl)-7H-pyrrolo[2,3-d]pyrimidin-2-yl)amino)-2,3-dihydrobenzo[b][1,4]dioxin-5-yl)methanone;
(163) $N^2$-(2-methoxy-4-(methylsulfonyl)phenyl)-$N^4$-methyl-5-(trifluoromethyl)-7H-pyrrolo [2,3 -d] pyrimidine-2,4-diamine;
(164) $N^4$-isopropyl-$N^2$-(2-methoxy-4-(morpholinosulfonyl)phenyl)-5-(trifluoromethyl)-7H-pyrrolo[2,3-d]pyrimidine-2,4-diamine;
(165) $N^4$-isopropyl-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;
(166) $N^4$-isopropyl-$N^2$-(2-methoxy-4-(methylsulfonyl)phenyl)-5-(trifluoromethyl)-7H-pyrrolo[2,3-d]pyrimidine-2,4-diamine;
(167) (8-((4-(isopropylamino)-5-(trifluoromethyl)-7H-pyrrolo[2,3-d]pyrimidin-2-yl)amino)-2,3-dihydrobenzo[b][1,4]dioxin-5-yl)(morpholino)methanone;
(168) (8-((4-((2-methoxyethyl)amino)-5-(trifluoromethyl)-7H-pyrrolo[2,3-d]pyrimidin-2-yl)amino)-2,3-dihydrobenzo[b][1,4]dioxin-5-yl)(morpholino)methanone;
(169) (8-((4-(isobutylamino)-5-(trifluoromethyl)-7H-pyrrolo[2,3-d]pyrimidin-2-yl)amino)-2,3-dihydrobenzo[b][1,4]dioxin-5-yl)(morpholino)methanone;
(170) $N^2$-(2-methoxy-4-((4-morpholinopiperidin-1-yl)sulfonyl)phenyl)-$N^4$-(2-methoxyethyl)-5-(trifluoromethyl)-7H-pyrrolo[2,3-d]pyrimidine-2,4-diamine;
(171) $N^2$-(2-methoxy-4-(methylsulfonyl)phenyl)-$N^4$-(2-methoxyethyl)-5-(trifluoromethyl)-7H-pyrrolo[2,3-d]pyrimidine-2,4-diamine;
(172) (R)-$N^4$-(sec-butyl)-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;
(173) (R)-$N^4$-(sec-butyl)-$N^2$-(2-methoxy-4-(methylsulfonyl)phenyl)-5-(trifluoromethyl)-7H-pyrrolo[2,3-d]pyrimidine-2,4-diamine;
(174) (R)-(8-((4-(sec-butylamino)-5-(trifluoromethyl)-7H-pyrrolo[2,3-d]pyrimidin-2-yl)amino)-2,3-dihydrobenzo[b][1,4]dioxin-5-yl)(morpholino)methanone;
(175) $N^4$-(1-methoxy-2-methylpropan-2-yl)-$N^2$-(2-methoxy-4-(morpholinosulfonyl)phenyl)-5-(trifluoromethyl)-7H-pyrrolo[2,3-d]pyrimidine-2,4-diamine;
(176) $N^4$-(1-methoxy-2-methylpropan-2-yl)-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;
(177) $N^4$-(1-methoxy-2-methylpropan-2-yl)-$N^2$-(2-methoxy-4-(methylsulfonyl)phenyl)-5-(trifluoromethyl)-7H-

pyrrolo[2,3-d]pyrimidine-2,4-diamine;

(178) (8-((4-((1-methoxy-2-methylpropan-2-yl)amino)-5-(trifluoromethyl)-7H-pyrrolo[2,3-d]pyrimidin-2-yl)amino)-2,3-dihydrobenzo[b][l,4]dioxin-5-yl)(morpholino)methanone;

(179) $N^4$-isobutyl-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;

(180) $N^4$-isobutyl-$N^2$-(2-methoxy-4-(methylsulfonyl)phenyl)-5-(trifluoromethyl)-7H-pyrrolo[2,3-d]pyrimidine-2,4-diamine;

(181) $N^2$-(2-methoxy-4-(methylsulfonyl)phenyl)-$N^4$-propyl-5-(trifluoromethyl)-7H-pyrrolo[2,3-d]pyrimidine-2,4-diamine;

(182) (8-((4-(cyclopropylamino)-5-(trifluoromethyl)-7H-pyrrolo[2,3-d]pyrimidin-2-yl)amino)-2,3-dihydrobenzo[b][1,4]dioxin-5-yl)(morpholino)methanone;

(183) $N^4$-cyclobutyl-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;

(184) $N^4$-cyclobutyl-$N^2$-(2-methoxy-4-(methylsulfonyl)phenyl)-5-(trifluoromethyl)-7H-pyrrolo[2,3-d]pyrimidine-2,4-diamine;

(185) (8-((4-(cyclobutylamino)-5-(trifluoromethyl)-7H-pyrrolo[2,3-d]pyrimidin-2-yl)amino)-2,3-dihydrobenzo[b][1,4]dioxin-5-yl)(morpholino)methanone;

(186) $N^4$-cyclopentyl-$N^2$-(2-methoxy-4-(morpholinosulfonyl)phenyl)-5-(trifluoromethyl)-7H-pyrrolo[2,3-d]pyrimidine-2,4-diamine;

(187) $N^4$-cyclopentyl-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;

(188) $N^4$-cyclopentyl-$N^2$-(2-methoxy-4-(methylsulfonyl)phenyl)-5-(trifluoromethyl)-7H-pyrrolo[2,3-d]pyrimidine-2,4-diamine;

(189) (8-((4-(cyclopentylamino)-5-(trifluoromethyl)-7H-pyrrolo[2,3-d]pyrimidin-2-yl)amino) -2,3 -dihydrobenzo[b][1,4]dioxin-5-yl) (morpholino)methanone;

(190) (8-((4-(cyclohexylamino)-5-(trifluoromethyl)-7H-pyrrolo[2,3-d]pyrimidin-2-yl)amino)-2,3-dihydrobenzo[b][1,4]dioxin-5-yl)(morpholino)methanone;

(191) $N^4$-ethyl-$N^2$-(2-methoxy-4-(morpholinosulfonyl)phenyl)-5-(trifluoromethyl)-7H-pyrrolo[2,3-d]pyrimidine-2,4-diamine;

(192) $N^4$-isobutyl-$N^2$-(2-methoxy-4-(morpholinosulfonyl)phenyl)-5-(trifluoromethyl)-7H-pyrrolo[2,3-d]pyrimidine-2,4-diamine;

(193) (8-((4-((cyclobutylmethyl)amino)-5-(trifluoromethyl)-7H-pyrrolo[2,3-d]pyrimidin-2-yl)amino)-2,3-dihydrobenzo[b][1,4]dioxin-5-yl)(morpholino)methanone;

194) $N^4$-(cyclopentylmethyl)-$N^2$-(2-methoxy-4-(morpholinosulfonyl)phenyl)-5-(trifluoromethyl)-7H-pyrrolo[2,3-d]pyrimidine-2,4-diamine;

(195) $N^4$-(cyclopentylmethyl)-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;

(196) (4-((4-(cyclohexylamino)-5-(trifluoromethyl)-7H-pyrrolo[2,3-d]pyrimidin-2-yl)amino)-3-methoxyphenyl)dimethylphosphine oxide;

(197) $N^4$-(cyclobutylmethyl)-$N^2$-(2-methoxy-4-((4-morpholinopiperidin-1-yl)sulfonyl)phenyl)-5-(trifluoromethyl)-7H-pyrrolo[2,3-d]pyrimidine-2,4-diamine;

(198) $N^4$-(cyclobutylmethyl)-$N^2$-(2-methoxy-4-(methylsulfonyl)phenyl)-5-(trifluoromethyl)-7H-pyrrolo[2,3-d]pyrimidine-2,4-diamine;

(199) (8-((4-(isobutylamino)-5-(trifluoromethyl)-7H-pyrrolo[2,3-d]pyrimidin-2-yl)amino)-2,3-dihydrobenzo[b][1,4]dioxin-5-yl)(4-morpholinopiperidin-1-yl)methanone;

(200) $N^4$-(cyclobutylmethyl)-$N^2$-(2-methoxy-4-(morpholinosulfonyl)phenyl)-5-(trifluoromethyl)-7H-pyrrolo[2,3-d]pyrimidine-2,4-diamine;

(201) (8-((4-((cyclopropylmethyl)amino)-5-(trifluoromethyl)-7H-pyrrolo[2,3-*d*]pyrimidin-2-yl)amino)-2,3-dihydrobenzo[*b*][1,4]dioxin-5-yl)(morpholino)methanone;

(202) *N*$^4$-butyl-*N*$^2$-(2-methoxy-4-((4-morpholinopiperidin-1-yl)sulfonyl)phenyl)-5-(trifluoromethyl)-*7H*-pyrrolo[2,3-*d*]pyrimidine-2,4-diamine;

(203) $N^4$-(cyclopropylmethyl)-*N*$^2$-(2-methoxy-4-(morpholinosulfonyl)phenyl)-5-(trifluoromethyl)-7*H*-pyrrolo[2,3-*d*]pyrimidine-2,4-diamine;

(204) (8-((4-(cyclohexylamino)-5-(trifluoromethyl)-7H-pyrrolo[2,3-d]pyrimidin-2-yl)amino)-2,3-dihydrobenzo[*b*][1,4]dioxin- 5-yl)(4-morpholinopiperidin- 1-yl)methanone;

(205) (8-((4-((cyclopentylmethyl)amino)-5-(trifluoromethyl)-*7H*-pyrrolo[2,3-*d*]pyrimidin-2-yl)amino)-2,3-dihydrobenzo[*b*][1,4]dioxin-S-yl)(morpholino)methanone;

(206) $N^4$-(cyclopentylmethyl)-*N*$^2$-(2-methoxy-4-(methylsulfonyl)phenyl)-5-(trifluoromethyl)-7*H*-pyrrolo[2,3-*d*]pyrimidine-2,4-diamine;

(207) *N*4-butyl-*N*2-(2-methoxy-4-(morpholinosulfonyl)phenyl)-5-(trifluoromethyl)-7*H*-pyrrolo[2,3-*d*]pyrimidine-2,4-diamine;

(208) (8-((4-(butylamino)-5-(trifluoromethyl)-7H-pyrrolo[2,3-d]pyrimidin-2-yl)amino)-2,3-dihydrobenzo[*b*][1,4]dioxin- 5-yl)(morpholino)methanone;

(209) (S)-morpholino(8-((4-((tetrahydrofuran-3-yl)amino)-5-(trifluoromethyl)-7H-pyrrolo[2,3-*d*]pyrimidin-2-yl)amino)-2,3-dihydrobenzo[*b*][1,4]dioxin-S-yl)methanone;

(210) (*R*)-*N*2-(2-methoxy-4-(methylsulfonyl)phenyl)-*N*4-(tetrahydrofuran-3-yl)-5-(trifluoromethyl)-7*H*-pyrrolo[2,3-*d*]pyrimidine-2,4-diamine;

(211) (S)-*N*4-(sec-butyl)-*N*2-(2-methoxy-4-((4-morpholinopiperidin-1-yl)sulfonyl)phenyl)-5-(trifluoromethyl)-7H-pyrrolo[2,3-d]pyrimidine-2,4-diamine;

(212) (7-((4-(ethylamino)-5-(trifluoromethyl)-7H-pyrrolo[2,3-d]pyrimidin-2-yl)amino)benzo[*d*][1,3]dioxol-4-yl)(morpholino)methanone;

(213) (7-((4-(ethylamino)-5-(trifluoromethyl)-7H-pyrrolo[2,3-d]pyrimidin-2-yl)amino)benzo[*d*][1,3]dioxol-4-yl)(4-morpholinopiperidin-1-yl)methanone;

(214) (7-((4-(methylamino)-5-(trifluoromethyl)-7H-pyrrolo[2,3-d]pyrimidin-2-yl)amino)benzo[*d*][1,3]dioxol-4-yl)(morpholino)methanone;

(215) (7-((4-(methylamino)-5-(trifluoromethyl)-7H-pyrrolo[2,3-d]pyrimidin-2-yl)amino)benzo[*d*][1,3]dioxol-4-yl)(4-morpholinopiperidin-1-yl)methanone;

(216) 1-cyclopropyl-4-(3-methoxy-4-((4-(methylamino)-5-(trifluoromethyl)-7H-pyrrolo[2,3-d]pyrimidin-2-yl)amino)phenyl)-1,4-azaphosphinane 4-oxide;

(217) (7-((4-(methylamino)-5-(trifluoromethyl)-7H-pyrrolo[2,3-d]pyrimidin-2-yl)amino)-2,3-dihydrobenzofuran-4-yl)(4-morpholinopiperidin-1-yl)methanone;

(218) (7-((4-(methylamino)-5-(trifluoromethyl)-7H-pyrrolo[2,3-d]pyrimidin-2-yl)amino)-2,3-dihydrobenzofuran-4-yl)(morpholino)methanone;

(219) *N*4-methyl-*N*2-(8-((4-morpholinopiperidin-1-yl)sulfonyl)-2,3-dihydrobenzo[*b*][1,4]dioxin-S-yl)-S-(trifluoromethyl)-7*H*-pyrrolo[2,3-*d*]pyrimidine-2,4-diamine;

(220) 4-cyclopropyl-2-((8-(morpholine-4-carbonyl)-2,3-dihydrobenzo[*b*][1,4]dioxin-5-yl)amino)-7*H*-pyrrolo[2,3-*d*]pyrimidine-5-carbonitrile;

(221) 4-cyclopropyl-2-((8-(4-morpholinopiperidine-1-carbonyl)-2,3-dihydrobenzo[*b*][1,4]dioxin-5-yl)amino)-7*H*-pyrrolo[2,3-*d*]pyrimidine-5-carbonitrile;

(222) 4-cyclopropyl-2-((2-methoxy-4-(methylsulfonyl)phenyl)amino)-7*H*-pyrrolo[2,3-d]pyrimidine-5-carbonitrile;

(223) 4-cyclopropyl-2-((2-methoxy-4-(morpholinosulfonyl)phenyl)amino)-7H-pyrrolo[2,3-*d*]pyrimidine-5-carbonitrile;

(224) 4-cyclopropyl-2-((2-methoxy-4-((4-morpholinopiperidin-1-yl)sulfonyl)phenyl)amino)-7*H*-pyrrolo[2,3-*d*]pyrimidine-5-carbonitrile;

(225) 4-cyclopropyl-*N*-(2-methoxy-4-(morpholinosulfonyl)phenyl)-5-(trifluoromethyl)-7 H-pyrrolo[2,3-d]pyrimidin-2-amine;

(226) 4-cyclopropyl-N-(2-methoxy-4-((morpholinopiperidin-1-yl)sulfonyl)phenyl)-5-(trifluoromethyl)-7 H-pyrrolo[2,3-d]pyrimidin-2-amine;

(227) (8-((4-cyclopropyl-S-(trifluoromethyl)-7*H*-pyrrolo[2,3-*d*]pyrimidin-2-yl)amino)-2,3 -dihydrobenzo[*b*][1,4]dioxin- 5-yl)(morpholino)methanone;

(228) (8-((4-cyclopropyl-5-(trifluoromethyl)-7*H*-pyrrolo[2,3-*d*]pyrimidin-2-yl)amino)-2,3-dihydrobenzo[*b*][1,4]dioxin- 5-yl)(4-morpholinopiperidin-1-yl)methanone;

(229) (4-((4-cyclopropyl-S-(trifluoromethyl)-7*H*-pyrrolo[2,3-*d*]pyrimidin-2-yl)amino)-3-methoxyphenyl)dimethylphosphine oxide;

(230) (4-((1*S*,4*S*)-2-oxa-5-azabicyclo[2.2.1]heptan-5-yl)piperidin-1-yl)(7-((4-(methylamino)-5-(trifluoromethyl)-7*H*-pyrrolo[2,3-*d*]pyrimidin-2-yl)amino)-2,3-dihydrobenzofuran-4-yl)methanone;

(231) *N*2-(5-fluoro-2-methoxy-4-((4-morpholinopiperidin-1-yl)sulfonyl)phenyl)-*N*4-methyl-5-(trifluoromethyl)-7H-pyrrolo[2,3-d]pyrimidine-2,4-diamine;

(232) *N*4-ethyl-*N*2-(S-fluoro-2-methoxy-4-((4-morpholinopiperidin-1-yl)sulfonyl)phenyl)-5-(trifluoromethyl)-7H-pyrrolo[2,3-d]pyrimidine-2,4-diamine;

(233) 2-((2-methoxy-4-((4-morpholinopiperidin-1-yl)sulfonyl)phenyl)amino)-4-((2-methoxyethyl)amino)-7*H*-pyrrolo[2,3-*d*]pyrimidine-5-carboxyamide;

(234) 2-((2-methoxy-4-(methylsulfonyl)phenyl)amino)-4-((2-methoxy)amino)-7H-pyrrolo[2,3-d]pyrimidine-5-carboxyamide;

(235) 4-((2-methoxyethyl)amino)-2-((8-(morpholine-4-carbonyl)-2,3-dihydrobenzo[b][1,4]dioxin-5-yl)amino)-7H-pyrrolo[2,3-d]pyrimidine-5-carboxyamide;

(236) (S)-(7-((4-(methylamino)-5-(trifluoromethyl)-7H-pyrrolo[2,3-d]pyrimidin-2-yl)amino)-2,3-dihydrobenzofuran-4-yl)(3-morpholinopyrrolidin-1-yl)methanone;

(237) (R)-(7-((4-(methylamino)-5-(trifluoromethyl)-7H-pyrrolo[2,3-d]pyrimidin-2-yl)amino)-2,3-dihydrobenzofuran-4-yl)(3-morpholinopyrrolidin-1-yl)methanone;

(238) (8-((4-(methylamino)-5-(trifluoromethyl)-7H-pyrrolo[2,3-d]pyrimidin-2-yl)amino)-2,3 -dihydrobenzo [b][1,4]dioxin- 5-yl)(4-morpholinopiperidin-1-yl)methanone;

(239) 4-(4-((4-(cyclopentylamino)-7*H*-pyrrolo[2,3-*d*]pyrimidin-2-yl)amino)-3-methoxyphenyl)-l-(oxetan-3-yl)-1,4-azaphosphinane 4-oxide;

(240) 4-(4-((4-(cyclohexylamino)-7H-pyrrolo[2,3-d]pyrimidin-2-yl)amino)-3-methoxyphenyl)-1-(oxetan-3-yl)-1,4-azaphosphinane 4-oxide;

(241) (7-((4-(cyclopentylamino)-7H-pyrrolo[2,3-d]pyrimidin-2-yl)amino)-2,3-dihydrobenzofuran-4-yl)(4-morpholinopiperidin-1-yl)methanone;

(242) 4-(4-((4-(cyclopentylamino)-7H-pyrrolo[2,3-d]pyrimidin-2-yl)amino)-3-methoxyphenyl)-1-cyclopropyl-1,4-azaphosphinane 4-oxide;

(243) 4-(4-((4-(cyclopentylamino)-7*H*-pyrrolo[2,3-*d*]pyrimidin-2-yl)amino)-3-methoxyphenyl)-1-(tetrahydro-2H-pyran-4-yl)-1,4-azaphosphinane 4-oxide;

(244) (7-((4-(cyclohexylamino)-7H-pyrrolo[2,3-d]pyrimidin-2-yl)amino)-2,3-dihydrobenzofuran-4-yl)(morpholino)methanone;

(245) (7-((4-(cyclohexylamino)-7H-pyrrolo[2,3-d]pyrimidin-2-yl)amino)-2,3-dihydrobenzofuran-4-yl)(4-morpholinopiperidin-1-yl)methanone;

(246) *N*$^4$-allyl-*N*$^2$-(2-methoxy-4-(morpholinosulfonyl)phenyl)-7*H*-pyrrolo[2,3-*d*]pyrimidine-2,4-diamine;

(247) *N*$^4$-allyl-*N*$^2$-(2-methoxy-4-((4-morpholinopiperidin-1-yl)sulfonyl)phenyl)-7*H*-pyrrolo[2,3-*d*]pyrimidine-2,4-diamine;

(248) 4-(cyclohexylamino-2-((8-(morpholine-4-carbonyl)-2,3-dihydrobenzo[*b*][1,4]dioxin-5-yl)amino)-7*H*-pyrrolo[2,3-*d*]pyrimidine-5-carbonitrile;

(249) 4-((1-methoxy-2-methylpropan-2-yl)amino)-2-((2-methoxy-4-(methylsulfonyl)phenyl)amino)-7*H*-pyrrolo[2,3-*d*]pyrimidine-5-carbonitrile;

(250) 4-(isopropylamino)-2-((2-methoxy-4-(morpholinosulfonyl)phenyl)amino)-7H-pyrrolo[2,3-d]pyrimidine-5-carbonitrile;

(251) 4-(cyclohexylamino)-2-((8-(4-morpholinopiperidine-1-carbonyl)-2,3-dihydrobenzo[*b*][1,4]dioxin-5-yl)amino)-7*H*-pyrrolo[2,3-*d*]pyrimidine-5-carbonitrile;

(252) 2-((4-(dimethylphosphoryl)-2-methoxyphenyl)amino)-4-(ethylamino)-7*H*-pyrrolo[2,3-*d*]pyrimidine-5-carbonitrile;

(253) 2-((4-(dimethylphosphoryl)-2-methoxyphenyl)amino)-4-(methylamino)-7*H*-pyrrolo[2,3-*d*]pyrimidine-5-carbonitrile;

(254) 2-((4-(dimethylphosphoryl)-2-methoxyphenyl)amino)-4-(propylamino)-7*H*-pyrrolo[2,3-*d*]pyrimidine-5-carbonitrile;

(255) 2-((4-(dimethylphosphoryl)-2-methoxyphenyl)amino)-4-(isobutylamino)-7*H*-pyrrolo[2,3-*d*]pyrimidine-5-carbonitrile;

(256) 4-((2-methoxyethyl)amino)-2-((8-(4-morpholinopiperidine-1-carbonyl)-2,3-dihydrobenzo[*b*][1,4]dioxin-5-yl)amino)-7*H*-pyrrolo[2,3-*d*]pyrimidine-5-carbonitrile;

(257) 2-((2-methoxy-4-(methylsulfonyl)phenyl)amino)-4-(methylamino)-7*H*-pyrrolo[2,3-*d*]pyrimidine-5-carbonitrile;

(258) 4-(ethylamino)-2-((8-(4-morpholinopiperidine-1-carbonyl)-2,3-dihydrobenzo[*b*][1,4]dioxin-5-yl)amino)-7*H*-pyrrolo[2,3-*d*]pyrimidine-5-carbonitrile;

(259) 4-(cyclobutylamino)-2-((8-(4-morpholinopiperidine-1-carbonyl)-2,3-dihydrobenzo[*b*][1,4]dioxin-5-yl)amino)-7*H*-pyrrolo[2,3-*d*]pyrimidine-5-carbonitrile;

(260) 2-((4-(1-ethyl-4-oxido-1,4-azaphosphinan-4-yl)-2-methoxyphenyl)amino)-4-(ethylamino)-7*H*-pyrrolo[2,3-*d*]pyrimidine-5-carbonitrile;

(261) 4-cyclopropyl-2-((4-(1-ethyl-4-oxido-1,4-azaphosphinan-4-yl)-2-methoxyphenyl)amino)-7*H*-pyrrolo[2,3-*d*]pyrimidine-5-carbonitrile;

(262) (*R*)-4-(*sec*-butylamino)-2-((2-methoxy-4-(methylsulfonyl)phenyl)amino)-7*H*-pyrrolo[2,3-*d*]pyrimidine-5-carbonitrile;

(263) (*S*)-4-(*sec*-butylamino)-2-((2-methoxy-4-((4-morpholinopiperidin-1-yl)sulfonyl)phenyl)amino)-7*H*-pyrrolo[2,3-*d*]pyrimidine-5-carbonitrile;

(264) 4-(ethylamino)-2-((7-(4-morpholinopiperidine-1-carbonyl)benzo[*d*][1,3]dioxol-4-yl)amino)-7*H*-pyrrolo[2,3-*d*]pyrimidine-5-carbonitrile;

(265) 4-(ethylamino)-2-((8-(4-(oxetan-3-yl)piperazine-1-carbonyl)-2,3-dihydrobenzo[*b*][1,4]dioxin-5-yl)amino)-7*H*-pyrrolo[2,3-*d*]pyrimidine-5-carbonitrile;

(266) (*R*)-4-(*sec*-butylamino)-2-((4-(dimethylphosphoryl)-2-methoxyphenyl)amino)-7*H*-pyrrolo[2,3-*d*]pyrimidine-5-carbonitrile;

(267) (*S*)-4-(*sec*-butylamino)-2-((4-(dimethylphosphoryl)-2-methoxyphenyl)amino)-7*H*-pyrrolo[2,3-*d*]pyrimidine-5-carbonitrile;

(268) 4-(cyclobutylamino)-2-((4-(dimethylphosphoryl)-2-methoxyphenyl)amino)-7*H*-pyrrolo[2,3-*d*]pyrimidine-5-carbonitrile;

(269) 4-(cyclopentylamino)-2-((4-(dimethylphosphoryl)-2-methoxyphenyl)amino)-7*H*-pyrrolo[2,3-*d*]pyrimidine-5-carbonitrile;

(270) (*R*)-2-((4-(dimethylphosphoryl)-2-methoxyphenyl)amino)-4-((tetrahydrofuran-3-yl)amino)-7*H*-pyrrolo[2,3-*d*]pyrimidine-5-carbonitrile;

(271) (S)-2-((4-(dimethylphosphoryl)-2-methoxyphenyl)amino)-4-((tetrahydrofuran-3-yl)amino)-7*H*-pyrrolo[2,3-*d*]pyrimidine-5-carbonitrile;

(272) 2-((4-(dimethylphosphoryl)-2-methoxyphenyl)amino)-4-((tetrahydro-2*H*-pyran-4-yl)amino)-7*H*-pyrrolo[2,3-*d*]pyrimidine-5-carbonitrile;

(273) 4-((cyclopropylmethyl)amino)-2-((4-(dimethylphosphoryl)-2-methoxyphenyl)amino)-7*H*-pyrrolo[2,3-*d*]pyrimidine-5-carbonitrile;

(274) 4-((cyclopentylmethyl)amino)-2-((4-(dimethylphosphoryl)-2-methoxyphenyl)amino)-7*H*-pyrrolo[2,3-*d*]pyrimidine-5-carbonitrile;

(275) 4-(butylamino)-2-((4-(dimethylphosphoryl)-2-methoxyphenyl)amino)-7*H*-pyrrolo[2,3-*d*]pyrimidine-5-carbonitrile;

(276) 2-((4-(dimethylphosphoryl)-2-methoxyphenyl)amino)-4-(oxetan-3-ylamino)-7H-pyrrolo[2,3-*d*]pyrimidine-5-carbonitrile;

(277) 4-(ethylamino)-2-((7-(morpholine-4-carbonyl)benzo[*d*][1,3]dioxol-4-yl)amino)-7*H*-pyrrolo[2,3-*d*]pyrimidine-5-carbonitrile;

(278) 4-(cycloheptylamino)-2-((4-(divinylphosphoryl)-2-methoxyphenyl)amino)-7*H*-pyrrolo[2,3-*d*]pyrimidine-5-carbonitrile;

(279) 2-((4-(1-cyclopropyl-4-oxido-1,4-azaphosphinan-4-yl)-2-methoxyphenyl)amino)-4-(methylamino)-7*H*-pyrrolo[2,3-*d*]pyrimidine-5-carbonitrile;

(280) 4-(methylamino)-2-((8-(4-(oxetan-3-yl)piperazine-1-carbonyl)-2,3-dihydrobenzo[*b*][1,4]dioxin-5-yl)amino)-7*H*-pyrrolo[2,3-*d*]pyrimidine-5-carbonitrile;

(281) 4-(ethylamino)-2-((8-(4-(oxetan-3-yl)piperazine-1-carbonyl)-2,3-dihydrobenzo[*b*][1,4]dioxin-5-yl)amino)-7*H*-pyrrolo[2,3-*d*]pyrimidine-5-carbonitrile;

(282) 2-((4-(1-cyclopropyl-4-oxido-1,4-azaphosphinan-4-yl)-2-methoxyphenyl)amino)-4-(propylamino)-7*H*-pyrrolo[2,3-*d*]pyrimidine-5-carbonitrile;

(283) 2-((2-methoxy-4-(1-(oxetan-3-yl)-4-oxido-1,4-azaphosphinan-4-yl)phenyl)amino)-4-(propylamino)-7*H*-pyrrolo[2,3-*d*]pyrimidine-5-carbonitrile;

(284) 2-((8-(4-(oxetan-3-yl)piperazine-1-carbonyl)-2,3-dihydrobenzo[*b*][1,4]dioxin-5-yl)amino)-4-(propylamino)-7*H*-pyrrolo[2,3-*d*]pyrimidine-5-carbonitrile;

(285) 2-((4-(1-cyclopropy1-4-oxido-1,4-azaphosphinan-4-yl)-2-methoxyphenyl)amino)-4-(cyclopropylamino)-7*H*-pyrrolo[2,3-*d*]pyrimidine-5-carbonitrile;

(286) 4-(cyclopropylamino)-2-((2-methoxy-4-(1-(oxetan-3-yl)-4-oxido-1,4-azaphosphinan-4-yl)phenyl)amino)-7*H*-pyrrolo[2,3-*d*]pyrimidine-5-carbonitrile;

(287) 4-(cyclopropylamino)-2-((8-(4-(oxetan-3-yl)piperazine-1-carbonyl)-2,3-dihydrobenzo[*b*][1,4]dioxin-5-yl)amino)-7*H*-pyrrolo[2,3-*d*]pyrimidine-5-carbonitrile;

(288) 4-(cyclobutylamino)-2-((2-methoxy-4-(1-(oxetan-3-yl)-4-oxido-1,4-azaphosphinan-4-yl)phenyl)amino)-7*H*-pyrrolo[2,3-*d*]pyrimidine-5-carbonitrile;

(289) 4-(cyclobutylamino)-2-((8-(4-(oxetan-3-yl)piperazine-1-carbonyl)-2,3-dihydrobenzo[*b*][1,4]dioxin-5-yl)amino)-7*H*-pyrrolo[2,3-*d*]pyrimidine-5-carbonitrile;

(290) 2-((4-(1-cyclopropyl-4-oxido-1,4-azaphosphinan-4-yl)-2-methoxyphenyl)amino)-4-(isobutylamino)-7H-pyrrolo[2,3-d]pyrimidine-5-carbonitrile;

(291) 4-(isobutylamino)-2-((2-methoxy-4-(1-(oxetan-3-yl)-4-oxido-1,4-azaphosphinan-4-yl)phenyl)amino)-7H-pyrrolo[2,3-d]pyrimidine-5-carbonitrile;

(292) 2-((4-(1-cyclopropyl-4-oxido-1,4-azaphosphinan-4-yl)-2-methoxyphenyl)amino)-4-(isopropylamino)-7H-pyrrolo[2,3-d]pyrimidine-5-carbonitrile;

(293) 4-(isopropylamino)-2-((2-methoxy-4-(1-(oxetan-3-yl)-4-oxido-1,4-azaphosphinan-4-yl)phenyl)amino)-7H-pyrrolo[2,3-d]pyrimidine-5-carbonitrile;

(294) (*R*)-4-(*sec*-butylamino)-2-((4-(1-cyclopropyl-4-oxido-1,4-azaphosphinan-4-yl)-2-methoxyphenyl)amino)-7*H*-pyrrolo[2,3-*d*]pyrimidine-5-carbonitrile;
(295) (*R*)-4-(*sec*-butylamino)-2-((2-methoxy-4-(1-(oxetan-3-yl)-4-oxido-1,4-azaphosphinan-4-yl)phenyl)amino)-7*H*-pyrrolo[2,3-*d*]pyrimidine-5-carbonitrile;
(296) (*S*)-4-(*sec*-butylamino)-2-((4-(1-cyclopropyl-4-oxido-1,4-azaphosphinan-4-yl)-2-methoxyphenyl)amino)-7*H*-pyrrolo[2,3-*d*]pyrimidine-5-carbonitrile;
(297) (*S*)-4-(*sec*-butylamino)-2-((2-methoxy-4-(1-(oxetan-3-yl)-4-oxido-1,4-azaphosphinan-4-yl)phenyl)amino)-7*H*-pyrrolo[2,3-*d*]pyrimidine-5-carbonitrile;
(298) (*R*)-2-((4-(1-cyclopropyl-4-oxido-1,4-azaphosphinan-4-yl)-2-methoxyphenyl)amino)-4-((tetrahydrofuran-3-yl)amino)-7*H*-pyrrolo[2,3-*d*]pyrimidine-5-carbonitrile;
(299) (*R*)-2-((2-methoxy-4-(1-(oxetan-3-yl)-4-oxido-1,4-azaphosphinan-4-yl)phenyl)amino)-4-((tetrahydrofuran-3-yl)amino)-7*H*-pyrrolo[2,3-*d*]pyrimidine-5-carbonitrile;
(300) (*S*)-2-((4-(1-cyclopropyl-4-oxido-1,4-azaphosphinan-4-yl)-2-methoxyphenyl)amino)-4-((tetrahydrofuran-3-yl)amino)-7*H*-pyrrolo[2,3-*d*]pyrimidine-5-carbonitrile;
(301) (S)-2-((2-methoxy-4-(1-(oxetan-3-yl)-4-oxido-1,4-azaphosphinan-4-yl)phenyl)amino)-4-((tetrahydrofuran-3-yl)amino)-7*H*-pyrrolo[2,3-*d*]pyrimidine-5-carbonitrile;
(302) 2-((4-(1-cyclopropyl-4-oxido-1,4-azaphosphinan-4-yl)-2-methoxyphenyl)amino)-4-((tetrahydro-2*H*-pyran-4-yl)amino)-7*H*-pyrrolo[2,3-*d*]pyrimidine-5-carbonitrile;
(303) 2-((2-methoxy-4-(1-(oxetan-3-yl)-4-oxido-1,4-azaphosphinan-4-yl)phenyl)amino)-4-((tetrahydro-2*H*-pyran-4-yl)amino)-7*H*-pyrrolo[2,3-*d*]pyrimidine-5-carbonitrile;
(304) 2-((4-(1-cyclopropyl-4-oxido-1,4-azaphosphinan-4-yl)-2-methoxyphenyl)amino)-4-((cyclopropylmethyl)amino)-7*H*-pyrrolo[2,3-*d*]pyrimidine-5-carbonitrile;
(305) 4-((cyclopropylmethyl)amino)-2-((2-methoxy-4-(1-(oxetan-3-yl)-4-oxido-1,4-azaphosphinan-4-yl)phenyl)amino)-7*H*-pyrrolo[2,3-*d*]pyrimidine-5-carbonitrile;
(306) 4-((cyclobutylmethyl)amino)-2-((4-(1-cyclopropyl-4-oxido-1,4-azaphosphinan-4-yl)-2-methoxyphenyl)amino)-7*H*-pyrrolo[2,3-*d*]pyrimidine-5-carbonitrile;
(307) 4-((cyclobutylmethyl)amino)-2-((2-methoxy-4-(1-(oxetan-3-yl)-4-oxido-1,4-azaphosphinan-4-yl)phenyl)amino)-7H-pyrrolo[2,3-d]pyrimidine-5-carbonitrile;
(308) 4-((cyclopentylmethyl)amino)-2-((4-(1-cyclopropyl-4-oxido-1,4-azaphosphinan-4-yl)-2-methoxyphenyl)amino)-7H-pyrrolo[2,3-d]pyrimidine-5-carbonitrile;
(309) 4-((cyclopentylmethyl)amino)-2-((2-methoxy-4-(1-(oxetan-3-yl)-4-oxido-1,4-azaphosphinan-4-yl)phenyl)amino)-7*H*-pyrrolo[2,3-*d*]pyrimidine-5-carbonitrile;
(310) 2-((4-(1-cyclopropyl-4-oxido-1,4-azaphosphinan-4-yl)-2-methoxyphenyl)amino)-4-((2-(methylsulfonyl)ethyl)amino)-7*H*-pyrrolo[2,3-*d*]pyrimidine-5-carbonitrile;
(311) 2-((2-methoxy-4-(1-(oxetan-3-yl)-4-oxido-1,4-azaphosphinan-4-yl)phenyl)amino)-4-(oxetan-3-ylamino)-7*H*-pyrrolo[2,3-*d*]pyrimidine-5-carbonitrile;
(312) 2-((2-methoxy-4-(1-(oxetan-3-yl)-4-oxido-1,4-azaphosphinan-4-yl)phenyl)amino)-4-((2-methoxyethyl)amino)-7*H*-pyrrolo[2,3-*d*]pyrimidine-5-carbonitrile;
(313) 4-(cyclopentylamino)-2-((2-methoxy-4-(1-(oxetan-3-yl)-4-oxido-1,4-azaphosphinan-4-yl)phenyl)amino)-7*H*-pyrrolo[2,3-*d*]pyrimidine-5-carbonitrile;
(314) 2-((2-methoxy-4-(1-(oxetan-3-yl)-4-oxido-1,4-azaphosphinan-4-yl)phenyl)amino)-4-((2-(methylsulfonyl)ethyl)amino)-7*H*-pyrrolo[2,3-*d*]pyrimidine-5-carbonitrile;
(315) (*S*)-2-((8-(4-(oxetan-3-yl)piperazine-1-carbonyl)-2,3-dihydrobenzo[*b*][1,4]dioxin-5-yl)amino)-4-((tetrahydrofuran-3-yl)amino)-7*H*-pyrrolo[2,3-*d*]pyrimidine-5-carbonitrile;
(316) 2-((8-(4-(oxetan-3-yl)piperazine-1-carbonyl)-2,3-dihydrobenzo[*b*][1,4]dioxin-5-yl)amino)-4-((tetrahydro-2*H*-pyran-4-yl)amino)-7*H*-pyrrolo[2,3-*d*]pyrimidine-5-carbonitrile;
(317) 4-((cyclopropylmethyl)amino)-2-((8-(4-(oxetan-3-yl)piperazine-1-carbonyl)-2,3-dihydrobenzo[*b*][1,4]dioxin-5-yl)amino)-7*H*-pyrrolo[2,3-*d*]pyrimidine-5-carbonitrile;
(318) 4-((cyclobutylmethyl)amino)-2-((8-(4-(oxetan-3-yl)piperazine-1-carbonyl)-2,3-dihydrobenzo[*b*][1,4]dioxin-5-yl)amino)-7*H*-pyrrolo[2,3-*d*]pyrimidine-5-carbonitrile;
(319) 4-(cyclopentylamino)-2-((4-(morpholine-4-carbonyl)-2,3-dihydrobenzofuran-7-yl)amino)-7*H*-pyrrolo[2,3-*d*]pyrimidine-5-carbonitrile;
(320) 4-(cyclopentylamino)-2-((4-(4-morpholinopiperidine-1-carbonyl)-2,3-dihydrobenzofuran-7-yl)amino)-7*H*-pyrrolo[2,3-*d*]pynmidine-5-carbonitrile;
(321) 4-(cyclohexylamino)-2-((4-(morpholine-4-carbonyl)-2,3-dihydrobenzofuran-7-yl)amino)-7*H*-pyrrolo[2,3-*d*]pyrimidine-5-carbonitrile;
(322) 4-(cyclohexylamino)-2-((4-(4-morpholinopiperidine-1-carbonyl)-2,3-dihydrobenzofuran-7-yl)amino)-7*H*-pyrrolo[2,3-*d*]pyrimidine-5-carbonitrile;

(323) 4-(cyclohexylamino)-2-((4-(1-cyclopropyl-4-oxido-1,4-azaphosphinan-4-yl)-2-methoxyphenyl)amino)-7*H*-pyrrolo[2,3-*d*]pyrimidine-5-carbonitrile;
(324) 4-(cyclohexylamino)-2-((2-methoxy-4-(4-oxido-1-(tetrahydro-2*H*-pyran-4-yl)-1,4-azaphosphinan-4-yl)phenyl)amino)-7*H*-pyrrolo[2,3-*d*]pyrimidine-5-carbonitrile;
(325) 2-((8-(3,3-bis(hydroxymethyl)azetidine-1-carbonyl)-2,3-dihydrobenzo[*b*][1,4]dioxin-5-yl)amino)-4-(methylamino)-7*H*-pyrrolo[2,3-*d*]pyrimidine-5-carbonitrile;
(326) 2-((8-(3,3-bis(hydroxymethyl)azetidine-1-carbonyl)-2,3-dihydrobenzo[*b*][1,4]dioxin-5-yl)amino)-4-(ethylamino)-7*H*-pyrrolo[2,3-*d*]pyrimidine-5-carbonitrile;
(327) 2-((8-(3,3-bis(hydroxymethyl)azetidine-1-carbonyl)-2,3-dihydrobenzo[*b*][1,4]dioxin-5-yl)amino)-4-(propylamino)-7*H*-pyrrolo[2,3-*d*]pyrimidine-5-carbonitrile;
(328) 2-((8-(3,3-bis(hydroxymethyl)azetidine-1-carbonyl)-2,3-dihydrobenzo[*b*][1,4]dioxin-5-yl)amino)-4-(isobutylamino)-7*H*-pyrrolo[2,3-*d*]pyrimidine-5-carbonitrile;
(329) 2-((8-(3,3-bis(hydroxymethyl)azetidine-1-carbonyl)-2,3-dihydrobenzo[*b*][1,4]dioxin-5-yl)amino)-4-(isopropylamino)-7*H*-pyrrolo[2,3-*d*]pyrimidine-5-carbonitrile;
(330) (*R*)-2-((8-(3,3-bis(hydroxymethyl)azetidine-1-carbonyl)-2,3-dihydrobenzo[*b*][1,4]dioxin-5-yl)amino)-4-(*sec*-butylamino)-7*H*-pyrrolo[2,3-*d*]pyrimidine-5-carbonitrile;
(331) (*S*)-2-((8-(3,3-bis(hydroxymethyl)azetidine-1-carbony1)-2,3-dihydrobenzo[*b*][1,4]dioxin-5-yl)amino)-4-(*sec*-butylamino)-7*H*-pyrrolo[2,3-*d*]pyrimidine-5-carbonitrile;
(332) 2-((8-(3,3-bis(hydroxymethyl)azetidine-1-carbonyl)-2,3-dihydrobenzo[*b*][1,4]dioxin-5-yl)amino)-4-(cyclopropylamino)-7*H*-pyrrolo[2,3-*d*]pyrimidine-5-carbonitrile;
(333) 2-((8-(3,3-bis(hydroxymethyl)azetidine-1-carbonyl)-2,3-dihydrobenzo[*b*][1,4]dioxin-5-yl)amino)-4-(cyclobutylamino)-7*H*-pyrrolo[2,3-*d*]pyrimidine-5-carbonitrile;
(334) 2-((8-(3,3-bis(hydroxymethyl)azetidine-1-carbonyl)-2,3-dihydrobenzo[*b*][1,4]dioxin-5-yl)amino)-4-(cyclopentylamino)-7*H*-pyrrolo[2,3-*d*]pyrimidine-5-carbonitrile;
(335) (*R*)-2-((8-(3,3-bis(hydroxymethyl)azetidine-1-carbonyl)-2,3-dihydrobenzo[*b*][1,4]dioxin-5-yl)amino)-4-((tetrahydrofuran-3-yl)amino)-7*H*-pyrrolo[2,3-*d*]pyrimidine-5-carbonitrile;
(336) (*S*)-2-((8-(3,3-bis(hydroxymethyl)azetidine-1-carbonyl-2,3-dihydrobenzo[*b*][1,4]dioxin-5-yl)amino)-4-((tetrahydrofuran-3-yl)amino)-7*H*-pyrrolo[2,3-*d*]pyrimidine-5-carbonitrile;
(337) 2-((8-(3,3-bis(hydroxymethyl)azetidine-1-carbonyl)-2,3-dihydrobenzo[*b*][1,4]dioxin-5-yl)amino)-4-((tetrahydro-2*H*-pyran-4-yl)amino)-7*H*-pyrrolo[2,3-*d*]pyrimidine-5-carbonitrile;
(338) 2-((8-(3,3-bis(hydroxymethyl)azetidine-1-carbonyl)-2,3-dihydrobenzo[b][1,4]dioxin-5-yl)amino)-4-((cyclopropylmethyl)amino)-7*H*-pyrrolo[2,3-*d*]pyrimidine-5-carbonitrile;
(339) 2-((8-(3,3-bis(hydroxymethyl)azetidine-1-carbonyl)-2,3-dihydrobenzo[b][1,4]dioxin-5-yl)amino)-4-((cyclobutylmethyl)amino)-7*H*-pyrrolo[2,3-*d*]pyrimidine-5-carbonitrile;
(340) 2-((8-(3,3-bis(hydroxymethyl)azetidine-1-carbonyl)-2,3-dihydrobenzo[b][1,4]dioxm-5-yl)amino)-4-((cyclopentylmethyl)amino)-7*H*-pyrrolo[2,3-*d*]pyrimidine-5-carbonitrile;
(341) 2-((8-(3,3-bis(hydroxymethyl)azetidine-1-carbonyl)-2,3-dihydrobenzo[b][1,4]dioxin-5-yl)amino)-4-(butylamino)-7*H*-pyrrolo[2,3-*d*]pyrimidine-5-carbonitrile;
(342) 4-((cyclopentylmethyl)amino)-2-((4-(morpholine-4-carbonyl)-2,3-dihydrobenzofuran-7-yl)amino)-7*H*-pyrrolo[2,3-*d*]pyrimidine-5-carbonitrile;
(343) 4-((cyclopentylmethyl)amino)-2-((4-(4-morpholinopiperidine-1-carbonyl)-2,3-dihydrobenzofuran-7-yl)amino)-7*H*-pyrrolo[2,3-*d*]pyrimidine-5-carbonitrile;
(344) 4-((2-(methylsulfonyl)ethyl)amino)-2-((4-(morpholine-4-carbonyl)-2,3-dihydrobenzofuran-7-yl)amino)-7*H*-pyrrolo[2,3-*d*]pyrimidine-5-carbonitrile;
(345) 4-((2-(methylsulfonyl)ethyl)amino)-2-((4-(4-morpholinopiperidine-1-carbonyl)-2,3-dihydrobenzofuran-7-yl)amino)-7*H*-pyrrolo[2,3-*d*]pyrimidine-5-carbonitrile;
(346) 4-(ethylamino)-2-((4-(morpholine-4-carbonyl)-2,3-dihydrobenzofuran-7-yl)amino)-7*H*-pyrrolo[2,3-*d*]pyrimidine-5-carbonitrile;
(347) 4-(ethylamino)-2-((4-(4-morpholnopiperidine-1-carbonyl)-2,3-dihydrobenzofuran-7-yl)amino)-7*H*-pyrrolo[2,3-*d*]pyrimidine-5-carbonitrile;
(348) 2-((4-(morpholine-4-carbonyl)-2,3-dihydrobenzofuran-7-yl)amino)-4-(propylamino)-7*H*-pyrrolo[2,3-*d*]pyrimidine-5-carbonitrile;
(349) 2-((4-(4-morpholinopiperidine-1-carbonyl)-2,3-dihydrobenzofuran-7-yl)amino)-4-(propylamino)-7*H*-pyrrolo[2,3-*d*]pyrimidine-5-carbonitrile;
(350) 4-(isobutylamino)-2-((4-(morpholine-4-carbonyl)-2,3-dihydrobenzofuran-7-yl)amino)-7*H*-pyrrolo[2,3-*d*]pyrimidine-5-carbonitrile;
(351) 4-(isobutylamino)-2-((4-(4-morpholinopiperidine-1-carbonyl)-2,3-dihydrobenzofuran-7-yl)amino)-7*H*-pyrrolo[2,3-*d*]pyrimidine-5-carbonitrile;

(352) 4-(isopropylamino)-2-((4-(morpholine-4-carbonyl)-2,3-dihydrobenzofuran-7-yl)amino)-7*H*-pyrrolo[2,3-*d*]pyrimidine-5-carbonitrile;

(353) 4-(isopropylamino)-2-((4-(4-morpholinopiperidine-1-carbonyl)-2,3-dihydrobenzofuran-7-yl)amino)-7*H*-pyrrolo[2,3-*d*]pyrimidine-5-carbonitrile;

(354) (*R*)-4-(*sec*-butylamino)-2-((4-(morpholine-4-carbonyl)-2,3-dihydrobenzofuran-7-yl)amino)-7*H*-pyrrolo[2,3-*d*]pyrimidine-5-carbonitrile;

(355) (R)-4-(sec-butylamino)-2-((4-(4-morpholinopiperidine-1-carbonyl)-2,3-dihydrobenzofuran-7-yl)amino)-7*H*-pyrrolo[2,3-*d*]pyrimidine-5-carbonitrile;

(356) (S)-4-(sec-butylamino)-2-((4-(morpholine-4-carbonyl)-2,3-dihydrobenzofuran-7-yl)amino)-7*H*-pyrrolo[2,3-*d*]pyrimidine-5-carbonitrile;

(357) (*S*)-4-(sec-butylamino)-2-((4-(4-morpholinopiperidine-1-carbonyl)-2,3-dihydrobenzofuran-7-yl)amino)-7*H*-pyrrolo[2,3-*d*]pyrimidine-5-carbonitrile;

(358) 4-((2-methoxyethyl)amino)-2-((4-(morpholine-4-carbonyl)-2,3-dihydrobenzofuran-7-yl)amino)-7*H*-pyrrolo[2,3-*d*]pyrimidine-5-carbonitrile;

(359) 4-((2-methoxyethyl)amino)-2-((4-(4-morpholinopiperidine-1-carbonyl)-2,3-dihydrobenzofuran-7-yl)amino)-7*H*-pyrrolo[2,3-*d*]pyrimidine-5-carbonitrile;

(360) 4-(cyclobutylamino)-2-((4-(morpholine-4-carbonyl)-2,3-dihydrobenzofuran-7-yl)amino)-7*H*-pyrrolo[2,3-*d*]pyrimidine-5-carbonitrile;

(361) 4-(allylamino)-2-((2-methoxy-4-((4-morpholinopiperidin-1-yl)sulfonyl)phenyl)amino)-7*H*-pyrrolo[2,3-*d*]pyrimidine-5-carbonitrile;

(362) 4-(allylamino)-2-((2-methoxy-4-(morpholinosulfonyl)phenyl)amino)-7*H*-pyrrolo[2,3-*d*]pyrimidine-5-carbonitrile;

(363) (8-((4-(ethylamino)-5-(trifluoromethyl)-7*H*-pyrrolo[2,3-*d*]pyrimidin-2-yl)amino)-2,3-dihydrobenzo[*b*][1,4]dioxin-5-yl)(4-morpholinopiperidin-1-yl)methanone;

(364) (4-morpholinopiperidin-1-yl)(8-((4-(propylamino)-5-(trifluoromethyl)-7*H*-pyrrolo[2,3-*d*]pyrimidin-2-yl)amino)-2,3-dihydrobenzo[*b*][1,4]dioxin-5-yl)methanone;

(365) (8-((4-(isopropylamino)-5-(trifluoromethyl)-7*H*-pyrrolo[2,3-d]pyrimidin-2-yl)amino)-2,3-dihydrobenzo[*b*][1,4]dioxin-5-yl)(4-morpholinopiperidin-1-yl)methanone;

(366) (8-((4-((2-methoxyethyl)amino)-5-(trifluoromethyl)-7*H*-pyrrolo[2,3-*d*]pyrimidin-2-yl)amino)-2,3-dihydrobenzo[*b*][1,4]dioxin-5-yl)(4-morpholinopiperidin-1-yl)methanone;

(367) (8-((4-(cyclopropylamino)-5-(trifluoromethyl)-7*H*-pyrrolo[2,3-*d*]pyrimidin-2-yl)amino)-2,3-dihydrobenzo[*b*][1,4]dioxin-5-yl)(4-morpholinopiperidin-1-yl)methanone;

(368) (4-((4-(ethylamino)-5-(trifluoromethyl)-7*H*-pyrrolo[2,3-*d*]pyrimidin-2-yl)amino)-3-methoxyphenyl)dimethylphosphine oxide;

(369) (3-methoxy-4-((4-(propylamino)-5-(trifluoromethyl)-7*H*-pyrrolo[2,3-d]pyrimidin-2-yl)amino)phenyl)dimethylphosphine oxide;

(370) (4-((4-(isobutylamino)-5-(trifluoromethyl)-7*H*-pyrrolo[2,3-*d*]pyrimidin-2-yl)amino)-3-methoxyphenyl)dimethylphosphine oxide;

(371) (4-((4-(isopropylamino)-5-(trifluoromethyl)-7*H*-pyrrolo[2,3-*d*]pyrimidin-2-yl)amino)-3-methoxyphenyl)dimethylphosphine oxide;

(372) $N^2$-(2-methoxy-4-(morpholinosulfonyl)phenyl)-$N^4$-propyl-5-(trifluoromethyl)-7*H*-pyrrolo[2,3-*d*]pyrimidine-2,4-diamine;

(373) $N^2$-(2-methoxy-4-(morpholinosulfonyl)phenyl)-$N^4$-(2-methoxyethyl)-5-(trifluoromethyl)-7*H*-pyrrolo[2,3-*d*]pyrimidine-2,4-diamine;

(374) $N^4$-cyclopropyl-$N^2$-(2-methoxy-4-(morpholinosulfonyl)phenyl)-5-(trifluoromethyl)-7*H*-pyrrolo[2,3-*d*]pyrimidine-2,4-diamine;

(375) $N^4$-cyclopropyl-$N^2$-(2-methoxy-4-((4-morpholinopiperidin-1-yl)sulfonyl)phenyl)-5-(trifluoromethyl)-7*H*-pyrrolo[2,3-*d*]pyrimidine-2,4-diamine;

(376) $N^4$-cyclopropyl-$N^2$-(2-methoxy-4-(methylsulfonyl)phenyl)-5-(trifluoromethyl)-7*H*-pyrrolo[2,3-*d*]pyrimidine-2,4-diamine;

(377) $N^4$-cyclobutyl-$N^2$-(2-methoxy-4-(morpholinosulfonyl)phenyl)-5-(trifluoromethyl)-7*H*-pyrrolo[2,3-*d*]pyrimidine-2,4-diamine;

(378) (8-((4-(cyclobutylamino)-5-(trifluoromethyl)-7H-pyrrolo[2,3-d]pyrimidin-2-yl)amino)-2,3-dihydrobenzo[b][1,4]dioxin-5-yl)(4-morpholinopiperidin-1-yl)methanone;

(379) (*S*)-$N^4$-(sec-butyl)-$N^2$-(2-methoxy-4-(morpholinosulfonyl)phenyl)-5-(trifluoromethyl)-7*H*-pyrrolo[2,3-*d*]pyrimidine-2,4-diamine;

(380) (*S*)-$N^4$-(*sec*-butyl)-$N^2$-(2-methoxy-4-(methylsulfonyl)phenyl)-5-(trifluoromethyl)-7*H*-pyrrolo[2,3-*d*]pyrimidine-2,4-diamine;

(381) (*S*)-(8-((4-(*sec*-butylamino)-5-(trifluoromethyl)-7*H*-pyrrolo[2,3-*d*]pyrimidin-2-yl)amino)-2,3-dihydrobenzo[*b*][1,4]dioxin-5-yl)(morpholino)methanone;
(382) (*R*)-$N^2$-(2-methoxy-4-(morpholinosulfonyl)phenyl)-$N^4$-(tetrahydrofuran-3-yl)-5-(trifluoromethyl)-7*H*-pyrrolo[2,3-*d*]pyrimidine-2,4-diamine;
(383) (*S*)-$N^2$-(2-methoxy-4-(morpholinosulfonyl)phenyl)-$N^4$-(tetrahydrofuran-3-yl)-5-(trifluoromethyl)-7*H*-pyrrolo[2,3-*d*]pyrimidine-2,4-diamine;
(384) (*S*)-$N^2$-(2-methoxy-4-(methylsulfonyl)phenyl)-$N^4$-(tetrahydrofuran-3-yl)-5-(trifluoromethyl)-7*H*-pyrrolo[2,3-*d*]pyrimidine-2,4-diamine;
(385) (*S*)-(4-((4-(*sec*-butylamino)-5-(trifluoromethyl)-7*H*-pyrrolo[2,3-*d*]pyrimidin-2-yl)amino)-3-methoxyphenyl)dimethylphosphine oxide;
(386) (4-((4-(cyclobutylamino)-5-(trifluoromethyl)-7*H*-pyrrolo[2,3-*d*]pyrimidin-2-yl)amino)-3-methoxyphenyl)dimethylphosphine oxide;
(387) (4-((4-(cyclopentylamino)-5-(trifluoromethyl)-7*H*-pyrrolo[2,3-*d*]pyrimidin-2-yl)amino)-3-methoxyphenyl)dimethylphosphine oxide;
(388) (R)-(3-methoxy-4-((4-((tetrahydrofuran-3-yl)amino)-5-(trifluoromethyl)-7*H*-pyrrolo[2,3-*d*]pyrimidin-2-yl)amino)phenyl)dimethylphosphine oxide;
(389) (3-methoxy-4-((4-((tetrahydro-2H-pyran-4-yl)amino)-5-(trifluoromethyl)-7*H*-pyrrolo[2,3-*d*]pyrimidin-2-yl)amino)phenyl)dimethylphosphine oxide;
(390) (4-((4-((cyclopropylmethyl)amino)-5-(trifluoromethyl)-7*H*-pyrrolo[2,3-*d*]pyrimidin-2-yl)amino)-3-methoxyphenyl)dimethylphosphine oxide;
(391) (4-((4-((cyclobutylmethyl)amino)-5-(trifluoromethyl)-7*H*-pyrrolo[2,3-*d*]pyrimidin-2-yl)amino)-3-methoxyphenyl)dimethylphosphine oxide;
(392) (4-((4-((cyclopentylmethyl)amino)-5-(trifluoromethyl)-7*H*-pyrrolo[2,3-*d*]pyrimidin-2-yl)amino)-3-methoxyphenyl)dimethylphosphine oxide;
(393) (4-((4-(butylamino)-5-(trifluoromethyl)-7*H*-pyrrolo[2,3-*d*]pyrimidin-2-yl)amino)-3-methoxyphenyl)dimethylphosphine oxide;
(394) 1-cyclopropyl-4-(4-((4-cyclopropyl-5-(trifluoromethyl)-7*H*-pyrrolo[2,3-*d*]pyrimidin-2-yl)amino)-3-methoxyphenyl)-1,4-azaphosphinane4-oxide;
(395) (8-((4-ethyl-5-(trifluoromethyl)-7*H*-pyrrolo[2,3-*d*]pyrimidin-2-yl)amino)-2,3-dihydrobenzo[*b*][1,4]dioxin-5-yl)(4-morpholinopiperidin-1-yl)methanone;
(396) 1-cyclopropyl-4-(4-((4-ethyl-5-(trifluoromethyl)-7*H*-pyrrolo[2,3-*d*]pyrimidin-2-yl)amino)-3-methoxyphenyl)-1,4-azaphosphinane-4-oxide;
(397) (8-((4-(methylamino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;
(398) 1-cyclopropyl-4-(4-((4-(ethylamino)-5-(trifluoromethyl)-7*H*-pyrrolo[2,3-*d*]pyrimidin-2-yl)amino)-3-methoxyphenyl)-1,4-azaphosphinane 4-oxide;
(399) 4-(4-((4-(ethylamino)-5-(trifluoromethyl)-7H-pyrrolo[2,3-d]pyrimidin-2-yl)amino)-3-methoxyphenyl)-1-(oxetan-3-yl)-1,4-azaphosphinane 4-oxide;
(400) (8-((4-(ethylamino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;
(401) 1-cyclopropyl-4-(3-methoxy-4-((4-(propylamino)-5-(trifluoromethyl)-7*H*-pyrrolo[2,3-*d*]pyrimidin-2-yl)amino)phenyl)-1,4-azaphosphinane 4-oxide;
(402) (4-(oxetan-3-yl)piperazin-1-yl)(8-((4-(propylamino)-5-(trifluoromethyl)-7*H*-pyrrolo[2,3-*d*]pyrimidin-2-yl)amino)-2,3-dihydrobenzo[*b*][1,4]dioxin-5-yl)methanone;
(403) (8-((4-((2-methoxyethyl)amino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;
(404) 1-cyclopropyl-4-(4-((4-(cyclopropylamino)5-(trifluoromethyl)-7*H*-pyrrolo[2,3-d]pyrimidin-2-yl)amino)3-methoxyphenyl)-1,4-azaphosphinane 4-oxide;
(405) (8-((4-(cyclopropylamino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;
(406) 4-(4-((4-((cyclobutylamino)-5-(trifluoromethyl)-7*H*-pyrrolo[2,3-*d*]pyrimidin-2-yl)amino)-3-methoxyphenyl)-1-cyclopropyl-1,4-azaphosphinane 4-oxide;
(407) (8-((4-(cyclobutylamino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;
(408) ((1S,4S)-2-oxa-5-azabicyclo[2.2.1]heptan-5-yl)(8-((4-(methylamino)-5-(trifluoromethyl)-7*H*-pyrrolo[2,3-*d*]pyrimidin-2-yl)amino)-2,3-dihydrobenzo[*b*][1,4]dioxin-5-yl)methanone;
(409) ((1S,4S)-2-oxa-5-azabicyclo[2.2.1]heptan-5-yl)(8-((4-(ethylamino)-5-(trifluoromethyl)-7*H*-pyrrolo[2,3-*d*]pyrimidin-2-yl)amino)-2,3-dihydrobenzo[*b*][1,4]dioxin-5-yl)methanone;

(410) ((1S,4S)-2-oxa-5-azabicyclo[2.2.1]heptan-5-yl)(8-((4-((2-methoxyethyl)amino)-5-(trifluoromethyl)-7*H*-pyrrolo[2,3-*d*]pyrimidn-2-yl)amino)-2,3-dihydrobenzo[*b*][1,4]dioxin-5-yl)methanone;

(411) 1-cyclopropyl-4-(3-methoxy-4-((4-(methylamino)-5-(trifluoromethyl)-7*H*-pyrrolo[2,3-*d*]pyrimidin-2-yl)amino)phenyl)-1,4-azaphosphinane 4-oxide;

(412) 1-cyclopropyl-4-(3-methoxy-4-((4-((2-methoxyethyl)amino)-5-(trifluoromethyl)-7*H*-pyrrolo[2,3-*d*]pyrimidin-2-yl)amino)phenyl)-1,4-azaphosphinane 4-oxide;

(413) 4-(4-((4-((cyclopentylamino)-5-(trifluoromethyl)-7*H*-pyrrolo[2,3-*d*]pyrimidin-2-yl)amino)-3-methoxyphenyl)-1-cyclopropyl-1,4-azaphosphinane 4-oxide;

(414) (8-((4-(cyclopentylamino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;

(415) (8-((4-(cyclohexylamino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;

(416) (*R*)-(4-(oxetan-3-yl)piperazin-1-yl)(8-((4-((tetrahydrofuran-3-yl)amino)-5-(trifluoromethyl)-7*H*-pyrrolo[2,3-*d*]pyrimidin-2-yl)amino)-2,3-dihydrobenzo[*b*][1,4]dioxin-5-yl)methanone;

(417) (S)-1-cyclopropyl-4-(3-methoxy-4-((4-((tetrahydrofuran-3-yl)amino)-5-(trifluoromethyl)-7*H*-pyrrolo[2,3-*d*]pyrimidin-2-yl)amino)phenyl)-1,4-azaphosphinane 4-oxide;

(418) (S)-(4-(oxetan-3-yl)piperazin-1-yl)(8-((4-((tetrahydrofuran-3-yl)amino)-5-(trifluoromethyl)-7*H*-pyrrolo[2,3-*d*]pyrimidin-2-yl)amino)-2,3-dihydrobenzo[*b*][1,4]dioxin-5-yl)methanone;

(419) 1-cyclopropyl-4-(3-methoxy-4-((4-((tetrahydro-2*H*-pyran-4-yl)amino)-5-(trifluoromethyl)-7*H*-pyrrolo[2,3-*d*]pyrimidin-2-yl)amino)phenyl)-1,4-azaphosphinane 4-oxide;

(420) (4-(oxetan-3-yl)piperazin-1-yl)(8-((4-((tetrahydro-2*H*-pyran-4-yl)amino)-5-(trifluoromethyl)-7*H*-pyrrolo[2,3-*d*]pyrimidin-2-yl)amino)-2,3-dihydrobenzo[*b*][1,4]dioxin-5-yl)methanone;

(421) 1-cyclopropyl-4-(4-((4-((cyclopropylmethyl)amino)-5-(trifluoromethyl)-7*H*-pyrrolo[2,3-d]pyrimidin-2-yl)amino)-3-methoxyphenyl)-1,4-azaphosphinane 4-oxide;

(422) (8-((4-((cyclopropylmethyl)amino)-5-(trifluoromethyl)-7*H*-pyrrolo[2,3- *d*]pyrimidin-2-yl)amino)-2,3-dihydrobenzo[b][1,4]dioxin-5-yl)(4-(oxetan-3-yl)piperazin-1-yl)methanone;

(423) 4-(4-((4-((cyclobutylmethyl)amino)-5-(trifluoromethyl)-7*H*-pyrrolo[2,3-*d*]pyrimidin-2-yl)amino)-3-methoxyphenyl)-1-cyclopropyl-1,4-azaphosphinane 4-oxide;

(424) (8-((4-((cyclobutylmethyl)amino)-5-(trifluoromethyl)-7*H*-pyrrolo[2,3-*d*]pyrimidin-2-yl)amino)-2,3-dihydrobenzo[b][1,4]dioxin-5-yl)(4-(oxetan-3-yl)piperazin-1-yl)methanone;

(425) (8-((4-((cyclopentylmethyl)amino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;

(426) 1-cyclopropyl-4-(3-methoxy-4-((4-((2-(methylsulfonyl)ethyl)amino)-5-(trifluoromethyl)-7*H*-pyrrolo[2,3-*d*]pyrimidin-2-yl)amino)phenyl)-1,4-azaphosphinane 4-oxide;

(427) (8-((4-((2-(methylsulfonyl)ethyl)amino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;

(428) 4-(4-((4-(butylamino)-5-(trifluoromethyl)-7*H*-pyrrolo[2,3-*d*]pyrimidin-2-yl)amino)-3-methoxyphenyl)-1-cyclopropyl-1,4-azaphosphinane 4-oxide;

(429) (8-((4-(butylamino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;

(430) $N^4$-ethyl-$N^2$-(8-((4-morpholinopiperidin-1-yl)sulfonyl)-2,3-dihydrobenzo[*b*][1,4]dioxin-5-yl)-5-(trifluoromethyl)-7*H*-pyrrolo[2,3-*d*]pyrimidine-2,4-diamine;

(431) $N^4$-methyl-$N^2$-(8-(morpholinosulfonyl)-2,3-dihydrobenzo[*b*][1,4]dioxin-5-yl)-5-(trifluoromethyl)-7*H*-pyrrolo[2,3-*d*]pyrimidine-2,4-diamine;

(432) $N^4$-ethyl-$N^2$-(8-(morpholinosulfonyl)-2,3-dihydrobenzo[*b*][1,4]dioxin-5-yl)-5-(trifluoromethyl)-7*H*-pyrrolo[2,3-*d*]pyrimidine-2,4-diamine;

(433) (7-((4-(cyclopropylamino)-5-(trifluoromethyl)-7*H*-pyrrolo[2,3-*d*]pyrimidin-2-yl)amino)-2,3-dihydrobenzofuran-4-yl)(morpholino)methanone;

(434) (7-((4-(cyclopropylamino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone;

(435) 4-(4-((4-(cyclopropylamino)-5-(trifluoromethyl)-7*H*-pyrrolo[2,3-*d*]pyrimidin-2-yl)amino)-3-methoxyphenyl)-1-(tetrahydro-2H-pyran-4-yl)-1,4-azaphosphinane 4-oxide;

(436) 4-(3-methoxy-4-((4-(methylamino)-5-(trifluoromethyl)-7H-pyrrolo[2,3-d]pyrimidin-2-yl)amino)phenyl)-1-(tetrahydro-2H-pyran-4-yl)-1,4-azaphosphinane 4-oxide;

(437) (7-((4-(ethylamino)-5-(trifluoromethyl)-7*H*-pyrrolo[2,3-*d*]pyrimidin-2-yl)amino)-2,3-dihydrobenzofuran-4-yl)(morpholino)methanone;

(438) (7-((4-(ethylamino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone;

(439) 4-(4-((4-(ethylamino)-5-(trifluoromethyl)-7*H*-pyrrolo[2,3-*d*]pyrimidin-2-yl)amino)-3-methoxyphenyl)-1-(tetrahydro-2*H*-pyran-4-yl)-1,4-azaphosphinane 4-oxide;
(440) 1-cyclopropyl-4-(4-((4-(isopropylamino)-5-(trifluoromethyl)-7*H*-pyrrolo[2,3-*d*]pyrimidin-2-yl)amino)-3-methoxyphenyl)-1,4-azaphosphinane 4-oxide;
(441) (8-((4-(isopropylamino)-5-(trifluoromethyl)-7*H*-pyrrolo[2,3-*d*]pyrimidin-2-yl)amino)-2,3-dihydrobenzo[*b*][1,4]dioxin-5-yl)(4-(oxetan-3-yl)piperazin-1-yl)methanone;
(442) (7-((4-((2-methoxyethyl)amino)-5-(trifluoromethyl)-7*H*-pyrrolo[2,3-*d*]pyrimidin-2-yl)amino)-2,3-dihydrobenzofuran-4-yl)(morpholino)methanone;
(443) (7-((4-((2-methoxyethyl)amino)-5-(trifluoromethyl)-7H-pyrrolo[2,3-d]pyrimidin-2-yl)amino)-2,3-dihydrobenzofuran-4-yl)(4-morpholinopiperidin-1-yl)methanone;
(444) 4-(3-methoxy-4-((4-((2-methoxyethyl)amino)-5-(trifluoromethyl)-7H-pyrrolo[2,3-d]pyrimidin-2-yl)amino)phenyl)-1-(tetrahydro-2H-pyran-4-yl)-1,4-azaphosphinane 4-oxide;
(445) morpholino(7-((4-(propylamino)-5-(trifluoromethyl)-7*H*-pyrrolo[2,3-*d*]pyrimidin-2-yl)amino)-2,3-dihydrobenzofuran-4-yl)methanone;
(446) (4-morpholinopiperidin-1-yl)(7-((4-(propylamino)-5-(trifluoromethyl)-7*H*-pyrrolo[2,3-*d*]pyrimidin-2-yl)amino)-2,3-dihydrobenzofuran-4-yl)methanone;
(447) (7-((4-(isobutylamino)-5-(trifluoromethyl)-7*H*-pyrrolo[2,3-*d*]pyrimidin-2-yl)amino)-2,3-dihydrobenzofuran-4-yl)(morpholino)methanone;
(448) (7-((4-(isobutylamino)-5-(trifluoromethyl)-7H-pyrrolo[2,3-d]pyrimidin-2-yl)amino)-2,3-dihydrobenzofuran-4-yl)(4-morpholinopiperidin-1-yl)methanone;
(449) (7-((4-(isopropylamino)-5-(trifluoromethyl)-7*H*-pyrrolo[2,3-*d*]pyrimidin-2-yl)amino)-2,3-dihydrobenzofuran-4-yl)(morpholino)methanone;
(450) (7-((4-(isopropylamino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone;
(451) (*S*)-(7-((4-(*sec*-butylamino)-5-(trifluoromethyl)-7*H*-pyrrolo[2,3-*d*]pyrimidin-2-yl)amino)-2,3-dihydrobenzofuran-4-yl)(morpholino)methanone;
(452) (*S*)-(7-((4-(*sec*-butylamino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone;
(453) (7-((4-(cyclopentylamino)-5-(trifluoromethyl)-7*H*-pyrrolo[2,3-*d*]pyrimidin-2-yl)amino)-2,3-dihydrobenzofuran-4-yl)(morpholino)methanone;
(454) (7-((4-(cyclohexylamino)-5-(trifluoromethyl)-7*H*-pyrrolo[2,3-*d*]pyrimidin-2-yl)amino)-2,3-dihydrobenzofuran-4-yl)(morpholino)methanone;
(455) (*S*)-morpholino(7-((4-((tetrahydrofuran-3-yl)amino)-5-(trifluoromethyl)-7*H*-pyrrolo[2,3-d]pyrimidin-2-yl)amino)-2,3-dihydrobenzofuran-4-yl)methanone;
(456) morpholino(7-((4-((tetrahydro-2H-pyran-4-yl)amino)-5-(trifluoromethyl)-7*H*-pyrrolo[2,3-*d*]pyrimidin-2-yl)amino)-2,3-dihydrobenzofuran-4-yl)methanone;
(457) (7-((4-((cyclopropylmethyl)amino)-5-(trifluoromethyl)-7*H*-pyrrolo[2,3-*d*]pyrimidin-2-yl)amino)-2,3-dihydrobenzofuran-4-yl)(morpholino)methanone;
(458) (7-((4-((cyclobutylmethyl)amino)-5-(trifluoromethyl)-7*H*-pyrrolo[2,3-*d*]pyrimidin-2-yl)amino)-2,3-dihydrobenzofuran-4-yl)(morpholino)methanone;
(459) (7-((4-(cyclopentylamino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone;
(460) (7-((4-(cyclohexylamino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone;
(461) (7-((4-((cyclopropylmethyl)amino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone;
(462) (7-((4-((cyclobutylmethyl)amino)-5-(trifluoromethyl)-7*H*-pyrrolo[2,3-*d*]pyrimidin-2-yl)amino)-2,3-dihydrobenzofuran-4-yl)(4-morpholinopiperidin-1-yl)methanone;
(463) (7-((4-((cyclopentylmethyl)amino)-5-(trifluoromethyl)-7H-pyrrolo[2,3-d]pyrimidin-2-yl)amino)-2,3-dihydrobenzofuran-4-yl)(morpholino)methanone;
(464) (7-((4-((cyclopentylmethyl)amino)-5-(trifluoromethyl)-7H-pyrrolo[2,3-d]pyrimidin-2-yl)amino)-2,3-dihydrobenzofuran-4-yl)(4-morpholinopiperidin-1-yl)methanone;
(465) (7-((4-(butylamino)-5-(trifluoromethyl)-7*H*-pyrrolo[2,3-*d*]pyrimidin-2-yl)amino)-2,3-dihydrobenzofuran-4-yl)(morpholino)methanone;
(466) (7-((4-(butylamino)-5-(trifluoromethyl)-7*H*-pyrrolo[2,3-d]pyrimidin-2-yl)amino)-2,3-dihydrobenzofuran-4-yl)(4-morpholinopiperidin-1-yl)methanone;
(467) *N*$^4$-ethyl-*N*$^2$-(4-((4-morpholinopiperidin-1-yl)sulfonyl)-2,3-dihydrobenzofuran-7-yl)-5-(trifluoromethyl)-7*H*-pyrrolo[2,3-*d*]pyrimidine-2,4-diamine;

(468) (4-((1*S*,4*S*)-2-oxa-5-azabicyclo[2.2.1]heptan-5-yl)piperidin-1-yl)(7-((4-(ethylamino)-5-(trifluoromethyl)-7*H*-pyrrolo[2,3-*d*]pyrimidin-2-yl)amino)-2,3-dihydrobenzofuran-4-yl)methanone;
(469) *N*4-allyl-*N*2-(2-methoxy-4-((4-morpholinopiperidin-1-yl)sulfonyl)phenyl)-5-(trifluoromethyl)-7*H*-pyrrolo[2,3-*d*]pyrimidine-2,4-diamine;
(470) 4-cyclopropyl-N-(8-(morpholinosulfonyl)-2,3-dihydrobenzo[*b*][1,4]dioxin-5-yl)-5-(trifluoromethyl)-7*H*-pyrrolo[2,3-*d*]pyrimidin-2-amine;
(471) (R)-(7-((4-cyclopropyl-5-(trifluoromethyl)-7H-pyrrolo[2,3-d]pyrimidin-2-yl)amino)-2,3-dihydrobenzofuran-4-yl)(3-morpholinopyrrolidin-1-yl)methanone;
(472) (*S*)-(7-((4-cyclopropyl-5-(trifluoromethyl)-7*H*-pyrrolo[2,3-*d*]pyrimidin-2-yl)amino)-2,3-dihydrobenzofuran-4-yl)(3-morpholinopyrrolidin-1-yl)methanone;
(473) *N*-(4-((4-((1*R*,4*R*)-2-oxa-5-azabicyclo[2.2.1]heptan-5-yl)piperidin-1-yl)sulfonyl)-2-methoxyphenyl)-4-cyclopropyl-5-(trifluoromethyl)-7*H*-pyrrolo[2,3-*d*]pyrimidin-2-amine;
(474) *N*4-allyl-*N*2-(2-methoxy-4-(morpholinosulfonyl)phenyl)-5-(trifluoromethyl)-7*H*-pyrrolo[2,3-*d*]pyrimidine-2,4-diamine;
(475) (*S*)-(7-((4-(ethylamino)-5-(trifluoromethyl)-7*H*-pyrrolo[2,3-*d*]pyrimidin-2-yl)amino)-2,3-dihydrobenzofuran-4-yl)(3-morpholinopyrrolidin-1-yl)methanone;
(476) (*R*)-(7-((4-(ethylamino)-5-(trifluoromethyl)-7*H*-pyrrolo[2,3-d]pyrimidin-2-yl)amino)-2,3-dihydrobenzofuran-4-yl)(3-morpholinopyrrolidin-1-yl)methanone; and
(477) 4-ethoxy-*N*-(2-methoxy-4-(morpholinosulfonyl)phenyl)-5-(trifluoromethyl)-7*H*-pyrrolo[2,3-d]pyrimidin-2-amine.

**10.** A method of preparing the compound represented by Formula 1, comprising:

reacting a compound represented by Formula 4 with a compound represented by Formula 3 to prepare a compound represented by Formula 2 (Step 1); and
reacting the compound represented by Formula 2 to prepare the compound represented by Formula 1:

[Scheme A]

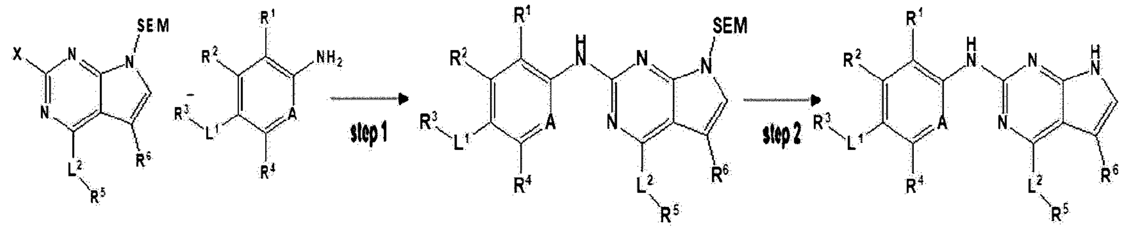


(wherein,

A represents a carbon atom or a nitrogen atom,
$R^1$ is hydrogen or a linear or branched $C_{1-6}$ alkoxy, and $R^2$ is hydrogen, or
$R^1$ and $R^2$, together with a benzene ring containing carbon atoms to which they are bonded, form a 8- to 10-membered bicyclic ring comprising one or more heteroatoms selected from the group consisting of N, O, and S,
$L^1$ is sulfonyl or carbonyl, or is absent,

when $L^1$ is sulfonyl or carbonyl, $R^3$ is a linear or branched $C_{1-6}$ alkyl, morpholinyl, piperidinyl, piperazinyl, pyrrolidinyl, amino, 2-oxa-5-azabicyclo[2.2.1]heptanyl, or azetidinyl, wherein $R^3$ is unsubstituted or substituted with one or more non-hydrogen substituents selected from the group consisting of morpholinyl, oxetanyl, 2-oxa-5-azabicyclo[2.2.1]heptanyl, piperazinyl, piperidinyl, a $C_{3-9}$ cycloalkyl, and a linear or branched $C_{1-6}$ alkyl, wherein the non-hydrogen substituents of $R^3$ are unsubstituted or substituted with a substituent selected from the group consisting of hydroxy, a $C_{3-9}$ cycloalkyl, and a linear or branched $C_{1-6}$ alkyl,
when $L^1$ is absent, $R^3$ is azaphosphinane oxide or phosphine oxide, wherein $R^3$ is unsubstituted or substituted with one or more non-hydrogen substituents selected from the group consisting of oxetanyl, a $C_{3-9}$ cycloalkyl, a linear or branched $C_{1-6}$ alkyl, vinyl, tetrahydropyranyl, and benzyl, wherein the non-hydrogen substituents of $R^3$ are unsubstituted or substituted with a $C_{1-6}$ alkoxy,

$R^4$ is hydrogen or a halogen,
$L^2$ is -NH- or -O-, or is absent,

when $L^2$ is -NH- or -O-, $R^5$ is selected from the group consisting of a linear or branched $C_{1-6}$ alkyl, a $C_{3-9}$ cycloalkyl, a 3- to 9-membered heterocycloalkyl comprising one or more O (oxygen) atoms as heteroatoms, and allyl, wherein $R^5$ is unsubstituted or substituted with one or more non-hydrogen substituents selected from the group consisting of a $C_{3-9}$ cycloalkyl, a $C_{1-6}$ alkylsulfonyl, a $C_{1-6}$ alkoxy, and a $C_{1-6}$ alkyl,
when $L^2$ is absent, $R^5$ is a linear or branched $C_{1-6}$ alkyl or a $C_{3-9}$ cycloalkyl,

$R^6$ is hydrogen, cyano, or a $C_{1-6}$ haloalkyl,
SEM is a protective group, and
X is a halogen atom.

**11.** A pharmaceutical composition for use in preventing or treating a disease associated with a protein kinase, comprising, as an active ingredient, the compound represented by Formula 1 of claim 1, or the isomer thereof, the solvate thereof, the hydrate thereof, or the pharmaceutically acceptable salt thereof, wherein the protein kinase is selected from the group consisting of CLK1, CLK2, CLK3, CLK4, CSNK1D, CSNK1E, DYRK1A, DYRK1B, DYRK2, FAK, GAK, LRRK2, LRRK2 (G2019S), PHKG1, PHKG2, PLK4, PYK2, and TTK.

**12.** The pharmaceutical composition of claim 11, wherein the disease associated with the protein kinase comprises one or more selected from the group consisting of cancer, a degenerative brain disease, and an inflammatory disease.

**13.** The pharmaceutical composition of claim 12, wherein the degenerative brain disease comprises one or more selected from the group consisting of Alzheimer's disease, Parkinson's disease, Lou Gehrig's disease, dementia, Huntington's disease, multiple sclerosis, proximal lateral sclerosis, apoplexy, stroke, and mild cognitive impairment.

**14.** The pharmaceutical composition of claim 12, wherein the inflammatory disease comprises one or more selected from the group consisting of dermatitis, allergies, gastric ulcers, duodenal ulcers, hepatitis, esophagitis, gastritis, enteritis, pancreatitis, colitis, nephritis, systemic edema, local edema, arthritis, keratitis, bronchitis, pleurisy, peritonitis, spondylitis, inflammatory pain, urethritis, cystitis, periodontitis, and gingivitis.

**15.** The pharmaceutical composition of claim 12, wherein the cancer comprises one or more selected from the group consisting of triple-negative breast cancer, brain cancer, brain tumors, benign astrocytoma, malignant astrocytoma, pituitary adenoma, meningioma, brain lymphoma, oligodendroglioma, intracranial carcinoma, ependymoma, brainstem tumors, head and neck tumors, larynx cancer, oropharyngeal cancer, nasal cavity/paranasal sinus cancer, nasopharyngeal cancer, salivary gland cancer, hypopharyngeal cancer, thyroid cancer, oral cancer, thoracic tumors, small cell lung cancer, non-small cell lung cancer, thymus cancer, mediastinal tumors, esophageal cancer, breast cancer, male breast cancer, abdominal tumors, stomach cancer, liver cancer, gallbladder cancer, biliary cancer, pancreatic cancer, small bowel cancer, colon cancer, rectal cancer, anal cancer, bladder cancer, kidney cancer, male genital tumors, penile cancer, prostate cancer, female genital tumors, cervical cancer, endometrial cancer, ovarian cancer, uterine sarcoma, vaginal cancer, female external genital cell cancer, female urethral cancer, and skin cancer.

**16.** A method of preventing or treating a disease associated with a protein kinase, comprising:

administering the compound of Formula 1 of claim 1, or the isomer thereof, the solvate thereof, the hydrate thereof, or the pharmaceutically acceptable salt thereof to a subject in need thereof,
wherein the protein kinase comprises one or more selected from the group consisting of CLK1, CLK2, CLK3, CLK4, CSNK1D, CSNK1E, DYRK1A, DYRK1B, DYRK2, FAK, GAK, LRRK2, LRRK2 (G2019S), PHKG1, PHKG2, PLK4, PYK2, and TTK.

**17.** The method of claim 16, wherein the disease associated with the protein kinase comprises one or more selected from the group consisting of cancer, a degenerative brain disease, and an inflammatory disease.

**18.** A use of the compound of claim 1, or the isomer thereof, the solvate thereof, the hydrate thereof, or the pharmaceutically acceptable salt thereof for use in preventing or treating a disease associated with a protein kinase, wherein the protein kinase comprises one or more selected from the group consisting of CLK1, CLK2, CLK3, CLK4, CSNK1D,

CSNK1E, DYRK1A, DYRK1B, DYRK2, FAK, GAK, LRRK2, LRRK2 (G2019S), PHKG1, PHKG2, PLK4, PYK2, and TTK.

**19.** The use of claim 18, wherein the disease associated with the protein kinase comprises one or more diseases selected from the group consisting of cancer, a degenerative brain disease, and an inflammatory disease.

**20.** A use of the compound of claim 1, or the isomer thereof, the solvate thereof, the hydrate thereof, or the pharmaceutically acceptable salt thereof for use in preparing medicaments for the prevention or treatment of a disease associated with a protein kinase, wherein the protein kinase comprises one or more selected from the group consisting of CLK1, CLK2, CLK3, CLK4, CSNK1D, CSNK1E, DYRK1A, DYRK1B, DYRK2, FAK, GAK, LRRK2, LRRK2 (G2019S), PHKG1, PHKG2, PLK4, PYK2, and TTK.

**21.** The use of claim 20, wherein the disease associated with the protein kinase comprises one or more diseases selected from the group consisting of cancer, a degenerative brain disease, and an inflammatory disease.

[Figure 1]

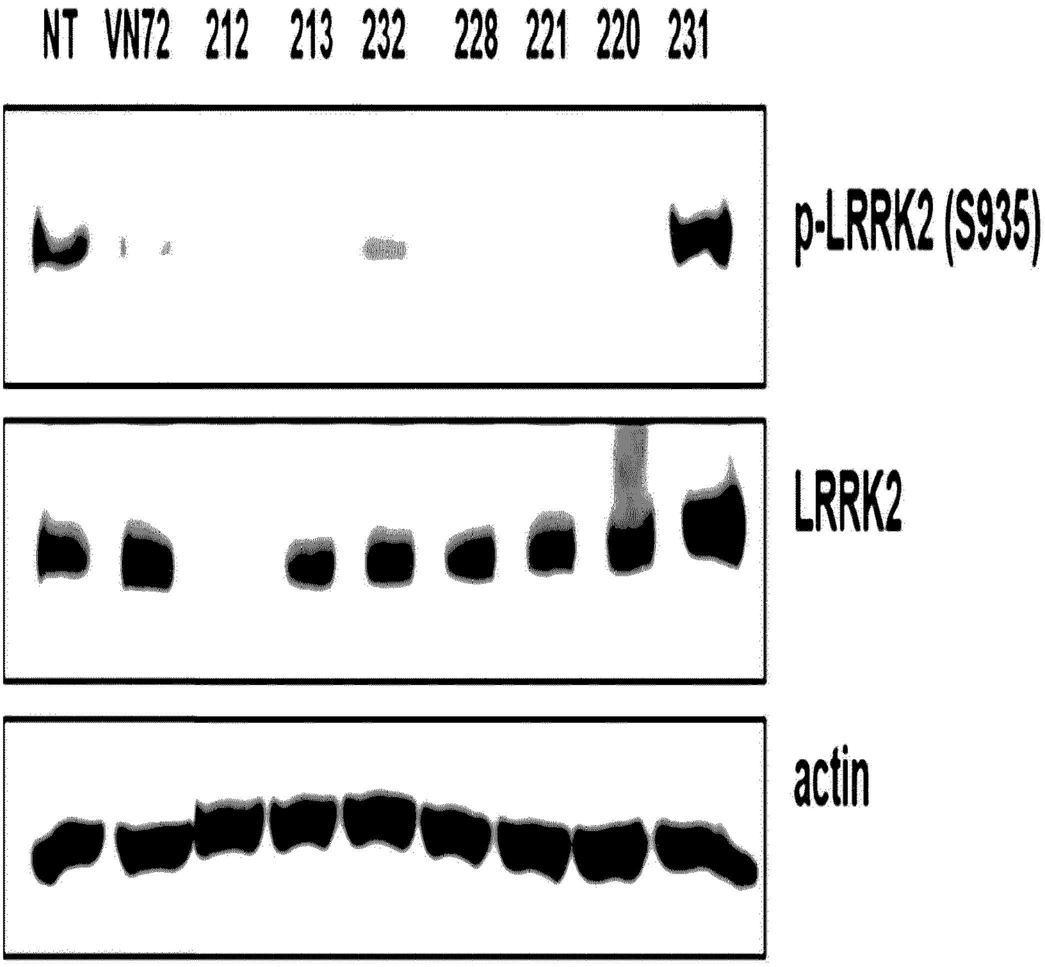

[Figure 2]

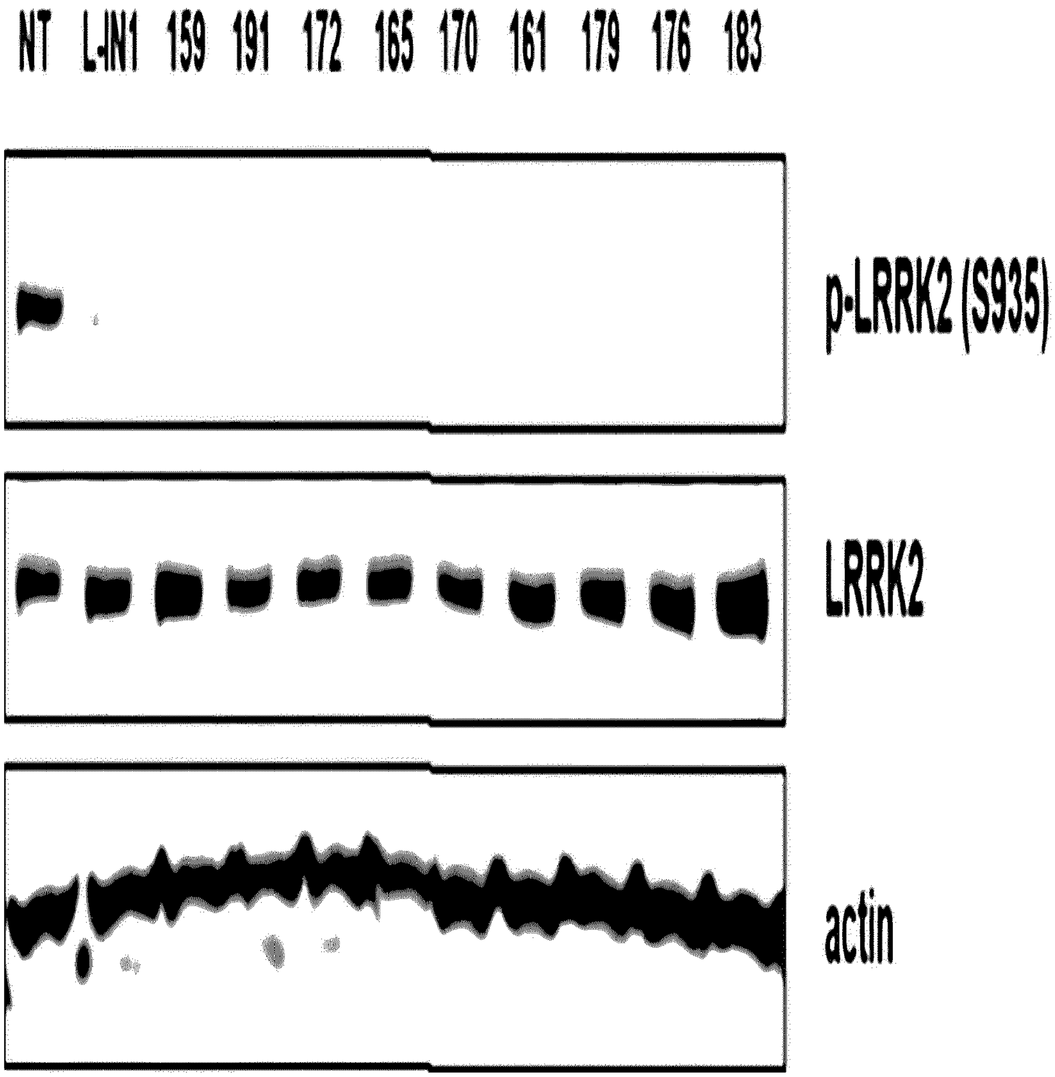
NT
L-IN1
159
191
172
165
170
161
179
176
183
p-LRRK2 (S935)
LRRK2
actin

[Figure 3]

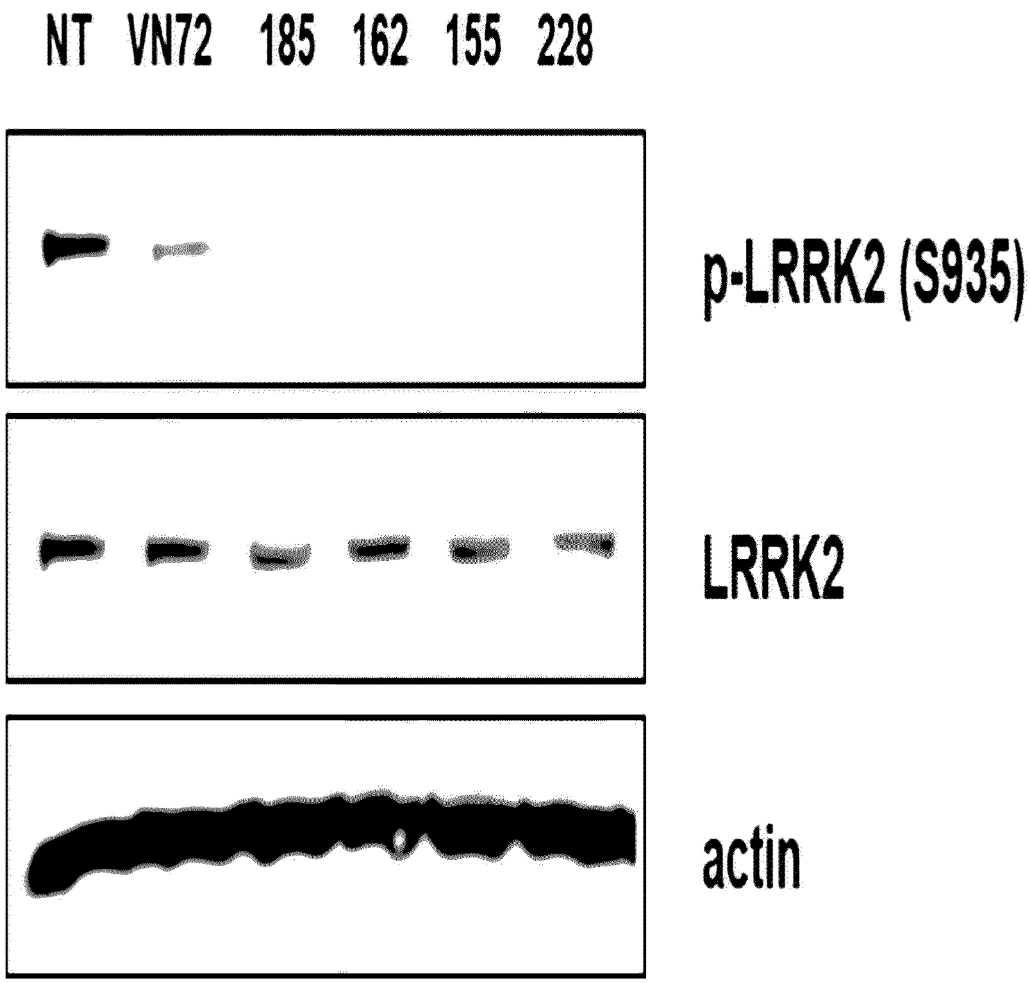

## INTERNATIONAL SEARCH REPORT

International application No.
**PCT/KR2020/000960**

A. CLASSIFICATION OF SUBJECT MATTER
***C07D 487/04(2006.01)i, C07F 9/6584(2006.01)i, C07F 9/6561(2006.01)i, A61K 31/519(2006.01)i, A61K 31/675(2006.01)i, A61P 25/28(2006.01)i, A61P 29/00(2006.01)i, A61P 35/00(2006.01)i***
According to International Patent Classification (IPC) or to both national classification and IPC

B. FIELDS SEARCHED

Minimum documentation searched (classification system followed by classification symbols)
C07D 487/04; A61K 31/519; C07D 413/12; C07D 487/00; C07F 9/6509; C07F 9/6558; C07F 9/6584; C07F 9/6561; A61K 31/675; A61P 25/28; A61P 29/00; A61P 35/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal), STN (Registry, Caplus), Google & Keywords: pyrrolo[2,3-d]pyrimidine, protein kinase, LRRK2

C. DOCUMENTS CONSIDERED TO BE RELEVANT

| Category* | Citation of document, with indication, where appropriate, of the relevant passages | Relevant to claim No. |
|---|---|---|
| X<br><br><br>A | WO 2018-155916 A2 (DAEGU-GYEONGBUK MEDICAL INNOVATION FOUNDATION et al.) 30 August 2018<br>See claims 1, 7, 9-11; paragraphs [255], [256], [267], [299], [434], [442]. | 1-4,8-15,18-21<br><br><br>5-7 |
| X | MOJZYCH, M. et al. Synthesis and kinase inhibitory activity of new sulfonamide derivatives of pyrazolo[4,3-e][1,2,4]triazines. European Journal of Medicinal Chemistry. 2014, vol. 78, pages 217-224<br>See abstract; formula 1. | 1-6,8-12,15,18-21 |
| X | US 2014-0066406 A1 (ARIAD PHARMACEUTICALS, INC.) 06 March 2014<br>See claims 21, 22, 30, 36; paragraph [0362]; pages 188, 195. | 1,2,7-12,15,18-21 |
| X | HATCHER, J. M. et al. Discovery of a Pyrrolopyrimidine (JH-II-127), a Highly Potent, Selective, and Brain Penetrant LRRK2 Inhibitor. ACS Med. Chem. Lett.. 2015, vol. 6, pages 584-589<br>See abstract; formula 1. | 1-4,8-13,18-21 |
| X | WO 2016-130920 A2 (DANA-FARBER CANCER INSTITUTE, INC.) 18 August 2016<br>See claims 1, 54. | 1-6,8-15,18-21 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 MAY 2020 (01.05.2020) | **01 MAY 2020 (01.05.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**

International application No.
**PCT/KR2020/000960**

C (Continuation). DOCUMENTS CONSIDERED TO BE RELEVANT

| Category* | Citation of document, with indication, where appropriate, of the relevant passages | Relevant to claim No. |
|---|---|---|
| X | WO 2015-113451 A1 (GLAXOSMITHKLINE INTELLECTUAL PROPERTY DEVELOPMENT LIMITED) 06 August 2015<br>See claims 1, 16; examples 62, 63. | 1-4,8-13,18-21 |

Form PCT/ISA/210 (continuation of second sheet) (January 2015)

## INTERNATIONAL SEARCH REPORT

International application No.
PCT/KR2020/000960

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **16, 17**
because they relate to subject matter not required to be searched by this Authority, namely:
Claims 16 and 17 pertain to a method for treatment of the human body by therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.
**PCT/KR2020/000960**

| Patent document cited in search report | Publication date | Patent family member | Publication date |
|---|---|---|---|
| WO 2018-155916 A2 | 30/08/2018 | EP 3587422 A2 | 01/01/2020 |
| | | KR 10-2018-0097162 A | 30/08/2018 |
| | | KR 10-2018-0132575 A | 12/12/2018 |
| | | KR 10-2092812 B1 | 25/03/2020 |
| | | WO 2018-155916 A3 | 06/12/2018 |
| US 2014-0066406 A1 | 06/03/2014 | AU 2009-248923 A1 | 26/11/2009 |
| | | AU 2009-248923 B2 | 29/01/2015 |
| | | BR PI0908637 A2 | 23/10/2018 |
| | | CA 2723961 A1 | 26/11/2009 |
| | | CA 2723961 C | 21/03/2017 |
| | | CN 102105150 A | 22/06/2011 |
| | | CN 102105150 B | 12/03/2014 |
| | | CY 1119534 T1 | 04/04/2018 |
| | | DK 2300013 T3 | 04/12/2017 |
| | | EA 029131 B1 | 28/02/2018 |
| | | EA 201071339 A1 | 30/06/2011 |
| | | EP 2300013 A1 | 30/03/2011 |
| | | EP 2300013 A4 | 12/09/2012 |
| | | EP 2300013 B1 | 06/09/2017 |
| | | EP 3210609 A1 | 30/08/2017 |
| | | ES 2645689 T3 | 07/12/2017 |
| | | HK 1158497 A1 | 14/11/2014 |
| | | HR P20171534 T1 | 26/01/2018 |
| | | HR P20171534 T8 | 24/08/2018 |
| | | HU E035029 T2 | 28/03/2018 |
| | | IL 208716 A | 28/02/2018 |
| | | IL 257083 A | 29/08/2019 |
| | | JP 2011-523646 A | 18/08/2011 |
| | | JP 2015-163621 A | 10/09/2015 |
| | | JP 2017-186345 A | 12/10/2017 |
| | | JP 2018-065864 A | 26/04/2018 |
| | | JP 2019-094344 A | 20/06/2019 |
| | | JP 6190415 B2 | 30/08/2017 |
| | | JP 6271064 B2 | 31/01/2018 |
| | | JP 6483233 B2 | 13/03/2019 |
| | | KR 10-1781605 B1 | 25/09/2017 |
| | | KR 10-1860057 B1 | 21/05/2018 |
| | | KR 10-1956261 B1 | 08/03/2019 |
| | | KR 10-2011-0010801 A | 07/02/2011 |
| | | KR 10-2016-0132127 A | 16/11/2016 |
| | | KR 10-2018-0014882 A | 09/02/2018 |
| | | LT 2300013 T | 27/12/2017 |
| | | LT PA2019510 I1 | 10/06/2019 |
| | | MX 2010012703 A | 21/12/2010 |
| | | MX 353308 B | 08/01/2018 |
| | | NL 300990 I1 | 29/05/2019 |
| | | NL 300990 I2 | 11/07/2019 |
| | | NO 2300013 T3 | 03/02/2018 |

Form PCT/ISA/210 (patent family annex) (January 2015)

**INTERNATIONAL SEARCH REPORT**
Information on patent family members

International application No.
**PCT/KR2020/000960**

| Patent document cited in search report | Publication date | Patent family member | Publication date |
|---|---|---|---|
| | | PL 2300013 T3 | 30/05/2018 |
| | | PT 2300013 T | 31/10/2017 |
| | | SI 2300013 T1 | 30/03/2018 |
| | | US 2012-0202776 A1 | 09/08/2012 |
| | | US 2013-0225527 A1 | 29/08/2013 |
| | | US 2013-0225528 A1 | 29/08/2013 |
| | | US 2015-0225436 A1 | 13/08/2015 |
| | | US 2016-0376297 A1 | 29/12/2016 |
| | | US 2017-0218000 A1 | 03/08/2017 |
| | | US 2020-0048288 A1 | 13/02/2020 |
| | | US 9012462 B2 | 21/04/2015 |
| | | US 9273077 B2 | 01/03/2016 |
| | | WO 2009-143389 A1 | 26/11/2009 |
| WO 2016-130920 A2 | 18/08/2016 | AU 2016-219102 A1 | 17/08/2017 |
| | | CA 2976109 A1 | 18/08/2016 |
| | | CN 107801397 A | 13/03/2018 |
| | | EP 3256475 A2 | 20/12/2017 |
| | | EP 3256475 A4 | 13/02/2019 |
| | | US 2018-0244676 A1 | 30/08/2018 |
| | | WO 2016-130920 A3 | 20/10/2016 |
| WO 2015-113451 A1 | 06/08/2015 | AU 2015-210554 A1 | 07/07/2016 |
| | | AU 2018-200271 A1 | 01/02/2018 |
| | | CA 2937430 A1 | 06/08/2015 |
| | | CN 105980388 A | 28/09/2016 |
| | | CN 105980388 B | 16/01/2018 |
| | | EP 3099694 A1 | 07/12/2016 |
| | | EP 3099694 A4 | 14/06/2017 |
| | | EP 3099694 B1 | 16/01/2019 |
| | | ES 2717757 T3 | 25/06/2019 |
| | | JP 2017-504635 A | 09/02/2017 |
| | | JP 6474826 B2 | 27/02/2019 |
| | | KR 10-2016-0106623 A | 12/09/2016 |
| | | RU 2016134751 A | 02/03/2018 |
| | | US 2017-0022204 A1 | 26/01/2017 |
| | | US 9815841 B2 | 14/11/2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *ACS Med. Chem. Lett.,* 2013, vol. 4 (1), 85-90